(19) Europäisches Patentamt
European Patent Office
Office européen des brevets

(11) **EP 1 947 187 B1**

(12) **EUROPEAN PATENT SPECIFICATION**


(45) Date of publication and mention of the grant of the patent:
**30.03.2011 Bulletin 2011/13**

(51) Int Cl.:
*C12N 15/62* (2006.01) *C07K 14/22* (2006.01)
*C07K 19/00* (2006.01)

(21) Application number: **08075111.8**

(22) Date of filing: **28.02.2001**


(54) **Hybrid expression of neisserial proteins**

Hybride Expression Neisserscher Proteine

Expression hybride de protéines neisseriales


(84) Designated Contracting States:
**AT BE CH CY DE DK ES FI FR GB GR IE IT LI LU MC NL PT SE TR**

(30) Priority: **28.02.2000 GB 0004695**
**13.11.2000 GB 0027675**

(43) Date of publication of application:
**23.07.2008 Bulletin 2008/30**

(62) Document number(s) of the earlier application(s) in accordance with Art. 76 EPC:
**01914098.7 / 1 261 723**



(73) Proprietor: **Novartis Vaccines and Diagnostics S.r.l.**
**53100 Siena (SI) (IT)**

(72) Inventors:
- **Arico, Maria Beatrice**
**53100 Siena (IT)**
- **Comanducci, Maurizio**
**53100 Siena (IT)**
- **Galeotti, Cesira**
**53100 Siena (IT)**
- **Masignani, Vega**
**53100 Siena (IT)**
- **Giuliani, Marzia Monica**
**53100 Siena (IT)**
- **Pizza, Mariagrazia**
**53100 Siena (IT)**

(74) Representative: **Marshall, Cameron John et al**
**Carpmaels & Ransford**
**One Southampton Row**
**London**
**WC1B 5HA (GB)**



(56) References cited:
**WO-A-92/16643 WO-A-99/36544**
**US-A- 5 547 670**

- **GUILLEN G ET AL: "EXPRESSION IN ESCHERICHIA COLI AND IMMUNOLOGICAL CHARACTERIZATION OF A HYBRID CLASS 1-P64K PROTEIN FROM NEISSERIA MENINGITIDIS" BIOTECNOLOGIA APLICADA, CU,LA HABANA, vol. 13, no. 4, 1 October 1996 (1996-10-01), pages 271-275, XP000671540 ISSN: 1027-2852**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).





Printed by Jouve, 75001 PARIS (FR)

## Description

### TECHNICAL FIELD

This invention is in the field of protein expression. In particular, it relates to the heterologous expression of proteins from *Neisseria* (*e.g. N.gonorrhoeae* or, preferably, *N.meningitidis*).

### BACKGROUND ART

International patent applications WO99/24578, WO99/36544, WO99/57280 and WO00/22430 disclose proteins from *Neisseria meningitidis* and *Neisseria gonorrhoeae.* These proteins are typically described as being expressed in *E.coli (i.e.* heterologous expression) as either N-ternninal GST-fusions or C-terminal His-tag fusions, although other expression systems, including expression in native *Neisseria,* are also disclosed.

Gruiller et al (Biotechnologia Adicada, 13(4), pg 1027-2852 (1996)) discloses a fusion of Neisseria P1.15 protein with Neisseria P64-k protein which is expressed in E.coli.

It is an object of the present invention to provide alternative and improved approaches for the heterologous expression of these proteins. These approaches will typically affect the level of expression, the ease of purification, the cellular localisation of expression, and/or the immunological properties of the expressed protein.

### DISCLOSURE OF THE INVENTION

In accordance with the invention, two proteins of the invention are expressed as a single hybrid protein. It is preferred that no non-Neisserial fusion partner (*e.g.* GST or poly-His) is used.

This offers two advantages. Firstly, a protein that may be unstable or poorly expressed on its own can be assisted by adding a suitable hybrid partner that overcomes the problem.

Secondly, commercial manufacture is simplified - only one expression and purification need be employed in order to produce two separately-useful proteins.

Thus the invention provides a method for the simultaneous heterologous expression of two proteins of the invention, in which said two or more proteins of the invention are fused (*i.e.* they are translated as a single polypeptide chain).

The method will typically involve the steps of obtaining a first nucleic acid encoding a first protein of the invention; obtaining a second nucleic acid encoding a second protein of the invention; ligating the first and second nucleic acids. The resulting nucleic acid may be inserted into an expression vector, or may already be part of an expression vector.

The hybrid protein can be represented by the formula $NH_2$-A-B-COOH. A comprises protein 287, and B comprises protein 961.

287, is its poly-glycine deletions (ΔG) form ΔG-287.

The hybrid protein of the invention is ΔG287-961.

287 is used at the N-terminus as a ΔG form of 287 as the N-terminus of a hybrid with 961.

287 is preferably from strain 2996 or from strain 394/98. Domain forms of 961 may be used.

Alignments of polymorphic forms of ORF46, 287, 919 and 953 are disclosed in WO00/66741. Any of these polymorphs can be used according to the present invention.

Preferably, the constituent proteins (A and B) in a hybrid protein according to the invention will be from the same strain.

The fused proteins in the hybrid are joined directly

The fused proteins may lack native leader peptides or may include the leader peptide sequence of the N-terminal fusion partner

#### *Host*

It is preferred to utilise a heterologous host. The heterologous host may be prokaryotic or eukaryotic. It is preferably *E.coli,* but other suitable hosts include *Bacillus subtilis; Vibrio cholerae, Salmonella typhi, Salmonenna typhimurium Neisseria meningitidis, Neisseria. gonorrhoeae, Neisseria lactamica, Neisseria cinerea, Mycobateria (e.g. M.tuberculosis),* yeast etc.

*Sequences*

The invention also provides a protein comprising the sequences of SEQ ID NO 8

The degree of 'sequence identity' of the proteins is greater than 70%. This includes mutants and allelic variants [*e.g.* see WO00/66741]. Identity is preferably determined by the Smith-Waterman homology search algorithm as implemented in the MPSRCH program (Oxford Molecular), using an affine gap search with parameters *gap open penalty=12 and gap extension penalty=1.*

Preferred proteins of the invention are found in *N.meningitidis* serogroup B.

Preferred proteins for use according to the invention are those-of serogroup B *N.meniingitidis* strain 2996 or strain 394/98 (a New Zealand strain). Unless otherwise stated, proteins mentioned herein are from *N.meningitidis* strain 2996. It will be appreciated, however, that the invention is not in general limited by strain. References to a particular protein (*e.g.* '287', '919' *etc.*) may be taken to include that protein from any strain.

## BRIEF DESCRIPTION OF DRAWINGS

Figures 1 and 2 show hybrid proteins according to the invention.

## MODES FOR CARRYING OUT THE INVENTION

*Example 1 hybrids of ΔG287*

The deletion of the $(Gly)_6$ sequence in 287 was found to have a dramatic effect on protein expression. The protein lacking the N-terminalamino acids up to GGGGGG is called 'ΔG287'. In strain MC58, its basic sequence (leader peptide underlined) is:

```
     SPDVKS ADTLSKPAAP VVSEKETEAK EDAPQAGSQG QGAPSAQGSQ DMAAVSEENT
GNGGAVTADN PKNEDEVAQN DMPQNAAGTD SSTPNHTPDP NMLAGNMENQ ATDAGESSQP
ANQPDMANAA DGMQGDDPSA GGQNAGNTAA QGANQAGNNQ AAGSSDPIPA SNPAPANGGS
NFGRVDLANG VLIDGPSQNI TLTHCKGDSC SGNNFLDEEV QLKSEFEKLS DADKISNYKK
DGKNDKFVGL VADSVQMKGI NQYIIFYKPK PTSFARFRRS ARSRRSLPAE MPLIPVNQAD
TLIVDGEAVS LTGHSGNIFA PEGNYRYLTY GAEKLPGGSY ALRVQGEPAK GEMLAGAAVY
NGEVLHFHTE NGRPYPTRGR FAAKVDFGSK SVDGIIDSGD DLHMGTQKFK AAIDGNGFKG
TWTENGSGDV SGKFYGPAGE EVAGKYSYRP TDAEKGGFGV FAGKKEQD*
```

ΔG287, with or without His-tag ('ΔG287-His'and 'ΔG287K', respectively), are expressed at very good levels in comparison witch the '287-His' or '287 untagged'.

On the basis of gene variability data, variants of ΔG287-His were expressed in E. coli from a number of MenB strains, in particular from strains 2996, MC58, 1000, and BZ232. The results were also good - each of these gave high ELISA titres and also serum bactericidal titres of >8192. ΔG287K, expressed from pET-24b, gave excellent titres in ELISA and the serum bactericidal assay.

ΔG287 was fused directly in-frame upstream of 961 (sequence shown below)

**ΔG287-961**

```
ATGGCTAGCCCCGATGTTAAATCGGCGGACACGCTGTCAAAACCGGCCGCTCCTGTTGTTGCTGAAAAAGAGACAGAG
GTAAAAGAAGATGCGCCACAGGCAGGTTCTCAAGGACAGGGCGCGCCATCCACACAAGGCAGCCAAGATATGGCGGCA
GTTTCGGCAGAAAATACAGGCAATGGCGGTGCGGCAACAACGGACAAACCCAAAAATGAAGACGAGGGACCGCAAAAT
GATATGCCGCAAAATTCCGCCGAATCCGCAAATCAAACAGGGAACAACCAACCCGCCGATTCTTCAGATTCCGCCCCC
GCGTCAAACCCTGCACCTGCGAATGGCGGTAGCAATTTTGGAAGGGTTGATTTGGCTAATGGCGTTTTGATTGATGGG
CCGTCGCAAAATATAACGTTGACCCACTGTAAAGGCGATTCTTGTAATGGTGATAATTTATTGGATGAAGAAGCACCG
TCAAAATCAGAATTTGAAAATTTAAATGAGTCTGAACGAATTGAGAAATATAAGAAAGATGGGAAAAGCGATAAATTT
ACTAATTTGGTTGCGACAGCAGTTCAAGCTAATGGAACTAACAAATATGTCATCATTTATAAAGACAAGTCCGCTTCA
TCTTCATCTGCGCGATTCAGGCGTTCTGCACGGTCGAGGAGGTCGCTTCCTGCCGAGATGCCGCTAATCCCCGTCAAT
CAGGCGGATACGCTGATTGTCGATGGGGAAGCGGTCAGCCTGACGGGGCATTCCGGCAATATCTTCGCGCCCGAAGGG
AATTACCGGTATCTGACTTACGGGGCGGAAAAATTGCCCGGCGGATCGTATGCCCTCCGTGTGCAAGGCGAACCGGCA
AAAGGCGAAATGCTTGCTGGCACGGCCGTGTACAACGGCGAAGTGCTGCATTTTCATACGGAAAACGGCCGTCCGTAC
CCGACTAGAGGCAGGTTTGCCGCAAAAGTCGATTTCGGCAGCAAATCTGTGGACGGCATTATCGACAGCGGCGATGAT
TTGCATATGGGTACGCAAAAATTCAAAGCCGCCATCGATGGAAACGGCTTTAAGGGGACTTGGACGGAAAATGGCGGC
GGGGATGTTTCCGGAAGGTTTTACGGCCCGGCCGGCGAGGAAGTGGCGGGAAAATACAGCTATCGCCCGACAGATGCG
GAAAAGGGCGGATTCGGCGTGTTTGCCGGCAAAAAAGAGCAGGATGGATCCGGAGGAGGAGGAGCCACAAACGACGAC
GATGTTAAAAAAGCTGCCACTGTGGCCATTGCTGCTGCCTACAACAATGGCCAAGAAATCAACGGTTTCAAAGCTGGA
GAGACCATCTACGACATTGATGAAGACGGCACAATTACCAAAAAAGACGCAACTGCAGCCGATGTTGAAGCCGACGAC
TTTAAAGGTCTGGGTCTGAAAAAAGTCGTGACTAACCTGACCAAAACCGTCAATGAAAACAAACAAAACGTCGATGCC
AAAGTAAAAGCTGCAGAATCTGAAATAGAAAAGTTAACAACCAAGTTAGCAGACACTGATGCCGCTTTAGCAGATACT
GATGCCGCTCTGGATGCAACCACCAACGCCTTGAATAAATTGGGAGAAAATATAACGACATTTGCTGAAGAGACTAAG
ACAAATATCGTAAAAATTGATGAAAAATTAGAAGCCGTGGCTGATACCGTCGACAAGCATGCCGAAGCATTCAACGAT
ATCGCCGATTCATTGGATGAAACCAACACTAAGGCAGACGAAGCCGTCAAAACCGCCAATGAAGCCAAACAGACGGCC
GAAGAAACCAAACAAAACGTCGATGCCAAAGTAAAAGCTGCAGAAACTGCAGCAGGCAAAGCCGAAGCTGCCGCTGGC
ACAGCTAATACTGCAGCCGACAAGGCCGAAGCTGTCGCTGCAAAAGTTACCGACATCAAAGCTGATATCGCTACGAAC
AAAGATAATATTGCTAAAAAAGCAAACAGTGCCGACGTGTACACCAGAGAAGAGTCTGACAGCAAATTTGTCAGAATT
GATGGTCTGAACGCTACTACCGAAAAATTGGACACACGCTTGGCTTCTGCTGAAAAATCCATTGCCGATCACGATACT
CGCCTGAACGGTTTGGATAAAACAGTGTCAGACCTGCGCAAAGAAACCCGCCAAGGCCTTGCAGAACAAGCCGCGCTC
TCCGGTCTGTTCCAACCTTACAACGTGGGTCGGTTCAATGTAACGGCTGCAGTCGGCGGCTACAAATCCGAATCGGCA
GTCGCCATCGGTACCGGCTTCCGCTTTACCGAAAACTTTGCCGCCAAAGCAGGCGTGGCAGTCGGCACTTCGTCCGGT
TCTTCCGCAGCCTACCATGTCGGCGTCAATTACGAGTGGTAACTCGAG
```

```
  1  MASPDVKSAD TLSKPAAPVV AEKETEVKED APQAGSQGQG APSTQGSQDM
 51  AAVSAENTGN GGAATTDKPK NEDEGPQNDM PQNSAESANQ TGNNQPADSS
101  DSAPASNPAP ANGGSNFGRV DLANGVLIDG PSQNITLTHC KGDSCNGDNL
151  LDEEAPSKSE FENLNESERI EKYKKDGKSD KFTNLVATAV QANGTNKYVI
201  IYKDKSASSS SARFRRSARS RRSLPAEMPL IPVNQADTLI VDGEAVSLTG
251  HSGNIFAPEG NYRYLTYGAE KLPGGSYALR VQGEPAKGEM LAGTAVYNGE
301  VLHFHTENGR PYPTRGRFAA KVDFGSKSVD GIIDSGDDLH MGTQKFKAAI
351  DGNGFKGTWT ENGGGDVSGR FYGPAGEEVA GKYSYRPTDA EKGGFGVFAG
401  KKEQDGSGGG GATNDDDVKK AATVAIAAAY NNGQEINGFK AGETIYDIDE
451  DGTITKKDAT AADVEADDFK GLGLKKVVTN LTKTVNENKQ NVDAKVKAAE
501  SEIEKLTTKL ADTDAALADT DAALDATTNA LNKLGENITT FAEETKTNIV
551  KIDEKLEAVA DTVDKHAEAF NDIADSLDET NTKADEAVKT ANEAKQTAEE
601  TKQNVDAKVK AAETAAGKAE AAAGTANTAA DKAEAVAAKV TDIKADIATN
651  KDNIAKKANS ADVYTREESD SKFVRIDGLN ATTEKLDTRL ASAEKSIADH
701  DTRLNGLDKT VSDLRKETRQ GLAEQAALSG LFQPYNVGRF NVTAAVGGYK
751  SESAVAIGTG FRFTENFAAK AGVAVGTSSG SSAAYHVGVN YEW*
```

| | ELISA | Bactericidal |
|---|---|---|
| ΔG287-961-His | 108627 | 65536 |

The same hybrid proteins were made using New Zealand strain 394/98 rather than 2996:

**ΔG287NZ-961**

ATGGCTAGCCCCGATGTCAAGTCGGCGGACACGCTGTCAAAACCTGCCGCCCCTGTTGTTTCTGAAAAAGAGACAGAG
GCAAAGGAAGATGCGCCACAGGCAGGTTCTCAAGGACAGGGCGCGCCATCCGCACAAGGCGGTCAAGATATGGCGGCG
GTTTCGGAAGAAAATACAGGCAATGGCGGTGCGGCAGCAACGGACAAACCCAAAAATGAAGACGAGGGGGCGCAAAAT
GATATGCCGCAAAATGCCGCCGATACAGATAGTTTGACACCGAATCACACCCCGGCTTCGAATATGCCGGCCGGAAAT
ATGGAAAACCAAGCACCGGATGCCGGGGAATCGGAGCAGCCGGCAAACCAACCGGATATGGCAAATACGGCGGACGGA
ATGCAGGGTGACGATCCGTCGGCAGGCGGGGAAAATGCCGGCAATACGGCTGCCCAAGGTACAAATCAAGCCGAAAAC
AATCAAACCGCCGGTTCTCAAAATCCTGCCTCTTCAACCAATCCTAGCGCCACGAATAGCGGTGGTGATTTTGGAAGG
ACGAACGTGGGCAATTCTGTTGTGATTGACGGGCCGTCGCAAAATATAACGTTGACCCACTGTAAAGGCGATTCTTGT
AGTGGCAATAATTTCTTGGATGAAGAAGTACAGCTAAAATCAGAATTTGAAAAATTAAGTGATGCAGACAAAATAAGT
AATTACAAGAAAGATGGGAAGAATGACGGGAAGAATGATAAATTTGTCGGTTTGGTTGCCGATAGTGTGCAGATGAAG
GGAATCAATCAATATATTATCTTTTATAAACCTAAACCCACTTCATTTGCGCGATTTAGGCGTTCTGCACGGTCGAGG
CGGTCGCTTCCGGCCGAGATGCCGCTGATTCCCGTCAATCAGGCGGATACGCTGATTGTCGATGGGGAAGCGGTCAGC
CTGACGGGGCATTCCGGCAATATCTTCGCGCCCGAAGGGAATTACCGGTATCTGACTTACGGGGCGGAAAAATTGCCC
GGCGGATCGTATGCCCTCCGTGTTCAAGGCGAACCTTCAAAAGGCGAAATGCTCGCGGGCACGGCAGTGTACAACGGC
GAAGTGCTGCATTTTCATACGGAAAACGGCCGTCCGTCCCCGTCCAGAGGCAGGTTTGCCGCAAAAGTCGATTTCGGC
AGCAAATCTGTGGACGGCATTATCGACAGCGGCGATGGTTTGCATATGGGTACGCAAAAATTCAAAGCCGCCATCGAT
GGAAACGGCTTTAAGGGGACTTGGACGGAAAATGGCGGCGGGGATGTTTCCGGAAAGTTTTACGGCCCGGCCGGCGAG
GAAGTGGCGGGAAAATACAGCTATCGCCCAACAGATGCGGAAAAGGGCGGATTCGGCGTGTTTGCCGGCAAAAAAGAG
CAGGATGGATCCGGAGGAGGAGGAGCCACAAACGACGACGATGTTAAAAAAGCTGCCACTGTGGCCATTGCTGCTGCC
TACAACAATGGCCAAGAAATCAACGGTTTCAAAGCTGGAGAGACCATCTACGACATTGATGAAGACGGCACAATTACC
AAAAAAGACGCAACTGCAGCCGATGTTGAAGCCGACGACTTTAAAGGTCTGGGTCTGAAAAAAGTCGTGACTAACCTG
ACCAAAACCGTCAATGAAAACAAACAAAACGTCGATGCCAAAGTAAAAGCTGCAGAATCTGAAATAGAAAAGTTAACA
ACCAAGTTAGCAGACACTGATGCCGCTTTAGCAGATACTGATGCCGCTCTGGATGCAACCACCAACGCCTTGAATAAA
TTGGGAGAAAATATAACGACATTTGCTGAAGAGACTAAGACAAATATCGTAAAAATTGATGAAAAATTAGAAGCCGTG
GCTGATACCGTCGACAAGCATGCCGAAGCATTCAACGATATCGCCGATTCATTGGATGAAACCAACACTAAGGCAGAC
GAAGCCGTCAAAACCGCCAATGAAGCCAAACAGACGGCCGAAGAAACCAAACAAAACGTCGATGCCAAAGTAAAAGCT
GCAGAAACTGCAGCAGGCAAAGCCGAAGCTGCCGCTGGCACAGCTAATACTGCAGCCGACAAGGCCGAAGCTGTCGCT
GCAAAAGTTACCGACATCAAAGCTGATATCGCTACGAACAAAGATAATATTGCTAAAAAAGCAAACAGTGCCGACGTG
TACACCAGAGAAGAGTCTGACAGCAAATTTGTCAGAATTGATGGTCTGAACGCTACTACCGAAAAATTGGACACACGC
TTGGCTTCTGCTGAAAAATCCATTGCCGATCACGATACTCGCCTGAACGGTTTGGATAAAACAGTGTCAGACCTGCGC
AAAGAAACCCGCCAAGGCCTTGCAGAACAAGCCGCGCTCTCCGGTCTGTTCCAACCTTACAACGTGGGTCGGTTCAAT
GTAACGGCTGCAGTCGGCGGCTACAAATCCGAATCGGCAGTCGCCATCGGTACCGGCTTCCGCTTTACCGAAAACTTT
GCCGCCAAAGCAGGCGTGGCAGTCGGCACTTCGTCCGGTTCTTCCGCAGCCTACCATGTCGGCGTCAATTACGAGTGG
TAAAAGCTT

```
1    MASPDVKSAD TLSKPAAPVV SEKETEAKED APQAGSQGQG APSAQGGQDM
51   AAVSEENTGN GGAAATDKPK NEDEGAQNDM PQNAADTDSL TPNHTPASNM
101  PAGNMENQAP DAGESEQPAN QPDMANTADG MQGDDPSAGG ENAGNTAAQG
151  TNQAENNQTA GSQNPASSTN PSATNSGGDF GRTNVGNSVV IDGPSQNITL
201  THCKGDSCSG NNFLDEEVQL KSEFEKLSDA DKISNYKKDG KNDGKNDKFV
251  GLVADSVQMK GINQYIIFYK PKPTSFARFR RSARSRRSLP AEMPLIPVNQ
301  ADTLIVDGEA VSLTGHSGNI FAPEGNYRYL TYGAEKLPGG SYALRVQGEP
351  SKGEMLAGTA VYNGEVLHFH TENGRPSPSR GRFAAKVDFG SKSVDGIIDS
401  GDGLHMGTQK FKAAIDGNGF KGTWTENGGG DVSGKFYGPA GEEVAGKYSY
451  RPTDAEKGGF GVFAGKKEQD GSGGGGATND DDVKKAATVA IAAAYNNGQE
501  INGFKAGETI YDIDEDGTIT KKDATAADVE ADDFKGLGLK KVVTNLTKTV
551  NENKQNVDAK VKAAESEIEK LTTKLADTDA ALADTDAALD ATTNALNKLG
601  ENITTFAEET KTNIVKIDEK LEAVADTVDK HAEAFNDIAD SLDETNTKAD
651  EAVKTANEAK QTAEETKQNV DAKVKAAETA AGKAEAAAGT ANTAADKAEA
701  VAAKVTDIKA DIATNKDNIA KKANSADVYT REESDSKFVR IDGLNATTEK
751  LDTRLASAEK SIADHDTRLN GLDKTVSDLR KETRQGLAEQ AALSGLFQPY
801  NVGRFNVTAA VGGYKSESAV AIGTGFRFTE NFAAKAGVAV GTSSGSSAAY
851  HVGVNYEW*
```

*Example 2 -hybrids of 287*

Expression of 287 as full-length with a C-terminal His-tag, or without its leader peptide but with a C-terminal His-tag, gives fairly low expression levels. Better expression is achieved using a N-terminal GST-fusion.

When fused to protein 961 [$NH_2$-ΔG287-961-COOH - sequence shown above], the resulting protein is insoluble and must be denatured and renatured for purification. Following denaturation, around 50% of the protein was found to remain insoluble. The soluble and insoluble proteins were compared, and much better bactericidal titres were obtained with the soluble protein (FCA as adjuvant):

| | 2996 | BZ232 | MC58 | NGH38 | F6124 | BZ133 |
|---|---|---|---|---|---|---|
| Soluble | 65536 | 128 | 4096 | >2048 | >2048 | 4096 |
| Insoluble | 8192. | <4 | <4 | 16 | n.d. | n.d. |

Titres with the insoluble form were, however, improved by using alum adjuvant instead:

| | | | | | | |
|---|---|---|---|---|---|---|
| Insoluble | 32768 | 128 | 4096 | >2048 | >2048 | 2048 |

961c was also used in hybrid proteins (see above). As 961 and its domain variants direct efficient expression, they are ideally suited as the N-terminal portion of a hybrid protein.

## EXPERIMENTAL DETAILS

### *Cloning strategy and oligonucleotide design*

Genes coding for antigens of interest were amplified by PCR, using oligonucleotides designed on the basis of the genomic sequence of *N. meningitidis* B MC58. Genomic DNA from strain 2996 was always used as a template in PCR reactions, unless otherwise specified, and the amplified fragments were cloned in the expression vector pET21b+ (Novagen) to express the protein as C-terminal His-tagged product, or in pET-24b+(Novagen) to express the protein in 'untagged' form (*e.g.* ΔG 287K).

Where a protein was expressed without a fusion partner and with its own leader peptide (if present), amplification of the open reading frame (ATG to STOP codons) was performed.

Where a protein was expressed in 'untagged' form, the leader peptide was omitted by designing the 5'-end amplification primer downstream from the predicted leader sequence.

The melting temperature of the primers used in PCR depended on the number and type of hybridising nucleotides in the whole primer, and was determined using the formulae:

$$T_{mi} = 4\,(G+C) + 2\,(A+T) \qquad \text{(tail excluded)}$$

$$T_{m2} = 64.9 + 0.41\,(\%\,GC) - 600/N \qquad \text{(whole primer)}$$

The melting temperatures of the selected oligonucleotides were usually 65-70°C for the whole oligo and 50-60°C for the hybridising region alone.

Oligonucleotides were synthesised using, a Perkin Elmer DNA/RNA Synthesizer, eluted from the columns in 2.0ml $NH_4OH$, and deprotected by 5 hours incubation at 56°C. The oligos were precipitated by addition of 0.3M Na-Acetate and 2 volumes ethanol. The samples were centrifuged and the pellets resuspended in water.

| | | Sequences | Restriction site |
|---|---|---|---|
| **fu (961)-741(MC58)-His** | Fwd | CGCGGATCC -GGAGGGGGTGGTGTCG | BamHI |
| | Rev | CCCGCTCGAG-TTGCTTGGCGGCAAGGG | XhoI |
| **fu (961.)-983-His** | Fwd | CGCGGATCC - GGCGGAGGCGGCACTT | BamHI |
| | Rev | CCCGCTCGAG-GAACCGGTAGCCTACG | XhoI |
| **fu (961)-Orf16.1-His** | Fwd | CGCGGATCCGGTGGTGGTGGT-TCAGATTTGGCAAACGATTC | BamHI |
| | Rev | CCCGCTCGAG-CGTATCATATTTCACGTGC | XhoI |
| **fu (961 c-L)-741(MC58)** | Fwd | CGCGGATCC -GGAGGGGGTGGTGTCG | BamHI |
| | Rev | CCCGCTCGAG-TTATTGCTTGGCGGCAAG | XhoI |
| **fu (961c-L)-983** | Fwd | CGCGGATCC - GGCGGAGGCGGCACTT | BamHI |
| | Rev | CCCGCTCGAG-TCAGAACCGGTAGCCTAC | XhoI |
| **fu (961c-L)-Orf46.1** | Fwd | CGCGGATCCGGTGGTGGTGGT-TCAGATTTGGCAAACGATTC | BamHI |
| | Rev | CCCGCTCGAG-TTACGTATCATATTTCACGTGC | XhoI |
| **fu-(ΔG287)-919-His** | Fwd | CGCGGATCCGGTGGTGGTGGT-CAAAGCAAGAGCATCCAAACC. | BamHI |
| | Rev | CCCAAGCTT-TTCGGGCGGTATTCGGGCTTC | HindIII |
| **fu-(ΔG287)-**953–Hισ | Fwd | CGCGGATCCGGTGGTGGTGGT-GCCACCTACAAAGTGGAC | BamHI |
| | Rev | GCCCAAGCTT-TTGTTTGGCTGCCTCGAT | HindIII |
| **fu-(ΔG287)-**961–Hισ | Fwd | CGCGGATCCGGTGGTGGTGGT-ACAAGCGACGACG | BamHI |
| | Rev | GCCCAAGCTT-CCACTCGTAATTGACGCC | HindIII |
| **fu-(ΔG287)-**Oρφ46.1–Hισ | Fwd | CGCGGATCCGGTGGTGGTGGT-TCAGATTTGGCAAACGATTC | BamHI |
| | Rev | CCCAAGCTT-CGTATCATATTTCACGTGC | HindIII |
| **fu-(ΔG287-919)-**Oρφ46.1–Hισ | Fwd | CCCAAGCTTGGTGGTGGTGGTGGT-TCAGATTTGGCAAACGATTC | HindIII |
| | Rev | CCCGCTCGAG-CGTATCATATTTCACGTGC | XhoI |
| **fu-(ΔP787-**Oρφ46.1)–919–Hισ | Fwd | CCCAAGCITGGTGGTGGTGGTGGT-CAAAGCAAGAGCATCCAAACC | HindIII |
| | | Rev CCCGCTCGAG-CGGGCGGTATTCGGGCTT | XhoI |

(continued)

| | | Sequences | Restriction site |
|---|---|---|---|
| **fu ΔG287**(394.98)**-** | Fwd | CGCGGATCCGCTAGC-CCCGATGTTAAATCGGC | NheI |
| | Rev | CGGGGATCC-ATCCTGCTCTTTTTGCCGG | BamHI |
| **fu Orf1-(Orf46.1)-His** | Fwd | CGCGGATCCGCCTAGC-GGACACACTTATTTCGGCATC | NheI |
| | Rev | CGCGGATCC-CCAGCGGTAGCGTAATTTGAT | |
| **fu (Orf1)-Orf46.1-His** | Fwd | CGCGGATCCGGTGGTGGTGGT-TCAGATTTGGCAAACGATTC | BamHI |
| | Rev | CCCAAGCTT-CGTATCATATTTCACGTGC | HindIII |
| **fu (919)-Orf46.1-His** | Fwd1 | GCGGCGTCGACGGTGGCGGAGGCACTGGATCCTCAG | SalI |
| | Fwd2 | GGAGGCACTGGATCCTCAGATTTGGAAACGATTC | |
| | **Rev** | CCCGCTCGAG-CGTATCATATTTCACGTGC | XhoI |
| **Fu (orf46)-287-His** | Fwd | CGGGATCCGGGGCGGCGGTGGCG | BamHI |
| | Rev | CCCAAGCTTATCCTGCTCTTTTTTGCCGGC | HindIII |
| **Fu(orf46).919-His** | Fwd | **CGCGGATCCGGTGGTGGTGGTCAAAGCAAGAGCATCCA AACC** | BamHI |
| | Rev | CCCAAGCGGGCGGTATTCGGGCTTC. | HindIII |
| **Fu (orf46-919)-287-His** | Fwd | CCCCAAGCTTGGGGGCGGCGGTGGCG | HindIII |
| | Rev | CCCGCTCGAGATCCTGCTCTTTTTTGCCGGC | XhoI |
| **Fu (ort46-287)-919-His** | Fwd | **CCCAAGCTTGGTGGTGGTGGTGGTCAAAGCAAGAGCAT CCAAACC** | HindIII |
| | Rev | CCCCGCTCGAGCGGGCGGTATTCGGGCTT | XhoI |
| **(ΔG741)-**961ε–Hισ | Fwd1<br>Fwd2 | GGAGGCACTGGATCCGCAGCCACAAACGACGACGA<br>GCGGCCTCGAG-GGTGGCGGAGGCACTGGATCCGCAG | XhoI |
| | Rev | CCCGCTCGAG-ACCCAGCTTGTAAGGTTG | XhoI |
| **(ΔG741)-**961–Hισ | Fwd1<br>Fwd2 | GGAGGCACTGGATCCGCAGCCACAAACGACGACGA<br>GCGGCCTCGAG-GGTGGCGGAGGCACTGGATCCGCAG | XhoI |
| | Rev | CCCGCTCGAG-CCACTCGTAATTGACGCC | XhoI |

(continued)

| | | Sequences | Restriction site |
|---|---|---|---|
| **(ΔG741)-**983.Hισ | Fwd | **GCGGCCTCGAG-GGATCCGGCGGAGGCGGCACTTCTGCG**. | XhoI |
| | Rev | CCCGCTCGAG-GAACCGGTAGCCTACG | XhoI |
| **(ΔG741 )-**ορφ46.1–Hισ | Fwd1<br>Fwd2 | GGAGGCACTGGATCCTCAGATTTGGCAAACGATTC<br>GCGGCGTCGACGGTGGCGGAGGCACTGGATCCTCAGA | SalI |
| | Rev | CCCGCTCGAG-CGTATCATATTTCACGTGC | XhoI |
| **(ΔG983)-**741(MX58) **-**Hισ | Fwd | GCGGCCTCGAG-GGATCCGGAGGGGGTGGTGTCGCC | XhoI |
| | Rev | CCCGCTCGAG-TTGCTTGGCGGCAAG | XhoI |
| **(ΔG983)-**961χ–Hισ | Fwd1<br>Fwd2 | GGAGGCACTGGATCCGCAGCCACAAACGACGACGA<br>GCGGCCTCGAG-GGTGGCGGAGGCACTGGATCCGCAG | XhoI |
| | Rev | CCCGCTCGAG-ACCCAGCTTGTAAGGTTG | XhoI |
| **(ΔG983)-**961–Hισ | Fwd1<br>Fwd2 | GGAGGCACTGGATCCGCAGCCACAAACGACGACGA<br>GCGGCCTCGAG-GGTGGCGGAGGCACTGGATCCGCAG. | XhoI |
| | Rev | CCCGCTCGAG-CCACTCGTAATTGACGCC | XhoI |
| **(ΔG983)-** Ορφ46.1**-** Fwd1 **His** | <br>Fwd2 | GGAGGCACTGGATCCTCAGATTTGGCAAACGATTC<br>GCGGCGTCGACGGTGGCGGAGGCACTGGATCCTCAGA | SalI |
| | Rev | CCCGCTCGAG-CGTATCATATTTCACGTGC | XhoI |

*This primer was used as a Reverse primer for all the C terminal fusions of 287 to the His-tag.
Forward primers used in combination with the 287-His Reverse primer. NB-All PCR reactions use-strain 2996 unless otherwise specified (*e.g.* strain MC58)

In all constructs starting with an ATG not followed by a unique *Nhe*I site, the ATG codon is part of the *Nde*I site used for cloning. The constructs made using *Nhe*I as a cloning site at then. 5' end (*e.g.* all those containing 287 at the N-terminus) have two additional codons (GCT AGC) fused to the coding sequence of the antigen.

### *Preparation of chromosomal DNA templates*

*N.meningitidis* strains 2996, MC58, 394.98, 1000 and BZ2.32 (and others) were grown to exponential phase in 100ml of GC medium, harvested by centrifugation, and resuspended in 5ml buffer (20% w/v sucrose, 50mM Tris-HCl, 50mM EDTA, pH8). After 10 minutes incubation on ice, the bacteria were lysed by adding 10ml of lysis solution (50mM NaCl, 1% Na-Sarkosyl, 50μg/ml Proteinase K), and the suspension incubated at 37°C for 2 hours. Two phenol extractions (equilibrated to pH 8) and one $CHCl_3$/isoamylalcohol (24: 1) extraction were performed. DNA was precipitated by addition of 0.3M sodium acetate and 2 volumes of ethanol, and collected by centrifugation. The pellet was washed once with 70%(v/v) ethanol and redissolved in 4.0ml TE buffer (10mM Tris-HCl, 1mM EDTA, pH 8.0). The DNA concentration was measured by reading $OD_{260}$.

### *PCR Amplification*

The standard PCR protocol was as follows: 200ng of genomic DNA from 2996, MC581000, or BZ232 strains or 10ng of plasmid DNA preparation of recombinant clones were used as template in the presence of 40μM of each oligonucletide primer, 400-800 μM dNTPs solution, 1x PCR buffer (including 1.5mM $MgCl_2$), 2.5 units *TaqI* DNA polymerase (using Perkin-Elmer AmpliTaQ, Boerhingher Mannheim Expand™ Long Template).

After a preliminary 3 minute incubation of the whole mix at 95°C, each sample underwent a two-step amplification: the first 5 cycles were performed using the hybridisation temperature that excluded the restriction enzymes tail of the primer ($T_{m1}$). This was followed by 30 cycles according to the hybridisation temperature calculated for the whole length oligos ($T_{m2}$). Elongation times, performed at 68°C for 72°C, varied according to the length of the Orf to be amplified. In the case of Orf1 the elongation time, starting from 3 minutes, was increased by 15 seconds each cycle. The cycles were completed with a 10 minute extension step at 72°C.

The amplified DNA was either loaded directly on a 1% agarose gel. The DNA fragment corresponding to the band of correct size was purified from the gel using the Qiagen Gel Extraction Kit, following the manufacturer's protocol.

### *Digestion of PCR fragments and of the cloning vectors*

The purified DNA corresponding to the amplified fragment was digested with the appropriate restriction enzymes for cloning into pET-21b+, pET22b+ or pET-24b+. Digested fragments were purified using the QIAquick PCR purification kit (following the manufacturer's instructions) and eluted with either $H_2O$ or 10mM Tris, pH 8.5. Plasmid vectors were digested with the appropriate restriction enzymes, loaded onto a 1.0% agarose gel and the band corresponding to the digested vector purified using the Qiagen QIAquick Gel Extraction Kit.

### *Cloning*

The fragments corresponding to each gene, previously digested and purified, were ligated into pET21b+, pET22b+ or pET-24b+. A molar ratio of 3:1 fragment/vector was used with T4 DNA ligase in the ligation buffer supplied by the manufacturer.

Recombinant plasmid was transformed into competent *E.coli* DH5 or HB101 by incubating the ligase reaction solution and bacteria for 40 minutes on ice, then at 37°C for 3 minutes. This was followed by the addition of 800μl LB broth and incubation at 37°C for 20 minutes. The cells were centrifuged at maximum speed in an Eppendorf microfuge, resuspended in approximately 200μl of the supernatant and plated onto LB ampicillin (100mg/ml) agar.

Screening for recombinant clones was performed by growing randomly selected colonies overnight at 37°C in 4.0ml of LB broth + 100μg/ml ampicillin. Cells were pelleted and plasmid DNA extracted using the Qiagen QIAprep Spin Miniprep Kit, following the manufacturer's instructions. Approximately 1μg of each individual miniprep was digested with the appropriate restriction enzymes and the digest loaded onto a 1-1.5% agarose gel (depending on the expected insert size), in parallel with the molecular weight marker (1kb DNA Ladder, GIBCO). Positive clones were selected on the basis of the size of insert.

### *Expression*

After cloning each gene into the expression vector, recombinant plasmids, were transformed into *E.coli* strains suitable for expression of the recombinant protein. 1μl of each construct was used to transform *E.coli* BL21-DE3 as described above. Single recombinant-colonies were inoculated into 2ml LB+Amp (100μg/ml), incubated at 37°C overnight, then diluted 1:30 in 20ml of LB+Amp (100μg/ml) in 100ml flasks, to give an $OD_{600}$ between 0.1 and 0.2. The flasks were incubated at 30°C or at 37°C in a gyratory water bath shaker until $OD_{600}$ indicated exponential growth suitable for induction of expression (0.4-0.8 OD). Protein expression was induced by addition of 1.0mM IPTG. After 3 hours incubation at 30°C or 37°C the $OD_{600}$ was measured and expression examined. 1.0ml of each sample was centrifuged in a microfuge, the pellet resuspended in PBS and analysed by SDS-PAGE and Coomassie Blue straining.

***Purification of His-tagged proteins***

Various forms of 287 were cloned from strains 2996 and MC58. They were constructed with a C-terminus His-tagged fusion and included a mature form (aa 18-427), constructs with deletion (Δ1, Δ2, Δ3 and Δ4) and clones composed of either B or C domains. For each clone purified as a His-fusion, a single colony was streaked and grown overnight at 37°C on a LB/Amp (100 μg/ml) agar plate. An isolated colony from this plate was inoculated into 20ml of LB/Amp (100 μg/ml) liquid medium and grown overnight at 37°C with shaking. The overnight culture was diluted 1:30 into 1.0 L LB/Amp (100 μg/ml) liquid medium and allowed to grow at the optimal temperature (30 or 37°C) until the $OD_{550}$ reached 0.6-0.8. Expression of recombinant protein was induced by addition of IPTG (final concentration 1.0mM) and the culture incubated for a further 3 hours. Bacteria were harvested by centrifugation at 8000g for 15 min at 4°C. The bacterial pellet was resuspended in 7.5 ml of either (i) cold buffer A (300 mM NaCl, 50 mM phosphate buffer, 10 mM imidazole, pH 8.0) for soluble proteins or (ii) buffer B (10mM Tris-HCl, 100 mM phosphate buffer, pH 8.8 and, optionally, 8M urea) for insoluble proteins. Proteins purified in a soluble form included 287-His, Δ1, Δ2, Δ3 and Δ4287-His, Δ4287MC58-His, 287c-His and 287cMC58-His. Protein 287bMC58-His was insoluble and purified accordingly. Cells were disrupted by sonication on ice four times for 30 sec at 40W using a Branson sonifier 450 and centrifuged at 13000xg for 30 min at 4°C. For insoluble proteins, pellets were resuspended in 2.0 ml buffer C (6 M guanidine hydrochloride; 100 mM phosphate buffer, 10 mM Tris- HCl, pH 7.5 and treated with 10 passes of a Dounce homogenizer. The homogenate was centrifuged at 13000g for 30 min and the supernatant retrained. Supernatants for both soluble and insoluble preparations were mixed with 150μl $Ni^{2+}$-resin (previously equilibrated with either buffer A or buffer B, as appropriate) and incubated at room temperature with gentle agitation for 30 min. The resin was Chelating Sepharose Fast Flow (Pharmacia), prepared according to the manufacturer's protocol. The batch-wise preparation was centrifuged at 700g for 5 min at 4°C and the supernatant discarded. The resin was washed twice (batch-wise) with 10ml buffer A or B for 10 min, resuspended in 1.0 ml buffer A or B and loaded onto a disposable column. The resin continued to be washed with either (i) buffer A at 4°C or (ii) buffer B at room temperature, until the $OD_{280}$ of the flow-through reached 0.02-0.01. The resin was further washed with either (i) cold buffer C (300mM NaCl, 50mM phosphate buffer, 20mM imidazole, pH 8.0) or (ii) buffer D (10mM Tris-HCl, 100mM phosphate buffer, pH 6.3 and, optionally, 8M urea) until $OD_{280}$ of the flow-through reached 0.02-0.01. The His-fusion protein was eluted by addition of 700μl of either (i) cold elution buffer A (300 mM NaCl, 50mM phosphate buffer, 250 mM imidazole, pH 8.0) or (ii) elution buffer B (10 mM Tris-HCl, 100 mM phosphate buffer, pH 4.5 and, optionally, 8M urea) and fractions collected until the $OD_{280}$ indicated all the recombinant protein was obtained. 20μl aliquots of each elution fraction were analysed by SDS-PAGE. Protein concentrations were estimated using the Bradford assay.

***Renaturation of denatured His-fusion proteins.***

Denaturation was required to solubilize 287bMC8, so a renaturation step was employed prior to immunisation. Glycerol was added to the denatured fractions obtained above to give a final concentration of 10% v/v. The proteins were diluted to 200 μg/ml using dialysis buffer I (10% v/v glycerol, 0.5M arginine, 50 mM phosphate buffer, 5.0 mM reduced glutathione, 0.5 mM oxidised glutathione, 2.0M urea, pH 8.8) and dialysed against the same buffer for 12-14 hours at 4°C. Further dialysis was performed with buffer II (10% v/v glycerol, 0.5M arginine, 50mM phosphate buffer, 5.0mM reduced glutathione, 0.5mM oxidised glutathione, pH 8.8) for 12-14 hours at 4°C. Protein concentration was estimated using the formula:

$$Protein\ (mg/ml) = (1.55 \times OD_{280}) - (0.76 \times OD_{260})$$

***Immunization***

Balb/C mice were immunized with antigens on days 0, 21 and 35 and sera analyzed at day 49.

### *Sera analysis - ELISA*

The acapsulated MenB M7 and the capsulated strains were plated on chocolate agar plates and incubated overnight at 37°C with 5% $CO_2$. Bacterial colonies were collected from the agar plates using a sterile dracon swab and inoculated into Mueller-Hinton Broth (Difco) containing 0.25% glucose. Bacterial growth was monitored every 30 minutes by following $OD_{620}$. The bacteria were let to grow until the OD reached the value of 0.4-0.5. The culture was centrifuged for 10 minutes at 4000rpm. The supernatant was discarded and bacteria were washed twice with PBS, resuspended in PBS containing 0.025% formaldehyde, and incubated for 1 hour at 37°C and then overnight at 4°C with stirring. 100μl bacterial cells were added to each well of a 96 well Greiner plate and incubated overnight at 4°C. The wells were then washed three times with PBT washing buffer (0.1% Tween-20 in PBS). 200μl of saturation buffer (2.7% polyvinylpyrrolidone 10 in water) was added to each well and the plates incubated for 2 hours at 37°C. Wells were washed three times with PBT. 200μl of diluted sera (Dilution buffer: 1% BSA, 0.1% Tween-20, 0.1% $NaN_3$ in PBS) were added to each well and the plates incubated for 2 hours at 37°C. Wells were washed three times with PBT. 100μl of HRP-conjugated rabbit anti-mouse (Dako) serum diluted 1:2000 in dilution buffer were added to each well and the plates were incubated for 90 minutes at 37°C. Wells were washed three times with PBT buffer. 100μl of substrate buffer for HRP (25ml of citrate buffer pH5, 10mg of O-phenildiamine and 10μl of $H_2O_2$) were added to each well and the plates were left at room temperature for 20 minutes. 100μl 12.5% $H_2SO_4$ was added to each well and $OD_{490}$ was followed. The ELISA titers were calculated abitrarely as the dilution of sera which gave an $OD_{490}$ value of 0.4 above the level of preimmune sera. The ELISA was considered positive when the dilution of sera with $OD_{490}$ of 0.4 was higher than 1:400.

### *Sera analysis - FACS Scan bacteria binding assay*

The acapsulated MenB M7 strain was plated on chocolate agar plates and incubated overnight at 37°C. with 5% $CO_2$. Bacterial colonies were collected from the agar plates using a sterile dracon swab and inoculated into 4 tubes containing 8ml each Mueller-Hinton Broth (Difco) containing 0.25% glucose. Bacterial growth was monitored every 30 minutes by following $OD_{620}$. The bacteria were let to grow until the OD reached the value of 0.35-0.5. The culture was centrifuged for 10 minutes at 4000rpm. The supernatant was discarded and the pellet was resuspended in blocking buffer (1% BSA in PBS, 0.4% $NaN_3$) and centrifuged for 5 minutes at 4000rpm. Cells were resuspended in blocking buffer to reach $OD_{620}$ of 0.05. 100μl bacterial cells were added to each well of a Costar 96 well plate. 100μl of diluted (1:100, 1:200, 1:400) sera (in blocking buffer) were added to each well and plates incubated for 2 hours at 4°C. Cells were centrifuged for 5 minutes at 4000rpm, the supernatant aspirated and cells washed by addition of 200μl/well of blocking buffet in each well. 100μl of R-Phicoerytrin conjugated $F(ab)_2$ goat anti-mouse, diluted 1:100, was added to each well and plates incubated for 1 hour at 4°C. Cells were spun down by centrifugation at 4000rpm for 5 minutes and washed by addition of 200μl/well of blocking buffer. The supernatant was aspirated and cells resuspended in 200μl/ well of PBS, 0.25% formaldehyde. Samples were transferred to FACScan tubes and read. The condition for FACScan (Laser Power 15mW) setting were: FL2 on; FSC-H threshold:92; FSC PMT Voltage: E 01; SSC PMT: 474; Amp. Gains 6.1; FL-2 PMT: 586; compensation values: 0.

### *Sera analysis - bactericidal assay*

*N. meningitidis* strain 2996 was grown overnight at 37°C on chocolate agar plates (starting from a frozen stock) with 5% $CO_2$. Colonies were collected and used to inoculate 7ml Mueller-Hinton broth, containing 0.25% glucose to reach an $OD_{620}$ of 0.05-0.08. The culture was incubated for approximately 1.5 hours at 37 degrees with shacking until the $OD_{620}$ reached the value of 0.23-0.24. Bacteria were diluted in 50mM Phosphate buffer pH 7.2 containing 10mM $MgCl_2$, 10mM $CaCl_2$ and 0.5% (w/v) BSA (assay buffer) at the working dilution of $10^5$ CFU/ml. The total volume of the final reaction mixture was 50 μl with 25 μl of serial two fold dilution of test serum, 12.5 μl of bacteria at the working dilution, 12.5 μl of baby rabbit complement (final concentration 25%).

Controls included bacteria incubated with complement serum, immune sera incubated with bacteria and with complement inactivated by heating at 56°C for 30'. Immediately after the addition of the baby rabbit complement, 10μl of the controls were plated on Mueller-Hinton agar plates using the tilt method (time 0). The 96-wells plate was incubated for 1 hour at 37°C with rotation. 7μl of each sample were plated on Mueller-Hinton agar plates as spots, whereas 10μl of the controls were plated on Mueller-Hinton agar plates using the tilt method (time 1). Agar plates were incubated for 18 hours at 37 degrees and the colonies corresponding to time 0 and time 1 were counted.

### *Sera analysis - western blots*

Purified proteins (500ng/lane), outer membrane vesicles (5μg) and total cell extracts (25μg) derived from MenB strain 2996 were loaded onto a 12% SDS-polyacrylamide gel and transferred to a nitrocellulose membrane. The transfer was performed for 2 hours at 150mA at 4˚C, using transfer buffer (0.3% Tris base, 1.44% glycine, 20% (v/v) ethanol). The membrane was saturated by overnight incubation at 4˚C in saturation buffer (10% skimmed milk, 0.1% Triton X100 in PBS). The membrane was washed twice with washing buffer (3% skimmed milk, 0.1% Triton X100 in PBS) and incubated for 2 hours at 37˚C with mice sera diluted 1:200 in washing buffer. The membrane was washed twice and incubated for 90 minutes with a 1:2000 dilution of horseradish peroxidase labelled anti-mouse Ig. The membrane was washed twice with 0.1% Triton X100 in PBS and developed with the Opti-4CN Substrate Kit (Bio-Rad). The reaction was stopped by adding water.

The OMVs were prepared as follows: *N. meningitidis* strain 2996 was grown overnight at 37 degrees with 5% $CO_2$ on 5 GC plates, harvested with a loop and resuspended in 10 ml of 20mM Tris-HCl pH 7.5, 2 mM EDTA. Heat inactivation was performed at 56˚C for 45 minutes and the bacteria disrupted by sonication for 5 minutes on ice (50% duty cycle, 50% output, Branson sonifier 3 mm microtip). Unbroken cells were removed by centrifugation at 5000g for 10 minutes, the supernatant containing the total cell envelope fraction recovered and further centrifuged overnight at 50000g at the temperature of 4˚C. The pellet containing the membranes was resuspended in 2% sarkosyl, 20mM Tris-HCl pH 7.5, 2 mM EDTA and incubated at room temperature for 20 minutes to solubilise the inner membranes. The suspension was centrifuged at 10000g for 10 minutes to remove aggregates, the supernatant was further centrifuges at 50000g for 3 hours. The pellet, containing the outer membranes was washed in PBS and resuspended in the same buffer. Protein concentration was measured by the D.C. Bio-Rad Protein assay (Modified Lowry method), using BSA as a standard.

Total cell extracts were prepared as follows: *N. meningitidis* strain 2996 was grown overnight on a GC plate, harvested with a loop and resuspended in 1ml of 20mM Tris-HCl. Heat inactivation was performed at 56˚C for 30 minutes.

## Claims

**1.** A hybrid protein of formula $NH_2$-A-B-COOH, wherein A comprises the Neisserial protein ΔG287 and B comprises the Neisserial protein 961, and wherein the amino acid sequence of the hybrid protein is as disclosed in SEQ ID NO: 8 or is a sequence having greater than 70% sequence identity thereto.

**2.** The protein of claim 1, wherein ΔG287 is from strain 2996 or 394/98.

**3.** The protein of claim 1, wherein 961 is from strain 2996 or 394/98.

**4.** The protein of claim 1, wherein A and B are from the same strain.

**5.** The hybrid protein of claim 1, comprising the amino acid sequence recited in SEQ ID NO: 8.

## Patentansprüche

**1.** Ein Hybridprotein mit der Formel $NH_2$-A-B-COOH, wobei A das Neisseriale Protein ΔG287 und B das Neisseriale Protein 961 umfasst, und wobei die Aminosäuresequenz des Hybridproteins wie in SEQ ID Nr. 8 geoffenbart oder eine Sequenz, welche eine mehr als 70% Sequenzidentität dazu hat, ist.

**2.** Das Protein gemäß Anspruch 1, wobei ΔG287 vom Stamm 2996 oder 394/98 ist.

3. Das Protein gemäß Anspruch 1, wobei 961 vom Stamm 2996 oder 394/98 ist.

4. Das Protein gemäß Anspruch 1, wobei A und B vom selben Stamm sind.

5. Das Hybridprotein gemäß Anspruch 1, umfassend die in SEQ ID Nr. 8 angegebene Aminosäuresequenz.

**Revendications**

1. Protéine hybride de formule $NH_2$-A-B-COOH, dans laquelle A comprend la protéine ΔG287 de Neisseria et B comprend la protéine 961 de Neisseria, et dans laquelle la séquence d'acides aminés de la protéine hybride est telle que décrite par SÉQ ID NO : 8 ou est une séquence présentant une identité de séquence supérieure à 70 % avec celle-ci.

2. Protéine selon la revendication 1, dans laquelle ΔG287 provient de la souche 2996 ou 394/98.

3. Protéine selon la revendication 1, dans laquelle 961 provient de la souche 2996 ou 394/98.

4. Protéine selon la revendication 1, dans laquelle A et B proviennent de la même souche.

5. Protéine hybride selon la revendication 1, comprenant la séquence d'acides aminés représentée par SÉQ ID NO : 8.

FIGURE 1 — ΔG287—961

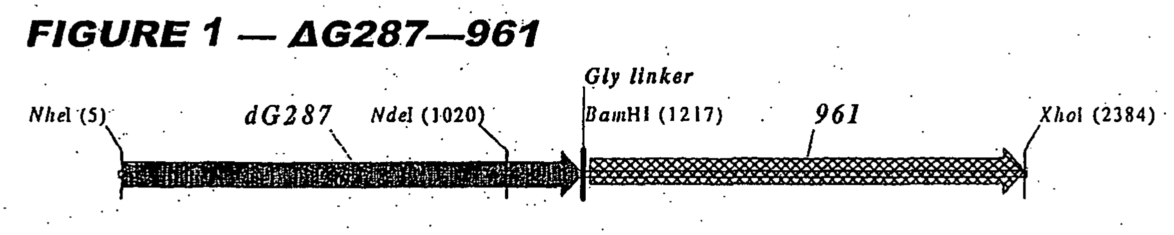


FIGURE 2 — ΔG287NZ—961

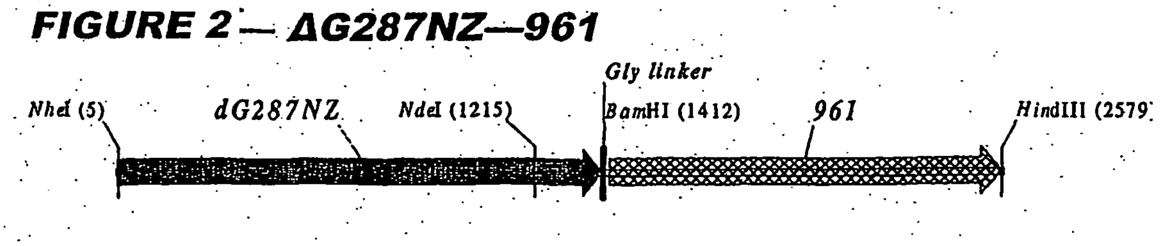

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9924578 A
- WO 9936544 A
- WO 9957280 A
- WO 0022430 A
- WO 0066741 A
- EP 01914098 A
- WO IB0100420 W
- GB 0004695 A
- GB 0027675 A

SEQUENCE LISTING

<110> Novartis Vaccines and Diagnostics SRL

<120> Hybrid Expression of Neisserial Proteins

<130> P048826EP

<150> EP-01914098.7
<151> 2001-02-28

<150> PCT/IB01/00420
<151> 2001-02-28

<150> GB 0004695.3
<151> 2000-02-28

<150> GB 0027675.8
<151> 2000-11-13

<160> 121

<170> SeqWin99, version 1.02

<210> 1
<211> 608
<212> PRT
<213> Neisseria meningitidis

<400> 1

```
Leu Gly Ile Ser Arg Lys Ile Ser Leu Ile Leu Ser Ile Leu Ala Val
1               5                   10                  15

Cys Leu Pro Met His Ala His Ala Ser Asp Leu Ala Asn Asp Ser Phe
            20                  25                  30

Ile Arg Gln Val Leu Asp Arg Gln His Phe Glu Pro Asp Gly Lys Tyr
        35                  40                  45

His Leu Phe Gly Ser Arg Gly Glu Leu Ala Glu Arg Ser Gly His Ile
    50                  55                  60

Gly Leu Gly Lys Ile Gln Ser His Gln Leu Gly Asn Leu Met Ile Gln
65                  70                  75                  80

Gln Ala Ala Ile Lys Gly Asn Ile Gly Tyr Ile Val Arg Phe Ser Asp
                85                  90                  95

His Gly His Glu Val His Ser Pro Phe Asp Asn His Ala Ser His Ser
            100                 105                 110

Asp Ser Asp Glu Ala Gly Ser Pro Val Asp Gly Phe Ser Leu Tyr Arg
        115                 120                 125

Ile His Trp Asp Gly Tyr Glu His His Pro Ala Asp Gly Tyr Asp Gly
    130                 135                 140

Pro Gln Gly Gly Gly Tyr Pro Ala Pro Lys Gly Ala Arg Asp Ile Tyr
145                 150                 155                 160

Ser Tyr Asp Ile Lys Gly Val Ala Gln Asn Ile Arg Leu Asn Leu Thr
```

```
                165                 170                 175

Asp Asn Arg Ser Thr Gly Gln Arg Leu Ala Asp Arg Phe His Asn Ala
            180                 185                 190

Gly Ser Met Leu Thr Gln Gly Val Gly Asp Gly Phe Lys Arg Ala Thr
        195                 200                 205

Arg Tyr Ser Pro Glu Leu Asp Arg Ser Gly Asn Ala Ala Glu Ala Phe
    210                 215                 220

Asn Gly Thr Ala Asp Ile Val Lys Asn Ile Ile Gly Ala Ala Gly Glu
225                 230                 235                 240

Ile Val Gly Ala Gly Asp Ala Val Gln Gly Ile Ser Glu Gly Ser Asn
                245                 250                 255

Ile Ala Val Met His Gly Leu Gly Leu Leu Ser Thr Glu Asn Lys Met
            260                 265                 270

Ala Arg Ile Asn Asp Leu Ala Asp Met Ala Gln Leu Lys Asp Tyr Ala
        275                 280                 285

Ala Ala Ala Ile Arg Asp Trp Ala Val Gln Asn Pro Asn Ala Ala Gln
    290                 295                 300

Gly Ile Glu Ala Val Ser Asn Ile Phe Met Ala Ala Ile Pro Ile Lys
305                 310                 315                 320

Gly Ile Gly Ala Val Arg Gly Lys Tyr Gly Leu Gly Gly Ile Thr Ala
                325                 330                 335

His Pro Ile Lys Arg Ser Gln Met Gly Ala Ile Ala Leu Pro Lys Gly
            340                 345                 350

Lys Ser Ala Val Ser Asp Asn Phe Ala Asp Ala Ala Tyr Ala Lys Tyr
        355                 360                 365

Pro Ser Pro Tyr His Ser Arg Asn Ile Arg Ser Asn Leu Glu Gln Arg
    370                 375                 380

Tyr Gly Lys Glu Asn Ile Thr Ser Ser Thr Val Pro Pro Ser Asn Gly
385                 390                 395                 400

Lys Asn Val Lys Leu Ala Asp Gln Arg His Pro Lys Thr Gly Val Pro
                405                 410                 415

Phe Asp Gly Lys Gly Phe Pro Asn Phe Glu Lys His Val Lys Tyr Asp
            420                 425                 430

Thr Lys Leu Asp Ile Gln Glu Leu Ser Gly Gly Gly Ile Pro Lys Ala
        435                 440                 445

Lys Pro Val Ser Asp Ala Lys Pro Arg Trp Glu Val Asp Arg Lys Leu
    450                 455                 460

Asn Lys Leu Thr Thr Arg Glu Gln Val Glu Lys Asn Val Gln Glu Ile
465                 470                 475                 480

Arg Asn Gly Asn Lys Asn Ser Asn Phe Ser Gln His Ala Gln Leu Glu
                485                 490                 495
```

```
Arg Glu Ile Asn Lys Leu Lys Ser Ala Asp Glu Ile Asn Phe Ala Asp
            500                 505                 510

Gly Met Gly Lys Phe Thr Asp Ser Met Asn Asp Lys Ala Phe Ser Arg
        515                 520                 525

Leu Val Lys Ser Val Lys Glu Asn Gly Phe Thr Asn Pro Val Val Glu
    530                 535                 540

Tyr Val Glu Ile Asn Gly Lys Ala Tyr Ile Val Arg Gly Asn Asn Arg
545                 550                 555                 560

Val Phe Ala Ala Glu Tyr Leu Gly Arg Ile His Glu Leu Lys Phe Lys
                565                 570                 575

Lys Val Asp Phe Pro Val Pro Asn Thr Ser Trp Lys Asn Pro Thr Asp
            580                 585                 590

Val Leu Asn Glu Ser Gly Asn Val Lys Arg Pro Arg Tyr Arg Ser Lys
        595                 600                 605
```

<210> 2
<211> 464
<212> PRT
<213> Artificial Sequence

<220>
<223> deltaG287

<400> 2

Ser Pro Asp Val Lys Ser Ala Asp Thr Leu Ser Lys Pro Ala Ala Pro
1 5 10 15

Val Val Ser Glu Lys Glu Thr Glu Ala Lys Glu Asp Ala Pro Gln Ala
20 25 30

Gly Ser Gln Gly Gln Gly Ala Pro Ser Ala Gln Gly Ser Gln Asp Met
35 40 45

Ala Ala Val Ser Glu Glu Asn Thr Gly Asn Gly Gly Ala Val Thr Ala
50 55 60

Asp Asn Pro Lys Asn Glu Asp Glu Val Ala Gln Asn Asp Met Pro Gln
65 70 75 80

Asn Ala Ala Gly Thr Asp Ser Ser Thr Pro Asn His Thr Pro Asp Pro
85 90 95

Asn Met Leu Ala Gly Asn Met Glu Asn Gln Ala Thr Asp Ala Gly Glu
100 105 110

Ser Ser Gln Pro Ala Asn Gln Pro Asp Met Ala Asn Ala Ala Asp Gly
115 120 125

Met Gln Gly Asp Asp Pro Ser Ala Gly Gly Gln Asn Ala Gly Asn Thr
130 135 140

Ala Ala Gln Gly Ala Asn Gln Ala Gly Asn Asn Gln Ala Ala Gly Ser
145 150 155 160

```
Ser Asp Pro Ile Pro Ala Ser Asn Pro Ala Pro Ala Asn Gly Gly Ser
                165                 170                 175

Asn Phe Gly Arg Val Asp Leu Ala Asn Gly Val Leu Ile Asp Gly Pro
            180                 185                 190

Ser Gln Asn Ile Thr Leu Thr His Cys Lys Gly Asp Ser Cys Ser Gly
        195                 200                 205

Asn Asn Phe Leu Asp Glu Glu Val Gln Leu Lys Ser Glu Phe Glu Lys
    210                 215                 220

Leu Ser Asp Ala Asp Lys Ile Ser Asn Tyr Lys Lys Asp Gly Lys Asn
225                 230                 235                 240

Asp Lys Phe Val Gly Leu Val Ala Asp Ser Val Gln Met Lys Gly Ile
                245                 250                 255

Asn Gln Tyr Ile Ile Phe Tyr Lys Pro Lys Pro Thr Ser Phe Ala Arg
            260                 265                 270

Phe Arg Arg Ser Ala Arg Ser Arg Arg Ser Leu Pro Ala Glu Met Pro
        275                 280                 285

Leu Ile Pro Val Asn Gln Ala Asp Thr Leu Ile Val Asp Gly Glu Ala
    290                 295                 300

Val Ser Leu Thr Gly His Ser Gly Asn Ile Phe Ala Pro Glu Gly Asn
305                 310                 315                 320

Tyr Arg Tyr Leu Thr Tyr Gly Ala Glu Lys Leu Pro Gly Gly Ser Tyr
                325                 330                 335

Ala Leu Arg Val Gln Gly Glu Pro Ala Lys Gly Glu Met Leu Ala Gly
            340                 345                 350

Ala Ala Val Tyr Asn Gly Glu Val Leu His Phe His Thr Glu Asn Gly
        355                 360                 365

Arg Pro Tyr Pro Thr Arg Gly Arg Phe Ala Ala Lys Val Asp Phe Gly
    370                 375                 380

Ser Lys Ser Val Asp Gly Ile Ile Asp Ser Gly Asp Asp Leu His Met
385                 390                 395                 400

Gly Thr Gln Lys Phe Lys Ala Ala Ile Asp Gly Asn Gly Phe Lys Gly
                405                 410                 415

Thr Trp Thr Glu Asn Gly Ser Gly Asp Val Ser Gly Lys Phe Tyr Gly
            420                 425                 430

Pro Ala Gly Glu Glu Val Ala Gly Lys Tyr Ser Tyr Arg Pro Thr Asp
        435                 440                 445

Ala Glu Lys Gly Gly Phe Gly Val Phe Ala Gly Lys Lys Glu Gln Asp
    450                 455                 460
```

<210> 3

<211> 2505
<212> DNA
<213> Artificial Sequence

<220>
<223> deltaG287-919

<400> 3

```
atggctagcc ccgatgttaa atcggcggac acgctgtcaa aaccggccgc tcctgttgtt   60
gctgaaaaag agacagaggt aaaagaagat gcgccacagg caggttctca aggacagggc  120
gcgccatcca cacaaggcag ccaagatatg gcggcagttt cggcagaaaa tacaggcaat  180
ggcggtgcgg caacaacgga caaacccaaa aatgaagacg agggaccgca aaatgatatg  240
ccgcaaaatt ccgccgaatc cgcaaatcaa acagggaaca accaacccgc cgattcttca  300
gattccgccc ccgcgtcaaa ccctgcacct gcgaatggcg gtagcaattt tggaagggtt  360
gatttggcta atggcgtttt gattgatggg ccgtcgcaaa atataacgtt gacccactgt  420
aaaggcgatt cttgtaatgg tgataattta ttggatgaag aagcaccgtc aaaatcagaa  480
tttgaaaatt taaatgagtc tgaacgaatt gagaaatata agaaagatgg gaaaagcgat  540
aaatttacta atttggttgc gacagcagtt caagctaatg gaactaacaa atatgtcatc  600
atttataaag acaagtccgc ttcatcttca tctgcgcgat tcaggcgttc tgcacggtcg  660
aggaggtcgc ttcctgccga gatgccgcta atccccgtca atcaggcgga tacgctgatt  720
gtcgatgggg aagcggtcag cctgacgggg cattccggca atatcttcgc gcccgaaggg  780
aattaccggt atctgactta cggggcggaa aaattgcccg gcggatcgta tgccctccgt  840
gtgcaaggcg aaccggcaaa aggcgaaatg cttgctggca cggccgtgta caacggcgaa  900
gtgctgcatt ttcatacgga aaacggccgt ccgtacccga ctagaggcag gtttgccgca  960
aaagtcgatt tcggcagcaa atctgtggac ggcattatcg acagcggcga tgatttgcat 1020
atgggtacgc aaaaattcaa agccgccatc gatggaaacg gctttaaggg gacttggacg 1080
gaaaatggcg gcggggatgt ttccggaagg ttttacggcc cggccggcga ggaagtggcg 1140
ggaaaataca gctatcgccc gacagatgcg gaaaagggcg gattcggcgt gtttgccggc 1200
aaaaaagagc aggatggatc cggaggagga ggatgccaaa gcaagagcat ccaaaccttt 1260
ccgcaacccg acacatccgt catcaacggc ccggaccggc cggtcggcat ccccgacccc 1320
gccggaacga cggtcggcgg cggcggggcc gtctataccg ttgtaccgca cctgtccctg 1380
ccccactggg cggcgcagga tttcgccaaa agcctgcaat ccttccgcct cggctgcgcc 1440
aatttgaaaa accgccaagg ctggcaggat gtgtgcgccc aagcctttca aaccccgtc  1500
cattcctttc aggcaaaaca gtttttgaa cgctatttca cgccgtggca ggttgcaggc  1560
aacggaagcc ttgccggtac ggttaccggc tattacgagc cggtgctgaa gggcgacgac 1620
aggcggacgg cacaagcccg cttcccgatt tacggtattc ccgacgattt tatctccgtc 1680
cccctgcctg ccggtttgcg gagcggaaaa gcccttgtcc gcatcaggca gacgggaaaa 1740
aacagcggca caatcgacaa taccggcggc acacataccg ccgacctctc ccgattcccc 1800
atcaccgcgc gcacaacggc aatcaaaggc aggtttgaag gaagccgctt cctcccctac 1860
cacacgcgca accaaatcaa cggcggcgcg cttgacggca aagccccgat actcggttac 1920
gccgaagacc ccgtcgaact tttttttatg cacatccaag gctcgggccg tctgaaaacc 1980
ccgtccggca aatacatccg catcggctat gccgacaaaa acgaacatcc ctacgtttcc 2040
atcggacgct atatggcgga caaaggctac ctcaagctcg ggcagacctc gatgcagggc 2100
atcaaagcct atatgcggca aaatccgcaa cgcctcgccg aagttttggg tcaaaacccc 2160
agctatatct tttttccgcga gcttgccgga agcagcaatg acggtcccgt cggcgcactg 2220
ggcacgccgt tgatgggga atatgccggc gcagtcgacc ggcactacat taccttgggc 2280
gcgcccttat ttgtcgccac cgcccatccg gttacccgca aagccctcaa ccgcctgatt 2340
atggcgcagg ataccggcag cgcgattaaa ggcgcggtgc gcgtggatta tttttgggga 2400
tacggcgacg aagccggcga acttgccggc aaacagaaaa ccacgggtta cgtctggcag 2460
ctcctaccca acggtatgaa gcccgaatac cgcccgtaac tcgag                  2505
```

<210> 4
<211> 832
<212> PRT
<213> Artificial Sequence

<220>
<223> deltaG287-919

<400> 4

```
Met Ala Ser Pro Asp Val Lys Ser Ala Asp Thr Leu Ser Lys Pro Ala
1               5                   10                  15
```

```
Ala Pro Val Val Ala Glu Lys Glu Thr Glu Val Lys Glu Asp Ala Pro
            20                  25                  30

Gln Ala Gly Ser Gln Gly Gln Gly Ala Pro Ser Thr Gln Gly Ser Gln
        35                  40                  45

Asp Met Ala Ala Val Ser Ala Glu Asn Thr Gly Asn Gly Gly Ala Ala
    50                  55                  60

Thr Thr Asp Lys Pro Lys Asn Glu Asp Glu Gly Pro Gln Asn Asp Met
65                  70                  75                  80

Pro Gln Asn Ser Ala Glu Ser Ala Asn Gln Thr Gly Asn Asn Gln Pro
                85                  90                  95

Ala Asp Ser Ser Asp Ser Ala Pro Ala Ser Asn Pro Ala Pro Ala Asn
            100                 105                 110

Gly Gly Ser Asn Phe Gly Arg Val Asp Leu Ala Asn Gly Val Leu Ile
        115                 120                 125

Asp Gly Pro Ser Gln Asn Ile Thr Leu Thr His Cys Lys Gly Asp Ser
    130                 135                 140

Cys Asn Gly Asp Asn Leu Leu Asp Glu Glu Ala Pro Ser Lys Ser Glu
145                 150                 155                 160

Phe Glu Asn Leu Asn Glu Ser Glu Arg Ile Glu Lys Tyr Lys Lys Asp
                165                 170                 175

Gly Lys Ser Asp Lys Phe Thr Asn Leu Val Ala Thr Ala Val Gln Ala
            180                 185                 190

Asn Gly Thr Asn Lys Tyr Val Ile Ile Tyr Lys Asp Lys Ser Ala Ser
        195                 200                 205

Ser Ser Ser Ala Arg Phe Arg Arg Ser Ala Arg Ser Arg Arg Ser Leu
    210                 215                 220

Pro Ala Glu Met Pro Leu Ile Pro Val Asn Gln Ala Asp Thr Leu Ile
225                 230                 235                 240

Val Asp Gly Glu Ala Val Ser Leu Thr Gly His Ser Gly Asn Ile Phe
                245                 250                 255

Ala Pro Glu Gly Asn Tyr Arg Tyr Leu Thr Tyr Gly Ala Glu Lys Leu
            260                 265                 270

Pro Gly Gly Ser Tyr Ala Leu Arg Val Gln Gly Glu Pro Ala Lys Gly
        275                 280                 285

Glu Met Leu Ala Gly Thr Ala Val Tyr Asn Gly Glu Val Leu His Phe
    290                 295                 300

His Thr Glu Asn Gly Arg Pro Tyr Pro Thr Arg Gly Arg Phe Ala Ala
305                 310                 315                 320

Lys Val Asp Phe Gly Ser Lys Ser Val Asp Gly Ile Ile Asp Ser Gly
                325                 330                 335
```

Asp Asp Leu His Met Gly Thr Gln Lys Phe Lys Ala Ala Ile Asp Gly
340 345 350

Asn Gly Phe Lys Gly Thr Trp Thr Glu Asn Gly Gly Gly Asp Val Ser
355 360 365

Gly Arg Phe Tyr Gly Pro Ala Gly Glu Glu Val Ala Gly Lys Tyr Ser
370 375 380

Tyr Arg Pro Thr Asp Ala Glu Lys Gly Gly Phe Gly Val Phe Ala Gly
385 390 395 400

Lys Lys Glu Gln Asp Gly Ser Gly Gly Gly Gly Cys Gln Ser Lys Ser
405 410 415

Ile Gln Thr Phe Pro Gln Pro Asp Thr Ser Val Ile Asn Gly Pro Asp
420 425 430

Arg Pro Val Gly Ile Pro Asp Pro Ala Gly Thr Thr Val Gly Gly Gly
435 440 445

Gly Ala Val Tyr Thr Val Val Pro His Leu Ser Leu Pro His Trp Ala
450 455 460

Ala Gln Asp Phe Ala Lys Ser Leu Gln Ser Phe Arg Leu Gly Cys Ala
465 470 475 480

Asn Leu Lys Asn Arg Gln Gly Trp Gln Asp Val Cys Ala Gln Ala Phe
485 490 495

Gln Thr Pro Val His Ser Phe Gln Ala Lys Gln Phe Phe Glu Arg Tyr
500 505 510

Phe Thr Pro Trp Gln Val Ala Gly Asn Gly Ser Leu Ala Gly Thr Val
515 520 525

Thr Gly Tyr Tyr Glu Pro Val Leu Lys Gly Asp Asp Arg Arg Thr Ala
530 535 540

Gln Ala Arg Phe Pro Ile Tyr Gly Ile Pro Asp Asp Phe Ile Ser Val
545 550 555 560

Pro Leu Pro Ala Gly Leu Arg Ser Gly Lys Ala Leu Val Arg Ile Arg
565 570 575

Gln Thr Gly Lys Asn Ser Gly Thr Ile Asp Asn Thr Gly Gly Thr His
580 585 590

Thr Ala Asp Leu Ser Arg Phe Pro Ile Thr Ala Arg Thr Thr Ala Ile
595 600 605

Lys Gly Arg Phe Glu Gly Ser Arg Phe Leu Pro Tyr His Thr Arg Asn
610 615 620

Gln Ile Asn Gly Gly Ala Leu Asp Gly Lys Ala Pro Ile Leu Gly Tyr
625 630 635 640

Ala Glu Asp Pro Val Glu Leu Phe Phe Met His Ile Gln Gly Ser Gly
645 650 655

Arg Leu Lys Thr Pro Ser Gly Lys Tyr Ile Arg Ile Gly Tyr Ala Asp

```
            660                 665                 670

Lys Asn Glu His Pro Tyr Val Ser Ile Gly Arg Tyr Met Ala Asp Lys
        675                 680                 685

Gly Tyr Leu Lys Leu Gly Gln Thr Ser Met Gln Gly Ile Lys Ala Tyr
    690                 695                 700

Met Arg Gln Asn Pro Gln Arg Leu Ala Glu Val Leu Gly Gln Asn Pro
705                 710                 715                 720

Ser Tyr Ile Phe Phe Arg Glu Leu Ala Gly Ser Ser Asn Asp Gly Pro
                725                 730                 735

Val Gly Ala Leu Gly Thr Pro Leu Met Gly Glu Tyr Ala Gly Ala Val
            740                 745                 750

Asp Arg His Tyr Ile Thr Leu Gly Ala Pro Leu Phe Val Ala Thr Ala
        755                 760                 765

His Pro Val Thr Arg Lys Ala Leu Asn Arg Leu Ile Met Ala Gln Asp
    770                 775                 780

Thr Gly Ser Ala Ile Lys Gly Ala Val Arg Val Asp Tyr Phe Trp Gly
785                 790                 795                 800

Tyr Gly Asp Glu Ala Gly Glu Leu Ala Gly Lys Gln Lys Thr Thr Gly
                805                 810                 815

Tyr Val Trp Gln Leu Leu Pro Asn Gly Met Lys Pro Glu Tyr Arg Pro
            820                 825                 830
```

<210> 5
<211> 1746
<212> DNA
<213> Artificial Sequence

<220>
<223> deltaG287-953

<400> 5

```
atggctagcc ccgatgttaa atcggcggac acgctgtcaa aaccggccgc tcctgttgtt   60
gctgaaaaag agacagaggt aaaagaagat gcgccacagg caggttctca aggacagggc  120
gcgccatcca cacaaggcag ccaagatatg gcggcagttt cggcagaaaa tacaggcaat  180
ggcggtgcgg caacaacgga caaacccaaa aatgaagacg agggaccgca aaatgatatg  240
ccgcaaaatt ccgccgaatc cgcaaatcaa acagggaaca accaacccgc cgattcttca  300
gattccgccc ccgcgtcaaa ccctgcacct gcgaatggcg gtagcaattt tggaagggtt  360
gatttggcta atggcgtttt gattgatggg ccgtcgcaaa atataacgtt gacccactgt  420
aaaggcgatt cttgtaatgg tgataattta ttggatgaag aagcaccgtc aaaatcagaa  480
tttgaaaatt taaatgagtc tgaacgaatt gagaaatata agaaagatgg gaaaagcgat  540
aaatttacta atttggttgc gacagcagtt caagctaatg gaactaacaa atatgtcatc  600
atttataaag acaagtccgc ttcatcttca tctgcgcgat tcaggcgttc tgcacggtcg  660
aggaggtcgc ttcctgccga gatgccgcta atccccgtca atcaggcgga tacgctgatt  720
gtcgatgggg aagcggtcag cctgacgggg cattccggca atatcttcgc gcccgaaggg  780
aattaccggt atctgactta cggggcggaa aaattgcccg gcggatcgta tgccctccgt  840
gtgcaaggcg aaccggcaaa aggcgaaatg cttgctggca cggccgtgta caacggcgaa  900
gtgctgcatt ttcatacgga aaacggccgt ccgtacccga ctagaggcag gtttgccgca  960
aaagtcgatt tcggcagcaa atctgtggac ggcattatcg acagcggcga tgatttgcat 1020
atgggtacgc aaaaattcaa agccgccatc gatggaaacg gctttaaggg gacttggacg 1080
gaaaatggcg gcggggatgt ttccggaagg ttttacggcc cggccggcga ggaagtggcg 1140

ggaaaataca gctatcgccc gacagatgcg gaaaagggcg gattcggcgt gtttgccggc 1200
aaaaaagagc aggatggatc cggaggagga ggagccacct acaaagtgga cgaatatcac 1260
gccaacgccc gtttcgccat cgaccatttc aacaccagca ccaacgtcgg cggtttttac 1320
ggtctgaccg gttccgtcga gttcgaccaa gcaaaacgcg acggtaaaat cgacatcacc 1380
atccccgttg ccaacctgca aagcggttcg caacacttta ccgaccacct gaaatcagcc 1440
gacatcttcg atgccgccca atatccggac atccgctttg tttccaccaa attcaacttc 1500
aacggcaaaa aactggtttc cgttgacggc aacctgacca tgcacggcaa aaccgccccc 1560
gtcaaactca aagccgaaaa attcaactgc taccaaagcc cgatggcgaa aaccgaagtt 1620
tgcggcggcg acttcagcac caccatcgac cgcaccaaat ggggcgtgga ctacctcgtt 1680
aacgttggta tgaccaaaag cgtccgcatc gacatccaaa tcgaggcagc caaacaataa 1740
ctcgag                                                            1746
```

<210> 6
<211> 579
<212> PRT
<213> Artificial Sequence

<220>
<223> deltaG287-953

<400> 6

Met Ala Ser Pro Asp Val Lys Ser Ala Asp Thr Leu Ser Lys Pro Ala
1 5 10 15

Ala Pro Val Val Ala Glu Lys Glu Thr Glu Val Lys Glu Asp Ala Pro
20 25 30

Gln Ala Gly Ser Gln Gly Gln Gly Ala Pro Ser Thr Gln Gly Ser Gln
35 40 45

Asp Met Ala Ala Val Ser Ala Glu Asn Thr Gly Asn Gly Gly Ala Ala
50 55 60

Thr Thr Asp Lys Pro Lys Asn Glu Asp Glu Gly Pro Gln Asn Asp Met
65 70 75 80

Pro Gln Asn Ser Ala Glu Ser Ala Asn Gln Thr Gly Asn Asn Gln Pro
85 90 95

Ala Asp Ser Ser Asp Ser Ala Pro Ala Ser Asn Pro Ala Pro Ala Asn
100 105 110

Gly Gly Ser Asn Phe Gly Arg Val Asp Leu Ala Asn Gly Val Leu Ile
115 120 125

Asp Gly Pro Ser Gln Asn Ile Thr Leu Thr His Cys Lys Gly Asp Ser
130 135 140

Cys Asn Gly Asp Asn Leu Leu Asp Glu Glu Ala Pro Ser Lys Ser Glu
145 150 155 160

Phe Glu Asn Leu Asn Glu Ser Glu Arg Ile Glu Lys Tyr Lys Lys Asp
165 170 175

Gly Lys Ser Asp Lys Phe Thr Asn Leu Val Ala Thr Ala Val Gln Ala
180 185 190

Asn Gly Thr Asn Lys Tyr Val Ile Ile Tyr Lys Asp Lys Ser Ala Ser
195 200 205

Ser Ser Ser Ala Arg Phe Arg Arg Ser Ala Arg Ser Arg Arg Ser Leu

```
    210                 215                 220

Pro Ala Glu Met Pro Leu Ile Pro Val Asn Gln Ala Asp Thr Leu Ile
225                 230                 235                 240

Val Asp Gly Glu Ala Val Ser Leu Thr Gly His Ser Gly Asn Ile Phe
                245                 250                 255

Ala Pro Glu Gly Asn Tyr Arg Tyr Leu Thr Tyr Gly Ala Glu Lys Leu
            260                 265                 270

Pro Gly Gly Ser Tyr Ala Leu Arg Val Gln Gly Glu Pro Ala Lys Gly
        275                 280                 285

Glu Met Leu Ala Gly Thr Ala Val Tyr Asn Gly Glu Val Leu His Phe
    290                 295                 300

His Thr Glu Asn Gly Arg Pro Tyr Pro Thr Arg Gly Arg Phe Ala Ala
305                 310                 315                 320

Lys Val Asp Phe Gly Ser Lys Ser Val Asp Gly Ile Ile Asp Ser Gly
                325                 330                 335

Asp Asp Leu His Met Gly Thr Gln Lys Phe Lys Ala Ala Ile Asp Gly
            340                 345                 350

Asn Gly Phe Lys Gly Thr Trp Thr Glu Asn Gly Gly Gly Asp Val Ser
        355                 360                 365

Gly Arg Phe Tyr Gly Pro Ala Gly Glu Glu Val Ala Gly Lys Tyr Ser
    370                 375                 380

Tyr Arg Pro Thr Asp Ala Glu Lys Gly Gly Phe Gly Val Phe Ala Gly
385                 390                 395                 400

Lys Lys Glu Gln Asp Gly Ser Gly Gly Gly Gly Ala Thr Tyr Lys Val
                405                 410                 415

Asp Glu Tyr His Ala Asn Ala Arg Phe Ala Ile Asp His Phe Asn Thr
            420                 425                 430

Ser Thr Asn Val Gly Gly Phe Tyr Gly Leu Thr Gly Ser Val Glu Phe
        435                 440                 445

Asp Gln Ala Lys Arg Asp Gly Lys Ile Asp Ile Thr Ile Pro Val Ala
    450                 455                 460

Asn Leu Gln Ser Gly Ser Gln His Phe Thr Asp His Leu Lys Ser Ala
465                 470                 475                 480

Asp Ile Phe Asp Ala Ala Gln Tyr Pro Asp Ile Arg Phe Val Ser Thr
                485                 490                 495

Lys Phe Asn Phe Asn Gly Lys Lys Leu Val Ser Val Asp Gly Asn Leu
            500                 505                 510

Thr Met His Gly Lys Thr Ala Pro Val Lys Leu Lys Ala Glu Lys Phe
        515                 520                 525

Asn Cys Tyr Gln Ser Pro Met Ala Lys Thr Glu Val Cys Gly Gly Asp
    530                 535                 540
```

Phe Ser Thr Thr Ile Asp Arg Thr Lys Trp Gly Val Asp Tyr Leu Val
545 550 555 560

Asn Val Gly Met Thr Lys Ser Val Arg Ile Asp Ile Gln Ile Glu Ala
565 570 575

Ala Lys Gln

<210> 7
<211> 2388
<212> DNA
<213> Artificial Sequence

<220>
<223> deltaG287-961

<400> 7

atggctagcc ccgatgttaa atcggcggac acgctgtcaa aaccggccgc tcctgttgtt 60
gctgaaaaag agacagaggt aaaagaagat gcgccacagg caggttctca aggacagggc 120
gcgccatcca cacaaggcag ccaagatatg gcggcagttt cggcagaaaa tacaggcaat 180
ggcggtgcgg caacaacgga caaacccaaa aatgaagacg agggaccgca aaatgatatg 240
ccgcaaaatt ccgccgaatc cgcaaatcaa acagggaaca accaacccgc cgattcttca 300
gattccgccc ccgcgtcaaa ccctgcacct gcgaatggcg gtagcaattt tggaagggtt 360
gatttggcta atggcgtttt gattgatggg ccgtcgcaaa atataacgtt gacccactgt 420
aaaggcgatt cttgtaatgg tgataattta ttggatgaag aagcaccgtc aaaatcagaa 480
tttgaaaatt taaatgagtc tgaacgaatt gagaaatata agaaagatgg gaaaagcgat 540
aaatttacta atttggttgc gacagcagtt caagctaatg gaactaacaa atatgtcatc 600
atttataaag acaagtccgc ttcatcttca tctgcgcgat tcaggcgttc tgcacggtcg 660
aggaggtcgc ttcctgccga gatgccgcta atccccgtca atcaggcgga tacgctgatt 720
gtcgatgggg aagcggtcag cctgacgggg cattccggca atatcttcgc gcccgaaggg 780
aattaccggt atctgactta cggggcggaa aaattgcccg gcggatcgta tgccctccgt 840
gtgcaaggcg aaccggcaaa aggcgaaatg cttgctggca cggccgtgta caacggcgaa 900
gtgctgcatt ttcatacgga aaacggccgt ccgtacccga ctagaggcag gtttgccgca 960
aaagtcgatt tcggcagcaa atctgtggac ggcattatcg acagcggcga tgatttgcat 1020
atgggtacgc aaaaattcaa agccgccatc gatggaaacg gctttaaggg gacttggacg 1080
gaaaatggcg gcggggatgt ttccggaagg ttttacggcc cggccggcga ggaagtggcg 1140
ggaaaataca gctatcgccc gacagatgcg gaaaagggcg gattcggcgt gtttgccggc 1200
aaaaaagagc aggatggatc cggaggagga ggagccacaa acgacgacga tgttaaaaaa 1260
gctgccactg tggccattgc tgctgcctac aacaatggcc aagaaatcaa cggtttcaaa 1320
gctggagaga ccatctacga cattgatgaa gacggcacaa ttaccaaaaa agacgcaact 1380
gcagccgatg ttgaagccga cgactttaaa ggtctgggtc tgaaaaaagt cgtgactaac 1440
ctgaccaaaa ccgtcaatga aaacaaacaa aacgtcgatg ccaaagtaaa agctgcagaa 1500
tctgaaatag aaaagttaac aaccaagtta gcagacactg atgccgcttt agcagatact 1560
gatgccgctc tggatgcaac caccaacgcc ttgaataaat tgggagaaaa tataacgaca 1620
tttgctgaag agactaagac aaatatcgta aaaattgatg aaaaattaga agccgtggct 1680
gataccgtcg acaagcatgc cgaagcattc aacgatatcg ccgattcatt ggatgaaacc 1740
aacactaagg cagacgaagc cgtcaaaacc gccaatgaag ccaaacagac ggccgaagaa 1800
accaaacaaa acgtcgatgc caaagtaaaa gctgcagaaa ctgcagcagg caaagccgaa 1860
gctgccgctg gcacagctaa tactgcagcc gacaaggccg aagctgtcgc tgcaaaagtt 1920
accgacatca aagctgatat cgctacgaac aaagataata ttgctaaaaa agcaaacagt 1980
gccgacgtgt acaccagaga agagtctgac agcaaatttg tcagaattga tggtctgaac 2040
gctactaccg aaaaattgga cacacgcttg gcttctgctg aaaaatccat tgccgatcac 2100
gatactcgcc tgaacggttt ggataaaaca gtgtcagacc tgcgcaaaga aacccgccaa 2160
ggccttgcag aacaagccgc gctctccggt ctgttccaac cttacaacgt gggtcggttc 2220
aatgtaacgg ctgcagtcgg cggctacaaa tccgaatcgg cagtcgccat cggtaccggc 2280
ttccgcttta ccgaaaactt tgccgccaaa gcaggcgtgg cagtcggcac ttcgtccggt 2340
tcttccgcag cctaccatgt cggcgtcaat tacgagtggt aactcgag 2388

<210> 8
<211> 793
<212> PRT
<213> Artificial Sequence

<220>
<223> deltaG287-961

<400> 8

Met Ala Ser Pro Asp Val Lys Ser Ala Asp Thr Leu Ser Lys Pro Ala
1 5 10 15

Ala Pro Val Val Ala Glu Lys Glu Thr Glu Val Lys Glu Asp Ala Pro
20 25 30

Gln Ala Gly Ser Gln Gly Gln Gly Ala Pro Ser Thr Gln Gly Ser Gln
35 40 45

Asp Met Ala Ala Val Ser Ala Glu Asn Thr Gly Asn Gly Gly Ala Ala
50 55 60

Thr Thr Asp Lys Pro Lys Asn Glu Asp Glu Gly Pro Gln Asn Asp Met
65 70 75 80

Pro Gln Asn Ser Ala Glu Ser Ala Asn Gln Thr Gly Asn Asn Gln Pro
85 90 95

Ala Asp Ser Ser Asp Ser Ala Pro Ala Ser Asn Pro Ala Pro Ala Asn
100 105 110

Gly Gly Ser Asn Phe Gly Arg Val Asp Leu Ala Asn Gly Val Leu Ile
115 120 125

Asp Gly Pro Ser Gln Asn Ile Thr Leu Thr His Cys Lys Gly Asp Ser
130 135 140

Cys Asn Gly Asp Asn Leu Leu Asp Glu Glu Ala Pro Ser Lys Ser Glu
145 150 155 160

Phe Glu Asn Leu Asn Glu Ser Glu Arg Ile Glu Lys Tyr Lys Lys Asp
165 170 175

Gly Lys Ser Asp Lys Phe Thr Asn Leu Val Ala Thr Ala Val Gln Ala
180 185 190

Asn Gly Thr Asn Lys Tyr Val Ile Ile Tyr Lys Asp Lys Ser Ala Ser
195 200 205

Ser Ser Ser Ala Arg Phe Arg Arg Ser Ala Arg Ser Arg Arg Ser Leu
210 215 220

Pro Ala Glu Met Pro Leu Ile Pro Val Asn Gln Ala Asp Thr Leu Ile
225 230 235 240

Val Asp Gly Glu Ala Val Ser Leu Thr Gly His Ser Gly Asn Ile Phe
245 250 255

Ala Pro Glu Gly Asn Tyr Arg Tyr Leu Thr Tyr Gly Ala Glu Lys Leu
260 265 270

Pro Gly Gly Ser Tyr Ala Leu Arg Val Gln Gly Glu Pro Ala Lys Gly
275 280 285

Glu Met Leu Ala Gly Thr Ala Val Tyr Asn Gly Glu Val Leu His Phe
    290                 295                 300

His Thr Glu Asn Gly Arg Pro Tyr Pro Thr Arg Gly Arg Phe Ala Ala
305                 310                 315                 320

Lys Val Asp Phe Gly Ser Lys Ser Val Asp Gly Ile Ile Asp Ser Gly
                325                 330                 335

Asp Asp Leu His Met Gly Thr Gln Lys Phe Lys Ala Ala Ile Asp Gly
            340                 345                 350

Asn Gly Phe Lys Gly Thr Trp Thr Glu Asn Gly Gly Gly Asp Val Ser
        355                 360                 365

Gly Arg Phe Tyr Gly Pro Ala Gly Glu Glu Val Ala Gly Lys Tyr Ser
    370                 375                 380

Tyr Arg Pro Thr Asp Ala Glu Lys Gly Gly Phe Gly Val Phe Ala Gly
385                 390                 395                 400

Lys Lys Glu Gln Asp Gly Ser Gly Gly Gly Gly Ala Thr Asn Asp Asp
                405                 410                 415

Asp Val Lys Lys Ala Ala Thr Val Ala Ile Ala Ala Ala Tyr Asn Asn
            420                 425                 430

Gly Gln Glu Ile Asn Gly Phe Lys Ala Gly Glu Thr Ile Tyr Asp Ile
        435                 440                 445

Asp Glu Asp Gly Thr Ile Thr Lys Lys Asp Ala Thr Ala Ala Asp Val
    450                 455                 460

Glu Ala Asp Asp Phe Lys Gly Leu Gly Leu Lys Lys Val Val Thr Asn
465                 470                 475                 480

Leu Thr Lys Thr Val Asn Glu Asn Lys Gln Asn Val Asp Ala Lys Val
                485                 490                 495

Lys Ala Ala Glu Ser Glu Ile Glu Lys Leu Thr Thr Lys Leu Ala Asp
            500                 505                 510

Thr Asp Ala Ala Leu Ala Asp Thr Asp Ala Ala Leu Asp Ala Thr Thr
        515                 520                 525

Asn Ala Leu Asn Lys Leu Gly Glu Asn Ile Thr Thr Phe Ala Glu Glu
    530                 535                 540

Thr Lys Thr Asn Ile Val Lys Ile Asp Glu Lys Leu Glu Ala Val Ala
545                 550                 555                 560

Asp Thr Val Asp Lys His Ala Glu Ala Phe Asn Asp Ile Ala Asp Ser
                565                 570                 575

Leu Asp Glu Thr Asn Thr Lys Ala Asp Glu Ala Val Lys Thr Ala Asn
            580                 585                 590

Glu Ala Lys Gln Thr Ala Glu Glu Thr Lys Gln Asn Val Asp Ala Lys
        595                 600                 605

```
Val Lys Ala Ala Glu Thr Ala Ala Gly Lys Ala Glu Ala Ala Ala Gly
    610                 615                 620

Thr Ala Asn Thr Ala Ala Asp Lys Ala Glu Ala Val Ala Ala Lys Val
625                 630                 635                 640

Thr Asp Ile Lys Ala Asp Ile Ala Thr Asn Lys Asp Asn Ile Ala Lys
                645                 650                 655

Lys Ala Asn Ser Ala Asp Val Tyr Thr Arg Glu Glu Ser Asp Ser Lys
            660                 665                 670

Phe Val Arg Ile Asp Gly Leu Asn Ala Thr Thr Glu Lys Leu Asp Thr
        675                 680                 685

Arg Leu Ala Ser Ala Glu Lys Ser Ile Ala Asp His Asp Thr Arg Leu
    690                 695                 700

Asn Gly Leu Asp Lys Thr Val Ser Asp Leu Arg Lys Glu Thr Arg Gln
705                 710                 715                 720

Gly Leu Ala Glu Gln Ala Ala Leu Ser Gly Leu Phe Gln Pro Tyr Asn
                725                 730                 735

Val Gly Arg Phe Asn Val Thr Ala Ala Val Gly Gly Tyr Lys Ser Glu
            740                 745                 750

Ser Ala Val Ala Ile Gly Thr Gly Phe Arg Phe Thr Glu Asn Phe Ala
        755                 760                 765

Ala Lys Ala Gly Val Ala Val Gly Thr Ser Ser Gly Ser Ser Ala Ala
    770                 775                 780

Tyr His Val Gly Val Asn Tyr Glu Trp
785                 790
```

<210> 9
<211> 2700
<212> DNA
<213> Artificial Sequence

<220>
<223> deltaG287NZ-919

<400> 9

```
atggctagcc ccgatgtcaa gtcggcggac acgctgtcaa aacctgccgc ccctgttgtt   60
tctgaaaaag agacagaggc aaaggaagat gcgccacagg caggttctca aggacagggc  120
gcgccatccg cacaaggcgg tcaagatatg gcggcggttt cggaagaaaa tacaggcaat  180
ggcggtgcgg cagcaacgga caaacccaaa aatgaagacg agggggcgca aaatgatatg  240
ccgcaaaatg ccgccgatac agatagtttg acaccgaatc acaccccggc ttcgaatatg  300
ccggccggaa atatggaaaa ccaagcaccg gatgccgggg aatcggagca gccggcaaac  360
caaccggata tggcaaatac ggcggacgga atgcagggtg acgatccgtc ggcaggcggg  420
gaaaatgccg gcaatacggc tgcccaaggt acaaatcaag ccgaaaacaa tcaaaccgcc  480
ggttctcaaa atcctgcctc ttcaaccaat cctagcgcca cgaatagcgg tggtgatttt  540
ggaaggacga acgtgggcaa ttctgttgtg attgacgggc cgtcgcaaaa tataacgttg  600
acccactgta aaggcgattc ttgtagtggc aataatttct tggatgaaga agtacagcta  660
aaatcagaat ttgaaaaatt aagtgatgca gacaaaataa gtaattacaa gaaagatggg  720
aagaatgacg ggaagaatga taaatttgtc ggtttggttg ccgatagtgt gcagatgaag  780
ggaatcaatc aatatattat cttttataaa cctaaaccca cttcatttgc gcgatttagg  840
cgttctgcac ggtcgaggcg gtcgcttccg gccgagatgc cgctgattcc cgtcaatcag  900
gcggatacgc tgattgtcga tggggaagcg gtcagcctga cggggcattc cggcaatatc  960

ttcgcgcccg aagggaatta ccggtatctg acttacgggg cggaaaaatt gcccggcgga  1020
tcgtatgccc tccgtgttca aggcgaacct tcaaaaggcg aaatgctcgc gggcacggca  1080
gtgtacaacg gcgaagtgct gcattttcat acggaaaacg gccgtccgtc cccgtccaga  1140
ggcaggtttg ccgcaaaagt cgatttcggc agcaaatctg tggacggcat tatcgacagc  1200
ggcgatggtt tgcatatggg tacgcaaaaa ttcaaagccg ccatcgatgg aaacggcttt  1260
aaggggactt ggacggaaaa tggcggcggg gatgtttccg gaaagtttta cggcccggcc  1320
ggcgaggaag tggcgggaaa atacagctat cgcccaacag atgcggaaaa gggcggattc  1380
ggcgtgtttg ccggcaaaaa agagcaggat ggatccggag gaggaggatg ccaaagcaag  1440
agcatccaaa cctttccgca acccgacaca tccgtcatca acggcccgga ccggccggtc  1500
ggcatccccg accccgccgg aacgacggtc ggcggcggcg ggccgtcta taccgttgta  1560
ccgcacctgt ccctgcccca ctgggcggcg caggatttcg ccaaaagcct gcaatccttc  1620
cgcctcggct gcgccaattt gaaaaaccgc caaggctggc aggatgtgtg cgcccaagcc  1680
tttcaaaccc ccgtccattc ctttcaggca aaacagtttt ttgaacgcta tttcacgccg  1740
tggcaggttg caggcaacgg aagccttgcc ggtacggtta ccggctatta cgagccggtg  1800
ctgaagggcg acgacaggcg gacggcacaa gcccgcttcc cgatttacgg tattcccgac  1860
gattttatct ccgtccccct gcctgccggt ttgcggagcg gaaaagccct tgtccgcatc  1920
aggcagacgg gaaaaaacag cggcacaatc gacaataccg gcggcacaca taccgccgac  1980
ctctcccgat tccccatcac cgcgcgcaca acggcaatca aaggcaggtt tgaaggaagc  2040
cgcttcctcc cctaccacac gcgcaaccaa atcaacggcg gcgcgcttga cggcaaagcc  2100
ccgatactcg gttacgccga agaccccgtc gaactttttt ttatgcacat ccaaggctcg  2160
ggccgtctga aaaccccgtc cggcaaatac atccgcatcg gctatgccga caaaaacgaa  2220
catccctacg tttccatcgg acgctatatg gcggacaaag gctacctcaa gctcgggcag  2280
acctcgatgc agggcatcaa agcctatatg cggcaaaatc cgcaacgcct cgccgaagtt  2340
ttgggtcaaa accccagcta tatctttttc cgcgagcttg ccggaagcag caatgacggt  2400
cccgtcggcg cactgggcac gccgttgatg gggggaatatg ccggcgcagt cgaccggcac  2460
tacattacct tggcgcgcc cttatttgtc gccaccgccc atccggttac ccgcaaagcc  2520
ctcaaccgcc tgattatggc gcaggatacc ggcagcgcga ttaaaggcgc ggtgcgcgtg  2580
gattattttt ggggatacgg cgacgaagcc ggcgaacttg ccggcaaaca gaaaaccacg  2640
ggttacgtct ggcagctcct acccaacggt atgaagcccg aataccgccc gtaaaagctt  2700
```

<210> 10
<211> 897
<212> PRT
<213> Artificial Sequence

<220>
<223> deltaG287NZ-919

<400> 10

```
Met Ala Ser Pro Asp Val Lys Ser Ala Asp Thr Leu Ser Lys Pro Ala
1               5                   10                  15

Ala Pro Val Val Ser Glu Lys Glu Thr Glu Ala Lys Glu Asp Ala Pro
            20                  25                  30

Gln Ala Gly Ser Gln Gly Gln Gly Ala Pro Ser Ala Gln Gly Gly Gln
        35                  40                  45

Asp Met Ala Ala Val Ser Glu Glu Asn Thr Gly Asn Gly Gly Ala Ala
    50                  55                  60

Ala Thr Asp Lys Pro Lys Asn Glu Asp Glu Gly Ala Gln Asn Asp Met
65                  70                  75                  80

Pro Gln Asn Ala Ala Asp Thr Asp Ser Leu Thr Pro Asn His Thr Pro
                85                  90                  95

Ala Ser Asn Met Pro Ala Gly Asn Met Glu Asn Gln Ala Pro Asp Ala
            100                 105                 110

Gly Glu Ser Glu Gln Pro Ala Asn Gln Pro Asp Met Ala Asn Thr Ala
```

115 120 125

Asp Gly Met Gln Gly Asp Asp Pro Ser Ala Gly Gly Glu Asn Ala Gly
130 135 140

Asn Thr Ala Ala Gln Gly Thr Asn Gln Ala Glu Asn Asn Gln Thr Ala
145 150 155 160

Gly Ser Gln Asn Pro Ala Ser Ser Thr Asn Pro Ser Ala Thr Asn Ser
165 170 175

Gly Gly Asp Phe Gly Arg Thr Asn Val Gly Asn Ser Val Val Ile Asp
180 185 190

Gly Pro Ser Gln Asn Ile Thr Leu Thr His Cys Lys Gly Asp Ser Cys
195 200 205

Ser Gly Asn Asn Phe Leu Asp Glu Glu Val Gln Leu Lys Ser Glu Phe
210 215 220

Glu Lys Leu Ser Asp Ala Asp Lys Ile Ser Asn Tyr Lys Lys Asp Gly
225 230 235 240

Lys Asn Asp Gly Lys Asn Asp Lys Phe Val Gly Leu Val Ala Asp Ser
245 250 255

Val Gln Met Lys Gly Ile Asn Gln Tyr Ile Ile Phe Tyr Lys Pro Lys
260 265 270

Pro Thr Ser Phe Ala Arg Phe Arg Arg Ser Ala Arg Ser Arg Arg Ser
275 280 285

Leu Pro Ala Glu Met Pro Leu Ile Pro Val Asn Gln Ala Asp Thr Leu
290 295 300

Ile Val Asp Gly Glu Ala Val Ser Leu Thr Gly His Ser Gly Asn Ile
305 310 315 320

Phe Ala Pro Glu Gly Asn Tyr Arg Tyr Leu Thr Tyr Gly Ala Glu Lys
325 330 335

Leu Pro Gly Gly Ser Tyr Ala Leu Arg Val Gln Gly Glu Pro Ser Lys
340 345 350

Gly Glu Met Leu Ala Gly Thr Ala Val Tyr Asn Gly Glu Val Leu His
355 360 365

Phe His Thr Glu Asn Gly Arg Pro Ser Pro Ser Arg Gly Arg Phe Ala
370 375 380

Ala Lys Val Asp Phe Gly Ser Lys Ser Val Asp Gly Ile Ile Asp Ser
385 390 395 400

Gly Asp Gly Leu His Met Gly Thr Gln Lys Phe Lys Ala Ala Ile Asp
405 410 415

Gly Asn Gly Phe Lys Gly Thr Trp Thr Glu Asn Gly Gly Gly Asp Val
420 425 430

Ser Gly Lys Phe Tyr Gly Pro Ala Gly Glu Glu Val Ala Gly Lys Tyr
435 440 445

```
Ser Tyr Arg Pro Thr Asp Ala Glu Lys Gly Gly Phe Gly Val Phe Ala
    450                 455                 460

Gly Lys Lys Glu Gln Asp Gly Ser Gly Gly Gly Gly Cys Gln Ser Lys
465                 470                 475                 480

Ser Ile Gln Thr Phe Pro Gln Pro Asp Thr Ser Val Ile Asn Gly Pro
                485                 490                 495

Asp Arg Pro Val Gly Ile Pro Asp Pro Ala Gly Thr Thr Val Gly Gly
            500                 505                 510

Gly Gly Ala Val Tyr Thr Val Val Pro His Leu Ser Leu Pro His Trp
        515                 520                 525

Ala Ala Gln Asp Phe Ala Lys Ser Leu Gln Ser Phe Arg Leu Gly Cys
    530                 535                 540

Ala Asn Leu Lys Asn Arg Gln Gly Trp Gln Asp Val Cys Ala Gln Ala
545                 550                 555                 560

Phe Gln Thr Pro Val His Ser Phe Gln Ala Lys Gln Phe Phe Glu Arg
                565                 570                 575

Tyr Phe Thr Pro Trp Gln Val Ala Gly Asn Gly Ser Leu Ala Gly Thr
            580                 585                 590

Val Thr Gly Tyr Tyr Glu Pro Val Leu Lys Gly Asp Asp Arg Arg Thr
        595                 600                 605

Ala Gln Ala Arg Phe Pro Ile Tyr Gly Ile Pro Asp Asp Phe Ile Ser
    610                 615                 620

Val Pro Leu Pro Ala Gly Leu Arg Ser Gly Lys Ala Leu Val Arg Ile
625                 630                 635                 640

Arg Gln Thr Gly Lys Asn Ser Gly Thr Ile Asp Asn Thr Gly Gly Thr
                645                 650                 655

His Thr Ala Asp Leu Ser Arg Phe Pro Ile Thr Ala Arg Thr Thr Ala
            660                 665                 670

Ile Lys Gly Arg Phe Glu Gly Ser Arg Phe Leu Pro Tyr His Thr Arg
        675                 680                 685

Asn Gln Ile Asn Gly Gly Ala Leu Asp Gly Lys Ala Pro Ile Leu Gly
    690                 695                 700

Tyr Ala Glu Asp Pro Val Glu Leu Phe Phe Met His Ile Gln Gly Ser
705                 710                 715                 720

Gly Arg Leu Lys Thr Pro Ser Gly Lys Tyr Ile Arg Ile Gly Tyr Ala
                725                 730                 735

Asp Lys Asn Glu His Pro Tyr Val Ser Ile Gly Arg Tyr Met Ala Asp
            740                 745                 750

Lys Gly Tyr Leu Lys Leu Gly Gln Thr Ser Met Gln Gly Ile Lys Ala
        755                 760                 765
```

```
Tyr Met Arg Gln Asn Pro Gln Arg Leu Ala Glu Val Leu Gly Gln Asn
    770                 775                 780

Pro Ser Tyr Ile Phe Phe Arg Glu Leu Ala Gly Ser Ser Asn Asp Gly
785                 790                 795                 800

Pro Val Gly Ala Leu Gly Thr Pro Leu Met Gly Glu Tyr Ala Gly Ala
                805                 810                 815

Val Asp Arg His Tyr Ile Thr Leu Gly Ala Pro Leu Phe Val Ala Thr
            820                 825                 830

Ala His Pro Val Thr Arg Lys Ala Leu Asn Arg Leu Ile Met Ala Gln
        835                 840                 845

Asp Thr Gly Ser Ala Ile Lys Gly Ala Val Arg Val Asp Tyr Phe Trp
    850                 855                 860

Gly Tyr Gly Asp Glu Ala Gly Glu Leu Ala Gly Lys Gln Lys Thr Thr
865                 870                 875                 880

Gly Tyr Val Trp Gln Leu Leu Pro Asn Gly Met Lys Pro Glu Tyr Arg
                885                 890                 895

Pro
```

<210> 11
<211> 1941
<212> DNA
<213> Artificial Sequence

<220>
<223> deltaG287NZ-953

<400> 11

```
atggctagcc ccgatgtcaa gtcggcggac acgctgtcaa aacctgccgc ccctgttgtt   60
tctgaaaaag agacagaggc aaaggaagat gcgccacagg caggttctca aggacagggc  120
gcgccatccg cacaaggcgg tcaagatatg gcggcggttt cggaagaaaa tacaggcaat  180
ggcggtgcgg cagcaacgga caaacccaaa aatgaagacg agggggcgca aaatgatatg  240
ccgcaaaatg ccgccgatac agatagtttg acaccgaatc acaccccggc ttcgaatatg  300
ccggccggaa atatggaaaa ccaagcaccg gatgccgggg aatcggagca gccggcaaac  360
caaccggata tggcaaatac ggcggacgga atgcagggtg acgatccgtc ggcaggcggg  420
gaaaatgccg gcaatacggc tgcccaaggt acaaatcaag ccgaaaacaa tcaaaccgcc  480
ggttctcaaa atcctgcctc ttcaaccaat cctagcgcca cgaatagcgg tggtgatttt  540
ggaaggacga acgtgggcaa ttctgttgtg attgacgggc cgtcgcaaaa tataacgttg  600
acccactgta aaggcgattc ttgtagtggc aataatttct tggatgaaga agtacagcta  660
aaatcagaat ttgaaaaatt aagtgatgca gacaaaataa gtaattacaa gaaagatggg  720
aagaatgacg ggaagaatga taaatttgtc ggtttggttg ccgatagtgt gcagatgaag  780
ggaatcaatc aatatattat cttttataaa cctaaaccca cttcatttgc gcgatttagg  840
cgttctgcac ggtcgaggcg gtcgcttccg gccgagatgc cgctgattcc cgtcaatcag  900
gcggatacgc tgattgtcga tggggaagcg gtcagcctga cggggcattc cggcaatatc  960
ttcgcgcccg aagggaatta ccggtatctg acttacgggg cggaaaaatt gcccggcgga 1020
tcgtatgccc tccgtgttca aggcgaacct tcaaaaggcg aaatgctcgc gggcacggca 1080
gtgtacaacg gcgaagtgct gcattttcat acggaaaacg gccgtccgtc cccgtccaga 1140
ggcaggtttg ccgcaaaagt cgatttcggc agcaaatctg tggacggcat tatcgacagc 1200
ggcgatggtt tgcatatggg tacgcaaaaa ttcaaagccg ccatcgatgg aaacggcttt 1260
aaggggactt ggacggaaaa tggcggcggg gatgtttccg gaaagtttta cggcccggcc 1320
ggcgaggaag tggcgggaaa atacagctat cgcccaacag atgcggaaaa gggcggattc 1380
ggcgtgtttg ccggcaaaaa agagcaggat ggatccggag gaggaggagc cacctacaaa 1440
gtggacgaat atcacgccaa cgcccgtttc gccatcgacc atttcaacac cagcaccaac 1500

gtcggcggtt tttacggtct gaccggttcc gtcgagttcg accaagcaaa acgcgacggt 1560
aaaatcgaca tcaccatccc cgttgccaac ctgcaaagcg gttcgcaaca ctttaccgac 1620
cacctgaaat cagccgacat cttcgatgcc gcccaatatc cggacatccg ctttgtttcc 1680
accaaattca acttcaacgg caaaaaactg gtttccgttg acggcaacct gaccatgcac 1740
ggcaaaaccg cccccgtcaa actcaaagcc gaaaaattca actgctacca aagcccgatg 1800
gcgaaaaccg aagtttgcgg cggcgacttc agcaccacca tcgaccgcac caaatggggc 1860
gtggactacc tcgttaacgt tggtatgacc aaaagcgtcc gcatcgacat ccaaatcgag 1920
gcagccaaac aataaaagct t                                           1941
```

<210> 12
<211> 644
<212> PRT
<213> Artificial Sequence

<220>
<223> deltaG287NZ-953

<400> 12

Met Ala Ser Pro Asp Val Lys Ser Ala Asp Thr Leu Ser Lys Pro Ala
1 5 10 15

Ala Pro Val Val Ser Glu Lys Glu Thr Glu Ala Lys Glu Asp Ala Pro
20 25 30

Gln Ala Gly Ser Gln Gly Gln Gly Ala Pro Ser Ala Gln Gly Gly Gln
35 40 45

Asp Met Ala Ala Val Ser Glu Glu Asn Thr Gly Asn Gly Gly Ala Ala
50 55 60

Ala Thr Asp Lys Pro Lys Asn Glu Asp Glu Gly Ala Gln Asn Asp Met
65 70 75 80

Pro Gln Asn Ala Ala Asp Thr Asp Ser Leu Thr Pro Asn His Thr Pro
85 90 95

Ala Ser Asn Met Pro Ala Gly Asn Met Glu Asn Gln Ala Pro Asp Ala
100 105 110

Gly Glu Ser Glu Gln Pro Ala Asn Gln Pro Asp Met Ala Asn Thr Ala
115 120 125

Asp Gly Met Gln Gly Asp Asp Pro Ser Ala Gly Gly Glu Asn Ala Gly
130 135 140

Asn Thr Ala Ala Gln Gly Thr Asn Gln Ala Glu Asn Asn Gln Thr Ala
145 150 155 160

Gly Ser Gln Asn Pro Ala Ser Ser Thr Asn Pro Ser Ala Thr Asn Ser
165 170 175

Gly Gly Asp Phe Gly Arg Thr Asn Val Gly Asn Ser Val Val Ile Asp
180 185 190

Gly Pro Ser Gln Asn Ile Thr Leu Thr His Cys Lys Gly Asp Ser Cys
195 200 205

Ser Gly Asn Asn Phe Leu Asp Glu Glu Val Gln Leu Lys Ser Glu Phe
210 215 220

Glu Lys Leu Ser Asp Ala Asp Lys Ile Ser Asn Tyr Lys Lys Asp Gly

225 230 235 240

Lys Asn Asp Gly Lys Asn Asp Lys Phe Val Gly Leu Val Ala Asp Ser
245 250 255

Val Gln Met Lys Gly Ile Asn Gln Tyr Ile Ile Phe Tyr Lys Pro Lys
260 265 270

Pro Thr Ser Phe Ala Arg Phe Arg Arg Ser Ala Arg Ser Arg Arg Ser
275 280 285

Leu Pro Ala Glu Met Pro Leu Ile Pro Val Asn Gln Ala Asp Thr Leu
290 295 300

Ile Val Asp Gly Glu Ala Val Ser Leu Thr Gly His Ser Gly Asn Ile
305 310 315 320

Phe Ala Pro Glu Gly Asn Tyr Arg Tyr Leu Thr Tyr Gly Ala Glu Lys
325 330 335

Leu Pro Gly Gly Ser Tyr Ala Leu Arg Val Gln Gly Glu Pro Ser Lys
340 345 350

Gly Glu Met Leu Ala Gly Thr Ala Val Tyr Asn Gly Glu Val Leu His
355 360 365

Phe His Thr Glu Asn Gly Arg Pro Ser Pro Ser Arg Gly Arg Phe Ala
370 375 380

Ala Lys Val Asp Phe Gly Ser Lys Ser Val Asp Gly Ile Ile Asp Ser
385 390 395 400

Gly Asp Gly Leu His Met Gly Thr Gln Lys Phe Lys Ala Ala Ile Asp
405 410 415

Gly Asn Gly Phe Lys Gly Thr Trp Thr Glu Asn Gly Gly Gly Asp Val
420 425 430

Ser Gly Lys Phe Tyr Gly Pro Ala Gly Glu Glu Val Ala Gly Lys Tyr
435 440 445

Ser Tyr Arg Pro Thr Asp Ala Glu Lys Gly Gly Phe Gly Val Phe Ala
450 455 460

Gly Lys Lys Glu Gln Asp Gly Ser Gly Gly Gly Gly Ala Thr Tyr Lys
465 470 475 480

Val Asp Glu Tyr His Ala Asn Ala Arg Phe Ala Ile Asp His Phe Asn
485 490 495

Thr Ser Thr Asn Val Gly Gly Phe Tyr Gly Leu Thr Gly Ser Val Glu
500 505 510

Phe Asp Gln Ala Lys Arg Asp Gly Lys Ile Asp Ile Thr Ile Pro Val
515 520 525

Ala Asn Leu Gln Ser Gly Ser Gln His Phe Thr Asp His Leu Lys Ser
530 535 540

Ala Asp Ile Phe Asp Ala Ala Gln Tyr Pro Asp Ile Arg Phe Val Ser
545 550 555 560

```
Thr Lys Phe Asn Phe Asn Gly Lys Lys Leu Val Ser Val Asp Gly Asn
                565                 570                 575

Leu Thr Met His Gly Lys Thr Ala Pro Val Lys Leu Lys Ala Glu Lys
            580                 585                 590

Phe Asn Cys Tyr Gln Ser Pro Met Ala Lys Thr Glu Val Cys Gly Gly
        595                 600                 605

Asp Phe Ser Thr Thr Ile Asp Arg Thr Lys Trp Gly Val Asp Tyr Leu
    610                 615                 620

Val Asn Val Gly Met Thr Lys Ser Val Arg Ile Asp Ile Gln Ile Glu
625                 630                 635                 640

Ala Ala Lys Gln
```

<210> 13
<211> 2583
<212> DNA
<213> Artificial Sequence

<220>
<223> deltaG287NZ-961

<400> 13

```
atggctagcc ccgatgtcaa gtcggcggac acgctgtcaa aacctgccgc ccctgttgtt   60
tctgaaaaag agacagaggc aaaggaagat gcgccacagg caggttctca aggacagggc   120
gcgccatccg cacaaggcgg tcaagatatg gcggcggttt cggaagaaaa tacaggcaat   180
ggcggtgcgg cagcaacgga caaacccaaa aatgaagacg agggggcgca aaatgatatg   240
ccgcaaaatg ccgccgatac agatagtttg acaccgaatc acaccccggc ttcgaatatg   300
ccggccggaa atatggaaaa ccaagcaccg gatgccgggg aatcggagca gccggcaaac   360
caaccggata tggcaaatac ggcggacgga atgcagggtg acgatccgtc ggcaggcggg   420
gaaaatgccg gcaatacggc tgcccaaggt acaaatcaag ccgaaaacaa tcaaaccgcc   480
ggttctcaaa atcctgcctc ttcaaccaat cctagcgcca cgaatagcgg tggtgatttt   540
ggaaggacga acgtgggcaa ttctgttgtg attgacgggc cgtcgcaaaa tataacgttg   600
acccactgta aaggcgattc ttgtagtggc aataatttct tggatgaaga agtacagcta   660
aaatcagaat ttgaaaaatt aagtgatgca gacaaaataa gtaattacaa gaaagatggg   720
aagaatgacg ggaagaatga taaatttgtc ggtttggttg ccgatagtgt gcagatgaag   780
ggaatcaatc aatatattat cttttataaa cctaaaccca cttcatttgc gcgatttagg   840
cgttctgcac ggtcgaggcg gtcgcttccg gccgagatgc cgctgattcc cgtcaatcag   900
gcggatacgc tgattgtcga tggggaagcg gtcagcctga cggggcattc cggcaatatc   960
ttcgcgcccg aagggaatta ccggtatctg acttacgggg cggaaaaatt gcccggcgga   1020
tcgtatgccc tccgtgttca aggcgaacct tcaaaaggcg aaatgctcgc gggcacggca   1080
gtgtacaacg gcgaagtgct gcattttcat acggaaaacg gccgtccgtc cccgtccaga   1140
ggcaggtttg ccgcaaaagt cgatttcggc agcaaatctg tggacggcat tatcgacagc   1200
ggcgatggtt tgcatatggg tacgcaaaaa ttcaaagccg ccatcgatgg aaacggcttt   1260
aaggggactt ggacggaaaa tggcggcggg gatgtttccg gaaagtttta cggcccggcc   1320
ggcgaggaag tggcgggaaa atacagctat cgcccaacag atgcggaaaa gggcggattc   1380
ggcgtgtttg ccggcaaaaa agagcaggat ggatccggag gaggaggagc cacaaacgac   1440
gacgatgtta aaaaagctgc cactgtggcc attgctgctg cctacaacaa tggccaagaa   1500
atcaacggtt tcaaagctgg agagaccatc tacgacattg atgaagacgg cacaattacc   1560
aaaaaagacg caactgcagc cgatgttgaa gccgacgact ttaaaggtct gggtctgaaa   1620
aaagtcgtga ctaacctgac caaaaccgtc aatgaaaaca aacaaaacgt cgatgccaaa   1680
gtaaaagctg cagaatctga aatagaaaag ttaacaacca agttagcaga cactgatgcc   1740
gctttagcag atactgatgc cgctctggat gcaaccacca acgccttgaa taaattggga   1800
gaaaatataa cgacatttgc tgaagagact aagacaaata tcgtaaaaat tgatgaaaaa   1860
ttagaagccg tggctgatac cgtcgacaag catgccgaag cattcaacga tatcgccgat   1920
tcattggatg aaaccaacac taaggcagac gaagccgtca aaaccgccaa tgaagccaaa   1980

cagacggccg aagaaaccaa acaaaacgtc gatgccaaag taaaagctgc agaaactgca   2040
gcaggcaaag ccgaagctgc cgctggcaca gctaatactg cagccgacaa ggccgaagct   2100
gtcgctgcaa aagttaccga catcaaagct gatatcgcta cgaacaaaga taatattgct   2160
aaaaaagcaa acagtgccga cgtgtacacc agagaagagt ctgacagcaa atttgtcaga   2220
attgatggtc tgaacgctac taccgaaaaa ttggacacac gcttggcttc tgctgaaaaa   2280
tccattgccg atcacgatac tcgcctgaac ggtttggata aaacagtgtc agacctgcgc   2340
aaagaaaccc gccaaggcct tgcagaacaa gccgcgctct ccggtctgtt ccaaccttac   2400
aacgtgggtc ggttcaatgt aacggctgca gtcggcggct acaaatccga atcggcagtc   2460
gccatcggta ccggcttccg ctttaccgaa aactttgccg ccaaagcagg cgtggcagtc   2520
ggcacttcgt ccggttcttc cgcagcctac catgtcggcg tcaattacga gtggtaaaag   2580
ctt                                                                 2583
```

<210> 14
<211> 858
<212> PRT
<213> Artificial Sequence

<220>
<223> deltaG287NZ-961

<400> 14

```
Met Ala Ser Pro Asp Val Lys Ser Ala Asp Thr Leu Ser Lys Pro Ala
1               5                   10                  15

Ala Pro Val Val Ser Glu Lys Glu Thr Glu Ala Lys Glu Asp Ala Pro
            20                  25                  30

Gln Ala Gly Ser Gln Gly Gln Gly Ala Pro Ser Ala Gln Gly Gly Gln
        35                  40                  45

Asp Met Ala Ala Val Ser Glu Glu Asn Thr Gly Asn Gly Gly Ala Ala
    50                  55                  60

Ala Thr Asp Lys Pro Lys Asn Glu Asp Glu Gly Ala Gln Asn Asp Met
65                  70                  75                  80

Pro Gln Asn Ala Ala Asp Thr Asp Ser Leu Thr Pro Asn His Thr Pro
                85                  90                  95

Ala Ser Asn Met Pro Ala Gly Asn Met Glu Asn Gln Ala Pro Asp Ala
            100                 105                 110

Gly Glu Ser Glu Gln Pro Ala Asn Gln Pro Asp Met Ala Asn Thr Ala
        115                 120                 125

Asp Gly Met Gln Gly Asp Asp Pro Ser Ala Gly Gly Glu Asn Ala Gly
    130                 135                 140

Asn Thr Ala Ala Gln Gly Thr Asn Gln Ala Glu Asn Asn Gln Thr Ala
145                 150                 155                 160

Gly Ser Gln Asn Pro Ala Ser Ser Thr Asn Pro Ser Ala Thr Asn Ser
                165                 170                 175

Gly Gly Asp Phe Gly Arg Thr Asn Val Gly Asn Ser Val Val Ile Asp
            180                 185                 190

Gly Pro Ser Gln Asn Ile Thr Leu Thr His Cys Lys Gly Asp Ser Cys
        195                 200                 205

Ser Gly Asn Asn Phe Leu Asp Glu Glu Val Gln Leu Lys Ser Glu Phe
```

210 215 220

Glu Lys Leu Ser Asp Ala Asp Lys Ile Ser Asn Tyr Lys Lys Asp Gly
225 230 235 240

Lys Asn Asp Gly Lys Asn Asp Lys Phe Val Gly Leu Val Ala Asp Ser
245 250 255

Val Gln Met Lys Gly Ile Asn Gln Tyr Ile Ile Phe Tyr Lys Pro Lys
260 265 270

Pro Thr Ser Phe Ala Arg Phe Arg Arg Ser Ala Arg Ser Arg Arg Ser
275 280 285

Leu Pro Ala Glu Met Pro Leu Ile Pro Val Asn Gln Ala Asp Thr Leu
290 295 300

Ile Val Asp Gly Glu Ala Val Ser Leu Thr Gly His Ser Gly Asn Ile
305 310 315 320

Phe Ala Pro Glu Gly Asn Tyr Arg Tyr Leu Thr Tyr Gly Ala Glu Lys
325 330 335

Leu Pro Gly Gly Ser Tyr Ala Leu Arg Val Gln Gly Glu Pro Ser Lys
340 345 350

Gly Glu Met Leu Ala Gly Thr Ala Val Tyr Asn Gly Glu Val Leu His
355 360 365

Phe His Thr Glu Asn Gly Arg Pro Ser Pro Ser Arg Gly Arg Phe Ala
370 375 380

Ala Lys Val Asp Phe Gly Ser Lys Ser Val Asp Gly Ile Ile Asp Ser
385 390 395 400

Gly Asp Gly Leu His Met Gly Thr Gln Lys Phe Lys Ala Ala Ile Asp
405 410 415

Gly Asn Gly Phe Lys Gly Thr Trp Thr Glu Asn Gly Gly Gly Asp Val
420 425 430

Ser Gly Lys Phe Tyr Gly Pro Ala Gly Glu Glu Val Ala Gly Lys Tyr
435 440 445

Ser Tyr Arg Pro Thr Asp Ala Glu Lys Gly Gly Phe Gly Val Phe Ala
450 455 460

Gly Lys Lys Glu Gln Asp Gly Ser Gly Gly Gly Gly Ala Thr Asn Asp
465 470 475 480

Asp Asp Val Lys Lys Ala Ala Thr Val Ala Ile Ala Ala Ala Tyr Asn
485 490 495

Asn Gly Gln Glu Ile Asn Gly Phe Lys Ala Gly Glu Thr Ile Tyr Asp
500 505 510

Ile Asp Glu Asp Gly Thr Ile Thr Lys Lys Asp Ala Thr Ala Ala Asp
515 520 525

Val Glu Ala Asp Asp Phe Lys Gly Leu Gly Leu Lys Lys Val Val Thr
530 535 540

Asn Leu Thr Lys Thr Val Asn Glu Asn Lys Gln Asn Val Asp Ala Lys
545 550 555 560

Val Lys Ala Ala Glu Ser Glu Ile Glu Lys Leu Thr Thr Lys Leu Ala
565 570 575

Asp Thr Asp Ala Ala Leu Ala Asp Thr Asp Ala Ala Leu Asp Ala Thr
580 585 590

Thr Asn Ala Leu Asn Lys Leu Gly Glu Asn Ile Thr Thr Phe Ala Glu
595 600 605

Glu Thr Lys Thr Asn Ile Val Lys Ile Asp Glu Lys Leu Glu Ala Val
610 615 620

Ala Asp Thr Val Asp Lys His Ala Glu Ala Phe Asn Asp Ile Ala Asp
625 630 635 640

Ser Leu Asp Glu Thr Asn Thr Lys Ala Asp Glu Ala Val Lys Thr Ala
645 650 655

Asn Glu Ala Lys Gln Thr Ala Glu Glu Thr Lys Gln Asn Val Asp Ala
660 665 670

Lys Val Lys Ala Ala Glu Thr Ala Ala Gly Lys Ala Glu Ala Ala Ala
675 680 685

Gly Thr Ala Asn Thr Ala Ala Asp Lys Ala Glu Ala Val Ala Ala Lys
690 695 700

Val Thr Asp Ile Lys Ala Asp Ile Ala Thr Asn Lys Asp Asn Ile Ala
705 710 715 720

Lys Lys Ala Asn Ser Ala Asp Val Tyr Thr Arg Glu Glu Ser Asp Ser
725 730 735

Lys Phe Val Arg Ile Asp Gly Leu Asn Ala Thr Thr Glu Lys Leu Asp
740 745 750

Thr Arg Leu Ala Ser Ala Glu Lys Ser Ile Ala Asp His Asp Thr Arg
755 760 765

Leu Asn Gly Leu Asp Lys Thr Val Ser Asp Leu Arg Lys Glu Thr Arg
770 775 780

Gln Gly Leu Ala Glu Gln Ala Ala Leu Ser Gly Leu Phe Gln Pro Tyr
785 790 795 800

Asn Val Gly Arg Phe Asn Val Thr Ala Ala Val Gly Gly Tyr Lys Ser
805 810 815

Glu Ser Ala Val Ala Ile Gly Thr Gly Phe Arg Phe Thr Glu Asn Phe
820 825 830

Ala Ala Lys Ala Gly Val Ala Val Gly Thr Ser Ser Gly Ser Ser Ala
835 840 845

Ala Tyr His Val Gly Val Asn Tyr Glu Trp
850 855

<210> 15
<211> 1082
<212> PRT
<213> Artificial Sequence

<220>
<223> 983

<400> 15

```
Met Arg Thr Thr Pro Thr Phe Pro Thr Lys Thr Phe Lys Pro Thr Ala
1               5                   10                  15

Met Ala Leu Ala Val Ala Thr Thr Leu Ser Ala Cys Leu Gly Gly Gly
            20                  25                  30

Gly Gly Gly Thr Ser Ala Pro Asp Phe Asn Ala Gly Gly Thr Gly Ile
        35                  40                  45

Gly Ser Asn Ser Arg Ala Thr Thr Ala Lys Ser Ala Ala Val Ser Tyr
    50                  55                  60

Ala Gly Ile Lys Asn Glu Met Cys Lys Asp Arg Ser Met Leu Cys Ala
65                  70                  75                  80

Gly Arg Asp Asp Val Ala Val Thr Asp Arg Asp Ala Lys Ile Asn Ala
                85                  90                  95

Pro Pro Pro Asn Leu His Thr Gly Asp Phe Pro Asn Pro Asn Asp Ala
            100                 105                 110

Tyr Lys Asn Leu Ile Asn Leu Lys Pro Ala Ile Glu Ala Gly Tyr Thr
        115                 120                 125

Gly Arg Gly Val Glu Val Gly Ile Val Asp Thr Gly Glu Ser Val Gly
    130                 135                 140

Ser Ile Ser Phe Pro Glu Leu Tyr Gly Arg Lys Glu His Gly Tyr Asn
145                 150                 155                 160

Glu Asn Tyr Lys Asn Tyr Thr Ala Tyr Met Arg Lys Glu Ala Pro Glu
                165                 170                 175

Asp Gly Gly Gly Lys Asp Ile Glu Ala Ser Phe Asp Asp Glu Ala Val
            180                 185                 190

Ile Glu Thr Glu Ala Lys Pro Thr Asp Ile Arg His Val Lys Glu Ile
        195                 200                 205

Gly His Ile Asp Leu Val Ser His Ile Ile Gly Gly Arg Ser Val Asp
    210                 215                 220

Gly Arg Pro Ala Gly Gly Ile Ala Pro Asp Ala Thr Leu His Ile Met
225                 230                 235                 240

Asn Thr Asn Asp Glu Thr Lys Asn Glu Met Met Val Ala Ala Ile Arg
                245                 250                 255

Asn Ala Trp Val Lys Leu Gly Glu Arg Gly Val Arg Ile Val Asn Asn
            260                 265                 270

Ser Phe Gly Thr Thr Ser Arg Ala Gly Thr Ala Asp Leu Phe Gln Ile
```

```
            275                 280                 285

Ala Asn Ser Glu Glu Gln Tyr Arg Gln Ala Leu Leu Asp Tyr Ser Gly
    290                 295                 300

Gly Asp Lys Thr Asp Glu Gly Ile Arg Leu Met Gln Gln Ser Asp Tyr
305                 310                 315                 320

Gly Asn Leu Ser Tyr His Ile Arg Asn Lys Asn Met Leu Phe Ile Phe
                325                 330                 335

Ser Thr Gly Asn Asp Ala Gln Ala Gln Pro Asn Thr Tyr Ala Leu Leu
            340                 345                 350

Pro Phe Tyr Glu Lys Asp Ala Gln Lys Gly Ile Ile Thr Val Ala Gly
        355                 360                 365

Val Asp Arg Ser Gly Glu Lys Phe Lys Arg Glu Met Tyr Gly Glu Pro
    370                 375                 380

Gly Thr Glu Pro Leu Glu Tyr Gly Ser Asn His Cys Gly Ile Thr Ala
385                 390                 395                 400

Met Trp Cys Leu Ser Ala Pro Tyr Glu Ala Ser Val Arg Phe Thr Arg
                405                 410                 415

Thr Asn Pro Ile Gln Ile Ala Gly Thr Ser Phe Ser Ala Pro Ile Val
            420                 425                 430

Thr Gly Thr Ala Ala Leu Leu Leu Gln Lys Tyr Pro Trp Met Ser Asn
        435                 440                 445

Asp Asn Leu Arg Thr Thr Leu Leu Thr Thr Ala Gln Asp Ile Gly Ala
    450                 455                 460

Val Gly Val Asp Ser Lys Phe Gly Trp Gly Leu Leu Asp Ala Gly Lys
465                 470                 475                 480

Ala Met Asn Gly Pro Ala Ser Phe Pro Phe Gly Asp Phe Thr Ala Asp
                485                 490                 495

Thr Lys Gly Thr Ser Asp Ile Ala Tyr Ser Phe Arg Asn Asp Ile Ser
            500                 505                 510

Gly Thr Gly Gly Leu Ile Lys Lys Gly Gly Ser Gln Leu Gln Leu His
        515                 520                 525

Gly Asn Asn Thr Tyr Thr Gly Lys Thr Ile Ile Glu Gly Gly Ser Leu
    530                 535                 540

Val Leu Tyr Gly Asn Asn Lys Ser Asp Met Arg Val Glu Thr Lys Gly
545                 550                 555                 560

Ala Leu Ile Tyr Asn Gly Ala Ala Ser Gly Gly Ser Leu Asn Ser Asp
                565                 570                 575

Gly Ile Val Tyr Leu Ala Asp Thr Asp Gln Ser Gly Ala Asn Glu Thr
            580                 585                 590

Val His Ile Lys Gly Ser Leu Gln Leu Asp Gly Lys Gly Thr Leu Tyr
        595                 600                 605
```

Thr Arg Leu Gly Lys Leu Leu Lys Val Asp Gly Thr Ala Ile Ile Gly
610 615 620

Gly Lys Leu Tyr Met Ser Ala Arg Gly Lys Gly Ala Gly Tyr Leu Asn
625 630 635 640

Ser Thr Gly Arg Arg Val Pro Phe Leu Ser Ala Ala Lys Ile Gly Gln
645 650 655

Asp Tyr Ser Phe Phe Thr Asn Ile Glu Thr Asp Gly Gly Leu Leu Ala
660 665 670

Ser Leu Asp Ser Val Glu Lys Thr Ala Gly Ser Glu Gly Asp Thr Leu
675 680 685

Ser Tyr Tyr Val Arg Arg Gly Asn Ala Ala Arg Thr Ala Ser Ala Ala
690 695 700

Ala His Ser Ala Pro Ala Gly Leu Lys His Ala Val Glu Gln Gly Gly
705 710 715 720

Ser Asn Leu Glu Asn Leu Met Val Glu Leu Asp Ala Ser Glu Ser Ser
725 730 735

Ala Thr Pro Glu Thr Val Glu Thr Ala Ala Ala Asp Arg Thr Asp Met
740 745 750

Pro Gly Ile Arg Pro Tyr Gly Ala Thr Phe Arg Ala Ala Ala Ala Val
755 760 765

Gln His Ala Asn Ala Ala Asp Gly Val Arg Ile Phe Asn Ser Leu Ala
770 775 780

Ala Thr Val Tyr Ala Asp Ser Thr Ala Ala His Ala Asp Met Gln Gly
785 790 795 800

Arg Arg Leu Lys Ala Val Ser Asp Gly Leu Asp His Asn Gly Thr Gly
805 810 815

Leu Arg Val Ile Ala Gln Thr Gln Gln Asp Gly Gly Thr Trp Glu Gln
820 825 830

Gly Gly Val Glu Gly Lys Met Arg Gly Ser Thr Gln Thr Val Gly Ile
835 840 845

Ala Ala Lys Thr Gly Glu Asn Thr Thr Ala Ala Ala Thr Leu Gly Met
850 855 860

Gly Arg Ser Thr Trp Ser Glu Asn Ser Ala Asn Ala Lys Thr Asp Ser
865 870 875 880

Ile Ser Leu Phe Ala Gly Ile Arg His Asp Ala Gly Asp Ile Gly Tyr
885 890 895

Leu Lys Gly Leu Phe Ser Tyr Gly Arg Tyr Lys Asn Ser Ile Ser Arg
900 905 910

Ser Thr Gly Ala Asp Glu His Ala Glu Gly Ser Val Asn Gly Thr Leu
915 920 925

```
Met Gln Leu Gly Ala Leu Gly Gly Val Asn Val Pro Phe Ala Ala Thr
    930                 935                 940

Gly Asp Leu Thr Val Glu Gly Gly Leu Arg Tyr Asp Leu Leu Lys Gln
945                 950                 955                 960

Asp Ala Phe Ala Glu Lys Gly Ser Ala Leu Gly Trp Ser Gly Asn Ser
                965                 970                 975

Leu Thr Glu Gly Thr Leu Val Gly Leu Ala Gly Leu Lys Leu Ser Gln
            980                 985                 990

Pro Leu Ser Asp Lys Ala Val Leu Phe Ala Thr Ala Gly Val Glu Arg
        995                 1000                1005

Asp Leu Asn Gly Arg Asp Tyr Thr Val Thr Gly Gly Phe Thr Gly Ala
    1010                1015                1020

Thr Ala Ala Thr Gly Lys Thr Gly Ala Arg Asn Met Pro His Thr Arg
1025                1030                1035                1040

Leu Val Ala Gly Leu Gly Ala Asp Val Glu Phe Gly Asn Gly Trp Asn
                1045                1050                1055

Gly Leu Ala Arg Tyr Ser Tyr Ala Gly Ser Lys Gln Tyr Gly Asn His
            1060                1065                1070

Ser Gly Arg Val Gly Val Gly Tyr Arg Phe
        1075                1080
```

<210> 16
<211> 1047
<212> PRT
<213> Artificial Sequence

<220>
<223> deltaG983

<400> 16

Thr Ser Ala Pro Asp Phe Asn Ala Gly Gly Thr Gly Ile Gly Ser Asn
1 5 10 15

Ser Arg Ala Thr Thr Ala Lys Ser Ala Ala Val Ser Tyr Ala Gly Ile
20 25 30

Lys Asn Glu Met Cys Lys Asp Arg Ser Met Leu Cys Ala Gly Arg Asp
35 40 45

Asp Val Ala Val Thr Asp Arg Asp Ala Lys Ile Asn Ala Pro Pro Pro
50 55 60

Asn Leu His Thr Gly Asp Phe Pro Asn Pro Asn Asp Ala Tyr Lys Asn
65 70 75 80

Leu Ile Asn Leu Lys Pro Ala Ile Glu Ala Gly Tyr Thr Gly Arg Gly
85 90 95

Val Glu Val Gly Ile Val Asp Thr Gly Glu Ser Val Gly Ser Ile Ser
100 105 110

Phe Pro Glu Leu Tyr Gly Arg Lys Glu His Gly Tyr Asn Glu Asn Tyr

115 120 125

Lys Asn Tyr Thr Ala Tyr Met Arg Lys Glu Ala Pro Glu Asp Gly Gly
130 135 140

Gly Lys Asp Ile Glu Ala Ser Phe Asp Asp Glu Ala Val Ile Glu Thr
145 150 155 160

Glu Ala Lys Pro Thr Asp Ile Arg His Val Lys Glu Ile Gly His Ile
165 170 175

Asp Leu Val Ser His Ile Ile Gly Gly Arg Ser Val Asp Gly Arg Pro
180 185 190

Ala Gly Gly Ile Ala Pro Asp Ala Thr Leu His Ile Met Asn Thr Asn
195 200 205

Asp Glu Thr Lys Asn Glu Met Met Val Ala Ala Ile Arg Asn Ala Trp
210 215 220

Val Lys Leu Gly Glu Arg Gly Val Arg Ile Val Asn Asn Ser Phe Gly
225 230 235 240

Thr Thr Ser Arg Ala Gly Thr Ala Asp Leu Phe Gln Ile Ala Asn Ser
245 250 255

Glu Glu Gln Tyr Arg Gln Ala Leu Leu Asp Tyr Ser Gly Gly Asp Lys
260 265 270

Thr Asp Glu Gly Ile Arg Leu Met Gln Gln Ser Asp Tyr Gly Asn Leu
275 280 285

Ser Tyr His Ile Arg Asn Lys Asn Met Leu Phe Ile Phe Ser Thr Gly
290 295 300

Asn Asp Ala Gln Ala Gln Pro Asn Thr Tyr Ala Leu Leu Pro Phe Tyr
305 310 315 320

Glu Lys Asp Ala Gln Lys Gly Ile Ile Thr Val Ala Gly Val Asp Arg
325 330 335

Ser Gly Glu Lys Phe Lys Arg Glu Met Tyr Gly Glu Pro Gly Thr Glu
340 345 350

Pro Leu Glu Tyr Gly Ser Asn His Cys Gly Ile Thr Ala Met Trp Cys
355 360 365

Leu Ser Ala Pro Tyr Glu Ala Ser Val Arg Phe Thr Arg Thr Asn Pro
370 375 380

Ile Gln Ile Ala Gly Thr Ser Phe Ser Ala Pro Ile Val Thr Gly Thr
385 390 395 400

Ala Ala Leu Leu Leu Gln Lys Tyr Pro Trp Met Ser Asn Asp Asn Leu
405 410 415

Arg Thr Thr Leu Leu Thr Thr Ala Gln Asp Ile Gly Ala Val Gly Val
420 425 430

Asp Ser Lys Phe Gly Trp Gly Leu Leu Asp Ala Gly Lys Ala Met Asn
435 440 445

Gly Pro Ala Ser Phe Pro Phe Gly Asp Phe Thr Ala Asp Thr Lys Gly
    450                 455                 460

Thr Ser Asp Ile Ala Tyr Ser Phe Arg Asn Asp Ile Ser Gly Thr Gly
465                 470                 475                 480

Gly Leu Ile Lys Lys Gly Gly Ser Gln Leu Gln Leu His Gly Asn Asn
                485                 490                 495

Thr Tyr Thr Gly Lys Thr Ile Ile Glu Gly Gly Ser Leu Val Leu Tyr
            500                 505                 510

Gly Asn Asn Lys Ser Asp Met Arg Val Glu Thr Lys Gly Ala Leu Ile
        515                 520                 525

Tyr Asn Gly Ala Ala Ser Gly Gly Ser Leu Asn Ser Asp Gly Ile Val
    530                 535                 540

Tyr Leu Ala Asp Thr Asp Gln Ser Gly Ala Asn Glu Thr Val His Ile
545                 550                 555                 560

Lys Gly Ser Leu Gln Leu Asp Gly Lys Gly Thr Leu Tyr Thr Arg Leu
                565                 570                 575

Gly Lys Leu Leu Lys Val Asp Gly Thr Ala Ile Ile Gly Gly Lys Leu
            580                 585                 590

Tyr Met Ser Ala Arg Gly Lys Gly Ala Gly Tyr Leu Asn Ser Thr Gly
        595                 600                 605

Arg Arg Val Pro Phe Leu Ser Ala Ala Lys Ile Gly Gln Asp Tyr Ser
    610                 615                 620

Phe Phe Thr Asn Ile Glu Thr Asp Gly Gly Leu Leu Ala Ser Leu Asp
625                 630                 635                 640

Ser Val Glu Lys Thr Ala Gly Ser Glu Gly Asp Thr Leu Ser Tyr Tyr
                645                 650                 655

Val Arg Arg Gly Asn Ala Ala Arg Thr Ala Ser Ala Ala Ala His Ser
            660                 665                 670

Ala Pro Ala Gly Leu Lys His Ala Val Glu Gln Gly Gly Ser Asn Leu
        675                 680                 685

Glu Asn Leu Met Val Glu Leu Asp Ala Ser Glu Ser Ser Ala Thr Pro
    690                 695                 700

Glu Thr Val Glu Thr Ala Ala Ala Asp Arg Thr Asp Met Pro Gly Ile
705                 710                 715                 720

Arg Pro Tyr Gly Ala Thr Phe Arg Ala Ala Ala Ala Val Gln His Ala
                725                 730                 735

Asn Ala Ala Asp Gly Val Arg Ile Phe Asn Ser Leu Ala Ala Thr Val
            740                 745                 750

Tyr Ala Asp Ser Thr Ala Ala His Ala Asp Met Gln Gly Arg Arg Leu
        755                 760                 765

```
Lys Ala Val Ser Asp Gly Leu Asp His Asn Gly Thr Gly Leu Arg Val
    770                 775                 780

Ile Ala Gln Thr Gln Gln Asp Gly Gly Thr Trp Glu Gln Gly Gly Val
785                 790                 795                 800

Glu Gly Lys Met Arg Gly Ser Thr Gln Thr Val Gly Ile Ala Ala Lys
                805                 810                 815

Thr Gly Glu Asn Thr Thr Ala Ala Ala Thr Leu Gly Met Gly Arg Ser
            820                 825                 830

Thr Trp Ser Glu Asn Ser Ala Asn Ala Lys Thr Asp Ser Ile Ser Leu
        835                 840                 845

Phe Ala Gly Ile Arg His Asp Ala Gly Asp Ile Gly Tyr Leu Lys Gly
    850                 855                 860

Leu Phe Ser Tyr Gly Arg Tyr Lys Asn Ser Ile Ser Arg Ser Thr Gly
865                 870                 875                 880

Ala Asp Glu His Ala Glu Gly Ser Val Asn Gly Thr Leu Met Gln Leu
                885                 890                 895

Gly Ala Leu Gly Gly Val Asn Val Pro Phe Ala Ala Thr Gly Asp Leu
            900                 905                 910

Thr Val Glu Gly Gly Leu Arg Tyr Asp Leu Leu Lys Gln Asp Ala Phe
        915                 920                 925

Ala Glu Lys Gly Ser Ala Leu Gly Trp Ser Gly Asn Ser Leu Thr Glu
    930                 935                 940

Gly Thr Leu Val Gly Leu Ala Gly Leu Lys Leu Ser Gln Pro Leu Ser
945                 950                 955                 960

Asp Lys Ala Val Leu Phe Ala Thr Ala Gly Val Glu Arg Asp Leu Asn
                965                 970                 975

Gly Arg Asp Tyr Thr Val Thr Gly Gly Phe Thr Gly Ala Thr Ala Ala
            980                 985                 990

Thr Gly Lys Thr Gly Ala Arg Asn Met Pro His Thr Arg Leu Val Ala
        995                 1000                1005

Gly Leu Gly Ala Asp Val Glu Phe Gly Asn Gly Trp Asn Gly Leu Ala
    1010                1015                1020

Arg Tyr Ser Tyr Ala Gly Ser Lys Gln Tyr Gly Asn His Ser Gly Arg
1025                1030                1035                1040

Val Gly Val Gly Tyr Arg Phe
                1045
```

<210> 17
<211> 4425
<212> DNA
<213> Artificial Sequence

<220>
<223> deltaG983-ORF46.1

<400> 17

atgacttctg cgcccgactt caatgcaggc ggtaccggta tcggcagcaa cagcagagca 60
acaacagcga aatcagcagc agtatcttac gccggtatca agaacgaaat gtgcaaagac 120
agaagcatgc tctgtgccgg tcgggatgac gttgcggtta cagacaggga tgccaaaatc 180
aatgcccccc ccccgaatct gcataccgga gactttccaa acccaaatga cgcatacaag 240
aatttgatca acctcaaacc tgcaattgaa gcaggctata caggacgcgg ggtagaggta 300
ggtatcgtcg acacaggcga atccgtcggc agcatatcct ttcccgaact gtatggcaga 360
aaagaacacg gctataacga aaattacaaa aactatacgg cgtatatgcg gaaggaagcg 420
cctgaagacg gaggcggtaa agacattgaa gcttctttcg acgatgaggc cgttatagag 480
actgaagcaa agccgacgga tatccgccac gtaaaagaaa tcggacacat cgatttggtc 540
tcccatatta ttggcgggcg ttccgtggac ggcagacctg caggcggtat tgcgcccgat 600
gcgacgctac acataatgaa tacgaatgat gaaaccaaga acgaaatgat ggttgcagcc 660
atccgcaatg catgggtcaa gctgggcgaa cgtggcgtgc gcatcgtcaa taacagtttt 720
ggaacaacat cgagggcagg cactgccgac cttttccaaa tagccaattc ggaggagcag 780
taccgccaag cgttgctcga ctattccggc ggtgataaaa cagacgaggg tatccgcctg 840
atgcaacaga gcgattacgg caacctgtcc taccacatcc gtaataaaaa catgcttttc 900
atcttttcga caggcaatga cgcacaagct cagcccaaca catatgccct attgccattt 960
tatgaaaaag acgctcaaaa aggcattatc acagtcgcag gcgtagaccg cagtggagaa 1020
aagttcaaac gggaaatgta tggagaaccg ggtacagaac cgcttgagta tggctccaac 1080
cattgcggaa ttactgccat gtggtgcctg tcggcaccct atgaagcaag cgtccgtttc 1140
acccgtacaa acccgattca aattgccgga acatcctttt ccgcacccat cgtaaccggc 1200
acggcggctc tgctgctgca gaaataccg tggatgagca acgacaacct gcgtaccacg 1260
ttgctgacga cggctcagga catcggtgca gtcggcgtgg acagcaagtt cggctgggga 1320
ctgctggatg cgggtaaggc catgaacgga cccgcgtcct ttccgttcgg cgactttacc 1380
gccgatacga aaggtacatc cgatattgcc tactccttcc gtaacgacat ttcaggcacg 1440
ggcggcctga tcaaaaaagg cggcagccaa ctgcaactgc acggcaacaa cacctatacg 1500
ggcaaaacca ttatcgaagg cggttcgctg gtgttgtacg gcaacaacaa atcggatatg 1560
cgcgtcgaaa ccaaaggtgc gctgatttat aacggggcgg catccggcgg cagcctgaac 1620
agcgacggca ttgtctatct ggcagatacc gaccaatccg gcgcaaacga aaccgtacac 1680
atcaaaggca gtctgcagct ggacggcaaa ggtacgctgt acacacgttt gggcaaactg 1740
ctgaaagtgg acggtacggc gattatcggc ggcaagctgt acatgtcggc acgcggcaag 1800
ggggcaggct atctcaacag taccggacga cgtgttccct tcctgagtgc cgccaaaatc 1860
gggcaggatt attctttctt cacaaacatc gaaaccgacg gcggcctgct ggcttccctc 1920
gacagcgtcg aaaaaacagc gggcagtgaa ggcgacacgc tgtcctatta tgtccgtcgc 1980
ggcaatgcgg cacggactgc ttcggcagcg gcacattccg cgcccgccgg tctgaaacac 2040
gccgtagaac agggcggcag caatctggaa aacctgatgg tcgaactgga tgcctccgaa 2100
tcatccgcaa cacccgagac ggttgaaact gcggcagccg accgcacaga tatgccgggc 2160
atccgcccct acggcgcaac tttccgcgca gcggcagccg tacagcatgc gaatgccgcc 2220
gacggtgtac gcatcttcaa cagtctcgcc gctaccgtct atgccgacag taccgccgcc 2280
catgccgata tgcagggacg ccgcctgaaa gccgtatcgg acgggttgga ccacaacggc 2340
acgggtctgc gcgtcatcgc gcaaacccaa caggacggtg gaacgtggga acagggcggt 2400
gttgaaggca aaatgcgcgg cagtacccaa accgtcggca ttgccgcgaa aaccggcgaa 2460
aatacgacag cagccgccac actgggcatg ggacgcagca catggagcga aaacagtgca 2520
aatgcaaaaa ccgacagcat tagtctgttt gcaggcatac ggcacgatgc gggcgatatc 2580
ggctatctca aaggcctgtt ctcctacgga cgctacaaaa acagcatcag ccgcagcacc 2640
ggtgcggacg aacatgcgga aggcagcgtc aacggcacgc tgatgcagct gggcgcactg 2700
ggcggtgtca acgttccgtt tgccgcaacg ggagatttga cggtcgaagg cggtctgcgc 2760
tacgacctgc tcaaacagga tgcattcgcc gaaaaaggca gtgctttggg ctggagcggc 2820
aacagcctca ctgaaggcac gctggtcgga ctcgcgggtc tgaagctgtc gcaacccttg 2880
agcgataaag ccgtcctgtt tgcaacggcg ggcgtggaac gcgacctgaa cggacgcgac 2940
tacacggtaa cgggcggctt taccggcgcg actgcagcaa ccggcaagac gggggcacgc 3000
aatatgccgc acacccgtct ggttgccggc ctgggcgcgg atgtcgaatt cggcaacggc 3060
tggaacggct tggcacgtta cagctacgcc ggttccaaac agtacggcaa ccacagcgga 3120
cgagtcggcg taggctaccg gttcctcgac ggtggcggag gcactggatc ctcagatttg 3180
gcaaacgatt cttttatccg gcaggttctc gaccgtcagc atttcgaacc cgacgggaaa 3240
taccacctat tcggcagcag gggggaactt gccgagcgca gcggccatat cggattggga 3300
aaaatacaaa gccatcagtt gggcaacctg atgattcaac aggcggccat taaaggaaat 3360
atcggctaca ttgtccgctt ttccgatcac gggcacgaag tccattcccc cttcgacaac 3420
catgcctcac attccgattc tgatgaagcc ggtagtcccg ttgacggatt tagcctttac 3480
cgcatccatt gggacggata cgaacaccat cccgccgacg gctatgacgg gccacagggc 3540

```
ggcggctatc ccgctcccaa aggcgcgagg gatatataca gctacgacat aaaaggcgtt   3600
gcccaaaata tccgcctcaa cctgaccgac aaccgcagca ccggacaacg gcttgccgac   3660
cgtttccaca atgccggtag tatgctgacg caaggagtag gcgacggatt caaacgcgcc   3720
acccgataca gccccgagct ggacagatcg ggcaatgccg ccgaagcctt caacggcact   3780
gcagatatcg ttaaaaacat catcggcgcg gcaggagaaa ttgtcggcgc aggcgatgcc   3840
gtgcagggca taagcgaagg ctcaaacatt gctgtcatgc acggcttggg tctgctttcc   3900
accgaaaaca agatggcgcg catcaacgat ttggcagata tggcgcaact caaagactat   3960
gccgcagcag ccatccgcga ttgggcagtc caaaacccca atgccgcaca aggcatagaa   4020
gccgtcagca atatctttat ggcagccatc cccatcaaag ggattggagc tgttcgggga   4080
aaatacggct tgggcggcat cacggcacat cctatcaagc ggtcgcagat gggcgcgatc   4140
gcattgccga aagggaaatc cgccgtcagc gacaattttg ccgatgcggc atacgccaaa   4200
tacccgtccc cttaccattc ccgaaatatc cgttcaaact tggagcagcg ttacggcaaa   4260
gaaaacatca cctcctcaac cgtgccgccg tcaaacggca aaaatgtcaa actggcagac   4320
caacgccacc cgaagacagg cgtaccgttt gacggtaaag ggtttccgaa ttttgagaag   4380
cacgtgaaat atgatacgct cgagcaccac caccaccacc actga                   4425
```

<210> 18
<211> 1474
<212> PRT
<213> Artificial Sequence

<220>
<223> deltaG983-ORF46.1

<400> 18

```
Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly
            20                  25                  30

Ile Lys Asn Glu Met Cys Lys Asp Arg Ser Met Leu Cys Ala Gly Arg
        35                  40                  45

Asp Asp Val Ala Val Thr Asp Arg Asp Ala Lys Ile Asn Ala Pro Pro
    50                  55                  60

Pro Asn Leu His Thr Gly Asp Phe Pro Asn Pro Asn Asp Ala Tyr Lys
65                  70                  75                  80

Asn Leu Ile Asn Leu Lys Pro Ala Ile Glu Ala Gly Tyr Thr Gly Arg
                85                  90                  95

Gly Val Glu Val Gly Ile Val Asp Thr Gly Glu Ser Val Gly Ser Ile
            100                 105                 110

Ser Phe Pro Glu Leu Tyr Gly Arg Lys Glu His Gly Tyr Asn Glu Asn
        115                 120                 125

Tyr Lys Asn Tyr Thr Ala Tyr Met Arg Lys Glu Ala Pro Glu Asp Gly
    130                 135                 140

Gly Gly Lys Asp Ile Glu Ala Ser Phe Asp Asp Glu Ala Val Ile Glu
145                 150                 155                 160

Thr Glu Ala Lys Pro Thr Asp Ile Arg His Val Lys Glu Ile Gly His
                165                 170                 175

Ile Asp Leu Val Ser His Ile Ile Gly Gly Arg Ser Val Asp Gly Arg
            180                 185                 190
```

Pro Ala Gly Gly Ile Ala Pro Asp Ala Thr Leu His Ile Met Asn Thr
195 200 205

Asn Asp Glu Thr Lys Asn Glu Met Met Val Ala Ala Ile Arg Asn Ala
210 215 220

Trp Val Lys Leu Gly Glu Arg Gly Val Arg Ile Val Asn Asn Ser Phe
225 230 235 240

Gly Thr Thr Ser Arg Ala Gly Thr Ala Asp Leu Phe Gln Ile Ala Asn
245 250 255

Ser Glu Glu Gln Tyr Arg Gln Ala Leu Leu Asp Tyr Ser Gly Gly Asp
260 265 270

Lys Thr Asp Glu Gly Ile Arg Leu Met Gln Gln Ser Asp Tyr Gly Asn
275 280 285

Leu Ser Tyr His Ile Arg Asn Lys Asn Met Leu Phe Ile Phe Ser Thr
290 295 300

Gly Asn Asp Ala Gln Ala Gln Pro Asn Thr Tyr Ala Leu Leu Pro Phe
305 310 315 320

Tyr Glu Lys Asp Ala Gln Lys Gly Ile Ile Thr Val Ala Gly Val Asp
325 330 335

Arg Ser Gly Glu Lys Phe Lys Arg Glu Met Tyr Gly Glu Pro Gly Thr
340 345 350

Glu Pro Leu Glu Tyr Gly Ser Asn His Cys Gly Ile Thr Ala Met Trp
355 360 365

Cys Leu Ser Ala Pro Tyr Glu Ala Ser Val Arg Phe Thr Arg Thr Asn
370 375 380

Pro Ile Gln Ile Ala Gly Thr Ser Phe Ser Ala Pro Ile Val Thr Gly
385 390 395 400

Thr Ala Ala Leu Leu Leu Gln Lys Tyr Pro Trp Met Ser Asn Asp Asn
405 410 415

Leu Arg Thr Thr Leu Leu Thr Thr Ala Gln Asp Ile Gly Ala Val Gly
420 425 430

Val Asp Ser Lys Phe Gly Trp Gly Leu Leu Asp Ala Gly Lys Ala Met
435 440 445

Asn Gly Pro Ala Ser Phe Pro Phe Gly Asp Phe Thr Ala Asp Thr Lys
450 455 460

Gly Thr Ser Asp Ile Ala Tyr Ser Phe Arg Asn Asp Ile Ser Gly Thr
465 470 475 480

Gly Gly Leu Ile Lys Lys Gly Gly Ser Gln Leu Gln Leu His Gly Asn
485 490 495

Asn Thr Tyr Thr Gly Lys Thr Ile Ile Glu Gly Gly Ser Leu Val Leu
500 505 510

Tyr Gly Asn Asn Lys Ser Asp Met Arg Val Glu Thr Lys Gly Ala Leu

```
            515                 520                 525

Ile Tyr Asn Gly Ala Ala Ser Gly Gly Ser Leu Asn Ser Asp Gly Ile
        530                 535                 540

Val Tyr Leu Ala Asp Thr Asp Gln Ser Gly Ala Asn Glu Thr Val His
545                 550                 555                 560

Ile Lys Gly Ser Leu Gln Leu Asp Gly Lys Gly Thr Leu Tyr Thr Arg
                565                 570                 575

Leu Gly Lys Leu Leu Lys Val Asp Gly Thr Ala Ile Ile Gly Gly Lys
            580                 585                 590

Leu Tyr Met Ser Ala Arg Gly Lys Gly Ala Gly Tyr Leu Asn Ser Thr
        595                 600                 605

Gly Arg Arg Val Pro Phe Leu Ser Ala Ala Lys Ile Gly Gln Asp Tyr
    610                 615                 620

Ser Phe Phe Thr Asn Ile Glu Thr Asp Gly Gly Leu Leu Ala Ser Leu
625                 630                 635                 640

Asp Ser Val Glu Lys Thr Ala Gly Ser Glu Gly Asp Thr Leu Ser Tyr
                645                 650                 655

Tyr Val Arg Arg Gly Asn Ala Ala Arg Thr Ala Ser Ala Ala Ala His
            660                 665                 670

Ser Ala Pro Ala Gly Leu Lys His Ala Val Glu Gln Gly Gly Ser Asn
        675                 680                 685

Leu Glu Asn Leu Met Val Glu Leu Asp Ala Ser Glu Ser Ser Ala Thr
    690                 695                 700

Pro Glu Thr Val Glu Thr Ala Ala Ala Asp Arg Thr Asp Met Pro Gly
705                 710                 715                 720

Ile Arg Pro Tyr Gly Ala Thr Phe Arg Ala Ala Ala Ala Val Gln His
                725                 730                 735

Ala Asn Ala Ala Asp Gly Val Arg Ile Phe Asn Ser Leu Ala Ala Thr
            740                 745                 750

Val Tyr Ala Asp Ser Thr Ala Ala His Ala Asp Met Gln Gly Arg Arg
        755                 760                 765

Leu Lys Ala Val Ser Asp Gly Leu Asp His Asn Gly Thr Gly Leu Arg
    770                 775                 780

Val Ile Ala Gln Thr Gln Gln Asp Gly Gly Thr Trp Glu Gln Gly Gly
785                 790                 795                 800

Val Glu Gly Lys Met Arg Gly Ser Thr Gln Thr Val Gly Ile Ala Ala
                805                 810                 815

Lys Thr Gly Glu Asn Thr Thr Ala Ala Ala Thr Leu Gly Met Gly Arg
            820                 825                 830

Ser Thr Trp Ser Glu Asn Ser Ala Asn Ala Lys Thr Asp Ser Ile Ser
        835                 840                 845
```

```
Leu Phe Ala Gly Ile Arg His Asp Ala Gly Asp Ile Gly Tyr Leu Lys
    850                 855                 860

Gly Leu Phe Ser Tyr Gly Arg Tyr Lys Asn Ser Ile Ser Arg Ser Thr
865                 870                 875                 880

Gly Ala Asp Glu His Ala Glu Gly Ser Val Asn Gly Thr Leu Met Gln
                885                 890                 895

Leu Gly Ala Leu Gly Gly Val Asn Val Pro Phe Ala Ala Thr Gly Asp
            900                 905                 910

Leu Thr Val Glu Gly Gly Leu Arg Tyr Asp Leu Leu Lys Gln Asp Ala
        915                 920                 925

Phe Ala Glu Lys Gly Ser Ala Leu Gly Trp Ser Gly Asn Ser Leu Thr
    930                 935                 940

Glu Gly Thr Leu Val Gly Leu Ala Gly Leu Lys Leu Ser Gln Pro Leu
945                 950                 955                 960

Ser Asp Lys Ala Val Leu Phe Ala Thr Ala Gly Val Glu Arg Asp Leu
                965                 970                 975

Asn Gly Arg Asp Tyr Thr Val Thr Gly Gly Phe Thr Gly Ala Thr Ala
            980                 985                 990

Ala Thr Gly Lys Thr Gly Ala Arg Asn Met Pro His Thr Arg Leu Val
        995                 1000                1005

Ala Gly Leu Gly Ala Asp Val Glu Phe Gly Asn Gly Trp Asn Gly Leu
    1010                1015                1020

Ala Arg Tyr Ser Tyr Ala Gly Ser Lys Gln Tyr Gly Asn His Ser Gly
1025                1030                1035                1040

Arg Val Gly Val Gly Tyr Arg Phe Leu Asp Gly Gly Gly Gly Thr Gly
                1045                1050                1055

Ser Ser Asp Leu Ala Asn Asp Ser Phe Ile Arg Gln Val Leu Asp Arg
            1060                1065                1070

Gln His Phe Glu Pro Asp Gly Lys Tyr His Leu Phe Gly Ser Arg Gly
        1075                1080                1085

Glu Leu Ala Glu Arg Ser Gly His Ile Gly Leu Gly Lys Ile Gln Ser
    1090                1095                1100

His Gln Leu Gly Asn Leu Met Ile Gln Gln Ala Ala Ile Lys Gly Asn
1105                1110                1115                1120

Ile Gly Tyr Ile Val Arg Phe Ser Asp His Gly His Glu Val His Ser
                1125                1130                1135

Pro Phe Asp Asn His Ala Ser His Ser Asp Ser Asp Glu Ala Gly Ser
            1140                1145                1150

Pro Val Asp Gly Phe Ser Leu Tyr Arg Ile His Trp Asp Gly Tyr Glu
        1155                1160                1165
```

```
His His Pro Ala Asp Gly Tyr Asp Gly Pro Gln Gly Gly Gly Tyr Pro
    1170                1175                1180

Ala Pro Lys Gly Ala Arg Asp Ile Tyr Ser Tyr Asp Ile Lys Gly Val
1185                1190                1195                1200

Ala Gln Asn Ile Arg Leu Asn Leu Thr Asp Asn Arg Ser Thr Gly Gln
                1205                1210                1215

Arg Leu Ala Asp Arg Phe His Asn Ala Gly Ser Met Leu Thr Gln Gly
            1220                1225                1230

Val Gly Asp Gly Phe Lys Arg Ala Thr Arg Tyr Ser Pro Glu Leu Asp
        1235                1240                1245

Arg Ser Gly Asn Ala Ala Glu Ala Phe Asn Gly Thr Ala Asp Ile Val
    1250                1255                1260

Lys Asn Ile Ile Gly Ala Ala Gly Glu Ile Val Gly Ala Gly Asp Ala
1265                1270                1275                1280

Val Gln Gly Ile Ser Glu Gly Ser Asn Ile Ala Val Met His Gly Leu
                1285                1290                1295

Gly Leu Leu Ser Thr Glu Asn Lys Met Ala Arg Ile Asn Asp Leu Ala
            1300                1305                1310

Asp Met Ala Gln Leu Lys Asp Tyr Ala Ala Ala Ala Ile Arg Asp Trp
        1315                1320                1325

Ala Val Gln Asn Pro Asn Ala Ala Gln Gly Ile Glu Ala Val Ser Asn
    1330                1335                1340

Ile Phe Met Ala Ala Ile Pro Ile Lys Gly Ile Gly Ala Val Arg Gly
1345                1350                1355                1360

Lys Tyr Gly Leu Gly Gly Ile Thr Ala His Pro Ile Lys Arg Ser Gln
                1365                1370                1375

Met Gly Ala Ile Ala Leu Pro Lys Gly Lys Ser Ala Val Ser Asp Asn
            1380                1385                1390

Phe Ala Asp Ala Ala Tyr Ala Lys Tyr Pro Ser Pro Tyr His Ser Arg
        1395                1400                1405

Asn Ile Arg Ser Asn Leu Glu Gln Arg Tyr Gly Lys Glu Asn Ile Thr
    1410                1415                1420

Ser Ser Thr Val Pro Pro Ser Asn Gly Lys Asn Val Lys Leu Ala Asp
1425                1430                1435                1440

Gln Arg His Pro Lys Thr Gly Val Pro Phe Asp Gly Lys Gly Phe Pro
                1445                1450                1455

Asn Phe Glu Lys His Val Lys Tyr Asp Thr Leu Glu His His His His
            1460                1465                1470

His His
```

<210> 19
<211> 3939
<212> DNA
<213> Artificial Sequence

<220>
<223> deltaG983-741

<400> 19

atgacttctg cgcccgactt caatgcaggc ggtaccggta tcggcagcaa cagcagagca 60
acaacagcga aatcagcagc agtatcttac gccggtatca agaacgaaat gtgcaaagac 120
agaagcatgc tctgtgccgg tcgggatgac gttgcggtta cagacaggga tgccaaaatc 180
aatgcccccc ccccgaatct gcataccgga gactttccaa acccaaatga cgcatacaag 240
aatttgatca acctcaaacc tgcaattgaa gcaggctata caggacgcgg ggtagaggta 300
ggtatcgtcg acacaggcga atccgtcggc agcatatcct ttcccgaact gtatggcaga 360
aaagaacacg gctataacga aaattacaaa aactatacgg cgtatatgcg gaaggaagcg 420
cctgaagacg gaggcggtaa agacattgaa gcttctttcg acgatgaggc cgttatagag 480
actgaagcaa agccgacgga tatccgccac gtaaaagaaa tcggacacat cgatttggtc 540
tcccatatta ttggcgggcg ttccgtggac ggcagacctg caggcggtat tgcgcccgat 600
gcgacgctac acataatgaa tacgaatgat gaaaccaaga acgaaatgat ggttgcagcc 660
atccgcaatg catgggtcaa gctgggcgaa cgtggcgtgc gcatcgtcaa taacagtttt 720
ggaacaacat cgagggcagg cactgccgac cttttccaaa tagccaattc ggaggagcag 780
taccgccaag cgttgctcga ctattccggc ggtgataaaa cagacgaggg tatccgcctg 840
atgcaacaga gcgattacgg caacctgtcc taccacatcc gtaataaaaa catgcttttc 900
atcttttcga caggcaatga cgcacaagct cagcccaaca catatgccct attgccattt 960
tatgaaaaag acgctcaaaa aggcattatc acagtcgcag gcgtagaccg cagtggagaa 1020
aagttcaaac gggaaatgta tggagaaccg ggtacagaac cgcttgagta tggctccaac 1080
cattgcggaa ttactgccat gtggtgcctg tcggcaccct atgaagcaag cgtccgtttc 1140
acccgtacaa acccgattca aattgccgga acatcctttt ccgcacccat cgtaaccggc 1200
acggcggctc tgctgctgca gaaatacccg tggatgagca acgacaacct gcgtaccacg 1260
ttgctgacga cggctcagga catcggtgca gtcggcgtgg acagcaagtt cggctgggga 1320
ctgctggatg cgggtaaggc catgaacgga cccgcgtcct ttccgttcgg cgactttacc 1380
gccgatacga aaggtacatc cgatattgcc tactccttcc gtaacgacat ttcaggcacg 1440
ggcggcctga tcaaaaaagg cggcagccaa ctgcaactgc acggcaacaa cacctatacg 1500
ggcaaaacca ttatcgaagg cggttcgctg gtgttgtacg gcaacaacaa atcggatatg 1560
cgcgtcgaaa ccaaaggtgc gctgatttat aacggggcgg catccggcgg cagcctgaac 1620
agcgacggca ttgtctatct ggcagatacc gaccaatccg gcgcaaacga aaccgtacac 1680
atcaaaggca gtctgcagct ggacggcaaa ggtacgctgt acacacgttt gggcaaactg 1740
ctgaaagtgg acggtacggc gattatcggc ggcaagctgt acatgtcggc acgcggcaag 1800
ggggcaggct atctcaacag taccggacga cgtgttccct tcctgagtgc cgccaaaatc 1860
gggcaggatt attctttctt cacaaacatc gaaaccgacg gcggcctgct ggcttccctc 1920
gacagcgtcg aaaaaacagc gggcagtgaa ggcgacacgc tgtcctatta tgtccgtcgc 1980
ggcaatgcgg cacggactgc ttcggcagcg gcacattccg cgcccgccgg tctgaaacac 2040
gccgtagaac agggcggcag caatctggaa aacctgatgg tcgaactgga tgcctccgaa 2100
tcatccgcaa cacccgagac ggttgaaact gcggcagccg accgcacaga tatgccgggc 2160
atccgcccct acggcgcaac tttccgcgca gcggcagccg tacagcatgc gaatgccgcc 2220
gacggtgtac gcatcttcaa cagtctcgcc gctaccgtct atgccgacag taccgccgcc 2280
catgccgata tgcagggacg ccgcctgaaa gccgtatcgg acgggttgga ccacaacggc 2340
acgggtctgc gcgtcatcgc gcaaacccaa caggacggtg gaacgtggga acagggcggt 2400
gttgaaggca aaatgcgcgg cagtacccaa accgtcggca ttgccgcgaa aaccggcgaa 2460
aatacgacag cagccgccac actgggcatg ggacgcagca catggagcga aaacagtgca 2520
aatgcaaaaa ccgacagcat tagtctgttt gcaggcatac ggcacgatgc gggcgatatc 2580
ggctatctca aaggcctgtt ctcctacgga cgctacaaaa acagcatcag ccgcagcacc 2640
ggtgcggacg aacatgcgga aggcagcgtc aacggcacgc tgatgcagct gggcgcactg 2700
ggcggtgtca acgttccgtt tgccgcaacg ggagatttga cggtcgaagg cggtctgcgc 2760
tacgacctgc tcaaacagga tgcattcgcc gaaaaaggca gtgctttggg ctggagcggc 2820
aacagcctca ctgaaggcac gctggtcgga ctcgcgggtc tgaagctgtc gcaacccttg 2880
agcgataaag ccgtcctgtt tgcaacggcg ggcgtggaac gcgacctgaa cggacgcgac 2940
tacacggtaa cgggcggctt taccggcgcg actgcagcaa ccggcaagac gggggcacgc 3000
aatatgccgc acacccgtct ggttgccggc ctgggcgcgg atgtcgaatt cggcaacggc 3060
tggaacggct tggcacgtta cagctacgcc ggttccaaac agtacggcaa ccacagcgga 3120
cgagtcggcg taggctaccg gttcctcgag ggatccggag ggggtggtgt cgccgccgac 3180

```
atcggtgcgg ggcttgccga tgcactaacc gcaccgctcg accataaaga caaaggtttg   3240
cagtctttga cgctggatca gtccgtcagg aaaaacgaga aactgaagct ggcggcacaa   3300
ggtgcggaaa aaacttatgg aaacggtgac agcctcaata cgggcaaatt gaagaacgac   3360
aaggtcagcc gtttcgactt tatccgccaa atcgaagtgg acgggcagct cattaccttg   3420
gagagtggag agttccaagt atacaaacaa agccattccg ccttaaccgc ctttcagacc   3480
gagcaaatac aagattcgga gcattccggg aagatggttg cgaaacgcca gttcagaatc   3540
ggcgacatag cgggcgaaca tacatctttt gacaagcttc ccgaaggcgg cagggcgaca   3600
tatcgcggga cggcgttcgg ttcagacgat gccggcggaa aactgaccta caccatagat   3660
ttcgccgcca agcagggaaa cggcaaaatc gaacatttga aatcgccaga actcaatgtc   3720
gacctggccg ccgccgatat caagccggat ggaaaacgcc atgccgtcat cagcggttcc   3780
gtcctttaca accaagccga gaaaggcagt tactccctcg gtatctttgg cggaaaagcc   3840
caggaagttg ccggcagcgc ggaagtgaaa accgtaaacg gcatacgcca tatcggcctt   3900
gccgccaagc aactcgagca ccaccaccac caccactga                          3939
```

<210> 20
<211> 1312
<212> PRT
<213> Artificial Sequence

<220>
<223> deltaG983-741

<400> 20

```
Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly
            20                  25                  30

Ile Lys Asn Glu Met Cys Lys Asp Arg Ser Met Leu Cys Ala Gly Arg
        35                  40                  45

Asp Asp Val Ala Val Thr Asp Arg Asp Ala Lys Ile Asn Ala Pro Pro
    50                  55                  60

Pro Asn Leu His Thr Gly Asp Phe Pro Asn Pro Asn Asp Ala Tyr Lys
65                  70                  75                  80

Asn Leu Ile Asn Leu Lys Pro Ala Ile Glu Ala Gly Tyr Thr Gly Arg
                85                  90                  95

Gly Val Glu Val Gly Ile Val Asp Thr Gly Glu Ser Val Gly Ser Ile
            100                 105                 110

Ser Phe Pro Glu Leu Tyr Gly Arg Lys Glu His Gly Tyr Asn Glu Asn
        115                 120                 125

Tyr Lys Asn Tyr Thr Ala Tyr Met Arg Lys Glu Ala Pro Glu Asp Gly
    130                 135                 140

Gly Gly Lys Asp Ile Glu Ala Ser Phe Asp Asp Glu Ala Val Ile Glu
145                 150                 155                 160

Thr Glu Ala Lys Pro Thr Asp Ile Arg His Val Lys Glu Ile Gly His
                165                 170                 175

Ile Asp Leu Val Ser His Ile Ile Gly Gly Arg Ser Val Asp Gly Arg
            180                 185                 190

Pro Ala Gly Gly Ile Ala Pro Asp Ala Thr Leu His Ile Met Asn Thr
        195                 200                 205
```

```
Asn Asp Glu Thr Lys Asn Glu Met Met Val Ala Ala Ile Arg Asn Ala
    210                 215                 220

Trp Val Lys Leu Gly Glu Arg Gly Val Arg Ile Val Asn Asn Ser Phe
225                 230                 235                 240

Gly Thr Thr Ser Arg Ala Gly Thr Ala Asp Leu Phe Gln Ile Ala Asn
                245                 250                 255

Ser Glu Glu Gln Tyr Arg Gln Ala Leu Leu Asp Tyr Ser Gly Gly Asp
            260                 265                 270

Lys Thr Asp Glu Gly Ile Arg Leu Met Gln Gln Ser Asp Tyr Gly Asn
        275                 280                 285

Leu Ser Tyr His Ile Arg Asn Lys Asn Met Leu Phe Ile Phe Ser Thr
    290                 295                 300

Gly Asn Asp Ala Gln Ala Gln Pro Asn Thr Tyr Ala Leu Leu Pro Phe
305                 310                 315                 320

Tyr Glu Lys Asp Ala Gln Lys Gly Ile Ile Thr Val Ala Gly Val Asp
                325                 330                 335

Arg Ser Gly Glu Lys Phe Lys Arg Glu Met Tyr Gly Glu Pro Gly Thr
            340                 345                 350

Glu Pro Leu Glu Tyr Gly Ser Asn His Cys Gly Ile Thr Ala Met Trp
        355                 360                 365

Cys Leu Ser Ala Pro Tyr Glu Ala Ser Val Arg Phe Thr Arg Thr Asn
    370                 375                 380

Pro Ile Gln Ile Ala Gly Thr Ser Phe Ser Ala Pro Ile Val Thr Gly
385                 390                 395                 400

Thr Ala Ala Leu Leu Leu Gln Lys Tyr Pro Trp Met Ser Asn Asp Asn
                405                 410                 415

Leu Arg Thr Thr Leu Leu Thr Thr Ala Gln Asp Ile Gly Ala Val Gly
            420                 425                 430

Val Asp Ser Lys Phe Gly Trp Gly Leu Leu Asp Ala Gly Lys Ala Met
        435                 440                 445

Asn Gly Pro Ala Ser Phe Pro Phe Gly Asp Phe Thr Ala Asp Thr Lys
    450                 455                 460

Gly Thr Ser Asp Ile Ala Tyr Ser Phe Arg Asn Asp Ile Ser Gly Thr
465                 470                 475                 480

Gly Gly Leu Ile Lys Lys Gly Gly Ser Gln Leu Gln Leu His Gly Asn
                485                 490                 495

Asn Thr Tyr Thr Gly Lys Thr Ile Ile Glu Gly Gly Ser Leu Val Leu
            500                 505                 510

Tyr Gly Asn Asn Lys Ser Asp Met Arg Val Glu Thr Lys Gly Ala Leu
        515                 520                 525
```

Ile Tyr Asn Gly Ala Ala Ser Gly Gly Ser Leu Asn Ser Asp Gly Ile
530 535 540

Val Tyr Leu Ala Asp Thr Asp Gln Ser Gly Ala Asn Glu Thr Val His
545 550 555 560

Ile Lys Gly Ser Leu Gln Leu Asp Gly Lys Gly Thr Leu Tyr Thr Arg
565 570 575

Leu Gly Lys Leu Leu Lys Val Asp Gly Thr Ala Ile Ile Gly Gly Lys
580 585 590

Leu Tyr Met Ser Ala Arg Gly Lys Gly Ala Gly Tyr Leu Asn Ser Thr
595 600 605

Gly Arg Arg Val Pro Phe Leu Ser Ala Ala Lys Ile Gly Gln Asp Tyr
610 615 620

Ser Phe Phe Thr Asn Ile Glu Thr Asp Gly Gly Leu Leu Ala Ser Leu
625 630 635 640

Asp Ser Val Glu Lys Thr Ala Gly Ser Glu Gly Asp Thr Leu Ser Tyr
645 650 655

Tyr Val Arg Arg Gly Asn Ala Ala Arg Thr Ala Ser Ala Ala Ala His
660 665 670

Ser Ala Pro Ala Gly Leu Lys His Ala Val Glu Gln Gly Gly Ser Asn
675 680 685

Leu Glu Asn Leu Met Val Glu Leu Asp Ala Ser Glu Ser Ser Ala Thr
690 695 700

Pro Glu Thr Val Glu Thr Ala Ala Ala Asp Arg Thr Asp Met Pro Gly
705 710 715 720

Ile Arg Pro Tyr Gly Ala Thr Phe Arg Ala Ala Ala Ala Val Gln His
725 730 735

Ala Asn Ala Ala Asp Gly Val Arg Ile Phe Asn Ser Leu Ala Ala Thr
740 745 750

Val Tyr Ala Asp Ser Thr Ala Ala His Ala Asp Met Gln Gly Arg Arg
755 760 765

Leu Lys Ala Val Ser Asp Gly Leu Asp His Asn Gly Thr Gly Leu Arg
770 775 780

Val Ile Ala Gln Thr Gln Gln Asp Gly Gly Thr Trp Glu Gln Gly Gly
785 790 795 800

Val Glu Gly Lys Met Arg Gly Ser Thr Gln Thr Val Gly Ile Ala Ala
805 810 815

Lys Thr Gly Glu Asn Thr Thr Ala Ala Ala Thr Leu Gly Met Gly Arg
820 825 830

Ser Thr Trp Ser Glu Asn Ser Ala Asn Ala Lys Thr Asp Ser Ile Ser
835 840 845

Leu Phe Ala Gly Ile Arg His Asp Ala Gly Asp Ile Gly Tyr Leu Lys

850 855 860

Gly Leu Phe Ser Tyr Gly Arg Tyr Lys Asn Ser Ile Ser Arg Ser Thr
865 870 875 880

Gly Ala Asp Glu His Ala Glu Gly Ser Val Asn Gly Thr Leu Met Gln
885 890 895

Leu Gly Ala Leu Gly Gly Val Asn Val Pro Phe Ala Ala Thr Gly Asp
900 905 910

Leu Thr Val Glu Gly Gly Leu Arg Tyr Asp Leu Leu Lys Gln Asp Ala
915 920 925

Phe Ala Glu Lys Gly Ser Ala Leu Gly Trp Ser Gly Asn Ser Leu Thr
930 935 940

Glu Gly Thr Leu Val Gly Leu Ala Gly Leu Lys Leu Ser Gln Pro Leu
945 950 955 960

Ser Asp Lys Ala Val Leu Phe Ala Thr Ala Gly Val Glu Arg Asp Leu
965 970 975

Asn Gly Arg Asp Tyr Thr Val Thr Gly Gly Phe Thr Gly Ala Thr Ala
980 985 990

Ala Thr Gly Lys Thr Gly Ala Arg Asn Met Pro His Thr Arg Leu Val
995 1000 1005

Ala Gly Leu Gly Ala Asp Val Glu Phe Gly Asn Gly Trp Asn Gly Leu
1010 1015 1020

Ala Arg Tyr Ser Tyr Ala Gly Ser Lys Gln Tyr Gly Asn His Ser Gly
1025 1030 1035 1040

Arg Val Gly Val Gly Tyr Arg Phe Leu Glu Gly Ser Gly Gly Gly Gly
1045 1050 1055

Val Ala Ala Asp Ile Gly Ala Gly Leu Ala Asp Ala Leu Thr Ala Pro
1060 1065 1070

Leu Asp His Lys Asp Lys Gly Leu Gln Ser Leu Thr Leu Asp Gln Ser
1075 1080 1085

Val Arg Lys Asn Glu Lys Leu Lys Leu Ala Ala Gln Gly Ala Glu Lys
1090 1095 1100

Thr Tyr Gly Asn Gly Asp Ser Leu Asn Thr Gly Lys Leu Lys Asn Asp
1105 1110 1115 1120

Lys Val Ser Arg Phe Asp Phe Ile Arg Gln Ile Glu Val Asp Gly Gln
1125 1130 1135

Leu Ile Thr Leu Glu Ser Gly Glu Phe Gln Val Tyr Lys Gln Ser His
1140 1145 1150

Ser Ala Leu Thr Ala Phe Gln Thr Glu Gln Ile Gln Asp Ser Glu His
1155 1160 1165

Ser Gly Lys Met Val Ala Lys Arg Gln Phe Arg Ile Gly Asp Ile Ala
1170 1175 1180

Gly Glu His Thr Ser Phe Asp Lys Leu Pro Glu Gly Gly Arg Ala Thr
1185 1190 1195 1200

Tyr Arg Gly Thr Ala Phe Gly Ser Asp Asp Ala Gly Gly Lys Leu Thr
1205 1210 1215

Tyr Thr Ile Asp Phe Ala Ala Lys Gln Gly Asn Gly Lys Ile Glu His
1220 1225 1230

Leu Lys Ser Pro Glu Leu Asn Val Asp Leu Ala Ala Ala Asp Ile Lys
1235 1240 1245

Pro Asp Gly Lys Arg His Ala Val Ile Ser Gly Ser Val Leu Tyr Asn
1250 1255 1260

Gln Ala Glu Lys Gly Ser Tyr Ser Leu Gly Ile Phe Gly Gly Lys Ala
1265 1270 1275 1280

Gln Glu Val Ala Gly Ser Ala Glu Val Lys Thr Val Asn Gly Ile Arg
1285 1290 1295

His Ile Gly Leu Ala Ala Lys Gln Leu Glu His His His His His His
1300 1305 1310

<210> 21
<211> 4344
<212> DNA
<213> Artificial Sequence

<220>
<223> deltaG983-961

<400> 21

```
atgacttctg cgcccgactt caatgcaggc ggtaccggta tcggcagcaa cagcagagca   60
acaacagcga aatcagcagc agtatcttac gccggtatca agaacgaaat gtgcaaagac  120
agaagcatgc tctgtgccgg tcgggatgac gttgcggtta cagacaggga tgccaaaatc  180
aatgcccccc ccccgaatct gcataccgga gactttccaa acccaaatga cgcatacaag  240
aatttgatca acctcaaacc tgcaattgaa gcaggctata caggacgcgg ggtagaggta  300
ggtatcgtcg acacaggcga atccgtcggc agcatatcct ttcccgaact gtatggcaga  360
aaagaacacg gctataacga aaattacaaa aactatacgg cgtatatgcg gaaggaagcg  420
cctgaagacg gaggcggtaa agacattgaa gcttctttcg acgatgaggc cgttatagag  480
actgaagcaa agccgacgga tatccgccac gtaaaagaaa tcggacacat cgatttggtc  540
tcccatatta ttggcgggcg ttccgtggac ggcagacctg caggcggtat tgcgcccgat  600
gcgacgctac acataatgaa tacgaatgat gaaaccaaga acgaaatgat ggttgcagcc  660
atccgcaatg catgggtcaa gctgggcgaa cgtggcgtgc gcatcgtcaa taacagtttt  720
ggaacaacat cgagggcagg cactgccgac cttttccaaa tagccaattc ggaggagcag  780
taccgccaag cgttgctcga ctattccggc ggtgataaaa cagacgaggg tatccgcctg  840
atgcaacaga gcgattacgg caacctgtcc taccacatcc gtaataaaaa catgcttttc  900
atcttttcga caggcaatga cgcacaagct cagcccaaca catatgccct attgccattt  960
tatgaaaaag acgctcaaaa aggcattatc acagtcgcag gcgtagaccg cagtggagaa 1020
aagttcaaac gggaaatgta tggagaaccg ggtacagaac cgcttgagta tggctccaac 1080
cattgcggaa ttactgccat gtggtgcctg tcggcaccct atgaagcaag cgtccgtttc 1140
acccgtacaa acccgattca aattgccgga acatcctttt ccgcacccat cgtaaccggc 1200
acggcggctc tgctgctgca gaaatacccg tggatgagca acgacaacct gcgtaccacg 1260
ttgctgacga cggctcagga catcggtgca gtcggcgtgg acagcaagtt cggctgggga 1320
ctgctggatg cgggtaaggc catgaacgga cccgcgtcct ttccgttcgg cgactttacc 1380
gccgatacga aaggtacatc cgatattgcc tactccttcc gtaacgacat ttcaggcacg 1440
ggcggcctga tcaaaaaagg cggcagccaa ctgcaactgc acggcaacaa cacctatacg 1500
ggcaaaacca ttatcgaagg cggttcgctg gtgttgtacg gcaacaacaa atcggatatg 1560
```

```
cgcgtcgaaa ccaaaggtgc gctgatttat aacggggcgg catccggcgg cagcctgaac    1620
agcgacggca ttgtctatct ggcagatacc gaccaatccg gcgcaaacga aaccgtacac    1680
atcaaaggca gtctgcagct ggacggcaaa ggtacgctgt acacacgttt gggcaaactg    1740
ctgaaagtgg acggtacggc gattatcggc ggcaagctgt acatgtcggc acgcggcaag    1800
ggggcaggct atctcaacag taccggacga cgtgttccct tcctgagtgc cgccaaaatc    1860
gggcaggatt attctttctt cacaaacatc gaaaccgacg gcggcctgct ggcttccctc    1920
gacagcgtcg aaaaaacagc gggcagtgaa ggcgacacgc tgtcctatta tgtccgtcgc    1980
ggcaatgcgg cacggactgc ttcggcagcg gcacattccg cgcccgccgg tctgaaacac    2040
gccgtagaac agggcggcag caatctggaa aacctgatgg tcgaactgga tgcctccgaa    2100
tcatccgcaa cacccgagac ggttgaaact gcggcagccg accgcacaga tatgccgggc    2160
atccgcccct acggcgcaac tttccgcgca gcggcagccg tacagcatgc gaatgccgcc    2220
gacggtgtac gcatcttcaa cagtctcgcc gctaccgtct atgccgacag taccgccgcc    2280
catgccgata tgcagggacg ccgcctgaaa gccgtatcgg acgggttgga ccacaacggc    2340
acgggtctgc gcgtcatcgc gcaaacccaa caggacggtg gaacgtggga acagggcggt    2400
gttgaaggca aaatgcgcgg cagtacccaa accgtcggca ttgccgcgaa aaccggcgaa    2460
aatacgacag cagccgccac actgggcatg ggacgcagca catggagcga aaacagtgca    2520
aatgcaaaaa ccgacagcat tagtctgttt gcaggcatac ggcacgatgc gggcgatatc    2580
ggctatctca aaggcctgtt ctcctacgga cgctacaaaa acagcatcag ccgcagcacc    2640
ggtgcggacg aacatgcgga aggcagcgtc aacggcacgc tgatgcagct gggcgcactg    2700
ggcggtgtca acgttccgtt tgccgcaacg ggagatttga cggtcgaagg cggtctgcgc    2760
tacgacctgc tcaaacagga tgcattcgcc gaaaaaggca gtgctttggg ctggagcggc    2820
aacagcctca ctgaaggcac gctggtcgga ctcgcgggtc tgaagctgtc gcaacccttg    2880
agcgataaag ccgtcctgtt tgcaacggcg ggcgtggaac gcgacctgaa cggacgcgac    2940
tacacggtaa cgggcggctt taccggcgcg actgcagcaa ccggcaagac ggggcacgc    3000
aatatgccgc acacccgtct ggttgccggc ctgggcgcgg atgtcgaatt cggcaacggc    3060
tggaacggct tggcacgtta cagctacgcc ggttccaaac agtacggcaa ccacagcgga    3120
cgagtcggcg taggctaccg gttcctcgag ggtggcggag gcactggatc cgccacaaac    3180
gacgacgatg ttaaaaaagc tgccactgtg gccattgctg ctgcctacaa caatggccaa    3240
gaaatcaacg gtttcaaagc tggagagacc atctacgaca ttgatgaaga cggcacaatt    3300
accaaaaaag acgcaactgc agccgatgtt gaagccgacg actttaaagg tctgggtctg    3360
aaaaaagtcg tgactaacct gaccaaaacc gtcaatgaaa acaaacaaaa cgtcgatgcc    3420
aaagtaaaag ctgcagaatc tgaaatagaa aagttaacaa ccaagttagc agacactgat    3480
gccgctttag cagatactga tgccgctctg gatgcaacca ccaacgcctt gaataaattg    3540
ggagaaaata taacgacatt tgctgaagag actaagacaa atatcgtaaa aattgatgaa    3600
aaattagaag ccgtggctga taccgtcgac aagcatgccg aagcattcaa cgatatcgcc    3660
gattcattgg atgaaaccaa cactaaggca gacgaagccg tcaaaaccgc caatgaagcc    3720
aaacagacgg ccgaagaaac caaacaaaac gtcgatgcca aagtaaaagc tgcagaaact    3780
gcagcaggca aagccgaagc tgccgctggc acagctaata ctgcagccga caaggccgaa    3840
gctgtcgctg caaaagttac cgacatcaaa gctgatatcg ctacgaacaa agataatatt    3900
gctaaaaaag caaacagtgc cgacgtgtac accagagaag agtctgacag caaatttgtc    3960
agaattgatg gtctgaacgc tactaccgaa aaattggaca cacgcttggc ttctgctgaa    4020
aaatccattg ccgatcacga tactcgcctg aacggtttgg ataaaacagt gtcagacctg    4080
cgcaaagaaa cccgccaagg ccttgcagaa caagccgcgc tctccggtct gttccaacct    4140
tacaacgtgg gtcggttcaa tgtaacggct gcagtcggcg gctacaaatc cgaatcggca    4200
gtcgccatcg gtaccggctt ccgctttacc gaaaactttg ccgccaaagc aggcgtggca    4260
gtcggcactt cgtccggttc ttccgcagcc taccatgtcg gcgtcaatta cgagtggctc    4320
gagcaccacc accaccacca ctga                                           4344
```

<210> 22
<211> 1447
<212> PRT
<213> Artificial Sequence

<220>
<223> deltaG983-961

<400> 22

Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly

```
                20                  25                  30
Ile Lys Asn Glu Met Cys Lys Asp Arg Ser Met Leu Cys Ala Gly Arg
        35                  40                  45
Asp Asp Val Ala Val Thr Asp Arg Asp Ala Lys Ile Asn Ala Pro Pro
    50                  55                  60
Pro Asn Leu His Thr Gly Asp Phe Pro Asn Pro Asn Asp Ala Tyr Lys
65                  70                  75                  80
Asn Leu Ile Asn Leu Lys Pro Ala Ile Glu Ala Gly Tyr Thr Gly Arg
                85                  90                  95
Gly Val Glu Val Gly Ile Val Asp Thr Gly Glu Ser Val Gly Ser Ile
            100                 105                 110
Ser Phe Pro Glu Leu Tyr Gly Arg Lys Glu His Gly Tyr Asn Glu Asn
        115                 120                 125
Tyr Lys Asn Tyr Thr Ala Tyr Met Arg Lys Glu Ala Pro Glu Asp Gly
    130                 135                 140
Gly Gly Lys Asp Ile Glu Ala Ser Phe Asp Asp Glu Ala Val Ile Glu
145                 150                 155                 160
Thr Glu Ala Lys Pro Thr Asp Ile Arg His Val Lys Glu Ile Gly His
                165                 170                 175
Ile Asp Leu Val Ser His Ile Ile Gly Gly Arg Ser Val Asp Gly Arg
            180                 185                 190
Pro Ala Gly Gly Ile Ala Pro Asp Ala Thr Leu His Ile Met Asn Thr
        195                 200                 205
Asn Asp Glu Thr Lys Asn Glu Met Met Val Ala Ala Ile Arg Asn Ala
    210                 215                 220
Trp Val Lys Leu Gly Glu Arg Gly Val Arg Ile Val Asn Asn Ser Phe
225                 230                 235                 240
Gly Thr Thr Ser Arg Ala Gly Thr Ala Asp Leu Phe Gln Ile Ala Asn
                245                 250                 255
Ser Glu Glu Gln Tyr Arg Gln Ala Leu Leu Asp Tyr Ser Gly Gly Asp
            260                 265                 270
Lys Thr Asp Glu Gly Ile Arg Leu Met Gln Gln Ser Asp Tyr Gly Asn
        275                 280                 285
Leu Ser Tyr His Ile Arg Asn Lys Asn Met Leu Phe Ile Phe Ser Thr
    290                 295                 300
Gly Asn Asp Ala Gln Ala Gln Pro Asn Thr Tyr Ala Leu Leu Pro Phe
305                 310                 315                 320
Tyr Glu Lys Asp Ala Gln Lys Gly Ile Ile Thr Val Ala Gly Val Asp
                325                 330                 335
Arg Ser Gly Glu Lys Phe Lys Arg Glu Met Tyr Gly Glu Pro Gly Thr
            340                 345                 350
```

Glu Pro Leu Glu Tyr Gly Ser Asn His Cys Gly Ile Thr Ala Met Trp
355 360 365

Cys Leu Ser Ala Pro Tyr Glu Ala Ser Val Arg Phe Thr Arg Thr Asn
370 375 380

Pro Ile Gln Ile Ala Gly Thr Ser Phe Ser Ala Pro Ile Val Thr Gly
385 390 395 400

Thr Ala Ala Leu Leu Leu Gln Lys Tyr Pro Trp Met Ser Asn Asp Asn
405 410 415

Leu Arg Thr Thr Leu Leu Thr Thr Ala Gln Asp Ile Gly Ala Val Gly
420 425 430

Val Asp Ser Lys Phe Gly Trp Gly Leu Leu Asp Ala Gly Lys Ala Met
435 440 445

Asn Gly Pro Ala Ser Phe Pro Phe Gly Asp Phe Thr Ala Asp Thr Lys
450 455 460

Gly Thr Ser Asp Ile Ala Tyr Ser Phe Arg Asn Asp Ile Ser Gly Thr
465 470 475 480

Gly Gly Leu Ile Lys Lys Gly Gly Ser Gln Leu Gln Leu His Gly Asn
485 490 495

Asn Thr Tyr Thr Gly Lys Thr Ile Ile Glu Gly Gly Ser Leu Val Leu
500 505 510

Tyr Gly Asn Asn Lys Ser Asp Met Arg Val Glu Thr Lys Gly Ala Leu
515 520 525

Ile Tyr Asn Gly Ala Ala Ser Gly Gly Ser Leu Asn Ser Asp Gly Ile
530 535 540

Val Tyr Leu Ala Asp Thr Asp Gln Ser Gly Ala Asn Glu Thr Val His
545 550 555 560

Ile Lys Gly Ser Leu Gln Leu Asp Gly Lys Gly Thr Leu Tyr Thr Arg
565 570 575

Leu Gly Lys Leu Leu Lys Val Asp Gly Thr Ala Ile Ile Gly Gly Lys
580 585 590

Leu Tyr Met Ser Ala Arg Gly Lys Gly Ala Gly Tyr Leu Asn Ser Thr
595 600 605

Gly Arg Arg Val Pro Phe Leu Ser Ala Ala Lys Ile Gly Gln Asp Tyr
610 615 620

Ser Phe Phe Thr Asn Ile Glu Thr Asp Gly Gly Leu Leu Ala Ser Leu
625 630 635 640

Asp Ser Val Glu Lys Thr Ala Gly Ser Glu Gly Asp Thr Leu Ser Tyr
645 650 655

Tyr Val Arg Arg Gly Asn Ala Ala Arg Thr Ala Ser Ala Ala Ala His
660 665 670

```
Ser Ala Pro Ala Gly Leu Lys His Ala Val Glu Gln Gly Gly Ser Asn
          675                 680                 685

Leu Glu Asn Leu Met Val Glu Leu Asp Ala Ser Glu Ser Ser Ala Thr
    690                 695                 700

Pro Glu Thr Val Glu Thr Ala Ala Ala Asp Arg Thr Asp Met Pro Gly
705                 710                 715                 720

Ile Arg Pro Tyr Gly Ala Thr Phe Arg Ala Ala Ala Ala Val Gln His
                725                 730                 735

Ala Asn Ala Ala Asp Gly Val Arg Ile Phe Asn Ser Leu Ala Ala Thr
            740                 745                 750

Val Tyr Ala Asp Ser Thr Ala Ala His Ala Asp Met Gln Gly Arg Arg
        755                 760                 765

Leu Lys Ala Val Ser Asp Gly Leu Asp His Asn Gly Thr Gly Leu Arg
    770                 775                 780

Val Ile Ala Gln Thr Gln Gln Asp Gly Gly Thr Trp Glu Gln Gly Gly
785                 790                 795                 800

Val Glu Gly Lys Met Arg Gly Ser Thr Gln Thr Val Gly Ile Ala Ala
                805                 810                 815

Lys Thr Gly Glu Asn Thr Thr Ala Ala Ala Thr Leu Gly Met Gly Arg
            820                 825                 830

Ser Thr Trp Ser Glu Asn Ser Ala Asn Ala Lys Thr Asp Ser Ile Ser
        835                 840                 845

Leu Phe Ala Gly Ile Arg His Asp Ala Gly Asp Ile Gly Tyr Leu Lys
    850                 855                 860

Gly Leu Phe Ser Tyr Gly Arg Tyr Lys Asn Ser Ile Ser Arg Ser Thr
865                 870                 875                 880

Gly Ala Asp Glu His Ala Glu Gly Ser Val Asn Gly Thr Leu Met Gln
                885                 890                 895

Leu Gly Ala Leu Gly Gly Val Asn Val Pro Phe Ala Ala Thr Gly Asp
            900                 905                 910

Leu Thr Val Glu Gly Gly Leu Arg Tyr Asp Leu Leu Lys Gln Asp Ala
        915                 920                 925

Phe Ala Glu Lys Gly Ser Ala Leu Gly Trp Ser Gly Asn Ser Leu Thr
    930                 935                 940

Glu Gly Thr Leu Val Gly Leu Ala Gly Leu Lys Leu Ser Gln Pro Leu
945                 950                 955                 960

Ser Asp Lys Ala Val Leu Phe Ala Thr Ala Gly Val Glu Arg Asp Leu
                965                 970                 975

Asn Gly Arg Asp Tyr Thr Val Thr Gly Gly Phe Thr Gly Ala Thr Ala
            980                 985                 990

Ala Thr Gly Lys Thr Gly Ala Arg Asn Met Pro His Thr Arg Leu Val
```

995 1000 1005

Ala Gly Leu Gly Ala Asp Val Glu Phe Gly Asn Gly Trp Asn Gly Leu
1010 1015 1020

Ala Arg Tyr Ser Tyr Ala Gly Ser Lys Gln Tyr Gly Asn His Ser Gly
1025 1030 1035 1040

Arg Val Gly Val Gly Tyr Arg Phe Leu Glu Gly Gly Gly Gly Thr Gly
1045 1050 1055

Ser Ala Thr Asn Asp Asp Asp Val Lys Lys Ala Ala Thr Val Ala Ile
1060 1065 1070

Ala Ala Ala Tyr Asn Asn Gly Gln Glu Ile Asn Gly Phe Lys Ala Gly
1075 1080 1085

Glu Thr Ile Tyr Asp Ile Asp Glu Asp Gly Thr Ile Thr Lys Lys Asp
1090 1095 1100

Ala Thr Ala Ala Asp Val Glu Ala Asp Asp Phe Lys Gly Leu Gly Leu
1105 1110 1115 1120

Lys Lys Val Val Thr Asn Leu Thr Lys Thr Val Asn Glu Asn Lys Gln
1125 1130 1135

Asn Val Asp Ala Lys Val Lys Ala Ala Glu Ser Glu Ile Glu Lys Leu
1140 1145 1150

Thr Thr Lys Leu Ala Asp Thr Asp Ala Ala Leu Ala Asp Thr Asp Ala
1155 1160 1165

Ala Leu Asp Ala Thr Thr Asn Ala Leu Asn Lys Leu Gly Glu Asn Ile
1170 1175 1180

Thr Thr Phe Ala Glu Glu Thr Lys Thr Asn Ile Val Lys Ile Asp Glu
1185 1190 1195 1200

Lys Leu Glu Ala Val Ala Asp Thr Val Asp Lys His Ala Glu Ala Phe
1205 1210 1215

Asn Asp Ile Ala Asp Ser Leu Asp Glu Thr Asn Thr Lys Ala Asp Glu
1220 1225 1230

Ala Val Lys Thr Ala Asn Glu Ala Lys Gln Thr Ala Glu Glu Thr Lys
1235 1240 1245

Gln Asn Val Asp Ala Lys Val Lys Ala Ala Glu Thr Ala Ala Gly Lys
1250 1255 1260

Ala Glu Ala Ala Ala Gly Thr Ala Asn Thr Ala Ala Asp Lys Ala Glu
1265 1270 1275 1280

Ala Val Ala Ala Lys Val Thr Asp Ile Lys Ala Asp Ile Ala Thr Asn
1285 1290 1295

Lys Asp Asn Ile Ala Lys Lys Ala Asn Ser Ala Asp Val Tyr Thr Arg
1300 1305 1310

Glu Glu Ser Asp Ser Lys Phe Val Arg Ile Asp Gly Leu Asn Ala Thr
1315 1320 1325

Thr Glu Lys Leu Asp Thr Arg Leu Ala Ser Ala Glu Lys Ser Ile Ala
1330 1335 1340

Asp His Asp Thr Arg Leu Asn Gly Leu Asp Lys Thr Val Ser Asp Leu
1345 1350 1355 1360

Arg Lys Glu Thr Arg Gln Gly Leu Ala Glu Gln Ala Ala Leu Ser Gly
1365 1370 1375

Leu Phe Gln Pro Tyr Asn Val Gly Arg Phe Asn Val Thr Ala Ala Val
1380 1385 1390

Gly Gly Tyr Lys Ser Glu Ser Ala Val Ala Ile Gly Thr Gly Phe Arg
1395 1400 1405

Phe Thr Glu Asn Phe Ala Ala Lys Ala Gly Val Ala Val Gly Thr Ser
1410 1415 1420

Ser Gly Ser Ser Ala Ala Tyr His Val Gly Val Asn Tyr Glu Trp Leu
1425 1430 1435 1440

Glu His His His His His His
1445

<210> 23
<211> 4179
<212> DNA
<213> Artificial Sequence

<220>
<223> deltaG963-961c

<400> 23

```
atgacttctg cgcccgactt caatgcaggc ggtaccggta tcggcagcaa cagcagagca   60
acaacagcga aatcagcagc agtatcttac gccggtatca agaacgaaat gtgcaaagac  120
agaagcatgc tctgtgccgg tcgggatgac gttgcggtta cagacaggga tgccaaaatc  180
aatgcccccc ccccgaatct gcataccgga gactttccaa acccaaatga cgcatacaag  240
aatttgatca acctcaaacc tgcaattgaa gcaggctata caggacgcgg ggtagaggta  300
ggtatcgtcg acacaggcga atccgtcggc agcatatcct ttcccgaact gtatggcaga  360
aaagaacacg gctataacga aaattacaaa aactatacgg cgtatatgcg gaaggaagcg  420
cctgaagacg gaggcggtaa agacattgaa gcttctttcg acgatgaggc cgttatagag  480
actgaagcaa agccgacgga tatccgccac gtaaaagaaa tcggacacat cgatttggtc  540
tcccatatta ttggcgggcg ttccgtggac ggcagacctg caggcggtat tgcgcccgat  600
gcgacgctac acataatgaa tacgaatgat gaaaccaaga acgaaatgat ggttgcagcc  660
atccgcaatg catgggtcaa gctgggcgaa cgtggcgtgc gcatcgtcaa taacagtttt  720
ggaacaacat cgagggcagg cactgccgac cttttccaaa tagccaattc ggaggagcag  780
taccgccaag cgttgctcga ctattccggc ggtgataaaa cagacgaggg tatccgcctg  840
atgcaacaga gcgattacgg caacctgtcc taccacatcc gtaataaaaa catgcttttc  900
atcttttcga caggcaatga cgcacaagct cagcccaaca catatgccct attgccattt  960
tatgaaaaag acgctcaaaa aggcattatc acagtcgcag gcgtagaccg cagtggagaa 1020
aagttcaaac gggaaatgta tggagaaccg ggtacagaac cgcttgagta tggctccaac 1080
cattgcggaa ttactgccat gtggtgcctg tcggcaccct atgaagcaag cgtccgtttc 1140
acccgtacaa acccgattca aattgccgga acatcctttt ccgcacccat cgtaaccggc 1200
acggcggctc tgctgctgca gaaatacccg tggatgagca acgacaacct gcgtaccacg 1260
ttgctgacga cggctcagga catcggtgca gtcggcgtgg acagcaagtt cggctgggga 1320
ctgctggatg cgggtaaggc catgaacgga cccgcgtcct ttccgttcgg cgactttacc 1380
gccgatacga aaggtacatc cgatattgcc tactccttcc gtaacgacat ttcaggcacg 1440
ggcggcctga tcaaaaaagg cggcagccaa ctgcaactgc acggcaacaa cacctatacg 1500
ggcaaaacca ttatcgaagg cggttcgctg gtgttgtacg gcaacaacaa atcggatatg 1560
cgcgtcgaaa ccaaaggtgc gctgatttat aacggggcgg catccggcgg cagcctgaac 1620
```

```
agcgacggca ttgtctatct ggcagatacc gaccaatccg gcgcaaacga aaccgtacac   1680
atcaaaggca gtctgcagct ggacggcaaa ggtacgctgt acacacgttt gggcaaactg   1740
ctgaaagtgg acggtacggc gattatcggc ggcaagctgt acatgtcggc acgcggcaag   1800
ggggcaggct atctcaacag taccggacga cgtgttccct tcctgagtgc cgccaaaatc   1860
gggcaggatt attctttctt cacaaacatc gaaaccgacg gcggcctgct ggcttccctc   1920
gacagcgtcg aaaaaacagc gggcagtgaa ggcgacacgc tgtcctatta tgtccgtcgc   1980
ggcaatgcgg cacggactgc ttcggcagcg gcacattccg cgcccgccgg tctgaaacac   2040
gccgtagaac agggcggcag caatctggaa aacctgatgg tcgaactgga tgcctccgaa   2100
tcatccgcaa cacccgagac ggttgaaact gcggcagccg accgcacaga tatgccgggc   2160
atccgcccct acggcgcaac tttccgcgca gcggcagccg tacagcatgc gaatgccgcc   2220
gacggtgtac gcatcttcaa cagtctcgcc gctaccgtct atgccgacag taccgccgcc   2280
catgccgata tgcagggacg ccgcctgaaa gccgtatcgg acgggttgga ccacaacggc   2340
acgggtctgc gcgtcatcgc gcaaacccaa caggacggtg gaacgtggga acagggcggt   2400
gttgaaggca aaatgcgcgg cagtacccaa accgtcggca ttgccgcgaa aaccggcgaa   2460
aatacgacag cagccgccac actgggcatg ggacgcagca catggagcga aaacagtgca   2520
aatgcaaaaa ccgacagcat tagtctgttt gcaggcatac ggcacgatgc gggcgatatc   2580
ggctatctca aggcctgttt ctcctacgga cgctacaaaa acagcatcag ccgcagcacc   2640
ggtgcggacg aacatgcgga aggcagcgtc aacggcacgc tgatgcagct gggcgcactg   2700
ggcggtgtca acgttccgtt tgccgcaacg ggagatttga cggtcgaagg cggtctgcgc   2760
tacgacctgc tcaaacagga tgcattcgcc gaaaaaggca gtgctttggg ctggagcggc   2820
aacagcctca ctgaaggcac gctggtcgga ctcgcgggtc tgaagctgtc gcaacccttg   2880
agcgataaag ccgtcctgtt tgcaacggcg ggcgtggaac gcgacctgaa cggacgcgac   2940
tacacggtaa cgggcggctt taccggcgcg actgcagcaa ccggcaagac gggggcacgc   3000
aatatgccgc acacccgtct ggttgccggc ctgggcgcgg atgtcgaatt cggcaacggc   3060
tggaacggct tggcacgtta cagctacgcc ggttccaaac agtacggcaa ccacagcgga   3120
cgagtcggcg taggctaccg gttcctcgag ggtggcggag gcactggatc cgccacaaac   3180
gacgacgatg ttaaaaaagc tgccactgtg gccattgctg ctgcctacaa caatggccaa   3240
gaaatcaacg gtttcaaagc tggagagacc atctacgaca ttgatgaaga cggcacaatt   3300
accaaaaaag acgcaactgc agccgatgtt gaagccgacg actttaaagg tctgggtctg   3360
aaaaaagtcg tgactaacct gaccaaaacc gtcaatgaaa acaaacaaaa cgtcgatgcc   3420
aaagtaaaag ctgcagaatc tgaaatagaa aagttaacaa ccaagttagc agacactgat   3480
gccgctttag cagatactga tgccgctctg gatgcaacca ccaacgcctt gaataaattg   3540
ggagaaaata taacgacatt tgctgaagag actaagacaa atatcgtaaa aattgatgaa   3600
aaattagaag ccgtggctga taccgtcgac aagcatgccg aagcattcaa cgatatcgcc   3660
gattcattgg atgaaaccaa cactaaggca gacgaagccg tcaaaaccgc caatgaagcc   3720
aaacagacgg ccgaagaaac caaacaaaac gtcgatgcca aagtaaaagc tgcagaaact   3780
gcagcaggca aagccgaagc tgccgctggc acagctaata ctgcagccga caaggccgaa   3840
gctgtcgctg caaaagttac cgacatcaaa gctgatatcg ctacgaacaa agataatatt   3900
gctaaaaaag caaacagtgc cgacgtgtac accagagaag agtctgacag caaatttgtc   3960
agaattgatg gtctgaacgc tactaccgaa aaattggaca cacgcttggc ttctgctgaa   4020
aaatccattg ccgatcacga tactcgcctg aacggtttgg ataaaacagt gtcagacctg   4080
cgcaaagaaa cccgccaagg ccttgcagaa caagccgcgc tctccggtct gttccaacct   4140
tacaacgtgg gtctcgagca ccaccaccac caccactga                          4179
```

<210> 24
<211> 1392
<212> PRT
<213> Artificial Sequence

<220>
<223> deltaG983-961c

<400> 24

```
Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly
            20                  25                  30

Ile Lys Asn Glu Met Cys Lys Asp Arg Ser Met Leu Cys Ala Gly Arg
        35                  40                  45
```

Asp Asp Val Ala Val Thr Asp Arg Asp Ala Lys Ile Asn Ala Pro Pro
    50                  55                  60

Pro Asn Leu His Thr Gly Asp Phe Pro Asn Pro Asn Asp Ala Tyr Lys
65                  70                  75                  80

Asn Leu Ile Asn Leu Lys Pro Ala Ile Glu Ala Gly Tyr Thr Gly Arg
                85                  90                  95

Gly Val Glu Val Gly Ile Val Asp Thr Gly Glu Ser Val Gly Ser Ile
            100                 105                 110

Ser Phe Pro Glu Leu Tyr Gly Arg Lys Glu His Gly Tyr Asn Glu Asn
        115                 120                 125

Tyr Lys Asn Tyr Thr Ala Tyr Met Arg Lys Glu Ala Pro Glu Asp Gly
    130                 135                 140

Gly Gly Lys Asp Ile Glu Ala Ser Phe Asp Asp Glu Ala Val Ile Glu
145                 150                 155                 160

Thr Glu Ala Lys Pro Thr Asp Ile Arg His Val Lys Glu Ile Gly His
                165                 170                 175

Ile Asp Leu Val Ser His Ile Ile Gly Gly Arg Ser Val Asp Gly Arg
            180                 185                 190

Pro Ala Gly Gly Ile Ala Pro Asp Ala Thr Leu His Ile Met Asn Thr
        195                 200                 205

Asn Asp Glu Thr Lys Asn Glu Met Met Val Ala Ala Ile Arg Asn Ala
    210                 215                 220

Trp Val Lys Leu Gly Glu Arg Gly Val Arg Ile Val Asn Asn Ser Phe
225                 230                 235                 240

Gly Thr Thr Ser Arg Ala Gly Thr Ala Asp Leu Phe Gln Ile Ala Asn
                245                 250                 255

Ser Glu Glu Gln Tyr Arg Gln Ala Leu Leu Asp Tyr Ser Gly Gly Asp
            260                 265                 270

Lys Thr Asp Glu Gly Ile Arg Leu Met Gln Gln Ser Asp Tyr Gly Asn
        275                 280                 285

Leu Ser Tyr His Ile Arg Asn Lys Asn Met Leu Phe Ile Phe Ser Thr
    290                 295                 300

Gly Asn Asp Ala Gln Ala Gln Pro Asn Thr Tyr Ala Leu Leu Pro Phe
305                 310                 315                 320

Tyr Glu Lys Asp Ala Gln Lys Gly Ile Ile Thr Val Ala Gly Val Asp
                325                 330                 335

Arg Ser Gly Glu Lys Phe Lys Arg Glu Met Tyr Gly Glu Pro Gly Thr
            340                 345                 350

Glu Pro Leu Glu Tyr Gly Ser Asn His Cys Gly Ile Thr Ala Met Trp
        355                 360                 365

```
Cys Leu Ser Ala Pro Tyr Glu Ala Ser Val Arg Phe Thr Arg Thr Asn
    370                 375                 380

Pro Ile Gln Ile Ala Gly Thr Ser Phe Ser Ala Pro Ile Val Thr Gly
385                 390                 395                 400

Thr Ala Ala Leu Leu Leu Gln Lys Tyr Pro Trp Met Ser Asn Asp Asn
                405                 410                 415

Leu Arg Thr Thr Leu Leu Thr Thr Ala Gln Asp Ile Gly Ala Val Gly
            420                 425                 430

Val Asp Ser Lys Phe Gly Trp Gly Leu Leu Asp Ala Gly Lys Ala Met
        435                 440                 445

Asn Gly Pro Ala Ser Phe Pro Phe Gly Asp Phe Thr Ala Asp Thr Lys
    450                 455                 460

Gly Thr Ser Asp Ile Ala Tyr Ser Phe Arg Asn Asp Ile Ser Gly Thr
465                 470                 475                 480

Gly Gly Leu Ile Lys Lys Gly Gly Ser Gln Leu Gln Leu His Gly Asn
                485                 490                 495

Asn Thr Tyr Thr Gly Lys Thr Ile Ile Glu Gly Gly Ser Leu Val Leu
            500                 505                 510

Tyr Gly Asn Asn Lys Ser Asp Met Arg Val Glu Thr Lys Gly Ala Leu
        515                 520                 525

Ile Tyr Asn Gly Ala Ala Ser Gly Gly Ser Leu Asn Ser Asp Gly Ile
    530                 535                 540

Val Tyr Leu Ala Asp Thr Asp Gln Ser Gly Ala Asn Glu Thr Val His
545                 550                 555                 560

Ile Lys Gly Ser Leu Gln Leu Asp Gly Lys Gly Thr Leu Tyr Thr Arg
                565                 570                 575

Leu Gly Lys Leu Leu Lys Val Asp Gly Thr Ala Ile Ile Gly Gly Lys
            580                 585                 590

Leu Tyr Met Ser Ala Arg Gly Lys Gly Ala Gly Tyr Leu Asn Ser Thr
        595                 600                 605

Gly Arg Arg Val Pro Phe Leu Ser Ala Ala Lys Ile Gly Gln Asp Tyr
    610                 615                 620

Ser Phe Phe Thr Asn Ile Glu Thr Asp Gly Gly Leu Leu Ala Ser Leu
625                 630                 635                 640

Asp Ser Val Glu Lys Thr Ala Gly Ser Glu Gly Asp Thr Leu Ser Tyr
                645                 650                 655

Tyr Val Arg Arg Gly Asn Ala Ala Arg Thr Ala Ser Ala Ala Ala His
            660                 665                 670

Ser Ala Pro Ala Gly Leu Lys His Ala Val Glu Gln Gly Gly Ser Asn
        675                 680                 685

Leu Glu Asn Leu Met Val Glu Leu Asp Ala Ser Glu Ser Ser Ala Thr
```

690 695 700

Pro Glu Thr Val Glu Thr Ala Ala Ala Asp Arg Thr Asp Met Pro Gly
705 710 715 720

Ile Arg Pro Tyr Gly Ala Thr Phe Arg Ala Ala Ala Ala Val Gln His
725 730 735

Ala Asn Ala Ala Asp Gly Val Arg Ile Phe Asn Ser Leu Ala Ala Thr
740 745 750

Val Tyr Ala Asp Ser Thr Ala Ala His Ala Asp Met Gln Gly Arg Arg
755 760 765

Leu Lys Ala Val Ser Asp Gly Leu Asp His Asn Gly Thr Gly Leu Arg
770 775 780

Val Ile Ala Gln Thr Gln Gln Asp Gly Gly Thr Trp Glu Gln Gly Gly
785 790 795 800

Val Glu Gly Lys Met Arg Gly Ser Thr Gln Thr Val Gly Ile Ala Ala
805 810 815

Lys Thr Gly Glu Asn Thr Thr Ala Ala Ala Thr Leu Gly Met Gly Arg
820 825 830

Ser Thr Trp Ser Glu Asn Ser Ala Asn Ala Lys Thr Asp Ser Ile Ser
835 840 845

Leu Phe Ala Gly Ile Arg His Asp Ala Gly Asp Ile Gly Tyr Leu Lys
850 855 860

Gly Leu Phe Ser Tyr Gly Arg Tyr Lys Asn Ser Ile Ser Arg Ser Thr
865 870 875 880

Gly Ala Asp Glu His Ala Glu Gly Ser Val Asn Gly Thr Leu Met Gln
885 890 895

Leu Gly Ala Leu Gly Gly Val Asn Val Pro Phe Ala Ala Thr Gly Asp
900 905 910

Leu Thr Val Glu Gly Gly Leu Arg Tyr Asp Leu Leu Lys Gln Asp Ala
915 920 925

Phe Ala Glu Lys Gly Ser Ala Leu Gly Trp Ser Gly Asn Ser Leu Thr
930 935 940

Glu Gly Thr Leu Val Gly Leu Ala Gly Leu Lys Leu Ser Gln Pro Leu
945 950 955 960

Ser Asp Lys Ala Val Leu Phe Ala Thr Ala Gly Val Glu Arg Asp Leu
965 970 975

Asn Gly Arg Asp Tyr Thr Val Thr Gly Gly Phe Thr Gly Ala Thr Ala
980 985 990

Ala Thr Gly Lys Thr Gly Ala Arg Asn Met Pro His Thr Arg Leu Val
995 1000 1005

Ala Gly Leu Gly Ala Asp Val Glu Phe Gly Asn Gly Trp Asn Gly Leu
1010 1015 1020

```
Ala Arg Tyr Ser Tyr Ala Gly Ser Lys Gln Tyr Gly Asn His Ser Gly
1025                1030                1035                1040

Arg Val Gly Val Gly Tyr Arg Phe Leu Glu Gly Gly Gly Gly Thr Gly
                1045                1050                1055

Ser Ala Thr Asn Asp Asp Asp Val Lys Lys Ala Ala Thr Val Ala Ile
            1060                1065                1070

Ala Ala Ala Tyr Asn Asn Gly Gln Glu Ile Asn Gly Phe Lys Ala Gly
        1075                1080                1085

Glu Thr Ile Tyr Asp Ile Asp Glu Asp Gly Thr Ile Thr Lys Lys Asp
    1090                1095                1100

Ala Thr Ala Ala Asp Val Glu Ala Asp Asp Phe Lys Gly Leu Gly Leu
1105                1110                1115                1120

Lys Lys Val Val Thr Asn Leu Thr Lys Thr Val Asn Glu Asn Lys Gln
                1125                1130                1135

Asn Val Asp Ala Lys Val Lys Ala Ala Glu Ser Glu Ile Glu Lys Leu
            1140                1145                1150

Thr Thr Lys Leu Ala Asp Thr Asp Ala Ala Leu Ala Asp Thr Asp Ala
        1155                1160                1165

Ala Leu Asp Ala Thr Thr Asn Ala Leu Asn Lys Leu Gly Glu Asn Ile
    1170                1175                1180

Thr Thr Phe Ala Glu Glu Thr Lys Thr Asn Ile Val Lys Ile Asp Glu
1185                1190                1195                1200

Lys Leu Glu Ala Val Ala Asp Thr Val Asp Lys His Ala Glu Ala Phe
                1205                1210                1215

Asn Asp Ile Ala Asp Ser Leu Asp Glu Thr Asn Thr Lys Ala Asp Glu
            1220                1225                1230

Ala Val Lys Thr Ala Asn Glu Ala Lys Gln Thr Ala Glu Glu Thr Lys
        1235                1240                1245

Gln Asn Val Asp Ala Lys Val Lys Ala Ala Glu Thr Ala Ala Gly Lys
    1250                1255                1260

Ala Glu Ala Ala Ala Gly Thr Ala Asn Thr Ala Ala Asp Lys Ala Glu
1265                1270                1275                1280

Ala Val Ala Ala Lys Val Thr Asp Ile Lys Ala Asp Ile Ala Thr Asn
                1285                1290                1295

Lys Asp Asn Ile Ala Lys Lys Ala Asn Ser Ala Asp Val Tyr Thr Arg
            1300                1305                1310

Glu Glu Ser Asp Ser Lys Phe Val Arg Ile Asp Gly Leu Asn Ala Thr
        1315                1320                1325

Thr Glu Lys Leu Asp Thr Arg Leu Ala Ser Ala Glu Lys Ser Ile Ala
    1330                1335                1340
```

Asp His Asp Thr Arg Leu Asn Gly Leu Asp Lys Thr Val Ser Asp Leu
1345                1350                1355                1360

Arg Lys Glu Thr Arg Gln Gly Leu Ala Glu Gln Ala Ala Leu Ser Gly
                1365                1370                1375

Leu Phe Gln Pro Tyr Asn Val Gly Leu Glu His His His His His His
            1380                1385                1390

<210> 25
<211> 274
<212> PRT
<213> Artificial Sequence

<220>
<223> 741

<400> 25

```
Val Asn Arg Thr Ala Phe Cys Cys Leu Ser Leu Thr Thr Ala Leu Ile
1               5                   10                  15

Leu Thr Ala Cys Ser Ser Gly Gly Gly Gly Val Ala Ala Asp Ile Gly
            20                  25                  30

Ala Gly Leu Ala Asp Ala Leu Thr Ala Pro Leu Asp His Lys Asp Lys
        35                  40                  45

Gly Leu Gln Ser Leu Thr Leu Asp Gln Ser Val Arg Lys Asn Glu Lys
    50                  55                  60

Leu Lys Leu Ala Ala Gln Gly Ala Glu Lys Thr Tyr Gly Asn Gly Asp
65                  70                  75                  80

Ser Leu Asn Thr Gly Lys Leu Lys Asn Asp Lys Val Ser Arg Phe Asp
                85                  90                  95

Phe Ile Arg Gln Ile Glu Val Asp Gly Gln Leu Ile Thr Leu Glu Ser
            100                 105                 110

Gly Glu Phe Gln Val Tyr Lys Gln Ser His Ser Ala Leu Thr Ala Phe
        115                 120                 125

Gln Thr Glu Gln Ile Gln Asp Ser Glu His Ser Gly Lys Met Val Ala
    130                 135                 140

Lys Arg Gln Phe Arg Ile Gly Asp Ile Ala Gly Glu His Thr Ser Phe
145                 150                 155                 160

Asp Lys Leu Pro Glu Gly Gly Arg Ala Thr Tyr Arg Gly Thr Ala Phe
                165                 170                 175

Gly Ser Asp Asp Ala Gly Gly Lys Leu Thr Tyr Thr Ile Asp Phe Ala
            180                 185                 190

Ala Lys Gln Gly Asn Gly Lys Ile Glu His Leu Lys Ser Pro Glu Leu
        195                 200                 205

Asn Val Asp Leu Ala Ala Ala Asp Ile Lys Pro Asp Gly Lys Arg His
    210                 215                 220

Ala Val Ile Ser Gly Ser Val Leu Tyr Asn Gln Ala Glu Lys Gly Ser
225                 230                 235                 240

Tyr Ser Leu Gly Ile Phe Gly Gly Lys Ala Gln Glu Val Ala Gly Ser
                245                 250                 255

Ala Glu Val Lys Thr Val Asn Gly Ile Arg His Ile Gly Leu Ala Ala
            260                 265                 270

Lys Gln
```

<210> 26
<211> 248
<212> PRT
<213> Artificial Sequence

<220>
<223> deltaG741

<400> 26

```
Val Ala Ala Asp Ile Gly Ala Gly Leu Ala Asp Ala Leu Thr Ala Pro
1               5                   10                  15

Leu Asp His Lys Asp Lys Gly Leu Gln Ser Leu Thr Leu Asp Gln Ser
            20                  25                  30

Val Arg Lys Asn Glu Lys Leu Lys Leu Ala Ala Gln Gly Ala Glu Lys
        35                  40                  45

Thr Tyr Gly Asn Gly Asp Ser Leu Asn Thr Gly Lys Leu Lys Asn Asp
    50                  55                  60

Lys Val Ser Arg Phe Asp Phe Ile Arg Gln Ile Glu Val Asp Gly Gln
65                  70                  75                  80

Leu Ile Thr Leu Glu Ser Gly Glu Phe Gln Val Tyr Lys Gln Ser His
                85                  90                  95

Ser Ala Leu Thr Ala Phe Gln Thr Glu Gln Ile Gln Asp Ser Glu His
            100                 105                 110

Ser Gly Lys Met Val Ala Lys Arg Gln Phe Arg Ile Gly Asp Ile Ala
        115                 120                 125

Gly Glu His Thr Ser Phe Asp Lys Leu Pro Glu Gly Gly Arg Ala Thr
    130                 135                 140

Tyr Arg Gly Thr Ala Phe Gly Ser Asp Asp Ala Gly Gly Lys Leu Thr
145                 150                 155                 160

Tyr Thr Ile Asp Phe Ala Ala Lys Gln Gly Asn Gly Lys Ile Glu His
                165                 170                 175

Leu Lys Ser Pro Glu Leu Asn Val Asp Leu Ala Ala Ala Asp Ile Lys
            180                 185                 190

Pro Asp Gly Lys Arg His Ala Val Ile Ser Gly Ser Val Leu Tyr Asn
        195                 200                 205

Gln Ala Glu Lys Gly Ser Tyr Ser Leu Gly Ile Phe Gly Gly Lys Ala
    210                 215                 220

Gln Glu Val Ala Gly Ser Ala Glu Val Lys Thr Val Asn Gly Ile Arg
225                 230                 235                 240

His Ile Gly Leu Ala Ala Lys Gln
                245
```

<210> 27
<211> 1947
<212> DNA

<213> Artificial Sequence

<220>
<223> deltaG741-961

<400> 27

```
atggtcgccg ccgacatcgg tgcggggctt gccgatgcac taaccgcacc gctcgaccat   60
aaagacaaag gtttgcagtc tttgacgctg gatcagtccg tcaggaaaaa cgagaaactg   120
aagctggcgg cacaaggtgc ggaaaaaact tatggaaacg gtgacagcct caatacgggc   180
aaattgaaga acgacaaggt cagccgtttc gactttatcc gccaaatcga agtggacggg   240
cagctcatta ccttggagag tggagagttc caagtataca aacaaagcca ttccgcctta   300
accgcctttc agaccgagca aatacaagat tcggagcatt ccgggaagat ggttgcgaaa   360
cgccagttca gaatcggcga catagcgggc gaacatacat cttttgacaa gcttcccgaa   420
ggcggcaggg cgacatatcg cgggacggcg ttcggttcag acgatgccgg cggaaaactg   480
acctacacca tagatttcgc cgccaagcag ggaaacggca aaatcgaaca tttgaaatcg   540
ccagaactca atgtcgacct ggccgccgcc gatatcaagc cggatggaaa acgccatgcc   600
gtcatcagcg gttccgtcct ttacaaccaa gccgagaaag gcagttactc cctcggtatc   660
tttggcggaa aagcccagga agttgccggc agcgcggaag tgaaaaccgt aaacggcata   720
cgccatatcg gccttgccgc caagcaactc gagggtggcg gaggcactgg atccgccaca   780
aacgacgacg atgttaaaaa agctgccact gtggccattg ctgctgccta caacaatggc   840
caagaaatca acggtttcaa agctggagag accatctacg acattgatga agacggcaca   900
attaccaaaa aagacgcaac tgcagccgat gttgaagccg acgactttaa aggtctgggt   960
ctgaaaaaag tcgtgactaa cctgaccaaa accgtcaatg aaaacaaaca aaacgtcgat   1020
gccaaagtaa aagctgcaga atctgaaata gaaaagttaa caaccaagtt agcagacact   1080
gatgccgctt tagcagatac tgatgccgct ctggatgcaa ccaccaacgc cttgaataaa   1140
ttgggagaaa atataacgac atttgctgaa gagactaaga caaatatcgt aaaaattgat   1200
gaaaaattag aagccgtggc tgataccgtc gacaagcatg ccgaagcatt caacgatatc   1260
gccgattcat tggatgaaac caacactaag gcagacgaag ccgtcaaaac cgccaatgaa   1320
gccaaacaga cggccgaaga aaccaaacaa aacgtcgatg ccaaagtaaa agctgcagaa   1380
actgcagcag gcaaagccga agctgccgct ggcacagcta atactgcagc cgacaaggcc   1440
gaagctgtcg ctgcaaaagt taccgacatc aaagctgata tcgctacgaa caaagataat   1500
attgctaaaa aagcaaacag tgccgacgtg tacaccagag aagagtctga cagcaaattt   1560
gtcagaattg atggtctgaa cgctactacc gaaaaattgg acacacgctt ggcttctgct   1620
gaaaaatcca ttgccgatca cgatactcgc ctgaacggtt tggataaaac agtgtcagac   1680
ctgcgcaaag aaacccgcca aggccttgca gaacaagccg cgctctccgg tctgttccaa   1740
ccttacaacg tgggtcggtt caatgtaacg gctgcagtcg gcggctacaa atccgaatcg   1800
gcagtcgcca tcggtaccgg cttccgcttt accgaaaact ttgccgccaa agcaggcgtg   1860
gcagtcggca cttcgtccgg ttcttccgca gcctaccatg tcggcgtcaa ttacgagtgg   1920
ctcgagcacc accaccacca ccactga                                       1947
```

<210> 28
<211> 648
<212> PRT
<213> Artificial Sequence

<220>
<223> deltaG791-961

<400> 28

```
Met Val Ala Ala Asp Ile Gly Ala Gly Leu Ala Asp Ala Leu Thr Ala
```

```
1               5                   10                  15
Pro Leu Asp His Lys Asp Lys Gly Leu Gln Ser Leu Thr Leu Asp Gln
            20                  25                  30
Ser Val Arg Lys Asn Glu Lys Leu Lys Leu Ala Ala Gln Gly Ala Glu
        35                  40                  45
Lys Thr Tyr Gly Asn Gly Asp Ser Leu Asn Thr Gly Lys Leu Lys Asn
    50                  55                  60
Asp Lys Val Ser Arg Phe Asp Phe Ile Arg Gln Ile Glu Val Asp Gly
65                  70                  75                  80
Gln Leu Ile Thr Leu Glu Ser Gly Glu Phe Gln Val Tyr Lys Gln Ser
                85                  90                  95
His Ser Ala Leu Thr Ala Phe Gln Thr Glu Gln Ile Gln Asp Ser Glu
            100                 105                 110
His Ser Gly Lys Met Val Ala Lys Arg Gln Phe Arg Ile Gly Asp Ile
        115                 120                 125
Ala Gly Glu His Thr Ser Phe Asp Lys Leu Pro Glu Gly Gly Arg Ala
    130                 135                 140
Thr Tyr Arg Gly Thr Ala Phe Gly Ser Asp Asp Ala Gly Gly Lys Leu
145                 150                 155                 160
Thr Tyr Thr Ile Asp Phe Ala Ala Lys Gln Gly Asn Gly Lys Ile Glu
                165                 170                 175
His Leu Lys Ser Pro Glu Leu Asn Val Asp Leu Ala Ala Ala Asp Ile
            180                 185                 190
Lys Pro Asp Gly Lys Arg His Ala Val Ile Ser Gly Ser Val Leu Tyr
        195                 200                 205
Asn Gln Ala Glu Lys Gly Ser Tyr Ser Leu Gly Ile Phe Gly Gly Lys
    210                 215                 220
Ala Gln Glu Val Ala Gly Ser Ala Glu Val Lys Thr Val Asn Gly Ile
225                 230                 235                 240
Arg His Ile Gly Leu Ala Ala Lys Gln Leu Glu Gly Gly Gly Gly Thr
                245                 250                 255
Gly Ser Ala Thr Asn Asp Asp Asp Val Lys Lys Ala Ala Thr Val Ala
            260                 265                 270
Ile Ala Ala Ala Tyr Asn Asn Gly Gln Glu Ile Asn Gly Phe Lys Ala
        275                 280                 285
Gly Glu Thr Ile Tyr Asp Ile Asp Glu Asp Gly Thr Ile Thr Lys Lys
    290                 295                 300
Asp Ala Thr Ala Ala Asp Val Glu Ala Asp Asp Phe Lys Gly Leu Gly
305                 310                 315                 320
Leu Lys Lys Val Val Thr Asn Leu Thr Lys Thr Val Asn Glu Asn Lys
                325                 330                 335
```

Gln Asn Val Asp Ala Lys Val Lys Ala Ala Glu Ser Glu Ile Glu Lys
340 345 350

Leu Thr Thr Lys Leu Ala Asp Thr Asp Ala Ala Leu Ala Asp Thr Asp
355 360 365

Ala Ala Leu Asp Ala Thr Thr Asn Ala Leu Asn Lys Leu Gly Glu Asn
370 375 380

Ile Thr Thr Phe Ala Glu Glu Thr Lys Thr Asn Ile Val Lys Ile Asp
385 390 395 400

Glu Lys Leu Glu Ala Val Ala Asp Thr Val Asp Lys His Ala Glu Ala
405 410 415

Phe Asn Asp Ile Ala Asp Ser Leu Asp Glu Thr Asn Thr Lys Ala Asp
420 425 430

Glu Ala Val Lys Thr Ala Asn Glu Ala Lys Gln Thr Ala Glu Glu Thr
435 440 445

Lys Gln Asn Val Asp Ala Lys Val Lys Ala Ala Glu Thr Ala Ala Gly
450 455 460

Lys Ala Glu Ala Ala Ala Gly Thr Ala Asn Thr Ala Ala Asp Lys Ala
465 470 475 480

Glu Ala Val Ala Ala Lys Val Thr Asp Ile Lys Ala Asp Ile Ala Thr
485 490 495

Asn Lys Asp Asn Ile Ala Lys Lys Ala Asn Ser Ala Asp Val Tyr Thr
500 505 510

Arg Glu Glu Ser Asp Ser Lys Phe Val Arg Ile Asp Gly Leu Asn Ala
515 520 525

Thr Thr Glu Lys Leu Asp Thr Arg Leu Ala Ser Ala Glu Lys Ser Ile
530 535 540

Ala Asp His Asp Thr Arg Leu Asn Gly Leu Asp Lys Thr Val Ser Asp
545 550 555 560

Leu Arg Lys Glu Thr Arg Gln Gly Leu Ala Glu Gln Ala Ala Leu Ser
565 570 575

Gly Leu Phe Gln Pro Tyr Asn Val Gly Arg Phe Asn Val Thr Ala Ala
580 585 590

Val Gly Gly Tyr Lys Ser Glu Ser Ala Val Ala Ile Gly Thr Gly Phe
595 600 605

Arg Phe Thr Glu Asn Phe Ala Ala Lys Ala Gly Val Ala Val Gly Thr
610 615 620

Ser Ser Gly Ser Ser Ala Ala Tyr His Val Gly Val Asn Tyr Glu Trp
625 630 635 640

Leu Glu His His His His His His
645

<210> 29
<211> 1782
<212> DNA
<213> Artificial Sequence

<220>
<223> deltaG741-961c

<400> 29

```
atggtcgccg ccgacatcgg tgcggggctt gccgatgcac taaccgcacc gctcgaccat   60
aaagacaaag gtttgcagtc tttgacgctg gatcagtccg tcaggaaaaa cgagaaactg   120
aagctggcgg cacaaggtgc ggaaaaaact tatggaaacg gtgacagcct caatacgggc   180
aaattgaaga acgacaaggt cagccgtttc gactttatcc gccaaatcga agtggacggg   240
cagctcatta ccttggagag tggagagttc caagtataca aacaaagcca ttccgcctta   300
accgcctttc agaccgagca aatacaagat tcggagcatt ccgggaagat ggttgcgaaa   360
cgccagttca gaatcggcga catagcgggc gaacatacat cttttgacaa gcttcccgaa   420
ggcggcaggg cgacatatcg cgggacggcg ttcggttcag acgatgccgg cggaaaactg   480
acctacacca tagatttcgc cgccaagcag ggaaacggca aaatcgaaca tttgaaatcg   540
ccagaactca atgtcgacct ggccgccgcc gatatcaagc cggatggaaa acgccatgcc   600
gtcatcagcg gttccgtcct ttacaaccaa gccgagaaag gcagttactc cctcggtatc   660
tttggcggaa aagcccagga agttgccggc agcgcggaag tgaaaaccgt aaacggcata   720
cgccatatcg gccttgccgc caagcaactc gagggtggcg gaggcactgg atccgccaca   780
aacgacgacg atgttaaaaa agctgccact gtggccattg ctgctgccta caacaatggc   840
caagaaatca acggtttcaa agctggagag accatctacg acattgatga agacggcaca   900
attaccaaaa aagacgcaac tgcagccgat gttgaagccg acgactttaa aggtctgggt   960
ctgaaaaaag tcgtgactaa cctgaccaaa accgtcaatg aaaacaaaca aaacgtcgat   1020
gccaaagtaa aagctgcaga atctgaaata gaaaagttaa caaccaagtt agcagacact   1080
gatgccgctt tagcagatac tgatgccgct ctggatgcaa ccaccaacgc cttgaataaa   1140
ttgggagaaa atataacgac atttgctgaa gagactaaga caaatatcgt aaaaattgat   1200
gaaaaattag aagccgtggc tgataccgtc gacaagcatg ccgaagcatt caacgatatc   1260
gccgattcat tggatgaaac caacactaag gcagacgaag ccgtcaaaac cgccaatgaa   1320
gccaaacaga cggccgaaga aaccaaacaa aacgtcgatg ccaaagtaaa agctgcagaa   1380
actgcagcag gcaaagccga agctgccgct ggcacagcta atactgcagc cgacaaggcc   1440
gaagctgtcg ctgcaaaagt taccgacatc aaagctgata tcgctacgaa caaagataat   1500
attgctaaaa aagcaaacag tgccgacgtg tacaccagag aagagtctga cagcaaattt   1560
gtcagaattg atggtctgaa cgctactacc gaaaaattgg acacacgctt ggcttctgct   1620
gaaaaatcca ttgccgatca cgatactcgc ctgaacggtt tggataaaac agtgtcagac   1680
ctgcgcaaag aaacccgcca aggccttgca gaacaagccg cgctctccgg tctgttccaa   1740
ccttacaacg tgggtctcga gcaccaccac caccaccact ga                      1782
```

<210> 30
<211> 593
<212> PRT
<213> Artificial Sequence

<220>
<223> deltaG741-961c

<400> 30

```
Met Val Ala Ala Asp Ile Gly Ala Gly Leu Ala Asp Ala Leu Thr Ala
1               5                   10                  15

Pro Leu Asp His Lys Asp Lys Gly Leu Gln Ser Leu Thr Leu Asp Gln
            20                  25                  30

Ser Val Arg Lys Asn Glu Lys Leu Lys Leu Ala Ala Gln Gly Ala Glu
        35                  40                  45

Lys Thr Tyr Gly Asn Gly Asp Ser Leu Asn Thr Gly Lys Leu Lys Asn
    50                  55                  60
```

```
Asp Lys Val Ser Arg Phe Asp Phe Ile Arg Gln Ile Glu Val Asp Gly
65                  70                  75                  80

Gln Leu Ile Thr Leu Glu Ser Gly Glu Phe Gln Val Tyr Lys Gln Ser
                85                  90                  95

His Ser Ala Leu Thr Ala Phe Gln Thr Glu Gln Ile Gln Asp Ser Glu
            100                 105                 110

His Ser Gly Lys Met Val Ala Lys Arg Gln Phe Arg Ile Gly Asp Ile
        115                 120                 125

Ala Gly Glu His Thr Ser Phe Asp Lys Leu Pro Glu Gly Gly Arg Ala
    130                 135                 140

Thr Tyr Arg Gly Thr Ala Phe Gly Ser Asp Asp Ala Gly Gly Lys Leu
145                 150                 155                 160

Thr Tyr Thr Ile Asp Phe Ala Ala Lys Gln Gly Asn Gly Lys Ile Glu
                165                 170                 175

His Leu Lys Ser Pro Glu Leu Asn Val Asp Leu Ala Ala Ala Asp Ile
            180                 185                 190

Lys Pro Asp Gly Lys Arg His Ala Val Ile Ser Gly Ser Val Leu Tyr
        195                 200                 205

Asn Gln Ala Glu Lys Gly Ser Tyr Ser Leu Gly Ile Phe Gly Gly Lys
    210                 215                 220

Ala Gln Glu Val Ala Gly Ser Ala Glu Val Lys Thr Val Asn Gly Ile
225                 230                 235                 240

Arg His Ile Gly Leu Ala Ala Lys Gln Leu Glu Gly Gly Gly Gly Thr
                245                 250                 255

Gly Ser Ala Thr Asn Asp Asp Asp Val Lys Lys Ala Ala Thr Val Ala
            260                 265                 270

Ile Ala Ala Ala Tyr Asn Asn Gly Gln Glu Ile Asn Gly Phe Lys Ala
        275                 280                 285

Gly Glu Thr Ile Tyr Asp Ile Asp Glu Asp Gly Thr Ile Thr Lys Lys
    290                 295                 300

Asp Ala Thr Ala Ala Asp Val Glu Ala Asp Asp Phe Lys Gly Leu Gly
305                 310                 315                 320

Leu Lys Lys Val Val Thr Asn Leu Thr Lys Thr Val Asn Glu Asn Lys
                325                 330                 335

Gln Asn Val Asp Ala Lys Val Lys Ala Ala Glu Ser Glu Ile Glu Lys
            340                 345                 350

Leu Thr Thr Lys Leu Ala Asp Thr Asp Ala Ala Leu Ala Asp Thr Asp
        355                 360                 365

Ala Ala Leu Asp Ala Thr Thr Asn Ala Leu Asn Lys Leu Gly Glu Asn
    370                 375                 380

Ile Thr Thr Phe Ala Glu Glu Thr Lys Thr Asn Ile Val Lys Ile Asp
```

```
385                 390                 395                 400

Glu Lys Leu Glu Ala Val Ala Asp Thr Val Asp Lys His Ala Glu Ala
                405                 410                 415

Phe Asn Asp Ile Ala Asp Ser Leu Asp Glu Thr Asn Thr Lys Ala Asp
            420                 425                 430

Glu Ala Val Lys Thr Ala Asn Glu Ala Lys Gln Thr Ala Glu Glu Thr
        435                 440                 445

Lys Gln Asn Val Asp Ala Lys Val Lys Ala Ala Glu Thr Ala Ala Gly
    450                 455                 460

Lys Ala Glu Ala Ala Ala Gly Thr Ala Asn Thr Ala Ala Asp Lys Ala
465                 470                 475                 480

Glu Ala Val Ala Ala Lys Val Thr Asp Ile Lys Ala Asp Ile Ala Thr
                485                 490                 495

Asn Lys Asp Asn Ile Ala Lys Lys Ala Asn Ser Ala Asp Val Tyr Thr
            500                 505                 510

Arg Glu Glu Ser Asp Ser Lys Phe Val Arg Ile Asp Gly Leu Asn Ala
        515                 520                 525

Thr Thr Glu Lys Leu Asp Thr Arg Leu Ala Ser Ala Glu Lys Ser Ile
    530                 535                 540

Ala Asp His Asp Thr Arg Leu Asn Gly Leu Asp Lys Thr Val Ser Asp
545                 550                 555                 560

Leu Arg Lys Glu Thr Arg Gln Gly Leu Ala Glu Gln Ala Ala Leu Ser
                565                 570                 575

Gly Leu Phe Gln Pro Tyr Asn Val Gly Leu Glu His His His His His
            580                 585                 590

His
```

<210> 31
<211> 3939
<212> DNA
<213> Artificial Sequence

<220>
<223> deltaG741-983

<400> 31

```
atggtcgccg ccgacatcgg tgcggggctt gccgatgcac taaccgcacc gctcgaccat   60
aaagacaaag gtttgcagtc tttgacgctg gatcagtccg tcaggaaaaa cgagaaactg   120
aagctggcgg cacaaggtgc ggaaaaaact tatggaaacg gtgacagcct caatacgggc   180
aaattgaaga acgacaaggt cagccgtttc gactttatcc gccaaatcga agtggacggg   240
cagctcatta ccttggagag tggagagttc caagtataca aacaaagcca ttccgcctta   300
accgcctttc agaccgagca aatacaagat tcggagcatt ccgggaagat ggttgcgaaa   360
cgccagttca gaatcggcga catagcgggc gaacatacat cttttgacaa gcttcccgaa   420
ggcggcaggg cgacatatcg cgggacggcg ttcggttcag acgatgccgg cggaaaactg   480
acctacacca tagatttcgc cgccaagcag ggaaacggca aaatcgaaca tttgaaatcg   540
ccagaactca atgtcgacct ggccgccgcc gatatcaagc cggatggaaa acgccatgcc   600
gtcatcagcg gttccgtcct ttacaaccaa gccgagaaag gcagttactc cctcggtatc   660
```

```
tttggcggaa aagcccagga agttgccggc agcgcggaag tgaaaaccgt aaacggcata   720
cgccatatcg gccttgccgc caagcaactc gagggatccg gcggaggcgg cacttctgcg   780
cccgacttca atgcaggcgg taccggtatc ggcagcaaca gcagagcaac aacagcgaaa   840
tcagcagcag tatcttacgc cggtatcaag aacgaaatgt gcaaagacag aagcatgctc   900
tgtgccggtc gggatgacgt tgcggttaca gacagggatg ccaaaatcaa tgcccccccc   960
ccgaatctgc ataccggaga ctttccaaac ccaaatgacg catacaagaa tttgatcaac   1020
ctcaaacctg caattgaagc aggctataca ggacgcgggg tagaggtagg tatcgtcgac   1080
acaggcgaat ccgtcggcag catatccttt cccgaactgt atggcagaaa agaacacggc   1140
tataacgaaa attacaaaaa ctatacggcg tatatgcgga aggaagcgcc tgaagacgga   1200
ggcggtaaag acattgaagc ttctttcgac gatgaggccg ttatagagac tgaagcaaag   1260
ccgacggata tccgccacgt aaaagaaatc ggacacatcg atttggtctc ccatattatt   1320
ggcgggcgtt ccgtggacgg cagacctgca ggcggtattg cgcccgatgc gacgctacac   1380
ataatgaata cgaatgatga aaccaagaac gaaatgatgg ttgcagccat ccgcaatgca   1440
tgggtcaagc tgggcgaacg tggcgtgcgc atcgtcaata acagtttttgg aacaacatcg   1500
agggcaggca ctgccgacct tttccaaata gccaattcgg aggagcagta ccgccaagcg   1560
ttgctcgact attccggcgg tgataaaaca gacgagggta tccgcctgat gcaacagagc   1620
gattacggca acctgtccta ccacatccgt aataaaaaca tgcttttcat cttttcgaca   1680
ggcaatgacg cacaagctca gcccaacaca tatgccctat tgccatttta tgaaaaagac   1740
gctcaaaaag gcattatcac agtcgcaggc gtagaccgca gtggagaaaa gttcaaacgg   1800
gaaatgtatg gagaaccggg tacagaaccg cttgagtatg gctccaacca ttgcggaatt   1860
actgccatgt ggtgcctgtc ggcaccctat gaagcaagcg tccgtttcac ccgtacaaac   1920
ccgattcaaa ttgccggaac atccttttcc gcacccatcg taaccggcac ggcggctctg   1980
ctgctgcaga aatacccgtg gatgagcaac gacaacctgc gtaccacgtt gctgacgacg   2040
gctcaggaca tcggtgcagt cggcgtggac agcaagttcg gctggggact gctggatgcg   2100
ggtaaggcca tgaacggacc cgcgtccttt ccgttcggcg actttaccgc cgatacgaaa   2160
ggtacatccg atattgccta ctccttccgt aacgacattt caggcacggg cggcctgatc   2220
aaaaaaggcg gcagccaact gcaactgcac ggcaacaaca cctatacggg caaaaccatt   2280
atcgaaggcg gttcgctggt gttgtacggc aacaacaaat cggatatgcg cgtcgaaacc   2340
aaaggtgcgc tgatttataa cggggcggca tccggcggca gcctgaacag cgacggcatt   2400
gtctatctgg cagataccga ccaatccggc gcaaacgaaa ccgtacacat caaaggcagt   2460
ctgcagctgg acggcaaagg tacgctgtac acacgtttgg gcaaactgct gaaagtggac   2520
ggtacggcga ttatcggcgg caagctgtac atgtcggcac gcggcaaggg ggcaggctat   2580
ctcaacagta ccggacgacg tgttcccttc ctgagtgccg ccaaaatcgg gcaggattat   2640
tctttcttca caaacatcga aaccgacggc ggcctgctgg cttccctcga cagcgtcgaa   2700
aaaacagcgg gcagtgaagg cgacacgctg tcctattatg tccgtcgcgg caatgcggca   2760
cggactgctt cggcagcggc acattccgcg cccgccggtc tgaaacacgc cgtagaacag   2820
ggcggcagca atctggaaaa cctgatggtc gaactggatg cctccgaatc atccgcaaca   2880
cccgagacgg ttgaaactgc ggcagccgac cgcacagata tgccgggcat ccgcccctac   2940
ggcgcaactt tccgcgcagc ggcagccgta cagcatgcga atgccgccga cggtgtacgc   3000
atcttcaaca gtctcgccgc taccgtctat gccgacagta ccgccgccca tgccgatatg   3060
cagggacgcc gcctgaaagc cgtatcggac gggttggacc acaacggcac gggtctgcgc   3120
gtcatcgcgc aaacccaaca ggacggtgga acgtgggaac agggcggtgt tgaaggcaaa   3180
atgcgcggca gtacccaaac cgtcggcatt gccgcgaaaa ccggcgaaaa tacgacagca   3240
gccgccacac tgggcatggg acgcagcaca tggagcgaaa acagtgcaaa tgcaaaaacc   3300
gacagcatta gtctgtttgc aggcatacgg cacgatgcgg gcgatatcgg ctatctcaaa   3360
ggcctgttct cctacggacg ctacaaaaac agcatcagcc gcagcaccgg tgcggacgaa   3420
catgcggaag gcagcgtcaa cggcacgctg atgcagctgg gcgcactggg cggtgtcaac   3480
gttccgtttg ccgcaacggg agatttgacg gtcgaaggcg gtctgcgcta cgacctgctc   3540
aaacaggatg cattcgccga aaaaggcagt gctttgggct ggagcggcaa cagcctcact   3600
gaaggcacgc tggtcggact cgcgggtctg aagctgtcgc aacccttgag cgataaagcc   3660
gtcctgtttg caacggcggg cgtggaacgc gacctgaacg gacgcgacta cacggtaacg   3720
ggcggcttta ccggcgcgac tgcagcaacc ggcaagacgg gggcacgcaa tatgccgcac   3780
acccgtctgg ttgccggcct gggcgcggat gtcgaattcg gcaacggctg gaacggcttg   3840
gcacgttaca gctacgccgg ttccaaacag tacggcaacc acagcggacg agtcggcgta   3900
ggctaccggt tcctcgagca ccaccaccac caccactga                          3939
```

<210> 32
<211> 1312
<212> PRT

<213> Artificial Sequence <220>

<223> deltaG741-983

<400> 32

```
Met Val Ala Ala Asp Ile Gly Ala Gly Leu Ala Asp Ala Leu Thr Ala
1               5                   10                  15

Pro Leu Asp His Lys Asp Lys Gly Leu Gln Ser Leu Thr Leu Asp Gln
            20                  25                  30

Ser Val Arg Lys Asn Glu Lys Leu Lys Leu Ala Ala Gln Gly Ala Glu
        35                  40                  45

Lys Thr Tyr Gly Asn Gly Asp Ser Leu Asn Thr Gly Lys Leu Lys Asn
    50                  55                  60

Asp Lys Val Ser Arg Phe Asp Phe Ile Arg Gln Ile Glu Val Asp Gly
65                  70                  75                  80

Gln Leu Ile Thr Leu Glu Ser Gly Glu Phe Gln Val Tyr Lys Gln Ser
                85                  90                  95

His Ser Ala Leu Thr Ala Phe Gln Thr Glu Gln Ile Gln Asp Ser Glu
            100                 105                 110

His Ser Gly Lys Met Val Ala Lys Arg Gln Phe Arg Ile Gly Asp Ile
        115                 120                 125

Ala Gly Glu His Thr Ser Phe Asp Lys Leu Pro Glu Gly Gly Arg Ala
    130                 135                 140

Thr Tyr Arg Gly Thr Ala Phe Gly Ser Asp Asp Ala Gly Gly Lys Leu
145                 150                 155                 160

Thr Tyr Thr Ile Asp Phe Ala Ala Lys Gln Gly Asn Gly Lys Ile Glu
                165                 170                 175

His Leu Lys Ser Pro Glu Leu Asn Val Asp Leu Ala Ala Ala Asp Ile
            180                 185                 190

Lys Pro Asp Gly Lys Arg His Ala Val Ile Ser Gly Ser Val Leu Tyr
        195                 200                 205

Asn Gln Ala Glu Lys Gly Ser Tyr Ser Leu Gly Ile Phe Gly Gly Lys
    210                 215                 220

Ala Gln Glu Val Ala Gly Ser Ala Glu Val Lys Thr Val Asn Gly Ile
225                 230                 235                 240

Arg His Ile Gly Leu Ala Ala Lys Gln Leu Glu Gly Ser Gly Gly Gly
                245                 250                 255

Gly Thr Ser Ala Pro Asp Phe Asn Ala Gly Gly Thr Gly Ile Gly Ser
            260                 265                 270

Asn Ser Arg Ala Thr Thr Ala Lys Ser Ala Ala Val Ser Tyr Ala Gly
        275                 280                 285

Ile Lys Asn Glu Met Cys Lys Asp Arg Ser Met Leu Cys Ala Gly Arg
    290                 295                 300
```

```
Asp Asp Val Ala Val Thr Asp Arg Asp Ala Lys Ile Asn Ala Pro Pro
305                 310                 315                 320

Pro Asn Leu His Thr Gly Asp Phe Pro Asn Pro Asn Asp Ala Tyr Lys
                325                 330                 335

Asn Leu Ile Asn Leu Lys Pro Ala Ile Glu Ala Gly Tyr Thr Gly Arg
            340                 345                 350

Gly Val Glu Val Gly Ile Val Asp Thr Gly Glu Ser Val Gly Ser Ile
        355                 360                 365

Ser Phe Pro Glu Leu Tyr Gly Arg Lys Glu His Gly Tyr Asn Glu Asn
    370                 375                 380

Tyr Lys Asn Tyr Thr Ala Tyr Met Arg Lys Glu Ala Pro Glu Asp Gly
385                 390                 395                 400

Gly Gly Lys Asp Ile Glu Ala Ser Phe Asp Asp Glu Ala Val Ile Glu
                405                 410                 415

Thr Glu Ala Lys Pro Thr Asp Ile Arg His Val Lys Glu Ile Gly His
            420                 425                 430

Ile Asp Leu Val Ser His Ile Ile Gly Gly Arg Ser Val Asp Gly Arg
        435                 440                 445

Pro Ala Gly Gly Ile Ala Pro Asp Ala Thr Leu His Ile Met Asn Thr
    450                 455                 460

Asn Asp Glu Thr Lys Asn Glu Met Met Val Ala Ala Ile Arg Asn Ala
465                 470                 475                 480

Trp Val Lys Leu Gly Glu Arg Gly Val Arg Ile Val Asn Asn Ser Phe
                485                 490                 495

Gly Thr Thr Ser Arg Ala Gly Thr Ala Asp Leu Phe Gln Ile Ala Asn
            500                 505                 510

Ser Glu Glu Gln Tyr Arg Gln Ala Leu Leu Asp Tyr Ser Gly Gly Asp
        515                 520                 525

Lys Thr Asp Glu Gly Ile Arg Leu Met Gln Gln Ser Asp Tyr Gly Asn
    530                 535                 540

Leu Ser Tyr His Ile Arg Asn Lys Asn Met Leu Phe Ile Phe Ser Thr
545                 550                 555                 560

Gly Asn Asp Ala Gln Ala Gln Pro Asn Thr Tyr Ala Leu Leu Pro Phe
                565                 570                 575

Tyr Glu Lys Asp Ala Gln Lys Gly Ile Ile Thr Val Ala Gly Val Asp
            580                 585                 590

Arg Ser Gly Glu Lys Phe Lys Arg Glu Met Tyr Gly Glu Pro Gly Thr
        595                 600                 605

Glu Pro Leu Glu Tyr Gly Ser Asn His Cys Gly Ile Thr Ala Met Trp
    610                 615                 620

Cys Leu Ser Ala Pro Tyr Glu Ala Ser Val Arg Phe Thr Arg Thr Asn
```

```
625                 630                 635                 640

Pro Ile Gln Ile Ala Gly Thr Ser Phe Ser Ala Pro Ile Val Thr Gly
                645                 650                 655

Thr Ala Ala Leu Leu Leu Gln Lys Tyr Pro Trp Met Ser Asn Asp Asn
            660                 665                 670

Leu Arg Thr Thr Leu Leu Thr Thr Ala Gln Asp Ile Gly Ala Val Gly
        675                 680                 685

Val Asp Ser Lys Phe Gly Trp Gly Leu Leu Asp Ala Gly Lys Ala Met
    690                 695                 700

Asn Gly Pro Ala Ser Phe Pro Phe Gly Asp Phe Thr Ala Asp Thr Lys
705                 710                 715                 720

Gly Thr Ser Asp Ile Ala Tyr Ser Phe Arg Asn Asp Ile Ser Gly Thr
                725                 730                 735

Gly Gly Leu Ile Lys Lys Gly Gly Ser Gln Leu Gln Leu His Gly Asn
            740                 745                 750

Asn Thr Tyr Thr Gly Lys Thr Ile Ile Glu Gly Gly Ser Leu Val Leu
        755                 760                 765

Tyr Gly Asn Asn Lys Ser Asp Met Arg Val Glu Thr Lys Gly Ala Leu
    770                 775                 780

Ile Tyr Asn Gly Ala Ala Ser Gly Gly Ser Leu Asn Ser Asp Gly Ile
785                 790                 795                 800

Val Tyr Leu Ala Asp Thr Asp Gln Ser Gly Ala Asn Glu Thr Val His
                805                 810                 815

Ile Lys Gly Ser Leu Gln Leu Asp Gly Lys Gly Thr Leu Tyr Thr Arg
            820                 825                 830

Leu Gly Lys Leu Leu Lys Val Asp Gly Thr Ala Ile Ile Gly Gly Lys
        835                 840                 845

Leu Tyr Met Ser Ala Arg Gly Lys Gly Ala Gly Tyr Leu Asn Ser Thr
    850                 855                 860

Gly Arg Arg Val Pro Phe Leu Ser Ala Ala Lys Ile Gly Gln Asp Tyr
865                 870                 875                 880

Ser Phe Phe Thr Asn Ile Glu Thr Asp Gly Gly Leu Leu Ala Ser Leu
                885                 890                 895

Asp Ser Val Glu Lys Thr Ala Gly Ser Glu Gly Asp Thr Leu Ser Tyr
            900                 905                 910

Tyr Val Arg Arg Gly Asn Ala Ala Arg Thr Ala Ser Ala Ala Ala His
        915                 920                 925

Ser Ala Pro Ala Gly Leu Lys His Ala Val Glu Gln Gly Gly Ser Asn
    930                 935                 940

Leu Glu Asn Leu Met Val Glu Leu Asp Ala Ser Glu Ser Ser Ala Thr
945                 950                 955                 960
```

```
Pro Glu Thr Val Glu Thr Ala Ala Ala Asp Arg Thr Asp Met Pro Gly
                965                 970                 975

Ile Arg Pro Tyr Gly Ala Thr Phe Arg Ala Ala Ala Ala Val Gln His
            980                 985                 990

Ala Asn Ala Ala Asp Gly Val Arg Ile Phe Asn Ser Leu Ala Ala Thr
        995                 1000                1005

Val Tyr Ala Asp Ser Thr Ala Ala His Ala Asp Met Gln Gly Arg Arg
    1010                1015                1020

Leu Lys Ala Val Ser Asp Gly Leu Asp His Asn Gly Thr Gly Leu Arg
1025                1030                1035                1040

Val Ile Ala Gln Thr Gln Gln Asp Gly Gly Thr Trp Glu Gln Gly Gly
                1045                1050                1055

Val Glu Gly Lys Met Arg Gly Ser Thr Gln Thr Val Gly Ile Ala Ala
            1060                1065                1070

Lys Thr Gly Glu Asn Thr Thr Ala Ala Ala Thr Leu Gly Met Gly Arg
        1075                1080                1085

Ser Thr Trp Ser Glu Asn Ser Ala Asn Ala Lys Thr Asp Ser Ile Ser
    1090                1095                1100

Leu Phe Ala Gly Ile Arg His Asp Ala Gly Asp Ile Gly Tyr Leu Lys
1105                1110                1115                1120

Gly Leu Phe Ser Tyr Gly Arg Tyr Lys Asn Ser Ile Ser Arg Ser Thr
                1125                1130                1135

Gly Ala Asp Glu His Ala Glu Gly Ser Val Asn Gly Thr Leu Met Gln
            1140                1145                1150

Leu Gly Ala Leu Gly Gly Val Asn Val Pro Phe Ala Ala Thr Gly Asp
        1155                1160                1165

Leu Thr Val Glu Gly Gly Leu Arg Tyr Asp Leu Leu Lys Gln Asp Ala
    1170                1175                1180

Phe Ala Glu Lys Gly Ser Ala Leu Gly Trp Ser Gly Asn Ser Leu Thr
1185                1190                1195                1200

Glu Gly Thr Leu Val Gly Leu Ala Gly Leu Lys Leu Ser Gln Pro Leu
                1205                1210                1215

Ser Asp Lys Ala Val Leu Phe Ala Thr Ala Gly Val Glu Arg Asp Leu
            1220                1225                1230

Asn Gly Arg Asp Tyr Thr Val Thr Gly Gly Phe Thr Gly Ala Thr Ala
        1235                1240                1245

Ala Thr Gly Lys Thr Gly Ala Arg Asn Met Pro His Thr Arg Leu Val
    1250                1255                1260

Ala Gly Leu Gly Ala Asp Val Glu Phe Gly Asn Gly Trp Asn Gly Leu
1265                1270                1275                1280
```

```
Ala Arg Tyr Ser Tyr Ala Gly Ser Lys Gln Tyr Gly Asn His Ser Gly
                1285                1290                1295

Arg Val Gly Val Gly Tyr Arg Phe Leu Glu His His His His His His
            1300                1305                1310
```

<210> 33
<211> 2028
<212> DNA
<213> Artificial Sequence

<220>
<223> deltaG741-ORF46.1

<400> 33

```
atggtcgccg ccgacatcgg tgcggggctt gccgatgcac taaccgcacc gctcgaccat   60
aaagacaaag gtttgcagtc tttgacgctg gatcagtccg tcaggaaaaa cgagaaactg   120
aagctggcgg cacaaggtgc ggaaaaaact tatggaaacg gtgacagcct caatacgggc   180
aaattgaaga acgacaaggt cagccgtttc gactttatcc gccaaatcga agtggacggg   240
cagctcatta ccttggagag tggagagttc caagtataca aacaaagcca ttccgcctta   300
accgcctttc agaccgagca aatacaagat tcggagcatt ccgggaagat ggttgcgaaa   360
cgccagttca gaatcggcga catagcgggc gaacatacat cttttgacaa gcttcccgaa   420
ggcggcaggg cgacatatcg cgggacggcg ttcggttcag acgatgccgg cggaaaactg   480
acctacacca tagatttcgc cgccaagcag ggaaacggca aaatcgaaca tttgaaatcg   540
ccagaactca atgtcgacct ggccgccgcc gatatcaagc cggatggaaa acgccatgcc   600
gtcatcagcg gttccgtcct ttacaaccaa gccgagaaag gcagttactc cctcggtatc   660
tttggcggaa aagcccagga agttgccggc agcgcggaag tgaaaaccgt aaacggcata   720
cgccatatcg gccttgccgc caagcaactc gacggtggcg gaggcactgg atcctcagat   780
ttggcaaacg attcttttat ccggcaggtt ctcgaccgtc agcatttcga acccgacggg   840
aaataccacc tattcggcag caggggggaa cttgccgagc gcagcggcca tatcggattg   900
ggaaaaatac aaagccatca gttgggcaac ctgatgattc aacaggcggc cattaaagga   960
aatatcggct acattgtccg cttttccgat cacgggcacg aagtccattc cccttcgac   1020
aaccatgcct cacattccga ttctgatgaa gccggtagtc ccgttgacgg atttagcctt   1080
taccgcatcc attgggacgg atacgaacac catcccgccg acggctatga cgggccacag   1140
ggcggcggct atcccgctcc caaaggcgcg agggatatat acagctacga cataaaaggc   1200
gttgcccaaa atatccgcct caacctgacc gacaaccgca gcaccggaca acggcttgcc   1260
gaccgtttcc acaatgccgg tagtatgctg acgcaaggag taggcgacgg attcaaacgc   1320
gccacccgat acagccccga gctggacaga tcgggcaatg ccgccgaagc cttcaacggc   1380
actgcagata tcgttaaaaa catcatcggc gcggcaggag aaattgtcgg cgcaggcgat   1440
gccgtgcagg gcataagcga aggctcaaac attgctgtca tgcacggctt gggtctgctt   1500
tccaccgaaa acaagatggc gcgcatcaac gatttggcag atatggcgca actcaaagac   1560
tatgccgcag cagccatccg cgattgggca gtccaaaacc ccaatgccgc acaaggcata   1620
gaagccgtca gcaatatctt tatggcagcc atccccatca aagggattgg agctgttcgg   1680
ggaaaatacg gcttgggcgg catcacggca catcctatca agcggtcgca gatgggcgcg   1740
atcgcattgc cgaaagggaa atccgccgtc agcgacaatt ttgccgatgc ggcatacgcc   1800
aaatacccgt ccccttacca ttcccgaaat atccgttcaa acttggagca gcgttacggc   1860
aaagaaaaca tcacctcctc aaccgtgccg ccgtcaaacg gcaaaaatgt caaactggca   1920
gaccaacgcc acccgaagac aggcgtaccg tttgacggta aagggtttcc gaattttgag   1980
aagcacgtga aatatgatac gctcgagcac caccaccacc accactga                 2028
```

<210> 34
<211> 675
<212> PRT
<213> Artificial Sequence

<220>
<223> deltaG741-ORF46.1

<400> 34

Met Val Ala Ala Asp Ile Gly Ala Gly Leu Ala Asp Ala Leu Thr Ala

```
1               5                   10                  15
Pro Leu Asp His Lys Asp Lys Gly Leu Gln Ser Leu Thr Leu Asp Gln
            20                  25                  30
Ser Val Arg Lys Asn Glu Lys Leu Lys Leu Ala Ala Gln Gly Ala Glu
        35                  40                  45
Lys Thr Tyr Gly Asn Gly Asp Ser Leu Asn Thr Gly Lys Leu Lys Asn
    50                  55                  60
Asp Lys Val Ser Arg Phe Asp Phe Ile Arg Gln Ile Glu Val Asp Gly
65                  70                  75                  80
Gln Leu Ile Thr Leu Glu Ser Gly Glu Phe Gln Val Tyr Lys Gln Ser
                85                  90                  95
His Ser Ala Leu Thr Ala Phe Gln Thr Glu Gln Ile Gln Asp Ser Glu
            100                 105                 110
His Ser Gly Lys Met Val Ala Lys Arg Gln Phe Arg Ile Gly Asp Ile
        115                 120                 125
Ala Gly Glu His Thr Ser Phe Asp Lys Leu Pro Glu Gly Gly Arg Ala
    130                 135                 140
Thr Tyr Arg Gly Thr Ala Phe Gly Ser Asp Asp Ala Gly Gly Lys Leu
145                 150                 155                 160
Thr Tyr Thr Ile Asp Phe Ala Ala Lys Gln Gly Asn Gly Lys Ile Glu
                165                 170                 175
His Leu Lys Ser Pro Glu Leu Asn Val Asp Leu Ala Ala Ala Asp Ile
            180                 185                 190
Lys Pro Asp Gly Lys Arg His Ala Val Ile Ser Gly Ser Val Leu Tyr
        195                 200                 205
Asn Gln Ala Glu Lys Gly Ser Tyr Ser Leu Gly Ile Phe Gly Gly Lys
    210                 215                 220
Ala Gln Glu Val Ala Gly Ser Ala Glu Val Lys Thr Val Asn Gly Ile
225                 230                 235                 240
Arg His Ile Gly Leu Ala Ala Lys Gln Leu Asp Gly Gly Gly Gly Thr
                245                 250                 255
Gly Ser Ser Asp Leu Ala Asn Asp Ser Phe Ile Arg Gln Val Leu Asp
            260                 265                 270
Arg Gln His Phe Glu Pro Asp Gly Lys Tyr His Leu Phe Gly Ser Arg
        275                 280                 285
Gly Glu Leu Ala Glu Arg Ser Gly His Ile Gly Leu Gly Lys Ile Gln
    290                 295                 300
Ser His Gln Leu Gly Asn Leu Met Ile Gln Gln Ala Ala Ile Lys Gly
305                 310                 315                 320
Asn Ile Gly Tyr Ile Val Arg Phe Ser Asp His Gly His Glu Val His
                325                 330                 335
```

```
Ser Pro Phe Asp Asn His Ala Ser His Ser Asp Ser Asp Glu Ala Gly
            340                 345                 350

Ser Pro Val Asp Gly Phe Ser Leu Tyr Arg Ile His Trp Asp Gly Tyr
        355                 360                 365

Glu His His Pro Ala Asp Gly Tyr Asp Gly Pro Gln Gly Gly Gly Tyr
    370                 375                 380

Pro Ala Pro Lys Gly Ala Arg Asp Ile Tyr Ser Tyr Asp Ile Lys Gly
385                 390                 395                 400

Val Ala Gln Asn Ile Arg Leu Asn Leu Thr Asp Asn Arg Ser Thr Gly
                405                 410                 415

Gln Arg Leu Ala Asp Arg Phe His Asn Ala Gly Ser Met Leu Thr Gln
            420                 425                 430

Gly Val Gly Asp Gly Phe Lys Arg Ala Thr Arg Tyr Ser Pro Glu Leu
        435                 440                 445

Asp Arg Ser Gly Asn Ala Ala Glu Ala Phe Asn Gly Thr Ala Asp Ile
    450                 455                 460

Val Lys Asn Ile Ile Gly Ala Ala Gly Glu Ile Val Gly Ala Gly Asp
465                 470                 475                 480

Ala Val Gln Gly Ile Ser Glu Gly Ser Asn Ile Ala Val Met His Gly
                485                 490                 495

Leu Gly Leu Leu Ser Thr Glu Asn Lys Met Ala Arg Ile Asn Asp Leu
            500                 505                 510

Ala Asp Met Ala Gln Leu Lys Asp Tyr Ala Ala Ala Ala Ile Arg Asp
        515                 520                 525

Trp Ala Val Gln Asn Pro Asn Ala Ala Gln Gly Ile Glu Ala Val Ser
    530                 535                 540

Asn Ile Phe Met Ala Ala Ile Pro Ile Lys Gly Ile Gly Ala Val Arg
545                 550                 555                 560

Gly Lys Tyr Gly Leu Gly Gly Ile Thr Ala His Pro Ile Lys Arg Ser
                565                 570                 575

Gln Met Gly Ala Ile Ala Leu Pro Lys Gly Lys Ser Ala Val Ser Asp
            580                 585                 590

Asn Phe Ala Asp Ala Ala Tyr Ala Lys Tyr Pro Ser Pro Tyr His Ser
        595                 600                 605

Arg Asn Ile Arg Ser Asn Leu Glu Gln Arg Tyr Gly Lys Glu Asn Ile
    610                 615                 620

Thr Ser Ser Thr Val Pro Pro Ser Asn Gly Lys Asn Val Lys Leu Ala
625                 630                 635                 640

Asp Gln Arg His Pro Lys Thr Gly Val Pro Phe Asp Gly Lys Gly Phe
                645                 650                 655
```

```
Pro Asn Phe Glu Lys His Val Lys Tyr Asp Thr Leu Glu His His His
            660                 665                 670

His His His
        675
```

<210> 35
<211> 2019
<212> DNA
<213> Artificial Sequence

<220>
<223> ORF46.1-741

<400> 35

```
atgtcagatt tggcaaacga ttcttttatc cggcaggttc tcgaccgtca gcatttcgaa    60
cccgacggga aataccacct attcggcagc aggggggaac ttgccgagcg cagcggccat   120
atcggattgg gaaaaataca aagccatcag ttgggcaacc tgatgattca acaggcggcc   180
attaaaggaa atatcggcta cattgtccgc ttttccgatc acgggcacga agtccattcc   240
cccttcgaca accatgcctc acattccgat tctgatgaag ccggtagtcc cgttgacgga   300
tttagccttt accgcatcca ttgggacgga tacgaacacc atcccgccga cggctatgac   360
gggccacagg gcggcggcta tcccgctccc aaaggcgcga gggatatata cagctacgac   420
ataaaaggcg ttgcccaaaa tatccgcctc aacctgaccg acaaccgcag caccggacaa   480
cggcttgccg accgtttcca caatgccggt agtatgctga cgcaaggagt aggcgacgga   540
ttcaaacgcg ccacccgata cagccccgag ctggacagat cgggcaatgc cgccgaagcc   600
ttcaacggca ctgcagatat cgttaaaaac atcatcggcg cggcaggaga aattgtcggc   660
gcaggcgatg ccgtgcaggg cataagcgaa ggctcaaaca ttgctgtcat gcacggcttg   720
ggtctgcttt ccaccgaaaa caagatggcg cgcatcaacg atttggcaga tatggcgcaa   780
ctcaaagact atgccgcagc agccatccgc gattgggcag tccaaaaccc caatgccgca   840
caaggcatag aagccgtcag caatatcttt atggcagcca tccccatcaa agggattgga   900
gctgttcggg gaaaatacgg cttgggcggc atcacggcac atcctatcaa gcggtcgcag   960
atgggcgcga tcgcattgcc gaaagggaaa tccgccgtca gcgacaattt tgccgatgcg  1020
gcatacgcca aatacccgtc cccttaccat tcccgaaata tccgttcaaa cttggagcag  1080
cgttacggca aagaaaacat cacctcctca accgtgccgc cgtcaaacgg caaaaatgtc  1140
aaactggcag accaacgcca cccgaagaca ggcgtaccgt ttgacggtaa agggtttccg  1200
aattttgaga agcacgtgaa atatgatacg ggatccggag ggggtggtgt cgccgccgac  1260
atcggtgcgg ggcttgccga tgcactaacc gcaccgctcg accataaaga caaaggtttg  1320
cagtctttga cgctggatca gtccgtcagg aaaaacgaga aactgaagct ggcggcacaa  1380
ggtgcggaaa aaacttatgg aaacggtgac agcctcaata cgggcaaatt gaagaacgac  1440
aaggtcagcc gtttcgactt tatccgccaa atcgaagtgg acgggcagct cattaccttg  1500
gagagtggag agttccaagt atacaaacaa agccattccg ccttaaccgc ctttcagacc  1560
gagcaaatac aagattcgga gcattccggg aagatggttg cgaaacgcca gttcagaatc  1620
ggcgacatag cgggcgaaca tacatctttt gacaagcttc ccgaaggcgg cagggcgaca  1680
tatcgcggga cggcgttcgg ttcagacgat gccggcggaa aactgaccta caccatagat  1740
ttcgccgcca agcagggaaa cggcaaaatc gaacatttga aatcgccaga actcaatgtc  1800
gacctggccg ccgccgatat caagccggat ggaaaacgcc atgccgtcat cagcggttcc  1860
gtcctttaca accaagccga gaaaggcagt tactccctcg gtatctttgg cggaaaagcc  1920
caggaagttg ccggcagcgc ggaagtgaaa accgtaaacg gcatacgcca tatcggcctt  1980
gccgccaagc aactcgagca ccaccaccac caccactga                          2019
```

<210> 36
<211> 672
<212> PRT
<213> Artificial Sequence

<220>

<223> ORF46.1-791

<400> 36

Met Ser Asp Leu Ala Asn Asp Ser Phe Ile Arg Gln Val Leu Asp Arg
1 5 10 15

```
Gln His Phe Glu Pro Asp Gly Lys Tyr His Leu Phe Gly Ser Arg Gly
            20                  25                  30

Glu Leu Ala Glu Arg Ser Gly His Ile Gly Leu Gly Lys Ile Gln Ser
        35                  40                  45

His Gln Leu Gly Asn Leu Met Ile Gln Gln Ala Ala Ile Lys Gly Asn
    50                  55                  60

Ile Gly Tyr Ile Val Arg Phe Ser Asp His Gly His Glu Val His Ser
65                  70                  75                  80

Pro Phe Asp Asn His Ala Ser His Ser Asp Ser Asp Glu Ala Gly Ser
                85                  90                  95

Pro Val Asp Gly Phe Ser Leu Tyr Arg Ile His Trp Asp Gly Tyr Glu
            100                 105                 110

His His Pro Ala Asp Gly Tyr Asp Gly Pro Gln Gly Gly Gly Tyr Pro
        115                 120                 125

Ala Pro Lys Gly Ala Arg Asp Ile Tyr Ser Tyr Asp Ile Lys Gly Val
    130                 135                 140

Ala Gln Asn Ile Arg Leu Asn Leu Thr Asp Asn Arg Ser Thr Gly Gln
145                 150                 155                 160

Arg Leu Ala Asp Arg Phe His Asn Ala Gly Ser Met Leu Thr Gln Gly
                165                 170                 175

Val Gly Asp Gly Phe Lys Arg Ala Thr Arg Tyr Ser Pro Glu Leu Asp
            180                 185                 190

Arg Ser Gly Asn Ala Ala Glu Ala Phe Asn Gly Thr Ala Asp Ile Val
        195                 200                 205

Lys Asn Ile Ile Gly Ala Ala Gly Glu Ile Val Gly Ala Gly Asp Ala
    210                 215                 220

Val Gln Gly Ile Ser Glu Gly Ser Asn Ile Ala Val Met His Gly Leu
225                 230                 235                 240

Gly Leu Leu Ser Thr Glu Asn Lys Met Ala Arg Ile Asn Asp Leu Ala
                245                 250                 255

Asp Met Ala Gln Leu Lys Asp Tyr Ala Ala Ala Ala Ile Arg Asp Trp
            260                 265                 270

Ala Val Gln Asn Pro Asn Ala Ala Gln Gly Ile Glu Ala Val Ser Asn
        275                 280                 285

Ile Phe Met Ala Ala Ile Pro Ile Lys Gly Ile Gly Ala Val Arg Gly
    290                 295                 300

Lys Tyr Gly Leu Gly Gly Ile Thr Ala His Pro Ile Lys Arg Ser Gln
305                 310                 315                 320

Met Gly Ala Ile Ala Leu Pro Lys Gly Lys Ser Ala Val Ser Asp Asn
                325                 330                 335
```

Phe Ala Asp Ala Ala Tyr Ala Lys Tyr Pro Ser Pro Tyr His Ser Arg
340 345 350

Asn Ile Arg Ser Asn Leu Glu Gln Arg Tyr Gly Lys Glu Asn Ile Thr
355 360 365

Ser Ser Thr Val Pro Pro Ser Asn Gly Lys Asn Val Lys Leu Ala Asp
370 375 380

Gln Arg His Pro Lys Thr Gly Val Pro Phe Asp Gly Lys Gly Phe Pro
385 390 395 400

Asn Phe Glu Lys His Val Lys Tyr Asp Thr Gly Ser Gly Gly Gly Gly
405 410 415

Val Ala Ala Asp Ile Gly Ala Gly Leu Ala Asp Ala Leu Thr Ala Pro
420 425 430

Leu Asp His Lys Asp Lys Gly Leu Gln Ser Leu Thr Leu Asp Gln Ser
435 440 445

Val Arg Lys Asn Glu Lys Leu Lys Leu Ala Ala Gln Gly Ala Glu Lys
450 455 460

Thr Tyr Gly Asn Gly Asp Ser Leu Asn Thr Gly Lys Leu Lys Asn Asp
465 470 475 480

Lys Val Ser Arg Phe Asp Phe Ile Arg Gln Ile Glu Val Asp Gly Gln
485 490 495

Leu Ile Thr Leu Glu Ser Gly Glu Phe Gln Val Tyr Lys Gln Ser His
500 505 510

Ser Ala Leu Thr Ala Phe Gln Thr Glu Gln Ile Gln Asp Ser Glu His
515 520 525

Ser Gly Lys Met Val Ala Lys Arg Gln Phe Arg Ile Gly Asp Ile Ala
530 535 540

Gly Glu His Thr Ser Phe Asp Lys Leu Pro Glu Gly Gly Arg Ala Thr
545 550 555 560

Tyr Arg Gly Thr Ala Phe Gly Ser Asp Asp Ala Gly Gly Lys Leu Thr
565 570 575

Tyr Thr Ile Asp Phe Ala Ala Lys Gln Gly Asn Gly Lys Ile Glu His
580 585 590

Leu Lys Ser Pro Glu Leu Asn Val Asp Leu Ala Ala Ala Asp Ile Lys
595 600 605

Pro Asp Gly Lys Arg His Ala Val Ile Ser Gly Ser Val Leu Tyr Asn
610 615 620

Gln Ala Glu Lys Gly Ser Tyr Ser Leu Gly Ile Phe Gly Gly Lys Ala
625 630 635 640

Gln Glu Val Ala Gly Ser Ala Glu Val Lys Thr Val Asn Gly Ile Arg
645 650 655

His Ile Gly Leu Ala Ala Lys Gln Leu Glu His His His His His His

660 665 670

<210> 37
<211> 2421
<212> DNA
<213> Artificial Sequence

<220>
<223> ORF46.1-961

<400> 37

```
atgtcagatt tggcaaacga ttcttttatc cggcaggttc tcgaccgtca gcatttcgaa   60
cccgacggga aataccacct attcggcagc aggggggaac ttgccgagcg cagcggccat  120
atcggattgg gaaaaataca aagccatcag ttgggcaacc tgatgattca acaggcggcc  180
attaaaggaa atatcggcta cattgtccgc ttttccgatc acgggcacga agtccattcc  240
cccttcgaca accatgcctc acattccgat tctgatgaag ccggtagtcc cgttgacgga  300
tttagccttt accgcatcca ttgggacgga tacgaacacc atcccgccga cggctatgac  360
gggccacagg gcggcggcta tcccgctccc aaaggcgcga gggatatata cagctacgac  420
ataaaaggcg ttgcccaaaa tatccgcctc aacctgaccg acaaccgcag caccggacaa  480
cggcttgccg accgtttcca caatgccggt agtatgctga cgcaaggagt aggcgacgga  540
ttcaaacgcg ccacccgata cagccccgag ctggacagat cgggcaatgc cgccgaagcc  600
ttcaacggca ctgcagatat cgttaaaaac atcatcggcg cggcaggaga aattgtcggc  660
gcaggcgatg ccgtgcaggg cataagcgaa ggctcaaaca ttgctgtcat gcacggcttg  720
ggtctgcttt ccaccgaaaa caagatggcg cgcatcaacg atttggcaga tatggcgcaa  780
ctcaaagact atgccgcagc agccatccgc gattgggcag tccaaaaccc caatgccgca  840
caaggcatag aagccgtcag caatatcttt atggcagcca tccccatcaa agggattgga  900
gctgttcggg gaaaatacgg cttgggcggc atcacggcac atcctatcaa gcggtcgcag  960
atgggcgcga tcgcattgcc gaaagggaaa tccgccgtca gcgacaattt tgccgatgcg  1020
gcatacgcca aatacccgtc cccttaccat tcccgaaata tccgttcaaa cttggagcag  1080
cgttacggca aagaaaacat cacctcctca accgtgccgc cgtcaaacgg caaaaatgtc  1140
aaactggcag accaacgcca cccgaagaca ggcgtaccgt ttgacggtaa agggtttccg  1200
aattttgaga agcacgtgaa atatgatacg ggatccggag gaggaggagc cacaaacgac  1260
gacgatgtta aaaaagctgc cactgtggcc attgctgctg cctacaacaa tggccaagaa  1320
atcaacggtt tcaaagctgg agagaccatc tacgacattg atgaagacgg cacaattacc  1380
aaaaaagacg caactgcagc cgatgttgaa gccgacgact ttaaaggtct gggtctgaaa  1440
aaagtcgtga ctaacctgac caaaaccgtc aatgaaaaca aacaaaacgt cgatgccaaa  1500
gtaaaagctg cagaatctga aatagaaaag ttaacaacca agttagcaga cactgatgcc  1560
gctttagcag atactgatgc cgctctggat gcaaccacca acgccttgaa taaattggga  1620
gaaaatataa cgacatttgc tgaagagact aagacaaata tcgtaaaaat tgatgaaaaa  1680
ttagaagccg tggctgatac cgtcgacaag catgccgaag cattcaacga tatcgccgat  1740
tcattggatg aaaccaacac taaggcagac gaagccgtca aaaccgccaa tgaagccaaa  1800
cagacggccg aagaaaccaa acaaaacgtc gatgccaaag taaaagctgc agaaactgca  1860
gcaggcaaag ccgaagctgc cgctggcaca gctaatactg cagccgacaa ggccgaagct  1920
gtcgctgcaa aagttaccga catcaaagct gatatcgcta cgaacaaaga taatattgct  1980
aaaaaagcaa acagtgccga cgtgtacacc agagaagagt ctgacagcaa atttgtcaga  2040
attgatggtc tgaacgctac taccgaaaaa ttggacacac gcttggcttc tgctgaaaaa  2100
tccattgccg atcacgatac tcgcctgaac ggtttggata aaacagtgtc agacctgcgc  2160
aaagaaaccc gccaaggcct tgcagaacaa gccgcgctct ccggtctgtt ccaaccttac  2220
aacgtgggtc ggttcaatgt aacggctgca gtcggcggct acaaatccga atcggcagtc  2280
gccatcggta ccggcttccg ctttaccgaa aactttgccg ccaaagcagg cgtggcagtc  2340
ggcacttcgt ccggttcttc cgcagcctac catgtcggcg tcaattacga gtggctcgag  2400
caccaccacc accaccactg a                                            2421
```

<210> 38
<211> 806
<212> PRT

<213> Artificial Sequence

<220>
<223> ORF46.1-961

<400> 38

```
Met Ser Asp Leu Ala Asn Asp Ser Phe Ile Arg Gln Val Leu Asp Arg
1               5                   10                  15

Gln His Phe Glu Pro Asp Gly Lys Tyr His Leu Phe Gly Ser Arg Gly
            20                  25                  30

Glu Leu Ala Glu Arg Ser Gly His Ile Gly Leu Gly Lys Ile Gln Ser
        35                  40                  45

His Gln Leu Gly Asn Leu Met Ile Gln Gln Ala Ala Ile Lys Gly Asn
    50                  55                  60

Ile Gly Tyr Ile Val Arg Phe Ser Asp His Gly His Glu Val His Ser
65                  70                  75                  80

Pro Phe Asp Asn His Ala Ser His Ser Asp Ser Asp Glu Ala Gly Ser
                85                  90                  95

Pro Val Asp Gly Phe Ser Leu Tyr Arg Ile His Trp Asp Gly Tyr Glu
            100                 105                 110

His His Pro Ala Asp Gly Tyr Asp Gly Pro Gln Gly Gly Gly Tyr Pro
        115                 120                 125

Ala Pro Lys Gly Ala Arg Asp Ile Tyr Ser Tyr Asp Ile Lys Gly Val
    130                 135                 140

Ala Gln Asn Ile Arg Leu Asn Leu Thr Asp Asn Arg Ser Thr Gly Gln
145                 150                 155                 160

Arg Leu Ala Asp Arg Phe His Asn Ala Gly Ser Met Leu Thr Gln Gly
                165                 170                 175

Val Gly Asp Gly Phe Lys Arg Ala Thr Arg Tyr Ser Pro Glu Leu Asp
            180                 185                 190

Arg Ser Gly Asn Ala Ala Glu Ala Phe Asn Gly Thr Ala Asp Ile Val
        195                 200                 205

Lys Asn Ile Ile Gly Ala Ala Gly Glu Ile Val Gly Ala Gly Asp Ala
    210                 215                 220

Val Gln Gly Ile Ser Glu Gly Ser Asn Ile Ala Val Met His Gly Leu
225                 230                 235                 240

Gly Leu Leu Ser Thr Glu Asn Lys Met Ala Arg Ile Asn Asp Leu Ala
                245                 250                 255

Asp Met Ala Gln Leu Lys Asp Tyr Ala Ala Ala Ala Ile Arg Asp Trp
            260                 265                 270

Ala Val Gln Asn Pro Asn Ala Ala Gln Gly Ile Glu Ala Val Ser Asn
        275                 280                 285

Ile Phe Met Ala Ala Ile Pro Ile Lys Gly Ile Gly Ala Val Arg Gly
    290                 295                 300

Lys Tyr Gly Leu Gly Gly Ile Thr Ala His Pro Ile Lys Arg Ser Gln
305                 310                 315                 320
```

```
Met Gly Ala Ile Ala Leu Pro Lys Gly Lys Ser Ala Val Ser Asp Asn
                325                 330                 335

Phe Ala Asp Ala Ala Tyr Ala Lys Tyr Pro Ser Pro Tyr His Ser Arg
            340                 345                 350

Asn Ile Arg Ser Asn Leu Glu Gln Arg Tyr Gly Lys Glu Asn Ile Thr
        355                 360                 365

Ser Ser Thr Val Pro Pro Ser Asn Gly Lys Asn Val Lys Leu Ala Asp
    370                 375                 380

Gln Arg His Pro Lys Thr Gly Val Pro Phe Asp Gly Lys Gly Phe Pro
385                 390                 395                 400

Asn Phe Glu Lys His Val Lys Tyr Asp Thr Gly Ser Gly Gly Gly Gly
                405                 410                 415

Ala Thr Asn Asp Asp Asp Val Lys Lys Ala Ala Thr Val Ala Ile Ala
            420                 425                 430

Ala Ala Tyr Asn Asn Gly Gln Glu Ile Asn Gly Phe Lys Ala Gly Glu
        435                 440                 445

Thr Ile Tyr Asp Ile Asp Glu Asp Gly Thr Ile Thr Lys Lys Asp Ala
    450                 455                 460

Thr Ala Ala Asp Val Glu Ala Asp Asp Phe Lys Gly Leu Gly Leu Lys
465                 470                 475                 480

Lys Val Val Thr Asn Leu Thr Lys Thr Val Asn Glu Asn Lys Gln Asn
                485                 490                 495

Val Asp Ala Lys Val Lys Ala Ala Glu Ser Glu Ile Glu Lys Leu Thr
            500                 505                 510

Thr Lys Leu Ala Asp Thr Asp Ala Ala Leu Ala Asp Thr Asp Ala Ala
        515                 520                 525

Leu Asp Ala Thr Thr Asn Ala Leu Asn Lys Leu Gly Glu Asn Ile Thr
    530                 535                 540

Thr Phe Ala Glu Glu Thr Lys Thr Asn Ile Val Lys Ile Asp Glu Lys
545                 550                 555                 560

Leu Glu Ala Val Ala Asp Thr Val Asp Lys His Ala Glu Ala Phe Asn
                565                 570                 575

Asp Ile Ala Asp Ser Leu Asp Glu Thr Asn Thr Lys Ala Asp Glu Ala
            580                 585                 590

Val Lys Thr Ala Asn Glu Ala Lys Gln Thr Ala Glu Glu Thr Lys Gln
        595                 600                 605

Asn Val Asp Ala Lys Val Lys Ala Ala Glu Thr Ala Ala Gly Lys Ala
    610                 615                 620

Glu Ala Ala Ala Gly Thr Ala Asn Thr Ala Ala Asp Lys Ala Glu Ala
625                 630                 635                 640
```

Val Ala Ala Lys Val Thr Asp Ile Lys Ala Asp Ile Ala Thr Asn Lys
645 650 655

Asp Asn Ile Ala Lys Lys Ala Asn Ser Ala Asp Val Tyr Thr Arg Glu
660 665 670

Glu Ser Asp Ser Lys Phe Val Arg Ile Asp Gly Leu Asn Ala Thr Thr
675 680 685

Glu Lys Leu Asp Thr Arg Leu Ala Ser Ala Glu Lys Ser Ile Ala Asp
690 695 700

His Asp Thr Arg Leu Asn Gly Leu Asp Lys Thr Val Ser Asp Leu Arg
705 710 715 720

Lys Glu Thr Arg Gln Gly Leu Ala Glu Gln Ala Ala Leu Ser Gly Leu
725 730 735

Phe Gln Pro Tyr Asn Val Gly Arg Phe Asn Val Thr Ala Ala Val Gly
740 745 750

Gly Tyr Lys Ser Glu Ser Ala Val Ala Ile Gly Thr Gly Phe Arg Phe
755 760 765

Thr Glu Asn Phe Ala Ala Lys Ala Gly Val Ala Val Gly Thr Ser Ser
770 775 780

Gly Ser Ser Ala Ala Tyr His Val Gly Val Asn Tyr Glu Trp Leu Glu
785 790 795 800

His His His His His His
805

<210> 39
<211> 2256
<212> DNA
<213> Artificial Sequence

<220>
<223> ORF46.1-961c

<400> 39

```
atgtcagatt tggcaaacga ttcttttatc cggcaggttc tcgaccgtca gcatttcgaa    60
cccgacggga aataccacct attcggcagc aggggggaac ttgccgagcg cagcggccat   120
atcggattgg gaaaaataca aagccatcag ttgggcaacc tgatgattca acaggcggcc   180
attaaaggaa atatcggcta cattgtccgc ttttccgatc acgggcacga agtccattcc   240
cccttcgaca accatgcctc acattccgat tctgatgaag ccggtagtcc cgttgacgga   300
tttagccttt accgcatcca ttgggacgga tacgaacacc atcccgccga cggctatgac   360
gggccacagg gcggcggcta tcccgctccc aaaggcgcga gggatatata cagctacgac   420
ataaaaggcg ttgcccaaaa tatccgcctc aacctgaccg acaaccgcag caccggacaa   480
cggcttgccg accgtttcca caatgccggt agtatgctga cgcaaggagt aggcgacgga   540
ttcaaacgcg ccacccgata cagccccgag ctggacagat cgggcaatgc cgccgaagcc   600
ttcaacggca ctgcagatat cgttaaaaac atcatcggcg cggcaggaga aattgtcggc   660
gcaggcgatg ccgtgcaggg cataagcgaa ggctcaaaca ttgctgtcat gcacggcttg   720
ggtctgcttt ccaccgaaaa caagatggcg cgcatcaacg atttggcaga tatggcgcaa   780
ctcaaagact atgccgcagc agccatccgc gattgggcag tccaaaaccc caatgccgca   840
caaggcatag aagccgtcag caatatcttt atggcagcca tccccatcaa agggattgga   900
gctgttcggg gaaaatacgg cttgggcggc atcacggcac atcctatcaa gcggtcgcag   960
atgggcgcga tcgcattgcc gaaagggaaa tccgccgtca gcgacaattt tgccgatgcg  1020
gcatacgcca aatacccgtc cccttaccat tcccgaaata tccgttcaaa cttggagcag  1080
cgttacggca aagaaaacat cacctcctca accgtgccgc cgtcaaacgg caaaaatgtc  1140

aaactggcag accaacgcca cccgaagaca ggcgtaccgt ttgacggtaa agggtttccg  1200
aattttgaga agcacgtgaa atatgatacg ggatccggag gaggaggagc cacaaacgac  1260
gacgatgtta aaaaagctgc cactgtggcc attgctgctg cctacaacaa tggccaagaa  1320
atcaacggtt tcaaagctgg agagaccatc tacgacattg atgaagacgg cacaattacc  1380
aaaaaagacg caactgcagc cgatgttgaa gccgacgact ttaaaggtct gggtctgaaa  1440
aaagtcgtga ctaacctgac caaaaccgtc aatgaaaaca aacaaaacgt cgatgccaaa  1500
gtaaaagctg cagaatctga aatagaaaag ttaacaacca agttagcaga cactgatgcc  1560
gctttagcag atactgatgc cgctctggat gcaaccacca acgccttgaa taaattggga  1620
gaaaatataa cgacatttgc tgaagagact aagacaaata tcgtaaaaat tgatgaaaaa  1680
ttagaagccg tggctgatac cgtcgacaag catgccgaag cattcaacga tatcgccgat  1740
tcattggatg aaaccaacac taaggcagac gaagccgtca aaaccgccaa tgaagccaaa  1800
cagacggccg aagaaaccaa acaaaacgtc gatgccaaag taaaagctgc agaaactgca  1860
gcaggcaaag ccgaagctgc cgctggcaca gctaatactg cagccgacaa ggccgaagct  1920
gtcgctgcaa aagttaccga catcaaagct gatatcgcta cgaacaaaga taatattgct  1980
aaaaaagcaa acagtgccga cgtgtacacc agagaagagt ctgacagcaa atttgtcaga  2040
attgatggtc tgaacgctac taccgaaaaa ttggacacac gcttggcttc tgctgaaaaa  2100
tccattgccg atcacgatac tcgcctgaac ggtttggata aaacagtgtc agacctgcgc  2160
aaagaaaccc gccaaggcct tgcagaacaa gccgcgctct ccggtctgtt ccaaccttac  2220
aacgtgggtc tcgagcacca ccaccaccac cactga                             2256
```

<210> 40
<211> 751
<212> PRT
<213> Artificial Sequence

<220>
<223> ORF46.1-961c

<400> 40

Met Ser Asp Leu Ala Asn Asp Ser Phe Ile Arg Gln Val Leu Asp Arg
1 5 10 15

Gln His Phe Glu Pro Asp Gly Lys Tyr His Leu Phe Gly Ser Arg Gly
20 25 30

Glu Leu Ala Glu Arg Ser Gly His Ile Gly Leu Gly Lys Ile Gln Ser
35 40 45

His Gln Leu Gly Asn Leu Met Ile Gln Gln Ala Ala Ile Lys Gly Asn
50 55 60

Ile Gly Tyr Ile Val Arg Phe Ser Asp His Gly His Glu Val His Ser
65 70 75 80

Pro Phe Asp Asn His Ala Ser His Ser Asp Ser Asp Glu Ala Gly Ser
85 90 95

Pro Val Asp Gly Phe Ser Leu Tyr Arg Ile His Trp Asp Gly Tyr Glu
100 105 110

His His Pro Ala Asp Gly Tyr Asp Gly Pro Gln Gly Gly Gly Tyr Pro
115 120 125

Ala Pro Lys Gly Ala Arg Asp Ile Tyr Ser Tyr Asp Ile Lys Gly Val
130 135 140

Ala Gln Asn Ile Arg Leu Asn Leu Thr Asp Asn Arg Ser Thr Gly Gln
145 150 155 160

Arg Leu Ala Asp Arg Phe His Asn Ala Gly Ser Met Leu Thr Gln Gly
165 170 175

Val Gly Asp Gly Phe Lys Arg Ala Thr Arg Tyr Ser Pro Glu Leu Asp
180 185 190

Arg Ser Gly Asn Ala Ala Glu Ala Phe Asn Gly Thr Ala Asp Ile Val
195 200 205

Lys Asn Ile Ile Gly Ala Ala Gly Glu Ile Val Gly Ala Gly Asp Ala
210 215 220

Val Gln Gly Ile Ser Glu Gly Ser Asn Ile Ala Val Met His Gly Leu
225 230 235 240

Gly Leu Leu Ser Thr Glu Asn Lys Met Ala Arg Ile Asn Asp Leu Ala
245 250 255

Asp Met Ala Gln Leu Lys Asp Tyr Ala Ala Ala Ala Ile Arg Asp Trp
260 265 270

Ala Val Gln Asn Pro Asn Ala Ala Gln Gly Ile Glu Ala Val Ser Asn
275 280 285

Ile Phe Met Ala Ala Ile Pro Ile Lys Gly Ile Gly Ala Val Arg Gly
290 295 300

Lys Tyr Gly Leu Gly Gly Ile Thr Ala His Pro Ile Lys Arg Ser Gln
305 310 315 320

Met Gly Ala Ile Ala Leu Pro Lys Gly Lys Ser Ala Val Ser Asp Asn
325 330 335

Phe Ala Asp Ala Ala Tyr Ala Lys Tyr Pro Ser Pro Tyr His Ser Arg
340 345 350

Asn Ile Arg Ser Asn Leu Glu Gln Arg Tyr Gly Lys Glu Asn Ile Thr
355 360 365

Ser Ser Thr Val Pro Pro Ser Asn Gly Lys Asn Val Lys Leu Ala Asp
370 375 380

Gln Arg His Pro Lys Thr Gly Val Pro Phe Asp Gly Lys Gly Phe Pro
385 390 395 400

Asn Phe Glu Lys His Val Lys Tyr Asp Thr Gly Ser Gly Gly Gly Gly
405 410 415

Ala Thr Asn Asp Asp Asp Val Lys Lys Ala Ala Thr Val Ala Ile Ala
420 425 430

Ala Ala Tyr Asn Asn Gly Gln Glu Ile Asn Gly Phe Lys Ala Gly Glu
435 440 445

Thr Ile Tyr Asp Ile Asp Glu Asp Gly Thr Ile Thr Lys Lys Asp Ala
450 455 460

Thr Ala Ala Asp Val Glu Ala Asp Asp Phe Lys Gly Leu Gly Leu Lys
465 470 475 480

Lys Val Val Thr Asn Leu Thr Lys Thr Val Asn Glu Asn Lys Gln Asn
485 490 495

```
Val Asp Ala Lys Val Lys Ala Ala Glu Ser Glu Ile Glu Lys Leu Thr
            500                 505                 510

Thr Lys Leu Ala Asp Thr Asp Ala Ala Leu Ala Asp Thr Asp Ala Ala
        515                 520                 525

Leu Asp Ala Thr Thr Asn Ala Leu Asn Lys Leu Gly Glu Asn Ile Thr
    530                 535                 540

Thr Phe Ala Glu Glu Thr Lys Thr Asn Ile Val Lys Ile Asp Glu Lys
545                 550                 555                 560

Leu Glu Ala Val Ala Asp Thr Val Asp Lys His Ala Glu Ala Phe Asn
                565                 570                 575

Asp Ile Ala Asp Ser Leu Asp Glu Thr Asn Thr Lys Ala Asp Glu Ala
            580                 585                 590

Val Lys Thr Ala Asn Glu Ala Lys Gln Thr Ala Glu Glu Thr Lys Gln
        595                 600                 605

Asn Val Asp Ala Lys Val Lys Ala Ala Glu Thr Ala Ala Gly Lys Ala
    610                 615                 620

Glu Ala Ala Ala Gly Thr Ala Asn Thr Ala Ala Asp Lys Ala Glu Ala
625                 630                 635                 640

Val Ala Ala Lys Val Thr Asp Ile Lys Ala Asp Ile Ala Thr Asn Lys
                645                 650                 655

Asp Asn Ile Ala Lys Lys Ala Asn Ser Ala Asp Val Tyr Thr Arg Glu
            660                 665                 670

Glu Ser Asp Ser Lys Phe Val Arg Ile Asp Gly Leu Asn Ala Thr Thr
        675                 680                 685

Glu Lys Leu Asp Thr Arg Leu Ala Ser Ala Glu Lys Ser Ile Ala Asp
    690                 695                 700

His Asp Thr Arg Leu Asn Gly Leu Asp Lys Thr Val Ser Asp Leu Arg
705                 710                 715                 720

Lys Glu Thr Arg Gln Gly Leu Ala Glu Gln Ala Ala Leu Ser Gly Leu
                725                 730                 735

Phe Gln Pro Tyr Asn Val Gly Leu Glu His His His His His His
            740                 745                 750
```

<210> 41
<211> 2421
<212> DNA
<213> Artificial Sequence

<220>
<223> 961-ORF46.1

<400> 41

```
atggccacaa acgacgacga tgttaaaaaa gctgccactg tggccattgc tgctgcctac     60
aacaatggcc aagaaatcaa cggtttcaaa gctggagaga ccatctacga cattgatgaa    120
gacggcacaa ttaccaaaaa agacgcaact gcagccgatg ttgaagccga cgactttaaa    180
ggtctgggtc tgaaaaaagt cgtgactaac ctgaccaaaa ccgtcaatga aaacaaacaa    240

aacgtcgatg ccaaagtaaa agctgcagaa tctgaaatag aaaagttaac aaccaagtta    300
gcagacactg atgccgcttt agcagatact gatgccgctc tggatgcaac caccaacgcc    360
ttgaataaat tgggagaaaa tataacgaca tttgctgaag agactaagac aaatatcgta    420
aaaattgatg aaaaattaga agccgtggct gataccgtcg acaagcatgc cgaagcattc    480
aacgatatcg ccgattcatt ggatgaaacc aacactaagg cagacgaagc cgtcaaaacc    540
gccaatgaag ccaaacagac ggccgaagaa accaaacaaa acgtcgatgc caaagtaaaa    600
gctgcagaaa ctgcagcagg caaagccgaa gctgccgctg gcacagctaa tactgcagcc    660
gacaaggccg aagctgtcgc tgcaaaagtt accgacatca aagctgatat cgctacgaac    720
aaagataata ttgctaaaaa agcaaacagt gccgacgtgt acaccagaga agagtctgac    780
agcaaatttg tcagaattga tggtctgaac gctactaccg aaaaattgga cacacgcttg    840
gcttctgctg aaaaatccat tgccgatcac gatactcgcc tgaacggttt ggataaaaca    900
gtgtcagacc tgcgcaaaga aacccgccaa ggccttgcag aacaagccgc gctctccggt    960
ctgttccaac cttacaacgt gggtcggttc aatgtaacgg ctgcagtcgg cggctacaaa   1020
tccgaatcgg cagtcgccat cggtaccggc ttccgcttta ccgaaaactt tgccgccaaa   1080
gcaggcgtgg cagtcggcac ttcgtccggt tcttccgcag cctaccatgt cggcgtcaat   1140
tacgagtggg gatccggagg aggaggatca gatttggcaa acgattcttt tatccggcag   1200
gttctcgacc gtcagcattt cgaacccgac gggaaatacc acctattcgg cagcaggggg   1260
gaacttgccg agcgcagcgg ccatatcgga ttgggaaaaa tacaaagcca tcagttgggc   1320
aacctgatga ttcaacaggc ggccattaaa ggaaatatcg gctacattgt ccgcttttcc   1380
gatcacgggc acgaagtcca ttcccccttc gacaaccatg cctcacattc cgattctgat   1440
gaagccggta gtcccgttga cggatttagc ctttaccgca tccattggga cggatacgaa   1500
caccatcccg ccgacggcta tgacgggcca cagggcggcg gctatcccgc tcccaaaggc   1560
gcgagggata tatacagcta cgacataaaa ggcgttgccc aaaatatccg cctcaacctg   1620
accgacaacc gcagcaccgg acaacggctt gccgaccgtt tccacaatgc cggtagtatg   1680
ctgacgcaag gagtaggcga cggattcaaa cgcgccaccc gatacagccc cgagctggac   1740
agatcgggca atgccgccga agccttcaac ggcactgcag atatcgttaa aaacatcatc   1800
ggcgcggcag gagaaattgt cggcgcaggc gatgccgtgc agggcataag cgaaggctca   1860
aacattgctg tcatgcacgg cttgggtctg ctttccaccg aaaacaagat ggcgcgcatc   1920
aacgatttgg cagatatggc gcaactcaaa gactatgccg cagcagccat ccgcgattgg   1980
gcagtccaaa accccaatgc cgcacaaggc atagaagccg tcagcaatat ctttatggca   2040
gccatcccca tcaaagggat tggagctgtt cggggaaaat acggcttggg cggcatcacg   2100
gcacatccta tcaagcggtc gcagatgggc gcgatcgcat tgccgaaagg gaaatccgcc   2160
gtcagcgaca attttgccga tgcggcatac gccaaatacc cgtcccctta ccattcccga   2220
aatatccgtt caaacttgga gcagcgttac ggcaaagaaa acatcacctc ctcaaccgtg   2280
ccgccgtcaa acggcaaaaa tgtcaaactg gcagaccaac gccacccgaa gacaggcgta   2340
ccgtttgacg gtaaagggtt tccgaatttt gagaagcacg tgaaatatga tacgctcgag   2400
caccaccacc accaccactg a                                             2421
```

<210> 42
<211> 806
<212> PRT
<213> Artificial Sequence

<220>
<223> 961-ORF46.1

<400> 42

```
Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80
```

```
Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110

Ala Leu Asp Ala Thr Thr Asn Ala Leu Asn Lys Leu Gly Glu Asn Ile
        115                 120                 125

Thr Thr Phe Ala Glu Glu Thr Lys Thr Asn Ile Val Lys Ile Asp Glu
    130                 135                 140

Lys Leu Glu Ala Val Ala Asp Thr Val Asp Lys His Ala Glu Ala Phe
145                 150                 155                 160

Asn Asp Ile Ala Asp Ser Leu Asp Glu Thr Asn Thr Lys Ala Asp Glu
                165                 170                 175

Ala Val Lys Thr Ala Asn Glu Ala Lys Gln Thr Ala Glu Glu Thr Lys
            180                 185                 190

Gln Asn Val Asp Ala Lys Val Lys Ala Ala Glu Thr Ala Ala Gly Lys
        195                 200                 205

Ala Glu Ala Ala Ala Gly Thr Ala Asn Thr Ala Ala Asp Lys Ala Glu
    210                 215                 220

Ala Val Ala Ala Lys Val Thr Asp Ile Lys Ala Asp Ile Ala Thr Asn
225                 230                 235                 240

Lys Asp Asn Ile Ala Lys Lys Ala Asn Ser Ala Asp Val Tyr Thr Arg
                245                 250                 255

Glu Glu Ser Asp Ser Lys Phe Val Arg Ile Asp Gly Leu Asn Ala Thr
            260                 265                 270

Thr Glu Lys Leu Asp Thr Arg Leu Ala Ser Ala Glu Lys Ser Ile Ala
        275                 280                 285

Asp His Asp Thr Arg Leu Asn Gly Leu Asp Lys Thr Val Ser Asp Leu
    290                 295                 300

Arg Lys Glu Thr Arg Gln Gly Leu Ala Glu Gln Ala Ala Leu Ser Gly
305                 310                 315                 320

Leu Phe Gln Pro Tyr Asn Val Gly Arg Phe Asn Val Thr Ala Ala Val
                325                 330                 335

Gly Gly Tyr Lys Ser Glu Ser Ala Val Ala Ile Gly Thr Gly Phe Arg
            340                 345                 350

Phe Thr Glu Asn Phe Ala Ala Lys Ala Gly Val Ala Val Gly Thr Ser
        355                 360                 365

Ser Gly Ser Ser Ala Ala Tyr His Val Gly Val Asn Tyr Glu Trp Gly
    370                 375                 380

Ser Gly Gly Gly Gly Ser Asp Leu Ala Asn Asp Ser Phe Ile Arg Gln
385                 390                 395                 400
```

Val Leu Asp Arg Gln His Phe Glu Pro Asp Gly Lys Tyr His Leu Phe
405 410 415

Gly Ser Arg Gly Glu Leu Ala Glu Arg Ser Gly His Ile Gly Leu Gly
420 425 430

Lys Ile Gln Ser His Gln Leu Gly Asn Leu Met Ile Gln Gln Ala Ala
435 440 445

Ile Lys Gly Asn Ile Gly Tyr Ile Val Arg Phe Ser Asp His Gly His
450 455 460

Glu Val His Ser Pro Phe Asp Asn His Ala Ser His Ser Asp Ser Asp
465 470 475 480

Glu Ala Gly Ser Pro Val Asp Gly Phe Ser Leu Tyr Arg Ile His Trp
485 490 495

Asp Gly Tyr Glu His His Pro Ala Asp Gly Tyr Asp Gly Pro Gln Gly
500 505 510

Gly Gly Tyr Pro Ala Pro Lys Gly Ala Arg Asp Ile Tyr Ser Tyr Asp
515 520 525

Ile Lys Gly Val Ala Gln Asn Ile Arg Leu Asn Leu Thr Asp Asn Arg
530 535 540

Ser Thr Gly Gln Arg Leu Ala Asp Arg Phe His Asn Ala Gly Ser Met
545 550 555 560

Leu Thr Gln Gly Val Gly Asp Gly Phe Lys Arg Ala Thr Arg Tyr Ser
565 570 575

Pro Glu Leu Asp Arg Ser Gly Asn Ala Ala Glu Ala Phe Asn Gly Thr
580 585 590

Ala Asp Ile Val Lys Asn Ile Ile Gly Ala Ala Gly Glu Ile Val Gly
595 600 605

Ala Gly Asp Ala Val Gln Gly Ile Ser Glu Gly Ser Asn Ile Ala Val
610 615 620

Met His Gly Leu Gly Leu Leu Ser Thr Glu Asn Lys Met Ala Arg Ile
625 630 635 640

Asn Asp Leu Ala Asp Met Ala Gln Leu Lys Asp Tyr Ala Ala Ala Ala
645 650 655

Ile Arg Asp Trp Ala Val Gln Asn Pro Asn Ala Ala Gln Gly Ile Glu
660 665 670

Ala Val Ser Asn Ile Phe Met Ala Ala Ile Pro Ile Lys Gly Ile Gly
675 680 685

Ala Val Arg Gly Lys Tyr Gly Leu Gly Gly Ile Thr Ala His Pro Ile
690 695 700

Lys Arg Ser Gln Met Gly Ala Ile Ala Leu Pro Lys Gly Lys Ser Ala
705 710 715 720

Val Ser Asp Asn Phe Ala Asp Ala Ala Tyr Ala Lys Tyr Pro Ser Pro

```
                725                     730                     735

Tyr His Ser Arg Asn Ile Arg Ser Asn Leu Glu Gln Arg Tyr Gly Lys
            740                     745                     750

Glu Asn Ile Thr Ser Ser Thr Val Pro Pro Ser Asn Gly Lys Asn Val
        755                     760                     765

Lys Leu Ala Asp Gln Arg His Pro Lys Thr Gly Val Pro Phe Asp Gly
    770                     775                     780

Lys Gly Phe Pro Asn Phe Glu Lys His Val Lys Tyr Asp Thr Leu Glu
785                     790                     795                     800

His His His His His His
                805
```

<210> 43
<211> 1938
<212> DNA
<213> Artificial Sequence

<220>
<223> 961-741

<400> 43

```
atggccacaa acgacgacga tgttaaaaaa gctgccactg tggccattgc tgctgcctac   60
aacaatggcc aagaaatcaa cggtttcaaa gctggagaga ccatctacga cattgatgaa  120
gacggcacaa ttaccaaaaa agacgcaact gcagccgatg ttgaagccga cgactttaaa  180
ggtctgggtc tgaaaaaagt cgtgactaac ctgaccaaaa ccgtcaatga aaacaaacaa  240
aacgtcgatg ccaaagtaaa agctgcagaa tctgaaatag aaaagttaac aaccaagtta  300
gcagacactg atgccgcttt agcagatact gatgccgctc tggatgcaac caccaacgcc  360
ttgaataaat tgggagaaaa tataacgaca tttgctgaag agactaagac aaatatcgta  420
aaaattgatg aaaaattaga agccgtggct gataccgtcg acaagcatgc cgaagcattc  480
aacgatatcg ccgattcatt ggatgaaacc aacactaagg cagacgaagc cgtcaaaacc  540
gccaatgaag ccaaacagac ggccgaagaa accaaacaaa acgtcgatgc caaagtaaaa  600
gctgcagaaa ctgcagcagg caaagccgaa gctgccgctg gcacagctaa tactgcagcc  660
gacaaggccg aagctgtcgc tgcaaaagtt accgacatca aagctgatat cgctacgaac  720
aaagataata ttgctaaaaa agcaaacagt gccgacgtgt acaccagaga agagtctgac  780
agcaaatttg tcagaattga tggtctgaac gctactaccg aaaaattgga cacacgcttg  840
gcttctgctg aaaaatccat tgccgatcac gatactcgcc tgaacggttt ggataaaaca  900
gtgtcagacc tgcgcaaaga aacccgccaa ggccttgcag aacaagccgc gctctccggt  960
ctgttccaac cttacaacgt gggtcggttc aatgtaacgg ctgcagtcgg cggctacaaa 1020
tccgaatcgg cagtcgccat cggtaccggc ttccgcttta ccgaaaactt tgccgccaaa 1080
gcaggcgtgg cagtcggcac ttcgtccggt tcttccgcag cctaccatgt cggcgtcaat 1140
tacgagtggg gatccggagg gggtggtgtc gccgccgaca tcggtgcggg gcttgccgat 1200
gcactaaccg caccgctcga ccataaagac aaaggtttgc agtctttgac gctggatcag 1260
tccgtcagga aaaacgagaa actgaagctg gcggcacaag gtgcggaaaa aacttatgga 1320
aacggtgaca gcctcaatac gggcaaattg aagaacgaca aggtcagccg tttcgacttt 1380
atccgccaaa tcgaagtgga cgggcagctc attaccttgg agagtggaga- gttccaagta 1440
tacaaacaaa gccattccgc cttaaccgcc tttcagaccg agcaaataca agattcggag 1500
cattccggga agatggttgc gaaacgccag ttcagaatcg gcgacatagc gggcgaacat 1560
acatcttttg acaagcttcc cgaaggcggc agggcgacat atcgcgggac ggcgttcggt 1620
tcagacgatg ccggcggaaa actgacctac accatagatt tcgccgccaa gcagggaaac 1680
ggcaaaatcg aacatttgaa atcgccagaa ctcaatgtcg acctggccgc cgccgatatc 1740
aagccggatg gaaaacgcca tgccgtcatc agcggttccg tcctttacaa ccaagccgag 1800
aaaggcagtt actccctcgg tatctttggc ggaaaagccc aggaagttgc cggcagcgcg 1860
gaagtgaaaa ccgtaaacgg catacgccat atcggccttg ccgccaagca actcgagcac 1920
caccaccacc accactga                                                1938
```

<210> 44
<211> 645
<212> PRT
<213> Artificial Sequence

<220>
<223> 961-741

<400> 44

```
Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110

Ala Leu Asp Ala Thr Thr Asn Ala Leu Asn Lys Leu Gly Glu Asn Ile
        115                 120                 125

Thr Thr Phe Ala Glu Glu Thr Lys Thr Asn Ile Val Lys Ile Asp Glu
    130                 135                 140

Lys Leu Glu Ala Val Ala Asp Thr Val Asp Lys His Ala Glu Ala Phe
145                 150                 155                 160

Asn Asp Ile Ala Asp Ser Leu Asp Glu Thr Asn Thr Lys Ala Asp Glu
                165                 170                 175

Ala Val Lys Thr Ala Asn Glu Ala Lys Gln Thr Ala Glu Glu Thr Lys
            180                 185                 190

Gln Asn Val Asp Ala Lys Val Lys Ala Ala Glu Thr Ala Ala Gly Lys
        195                 200                 205

Ala Glu Ala Ala Ala Gly Thr Ala Asn Thr Ala Ala Asp Lys Ala Glu
    210                 215                 220

Ala Val Ala Ala Lys Val Thr Asp Ile Lys Ala Asp Ile Ala Thr Asn
225                 230                 235                 240

Lys Asp Asn Ile Ala Lys Lys Ala Asn Ser Ala Asp Val Tyr Thr Arg
                245                 250                 255

Glu Glu Ser Asp Ser Lys Phe Val Arg Ile Asp Gly Leu Asn Ala Thr
            260                 265                 270

Thr Glu Lys Leu Asp Thr Arg Leu Ala Ser Ala Glu Lys Ser Ile Ala
        275                 280                 285
```

Asp His Asp Thr Arg Leu Asn Gly Leu Asp Lys Thr Val Ser Asp Leu
290 295 300

Arg Lys Glu Thr Arg Gln Gly Leu Ala Glu Gln Ala Ala Leu Ser Gly
305 310 315 320

Leu Phe Gln Pro Tyr Asn Val Gly Arg Phe Asn Val Thr Ala Ala Val
325 330 335

Gly Gly Tyr Lys Ser Glu Ser Ala Val Ala Ile Gly Thr Gly Phe Arg
340 345 350

Phe Thr Glu Asn Phe Ala Ala Lys Ala Gly Val Ala Val Gly Thr Ser
355 360 365

Ser Gly Ser Ser Ala Ala Tyr His Val Gly Val Asn Tyr Glu Trp Gly
370 375 380

Ser Gly Gly Gly Gly Val Ala Ala Asp Ile Gly Ala Gly Leu Ala Asp
385 390 395 400

Ala Leu Thr Ala Pro Leu Asp His Lys Asp Lys Gly Leu Gln Ser Leu
405 410 415

Thr Leu Asp Gln Ser Val Arg Lys Asn Glu Lys Leu Lys Leu Ala Ala
420 425 430

Gln Gly Ala Glu Lys Thr Tyr Gly Asn Gly Asp Ser Leu Asn Thr Gly
435 440 445

Lys Leu Lys Asn Asp Lys Val Ser Arg Phe Asp Phe Ile Arg Gln Ile
450 455 460

Glu Val Asp Gly Gln Leu Ile Thr Leu Glu Ser Gly Glu Phe Gln Val
465 470 475 480

Tyr Lys Gln Ser His Ser Ala Leu Thr Ala Phe Gln Thr Glu Gln Ile
485 490 495

Gln Asp Ser Glu His Ser Gly Lys Met Val Ala Lys Arg Gln Phe Arg
500 505 510

Ile Gly Asp Ile Ala Gly Glu His Thr Ser Phe Asp Lys Leu Pro Glu
515 520 525

Gly Gly Arg Ala Thr Tyr Arg Gly Thr Ala Phe Gly Ser Asp Asp Ala
530 535 540

Gly Gly Lys Leu Thr Tyr Thr Ile Asp Phe Ala Ala Lys Gln Gly Asn
545 550 555 560

Gly Lys Ile Glu His Leu Lys Ser Pro Glu Leu Asn Val Asp Leu Ala
565 570 575

Ala Ala Asp Ile Lys Pro Asp Gly Lys Arg His Ala Val Ile Ser Gly
580 585 590

Ser Val Leu Tyr Asn Gln Ala Glu Lys Gly Ser Tyr Ser Leu Gly Ile
595 600 605

```
Phe Gly Gly Lys Ala Gln Glu Val Ala Gly Ser Ala Glu Val Lys Thr
    610                 615                 620

Val Asn Gly Ile Arg His Ile Gly Leu Ala Ala Lys Gln Leu Glu His
625                 630                 635                 640

His His His His His
                645
```

<210> 45
<211> 4335
<212> DNA
<213> Artificial Sequence

<220>
<223> 961-983

<400> 45

atggccacaa acgacgacga tgttaaaaaa gctgccactg tggccattgc tgctgcctac 60
aacaatggcc aagaaatcaa cggtttcaaa gctggagaga ccatctacga cattgatgaa 120
gacggcacaa ttaccaaaaa agacgcaact gcagccgatg ttgaagccga cgactttaaa 180
ggtctgggtc tgaaaaaagt cgtgactaac ctgaccaaaa ccgtcaatga aaacaaacaa 240
aacgtcgatg ccaaagtaaa agctgcagaa tctgaaatag aaaagttaac aaccaagtta 300
gcagacactg atgccgcttt agcagatact gatgccgctc tggatgcaac caccaacgcc 360
ttgaataaat tgggagaaaa tataacgaca tttgctgaag agactaagac aaatatcgta 420
aaaattgatg aaaaattaga agccgtggct gataccgtcg acaagcatgc cgaagcattc 480
aacgatatcg ccgattcatt ggatgaaacc aacactaagg cagacgaagc cgtcaaaacc 540
gccaatgaag ccaaacagac ggccgaagaa accaaacaaa acgtcgatgc caaagtaaaa 600
gctgcagaaa ctgcagcagg caaagccgaa gctgccgctg gcacagctaa tactgcagcc 660
gacaaggccg aagctgtcgc tgcaaaagtt accgacatca aagctgatat cgctacgaac 720
aaagataata ttgctaaaaa agcaaacagt gccgacgtgt acaccagaga agagtctgac 780
agcaaatttg tcagaattga tggtctgaac gctactaccg aaaaattgga cacacgcttg 840
gcttctgctg aaaaatccat tgccgatcac gatactcgcc tgaacggttt ggataaaaca 900
gtgtcagacc tgcgcaaaga aacccgccaa ggccttgcag aacaagccgc gctctccggt 960
ctgttccaac cttacaacgt gggtcggttc aatgtaacgg ctgcagtcgg cggctacaaa 1020
tccgaatcgg cagtcgccat cggtaccggc ttccgcttta ccgaaaactt tgccgccaaa 1080
gcaggcgtgg cagtcggcac ttcgtccggt tcttccgcag cctaccatgt cggcgtcaat 1140
tacgagtggg gatccggcgg aggcggcact tctgcgcccg acttcaatgc aggcggtacc 1200
ggtatcggca gcaacagcag agcaacaaca gcgaaatcag cagcagtatc ttacgccggt 1260
atcaagaacg aaatgtgcaa agacagaagc atgctctgtg ccggtcggga tgacgttgcg 1320
gttacagaca gggatgccaa aatcaatgcc cccccccga atctgcatac cggagacttt 1380
ccaaacccaa atgacgcata caagaatttg atcaacctca aacctgcaat tgaagcaggc 1440
tatacaggac gcggggtaga ggtaggtatc gtcgacacag gcgaatccgt cggcagcata 1500
tcctttcccg aactgtatgg cagaaaagaa cacggctata acgaaaatta caaaaactat 1560
acggcgtata tgcggaagga agcgcctgaa gacggaggcg gtaaagacat tgaagcttct 1620
ttcgacgatg aggccgttat agagactgaa gcaaagccga cggatatccg ccacgtaaaa 1680
gaaatcggac acatcgattt ggtctcccat attattggcg ggcgttccgt ggacggcaga 1740
cctgcaggcg gtattgcgcc cgatgcgacg ctacacataa tgaatacgaa tgatgaaacc 1800
aagaacgaaa tgatggttgc agccatccgc aatgcatggg tcaagctggg cgaacgtggc 1860
gtgcgcatcg tcaataacag ttttggaaca acatcgaggg caggcactgc cgaccttttc 1920
caaatagcca attcggagga gcagtaccgc caagcgttgc tcgactattc cggcggtgat 1980
aaaacagacg agggtatccg cctgatgcaa cagagcgatt acggcaacct gtcctaccac 2040
atccgtaata aaaacatgct tttcatcttt tcgacaggca atgacgcaca agctcagccc 2100
aacacatatg ccctattgcc attttatgaa aaagacgctc aaaaaggcat tatcacagtc 2160
gcaggcgtag accgcagtgg agaaaagttc aaacgggaaa tgtatggaga accgggtaca 2220
gaaccgcttg agtatggctc caaccattgc ggaattactg ccatgtggtg cctgtcggca 2280
ccctatgaag caagcgtccg tttcacccgt acaaacccga ttcaaattgc cggaacatcc 2340
ttttccgcac ccatcgtaac cggcacggcg gctctgctgc tgcagaaata cccgtggatg 2400
agcaacgaca acctgcgtac cacgttgctg acgacggctc aggacatcgg tgcagtcggc 2460
gtggacagca agttcggctg gggactgctg gatgcgggta aggccatgaa cggacccgcg 2520
tcctttccgt tcggcgactt taccgccgat acgaaaggta catccgatat tgcctactcc 2580

```
ttccgtaacg acatttcagg cacgggcggc ctgatcaaaa aaggcggcag ccaactgcaa   2640
ctgcacggca acaacaccta tacgggcaaa accattatcg aaggcggttc gctggtgttg   2700
tacggcaaca acaaatcgga tatgcgcgtc gaaaccaaag gtgcgctgat ttataacggg   2760
gcggcatccg gcggcagcct gaacagcgac ggcattgtct atctggcaga taccgaccaa   2820
tccggcgcaa acgaaaccgt acacatcaaa ggcagtctgc agctggacgg caaaggtacg   2880
ctgtacacac gtttgggcaa actgctgaaa gtggacggta cggcgattat cggcggcaag   2940
ctgtacatgt cggcacgcgg caaggggggca ggctatctca acagtaccgg acgacgtgtt   3000
cccttcctga gtgccgccaa aatcgggcag gattattctt tcttcacaaa catcgaaacc   3060
gacggcggcc tgctggcttc cctcgacagc gtcgaaaaaa cagcgggcag tgaaggcgac   3120
acgctgtcct attatgtccg tcgcggcaat gcggcacgga ctgcttcggc agcggcacat   3180
tccgcgcccg ccggtctgaa acacgccgta gaacagggcg gcagcaatct ggaaaacctg   3240
atggtcgaac tggatgcctc cgaatcatcc gcaacacccg agacggttga aactgcggca   3300
gccgaccgca cagatatgcc gggcatccgc ccctacggcg caactttccg cgcagcggca   3360
gccgtacagc atgcgaatgc cgccgacggt gtacgcatct tcaacagtct cgccgctacc   3420
gtctatgccg acagtaccgc cgcccatgcc gatatgcagg gacgccgcct gaaagccgta   3480
tcggacgggt tggaccacaa cggcacgggt ctgcgcgtca tcgcgcaaac ccaacaggac   3540
ggtggaacgt gggaacaggg cggtgttgaa ggcaaaatgc gcggcagtac ccaaaccgtc   3600
ggcattgccg cgaaaaccgg cgaaaatacg acagcagccg ccacactggg catgggacgc   3660
agcacatgga gcgaaaacag tgcaaatgca aaaaccgaca gcattagtct gtttgcaggc   3720
atacggcacg atgcgggcga tatcggctat ctcaaaggcc tgttctccta cggacgctac   3780
aaaaacagca tcagccgcag caccggtgcg gacgaacatg cggaaggcag cgtcaacggc   3840
acgctgatgc agctgggcgc actgggcggt gtcaacgttc cgtttgccgc aacgggagat   3900
ttgacggtcg aaggcggtct gcgctacgac ctgctcaaac aggatgcatt cgccgaaaaa   3960
ggcagtgctt tgggctggag cggcaacagc ctcactgaag gcacgctggt cggactcgcg   4020
ggtctgaagc tgtcgcaacc cttgagcgat aaagccgtcc tgtttgcaac ggcgggcgtg   4080
gaacgcgacc tgaacggacg cgactacacg gtaacgggcg gctttaccgg cgcgactgca   4140
gcaaccggca agacgggggc acgcaatatg ccgcacaccc gtctggttgc cggcctgggc   4200
gcggatgtcg aattcggcaa cggctggaac ggcttggcac gttacagcta cgccggttcc   4260
aaacagtacg gcaaccacag cggacgagtc ggcgtaggct accggttcct cgagcaccac   4320
caccaccacc actga                                                    4335
```

<210> 46
<211> 1444
<212> PRT
<213> Artificial Sequence

<220>
<223> 961-983

<400> 46

```
Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110
```

Ala Leu Asp Ala Thr Thr Asn Ala Leu Asn Lys Leu Gly Glu Asn Ile
115 120 125

Thr Thr Phe Ala Glu Glu Thr Lys Thr Asn Ile Val Lys Ile Asp Glu
130 135 140

Lys Leu Glu Ala Val Ala Asp Thr Val Asp Lys His Ala Glu Ala Phe
145 150 155 160

Asn Asp Ile Ala Asp Ser Leu Asp Glu Thr Asn Thr Lys Ala Asp Glu
165 170 175

Ala Val Lys Thr Ala Asn Glu Ala Lys Gln Thr Ala Glu Glu Thr Lys
180 185 190

Gln Asn Val Asp Ala Lys Val Lys Ala Ala Glu Thr Ala Ala Gly Lys
195 200 205

Ala Glu Ala Ala Ala Gly Thr Ala Asn Thr Ala Ala Asp Lys Ala Glu
210 215 220

Ala Val Ala Ala Lys Val Thr Asp Ile Lys Ala Asp Ile Ala Thr Asn
225 230 235 240

Lys Asp Asn Ile Ala Lys Lys Ala Asn Ser Ala Asp Val Tyr Thr Arg
245 250 255

Glu Glu Ser Asp Ser Lys Phe Val Arg Ile Asp Gly Leu Asn Ala Thr
260 265 270

Thr Glu Lys Leu Asp Thr Arg Leu Ala Ser Ala Glu Lys Ser Ile Ala
275 280 285

Asp His Asp Thr Arg Leu Asn Gly Leu Asp Lys Thr Val Ser Asp Leu
290 295 300

Arg Lys Glu Thr Arg Gln Gly Leu Ala Glu Gln Ala Ala Leu Ser Gly
305 310 315 320

Leu Phe Gln Pro Tyr Asn Val Gly Arg Phe Asn Val Thr Ala Ala Val
325 330 335

Gly Gly Tyr Lys Ser Glu Ser Ala Val Ala Ile Gly Thr Gly Phe Arg
340 345 350

Phe Thr Glu Asn Phe Ala Ala Lys Ala Gly Val Ala Val Gly Thr Ser
355 360 365

Ser Gly Ser Ser Ala Ala Tyr His Val Gly Val Asn Tyr Glu Trp Gly
370 375 380

Ser Gly Gly Gly Gly Thr Ser Ala Pro Asp Phe Asn Ala Gly Gly Thr
385 390 395 400

Gly Ile Gly Ser Asn Ser Arg Ala Thr Thr Ala Lys Ser Ala Ala Val
405 410 415

Ser Tyr Ala Gly Ile Lys Asn Glu Met Cys Lys Asp Arg Ser Met Leu
420 425 430

Cys Ala Gly Arg Asp Asp Val Ala Val Thr Asp Arg Asp Ala Lys Ile

435 440 445

Asn Ala Pro Pro Pro Asn Leu His Thr Gly Asp Phe Pro Asn Pro Asn
450 455 460

Asp Ala Tyr Lys Asn Leu Ile Asn Leu Lys Pro Ala Ile Glu Ala Gly
465 470 475 480

Tyr Thr Gly Arg Gly Val Glu Val Gly Ile Val Asp Thr Gly Glu Ser
485 490 495

Val Gly Ser Ile Ser Phe Pro Glu Leu Tyr Gly Arg Lys Glu His Gly
500 505 510

Tyr Asn Glu Asn Tyr Lys Asn Tyr Thr Ala Tyr Met Arg Lys Glu Ala
515 520 525

Pro Glu Asp Gly Gly Gly Lys Asp Ile Glu Ala Ser Phe Asp Asp Glu
530 535 540

Ala Val Ile Glu Thr Glu Ala Lys Pro Thr Asp Ile Arg His Val Lys
545 550 555 560

Glu Ile Gly His Ile Asp Leu Val Ser His Ile Ile Gly Gly Arg Ser
565 570 575

Val Asp Gly Arg Pro Ala Gly Gly Ile Ala Pro Asp Ala Thr Leu His
580 585 590

Ile Met Asn Thr Asn Asp Glu Thr Lys Asn Glu Met Met Val Ala Ala
595 600 605

Ile Arg Asn Ala Trp Val Lys Leu Gly Glu Arg Gly Val Arg Ile Val
610 615 620

Asn Asn Ser Phe Gly Thr Thr Ser Arg Ala Gly Thr Ala Asp Leu Phe
625 630 635 640

Gln Ile Ala Asn Ser Glu Glu Gln Tyr Arg Gln Ala Leu Leu Asp Tyr
645 650 655

Ser Gly Gly Asp Lys Thr Asp Glu Gly Ile Arg Leu Met Gln Gln Ser
660 665 670

Asp Tyr Gly Asn Leu Ser Tyr His Ile Arg Asn Lys Asn Met Leu Phe
675 680 685

Ile Phe Ser Thr Gly Asn Asp Ala Gln Ala Gln Pro Asn Thr Tyr Ala
690 695 700

Leu Leu Pro Phe Tyr Glu Lys Asp Ala Gln Lys Gly Ile Ile Thr Val
705 710 715 720

Ala Gly Val Asp Arg Ser Gly Glu Lys Phe Lys Arg Glu Met Tyr Gly
725 730 735

Glu Pro Gly Thr Glu Pro Leu Glu Tyr Gly Ser Asn His Cys Gly Ile
740 745 750

Thr Ala Met Trp Cys Leu Ser Ala Pro Tyr Glu Ala Ser Val Arg Phe
755 760 765

```
Thr Arg Thr Asn Pro Ile Gln Ile Ala Gly Thr Ser Phe Ser Ala Pro
    770                 775                 780

Ile Val Thr Gly Thr Ala Ala Leu Leu Leu Gln Lys Tyr Pro Trp Met
785                 790                 795                 800

Ser Asn Asp Asn Leu Arg Thr Thr Leu Leu Thr Thr Ala Gln Asp Ile
                805                 810                 815

Gly Ala Val Gly Val Asp Ser Lys Phe Gly Trp Gly Leu Leu Asp Ala
            820                 825                 830

Gly Lys Ala Met Asn Gly Pro Ala Ser Phe Pro Phe Gly Asp Phe Thr
        835                 840                 845

Ala Asp Thr Lys Gly Thr Ser Asp Ile Ala Tyr Ser Phe Arg Asn Asp
    850                 855                 860

Ile Ser Gly Thr Gly Gly Leu Ile Lys Lys Gly Gly Ser Gln Leu Gln
865                 870                 875                 880

Leu His Gly Asn Asn Thr Tyr Thr Gly Lys Thr Ile Ile Glu Gly Gly
                885                 890                 895

Ser Leu Val Leu Tyr Gly Asn Asn Lys Ser Asp Met Arg Val Glu Thr
            900                 905                 910

Lys Gly Ala Leu Ile Tyr Asn Gly Ala Ala Ser Gly Gly Ser Leu Asn
        915                 920                 925

Ser Asp Gly Ile Val Tyr Leu Ala Asp Thr Asp Gln Ser Gly Ala Asn
    930                 935                 940

Glu Thr Val His Ile Lys Gly Ser Leu Gln Leu Asp Gly Lys Gly Thr
945                 950                 955                 960

Leu Tyr Thr Arg Leu Gly Lys Leu Leu Lys Val Asp Gly Thr Ala Ile
                965                 970                 975

Ile Gly Gly Lys Leu Tyr Met Ser Ala Arg Gly Lys Gly Ala Gly Tyr
            980                 985                 990

Leu Asn Ser Thr Gly Arg Arg Val Pro Phe Leu Ser Ala Ala Lys Ile
        995                 1000                1005

Gly Gln Asp Tyr Ser Phe Phe Thr Asn Ile Glu Thr Asp Gly Gly Leu
    1010                1015                1020

Leu Ala Ser Leu Asp Ser Val Glu Lys Thr Ala Gly Ser Glu Gly Asp
1025                1030                1035                1040

Thr Leu Ser Tyr Tyr Val Arg Arg Gly Asn Ala Ala Arg Thr Ala Ser
                1045                1050                1055

Ala Ala Ala His Ser Ala Pro Ala Gly Leu Lys His Ala Val Glu Gln
            1060                1065                1070

Gly Gly Ser Asn Leu Glu Asn Leu Met Val Glu Leu Asp Ala Ser Glu
        1075                1080                1085
```

Ser Ser Ala Thr Pro Glu Thr Val Glu Thr Ala Ala Ala Asp Arg Thr
1090 1095 1100

Asp Met Pro Gly Ile Arg Pro Tyr Gly Ala Thr Phe Arg Ala Ala Ala
1105 1110 1115 1120

Ala Val Gln His Ala Asn Ala Ala Asp Gly Val Arg Ile Phe Asn Ser
1125 1130 1135

Leu Ala Ala Thr Val Tyr Ala Asp Ser Thr Ala Ala His Ala Asp Met
1140 1145 1150

Gln Gly Arg Arg Leu Lys Ala Val Ser Asp Gly Leu Asp His Asn Gly
1155 1160 1165

Thr Gly Leu Arg Val Ile Ala Gln Thr Gln Gln Asp Gly Gly Thr Trp
1170 1175 1180

Glu Gln Gly Gly Val Glu Gly Lys Met Arg Gly Ser Thr Gln Thr Val
1185 1190 1195 1200

Gly Ile Ala Ala Lys Thr Gly Glu Asn Thr Thr Ala Ala Ala Thr Leu
1205 1210 1215

Gly Met Gly Arg Ser Thr Trp Ser Glu Asn Ser Ala Asn Ala Lys Thr
1220 1225 1230

Asp Ser Ile Ser Leu Phe Ala Gly Ile Arg His Asp Ala Gly Asp Ile
1235 1240 1245

Gly Tyr Leu Lys Gly Leu Phe Ser Tyr Gly Arg Tyr Lys Asn Ser Ile
1250 1255 1260

Ser Arg Ser Thr Gly Ala Asp Glu His Ala Glu Gly Ser Val Asn Gly
1265 1270 1275 1280

Thr Leu Met Gln Leu Gly Ala Leu Gly Gly Val Asn Val Pro Phe Ala
1285 1290 1295

Ala Thr Gly Asp Leu Thr Val Glu Gly Gly Leu Arg Tyr Asp Leu Leu
1300 1305 1310

Lys Gln Asp Ala Phe Ala Glu Lys Gly Ser Ala Leu Gly Trp Ser Gly
1315 1320 1325

Asn Ser Leu Thr Glu Gly Thr Leu Val Gly Leu Ala Gly Leu Lys Leu
1330 1335 1340

Ser Gln Pro Leu Ser Asp Lys Ala Val Leu Phe Ala Thr Ala Gly Val
1345 1350 1355 1360

Glu Arg Asp Leu Asn Gly Arg Asp Tyr Thr Val Thr Gly Gly Phe Thr
1365 1370 1375

Gly Ala Thr Ala Ala Thr Gly Lys Thr Gly Ala Arg Asn Met Pro His
1380 1385 1390

Thr Arg Leu Val Ala Gly Leu Gly Ala Asp Val Glu Phe Gly Asn Gly
1395 1400 1405

Trp Asn Gly Leu Ala Arg Tyr Ser Tyr Ala Gly Ser Lys Gln Tyr Gly

```
        1410                1415                1420

Asn His Ser Gly Arg Val Gly Val Gly Tyr Arg Phe Leu Glu His His
1425                1430                1435                1440

His His His His
```

<210> 47
<211> 2256
<212> DNA
<213> Artificial Sequence

<220>
<223> 961c-ORF46.1

<400> 47

```
atggccacaa acgacgacga tgttaaaaaa gctgccactg tggccattgc tgctgcctac   60
aacaatggcc aagaaatcaa cggtttcaaa gctggagaga ccatctacga cattgatgaa  120
gacggcacaa ttaccaaaaa agacgcaact gcagccgatg ttgaagccga cgactttaaa  180
ggtctgggtc tgaaaaaagt cgtgactaac ctgaccaaaa ccgtcaatga aaacaaacaa  240
aacgtcgatg ccaaagtaaa agctgcagaa tctgaaatag aaaagttaac aaccaagtta  300
gcagacactg atgccgcttt agcagatact gatgccgctc tggatgcaac caccaacgcc  360
ttgaataaat tgggagaaaa tataacgaca tttgctgaag agactaagac aaatatcgta  420
aaaattgatg aaaaattaga agccgtggct gataccgtcg acaagcatgc cgaagcattc  480
aacgatatcg ccgattcatt ggatgaaacc aacactaagg cagacgaagc cgtcaaaacc  540
gccaatgaag ccaaacagac ggccgaagaa accaaacaaa acgtcgatgc caaagtaaaa  600
gctgcagaaa ctgcagcagg caaagccgaa gctgccgctg gcacagctaa tactgcagcc  660
gacaaggccg aagctgtcgc tgcaaaagtt accgacatca aagctgatat cgctacgaac  720
aaagataata ttgctaaaaa agcaaacagt gccgacgtgt acaccagaga agagtctgac  780
agcaaatttg tcagaattga tggtctgaac gctactaccg aaaaattgga cacacgcttg  840
gcttctgctg aaaaatccat tgccgatcac gatactcgcc tgaacggttt ggataaaaca  900
gtgtcagacc tgcgcaaaga aacccgccaa ggccttgcag aacaagccgc gctctccggt  960
ctgttccaac cttacaacgt gggtggatcc ggaggaggag gatcagattt ggcaaacgat 1020
tcttttatcc ggcaggttct cgaccgtcag catttcgaac ccgacgggaa ataccaccta 1080
ttcggcagca gggggggaact tgccgagcgc agcggccata tcggattggg aaaaatacaa 1140
agccatcagt tgggcaacct gatgattcaa caggcggcca ttaaaggaaa tatcggctac 1200
attgtccgct tttccgatca cgggcacgaa gtccattccc ccttcgacaa ccatgcctca 1260
cattccgatt ctgatgaagc cggtagtccc gttgacggat ttagccttta ccgcatccat 1320
tgggacggat acgaacacca tcccgccgac ggctatgacg ggccacaggg cggcggctat 1380
cccgctccca aaggcgcgag ggatatatac agctacgaca taaaaggcgt tgcccaaaat 1440
atccgcctca acctgaccga caaccgcagc accggacaac ggcttgccga ccgtttccac 1500
aatgccggta gtatgctgac gcaaggagta ggcgacggat tcaaacgcgc cacccgatac 1560
agccccgagc tggacagatc gggcaatgcc gccgaagcct tcaacggcac tgcagatatc 1620
gttaaaaaca tcatcggcgc ggcaggagaa attgtcggcg caggcgatgc cgtgcagggc 1680
ataagcgaag gctcaaacat tgctgtcatg cacggcttgg gtctgctttc caccgaaaac 1740
aagatggcgc gcatcaacga tttggcagat atggcgcaac tcaaagacta tgccgcagca 1800
gccatccgcg attgggcagt ccaaaacccc aatgccgcac aaggcataga agccgtcagc 1860
aatatcttta tggcagccat ccccatcaaa gggattggag ctgttcgggg aaaatacggc 1920
ttgggcggca tcacggcaca tcctatcaag cggtcgcaga tgggcgcgat cgcattgccg 1980
aaagggaaat ccgccgtcag cgacaatttt gccgatgcgg catacgccaa atacccgtcc 2040
ccttaccatt cccgaaatat ccgttcaaac ttggagcagc gttacggcaa agaaaacatc 2100
acctcctcaa ccgtgccgcc gtcaaacggc aaaaatgtca aactggcaga ccaacgccac 2160
ccgaagacag gcgtaccgtt tgacggtaaa gggtttccga attttgagaa gcacgtgaaa 2220
tatgatacgc tcgagcacca ccaccaccac cactga                            2256
```

<210> 48
<211> 751
<212> PRT
<213> Artificial Sequence

<220>
<223> 961c-ORF46.1

<400> 48

```
Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110

Ala Leu Asp Ala Thr Thr Asn Ala Leu Asn Lys Leu Gly Glu Asn Ile
        115                 120                 125

Thr Thr Phe Ala Glu Glu Thr Lys Thr Asn Ile Val Lys Ile Asp Glu
    130                 135                 140

Lys Leu Glu Ala Val Ala Asp Thr Val Asp Lys His Ala Glu Ala Phe
145                 150                 155                 160

Asn Asp Ile Ala Asp Ser Leu Asp Glu Thr Asn Thr Lys Ala Asp Glu
                165                 170                 175

Ala Val Lys Thr Ala Asn Glu Ala Lys Gln Thr Ala Glu Glu Thr Lys
            180                 185                 190

Gln Asn Val Asp Ala Lys Val Lys Ala Ala Glu Thr Ala Ala Gly Lys
        195                 200                 205

Ala Glu Ala Ala Ala Gly Thr Ala Asn Thr Ala Ala Asp Lys Ala Glu
    210                 215                 220

Ala Val Ala Ala Lys Val Thr Asp Ile Lys Ala Asp Ile Ala Thr Asn
225                 230                 235                 240

Lys Asp Asn Ile Ala Lys Lys Ala Asn Ser Ala Asp Val Tyr Thr Arg
                245                 250                 255

Glu Glu Ser Asp Ser Lys Phe Val Arg Ile Asp Gly Leu Asn Ala Thr
            260                 265                 270

Thr Glu Lys Leu Asp Thr Arg Leu Ala Ser Ala Glu Lys Ser Ile Ala
        275                 280                 285

Asp His Asp Thr Arg Leu Asn Gly Leu Asp Lys Thr Val Ser Asp Leu
    290                 295                 300
```

```
Arg Lys Glu Thr Arg Gln Gly Leu Ala Glu Gln Ala Ala Leu Ser Gly
305                 310                 315                 320

Leu Phe Gln Pro Tyr Asn Val Gly Gly Ser Gly Gly Gly Gly Ser Asp
                325                 330                 335

Leu Ala Asn Asp Ser Phe Ile Arg Gln Val Leu Asp Arg Gln His Phe
            340                 345                 350

Glu Pro Asp Gly Lys Tyr His Leu Phe Gly Ser Arg Gly Glu Leu Ala
        355                 360                 365

Glu Arg Ser Gly His Ile Gly Leu Gly Lys Ile Gln Ser His Gln Leu
    370                 375                 380

Gly Asn Leu Met Ile Gln Gln Ala Ala Ile Lys Gly Asn Ile Gly Tyr
385                 390                 395                 400

Ile Val Arg Phe Ser Asp His Gly His Glu Val His Ser Pro Phe Asp
                405                 410                 415

Asn His Ala Ser His Ser Asp Ser Asp Glu Ala Gly Ser Pro Val Asp
            420                 425                 430

Gly Phe Ser Leu Tyr Arg Ile His Trp Asp Gly Tyr Glu His His Pro
        435                 440                 445

Ala Asp Gly Tyr Asp Gly Pro Gln Gly Gly Gly Tyr Pro Ala Pro Lys
    450                 455                 460

Gly Ala Arg Asp Ile Tyr Ser Tyr Asp Ile Lys Gly Val Ala Gln Asn
465                 470                 475                 480

Ile Arg Leu Asn Leu Thr Asp Asn Arg Ser Thr Gly Gln Arg Leu Ala
                485                 490                 495

Asp Arg Phe His Asn Ala Gly Ser Met Leu Thr Gln Gly Val Gly Asp
            500                 505                 510

Gly Phe Lys Arg Ala Thr Arg Tyr Ser Pro Glu Leu Asp Arg Ser Gly
        515                 520                 525

Asn Ala Ala Glu Ala Phe Asn Gly Thr Ala Asp Ile Val Lys Asn Ile
    530                 535                 540

Ile Gly Ala Ala Gly Glu Ile Val Gly Ala Gly Asp Ala Val Gln Gly
545                 550                 555                 560

Ile Ser Glu Gly Ser Asn Ile Ala Val Met His Gly Leu Gly Leu Leu
                565                 570                 575

Ser Thr Glu Asn Lys Met Ala Arg Ile Asn Asp Leu Ala Asp Met Ala
            580                 585                 590

Gln Leu Lys Asp Tyr Ala Ala Ala Ala Ile Arg Asp Trp Ala Val Gln
        595                 600                 605

Asn Pro Asn Ala Ala Gln Gly Ile Glu Ala Val Ser Asn Ile Phe Met
    610                 615                 620

Ala Ala Ile Pro Ile Lys Gly Ile Gly Ala Val Arg Gly Lys Tyr Gly
```

```
625                 630                 635                 640

Leu Gly Gly Ile Thr Ala His Pro Ile Lys Arg Ser Gln Met Gly Ala
                645                 650                 655

Ile Ala Leu Pro Lys Gly Lys Ser Ala Val Ser Asp Asn Phe Ala Asp
            660                 665                 670

Ala Ala Tyr Ala Lys Tyr Pro Ser Pro Tyr His Ser Arg Asn Ile Arg
        675                 680                 685

Ser Asn Leu Glu Gln Arg Tyr Gly Lys Glu Asn Ile Thr Ser Ser Thr
    690                 695                 700

Val Pro Pro Ser Asn Gly Lys Asn Val Lys Leu Ala Asp Gln Arg His
705                 710                 715                 720

Pro Lys Thr Gly Val Pro Phe Asp Gly Lys Gly Phe Pro Asn Phe Glu
                725                 730                 735

Lys His Val Lys Tyr Asp Thr Leu Glu His His His His His His
            740                 745                 750
```

<210> 49
<211> 1773
<212> DNA
<213> Artificial Sequence

<220>
<223> 961c-741

<400> 49

```
atggccacaa acgacgacga tgttaaaaaa gctgccactg tggccattgc tgctgcctac    60
aacaatggcc aagaaatcaa cggtttcaaa gctggagaga ccatctacga cattgatgaa   120
gacggcacaa ttaccaaaaa agacgcaact gcagccgatg ttgaagccga cgactttaaa   180
ggtctgggtc tgaaaaaagt cgtgactaac ctgaccaaaa ccgtcaatga aaacaaacaa   240
aacgtcgatg ccaaagtaaa agctgcagaa tctgaaatag aaaagttaac aaccaagtta   300
gcagacactg atgccgcttt agcagatact gatgccgctc tggatgcaac caccaacgcc   360
ttgaataaat tgggagaaaa tataacgaca tttgctgaag agactaagac aaatatcgta   420
aaaattgatg aaaaattaga agccgtggct gataccgtcg acaagcatgc cgaagcattc   480
aacgatatcg ccgattcatt ggatgaaacc aacactaagg cagacgaagc cgtcaaaacc   540
gccaatgaag ccaaacagac ggccgaagaa accaaacaaa acgtcgatgc caaagtaaaa   600
gctgcagaaa ctgcagcagg caaagccgaa gctgccgctg gcacagctaa tactgcagcc   660
gacaaggccg aagctgtcgc tgcaaaagtt accgacatca aagctgatat cgctacgaac   720
aaagataata ttgctaaaaa agcaaacagt gccgacgtgt acaccagaga agagtctgac   780
agcaaatttg tcagaattga tggtctgaac gctactaccg aaaaattgga cacacgcttg   840
gcttctgctg aaaaatccat tgccgatcac gatactcgcc tgaacggttt ggataaaaca   900
gtgtcagacc tgcgcaaaga aacccgccaa ggccttgcag aacaagccgc gctctccggt   960
ctgttccaac cttacaacgt gggtggatcc ggagggggtg gtgtcgccgc cgacatcggt  1020
gcggggcttg ccgatgcact aaccgcaccg ctcgaccata aagacaaagg tttgcagtct  1080
ttgacgctgg atcagtccgt caggaaaaac gagaaactga agctggcggc acaaggtgcg  1140
gaaaaaactt atggaaacgg tgacagcctc aatacgggca aattgaagaa cgacaaggtc  1200
agccgtttcg actttatccg ccaaatcgaa gtggacgggc agctcattac cttggagagt  1260
ggagagttcc aagtatacaa acaaagccat tccgccttaa ccgcctttca gaccgagcaa  1320
atacaagatt cggagcattc cgggaagatg gttgcgaaac gccagttcag aatcggcgac  1380
atagcgggcg aacatacatc ttttgacaag cttcccgaag gcggcagggc gacatatcgc  1440
gggacggcgt tcggttcaga cgatgccggc ggaaaactga cctacaccat agatttcgcc  1500
gccaagcagg gaaacggcaa aatcgaacat ttgaaatcgc cagaactcaa tgtcgacctg  1560
gccgccgccg atatcaagcc ggatggaaaa cgccatgccg tcatcagcgg ttccgtcctt  1620
tacaaccaag ccgagaaagg cagttactcc ctcggtatct ttggcggaaa agcccaggaa  1680
gttgccggca gcgcggaagt gaaaaccgta aacggcatac gccatatcgg ccttgccgcc  1740

aagcaactcg agcaccacca ccaccaccac tga                                1773
```

<210> 50
<211> 590
<212> PRT
<213> Artificial Sequence

<220>
<223> 961c-741

<400> 50

Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110

Ala Leu Asp Ala Thr Thr Asn Ala Leu Asn Lys Leu Gly Glu Asn Ile
        115                 120                 125

Thr Thr Phe Ala Glu Glu Thr Lys Thr Asn Ile Val Lys Ile Asp Glu
    130                 135                 140

Lys Leu Glu Ala Val Ala Asp Thr Val Asp Lys His Ala Glu Ala Phe
145                 150                 155                 160

Asn Asp Ile Ala Asp Ser Leu Asp Glu Thr Asn Thr Lys Ala Asp Glu
                165                 170                 175

Ala Val Lys Thr Ala Asn Glu Ala Lys Gln Thr Ala Glu Glu Thr Lys
            180                 185                 190

Gln Asn Val Asp Ala Lys Val Lys Ala Ala Glu Thr Ala Ala Gly Lys
        195                 200                 205

Ala Glu Ala Ala Ala Gly Thr Ala Asn Thr Ala Ala Asp Lys Ala Glu
    210                 215                 220

Ala Val Ala Ala Lys Val Thr Asp Ile Lys Ala Asp Ile Ala Thr Asn
225                 230                 235                 240

Lys Asp Asn Ile Ala Lys Lys Ala Asn Ser Ala Asp Val Tyr Thr Arg
                245                 250                 255

Glu Glu Ser Asp Ser Lys Phe Val Arg Ile Asp Gly Leu Asn Ala Thr
            260                 265                 270

```
Thr Glu Lys Leu Asp Thr Arg Leu Ala Ser Ala Glu Lys Ser Ile Ala
        275                 280                 285

Asp His Asp Thr Arg Leu Asn Gly Leu Asp Lys Thr Val Ser Asp Leu
    290                 295                 300

Arg Lys Glu Thr Arg Gln Gly Leu Ala Glu Gln Ala Ala Leu Ser Gly
305                 310                 315                 320

Leu Phe Gln Pro Tyr Asn Val Gly Gly Ser Gly Gly Gly Gly Val Ala
                325                 330                 335

Ala Asp Ile Gly Ala Gly Leu Ala Asp Ala Leu Thr Ala Pro Leu Asp
            340                 345                 350

His Lys Asp Lys Gly Leu Gln Ser Leu Thr Leu Asp Gln Ser Val Arg
        355                 360                 365

Lys Asn Glu Lys Leu Lys Leu Ala Ala Gln Gly Ala Glu Lys Thr Tyr
    370                 375                 380

Gly Asn Gly Asp Ser Leu Asn Thr Gly Lys Leu Lys Asn Asp Lys Val
385                 390                 395                 400

Ser Arg Phe Asp Phe Ile Arg Gln Ile Glu Val Asp Gly Gln Leu Ile
                405                 410                 415

Thr Leu Glu Ser Gly Glu Phe Gln Val Tyr Lys Gln Ser His Ser Ala
            420                 425                 430

Leu Thr Ala Phe Gln Thr Glu Gln Ile Gln Asp Ser Glu His Ser Gly
        435                 440                 445

Lys Met Val Ala Lys Arg Gln Phe Arg Ile Gly Asp Ile Ala Gly Glu
    450                 455                 460

His Thr Ser Phe Asp Lys Leu Pro Glu Gly Gly Arg Ala Thr Tyr Arg
465                 470                 475                 480

Gly Thr Ala Phe Gly Ser Asp Asp Ala Gly Gly Lys Leu Thr Tyr Thr
                485                 490                 495

Ile Asp Phe Ala Ala Lys Gln Gly Asn Gly Lys Ile Glu His Leu Lys
            500                 505                 510

Ser Pro Glu Leu Asn Val Asp Leu Ala Ala Ala Asp Ile Lys Pro Asp
        515                 520                 525

Gly Lys Arg His Ala Val Ile Ser Gly Ser Val Leu Tyr Asn Gln Ala
    530                 535                 540

Glu Lys Gly Ser Tyr Ser Leu Gly Ile Phe Gly Gly Lys Ala Gln Glu
545                 550                 555                 560

Val Ala Gly Ser Ala Glu Val Lys Thr Val Asn Gly Ile Arg His Ile
                565                 570                 575

Gly Leu Ala Ala Lys Gln Leu Glu His His His His His His
            580                 585                 590
```

<210> 51
<211> 4170
<212> DNA
<213> Artificial Sequence <220>

<223> 961c-983

<400> 51

```
atggccacaa acgacgacga tgttaaaaaa gctgccactg tggccattgc tgctgcctac   60
aacaatggcc aagaaatcaa cggtttcaaa gctggagaga ccatctacga cattgatgaa  120
gacggcacaa ttaccaaaaa agacgcaact gcagccgatg ttgaagccga cgactttaaa  180
ggtctgggtc tgaaaaaagt cgtgactaac ctgaccaaaa ccgtcaatga aaacaaacaa  240
aacgtcgatg ccaaagtaaa agctgcagaa tctgaaatag aaaagttaac aaccaagtta  300
gcagacactg atgccgcttt agcagatact gatgccgctc tggatgcaac caccaacgcc  360
ttgaataaat tgggagaaaa tataacgaca tttgctgaag agactaagac aaatatcgta  420
aaaattgatg aaaaattaga agccgtggct gataccgtcg acaagcatgc cgaagcattc  480
aacgatatcg ccgattcatt ggatgaaacc aacactaagg cagacgaagc cgtcaaaacc  540
gccaatgaag ccaaacagac ggccgaagaa accaaacaaa acgtcgatgc caaagtaaaa  600
gctgcagaaa ctgcagcagg caaagccgaa gctgccgctg gcacagctaa tactgcagcc  660
gacaaggccg aagctgtcgc tgcaaaagtt accgacatca aagctgatat cgctacgaac  720
aaagataata ttgctaaaaa agcaaacagt gccgacgtgt acaccagaga agagtctgac  780
agcaaatttg tcagaattga tggtctgaac gctactaccg aaaaattgga cacacgcttg  840
gcttctgctg aaaaatccat tgccgatcac gatactcgcc tgaacggttt ggataaaaca  900
gtgtcagacc tgcgcaaaga aacccgccaa ggccttgcag aacaagccgc gctctccggt  960
ctgttccaac cttacaacgt gggtggatcc ggcggaggcg gcacttctgc gcccgacttc 1020
aatgcaggcg gtaccggtat cggcagcaac agcagagcaa caacagcgaa atcagcagca 1080
gtatcttacg ccggtatcaa gaacgaaatg tgcaaagaca gaagcatgct ctgtgccggt 1140
cgggatgacg ttgcggttac agacagggat gccaaaatca atgccccccc cccgaatctg 1200
cataccggag actttccaaa cccaaatgac gcatacaaga atttgatcaa cctcaaacct 1260
gcaattgaag caggctatac aggacgcggg gtagaggtag gtatcgtcga cacaggcgaa 1320
tccgtcggca gcatatcctt tcccgaactg tatggcagaa aagaacacgg ctataacgaa 1380
aattacaaaa actatacggc gtatatgcgg aaggaagcgc ctgaagacgg aggcggtaaa 1440
gacattgaag cttctttcga cgatgaggcc gttatagaga ctgaagcaaa gccgacggat 1500
atccgccacg taaaagaaat cggacacatc gatttggtct cccatattat tggcgggcgt 1560
tccgtggacg gcagacctgc aggcggtatt gcgcccgatg cgacgctaca cataatgaat 1620
acgaatgatg aaaccaagaa cgaaatgatg gttgcagcca tccgcaatgc atgggtcaag 1680
ctgggcgaac gtggcgtgcg catcgtcaat aacagttttg gaacaacatc gagggcaggc 1740
actgccgacc ttttccaaat agccaattcg gaggagcagt accgccaagc gttgctcgac 1800
tattccggcg gtgataaaac agacgagggt atccgcctga tgcaacagag cgattacggc 1860
aacctgtcct accacatccg taataaaaac atgcttttca tcttttcgac aggcaatgac 1920
gcacaagctc agcccaacac atatgcccta ttgccatttt atgaaaaaga cgctcaaaaa 1980
ggcattatca cagtcgcagg cgtagaccgc agtggagaaa agttcaaacg ggaaatgtat 2040
ggagaaccgg gtacagaacc gcttgagtat ggctccaacc attgcggaat tactgccatg 2100
tggtgcctgt cggcacccta tgaagcaagc gtccgtttca cccgtacaaa cccgattcaa 2160
attgccggaa catccttttc cgcacccatc gtaaccggca cggcggctct gctgctgcag 2220
aaatacccgt ggatgagcaa cgacaacctg cgtaccacgt tgctgacgac ggctcaggac 2280
atcggtgcag tcggcgtgga cagcaagttc ggctggggac tgctggatgc gggtaaggcc 2340
atgaacggac ccgcgtcctt tccgttcggc gactttaccg ccgatacgaa aggtacatcc 2400
gatattgcct actccttccg taacgacatt tcaggcacgg gcggcctgat caaaaaaggc 2460
ggcagccaac tgcaactgca cggcaacaac acctatacgg gcaaaaccat tatcgaaggc 2520
ggttcgctgg tgttgtacgg caacaacaaa tcggatatgc gcgtcgaaac caaaggtgcg 2580
ctgatttata acggggcggc atccggcggc agcctgaaca gcgacggcat tgtctatctg 2640
gcagataccg accaatccgg cgcaaacgaa accgtacaca tcaaaggcag tctgcagctg 2700
gacggcaaag gtacgctgta cacacgtttg ggcaaactgc tgaaagtgga cggtacggcg 2760
attatcggcg gcaagctgta catgtcggca cgcggcaagg gggcaggcta tctcaacagt 2820
accggacgac gtgttccctt cctgagtgcc gccaaaatcg ggcaggatta ttctttcttc 2880
acaaacatcg aaaccgacgg cggcctgctg gcttccctcg acagcgtcga aaaaacagcg 2940
ggcagtgaag gcgacacgct gtcctattat gtccgtcgcg gcaatgcggc acggactgct 3000
tcggcagcgg cacattccgc gcccgccggt ctgaaacacg ccgtagaaca gggcggcagc 3060
aatctggaaa acctgatggt cgaactggat gcctccgaat catccgcaac acccgagacg 3120
```

```
gttgaaactg cggcagccga ccgcacagat atgccgggca tccgccccta cggcgcaact   3180
ttccgcgcag cggcagccgt acagcatgcg aatgccgccg acggtgtacg catcttcaac   3240
agtctcgccg ctaccgtcta tgccgacagt accgccgccc atgccgatat gcagggacgc   3300
cgcctgaaag ccgtatcgga cgggttggac cacaacggca cgggtctgcg cgtcatcgcg   3360
caaacccaac aggacggtgg aacgtgggaa cagggcggtg ttgaaggcaa aatgcgcggc   3420
agtacccaaa ccgtcggcat tgccgcgaaa accggcgaaa atacgacagc agccgccaca   3480
ctgggcatgg gacgcagcac atggagcgaa aacagtgcaa atgcaaaaac cgacagcatt   3540
agtctgtttg caggcatacg gcacgatgcg ggcgatatcg gctatctcaa aggcctgttc   3600
tcctacggac gctacaaaaa cagcatcagc cgcagcaccg gtgcggacga acatgcggaa   3660
ggcagcgtca acggcacgct gatgcagctg ggcgcactgg gcggtgtcaa cgttccgttt   3720
gccgcaacgg gagatttgac ggtcgaaggc ggtctgcgct acgacctgct caaacaggat   3780
gcattcgccg aaaaaggcag tgctttgggc tggagcggca acagcctcac tgaaggcacg   3840
ctggtcggac tcgcgggtct gaagctgtcg caacccttga gcgataaagc cgtcctgttt   3900
gcaacggcgg gcgtggaacg cgacctgaac ggacgcgact acacggtaac gggcggcttt   3960
accggcgcga ctgcagcaac cggcaagacg gggcacgca atatgccgca cacccgtctg    4020
gttgccggcc tgggcgcgga tgtcgaattc ggcaacggct ggaacggctt ggcacgttac   4080
agctacgccg gttccaaaca gtacggcaac cacagcggac gagtcggcgt aggctaccgg   4140
ttcctcgagc accaccacca ccaccactga                                    4170
```

<210> 52
<211> 1389
<212> PRT
<213> Artificial Sequence

<220>
<223> 961c-983

<400> 52

Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110

Ala Leu Asp Ala Thr Thr Asn Ala Leu Asn Lys Leu Gly Glu Asn Ile
        115                 120                 125

Thr Thr Phe Ala Glu Glu Thr Lys Thr Asn Ile Val Lys Ile Asp Glu
    130                 135                 140

Lys Leu Glu Ala Val Ala Asp Thr Val Asp Lys His Ala Glu Ala Phe
145                 150                 155                 160

Asn Asp Ile Ala Asp Ser Leu Asp Glu Thr Asn Thr Lys Ala Asp Glu
                165                 170                 175

```
Ala Val Lys Thr Ala Asn Glu Ala Lys Gln Thr Ala Glu Glu Thr Lys
            180                 185                 190

Gln Asn Val Asp Ala Lys Val Lys Ala Ala Glu Thr Ala Ala Gly Lys
        195                 200                 205

Ala Glu Ala Ala Ala Gly Thr Ala Asn Thr Ala Ala Asp Lys Ala Glu
    210                 215                 220

Ala Val Ala Ala Lys Val Thr Asp Ile Lys Ala Asp Ile Ala Thr Asn
225                 230                 235                 240

Lys Asp Asn Ile Ala Lys Lys Ala Asn Ser Ala Asp Val Tyr Thr Arg
                245                 250                 255

Glu Glu Ser Asp Ser Lys Phe Val Arg Ile Asp Gly Leu Asn Ala Thr
            260                 265                 270

Thr Glu Lys Leu Asp Thr Arg Leu Ala Ser Ala Glu Lys Ser Ile Ala
        275                 280                 285

Asp His Asp Thr Arg Leu Asn Gly Leu Asp Lys Thr Val Ser Asp Leu
    290                 295                 300

Arg Lys Glu Thr Arg Gln Gly Leu Ala Glu Gln Ala Ala Leu Ser Gly
305                 310                 315                 320

Leu Phe Gln Pro Tyr Asn Val Gly Gly Ser Gly Gly Gly Gly Thr Ser
                325                 330                 335

Ala Pro Asp Phe Asn Ala Gly Gly Thr Gly Ile Gly Ser Asn Ser Arg
            340                 345                 350

Ala Thr Thr Ala Lys Ser Ala Ala Val Ser Tyr Ala Gly Ile Lys Asn
        355                 360                 365

Glu Met Cys Lys Asp Arg Ser Met Leu Cys Ala Gly Arg Asp Asp Val
    370                 375                 380

Ala Val Thr Asp Arg Asp Ala Lys Ile Asn Ala Pro Pro Pro Asn Leu
385                 390                 395                 400

His Thr Gly Asp Phe Pro Asn Pro Asn Asp Ala Tyr Lys Asn Leu Ile
                405                 410                 415

Asn Leu Lys Pro Ala Ile Glu Ala Gly Tyr Thr Gly Arg Gly Val Glu
            420                 425                 430

Val Gly Ile Val Asp Thr Gly Glu Ser Val Gly Ser Ile Ser Phe Pro
        435                 440                 445

Glu Leu Tyr Gly Arg Lys Glu His Gly Tyr Asn Glu Asn Tyr Lys Asn
    450                 455                 460

Tyr Thr Ala Tyr Met Arg Lys Glu Ala Pro Glu Asp Gly Gly Gly Lys
465                 470                 475                 480

Asp Ile Glu Ala Ser Phe Asp Asp Glu Ala Val Ile Glu Thr Glu Ala
                485                 490                 495

Lys Pro Thr Asp Ile Arg His Val Lys Glu Ile Gly His Ile Asp Leu
```

500 505 510

Val Ser His Ile Ile Gly Gly Arg Ser Val Asp Gly Arg Pro Ala Gly
515 520 525

Gly Ile Ala Pro Asp Ala Thr Leu His Ile Met Asn Thr Asn Asp Glu
530 535 540

Thr Lys Asn Glu Met Met Val Ala Ala Ile Arg Asn Ala Trp Val Lys
545 550 555 560

Leu Gly Glu Arg Gly Val Arg Ile Val Asn Asn Ser Phe Gly Thr Thr
565 570 575

Ser Arg Ala Gly Thr Ala Asp Leu Phe Gln Ile Ala Asn Ser Glu Glu
580 585 590

Gln Tyr Arg Gln Ala Leu Leu Asp Tyr Ser Gly Gly Asp Lys Thr Asp
595 600 605

Glu Gly Ile Arg Leu Met Gln Gln Ser Asp Tyr Gly Asn Leu Ser Tyr
610 615 620

His Ile Arg Asn Lys Asn Met Leu Phe Ile Phe Ser Thr Gly Asn Asp
625 630 635 640

Ala Gln Ala Gln Pro Asn Thr Tyr Ala Leu Leu Pro Phe Tyr Glu Lys
645 650 655

Asp Ala Gln Lys Gly Ile Ile Thr Val Ala Gly Val Asp Arg Ser Gly
660 665 670

Glu Lys Phe Lys Arg Glu Met Tyr Gly Glu Pro Gly Thr Glu Pro Leu
675 680 685

Glu Tyr Gly Ser Asn His Cys Gly Ile Thr Ala Met Trp Cys Leu Ser
690 695 700

Ala Pro Tyr Glu Ala Ser Val Arg Phe Thr Arg Thr Asn Pro Ile Gln
705 710 715 720

Ile Ala Gly Thr Ser Phe Ser Ala Pro Ile Val Thr Gly Thr Ala Ala
725 730 735

Leu Leu Leu Gln Lys Tyr Pro Trp Met Ser Asn Asp Asn Leu Arg Thr
740 745 750

Thr Leu Leu Thr Thr Ala Gln Asp Ile Gly Ala Val Gly Val Asp Ser
755 760 765

Lys Phe Gly Trp Gly Leu Leu Asp Ala Gly Lys Ala Met Asn Gly Pro
770 775 780

Ala Ser Phe Pro Phe Gly Asp Phe Thr Ala Asp Thr Lys Gly Thr Ser
785 790 795 800

Asp Ile Ala Tyr Ser Phe Arg Asn Asp Ile Ser Gly Thr Gly Gly Leu
805 810 815

Ile Lys Lys Gly Gly Ser Gln Leu Gln Leu His Gly Asn Asn Thr Tyr
820 825 830

```
Thr Gly Lys Thr Ile Ile Glu Gly Gly Ser Leu Val Leu Tyr Gly Asn
        835                 840                 845

Asn Lys Ser Asp Met Arg Val Glu Thr Lys Gly Ala Leu Ile Tyr Asn
    850                 855                 860

Gly Ala Ala Ser Gly Gly Ser Leu Asn Ser Asp Gly Ile Val Tyr Leu
865                 870                 875                 880

Ala Asp Thr Asp Gln Ser Gly Ala Asn Glu Thr Val His Ile Lys Gly
                885                 890                 895

Ser Leu Gln Leu Asp Gly Lys Gly Thr Leu Tyr Thr Arg Leu Gly Lys
            900                 905                 910

Leu Leu Lys Val Asp Gly Thr Ala Ile Ile Gly Gly Lys Leu Tyr Met
        915                 920                 925

Ser Ala Arg Gly Lys Gly Ala Gly Tyr Leu Asn Ser Thr Gly Arg Arg
    930                 935                 940

Val Pro Phe Leu Ser Ala Ala Lys Ile Gly Gln Asp Tyr Ser Phe Phe
945                 950                 955                 960

Thr Asn Ile Glu Thr Asp Gly Gly Leu Leu Ala Ser Leu Asp Ser Val
                965                 970                 975

Glu Lys Thr Ala Gly Ser Glu Gly Asp Thr Leu Ser Tyr Tyr Val Arg
            980                 985                 990

Arg Gly Asn Ala Ala Arg Thr Ala Ser Ala Ala Ala His Ser Ala Pro
        995                 1000                1005

Ala Gly Leu Lys His Ala Val Glu Gln Gly Gly Ser Asn Leu Glu Asn
    1010                1015                1020

Leu Met Val Glu Leu Asp Ala Ser Glu Ser Ser Ala Thr Pro Glu Thr
1025                1030                1035                1040

Val Glu Thr Ala Ala Ala Asp Arg Thr Asp Met Pro Gly Ile Arg Pro
                1045                1050                1055

Tyr Gly Ala Thr Phe Arg Ala Ala Ala Ala Val Gln His Ala Asn Ala
            1060                1065                1070

Ala Asp Gly Val Arg Ile Phe Asn Ser Leu Ala Ala Thr Val Tyr Ala
        1075                1080                1085

Asp Ser Thr Ala Ala His Ala Asp Met Gln Gly Arg Arg Leu Lys Ala
    1090                1095                1100

Val Ser Asp Gly Leu Asp His Asn Gly Thr Gly Leu Arg Val Ile Ala
1105                1110                1115                1120

Gln Thr Gln Gln Asp Gly Gly Thr Trp Glu Gln Gly Gly Val Glu Gly
                1125                1130                1135

Lys Met Arg Gly Ser Thr Gln Thr Val Gly Ile Ala Ala Lys Thr Gly
            1140                1145                1150
```

```
Glu Asn Thr Thr Ala Ala Ala Thr Leu Gly Met Gly Arg Ser Thr Trp
        1155                1160                1165

Ser Glu Asn Ser Ala Asn Ala Lys Thr Asp Ser Ile Ser Leu Phe Ala
    1170                1175                1180

Gly Ile Arg His Asp Ala Gly Asp Ile Gly Tyr Leu Lys Gly Leu Phe
1185                1190                1195                1200

Ser Tyr Gly Arg Tyr Lys Asn Ser Ile Ser Arg Ser Thr Gly Ala Asp
                1205                1210                1215

Glu His Ala Glu Gly Ser Val Asn Gly Thr Leu Met Gln Leu Gly Ala
            1220                1225                1230

Leu Gly Gly Val Asn Val Pro Phe Ala Ala Thr Gly Asp Leu Thr Val
        1235                1240                1245

Glu Gly Gly Leu Arg Tyr Asp Leu Leu Lys Gln Asp Ala Phe Ala Glu
    1250                1255                1260

Lys Gly Ser Ala Leu Gly Trp Ser Gly Asn Ser Leu Thr Glu Gly Thr
1265                1270                1275                1280

Leu Val Gly Leu Ala Gly Leu Lys Leu Ser Gln Pro Leu Ser Asp Lys
                1285                1290                1295

Ala Val Leu Phe Ala Thr Ala Gly Val Glu Arg Asp Leu Asn Gly Arg
            1300                1305                1310

Asp Tyr Thr Val Thr Gly Gly Phe Thr Gly Ala Thr Ala Ala Thr Gly
        1315                1320                1325

Lys Thr Gly Ala Arg Asn Met Pro His Thr Arg Leu Val Ala Gly Leu
    1330                1335                1340

Gly Ala Asp Val Glu Phe Gly Asn Gly Trp Asn Gly Leu Ala Arg Tyr
1345                1350                1355                1360

Ser Tyr Ala Gly Ser Lys Gln Tyr Gly Asn His Ser Gly Arg Val Gly
                1365                1370                1375

Val Gly Tyr Arg Phe Leu Glu His His His His His His
            1380                1385
```

<210> 53
<211> 2304
<212> DNA
<213> Artificial Sequence

<220>
<223> 961cL-ORF46.1

<400> 53

```
atgaaacact ttccatccaa agtactgacc acagccatcc ttgccacttt ctgtagcggc   60
gcactggcag ccacaaacga cgacgatgtt aaaaaagctg ccactgtggc cattgctgct  120
gcctacaaca atggccaaga aatcaacggt ttcaaagctg gagagaccat ctacgacatt  180
gatgaagacg gcacaattac caaaaaagac gcaactgcag ccgatgttga agccgacgac  240
tttaaaggtc tgggtctgaa aaaagtcgtg actaacctga ccaaaaccgt caatgaaaac  300
aaacaaaacg tcgatgccaa agtaaaagct gcagaatctg aaatagaaaa gttaacaacc  360
aagttagcag acactgatgc cgctttagca gatactgatg ccgctctgga tgcaaccacc  420

aacgccttga ataaattggg agaaaatata acgacatttg ctgaagagac taagacaaat  480
atcgtaaaaa ttgatgaaaa attagaagcc gtggctgata ccgtcgacaa gcatgccgaa  540
gcattcaacg atatcgccga ttcattggat gaaaccaaca ctaaggcaga cgaagccgtc  600
aaaaccgcca atgaagccaa acagacggcc gaagaaacca aacaaaacgt cgatgccaaa  660
gtaaaagctg cagaaactgc agcaggcaaa gccgaagctg ccgctggcac agctaatact  720
gcagccgaca aggccgaagc tgtcgctgca aaagttaccg acatcaaagc tgatatcgct  780
acgaacaaag ataatattgc taaaaaagca aacagtgccg acgtgtacac cagagaagag  840
tctgacagca aatttgtcag aattgatggt ctgaacgcta ctaccgaaaa attggacaca  900
cgcttggctt ctgctgaaaa atccattgcc gatcacgata ctcgcctgaa cggtttggat  960
aaaacagtgt cagacctgcg caaagaaacc cgccaaggcc ttgcagaaca agccgcgctc 1020
tccggtctgt tccaacctta caacgtgggt ggatccggag gaggaggatc agatttggca 1080
aacgattctt ttatccggca ggttctcgac cgtcagcatt tcgaacccga cgggaaatac 1140
cacctattcg gcagcagggg ggaacttgcc gagcgcagcg gccatatcgg attgggaaaa 1200
atacaaagcc atcagttggg caacctgatg attcaacagg cggccattaa aggaaatatc 1260
ggctacattg tccgcttttc cgatcacggg cacgaagtcc attccccctt cgacaaccat 1320
gcctcacatt ccgattctga tgaagccggt agtcccgttg acggatttag cctttaccgc 1380
atccattggg acggatacga acaccatccc gccgacggct atgacgggcc acagggcggc 1440
ggctatcccg ctcccaaagg cgcgagggat atatacagct acgacataaa aggcgttgcc 1500
caaaatatcc gcctcaacct gaccgacaac cgcagcaccg gacaacggct tgccgaccgt 1560
ttccacaatg ccggtagtat gctgacgcaa ggagtaggcg acggattcaa acgcgccacc 1620
cgatacagcc ccgagctgga cagatcgggc aatgccgccg aagccttcaa cggcactgca 1680
gatatcgtta aaaacatcat cggcgcggca ggagaaattg tcggcgcagg cgatgccgtg 1740
cagggcataa gcgaaggctc aaacattgct gtcatgcacg gcttgggtct gctttccacc 1800
gaaaacaaga tggcgcgcat caacgatttg gcagatatgg cgcaactcaa agactatgcc 1860
gcagcagcca tccgcgattg ggcagtccaa aaccccaatg ccgcacaagg catagaagcc 1920
gtcagcaata tctttatggc agccatcccc atcaaaggga ttggagctgt tcggggaaaa 1980
tacggcttgg gcggcatcac ggcacatcct atcaagcggt cgcagatggg cgcgatcgca 2040
ttgccgaaag ggaaatccgc cgtcagcgac aattttgccg atgcggcata cgccaaatac 2100
ccgtcccctt accattcccg aaatatccgt tcaaacttgg agcagcgtta cggcaaagaa 2160
aacatcacct cctcaaccgt gccgccgtca aacggcaaaa atgtcaaact ggcagaccaa 2220
cgccacccga agacaggcgt accgtttgac ggtaaagggt ttccgaattt tgagaagcac 2280
gtgaaatatg atacgtaact cgag                                        2304
```

<210> 54
<211> 765
<212> PRT
<213> Artificial Sequence

<220>
<223> 961cL-ORF46.1

<400> 54

Met Lys His Phe Pro Ser Lys Val Leu Thr Thr Ala Ile Leu Ala Thr
1 5 10 15

Phe Cys Ser Gly Ala Leu Ala Ala Thr Asn Asp Asp Asp Val Lys Lys
20 25 30

Ala Ala Thr Val Ala Ile Ala Ala Ala Tyr Asn Asn Gly Gln Glu Ile
35 40 45

Asn Gly Phe Lys Ala Gly Glu Thr Ile Tyr Asp Ile Asp Glu Asp Gly
50 55 60

Thr Ile Thr Lys Lys Asp Ala Thr Ala Ala Asp Val Glu Ala Asp Asp
65 70 75 80

Phe Lys Gly Leu Gly Leu Lys Lys Val Val Thr Asn Leu Thr Lys Thr
85 90 95

Val Asn Glu Asn Lys Gln Asn Val Asp Ala Lys Val Lys Ala Ala Glu

100 105 110

Ser Glu Ile Glu Lys Leu Thr Thr Lys Leu Ala Asp Thr Asp Ala Ala
115 120 125

Leu Ala Asp Thr Asp Ala Ala Leu Asp Ala Thr Thr Asn Ala Leu Asn
130 135 140

Lys Leu Gly Glu Asn Ile Thr Thr Phe Ala Glu Glu Thr Lys Thr Asn
145 150 155 160

Ile Val Lys Ile Asp Glu Lys Leu Glu Ala Val Ala Asp Thr Val Asp
165 170 175

Lys His Ala Glu Ala Phe Asn Asp Ile Ala Asp Ser Leu Asp Glu Thr
180 185 190

Asn Thr Lys Ala Asp Glu Ala Val Lys Thr Ala Asn Glu Ala Lys Gln
195 200 205

Thr Ala Glu Glu Thr Lys Gln Asn Val Asp Ala Lys Val Lys Ala Ala
210 215 220

Glu Thr Ala Ala Gly Lys Ala Glu Ala Ala Ala Gly Thr Ala Asn Thr
225 230 235 240

Ala Ala Asp Lys Ala Glu Ala Val Ala Ala Lys Val Thr Asp Ile Lys
245 250 255

Ala Asp Ile Ala Thr Asn Lys Asp Asn Ile Ala Lys Lys Ala Asn Ser
260 265 270

Ala Asp Val Tyr Thr Arg Glu Glu Ser Asp Ser Lys Phe Val Arg Ile
275 280 285

Asp Gly Leu Asn Ala Thr Thr Glu Lys Leu Asp Thr Arg Leu Ala Ser
290 295 300

Ala Glu Lys Ser Ile Ala Asp His Asp Thr Arg Leu Asn Gly Leu Asp
305 310 315 320

Lys Thr Val Ser Asp Leu Arg Lys Glu Thr Arg Gln Gly Leu Ala Glu
325 330 335

Gln Ala Ala Leu Ser Gly Leu Phe Gln Pro Tyr Asn Val Gly Gly Ser
340 345 350

Gly Gly Gly Gly Ser Asp Leu Ala Asn Asp Ser Phe Ile Arg Gln Val
355 360 365

Leu Asp Arg Gln His Phe Glu Pro Asp Gly Lys Tyr His Leu Phe Gly
370 375 380

Ser Arg Gly Glu Leu Ala Glu Arg Ser Gly His Ile Gly Leu Gly Lys
385 390 395 400

Ile Gln Ser His Gln Leu Gly Asn Leu Met Ile Gln Gln Ala Ala Ile
405 410 415

Lys Gly Asn Ile Gly Tyr Ile Val Arg Phe Ser Asp His Gly His Glu
420 425 430

Val His Ser Pro Phe Asp Asn His Ala Ser His Ser Asp Ser Asp Glu
435 440 445

Ala Gly Ser Pro Val Asp Gly Phe Ser Leu Tyr Arg Ile His Trp Asp
450 455 460

Gly Tyr Glu His His Pro Ala Asp Gly Tyr Asp Gly Pro Gln Gly Gly
465 470 475 480

Gly Tyr Pro Ala Pro Lys Gly Ala Arg Asp Ile Tyr Ser Tyr Asp Ile
485 490 495

Lys Gly Val Ala Gln Asn Ile Arg Leu Asn Leu Thr Asp Asn Arg Ser
500 505 510

Thr Gly Gln Arg Leu Ala Asp Arg Phe His Asn Ala Gly Ser Met Leu
515 520 525

Thr Gln Gly Val Gly Asp Gly Phe Lys Arg Ala Thr Arg Tyr Ser Pro
530 535 540

Glu Leu Asp Arg Ser Gly Asn Ala Ala Glu Ala Phe Asn Gly Thr Ala
545 550 555 560

Asp Ile Val Lys Asn Ile Ile Gly Ala Ala Gly Glu Ile Val Gly Ala
565 570 575

Gly Asp Ala Val Gln Gly Ile Ser Glu Gly Ser Asn Ile Ala Val Met
580 585 590

His Gly Leu Gly Leu Leu Ser Thr Glu Asn Lys Met Ala Arg Ile Asn
595 600 605

Asp Leu Ala Asp Met Ala Gln Leu Lys Asp Tyr Ala Ala Ala Ala Ile
610 615 620

Arg Asp Trp Ala Val Gln Asn Pro Asn Ala Ala Gln Gly Ile Glu Ala
625 630 635 640

Val Ser Asn Ile Phe Met Ala Ala Ile Pro Ile Lys Gly Ile Gly Ala
645 650 655

Val Arg Gly Lys Tyr Gly Leu Gly Gly Ile Thr Ala His Pro Ile Lys
660 665 670

Arg Ser Gln Met Gly Ala Ile Ala Leu Pro Lys Gly Lys Ser Ala Val
675 680 685

Ser Asp Asn Phe Ala Asp Ala Ala Tyr Ala Lys Tyr Pro Ser Pro Tyr
690 695 700

His Ser Arg Asn Ile Arg Ser Asn Leu Glu Gln Arg Tyr Gly Lys Glu
705 710 715 720

Asn Ile Thr Ser Ser Thr Val Pro Pro Ser Asn Gly Lys Asn Val Lys
725 730 735

Leu Ala Asp Gln Arg His Pro Lys Thr Gly Val Pro Phe Asp Gly Lys
740 745 750

```
Gly Phe Pro Asn Phe Glu Lys His Val Lys Tyr Asp Thr
        755                 760                 765
```

<210> 55
<211> 1839
<212> DNA
<213> Artificial Sequence

<220>
<223> 961cL-741

<400> 55

```
atgaaacact ttccatccaa agtactgacc acagccatcc ttgccacttt ctgtagcggc   60
gcactggcag ccacaaacga cgacgatgtt aaaaaagctg ccactgtggc cattgctgct   120
gcctacaaca atggccaaga aatcaacggt ttcaaagctg gagagaccat ctacgacatt   180
gatgaagacg gcacaattac caaaaaagac gcaactgcag ccgatgttga agccgacgac   240
tttaaaggtc tgggtctgaa aaaagtcgtg actaacctga ccaaaaccgt caatgaaaac   300
aaacaaaacg tcgatgccaa agtaaaagct gcagaatctg aaatagaaaa gttaacaacc   360
aagttagcag acactgatgc cgctttagca gatactgatg ccgctctgga tgcaaccacc   420
aacgccttga ataaattggg agaaaatata acgacatttg ctgaagagac taagacaaat   480
atcgtaaaaa ttgatgaaaa attagaagcc gtggctgata ccgtcgacaa gcatgccgaa   540
gcattcaacg atatcgccga ttcattggat gaaaccaaca ctaaggcaga cgaagccgtc   600
aaaaccgcca atgaagccaa acagacggcc gaagaaacca aacaaaacgt cgatgccaaa   660
gtaaaagctg cagaaactgc agcaggcaaa gccgaagctg ccgctggcac agctaatact   720
gcagccgaca aggccgaagc tgtcgctgca aaagttaccg acatcaaagc tgatatcgct   780
acgaacaaag ataatattgc taaaaaagca aacagtgccg acgtgtacac cagagaagag   840
tctgacagca aatttgtcag aattgatggt ctgaacgcta ctaccgaaaa attggacaca   900
cgcttggctt ctgctgaaaa atccattgcc gatcacgata ctcgcctgaa cggtttggat   960
aaaacagtgt cagacctgcg caaagaaacc cgccaaggcc ttgcagaaca agccgcgctc   1020
tccggtctgt tccaacctta caacgtgggt ggatccggag ggggtggtgt cgccgccgac   1080
atcggtgcgg ggcttgccga tgcactaacc gcaccgctcg accataaaga caaaggtttg   1140
cagtctttga cgctggatca gtccgtcagg aaaaacgaga aactgaagct ggcggcacaa   1200
ggtgcggaaa aaacttatgg aaacggtgac agcctcaata cgggcaaatt gaagaacgac   1260
aaggtcagcc gtttcgactt tatccgccaa atcgaagtgg acgggcagct cattaccttg   1320
gagagtggag agttccaagt atacaaacaa agccattccg ccttaaccgc ctttcagacc   1380
gagcaaatac aagattcgga gcattccggg aagatggttg cgaaacgcca gttcagaatc   1440
ggcgacatag cgggcgaaca tacatctttt gacaagcttc ccgaaggcgg cagggcgaca   1500
tatcgcggga cggcgttcgg ttcagacgat gccggcggaa aactgaccta caccatagat   1560
ttcgccgcca agcagggaaa cggcaaaatc gaacatttga aatcgccaga actcaatgtc   1620
gacctggccg ccgccgatat caagccggat ggaaaacgcc atgccgtcat cagcggttcc   1680
gtcctttaca accaagccga gaaaggcagt tactccctcg gtatctttgg cggaaaagcc   1740
caggaagttg ccggcagcgc ggaagtgaaa accgtaaacg gcatacgcca tatcggcctt   1800
gccgccaagc aactcgagca ccaccaccac caccactga                          1839
```

<210> 56
<211> 612
<212> PRT
<213> Artificial Sequence

<220>
<223> 961cL-741

<400> 56

```
Met Lys His Phe Pro Ser Lys Val Leu Thr Thr Ala Ile Leu Ala Thr
1               5                   10                  15

Phe Cys Ser Gly Ala Leu Ala Ala Thr Asn Asp Asp Asp Val Lys Lys
            20                  25                  30

Ala Ala Thr Val Ala Ile Ala Ala Ala Tyr Asn Asn Gly Gln Glu Ile
        35                  40                  45
```

```
Asn Gly Phe Lys Ala Gly Glu Thr Ile Tyr Asp Ile Asp Glu Asp Gly
    50                  55                  60

Thr Ile Thr Lys Lys Asp Ala Thr Ala Ala Asp Val Glu Ala Asp Asp
65                  70                  75                  80

Phe Lys Gly Leu Gly Leu Lys Lys Val Val Thr Asn Leu Thr Lys Thr
                85                  90                  95

Val Asn Glu Asn Lys Gln Asn Val Asp Ala Lys Val Lys Ala Ala Glu
            100                 105                 110

Ser Glu Ile Glu Lys Leu Thr Thr Lys Leu Ala Asp Thr Asp Ala Ala
        115                 120                 125

Leu Ala Asp Thr Asp Ala Ala Leu Asp Ala Thr Thr Asn Ala Leu Asn
    130                 135                 140

Lys Leu Gly Glu Asn Ile Thr Thr Phe Ala Glu Glu Thr Lys Thr Asn
145                 150                 155                 160

Ile Val Lys Ile Asp Glu Lys Leu Glu Ala Val Ala Asp Thr Val Asp
                165                 170                 175

Lys His Ala Glu Ala Phe Asn Asp Ile Ala Asp Ser Leu Asp Glu Thr
            180                 185                 190

Asn Thr Lys Ala Asp Glu Ala Val Lys Thr Ala Asn Glu Ala Lys Gln
        195                 200                 205

Thr Ala Glu Glu Thr Lys Gln Asn Val Asp Ala Lys Val Lys Ala Ala
    210                 215                 220

Glu Thr Ala Ala Gly Lys Ala Glu Ala Ala Ala Gly Thr Ala Asn Thr
225                 230                 235                 240

Ala Ala Asp Lys Ala Glu Ala Val Ala Ala Lys Val Thr Asp Ile Lys
                245                 250                 255

Ala Asp Ile Ala Thr Asn Lys Asp Asn Ile Ala Lys Lys Ala Asn Ser
            260                 265                 270

Ala Asp Val Tyr Thr Arg Glu Glu Ser Asp Ser Lys Phe Val Arg Ile
        275                 280                 285

Asp Gly Leu Asn Ala Thr Thr Glu Lys Leu Asp Thr Arg Leu Ala Ser
    290                 295                 300

Ala Glu Lys Ser Ile Ala Asp His Asp Thr Arg Leu Asn Gly Leu Asp
305                 310                 315                 320

Lys Thr Val Ser Asp Leu Arg Lys Glu Thr Arg Gln Gly Leu Ala Glu
                325                 330                 335

Gln Ala Ala Leu Ser Gly Leu Phe Gln Pro Tyr Asn Val Gly Gly Ser
            340                 345                 350

Gly Gly Gly Gly Val Ala Ala Asp Ile Gly Ala Gly Leu Ala Asp Ala
        355                 360                 365
```

```
Leu Thr Ala Pro Leu Asp His Lys Asp Lys Gly Leu Gln Ser Leu Thr
    370                 375                 380

Leu Asp Gln Ser Val Arg Lys Asn Glu Lys Leu Lys Leu Ala Ala Gln
385                 390                 395                 400

Gly Ala Glu Lys Thr Tyr Gly Asn Gly Asp Ser Leu Asn Thr Gly Lys
                405                 410                 415

Leu Lys Asn Asp Lys Val Ser Arg Phe Asp Phe Ile Arg Gln Ile Glu
            420                 425                 430

Val Asp Gly Gln Leu Ile Thr Leu Glu Ser Gly Glu Phe Gln Val Tyr
        435                 440                 445

Lys Gln Ser His Ser Ala Leu Thr Ala Phe Gln Thr Glu Gln Ile Gln
    450                 455                 460

Asp Ser Glu His Ser Gly Lys Met Val Ala Lys Arg Gln Phe Arg Ile
465                 470                 475                 480

Gly Asp Ile Ala Gly Glu His Thr Ser Phe Asp Lys Leu Pro Glu Gly
                485                 490                 495

Gly Arg Ala Thr Tyr Arg Gly Thr Ala Phe Gly Ser Asp Asp Ala Gly
            500                 505                 510

Gly Lys Leu Thr Tyr Thr Ile Asp Phe Ala Ala Lys Gln Gly Asn Gly
        515                 520                 525

Lys Ile Glu His Leu Lys Ser Pro Glu Leu Asn Val Asp Leu Ala Ala
    530                 535                 540

Ala Asp Ile Lys Pro Asp Gly Lys Arg His Ala Val Ile Ser Gly Ser
545                 550                 555                 560

Val Leu Tyr Asn Gln Ala Glu Lys Gly Ser Tyr Ser Leu Gly Ile Phe
                565                 570                 575

Gly Gly Lys Ala Gln Glu Val Ala Gly Ser Ala Glu Val Lys Thr Val
            580                 585                 590

Asn Gly Ile Arg His Ile Gly Leu Ala Ala Lys Gln Leu Glu His His
        595                 600                 605

His His His His
    610
```

<210> 57
<211> 4218
<212> DNA
<213> Artificial Sequence

<220>
<223> 961cL-983

<400> 57

```
atgaaacact ttccatccaa agtactgacc acagccatcc ttgccacttt ctgtagcggc   60
gcactggcag ccacaaacga cgacgatgtt aaaaaagctg ccactgtggc cattgctgct  120
gcctacaaca atggccaaga aatcaacggt ttcaaagctg gagagaccat ctacgacatt  180
gatgaagacg gcacaattac caaaaaagac gcaactgcag ccgatgttga agccgacgac  240
```

tttaaaggtc tgggtctgaa aaaagtcgtg actaacctga ccaaaaccgt caatgaaaac 300
aaacaaaacg tcgatgccaa agtaaaagct gcagaatctg aaatagaaaa gttaacaacc 360
aagttagcag acactgatgc cgctttagca gatactgatg ccgctctgga tgcaaccacc 420
aacgccttga ataaattggg agaaaatata acgacatttg ctgaagagac taagacaaat 480
atcgtaaaaa ttgatgaaaa attagaagcc gtggctgata ccgtcgacaa gcatgccgaa 540
gcattcaacg atatcgccga ttcattggat gaaaccaaca ctaaggcaga cgaagccgtc 600
aaaaccgcca atgaagccaa acagacggcc gaagaaacca aacaaaacgt cgatgccaaa 660
gtaaaagctg cagaaactgc agcaggcaaa gccgaagctg ccgctggcac agctaatact 720
gcagccgaca aggccgaagc tgtcgctgca aaagttaccg acatcaaagc tgatatcgct 780
acgaacaaag ataatattgc taaaaaagca aacagtgccg acgtgtacac cagagaagag 840
tctgacagca aatttgtcag aattgatggt ctgaacgcta ctaccgaaaa attggacaca 900
cgcttggctt ctgctgaaaa atccattgcc gatcacgata ctcgcctgaa cggtttggat 960
aaaacagtgt cagacctgcg caaagaaacc cgccaaggcc ttgcagaaca agccgcgctc 1020
tccggtctgt tccaacctta caacgtgggt ggatccggcg gaggcggcac ttctgcgccc 1080
gacttcaatg caggcggtac cggtatcggc agcaacagca gagcaacaac agcgaaatca 1140
gcagcagtat cttacgccgg tatcaagaac gaaatgtgca aagacagaag catgctctgt 1200
gccggtcggg atgacgttgc ggttacagac agggatgcca aaatcaatgc cccccccccg 1260
aatctgcata ccggagactt tccaaaccca aatgacgcat acaagaattt gatcaacctc 1320
aaacctgcaa ttgaagcagg ctatacagga cgcggggtag aggtaggtat cgtcgacaca 1380
ggcgaatccg tcggcagcat atcctttccc gaactgtatg gcagaaaaga acacggctat 1440
aacgaaaatt acaaaaacta tacggcgtat atgcggaagg aagcgcctga agacggaggc 1500
ggtaaagaca ttgaagcttc tttcgacgat gaggccgtta tagagactga agcaaagccg 1560
acggatatcc gccacgtaaa agaaatcgga cacatcgatt tggtctccca tattattggc 1620
gggcgttccg tggacggcag acctgcaggc ggtattgcgc ccgatgcgac gctacacata 1680
atgaatacga atgatgaaac caagaacgaa atgatggttg cagccatccg caatgcatgg 1740
gtcaagctgg gcgaacgtgg cgtgcgcatc gtcaataaca gttttggaac aacatcgagg 1800
gcaggcactg ccgacctttt ccaaatagcc aattcggagg agcagtaccg ccaagcgttg 1860
ctcgactatt ccggcggtga taaaacagac gagggtatcc gcctgatgca acagagcgat 1920
tacggcaacc tgtcctacca catccgtaat aaaaacatgc ttttcatctt ttcgacaggc 1980
aatgacgcac aagctcagcc caacacatat gccctattgc cattttatga aaaagacgct 2040
caaaaaggca ttatcacagt cgcaggcgta gaccgcagtg gagaaaagtt caaacgggaa 2100
atgtatggag aaccgggtac agaaccgctt gagtatggct ccaaccattg cggaattact 2160
gccatgtggt gcctgtcggc accctatgaa gcaagcgtcc gtttcacccg tacaaacccg 2220
attcaaattg ccggaacatc cttttccgca cccatcgtaa ccggcacggc ggctctgctg 2280
ctgcagaaat acccgtggat gagcaacgac aacctgcgta ccacgttgct gacgacggct 2340
caggacatcg gtgcagtcgg cgtggacagc aagttcggct ggggactgct ggatgcgggt 2400
aaggccatga acggacccgc gtcctttccg ttcggcgact ttaccgccga tacgaaaggt 2460
acatccgata ttgcctactc cttccgtaac gacatttcag gcacgggcgg cctgatcaaa 2520
aaaggcggca gccaactgca actgcacggc aacaacacct atacgggcaa aaccattatc 2580
gaaggcggtt cgctggtgtt gtacggcaac aacaaatcgg atatgcgcgt cgaaaccaaa 2640
ggtgcgctga tttataacgg ggcggcatcc ggcggcagcc tgaacagcga cggcattgtc 2700
tatctggcag ataccgacca atccggcgca aacgaaaccg tacacatcaa aggcagtctg 2760
cagctggacg gcaaaggtac gctgtacaca cgtttgggca aactgctgaa agtggacggt 2820
acggcgatta tcggcggcaa gctgtacatg tcggcacgcg gcaagggggc aggctatctc 2880
aacagtaccg gacgacgtgt tcccttcctg agtgccgcca aaatcgggca ggattattct 2940
ttcttcacaa acatcgaaac cgacggcggc ctgctggctt ccctcgacag cgtcgaaaaa 3000
acagcgggca gtgaaggcga cacgctgtcc tattatgtcc gtcgcggcaa tgcggcacgg 3060
actgcttcgg cagcggcaca ttccgcgccc gccggtctga aacacgccgt agaacagggc 3120
ggcagcaatc tggaaaacct gatggtcgaa ctggatgcct ccgaatcatc cgcaacaccc 3180
gagacggttg aaactgcggc agccgaccgc acagatatgc cgggcatccg cccctacggc 3240
gcaactttcc gcgcagcggc agccgtacag catgcgaatg ccgccgacgg tgtacgcatc 3300
ttcaacagtc tcgccgctac cgtctatgcc gacagtaccg ccgcccatgc cgatatgcag 3360
ggacgccgcc tgaaagccgt atcggacggg ttggaccaca acggcacggg tctgcgcgtc 3420
atcgcgcaaa cccaacagga cggtggaacg tgggaacagg gcggtgttga aggcaaaatg 3480
cgcggcagta cccaaaccgt cggcattgcc gcgaaaaccg gcgaaaatac gacagcagcc 3540
gccacactgg gcatgggacg cagcacatgg agcgaaaaca gtgcaaatgc aaaaaccgac 3600
agcattagtc tgtttgcagg catacggcac gatgcgggcg atatcggcta tctcaaaggc 3660
ctgttctcct acggacgcta caaaaacagc atcagccgca gcaccggtgc ggacgaacat 3720
gcggaaggca gcgtcaacgg cacgctgatg cagctgggcg cactgggcgg tgtcaacgtt 3780
ccgtttgccg caacgggaga tttgacggtc gaaggcggtc tgcgctacga cctgctcaaa 3840
caggatgcat tcgccgaaaa aggcagtgct ttgggctgga gcggcaacag cctcactgaa 3900

```
ggcacgctgg tcggactcgc gggtctgaag ctgtcgcaac ccttgagcga taaagccgtc   3960
ctgtttgcaa cggcgggcgt ggaacgcgac ctgaacggac gcgactacac ggtaacgggc   4020
ggctttaccg gcgcgactgc agcaaccggc aagacggggg cacgcaatat gccgcacacc   4080
cgtctggttg ccggcctggg cgcggatgtc gaattcggca acggctggaa cggcttggca   4140
cgttacagct acgccggttc caaacagtac ggcaaccaca gcggacgagt cggcgtaggc   4200
taccggttct gactcgag                                                 4218
```

<210> 58
<211> 1403
<212> PRT
<213> Artificial Sequence

<220>
<223> 961cL-983

<400> 58

```
Met Lys His Phe Pro Ser Lys Val Leu Thr Thr Ala Ile Leu Ala Thr
1               5                   10                  15

Phe Cys Ser Gly Ala Leu Ala Ala Thr Asn Asp Asp Asp Val Lys Lys
            20                  25                  30

Ala Ala Thr Val Ala Ile Ala Ala Ala Tyr Asn Asn Gly Gln Glu Ile
        35                  40                  45

Asn Gly Phe Lys Ala Gly Glu Thr Ile Tyr Asp Ile Asp Glu Asp Gly
    50                  55                  60

Thr Ile Thr Lys Lys Asp Ala Thr Ala Ala Asp Val Glu Ala Asp Asp
65                  70                  75                  80

Phe Lys Gly Leu Gly Leu Lys Lys Val Val Thr Asn Leu Thr Lys Thr
                85                  90                  95

Val Asn Glu Asn Lys Gln Asn Val Asp Ala Lys Val Lys Ala Ala Glu
            100                 105                 110

Ser Glu Ile Glu Lys Leu Thr Thr Lys Leu Ala Asp Thr Asp Ala Ala
        115                 120                 125

Leu Ala Asp Thr Asp Ala Ala Leu Asp Ala Thr Thr Asn Ala Leu Asn
    130                 135                 140

Lys Leu Gly Glu Asn Ile Thr Thr Phe Ala Glu Glu Thr Lys Thr Asn
145                 150                 155                 160

Ile Val Lys Ile Asp Glu Lys Leu Glu Ala Val Ala Asp Thr Val Asp
                165                 170                 175

Lys His Ala Glu Ala Phe Asn Asp Ile Ala Asp Ser Leu Asp Glu Thr
            180                 185                 190

Asn Thr Lys Ala Asp Glu Ala Val Lys Thr Ala Asn Glu Ala Lys Gln
        195                 200                 205

Thr Ala Glu Glu Thr Lys Gln Asn Val Asp Ala Lys Val Lys Ala Ala
    210                 215                 220

Glu Thr Ala Ala Gly Lys Ala Glu Ala Ala Ala Gly Thr Ala Asn Thr
225                 230                 235                 240
```

Ala Ala Asp Lys Ala Glu Ala Val Ala Ala Lys Val Thr Asp Ile Lys
245 250 255

Ala Asp Ile Ala Thr Asn Lys Asp Asn Ile Ala Lys Lys Ala Asn Ser
260 265 270

Ala Asp Val Tyr Thr Arg Glu Glu Ser Asp Ser Lys Phe Val Arg Ile
275 280 285

Asp Gly Leu Asn Ala Thr Thr Glu Lys Leu Asp Thr Arg Leu Ala Ser
290 295 300

Ala Glu Lys Ser Ile Ala Asp His Asp Thr Arg Leu Asn Gly Leu Asp
305 310 315 320

Lys Thr Val Ser Asp Leu Arg Lys Glu Thr Arg Gln Gly Leu Ala Glu
325 330 335

Gln Ala Ala Leu Ser Gly Leu Phe Gln Pro Tyr Asn Val Gly Gly Ser
340 345 350

Gly Gly Gly Gly Thr Ser Ala Pro Asp Phe Asn Ala Gly Gly Thr Gly
355 360 365

Ile Gly Ser Asn Ser Arg Ala Thr Thr Ala Lys Ser Ala Ala Val Ser
370 375 380

Tyr Ala Gly Ile Lys Asn Glu Met Cys Lys Asp Arg Ser Met Leu Cys
385 390 395 400

Ala Gly Arg Asp Asp Val Ala Val Thr Asp Arg Asp Ala Lys Ile Asn
405 410 415

Ala Pro Pro Pro Asn Leu His Thr Gly Asp Phe Pro Asn Pro Asn Asp
420 425 430

Ala Tyr Lys Asn Leu Ile Asn Leu Lys Pro Ala Ile Glu Ala Gly Tyr
435 440 445

Thr Gly Arg Gly Val Glu Val Gly Ile Val Asp Thr Gly Glu Ser Val
450 455 460

Gly Ser Ile Ser Phe Pro Glu Leu Tyr Gly Arg Lys Glu His Gly Tyr
465 470 475 480

Asn Glu Asn Tyr Lys Asn Tyr Thr Ala Tyr Met Arg Lys Glu Ala Pro
485 490 495

Glu Asp Gly Gly Gly Lys Asp Ile Glu Ala Ser Phe Asp Asp Glu Ala
500 505 510

Val Ile Glu Thr Glu Ala Lys Pro Thr Asp Ile Arg His Val Lys Glu
515 520 525

Ile Gly His Ile Asp Leu Val Ser His Ile Ile Gly Gly Arg Ser Val
530 535 540

Asp Gly Arg Pro Ala Gly Gly Ile Ala Pro Asp Ala Thr Leu His Ile
545 550 555 560

Met Asn Thr Asn Asp Glu Thr Lys Asn Glu Met Met Val Ala Ala Ile

565 570 575

Arg Asn Ala Trp Val Lys Leu Gly Glu Arg Gly Val Arg Ile Val Asn
580 585 590

Asn Ser Phe Gly Thr Thr Ser Arg Ala Gly Thr Ala Asp Leu Phe Gln
595 600 605

Ile Ala Asn Ser Glu Glu Gln Tyr Arg Gln Ala Leu Leu Asp Tyr Ser
610 615 620

Gly Gly Asp Lys Thr Asp Glu Gly Ile Arg Leu Met Gln Gln Ser Asp
625 630 635 640

Tyr Gly Asn Leu Ser Tyr His Ile Arg Asn Lys Asn Met Leu Phe Ile
645 650 655

Phe Ser Thr Gly Asn Asp Ala Gln Ala Gln Pro Asn Thr Tyr Ala Leu
660 665 670

Leu Pro Phe Tyr Glu Lys Asp Ala Gln Lys Gly Ile Ile Thr Val Ala
675 680 685

Gly Val Asp Arg Ser Gly Glu Lys Phe Lys Arg Glu Met Tyr Gly Glu
690 695 700

Pro Gly Thr Glu Pro Leu Glu Tyr Gly Ser Asn His Cys Gly Ile Thr
705 710 715 720

Ala Met Trp Cys Leu Ser Ala Pro Tyr Glu Ala Ser Val Arg Phe Thr
725 730 735

Arg Thr Asn Pro Ile Gln Ile Ala Gly Thr Ser Phe Ser Ala Pro Ile
740 745 750

Val Thr Gly Thr Ala Ala Leu Leu Leu Gln Lys Tyr Pro Trp Met Ser
755 760 765

Asn Asp Asn Leu Arg Thr Thr Leu Leu Thr Thr Ala Gln Asp Ile Gly
770 775 780

Ala Val Gly Val Asp Ser Lys Phe Gly Trp Gly Leu Leu Asp Ala Gly
785 790 795 800

Lys Ala Met Asn Gly Pro Ala Ser Phe Pro Phe Gly Asp Phe Thr Ala
805 810 815

Asp Thr Lys Gly Thr Ser Asp Ile Ala Tyr Ser Phe Arg Asn Asp Ile
820 825 830

Ser Gly Thr Gly Gly Leu Ile Lys Lys Gly Gly Ser Gln Leu Gln Leu
835 840 845

His Gly Asn Asn Thr Tyr Thr Gly Lys Thr Ile Ile Glu Gly Gly Ser
850 855 860

Leu Val Leu Tyr Gly Asn Asn Lys Ser Asp Met Arg Val Glu Thr Lys
865 870 875 880

Gly Ala Leu Ile Tyr Asn Gly Ala Ala Ser Gly Gly Ser Leu Asn Ser
885 890 895

Asp Gly Ile Val Tyr Leu Ala Asp Thr Asp Gln Ser Gly Ala Asn Glu
900 905 910

Thr Val His Ile Lys Gly Ser Leu Gln Leu Asp Gly Lys Gly Thr Leu
915 920 925

Tyr Thr Arg Leu Gly Lys Leu Leu Lys Val Asp Gly Thr Ala Ile Ile
930 935 940

Gly Gly Lys Leu Tyr Met Ser Ala Arg Gly Lys Gly Ala Gly Tyr Leu
945 950 955 960

Asn Ser Thr Gly Arg Arg Val Pro Phe Leu Ser Ala Ala Lys Ile Gly
965 970 975

Gln Asp Tyr Ser Phe Phe Thr Asn Ile Glu Thr Asp Gly Gly Leu Leu
980 985 990

Ala Ser Leu Asp Ser Val Glu Lys Thr Ala Gly Ser Glu Gly Asp Thr
995 1000 1005

Leu Ser Tyr Tyr Val Arg Arg Gly Asn Ala Ala Arg Thr Ala Ser Ala
1010 1015 1020

Ala Ala His Ser Ala Pro Ala Gly Leu Lys His Ala Val Glu Gln Gly
1025 1030 1035 1040

Gly Ser Asn Leu Glu Asn Leu Met Val Glu Leu Asp Ala Ser Glu Ser
1045 1050 1055

Ser Ala Thr Pro Glu Thr Val Glu Thr Ala Ala Ala Asp Arg Thr Asp
1060 1065 1070

Met Pro Gly Ile Arg Pro Tyr Gly Ala Thr Phe Arg Ala Ala Ala Ala
1075 1080 1085

Val Gln His Ala Asn Ala Ala Asp Gly Val Arg Ile Phe Asn Ser Leu
1090 1095 1100

Ala Ala Thr Val Tyr Ala Asp Ser Thr Ala Ala His Ala Asp Met Gln
1105 1110 1115 1120

Gly Arg Arg Leu Lys Ala Val Ser Asp Gly Leu Asp His Asn Gly Thr
1125 1130 1135

Gly Leu Arg Val Ile Ala Gln Thr Gln Gln Asp Gly Gly Thr Trp Glu
1140 1145 1150

Gln Gly Gly Val Glu Gly Lys Met Arg Gly Ser Thr Gln Thr Val Gly
1155 1160 1165

Ile Ala Ala Lys Thr Gly Glu Asn Thr Thr Ala Ala Ala Thr Leu Gly
1170 1175 1180

Met Gly Arg Ser Thr Trp Ser Glu Asn Ser Ala Asn Ala Lys Thr Asp
1185 1190 1195 1200

Ser Ile Ser Leu Phe Ala Gly Ile Arg His Asp Ala Gly Asp Ile Gly
1205 1210 1215

Tyr Leu Lys Gly Leu Phe Ser Tyr Gly Arg Tyr Lys Asn Ser Ile Ser
1220 1225 1230

Arg Ser Thr Gly Ala Asp Glu His Ala Glu Gly Ser Val Asn Gly Thr
1235 1240 1245

Leu Met Gln Leu Gly Ala Leu Gly Gly Val Asn Val Pro Phe Ala Ala
1250 1255 1260

Thr Gly Asp Leu Thr Val Glu Gly Gly Leu Arg Tyr Asp Leu Leu Lys
1265 1270 1275 1280

Gln Asp Ala Phe Ala Glu Lys Gly Ser Ala Leu Gly Trp Ser Gly Asn
1285 1290 1295

Ser Leu Thr Glu Gly Thr Leu Val Gly Leu Ala Gly Leu Lys Leu Ser
1300 1305 1310

Gln Pro Leu Ser Asp Lys Ala Val Leu Phe Ala Thr Ala Gly Val Glu
1315 1320 1325

Arg Asp Leu Asn Gly Arg Asp Tyr Thr Val Thr Gly Gly Phe Thr Gly
1330 1335 1340

Ala Thr Ala Ala Thr Gly Lys Thr Gly Ala Arg Asn Met Pro His Thr
1345 1350 1355 1360

Arg Leu Val Ala Gly Leu Gly Ala Asp Val Glu Phe Gly Asn Gly Trp
1365 1370 1375

Asn Gly Leu Ala Arg Tyr Ser Tyr Ala Gly Ser Lys Gln Tyr Gly Asn
1380 1385 1390

His Ser Gly Arg Val Gly Val Gly Tyr Arg Phe
1395 1400

<210> 59
<211> 25
<212> DNA
<213> Artificial Sequence

<220>
<223> Oligonucleotide

<400> 59
cgcggatccg gagggggtgg tgtcg 25

<210> 60
<211> 27
<212> DNA
<213> Artificial Sequence

<220>
<223> Oligonucleotide

<400> 60
cccgctcgag ttgcttggcg gcaaggc 27

<210> 61
<211> 25
<212> DNA
<213> Artificial Sequence

<220>
<223> Oligonucleotide

<400> 61
cgcggatccg gcggaggcgg cactt 25

<210> 62
<211> 26
<212> DNA
<213> Artificial Sequence

<220>
<223> Oligonucleotide

<400> 62
cccgctcgag gaaccggtag cctacg 26

<210> 63
<211> 41
<212> DNA
<213> Artificial Sequence

<220>
<223> Oligonucleotide

<400> 63
cgcggatccg gtggtggtgg ttcagatttg gcaaacgatt c 41

<210> 64
<211> 29
<212> DNA
<213> Artificial Sequence

<220>
<223> Oligonucleotide

<400> 64
cccgctcgag cgtatcatat ttcacgtgc 29

<210> 65
<211> 25
<212> DNA
<213> Artificial Sequence

<220>
<223> Oligonucleotide

<400> 65
cgcggatccg gagggggtgg tgtcg 25

<210> 66
<211> 28
<212> DNA

<213> Artificial Sequence

<220>
<223> Oligonucleotide

<400> 66
cccgctcgag ttattgcttg gcggcaag 28

<210> 67
<211> 25
<212> DNA
<213> Artificial Sequence

<220>
<223> Oligonucleotide

<400> 67

cgcggatccg gcggaggcgg cactt 25

<210> 68
<211> 28
<212> DNA
<213> Artificial Sequence

<220>
<223> Oligonucleotide

<400> 68
cccgctcgag tcagaaccgg tagcctac 28

<210> 69
<211> 41
<212> DNA
<213> Artificial Sequence

<220>
<223> Oligonucleotide

<400> 69
cgcggatccg gtggtggtgg ttcagatttg gcaaacgatt c 41

<210> 70
<211> 32
<212> DNA
<213> Artificial Sequence

<220>
<223> Oligonucleotide

<400> 70
cccgctcgag ttacgtatca tatttcacgt gc 32

<210> 71
<211> 42
<212> DNA
<213> Artificial Sequence

<220>
<223> Oligonucleotide

<400> 71
cgcggatccg gtggtggtgg tcaaagcaag agcatccaaa cc 42

<210> 72
<211> 30
<212> DNA
<213> Artificial Sequence

<220>
<223> Oligonucleotide

<400> 72
cccaagcttt tcgggcggta ttcgggcttc 30

<210> 73
<211> 39
<212> DNA
<213> Artificial Sequence

<220>
<223> Oligonucleotide

<400> 73
cgcggatccg gtggtggtgg tgccacctac aaagtggac 39

<210> 74
<211> 28
<212> DNA
<213> Artificial Sequence

<220>
<223> Oligonucleotide

<400> 74
gcccaagctt ttgtttggct gcctcgat 28

<210> 75
<211> 34
<212> DNA
<213> Artificial Sequence

<220>
<223> Oligonucleotide

<400> 75
cgcggatccg gtggtggtgg tacaagcgac gacg 34

<210> 76
<211> 28
<212> DNA
<213> Artificial Sequence

<220>
<223> Oligonucleotide

<400> 76
gcccaagctt ccactcgtaa ttgacgcc 28

<210> 77
<211> 41
<212> DNA
<213> Artificial Sequence

<220>
<223> Oligonucleotide

<400> 77
cgcggatccg gtggtggtgg ttcagatttg gcaaacgatt c 41

<210> 78
<211> 28
<212> DNA
<213> Artificial Sequence

<220>
<223> Oligonucleotide

<400> 78
cccaagcttc gtatcatatt tcacgtgc 28

<210> 79
<211> 44
<212> DNA
<213> Artificial Sequence

<220>
<223> Oligonucleotide

<400> 79
cccaagcttg gtggtggtgg tggttcagat ttggcaaacg attc 44

<210> 80
<211> 29
<212> DNA
<213> Artificial Sequence

<220>
<223> Oligonucleotide

<400> 80
cccgctcgag cgtatcatat ttcacgtgc 29

<210> 81
<211> 45
<212> DNA
<213> Artificial Sequence

<220>
<223> Oligonucleotide

<400> 81
cccaagcttg gtggtggtgg tggtcaaagc aagagcatcc aaacc 45

<210> 82
<211> 28
<212> DNA
<213> Artificial Sequence

<220>
<223> Oligonucleotide

<400> 82
cccgctcgag cgggcggtat tcgggctt 28

<210> 83
<211> 32
<212> DNA
<213> Artificial Sequence

<220>
<223> Oligonucleotide

<400> 83
cgcggatccg ctagccccga tgttaaatcg gc 32

<210> 84
<211> 29
<212> DNA
<213> Artificial Sequence

<220>
<223> Oligonucleotide

<400> 84
cggggatcca tcctgctctt ttttgccgg 29

<210> 85
<211> 36
<212> DNA
<213> Artificial Sequence

<220>
<223> Oligonucleotide

<400> 85
cgcggatccg ctagcggaca cacttatttc ggcatc 36

<210> 86
<211> 30
<212> DNA
<213> Artificial Sequence

<220>
<223> Oligonucleotide

<400> 86
cgcggatccc cagcggtagc ctaatttgat 30

<210> 87
<211> 41
<212> DNA

<213> Artificial Sequence

<220>
<223> Oligonucleotide

<400> 87
cgcggatccg gtggtggtgg ttcagatttg gcaaacgatt c 41

<210> 88
<211> 28
<212> DNA
<213> Artificial Sequence

<220>
<223> Oligonucleotide

<400> 88
cccaagcttc gtatcatatt tcacgtgc 28

<210> 89
<211> 36
<212> DNA
<213> Artificial Sequence

<220>
<223> Oligonucleotide

<400> 89
gcggcgtcga cggtggcgga ggcactggat cctcag 36

<210> 90
<211> 35
<212> DNA
<213> Artificial Sequence

<220>
<223> Oligonucleotide

<400> 90
ggaggcactg gatcctcaga tttggcaaac gattc 35

<210> 91
<211> 29
<212> DNA
<213> Artificial Sequence

<220>
<223> Oligonucleotide

<400> 91
cccgctcgag cgtatcatat ttcacgtgc 29

<210> 92
<211> 25
<212> DNA
<213> Artificial Sequence

<220>

<223> Oligonucleotide

<400> 92
cggggatccg ggggcggcgg tggcg 25

<210> 93
<211> 30
<212> DNA
<213> Artificial Sequence

<220>
<223> Oligonucleotide

<400> 93
cccaagctta tcctgctctt ttttgccggc 30

<210> 94
<211> 42
<212> DNA
<213> Artificial Sequence

<220>
<223> Oligonucleotide

<400> 94
cgcggatccg gtggtggtgg tcaaagcaag agcatccaaa cc 42

<210> 95
<211> 28
<212> DNA
<213> Artificial Sequence

<220>
<223> Oligonucleotide

<400> 95
cccaagcttc gggcggtatt cgggcttc 28

<210> 96
<211> 26
<212> DNA
<213> Artificial Sequence

<220>
<223> Oligonucleotide

<400> 96
ccccaagctt gggggcggcg gtggcg 26

<210> 97
<211> 31
<212> DNA
<213> Artificial Sequence

<220>
<223> Oligonucleotide

<400> 97

cccgctcgag atcctgctct tttttgccgg c 31

<210> 98
<211> 45
<212> DNA
<213> Artificial Sequence

<220>
<223> Oligonucleotide

<400> 98
cccaagcttg gtggtggtgg tggtcaaagc aagagcatcc aaacc 45

<210> 99
<211> 28
<212> DNA
<213> Artificial Sequence

<220>
<223> Oligonucleotide

<400> 99
cccgctcgag cgggcggtat tcgggctt 28

<210> 100
<211> 35
<212> DNA
<213> Artificial Sequence

<220>
<223> Oligonucleotide

<400> 100
ggaggcactg gatccgcagc cacaaacgac gacga 35

<210> 101
<211> 36
<212> DNA
<213> Artificial Sequence

<220>
<223> Oligonucleotide

<400> 101
gcggcctcga gggtggcgga ggcactggat ccgcag 36

<210> 102
<211> 28
<212> DNA
<213> Artificial Sequence

<220>
<223> Oligonucleotide

<400> 102
cccgctcgag acccaqcttg taaggttg 28

<210> 103

<211> 35
<212> DNA
<213> Artificial Sequence

<220>
<223> Oligonucleotide <400> 103

ggaggcactg gatccgcagc cacaaacgac gacga 35

<210> 104
<211> 36
<212> DNA
<213> Artificial Sequence

<220>
<223> Oligonucleotide

<400> 104
gcggcctcga gggtggcgga ggcactggat ccgcag 36

<210> 105
<211> 28
<212> DNA
<213> Artificial Sequence

<220>
<223> Oligonucleotide

<400> 105
cccgctcgag ccactcgtaa ttgacgcc 28

<210> 106
<211> 38
<212> DNA
<213> Artificial Sequence

<220>
<223> Oligonucleotide

<400> 106
gcggcctcga gggatccggc ggaggcggca cttctgcg 38

<210> 107
<211> 26
<212> DNA
<213> Artificial Sequence

<220>
<223> Oligonucleotide

<400> 107
cccgctcgag gaaccggtag cctacg 26

<210> 108
<211> 35
<212> DNA
<213> Artificial Sequence

<220>
<223> Oligonucleotide

<400> 108
ggaggcactg gatcctcaga tttggcaaac gattc 35

<210> 109
<211> 37
<212> DNA
<213> Artificial Sequence

<220>
<223> Oligonucleotide

<400> 109
gcggcgtcga cggtggcgga ggcactggat cctcaga 37

<210> 110
<211> 29
<212> DNA
<213> Artificial Sequence

<220>
<223> Oligonucleotide

<400> 110
cccgctcgag cgtatcatat ttcacgtgc 29

<210> 111
<211> 35
<212> DNA
<213> Artificial Sequence

<220>
<223> Oligonucleotide

<400> 111
gcggcctcga gggatccgga gggggtggtg tcgcc 35

<210> 112
<211> 25
<212> DNA
<213> Artificial Sequence

<220>
<223> Oligonucleotide

<400> 112
cccgctcgag ttgcttggcg gcaag 25

<210> 113
<211> 35
<212> DNA
<213> Artificial Sequence

<220>
<223> Oligonucleotide

<400> 113
ggaggcactg gatccgcagc cacaaacgac gacga 35

<210> 114
<211> 36
<212> DNA
<213> Artificial Sequence

<220>
<223> Oligonucleotide

<400> 114
gcggcctcga gggtggcgga ggcactggat ccgcag 36

<210> 115
<211> 28
<212> DNA
<213> Artificial Sequence

<220>
<223> Oligonucleotide

<400> 115
cccgctcgag acccagcttg taaggttg 28

<210> 116
<211> 35
<212> DNA
<213> Artificial Sequence

<220>
<223> Oligonucleotide

<400> 116
ggaggcactg gatccgcagc cacaaacgac gacga 35

<210> 117
<211> 36
<212> DNA
<213> Artificial Sequence

<220>
<223> Oligonucleotide

<400> 117
gcggcctcga gggtggcgga ggcactggat ccgcag 36

<210> 118
<211> 28
<212> DNA
<213> Artificial Sequence

<220>
<223> Oligonucleotide

<400> 118
cccgctcgag ccactcgtaa ttgacgcc 28

<210> 119
<211> 35
<212> DNA
<213> Artificial Sequence

<220>
<223> Oligonucleotide

<400> 119
ggaggcactg gatcctcaga tttggcaaac gattc 35

<210> 120
<211> 37
<212> DNA
<213> Artificial Sequence

<220>
<223> Oligonucleotide

<400> 120
gcggcgtcga cggtggcgga ggcactggat cctcaga 37

<210> 121
<211> 29
<212> DNA
<213> Artificial Sequence

<220>
<223> Oligonucleotide

<400> 121
cccgctcgag cgtatcatat ttcacgtgc 29

SEQUENCE LISTING

<110> Novartis Vaccines and Diagnostics SRL

<120> Hybrid Expression of Neisserial Proteins

<130> P048826EP

<150> EP-01914098.7
<151> 2001-02-28

<150> PCT/IB01/00420
<151> 2001-02-28

<150> GB 0004695.3
<151> 2000-02-28

<150> GB 0027675.8
<151> 2000-11-13

<160> 121

<170> SeqWin99, version 1.02

<210> 1
<211> 608
<212> PRT
<213> Neisseria meningitidis

<400> 1

```
Leu Gly Ile Ser Arg Lys Ile Ser Leu Ile Leu Ser Ile Leu Ala Val
1               5                   10                  15

Cys Leu Pro Met His Ala His Ala Ser Asp Leu Ala Asn Asp Ser Phe
            20                  25                  30

Ile Arg Gln Val Leu Asp Arg Gln His Phe Glu Pro Asp Gly Lys Tyr
        35                  40                  45

His Leu Phe Gly Ser Arg Gly Glu Leu Ala Glu Arg Ser Gly His Ile
    50                  55                  60

Gly Leu Gly Lys Ile Gln Ser His Gln Leu Gly Asn Leu Met Ile Gln
65                  70                  75                  80

Gln Ala Ala Ile Lys Gly Asn Ile Gly Tyr Ile Val Arg Phe Ser Asp
                85                  90                  95

His Gly His Glu Val His Ser Pro Phe Asp Asn His Ala Ser His Ser
            100                 105                 110

Asp Ser Asp Glu Ala Gly Ser Pro Val Asp Gly Phe Ser Leu Tyr Arg
        115                 120                 125

Ile His Trp Asp Gly Tyr Glu His His Pro Ala Asp Gly Tyr Asp Gly
    130                 135                 140

Pro Gln Gly Gly Gly Tyr Pro Ala Pro Lys Gly Ala Arg Asp Ile Tyr
145                 150                 155                 160

Ser Tyr Asp Ile Lys Gly Val Ala Gln Asn Ile Arg Leu Asn Leu Thr
```

165 170 175

Asp Asn Arg Ser Thr Gly Gln Arg Leu Ala Asp Arg Phe His Asn Ala
180 185 190

Gly Ser Met Leu Thr Gln Gly Val Gly Asp Gly Phe Lys Arg Ala Thr
195 200 205

Arg Tyr Ser Pro Glu Leu Asp Arg Ser Gly Asn Ala Ala Glu Ala Phe
210 215 220

Asn Gly Thr Ala Asp Ile Val Lys Asn Ile Ile Gly Ala Ala Gly Glu
225 230 235 240

Ile Val Gly Ala Gly Asp Ala Val Gln Gly Ile Ser Glu Gly Ser Asn
245 250 255

Ile Ala Val Met His Gly Leu Gly Leu Leu Ser Thr Glu Asn Lys Met
260 265 270

Ala Arg Ile Asn Asp Leu Ala Asp Met Ala Gln Leu Lys Asp Tyr Ala
275 280 285

Ala Ala Ala Ile Arg Asp Trp Ala Val Gln Asn Pro Asn Ala Ala Gln
290 295 300

Gly Ile Glu Ala Val Ser Asn Ile Phe Met Ala Ala Ile Pro Ile Lys
305 310 315 320

Gly Ile Gly Ala Val Arg Gly Lys Tyr Gly Leu Gly Gly Ile Thr Ala
325 330 335

His Pro Ile Lys Arg Ser Gln Met Gly Ala Ile Ala Leu Pro Lys Gly
340 345 350

Lys Ser Ala Val Ser Asp Asn Phe Ala Asp Ala Ala Tyr Ala Lys Tyr
355 360 365

Pro Ser Pro Tyr His Ser Arg Asn Ile Arg Ser Asn Leu Glu Gln Arg
370 375 380

Tyr Gly Lys Glu Asn Ile Thr Ser Ser Thr Val Pro Pro Ser Asn Gly
385 390 395 400

Lys Asn Val Lys Leu Ala Asp Gln Arg His Pro Lys Thr Gly Val Pro
405 410 415

Phe Asp Gly Lys Gly Phe Pro Asn Phe Glu Lys His Val Lys Tyr Asp
420 425 430

Thr Lys Leu Asp Ile Gln Glu Leu Ser Gly Gly Gly Ile Pro Lys Ala
435 440 445

Lys Pro Val Ser Asp Ala Lys Pro Arg Trp Glu Val Asp Arg Lys Leu
450 455 460

Asn Lys Leu Thr Thr Arg Glu Gln Val Glu Lys Asn Val Gln Glu Ile
465 470 475 480

Arg Asn Gly Asn Lys Asn Ser Asn Phe Ser Gln His Ala Gln Leu Glu
485 490 495

```
Arg Glu Ile Asn Lys Leu Lys Ser Ala Asp Glu Ile Asn Phe Ala Asp
            500                 505                 510

Gly Met Gly Lys Phe Thr Asp Ser Met Asn Asp Lys Ala Phe Ser Arg
        515                 520                 525

Leu Val Lys Ser Val Lys Glu Asn Gly Phe Thr Asn Pro Val Val Glu
    530                 535                 540

Tyr Val Glu Ile Asn Gly Lys Ala Tyr Ile Val Arg Gly Asn Asn Arg
545                 550                 555                 560

Val Phe Ala Ala Glu Tyr Leu Gly Arg Ile His Glu Leu Lys Phe Lys
                565                 570                 575

Lys Val Asp Phe Pro Val Pro Asn Thr Ser Trp Lys Asn Pro Thr Asp
            580                 585                 590

Val Leu Asn Glu Ser Gly Asn Val Lys Arg Pro Arg Tyr Arg Ser Lys
        595                 600                 605
```

<210> 2
<211> 464
<212> PRT
<213> Artificial Sequence

<220>
<223> deltaG287

<400> 2

```
Ser Pro Asp Val Lys Ser Ala Asp Thr Leu Ser Lys Pro Ala Ala Pro
1               5                   10                  15

Val Val Ser Glu Lys Glu Thr Glu Ala Lys Glu Asp Ala Pro Gln Ala
            20                  25                  30

Gly Ser Gln Gly Gln Gly Ala Pro Ser Ala Gln Gly Ser Gln Asp Met
        35                  40                  45

Ala Ala Val Ser Glu Glu Asn Thr Gly Asn Gly Gly Ala Val Thr Ala
    50                  55                  60

Asp Asn Pro Lys Asn Glu Asp Glu Val Ala Gln Asn Asp Met Pro Gln
65                  70                  75                  80

Asn Ala Ala Gly Thr Asp Ser Ser Thr Pro Asn His Thr Pro Asp Pro
                85                  90                  95

Asn Met Leu Ala Gly Asn Met Glu Asn Gln Ala Thr Asp Ala Gly Glu
            100                 105                 110

Ser Ser Gln Pro Ala Asn Gln Pro Asp Met Ala Asn Ala Ala Asp Gly
        115                 120                 125

Met Gln Gly Asp Asp Pro Ser Ala Gly Gly Gln Asn Ala Gly Asn Thr
    130                 135                 140

Ala Ala Gln Gly Ala Asn Gln Ala Gly Asn Asn Gln Ala Ala Gly Ser
145                 150                 155                 160
```

Ser Asp Pro Ile Pro Ala Ser Asn Pro Ala Pro Ala Asn Gly Gly Ser
165 170 175

Asn Phe Gly Arg Val Asp Leu Ala Asn Gly Val Leu Ile Asp Gly Pro
180 185 190

Ser Gln Asn Ile Thr Leu Thr His Cys Lys Gly Asp Ser Cys Ser Gly
195 200 205

Asn Asn Phe Leu Asp Glu Glu Val Gln Leu Lys Ser Glu Phe Glu Lys
210 215 220

Leu Ser Asp Ala Asp Lys Ile Ser Asn Tyr Lys Lys Asp Gly Lys Asn
225 230 235 240

Asp Lys Phe Val Gly Leu Val Ala Asp Ser Val Gln Met Lys Gly Ile
245 250 255

Asn Gln Tyr Ile Ile Phe Tyr Lys Pro Lys Pro Thr Ser Phe Ala Arg
260 265 270

Phe Arg Arg Ser Ala Arg Ser Arg Arg Ser Leu Pro Ala Glu Met Pro
275 280 285

Leu Ile Pro Val Asn Gln Ala Asp Thr Leu Ile Val Asp Gly Glu Ala
290 295 300

Val Ser Leu Thr Gly His Ser Gly Asn Ile Phe Ala Pro Glu Gly Asn
305 310 315 320

Tyr Arg Tyr Leu Thr Tyr Gly Ala Glu Lys Leu Pro Gly Gly Ser Tyr
325 330 335

Ala Leu Arg Val Gln Gly Glu Pro Ala Lys Gly Glu Met Leu Ala Gly
340 345 350

Ala Ala Val Tyr Asn Gly Glu Val Leu His Phe His Thr Glu Asn Gly
355 360 365

Arg Pro Tyr Pro Thr Arg Gly Arg Phe Ala Ala Lys Val Asp Phe Gly
370 375 380

Ser Lys Ser Val Asp Gly Ile Ile Asp Ser Gly Asp Asp Leu His Met
385 390 395 400

Gly Thr Gln Lys Phe Lys Ala Ala Ile Asp Gly Asn Gly Phe Lys Gly
405 410 415

Thr Trp Thr Glu Asn Gly Ser Gly Asp Val Ser Gly Lys Phe Tyr Gly
420 425 430

Pro Ala Gly Glu Glu Val Ala Gly Lys Tyr Ser Tyr Arg Pro Thr Asp
435 440 445

Ala Glu Lys Gly Gly Phe Gly Val Phe Ala Gly Lys Lys Glu Gln Asp
450 455 460

<210> 3
<211> 2505
<212> DNA
<213> Artificial Sequence

<220>
<223> deltaG287-919

<400> 3

```
atggctagcc ccgatgttaa atcggcggac acgctgtcaa.aaccggccgc tcctgttgtt   60
gctgaaaaag agacagaggt aaaagaagat gcgccacagg caggttctca aggacagggc   120
gcgccatcca cacaaggcag ccaagatatg gcggcagttt cggcagaaaa tacaggcaat   180
ggcggtgcgg caacaacgga caaacccaaa aatgaagacg agggaccgca aaatgatatg   240
ccgcaaaatt ccgccgaatc cgcaaatcaa acagggaaca accaacccgc cgattcttca   300
gattccgccc ccgcgtcaaa ccctgcacct gcgaatggcg gtagcaattt tggaagggtt   360
gatttggcta atggcgtttt gattgatggg ccgtcgcaaa atataacgtt gacccactgt   420
aaaggcgatt cttgtaatgg tgataattta ttggatgaag aagcaccgtc aaaatcagaa   480
tttgaaaatt taaatgagtc tgaacgaatt gagaaatata agaaagatgg gaaaagcgat   540
aaatttacta atttggttgc gacagcagtt caagctaatg gaactaacaa atatgtcatc   600
atttataaag acaagtccgc ttcatcttca tctgcgcgat tcaggcgttc tgcacggtcg   660
aggaggtcgc ttcctgccga gatgccgcta atccccgtca atcaggcgga tacgctgatt   720
gtcgatgggg aagcggtcag cctgacgggg cattccggca atatcttcgc gcccgaaggg   780
aattaccggt atctgactta cggggcggaa aaattgcccg gcggatcgta tgccctccgt   840
gtgcaaggcg aaccggcaaa aggcgaaatg cttgctggca cggccgtgta caacggcgaa   900
gtgctgcatt ttcatacgga aaacggccgt ccgtacccga ctagaggcag gtttgccgca   960
aaagtcgatt tcggcagcaa atctgtggac ggcattatcg acagcggcga tgatttgcat   1020
atgggtacgc aaaaattcaa agccgccatc gatggaaacg gctttaaggg gacttggacg   1080
gaaaatggcg gcggggatgt ttccggaagg ttttacggcc cggccggcga ggaagtggcg   1140
ggaaaataca gctatcgccc gacagatgcg gaaaagggcg gattcggcgt gtttgccggc   1200
aaaaaagagc aggatggatc cggaggagga ggatgccaaa gcaagagcat ccaaaccttt   1260
ccgcaacccg acacatccgt catcaacggc ccggaccggc cggtcggcat ccccgacccc   1320
gccggaacga cggtcggcgg cggcggggcc gtctataccg ttgtaccgca cctgtccctg   1380
ccccactggg cggcgcagga tttcgccaaa agcctgcaat ccttccgcct cggctgcgcc   1440
aatttgaaaa accgccaagg ctggcaggat gtgtgcgccc aagcctttca aaccccccgtc   1500
cattcctttc aggcaaaaca gtttttttgaa cgctatttca cgccgtggca ggttgcaggc   1560
aacggaagcc ttgccggtac ggttaccggc tattacgagc cggtgctgaa gggcgacgac   1620
aggcggacgg cacaagcccg cttcccgatt tacggtattc ccgacgattt tatctccgtc   1680
cccctgcctg ccggtttgcg gagcggaaaa gcccttgtcc gcatcaggca gacgggaaaa   1740
aacagcggca caatcgacaa taccggcggc acacataccg ccgacctctc ccgattcccc   1800
atcaccgcgc gcacaacggc aatcaaaggc aggtttgaag gaagccgctt cctcccctac   1860
cacacgcgca accaaatcaa cggcggcgcg cttgacggca aagccccgat actcggttac   1920
gccgaagacc ccgtcgaact ttttttttatg cacatccaag gctcgggccg tctgaaaacc   1980
ccgtccggca aatacatccg catcggctat gccgacaaaa acgaacatcc ctacgtttcc   2040
atcggacgct atatggcgga caaaggctac ctcaagctcg ggcagacctc gatgcagggc   2100
atcaaagcct atatgcggca aaatccgcaa cgcctcgccg aagttttggg tcaaaacccc   2160
agctatatct tttccgcga gcttgccgga agcagcaatg acggtcccgt cggcgcactg   2220
ggcacgccgt tgatggggga atatgccggc gcagtcgacc ggcactacat taccttgggc   2280
gcgcccttat ttgtcgccac cgcccatccg gttacccgca aagccctcaa ccgcctgatt   2340
atggcgcagg ataccggcag cgcgattaaa ggcgcggtgc gcgtggatta tttttgggga   2400
tacggcgacg aagccggcga acttgccggc aaacagaaaa ccacgggtta cgtctggcag   2460
ctcctaccca acggtatgaa gcccgaatac cgcccgtaac tcgag   2505
```

<210> 4
<211> 832
<212> PRT
<213> Artificial Sequence

<220>
<223> deltaG287-919

<400> 4

```
Met Ala Ser Pro Asp Val Lys Ser Ala Asp Thr Leu Ser Lys Pro Ala
1               5                   10                  15
```

```
Ala Pro Val Val Ala Glu Lys Glu Thr Glu Val Lys Glu Asp Ala Pro
            20                  25                  30

Gln Ala Gly Ser Gln Gly Gln Gly Ala Pro Ser Thr Gln Gly Ser Gln
        35                  40                  45

Asp Met Ala Ala Val Ser Ala Glu Asn Thr Gly Asn Gly Gly Ala Ala
    50                  55                  60

Thr Thr Asp Lys Pro Lys Asn Glu Asp Glu Gly Pro Gln Asn Asp Met
65                  70                  75                  80

Pro Gln Asn Ser Ala Glu Ser Ala Asn Gln Thr Gly Asn Asn Gln Pro
                85                  90                  95

Ala Asp Ser Ser Asp Ser Ala Pro Ala Ser Asn Pro Ala Pro Ala Asn
            100                 105                 110

Gly Gly Ser Asn Phe Gly Arg Val Asp Leu Ala Asn Gly Val Leu Ile
        115                 120                 125

Asp Gly Pro Ser Gln Asn Ile Thr Leu Thr His Cys Lys Gly Asp Ser
    130                 135                 140

Cys Asn Gly Asp Asn Leu Leu Asp Glu Glu Ala Pro Ser Lys Ser Glu
145                 150                 155                 160

Phe Glu Asn Leu Asn Glu Ser Glu Arg Ile Glu Lys Tyr Lys Lys Asp
                165                 170                 175

Gly Lys Ser Asp Lys Phe Thr Asn Leu Val Ala Thr Ala Val Gln Ala
            180                 185                 190

Asn Gly Thr Asn Lys Tyr Val Ile Ile Tyr Lys Asp Lys Ser Ala Ser
        195                 200                 205

Ser Ser Ser Ala Arg Phe Arg Arg Ser Ala Arg Ser Arg Arg Ser Leu
    210                 215                 220

Pro Ala Glu Met Pro Leu Ile Pro Val Asn Gln Ala Asp Thr Leu Ile
225                 230                 235                 240

Val Asp Gly Glu Ala Val Ser Leu Thr Gly His Ser Gly Asn Ile Phe
                245                 250                 255

Ala Pro Glu Gly Asn Tyr Arg Tyr Leu Thr Tyr Gly Ala Glu Lys Leu
            260                 265                 270

Pro Gly Gly Ser Tyr Ala Leu Arg Val Gln Gly Glu Pro Ala Lys Gly
        275                 280                 285

Glu Met Leu Ala Gly Thr Ala Val Tyr Asn Gly Glu Val Leu His Phe
    290                 295                 300

His Thr Glu Asn Gly Arg Pro Tyr Pro Thr Arg Gly Arg Phe Ala Ala
305                 310                 315                 320

Lys Val Asp Phe Gly Ser Lys Ser Val Asp Gly Ile Ile Asp Ser Gly
                325                 330                 335
```

Asp Asp Leu His Met Gly Thr Gln Lys Phe Lys Ala Ala Ile Asp Gly
340 345 350

Asn Gly Phe Lys Gly Thr Trp Thr Glu Asn Gly Gly Gly Asp Val Ser
355 360 365

Gly Arg Phe Tyr Gly Pro Ala Gly Glu Glu Val Ala Gly Lys Tyr Ser
370 375 380

Tyr Arg Pro Thr Asp Ala Glu Lys Gly Gly Phe Gly Val Phe Ala Gly
385 390 395 400

Lys Lys Glu Gln Asp Gly Ser Gly Gly Gly Gly Cys Gln Ser Lys Ser
405 410 415

Ile Gln Thr Phe Pro Gln Pro Asp Thr Ser Val Ile Asn Gly Pro Asp
420 425 430

Arg Pro Val Gly Ile Pro Asp Pro Ala Gly Thr Thr Val Gly Gly Gly
435 440 445

Gly Ala Val Tyr Thr Val Val Pro His Leu Ser Leu Pro His Trp Ala
450 455 460

Ala Gln Asp Phe Ala Lys Ser Leu Gln Ser Phe Arg Leu Gly Cys Ala
465 470 475 480

Asn Leu Lys Asn Arg Gln Gly Trp Gln Asp Val Cys Ala Gln Ala Phe
485 490 495

Gln Thr Pro Val His Ser Phe Gln Ala Lys Gln Phe Phe Glu Arg Tyr
500 505 510

Phe Thr Pro Trp Gln Val Ala Gly Asn Gly Ser Leu Ala Gly Thr Val
515 520 525

Thr Gly Tyr Tyr Glu Pro Val Leu Lys Gly Asp Asp Arg Arg Thr Ala
530 535 540

Gln Ala Arg Phe Pro Ile Tyr Gly Ile Pro Asp Asp Phe Ile Ser Val
545 550 555 560

Pro Leu Pro Ala Gly Leu Arg Ser Gly Lys Ala Leu Val Arg Ile Arg
565 570 575

Gln Thr Gly Lys Asn Ser Gly Thr Ile Asp Asn Thr Gly Gly Thr His
580 585 590

Thr Ala Asp Leu Ser Arg Phe Pro Ile Thr Ala Arg Thr Thr Ala Ile
595 600 605

Lys Gly Arg Phe Glu Gly Ser Arg Phe Leu Pro Tyr His Thr Arg Asn
610 615 620

Gln Ile Asn Gly Gly Ala Leu Asp Gly Lys Ala Pro Ile Leu Gly Tyr
625 630 635 640

Ala Glu Asp Pro Val Glu Leu Phe Phe Met His Ile Gln Gly Ser Gly
645 650 655

Arg Leu Lys Thr Pro Ser Gly Lys Tyr Ile Arg Ile Gly Tyr Ala Asp

```
            660                 665                 670

Lys Asn Glu His Pro Tyr Val Ser Ile Gly Arg Tyr Met Ala Asp Lys
        675                 680                 685

Gly Tyr Leu Lys Leu Gly Gln Thr Ser Met Gln Gly Ile Lys Ala Tyr
    690                 695                 700

Met Arg Gln Asn Pro Gln Arg Leu Ala Glu Val Leu Gly Gln Asn Pro
705                 710                 715                 720

Ser Tyr Ile Phe Phe Arg Glu Leu Ala Gly Ser Ser Asn Asp Gly Pro
                725                 730                 735

Val Gly Ala Leu Gly Thr Pro Leu Met Gly Glu Tyr Ala Gly Ala Val
            740                 745                 750

Asp Arg His Tyr Ile Thr Leu Gly Ala Pro Leu Phe Val Ala Thr Ala
        755                 760                 765

His Pro Val Thr Arg Lys Ala Leu Asn Arg Leu Ile Met Ala Gln Asp
    770                 775                 780

Thr Gly Ser Ala Ile Lys Gly Ala Val Arg Val Asp Tyr Phe Trp Gly
785                 790                 795                 800

Tyr Gly Asp Glu Ala Gly Glu Leu Ala Gly Lys Gln Lys Thr Thr Gly
                805                 810                 815

Tyr Val Trp Gln Leu Leu Pro Asn Gly Met Lys Pro Glu Tyr Arg Pro
            820                 825                 830
```

<210> 5
<211> 1746
<212> DNA
<213> Artificial Sequence

<220>
<223> deltaG287-953

<400> 5

```
atggctagcc ccgatgttaa atcggcggac acgctgtcaa aaccggccgc tcctgttgtt   60
gctgaaaaag agacagaggt aaaagaagat gcgccacagg caggttctca aggacagggc  120
gcgccatcca cacaaggcag ccaagatatg gcggcagttt cggcagaaaa tacaggcaat  180
ggcggtgcgg caacaacgga caaacccaaa aatgaagacg agggaccgca aaatgatatg  240
ccgcaaaatt ccgccgaatc cgcaaatcaa acagggaaca accaacccgc cgattcttca  300
gattccgccc ccgcgtcaaa ccctgcacct gcgaatggcg gtagcaattt tggaagggtt  360
gatttggcta atggcgtttt gattgatggg ccgtcgcaaa atataacgtt gacccactgt  420
aaaggcgatt cttgtaatgg tgataattta ttggatgaag aagcaccgtc aaaatcagaa  480
tttgaaaatt taaatgagtc tgaacgaatt gagaaatata agaaagatgg gaaaagcgat  540
aaatttacta atttggttgc gacagcagtt caagctaatg gaactaacaa atatgtcatc  600
atttataaag acaagtccgc ttcatcttca tctgcgcgat tcaggcgttc tgcacggtcg  660
aggaggtcgc ttcctgccga gatgccgcta atccccgtca atcaggcgga tacgctgatt  720
gtcgatgggg aagcggtcag cctgacgggg cattccggca atatcttcgc gcccgaaggg  780
aattaccggt atctgactta cggggcggaa aaattgcccg gcggatcgta tgccctccgt  840
gtgcaaggcg aaccggcaaa aggcgaaatg cttgctggca cggccgtgta caacggcgaa  900
gtgctgcatt ttcatacgga aaacggccgt ccgtacccga ctagaggcag gtttgccgca  960
aaagtcgatt tcggcagcaa atctgtggac ggcattatcg acagcggcga tgatttgcat 1020
atgggtacgc aaaaattcaa agccgccatc gatggaaacg gctttaaggg gacttggacg 1080
gaaaatggcg gcggggatgt ttccggaagg ttttacggcc cggccggcga ggaagtggcg 1140
ggaaaataca gctatcgccc gacagatgcg gaaaagggcg gattcggcgt gtttgccggc 1200
aaaaaagagc aggatggatc cggaggagga ggagccacct acaaagtgga cgaatatcac 1260
gccaacgccc gtttcgccat cgaccatttc aacaccagca ccaacgtcgg cggtttttac 1320
ggtctgaccg gttccgtcga gttcgaccaa gcaaaacgcg acggtaaaat cgacatcacc 1380
atccccgttg ccaacctgca aagcggttcg caacacttta ccgaccacct gaaatcagcc 1440
gacatcttcg atgccgccca atatccggac atccgctttg tttccaccaa attcaacttc 1500
aacggcaaaa aactggtttc cgttgacggc aacctgacca tgcacggcaa aaccgccccc 1560
gtcaaactca aagccgaaaa attcaactgc taccaaagcc cgatggcgaa aaccgaagtt 1620
tgcggcggcg acttcagcac caccatcgac cgcaccaaat ggggcgtgga ctacctcgtt 1680
aacgttggta tgaccaaaag cgtccgcatc gacatccaaa tcgaggcagc caaacaataa 1740
ctcgag                                                            1746
```

<210> 6
<211> 579
<212> PRT
<213> Artificial Sequence

<220>
<223> deltaG287-953

<400> 6

```
Met Ala Ser Pro Asp Val Lys Ser Ala Asp Thr Leu Ser Lys Pro Ala
1               5                   10                  15

Ala Pro Val Val Ala Glu Lys Glu Thr Glu Val Lys Glu Asp Ala Pro
            20                  25                  30

Gln Ala Gly Ser Gln Gly Gln Gly Ala Pro Ser Thr Gln Gly Ser Gln
        35                  40                  45

Asp Met Ala Ala Val Ser Ala Glu Asn Thr Gly Asn Gly Gly Ala Ala
    50                  55                  60

Thr Thr Asp Lys Pro Lys Asn Glu Asp Glu Gly Pro Gln Asn Asp Met
65                  70                  75                  80

Pro Gln Asn Ser Ala Glu Ser Ala Asn Gln Thr Gly Asn Asn Gln Pro
                85                  90                  95

Ala Asp Ser Ser Asp Ser Ala Pro Ala Ser Asn Pro Ala Pro Ala Asn
            100                 105                 110

Gly Gly Ser Asn Phe Gly Arg Val Asp Leu Ala Asn Gly Val Leu Ile
        115                 120                 125

Asp Gly Pro Ser Gln Asn Ile Thr Leu Thr His Cys Lys Gly Asp Ser
    130                 135                 140

Cys Asn Gly Asp Asn Leu Leu Asp Glu Glu Ala Pro Ser Lys Ser Glu
145                 150                 155                 160

Phe Glu Asn Leu Asn Glu Ser Glu Arg Ile Glu Lys Tyr Lys Lys Asp
                165                 170                 175

Gly Lys Ser Asp Lys Phe Thr Asn Leu Val Ala Thr Ala Val Gln Ala
            180                 185                 190

Asn Gly Thr Asn Lys Tyr Val Ile Ile Tyr Lys Asp Lys Ser Ala Ser
        195                 200                 205

Ser Ser Ser Ala Arg Phe Arg Arg Ser Ala Arg Ser Arg Arg Ser Leu
```

```
        210                 215                 220

Pro Ala Glu Met Pro Leu Ile Pro Val Asn Gln Ala Asp Thr Leu Ile
225                 230                 235                 240

Val Asp Gly Glu Ala Val Ser Leu Thr Gly His Ser Gly Asn Ile Phe
                245                 250                 255

Ala Pro Glu Gly Asn Tyr Arg Tyr Leu Thr Tyr Gly Ala Glu Lys Leu
            260                 265                 270

Pro Gly Gly Ser Tyr Ala Leu Arg Val Gln Gly Glu Pro Ala Lys Gly
        275                 280                 285

Glu Met Leu Ala Gly Thr Ala Val Tyr Asn Gly Glu Val Leu His Phe
    290                 295                 300

His Thr Glu Asn Gly Arg Pro Tyr Pro Thr Arg Gly Arg Phe Ala Ala
305                 310                 315                 320

Lys Val Asp Phe Gly Ser Lys Ser Val Asp Gly Ile Ile Asp Ser Gly
                325                 330                 335

Asp Asp Leu His Met Gly Thr Gln Lys Phe Lys Ala Ala Ile Asp Gly
            340                 345                 350

Asn Gly Phe Lys Gly Thr Trp Thr Glu Asn Gly Gly Gly Asp Val Ser
        355                 360                 365

Gly Arg Phe Tyr Gly Pro Ala Gly Glu Glu Val Ala Gly Lys Tyr Ser
    370                 375                 380

Tyr Arg Pro Thr Asp Ala Glu Lys Gly Gly Phe Gly Val Phe Ala Gly
385                 390                 395                 400

Lys Lys Glu Gln Asp Gly Ser Gly Gly Gly Gly Ala Thr Tyr Lys Val
                405                 410                 415

Asp Glu Tyr His Ala Asn Ala Arg Phe Ala Ile Asp His Phe Asn Thr
            420                 425                 430

Ser Thr Asn Val Gly Gly Phe Tyr Gly Leu Thr Gly Ser Val Glu Phe
        435                 440                 445

Asp Gln Ala Lys Arg Asp Gly Lys Ile Asp Ile Thr Ile Pro Val Ala
    450                 455                 460

Asn Leu Gln Ser Gly Ser Gln His Phe Thr Asp His Leu Lys Ser Ala
465                 470                 475                 480

Asp Ile Phe Asp Ala Ala Gln Tyr Pro Asp Ile Arg Phe Val Ser Thr
                485                 490                 495

Lys Phe Asn Phe Asn Gly Lys Lys Leu Val Ser Val Asp Gly Asn Leu
            500                 505                 510

Thr Met His Gly Lys Thr Ala Pro Val Lys Leu Lys Ala Glu Lys Phe
        515                 520                 525

Asn Cys Tyr Gln Ser Pro Met Ala Lys Thr Glu Val Cys Gly Gly Asp
    530                 535                 540
```

```
Phe Ser Thr Thr Ile Asp Arg Thr Lys Trp Gly Val Asp Tyr Leu Val
545                 550                 555                 560

Asn Val Gly Met Thr Lys Ser Val Arg Ile Asp Ile Gln Ile Glu Ala
                565                 570                 575

Ala Lys Gln
```

<210> 7
<211> 2388
<212> DNA
<213> Artificial Sequence

<220>
<223> deltaG287-961

<400> 7

```
atggctagcc ccgatgttaa atcggcggac acgctgtcaa aaccggccgc tcctgttgtt   60
gctgaaaaag agacagaggt aaaagaagat gcgccacagg caggttctca aggacagggc  120
gcgccatcca cacaaggcag ccaagatatg gcggcagttt cggcagaaaa tacaggcaat  180
ggcggtgcgg caacaacgga caaacccaaa aatgaagacg agggaccgca aaatgatatg  240
ccgcaaaatt ccgccgaatc cgcaaatcaa acagggaaca accaacccgc cgattcttca  300
gattccgccc ccgcgtcaaa ccctgcacct gcgaatggcg gtagcaattt tggaagggtt  360
gatttggcta atggcgtttt gattgatggg ccgtcgcaaa atataacgtt gacccactgt  420
aaaggcgatt cttgtaatgg tgataattta ttggatgaag aagcaccgtc aaaatcagaa  480
tttgaaaatt taaatgagtc tgaacgaatt gagaaatata agaaagatgg gaaaagcgat  540
aaatttacta atttggttgc gacagcagtt caagctaatg gaactaacaa atatgtcatc  600
atttataaag acaagtccgc ttcatcttca tctgcgcgat tcaggcgttc tgcacggtcg  660
aggaggtcgc ttcctgccga gatgccgcta atccccgtca atcaggcgga tacgctgatt  720
gtcgatgggg aagcggtcag cctgacgggg cattccggca atatcttcgc gcccgaaggg  780
aattaccggt atctgactta cggggcggaa aaattgcccg gcggatcgta tgccctccgt  840
gtgcaaggcg aaccggcaaa aggcgaaatg cttgctggca cggccgtgta caacggcgaa  900
gtgctgcatt ttcatacgga aaacggccgt ccgtacccga ctagaggcag gtttgccgca  960
aaagtcgatt tcggcagcaa atctgtggac ggcattatcg acagcggcga tgatttgcat 1020
atgggtacgc aaaaattcaa agccgccatc gatggaaacg gctttaaggg gacttggacg 1080
gaaaatggcg gcggggatgt ttccggaagg ttttacggcc cggccggcga ggaagtggcg 1140
ggaaaataca gctatcgccc gacagatgcg gaaaagggcg gattcggcgt gtttgccggc 1200
aaaaaagagc aggatggatc cggaggagga ggagccacaa acgacgacga tgttaaaaaa 1260
gctgccactg tggccattgc tgctgcctac aacaatggcc aagaaatcaa cggtttcaaa 1320
gctggagaga ccatctacga cattgatgaa gacggcacaa ttaccaaaaa agacgcaact 1380
gcagccgatg ttgaagccga cgactttaaa ggtctgggtc tgaaaaaagt cgtgactaac 1440
ctgaccaaaa ccgtcaatga aaacaaacaa aacgtcgatg ccaaagtaaa agctgcagaa 1500
tctgaaatag aaaagttaac aaccaagtta gcagacactg atgccgcttt agcagatact 1560
gatgccgctc tggatgcaac caccaacgcc ttgaataaat tgggagaaaa tataacgaca 1620
tttgctgaag agactaagac aaatatcgta aaaattgatg aaaaattaga agccgtggct 1680
gataccgtcg acaagcatgc cgaagcattc aacgatatcg ccgattcatt ggatgaaacc 1740
aacactaagg cagacgaagc cgtcaaaacc gccaatgaag ccaaacagac ggccgaagaa 1800
accaaacaaa acgtcgatgc caaagtaaaa gctgcagaaa ctgcagcagg caaagccgaa 1860
gctgccgctg gcacagctaa tactgcagcc gacaaggccg aagctgtcgc tgcaaaagtt 1920
accgacatca aagctgatat cgctacgaac aaagataata ttgctaaaaa agcaaacagt 1980
gccgacgtgt acaccagaga agagtctgac agcaaatttg tcagaattga tggtctgaac 2040
gctactaccg aaaaattgga cacacgcttg gcttctgctg aaaaatccat tgccgatcac 2100
gatactcgcc tgaacggttt ggataaaaca gtgtcagacc tgcgcaaaga aacccgccaa 2160
ggccttgcag aacaagccgc gctctccggt ctgttccaac cttacaacgt gggtcggttc 2220
aatgtaacgg ctgcagtcgg cggctacaaa tccgaatcgg cagtcgccat cggtaccggc 2280
ttccgcttta ccgaaaactt tgccgccaaa gcaggcgtgg cagtcggcac ttcgtccggt 2340
tcttccgcag cctaccatgt cggcgtcaat tacgagtggt aactcgag             2388
```

<210> 8
<211> 793
<212> PRT
<213> Artificial Sequence

<220>
<223> deltaG287-961

<400> 8

```
Met Ala Ser Pro Asp Val Lys Ser Ala Asp Thr Leu Ser Lys Pro Ala
1               5                   10                  15

Ala Pro Val Val Ala Glu Lys Glu Thr Glu Val Lys Glu Asp Ala Pro
            20                  25                  30

Gln Ala Gly Ser Gln Gly Gln Gly Ala Pro Ser Thr Gln Gly Ser Gln
        35                  40                  45

Asp Met Ala Ala Val Ser Ala Glu Asn Thr Gly Asn Gly Gly Ala Ala
    50                  55                  60

Thr Thr Asp Lys Pro Lys Asn Glu Asp Glu Gly Pro Gln Asn Asp Met
65                  70                  75                  80

Pro Gln Asn Ser Ala Glu Ser Ala Asn Gln Thr Gly Asn Asn Gln Pro
                85                  90                  95

Ala Asp Ser Ser Asp Ser Ala Pro Ala Ser Asn Pro Ala Pro Ala Asn
            100                 105                 110

Gly Gly Ser Asn Phe Gly Arg Val Asp Leu Ala Asn Gly Val Leu Ile
        115                 120                 125

Asp Gly Pro Ser Gln Asn Ile Thr Leu Thr His Cys Lys Gly Asp Ser
    130                 135                 140

Cys Asn Gly Asp Asn Leu Leu Asp Glu Glu Ala Pro Ser Lys Ser Glu
145                 150                 155                 160

Phe Glu Asn Leu Asn Glu Ser Glu Arg Ile Glu Lys Tyr Lys Lys Asp
                165                 170                 175

Gly Lys Ser Asp Lys Phe Thr Asn Leu Val Ala Thr Ala Val Gln Ala
            180                 185                 190

Asn Gly Thr Asn Lys Tyr Val Ile Ile Tyr Lys Asp Lys Ser Ala Ser
        195                 200                 205

Ser Ser Ser Ala Arg Phe Arg Arg Ser Ala Arg Ser Arg Arg Ser Leu
    210                 215                 220

Pro Ala Glu Met Pro Leu Ile Pro Val Asn Gln Ala Asp Thr Leu Ile
225                 230                 235                 240

Val Asp Gly Glu Ala Val Ser Leu Thr Gly His Ser Gly Asn Ile Phe
                245                 250                 255

Ala Pro Glu Gly Asn Tyr Arg Tyr Leu Thr Tyr Gly Ala Glu Lys Leu
            260                 265                 270

Pro Gly Gly Ser Tyr Ala Leu Arg Val Gln Gly Glu Pro Ala Lys Gly
        275                 280                 285
```

Glu Met Leu Ala Gly Thr Ala Val Tyr Asn Gly Glu Val Leu His Phe
290 295 300

His Thr Glu Asn Gly Arg Pro Tyr Pro Thr Arg Gly Arg Phe Ala Ala
305 310 315 320

Lys Val Asp Phe Gly Ser Lys Ser Val Asp Gly Ile Ile Asp Ser Gly
325 330 335

Asp Asp Leu His Met Gly Thr Gln Lys Phe Lys Ala Ala Ile Asp Gly
340 345 350

Asn Gly Phe Lys Gly Thr Trp Thr Glu Asn Gly Gly Gly Asp Val Ser
355 360 365

Gly Arg Phe Tyr Gly Pro Ala Gly Glu Glu Val Ala Gly Lys Tyr Ser
370 375 380

Tyr Arg Pro Thr Asp Ala Glu Lys Gly Gly Phe Gly Val Phe Ala Gly
385 390 395 400

Lys Lys Glu Gln Asp Gly Ser Gly Gly Gly Gly Ala Thr Asn Asp Asp
405 410 415

Asp Val Lys Lys Ala Ala Thr Val Ala Ile Ala Ala Ala Tyr Asn Asn
420 425 430

Gly Gln Glu Ile Asn Gly Phe Lys Ala Gly Glu Thr Ile Tyr Asp Ile
435 440 445

Asp Glu Asp Gly Thr Ile Thr Lys Lys Asp Ala Thr Ala Ala Asp Val
450 455 460

Glu Ala Asp Asp Phe Lys Gly Leu Gly Leu Lys Lys Val Val Thr Asn
465 470 475 480

Leu Thr Lys Thr Val Asn Glu Asn Lys Gln Asn Val Asp Ala Lys Val
485 490 495

Lys Ala Ala Glu Ser Glu Ile Glu Lys Leu Thr Thr Lys Leu Ala Asp
500 505 510

Thr Asp Ala Ala Leu Ala Asp Thr Asp Ala Ala Leu Asp Ala Thr Thr
515 520 525

Asn Ala Leu Asn Lys Leu Gly Glu Asn Ile Thr Thr Phe Ala Glu Glu
530 535 540

Thr Lys Thr Asn Ile Val Lys Ile Asp Glu Lys Leu Glu Ala Val Ala
545 550 555 560

Asp Thr Val Asp Lys His Ala Glu Ala Phe Asn Asp Ile Ala Asp Ser
565 570 575

Leu Asp Glu Thr Asn Thr Lys Ala Asp Glu Ala Val Lys Thr Ala Asn
580 585 590

Glu Ala Lys Gln Thr Ala Glu Glu Thr Lys Gln Asn Val Asp Ala Lys
595 600 605

```
Val Lys Ala Ala Glu Thr Ala Ala Gly Lys Ala Glu Ala Ala Ala Gly
    610                 615                 620

Thr Ala Asn Thr Ala Ala Asp Lys Ala Glu Ala Val Ala Ala Lys Val
625                 630                 635                 640

Thr Asp Ile Lys Ala Asp Ile Ala Thr Asn Lys Asp Asn Ile Ala Lys
                645                 650                 655

Lys Ala Asn Ser Ala Asp Val Tyr Thr Arg Glu Glu Ser Asp Ser Lys
            660                 665                 670

Phe Val Arg Ile Asp Gly Leu Asn Ala Thr Thr Glu Lys Leu Asp Thr
        675                 680                 685

Arg Leu Ala Ser Ala Glu Lys Ser Ile Ala Asp His Asp Thr Arg Leu
    690                 695                 700

Asn Gly Leu Asp Lys Thr Val Ser Asp Leu Arg Lys Glu Thr Arg Gln
705                 710                 715                 720

Gly Leu Ala Glu Gln Ala Ala Leu Ser Gly Leu Phe Gln Pro Tyr Asn
                725                 730                 735

Val Gly Arg Phe Asn Val Thr Ala Ala Val Gly Gly Tyr Lys Ser Glu
            740                 745                 750

Ser Ala Val Ala Ile Gly Thr Gly Phe Arg Phe Thr Glu Asn Phe Ala
        755                 760                 765

Ala Lys Ala Gly Val Ala Val Gly Thr Ser Ser Gly Ser Ser Ala Ala
    770                 775                 780

Tyr His Val Gly Val Asn Tyr Glu Trp
785                 790
```

<210> 9
<211> 2700
<212> DNA
<213> Artificial Sequence

<220>
<223> deltaG287NZ-919

<400> 9

```
atggctagcc ccgatgtcaa gtcggcggac acgctgtcaa aacctgccgc ccctgttgtt   60
tctgaaaaag agacagaggc aaaggaagat gcgccacagg caggttctca aggacagggc  120
gcgccatccg cacaaggcgg tcaagatatg gcggcggttt cggaagaaaa tacaggcaat  180
ggcggtgcgg cagcaacgga caaacccaaa aatgaagacg aggggggcgca aaatgatatg  240
ccgcaaaatg ccgccgatac agatagtttg acaccgaatc acaccccggc ttcgaatatg  300
ccggccggaa atatggaaaa ccaagcaccg gatgccgggg aatcggagca gccggcaaac  360
caaccggata tggcaaatac ggcggacgga atgcagggtg acgatccgtc ggcaggcggg  420
gaaaatgccg gcaatacggc tgcccaaggt acaaatcaag ccgaaaacaa tcaaaccgcc  480
ggttctcaaa atcctgcctc ttcaaccaat cctagcgcca cgaatagcgg tggtgatttt  540
ggaaggacga acgtgggcaa ttctgttgtg attgacgggc cgtcgcaaaa tataacgttg  600
acccactgta aaggcgattc ttgtagtggc aataatttct tggatgaaga agtacagcta  660
aaatcagaat ttgaaaaatt aagtgatgca gacaaaataa gtaattacaa gaaagatggg  720
aagaatgacg ggaagaatga taaatttgtc ggtttggttg ccgatagtgt gcagatgaag  780
ggaatcaatc aatatattat cttttataaa cctaaaccca cttcatttgc gcgatttagg  840
cgttctgcac ggtcgaggcg gtcgcttccg gccgagatgc cgctgattcc cgtcaatcag  900
gcggatacgc tgattgtcga tggggaagcg gtcagcctga cggggcattc cggcaatatc  960

ttcgcgcccg aagggaatta ccggtatctg acttacgggg cggaaaaatt gcccggcgga 1020
tcgtatgccc tccgtgttca aggcgaacct tcaaaaggcg aaatgctcgc gggcacggca 1080
gtgtacaacg gcgaagtgct gcattttcat acggaaaacg gccgtccgtc cccgtccaga 1140
ggcaggtttg ccgcaaaagt cgatttcggc agcaaatctg tggacggcat tatcgacagc 1200
ggcgatggtt tgcatatggg tacgcaaaaa ttcaaagccg ccatcgatgg aaacggcttt 1260
aaggggactt ggacggaaaa tggcggcggg gatgtttccg gaaagtttta cggcccggcc 1320
ggcgaggaag tggcgggaaa atacagctat cgcccaacag atgcggaaaa gggcggattc 1380
ggcgtgtttg ccggcaaaaa agagcaggat ggatccggag gaggaggatg ccaaagcaag 1440
agcatccaaa cctttccgca acccgacaca tccgtcatca acggcccgga ccggccggtc 1500
ggcatccccg accccgccgg aacgacggtc ggcggcggcg ggccgtcta taccgttgta 1560
ccgcacctgt ccctgcccca ctgggcggcg caggatttcg ccaaaagcct gcaatccttc 1620
cgcctcggct gcgccaattt gaaaaaccgc caaggctggc aggatgtgtg cgcccaagcc 1680
tttcaaaccc ccgtccattc ctttcaggca aaacagtttt ttgaacgcta tttcacgccg 1740
tggcaggttg caggcaacgg aagccttgcc ggtacggtta ccggctatta cgagccggtg 1800
ctgaagggcg acgacaggcg gacggcacaa gcccgcttcc cgatttacgg tattcccgac 1860
gattttatct ccgtcccct gcctgccggt ttgcggagcg gaaaagccct tgtccgcatc 1920
aggcagacgg gaaaaaacag cggcacaatc gacaataccg gcggcacaca taccgccgac 1980
ctctcccgat tccccatcac cgcgcgcaca acggcaatca aaggcaggtt tgaaggaagc 2040
cgcttcctcc cctaccacac gcgcaaccaa atcaacggcg gcgcgcttga cggcaaagcc 2100
ccgatactcg gttacgccga agaccccgtc gaactttttt ttatgcacat ccaaggctcg 2160
ggccgtctga aaccccgtc cggcaaatac atccgcatcg gctatgccga caaaaacgaa 2220
catccctacg tttccatcgg acgctatatg gcggacaaag gctacctcaa gctcgggcag 2280
acctcgatgc agggcatcaa agcctatatg cggcaaaatc cgcaacgcct cgccgaagtt 2340
ttgggtcaaa accccagcta tatcttttc cgcgagcttg ccggaagcag caatgacggt 2400
cccgtcggcg cactgggcac gccgttgatg gggaatatg ccggcgcagt cgaccggcac 2460
tacattacct tgggcgcgcc cttatttgtc gccaccgccc atccggttac ccgcaaagcc 2520
ctcaaccgcc tgattatggc gcaggatacc ggcagcgcga ttaaaggcgc ggtgcgcgtg 2580
gattatttt ggggatacgg cgacgaagcc ggcgaacttg ccggcaaaca gaaaaccacg 2640
ggttacgtct ggcagctcct acccaacggt atgaagcccg aataccgccc gtaaaagctt 2700
```

<210> 10
<211> 897
<212> PRT
<213> Artificial Sequence

<220>
<223> deltaG287NZ-919

<400> 10

```
Met Ala Ser Pro Asp Val Lys Ser Ala Asp Thr Leu Ser Lys Pro Ala
1               5                   10                  15

Ala Pro Val Val Ser Glu Lys Glu Thr Glu Ala Lys Glu Asp Ala Pro
            20                  25                  30

Gln Ala Gly Ser Gln Gly Gln Gly Ala Pro Ser Ala Gln Gly Gly Gln
        35                  40                  45

Asp Met Ala Ala Val Ser Glu Glu Asn Thr Gly Asn Gly Gly Ala Ala
    50                  55                  60

Ala Thr Asp Lys Pro Lys Asn Glu Asp Glu Gly Ala Gln Asn Asp Met
65                  70                  75                  80

Pro Gln Asn Ala Ala Asp Thr Asp Ser Leu Thr Pro Asn His Thr Pro
                85                  90                  95

Ala Ser Asn Met Pro Ala Gly Asn Met Glu Asn Gln Ala Pro Asp Ala
            100                 105                 110

Gly Glu Ser Glu Gln Pro Ala Asn Gln Pro Asp Met Ala Asn Thr Ala
```

115 120 125

Asp Gly Met Gln Gly Asp Asp Pro Ser Ala Gly Gly Glu Asn Ala Gly
130 135 140

Asn Thr Ala Ala Gln Gly Thr Asn Gln Ala Glu Asn Asn Gln Thr Ala
145 150 155 160

Gly Ser Gln Asn Pro Ala Ser Ser Thr Asn Pro Ser Ala Thr Asn Ser
165 170 175

Gly Gly Asp Phe Gly Arg Thr Asn Val Gly Asn Ser Val Val Ile Asp
180 185 190

Gly Pro Ser Gln Asn Ile Thr Leu Thr His Cys Lys Gly Asp Ser Cys
195 200 205

Ser Gly Asn Asn Phe Leu Asp Glu Glu Val Gln Leu Lys Ser Glu Phe
210 215 220

Glu Lys Leu Ser Asp Ala Asp Lys Ile Ser Asn Tyr Lys Lys Asp Gly
225 230 235 240

Lys Asn Asp Gly Lys Asn Asp Lys Phe Val Gly Leu Val Ala Asp Ser
245 250 255

Val Gln Met Lys Gly Ile Asn Gln Tyr Ile Ile Phe Tyr Lys Pro Lys
260 265 270

Pro Thr Ser Phe Ala Arg Phe Arg Arg Ser Ala Arg Ser Arg Arg Ser
275 280 285

Leu Pro Ala Glu Met Pro Leu Ile Pro Val Asn Gln Ala Asp Thr Leu
290 295 300

Ile Val Asp Gly Glu Ala Val Ser Leu Thr Gly His Ser Gly Asn Ile
305 310 315 320

Phe Ala Pro Glu Gly Asn Tyr Arg Tyr Leu Thr Tyr Gly Ala Glu Lys
325 330 335

Leu Pro Gly Gly Ser Tyr Ala Leu Arg Val Gln Gly Glu Pro Ser Lys
340 345 350

Gly Glu Met Leu Ala Gly Thr Ala Val Tyr Asn Gly Glu Val Leu His
355 360 365

Phe His Thr Glu Asn Gly Arg Pro Ser Pro Ser Arg Gly Arg Phe Ala
370 375 380

Ala Lys Val Asp Phe Gly Ser Lys Ser Val Asp Gly Ile Ile Asp Ser
385 390 395 400

Gly Asp Gly Leu His Met Gly Thr Gln Lys Phe Lys Ala Ala Ile Asp
405 410 415

Gly Asn Gly Phe Lys Gly Thr Trp Thr Glu Asn Gly Gly Gly Asp Val
420 425 430

Ser Gly Lys Phe Tyr Gly Pro Ala Gly Glu Glu Val Ala Gly Lys Tyr
435 440 445

```
Ser Tyr Arg Pro Thr Asp Ala Glu Lys Gly Gly Phe Gly Val Phe Ala
    450                 455                 460

Gly Lys Lys Glu Gln Asp Gly Ser Gly Gly Gly Gly Cys Gln Ser Lys
465                 470                 475                 480

Ser Ile Gln Thr Phe Pro Gln Pro Asp Thr Ser Val Ile Asn Gly Pro
                485                 490                 495

Asp Arg Pro Val Gly Ile Pro Asp Pro Ala Gly Thr Thr Val Gly Gly
            500                 505                 510

Gly Gly Ala Val Tyr Thr Val Val Pro His Leu Ser Leu Pro His Trp
        515                 520                 525

Ala Ala Gln Asp Phe Ala Lys Ser Leu Gln Ser Phe Arg Leu Gly Cys
    530                 535                 540

Ala Asn Leu Lys Asn Arg Gln Gly Trp Gln Asp Val Cys Ala Gln Ala
545                 550                 555                 560

Phe Gln Thr Pro Val His Ser Phe Gln Ala Lys Gln Phe Phe Glu Arg
                565                 570                 575

Tyr Phe Thr Pro Trp Gln Val Ala Gly Asn Gly Ser Leu Ala Gly Thr
            580                 585                 590

Val Thr Gly Tyr Tyr Glu Pro Val Leu Lys Gly Asp Asp Arg Arg Thr
        595                 600                 605

Ala Gln Ala Arg Phe Pro Ile Tyr Gly Ile Pro Asp Asp Phe Ile Ser
    610                 615                 620

Val Pro Leu Pro Ala Gly Leu Arg Ser Gly Lys Ala Leu Val Arg Ile
625                 630                 635                 640

Arg Gln Thr Gly Lys Asn Ser Gly Thr Ile Asp Asn Thr Gly Gly Thr
                645                 650                 655

His Thr Ala Asp Leu Ser Arg Phe Pro Ile Thr Ala Arg Thr Thr Ala
            660                 665                 670

Ile Lys Gly Arg Phe Glu Gly Ser Arg Phe Leu Pro Tyr His Thr Arg
        675                 680                 685

Asn Gln Ile Asn Gly Gly Ala Leu Asp Gly Lys Ala Pro Ile Leu Gly
    690                 695                 700

Tyr Ala Glu Asp Pro Val Glu Leu Phe Phe Met His Ile Gln Gly Ser
705                 710                 715                 720

Gly Arg Leu Lys Thr Pro Ser Gly Lys Tyr Ile Arg Ile Gly Tyr Ala
                725                 730                 735

Asp Lys Asn Glu His Pro Tyr Val Ser Ile Gly Arg Tyr Met Ala Asp
            740                 745                 750

Lys Gly Tyr Leu Lys Leu Gly Gln Thr Ser Met Gln Gly Ile Lys Ala
        755                 760                 765
```

```
Tyr Met Arg Gln Asn Pro Gln Arg Leu Ala Glu Val Leu Gly Gln Asn
    770                 775                 780

Pro Ser Tyr Ile Phe Phe Arg Glu Leu Ala Gly Ser Ser Asn Asp Gly
785                 790                 795                 800

Pro Val Gly Ala Leu Gly Thr Pro Leu Met Gly Glu Tyr Ala Gly Ala
                805                 810                 815

Val Asp Arg His Tyr Ile Thr Leu Gly Ala Pro Leu Phe Val Ala Thr
            820                 825                 830

Ala His Pro Val Thr Arg Lys Ala Leu Asn Arg Leu Ile Met Ala Gln
        835                 840                 845

Asp Thr Gly Ser Ala Ile Lys Gly Ala Val Arg Val Asp Tyr Phe Trp
    850                 855                 860

Gly Tyr Gly Asp Glu Ala Gly Glu Leu Ala Gly Lys Gln Lys Thr Thr
865                 870                 875                 880

Gly Tyr Val Trp Gln Leu Leu Pro Asn Gly Met Lys Pro Glu Tyr Arg
                885                 890                 895

Pro
```

<210> 11
<211> 1941
<212> DNA
<213> Artificial Sequence

<220>
<223> deltaG287NZ-953

<400> 11

```
atggctagcc ccgatgtcaa gtcggcggac acgctgtcaa aacctgccgc ccctgttgtt   60
tctgaaaaag agacagaggc aaaggaagat gcgccacagg caggttctca aggacagggc  120
gcgccatccg cacaaggcgg tcaagatatg gcggcggttt cggaagaaaa tacaggcaat  180
ggcggtgcgg cagcaacgga caaacccaaa aatgaagacg aggggcgca aaatgatatg   240
ccgcaaaatg ccgccgatac agatagtttg acaccgaatc acaccccggc ttcgaatatg  300
ccggccggaa atatggaaaa ccaagcaccg gatgccgggg aatcggagca gccggcaaac  360
caaccggata tggcaaatac ggcggacgga atgcagggtg acgatccgtc ggcaggcggg  420
gaaaatgccg gcaatacggc tgcccaaggt acaaatcaag ccgaaaacaa tcaaaccgcc  480
ggttctcaaa atcctgcctc ttcaaccaat cctagcgcca cgaatagcgg tggtgatttt  540
ggaaggacga acgtgggcaa ttctgttgtg attgacgggc cgtcgcaaaa tataacgttg  600
acccactgta aaggcgattc ttgtagtggc aataatttct tggatgaaga agtacagcta  660
aaatcagaat ttgaaaaatt aagtgatgca gacaaaataa gtaattacaa gaaagatggg  720
aagaatgacg ggaagaatga taaatttgtc ggtttggttg ccgatagtgt gcagatgaag  780
ggaatcaatc aatatattat cttttataaa cctaaaccca cttcatttgc gcgatttagg  840
cgttctgcac ggtcgaggcg gtcgcttccg gccgagatgc cgctgattcc cgtcaatcag  900
gcggatacgc tgattgtcga tggggaagcg gtcagcctga cggggcattc cggcaatatc  960
ttcgcgcccg aagggaatta ccggtatctg acttacgggg cggaaaaatt gcccggcgga 1020
tcgtatgccc tccgtgttca aggcgaacct tcaaaaggcg aaatgctcgc gggcacggca 1080
gtgtacaacg gcgaagtgct gcattttcat acggaaaacg gccgtccgtc cccgtccaga 1140
ggcaggtttg ccgcaaaagt cgatttcggc agcaaatctg tggacggcat tatcgacagc 1200
ggcgatggtt tgcatatggg tacgcaaaaa ttcaaagccg ccatcgatgg aaacggcttt 1260
aaggggactt ggacggaaaa tggcggcggg gatgtttccg gaaagtttta cggcccggcc 1320
ggcgaggaag tggcgggaaa atacagctat cgcccaacag atgcggaaaa gggcggattc 1380
ggcgtgtttg ccggcaaaaa agagcaggat ggatccggag gaggaggagc cacctacaaa 1440
gtggacgaat atcacgccaa cgcccgtttc gccatcgacc atttcaacac cagcaccaac 1500

gtcggcggtt tttacggtct gaccggttcc gtcgagttcg accaagcaaa acgcgacggt 1560
aaaatcgaca tcaccatccc cgttgccaac ctgcaaagcg gttcgcaaca ctttaccgac 1620
cacctgaaat cagccgacat cttcgatgcc gcccaatatc cggacatccg ctttgtttcc 1680
accaaattca acttcaacgg caaaaaactg gtttccgttg acggcaacct gaccatgcac 1740
ggcaaaaccg cccccgtcaa actcaaagcc gaaaaattca actgctacca aagcccgatg 1800
gcgaaaaccg aagtttgcgg cggcgacttc agcaccacca tcgaccgcac caaatggggc 1860
gtggactacc tcgttaacgt tggtatgacc aaaagcgtcc gcatcgacat ccaaatcgag 1920
gcagccaaac aataaaagct t                                           1941
```

<210> 12
<211> 644
<212> PRT
<213> Artificial Sequence

<220>
<223> deltaG287NZ-953

<400> 12

```
Met Ala Ser Pro Asp Val Lys Ser Ala Asp Thr Leu Ser Lys Pro Ala
1               5                   10                  15

Ala Pro Val Val Ser Glu Lys Glu Thr Glu Ala Lys Glu Asp Ala Pro
            20                  25                  30

Gln Ala Gly Ser Gln Gly Gln Gly Ala Pro Ser Ala Gln Gly Gly Gln
        35                  40                  45

Asp Met Ala Ala Val Ser Glu Glu Asn Thr Gly Asn Gly Gly Ala Ala
    50                  55                  60

Ala Thr Asp Lys Pro Lys Asn Glu Asp Glu Gly Ala Gln Asn Asp Met
65                  70                  75                  80

Pro Gln Asn Ala Ala Asp Thr Asp Ser Leu Thr Pro Asn His Thr Pro
                85                  90                  95

Ala Ser Asn Met Pro Ala Gly Asn Met Glu Asn Gln Ala Pro Asp Ala
            100                 105                 110

Gly Glu Ser Glu Gln Pro Ala Asn Gln Pro Asp Met Ala Asn Thr Ala
        115                 120                 125

Asp Gly Met Gln Gly Asp Asp Pro Ser Ala Gly Gly Glu Asn Ala Gly
    130                 135                 140

Asn Thr Ala Ala Gln Gly Thr Asn Gln Ala Glu Asn Asn Gln Thr Ala
145                 150                 155                 160

Gly Ser Gln Asn Pro Ala Ser Ser Thr Asn Pro Ser Ala Thr Asn Ser
                165                 170                 175

Gly Gly Asp Phe Gly Arg Thr Asn Val Gly Asn Ser Val Val Ile Asp
            180                 185                 190

Gly Pro Ser Gln Asn Ile Thr Leu Thr His Cys Lys Gly Asp Ser Cys
        195                 200                 205

Ser Gly Asn Asn Phe Leu Asp Glu Glu Val Gln Leu Lys Ser Glu Phe
    210                 215                 220

Glu Lys Leu Ser Asp Ala Asp Lys Ile Ser Asn Tyr Lys Lys Asp Gly
```

```
225                 230                 235                 240

Lys Asn Asp Gly Lys Asn Asp Lys Phe Val Gly Leu Val Ala Asp Ser
                245                 250                 255

Val Gln Met Lys Gly Ile Asn Gln Tyr Ile Ile Phe Tyr Lys Pro Lys
            260                 265                 270

Pro Thr Ser Phe Ala Arg Phe Arg Arg Ser Ala Arg Ser Arg Arg Ser
        275                 280                 285

Leu Pro Ala Glu Met Pro Leu Ile Pro Val Asn Gln Ala Asp Thr Leu
    290                 295                 300

Ile Val Asp Gly Glu Ala Val Ser Leu Thr Gly His Ser Gly Asn Ile
305                 310                 315                 320

Phe Ala Pro Glu Gly Asn Tyr Arg Tyr Leu Thr Tyr Gly Ala Glu Lys
                325                 330                 335

Leu Pro Gly Gly Ser Tyr Ala Leu Arg Val Gln Gly Glu Pro Ser Lys
            340                 345                 350

Gly Glu Met Leu Ala Gly Thr Ala Val Tyr Asn Gly Glu Val Leu His
        355                 360                 365

Phe His Thr Glu Asn Gly Arg Pro Ser Pro Ser Arg Gly Arg Phe Ala
    370                 375                 380

Ala Lys Val Asp Phe Gly Ser Lys Ser Val Asp Gly Ile Ile Asp Ser
385                 390                 395                 400

Gly Asp Gly Leu His Met Gly Thr Gln Lys Phe Lys Ala Ala Ile Asp
                405                 410                 415

Gly Asn Gly Phe Lys Gly Thr Trp Thr Glu Asn Gly Gly Gly Asp Val
            420                 425                 430

Ser Gly Lys Phe Tyr Gly Pro Ala Gly Glu Glu Val Ala Gly Lys Tyr
        435                 440                 445

Ser Tyr Arg Pro Thr Asp Ala Glu Lys Gly Gly Phe Gly Val Phe Ala
    450                 455                 460

Gly Lys Lys Glu Gln Asp Gly Ser Gly Gly Gly Gly Ala Thr Tyr Lys
465                 470                 475                 480

Val Asp Glu Tyr His Ala Asn Ala Arg Phe Ala Ile Asp His Phe Asn
                485                 490                 495

Thr Ser Thr Asn Val Gly Gly Phe Tyr Gly Leu Thr Gly Ser Val Glu
            500                 505                 510

Phe Asp Gln Ala Lys Arg Asp Gly Lys Ile Asp Ile Thr Ile Pro Val
        515                 520                 525

Ala Asn Leu Gln Ser Gly Ser Gln His Phe Thr Asp His Leu Lys Ser
    530                 535                 540

Ala Asp Ile Phe Asp Ala Ala Gln Tyr Pro Asp Ile Arg Phe Val Ser
545                 550                 555                 560
```

```
Thr Lys Phe Asn Phe Asn Gly Lys Lys Leu Val Ser Val Asp Gly Asn
                565                 570                 575

Leu Thr Met His Gly Lys Thr Ala Pro Val Lys Leu Lys Ala Glu Lys
            580                 585                 590

Phe Asn Cys Tyr Gln Ser Pro Met Ala Lys Thr Glu Val Cys Gly Gly
        595                 600                 605

Asp Phe Ser Thr Thr Ile Asp Arg Thr Lys Trp Gly Val Asp Tyr Leu
    610                 615                 620

Val Asn Val Gly Met Thr Lys Ser Val Arg Ile Asp Ile Gln Ile Glu
625                 630                 635                 640

Ala Ala Lys Gln
```

<210> 13
<211> 2583
<212> DNA
<213> Artificial Sequence

<220>
<223> deltaG287NZ-961

<400> 13

```
atggctagcc ccgatgtcaa gtcggcggac acgctgtcaa aacctgccgc ccctgttgtt   60
tctgaaaaag agacagaggc aaaggaagat gcgccacagg caggttctca aggacagggc   120
gcgccatccg cacaaggcgg tcaagatatg gcggcggttt cggaagaaaa tacaggcaat   180
ggcggtgcgg cagcaacgga caaacccaaa aatgaagacg agggggcgca aaatgatatg   240
ccgcaaaatg ccgccgatac agatagtttg acaccgaatc acaccccggc ttcgaatatg   300
ccggccggaa atatggaaaa ccaagcaccg gatgccgggg aatcggagca gccggcaaac   360
caaccggata tggcaaatac ggcggacgga atgcagggtg acgatccgtc ggcaggcggg   420
gaaaatgccg gcaatacggc tgcccaaggt acaaatcaag ccgaaaacaa tcaaaccgcc   480
ggttctcaaa atcctgcctc ttcaaccaat cctagcgcca cgaatagcgg tggtgatttt   540
ggaaggacga acgtgggcaa ttctgttgtg attgacgggc cgtcgcaaaa tataacgttg   600
acccactgta aaggcgattc ttgtagtggc aataatttct tggatgaaga agtacagcta   660
aaatcagaat ttgaaaaatt aagtgatgca gacaaaataa gtaattacaa gaaagatggg   720
aagaatgacg ggaagaatga taaatttgtc ggtttggttg ccgatagtgt gcagatgaag   780
ggaatcaatc aatatattat cttttataaa cctaaaccca cttcatttgc gcgatttagg   840
cgttctgcac ggtcgaggcg gtcgcttccg gccgagatgc cgctgattcc cgtcaatcag   900
gcggatacgc tgattgtcga tggggaagcg gtcagcctga cggggcattc cggcaatatc   960
ttcgcgcccg aagggaatta ccggtatctg acttacgggg cggaaaaatt gcccggcgga   1020
tcgtatgccc tccgtgttca aggcgaacct tcaaaaggcg aaatgctcgc gggcacggca   1080
gtgtacaacg gcgaagtgct gcattttcat acggaaaacg gccgtccgtc cccgtccaga   1140
ggcaggtttg ccgcaaaagt cgatttcggc agcaaatctg tggacggcat tatcgacagc   1200
ggcgatggtt tgcatatggg tacgcaaaaa ttcaaagccg ccatcgatgg aaacggcttt   1260
aaggggactt ggacggaaaa tggcggcggg gatgtttccg gaaagtttta cggcccggcc   1320
ggcgaggaag tggcgggaaa atacagctat cgcccaacag atgcggaaaa gggcggattc   1380
ggcgtgtttg ccggcaaaaa agagcaggat ggatccggag gaggaggagc cacaaacgac   1440
gacgatgtta aaaaagctgc cactgtggcc attgctgctg cctacaacaa tggccaagaa   1500
atcaacggtt tcaaagctgg agagaccatc tacgacattg atgaagacgg cacaattacc   1560
aaaaaagacg caactgcagc cgatgttgaa gccgacgact ttaaaggtct gggtctgaaa   1620
aaagtcgtga ctaacctgac caaaaccgtc aatgaaaaca aacaaaacgt cgatgccaaa   1680
gtaaaagctg cagaatctga aatagaaaag ttaacaacca agttagcaga cactgatgcc   1740
gctttagcag atactgatgc cgctctggat gcaaccacca acgccttgaa taaattggga   1800
gaaaatataa cgacatttgc tgaagagact aagacaaata tcgtaaaaat tgatgaaaaa   1860
ttagaagccg tggctgatac cgtcgacaag catgccgaag cattcaacga tatcgccgat   1920
tcattggatg aaaccaacac taaggcagac gaagccgtca aaaccgccaa tgaagccaaa   1980

cagacggccg aagaaaccaa acaaaacgtc gatgccaaag taaaagctgc agaaactgca   2040
gcaggcaaag ccgaagctgc cgctggcaca gctaatactg cagccgacaa ggccgaagct   2100
gtcgctgcaa aagttaccga catcaaagct gatatcgcta cgaacaaaga taatattgct   2160
aaaaaagcaa acagtgccga cgtgtacacc agagaagagt ctgacagcaa atttgtcaga   2220
attgatggtc tgaacgctac taccgaaaaa ttggacacac gcttggcttc tgctgaaaaa   2280
tccattgccg atcacgatac tcgcctgaac ggtttggata aaacagtgtc agacctgcgc   2340
aaagaaaccc gccaaggcct tgcagaacaa gccgcgctct ccggtctgtt ccaaccttac   2400
aacgtgggtc ggttcaatgt aacggctgca gtcggcggct acaaatccga atcggcagtc   2460
gccatcggta ccggcttccg ctttaccgaa aactttgccg ccaaagcagg cgtggcagtc   2520
ggcacttcgt ccggttcttc cgcagcctac catgtcggcg tcaattacga gtggtaaaag   2580
ctt                                                                 2583
```

<210> 14
<211> 858
<212> PRT
<213> Artificial Sequence

<220>
<223> deltaG287NZ-961

<400> 14

```
Met Ala Ser Pro Asp Val Lys Ser Ala Asp Thr Leu Ser Lys Pro Ala
1               5                   10                  15

Ala Pro Val Val Ser Glu Lys Glu Thr Glu Ala Lys Glu Asp Ala Pro
            20                  25                  30

Gln Ala Gly Ser Gln Gly Gln Gly Ala Pro Ser Ala Gln Gly Gly Gln
        35                  40                  45

Asp Met Ala Ala Val Ser Glu Glu Asn Thr Gly Asn Gly Gly Ala Ala
    50                  55                  60

Ala Thr Asp Lys Pro Lys Asn Glu Asp Glu Gly Ala Gln Asn Asp Met
65                  70                  75                  80

Pro Gln Asn Ala Ala Asp Thr Asp Ser Leu Thr Pro Asn His Thr Pro
                85                  90                  95

Ala Ser Asn Met Pro Ala Gly Asn Met Glu Asn Gln Ala Pro Asp Ala
            100                 105                 110

Gly Glu Ser Glu Gln Pro Ala Asn Gln Pro Asp Met Ala Asn Thr Ala
        115                 120                 125

Asp Gly Met Gln Gly Asp Asp Pro Ser Ala Gly Gly Glu Asn Ala Gly
    130                 135                 140

Asn Thr Ala Ala Gln Gly Thr Asn Gln Ala Glu Asn Asn Gln Thr Ala
145                 150                 155                 160

Gly Ser Gln Asn Pro Ala Ser Ser Thr Asn Pro Ser Ala Thr Asn Ser
                165                 170                 175

Gly Gly Asp Phe Gly Arg Thr Asn Val Gly Asn Ser Val Val Ile Asp
            180                 185                 190

Gly Pro Ser Gln Asn Ile Thr Leu Thr His Cys Lys Gly Asp Ser Cys
        195                 200                 205

Ser Gly Asn Asn Phe Leu Asp Glu Glu Val Gln Leu Lys Ser Glu Phe
```

```
    210                 215                 220

Glu Lys Leu Ser Asp Ala Asp Lys Ile Ser Asn Tyr Lys Lys Asp Gly
225                 230                 235                 240

Lys Asn Asp Gly Lys Asn Asp Lys Phe Val Gly Leu Val Ala Asp Ser
                245                 250                 255

Val Gln Met Lys Gly Ile Asn Gln Tyr Ile Ile Phe Tyr Lys Pro Lys
            260                 265                 270

Pro Thr Ser Phe Ala Arg Phe Arg Arg Ser Ala Arg Ser Arg Arg Ser
        275                 280                 285

Leu Pro Ala Glu Met Pro Leu Ile Pro Val Asn Gln Ala Asp Thr Leu
    290                 295                 300

Ile Val Asp Gly Glu Ala Val Ser Leu Thr Gly His Ser Gly Asn Ile
305                 310                 315                 320

Phe Ala Pro Glu Gly Asn Tyr Arg Tyr Leu Thr Tyr Gly Ala Glu Lys
                325                 330                 335

Leu Pro Gly Gly Ser Tyr Ala Leu Arg Val Gln Gly Glu Pro Ser Lys
            340                 345                 350

Gly Glu Met Leu Ala Gly Thr Ala Val Tyr Asn Gly Glu Val Leu His
        355                 360                 365

Phe His Thr Glu Asn Gly Arg Pro Ser Pro Ser Arg Gly Arg Phe Ala
    370                 375                 380

Ala Lys Val Asp Phe Gly Ser Lys Ser Val Asp Gly Ile Ile Asp Ser
385                 390                 395                 400

Gly Asp Gly Leu His Met Gly Thr Gln Lys Phe Lys Ala Ala Ile Asp
                405                 410                 415

Gly Asn Gly Phe Lys Gly Thr Trp Thr Glu Asn Gly Gly Gly Asp Val
            420                 425                 430

Ser Gly Lys Phe Tyr Gly Pro Ala Gly Glu Glu Val Ala Gly Lys Tyr
        435                 440                 445

Ser Tyr Arg Pro Thr Asp Ala Glu Lys Gly Gly Phe Gly Val Phe Ala
    450                 455                 460

Gly Lys Lys Glu Gln Asp Gly Ser Gly Gly Gly Gly Ala Thr Asn Asp
465                 470                 475                 480

Asp Asp Val Lys Lys Ala Ala Thr Val Ala Ile Ala Ala Ala Tyr Asn
                485                 490                 495

Asn Gly Gln Glu Ile Asn Gly Phe Lys Ala Gly Glu Thr Ile Tyr Asp
            500                 505                 510

Ile Asp Glu Asp Gly Thr Ile Thr Lys Lys Asp Ala Thr Ala Ala Asp
        515                 520                 525

Val Glu Ala Asp Asp Phe Lys Gly Leu Gly Leu Lys Lys Val Val Thr
    530                 535                 540
```

```
Asn Leu Thr Lys Thr Val Asn Glu Asn Lys Gln Asn Val Asp Ala Lys
545                 550                 555                 560

Val Lys Ala Ala Glu Ser Glu Ile Glu Lys Leu Thr Thr Lys Leu Ala
                565                 570                 575

Asp Thr Asp Ala Ala Leu Ala Asp Thr Asp Ala Ala Leu Asp Ala Thr
            580                 585                 590

Thr Asn Ala Leu Asn Lys Leu Gly Glu Asn Ile Thr Thr Phe Ala Glu
        595                 600                 605

Glu Thr Lys Thr Asn Ile Val Lys Ile Asp Glu Lys Leu Glu Ala Val
    610                 615                 620

Ala Asp Thr Val Asp Lys His Ala Glu Ala Phe Asn Asp Ile Ala Asp
625                 630                 635                 640

Ser Leu Asp Glu Thr Asn Thr Lys Ala Asp Glu Ala Val Lys Thr Ala
                645                 650                 655

Asn Glu Ala Lys Gln Thr Ala Glu Glu Thr Lys Gln Asn Val Asp Ala
            660                 665                 670

Lys Val Lys Ala Ala Glu Thr Ala Ala Gly Lys Ala Glu Ala Ala Ala
        675                 680                 685

Gly Thr Ala Asn Thr Ala Ala Asp Lys Ala Glu Ala Val Ala Ala Lys
    690                 695                 700

Val Thr Asp Ile Lys Ala Asp Ile Ala Thr Asn Lys Asp Asn Ile Ala
705                 710                 715                 720

Lys Lys Ala Asn Ser Ala Asp Val Tyr Thr Arg Glu Glu Ser Asp Ser
                725                 730                 735

Lys Phe Val Arg Ile Asp Gly Leu Asn Ala Thr Thr Glu Lys Leu Asp
            740                 745                 750

Thr Arg Leu Ala Ser Ala Glu Lys Ser Ile Ala Asp His Asp Thr Arg
        755                 760                 765

Leu Asn Gly Leu Asp Lys Thr Val Ser Asp Leu Arg Lys Glu Thr Arg
    770                 775                 780

Gln Gly Leu Ala Glu Gln Ala Ala Leu Ser Gly Leu Phe Gln Pro Tyr
785                 790                 795                 800

Asn Val Gly Arg Phe Asn Val Thr Ala Ala Val Gly Gly Tyr Lys Ser
                805                 810                 815

Glu Ser Ala Val Ala Ile Gly Thr Gly Phe Arg Phe Thr Glu Asn Phe
            820                 825                 830

Ala Ala Lys Ala Gly Val Ala Val Gly Thr Ser Ser Gly Ser Ser Ala
        835                 840                 845

Ala Tyr His Val Gly Val Asn Tyr Glu Trp
    850                 855
```

<210> 15
<211> 1082
<212> PRT
<213> Artificial Sequence

<220>
<223> 983

<400> 15

Met Arg Thr Thr Pro Thr Phe Pro Thr Lys Thr Phe Lys Pro Thr Ala
1 5 10 15

Met Ala Leu Ala Val Ala Thr Thr Leu Ser Ala Cys Leu Gly Gly Gly
20 25 30

Gly Gly Gly Thr Ser Ala Pro Asp Phe Asn Ala Gly Gly Thr Gly Ile
35 40 45

Gly Ser Asn Ser Arg Ala Thr Thr Ala Lys Ser Ala Ala Val Ser Tyr
50 55 60

Ala Gly Ile Lys Asn Glu Met Cys Lys Asp Arg Ser Met Leu Cys Ala
65 70 75 80

Gly Arg Asp Asp Val Ala Val Thr Asp Arg Asp Ala Lys Ile Asn Ala
85 90 95

Pro Pro Pro Asn Leu His Thr Gly Asp Phe Pro Asn Pro Asn Asp Ala
100 105 110

Tyr Lys Asn Leu Ile Asn Leu Lys Pro Ala Ile Glu Ala Gly Tyr Thr
115 120 125

Gly Arg Gly Val Glu Val Gly Ile Val Asp Thr Gly Glu Ser Val Gly
130 135 140

Ser Ile Ser Phe Pro Glu Leu Tyr Gly Arg Lys Glu His Gly Tyr Asn
145 150 155 160

Glu Asn Tyr Lys Asn Tyr Thr Ala Tyr Met Arg Lys Glu Ala Pro Glu
165 170 175

Asp Gly Gly Gly Lys Asp Ile Glu Ala Ser Phe Asp Asp Glu Ala Val
180 185 190

Ile Glu Thr Glu Ala Lys Pro Thr Asp Ile Arg His Val Lys Glu Ile
195 200 205

Gly His Ile Asp Leu Val Ser His Ile Ile Gly Gly Arg Ser Val Asp
210 215 220

Gly Arg Pro Ala Gly Gly Ile Ala Pro Asp Ala Thr Leu His Ile Met
225 230 235 240

Asn Thr Asn Asp Glu Thr Lys Asn Glu Met Met Val Ala Ala Ile Arg
245 250 255

Asn Ala Trp Val Lys Leu Gly Glu Arg Gly Val Arg Ile Val Asn Asn
260 265 270

Ser Phe Gly Thr Thr Ser Arg Ala Gly Thr Ala Asp Leu Phe Gln Ile

275 280 285

Ala Asn Ser Glu Glu Gln Tyr Arg Gln Ala Leu Leu Asp Tyr Ser Gly
290 295 300

Gly Asp Lys Thr Asp Glu Gly Ile Arg Leu Met Gln Gln Ser Asp Tyr
305 310 315 320

Gly Asn Leu Ser Tyr His Ile Arg Asn Lys Asn Met Leu Phe Ile Phe
325 330 335

Ser Thr Gly Asn Asp Ala Gln Ala Gln Pro Asn Thr Tyr Ala Leu Leu
340 345 350

Pro Phe Tyr Glu Lys Asp Ala Gln Lys Gly Ile Ile Thr Val Ala Gly
355 360 365

Val Asp Arg Ser Gly Glu Lys Phe Lys Arg Glu Met Tyr Gly Glu Pro
370 375 380

Gly Thr Glu Pro Leu Glu Tyr Gly Ser Asn His Cys Gly Ile Thr Ala
385 390 395 400

Met Trp Cys Leu Ser Ala Pro Tyr Glu Ala Ser Val Arg Phe Thr Arg
405 410 415

Thr Asn Pro Ile Gln Ile Ala Gly Thr Ser Phe Ser Ala Pro Ile Val
420 425 430

Thr Gly Thr Ala Ala Leu Leu Leu Gln Lys Tyr Pro Trp Met Ser Asn
435 440 445

Asp Asn Leu Arg Thr Thr Leu Leu Thr Thr Ala Gln Asp Ile Gly Ala
450 455 460

Val Gly Val Asp Ser Lys Phe Gly Trp Gly Leu Leu Asp Ala Gly Lys
465 470 475 480

Ala Met Asn Gly Pro Ala Ser Phe Pro Phe Gly Asp Phe Thr Ala Asp
485 490 495

Thr Lys Gly Thr Ser Asp Ile Ala Tyr Ser Phe Arg Asn Asp Ile Ser
500 505 510

Gly Thr Gly Gly Leu Ile Lys Lys Gly Gly Ser Gln Leu Gln Leu His
515 520 525

Gly Asn Asn Thr Tyr Thr Gly Lys Thr Ile Ile Glu Gly Gly Ser Leu
530 535 540

Val Leu Tyr Gly Asn Asn Lys Ser Asp Met Arg Val Glu Thr Lys Gly
545 550 555 560

Ala Leu Ile Tyr Asn Gly Ala Ala Ser Gly Gly Ser Leu Asn Ser Asp
565 570 575

Gly Ile Val Tyr Leu Ala Asp Thr Asp Gln Ser Gly Ala Asn Glu Thr
580 585 590

Val His Ile Lys Gly Ser Leu Gln Leu Asp Gly Lys Gly Thr Leu Tyr
595 600 605

```
Thr Arg Leu Gly Lys Leu Leu Lys Val Asp Gly Thr Ala Ile Ile Gly
    610                 615                 620

Gly Lys Leu Tyr Met Ser Ala Arg Gly Lys Gly Ala Gly Tyr Leu Asn
625                 630                 635                 640

Ser Thr Gly Arg Arg Val Pro Phe Leu Ser Ala Ala Lys Ile Gly Gln
                645                 650                 655

Asp Tyr Ser Phe Phe Thr Asn Ile Glu Thr Asp Gly Gly Leu Leu Ala
            660                 665                 670

Ser Leu Asp Ser Val Glu Lys Thr Ala Gly Ser Glu Gly Asp Thr Leu
        675                 680                 685

Ser Tyr Tyr Val Arg Arg Gly Asn Ala Ala Arg Thr Ala Ser Ala Ala
    690                 695                 700

Ala His Ser Ala Pro Ala Gly Leu Lys His Ala Val Glu Gln Gly Gly
705                 710                 715                 720

Ser Asn Leu Glu Asn Leu Met Val Glu Leu Asp Ala Ser Glu Ser Ser
                725                 730                 735

Ala Thr Pro Glu Thr Val Glu Thr Ala Ala Ala Asp Arg Thr Asp Met
            740                 745                 750

Pro Gly Ile Arg Pro Tyr Gly Ala Thr Phe Arg Ala Ala Ala Ala Val
        755                 760                 765

Gln His Ala Asn Ala Ala Asp Gly Val Arg Ile Phe Asn Ser Leu Ala
    770                 775                 780

Ala Thr Val Tyr Ala Asp Ser Thr Ala Ala His Ala Asp Met Gln Gly
785                 790                 795                 800

Arg Arg Leu Lys Ala Val Ser Asp Gly Leu Asp His Asn Gly Thr Gly
                805                 810                 815

Leu Arg Val Ile Ala Gln Thr Gln Gln Asp Gly Gly Thr Trp Glu Gln
            820                 825                 830

Gly Gly Val Glu Gly Lys Met Arg Gly Ser Thr Gln Thr Val Gly Ile
        835                 840                 845

Ala Ala Lys Thr Gly Glu Asn Thr Thr Ala Ala Ala Thr Leu Gly Met
    850                 855                 860

Gly Arg Ser Thr Trp Ser Glu Asn Ser Ala Asn Ala Lys Thr Asp Ser
865                 870                 875                 880

Ile Ser Leu Phe Ala Gly Ile Arg His Asp Ala Gly Asp Ile Gly Tyr
                885                 890                 895

Leu Lys Gly Leu Phe Ser Tyr Gly Arg Tyr Lys Asn Ser Ile Ser Arg
            900                 905                 910

Ser Thr Gly Ala Asp Glu His Ala Glu Gly Ser Val Asn Gly Thr Leu
        915                 920                 925
```

```
Met Gln Leu Gly Ala Leu Gly Gly Val Asn Val Pro Phe Ala Ala Thr
    930                 935                 940

Gly Asp Leu Thr Val Glu Gly Gly Leu Arg Tyr Asp Leu Leu Lys Gln
945                 950                 955                 960

Asp Ala Phe Ala Glu Lys Gly Ser Ala Leu Gly Trp Ser Gly Asn Ser
                965                 970                 975

Leu Thr Glu Gly Thr Leu Val Gly Leu Ala Gly Leu Lys Leu Ser Gln
            980                 985                 990

Pro Leu Ser Asp Lys Ala Val Leu Phe Ala Thr Ala Gly Val Glu Arg
        995                 1000                1005

Asp Leu Asn Gly Arg Asp Tyr Thr Val Thr Gly Gly Phe Thr Gly Ala
    1010                1015                1020

Thr Ala Ala Thr Gly Lys Thr Gly Ala Arg Asn Met Pro His Thr Arg
1025                1030                1035                1040

Leu Val Ala Gly Leu Gly Ala Asp Val Glu Phe Gly Asn Gly Trp Asn
                1045                1050                1055

Gly Leu Ala Arg Tyr Ser Tyr Ala Gly Ser Lys Gln Tyr Gly Asn His
            1060                1065                1070

Ser Gly Arg Val Gly Val Gly Tyr Arg Phe
        1075                1080
```

<210> 16
<211> 1047
<212> PRT
<213> Artificial Sequence

<220>
<223> deltaG983

<400> 16

Thr Ser Ala Pro Asp Phe Asn Ala Gly Gly Thr Gly Ile Gly Ser Asn
1 5 10 15

Ser Arg Ala Thr Thr Ala Lys Ser Ala Ala Val Ser Tyr Ala Gly Ile
20 25 30

Lys Asn Glu Met Cys Lys Asp Arg Ser Met Leu Cys Ala Gly Arg Asp
35 40 45

Asp Val Ala Val Thr Asp Arg Asp Ala Lys Ile Asn Ala Pro Pro Pro
50 55 60

Asn Leu His Thr Gly Asp Phe Pro Asn Pro Asn Asp Ala Tyr Lys Asn
65 70 75 80

Leu Ile Asn Leu Lys Pro Ala Ile Glu Ala Gly Tyr Thr Gly Arg Gly
85 90 95

Val Glu Val Gly Ile Val Asp Thr Gly Glu Ser Val Gly Ser Ile Ser
100 105 110

Phe Pro Glu Leu Tyr Gly Arg Lys Glu His Gly Tyr Asn Glu Asn Tyr

115 120 125

Lys Asn Tyr Thr Ala Tyr Met Arg Lys Glu Ala Pro Glu Asp Gly Gly
130 135 140

Gly Lys Asp Ile Glu Ala Ser Phe Asp Asp Glu Ala Val Ile Glu Thr
145 150 155 160

Glu Ala Lys Pro Thr Asp Ile Arg His Val Lys Glu Ile Gly His Ile
165 170 175

Asp Leu Val Ser His Ile Ile Gly Gly Arg Ser Val Asp Gly Arg Pro
180 185 190

Ala Gly Gly Ile Ala Pro Asp Ala Thr Leu His Ile Met Asn Thr Asn
195 200 205

Asp Glu Thr Lys Asn Glu Met Met Val Ala Ala Ile Arg Asn Ala Trp
210 215 220

Val Lys Leu Gly Glu Arg Gly Val Arg Ile Val Asn Asn Ser Phe Gly
225 230 235 240

Thr Thr Ser Arg Ala Gly Thr Ala Asp Leu Phe Gln Ile Ala Asn Ser
245 250 255

Glu Glu Gln Tyr Arg Gln Ala Leu Leu Asp Tyr Ser Gly Gly Asp Lys
260 265 270

Thr Asp Glu Gly Ile Arg Leu Met Gln Gln Ser Asp Tyr Gly Asn Leu
275 280 285

Ser Tyr His Ile Arg Asn Lys Asn Met Leu Phe Ile Phe Ser Thr Gly
290 295 300

Asn Asp Ala Gln Ala Gln Pro Asn Thr Tyr Ala Leu Leu Pro Phe Tyr
305 310 315 320

Glu Lys Asp Ala Gln Lys Gly Ile Ile Thr Val Ala Gly Val Asp Arg
325 330 335

Ser Gly Glu Lys Phe Lys Arg Glu Met Tyr Gly Glu Pro Gly Thr Glu
340 345 350

Pro Leu Glu Tyr Gly Ser Asn His Cys Gly Ile Thr Ala Met Trp Cys
355 360 365

Leu Ser Ala Pro Tyr Glu Ala Ser Val Arg Phe Thr Arg Thr Asn Pro
370 375 380

Ile Gln Ile Ala Gly Thr Ser Phe Ser Ala Pro Ile Val Thr Gly Thr
385 390 395 400

Ala Ala Leu Leu Leu Gln Lys Tyr Pro Trp Met Ser Asn Asp Asn Leu
405 410 415

Arg Thr Thr Leu Leu Thr Thr Ala Gln Asp Ile Gly Ala Val Gly Val
420 425 430

Asp Ser Lys Phe Gly Trp Gly Leu Leu Asp Ala Gly Lys Ala Met Asn
435 440 445

```
Gly Pro Ala Ser Phe Pro Phe Gly Asp Phe Thr Ala Asp Thr Lys Gly
    450                 455                 460

Thr Ser Asp Ile Ala Tyr Ser Phe Arg Asn Asp Ile Ser Gly Thr Gly
465                 470                 475                 480

Gly Leu Ile Lys Lys Gly Gly Ser Gln Leu Gln Leu His Gly Asn Asn
                485                 490                 495

Thr Tyr Thr Gly Lys Thr Ile Ile Glu Gly Gly Ser Leu Val Leu Tyr
            500                 505                 510

Gly Asn Asn Lys Ser Asp Met Arg Val Glu Thr Lys Gly Ala Leu Ile
        515                 520                 525

Tyr Asn Gly Ala Ala Ser Gly Gly Ser Leu Asn Ser Asp Gly Ile Val
    530                 535                 540

Tyr Leu Ala Asp Thr Asp Gln Ser Gly Ala Asn Glu Thr Val His Ile
545                 550                 555                 560

Lys Gly Ser Leu Gln Leu Asp Gly Lys Gly Thr Leu Tyr Thr Arg Leu
                565                 570                 575

Gly Lys Leu Leu Lys Val Asp Gly Thr Ala Ile Ile Gly Gly Lys Leu
            580                 585                 590

Tyr Met Ser Ala Arg Gly Lys Gly Ala Gly Tyr Leu Asn Ser Thr Gly
        595                 600                 605

Arg Arg Val Pro Phe Leu Ser Ala Ala Lys Ile Gly Gln Asp Tyr Ser
    610                 615                 620

Phe Phe Thr Asn Ile Glu Thr Asp Gly Gly Leu Leu Ala Ser Leu Asp
625                 630                 635                 640

Ser Val Glu Lys Thr Ala Gly Ser Glu Gly Asp Thr Leu Ser Tyr Tyr
                645                 650                 655

Val Arg Arg Gly Asn Ala Ala Arg Thr Ala Ser Ala Ala Ala His Ser
            660                 665                 670

Ala Pro Ala Gly Leu Lys His Ala Val Glu Gln Gly Gly Ser Asn Leu
        675                 680                 685

Glu Asn Leu Met Val Glu Leu Asp Ala Ser Glu Ser Ser Ala Thr Pro
    690                 695                 700

Glu Thr Val Glu Thr Ala Ala Ala Asp Arg Thr Asp Met Pro Gly Ile
705                 710                 715                 720

Arg Pro Tyr Gly Ala Thr Phe Arg Ala Ala Ala Ala Val Gln His Ala
                725                 730                 735

Asn Ala Ala Asp Gly Val Arg Ile Phe Asn Ser Leu Ala Ala Thr Val
            740                 745                 750

Tyr Ala Asp Ser Thr Ala Ala His Ala Asp Met Gln Gly Arg Arg Leu
        755                 760                 765
```

```
Lys Ala Val Ser Asp Gly Leu Asp His Asn Gly Thr Gly Leu Arg Val
    770                 775                 780

Ile Ala Gln Thr Gln Gln Asp Gly Gly Thr Trp Glu Gln Gly Gly Val
785                 790                 795                 800

Glu Gly Lys Met Arg Gly Ser Thr Gln Thr Val Gly Ile Ala Ala Lys
                805                 810                 815

Thr Gly Glu Asn Thr Thr Ala Ala Ala Thr Leu Gly Met Gly Arg Ser
            820                 825                 830

Thr Trp Ser Glu Asn Ser Ala Asn Ala Lys Thr Asp Ser Ile Ser Leu
        835                 840                 845

Phe Ala Gly Ile Arg His Asp Ala Gly Asp Ile Gly Tyr Leu Lys Gly
    850                 855                 860

Leu Phe Ser Tyr Gly Arg Tyr Lys Asn Ser Ile Ser Arg Ser Thr Gly
865                 870                 875                 880

Ala Asp Glu His Ala Glu Gly Ser Val Asn Gly Thr Leu Met Gln Leu
                885                 890                 895

Gly Ala Leu Gly Gly Val Asn Val Pro Phe Ala Ala Thr Gly Asp Leu
            900                 905                 910

Thr Val Glu Gly Gly Leu Arg Tyr Asp Leu Leu Lys Gln Asp Ala Phe
        915                 920                 925

Ala Glu Lys Gly Ser Ala Leu Gly Trp Ser Gly Asn Ser Leu Thr Glu
    930                 935                 940

Gly Thr Leu Val Gly Leu Ala Gly Leu Lys Leu Ser Gln Pro Leu Ser
945                 950                 955                 960

Asp Lys Ala Val Leu Phe Ala Thr Ala Gly Val Glu Arg Asp Leu Asn
                965                 970                 975

Gly Arg Asp Tyr Thr Val Thr Gly Gly Phe Thr Gly Ala Thr Ala Ala
            980                 985                 990

Thr Gly Lys Thr Gly Ala Arg Asn Met Pro His Thr Arg Leu Val Ala
        995                 1000                1005

Gly Leu Gly Ala Asp Val Glu Phe Gly Asn Gly Trp Asn Gly Leu Ala
    1010                1015                1020

Arg Tyr Ser Tyr Ala Gly Ser Lys Gln Tyr Gly Asn His Ser Gly Arg
1025                1030                1035                1040

Val Gly Val Gly Tyr Arg Phe
                1045
```

<210> 17
<211> 4425
<212> DNA
<213> Artificial Sequence

<220>
<223> deltaG983-ORF46.1

<400> 17

```
atgacttctg cgcccgactt caatgcaggc ggtaccggta tcggcagcaa cagcagagca   60
acaacagcga aatcagcagc agtatcttac gccggtatca agaacgaaat gtgcaaagac  120
agaagcatgc tctgtgccgg tcgggatgac gttgcggtta cagacaggga tgccaaaatc  180
aatgcccccc ccccgaatct gcataccgga gactttccaa acccaaatga cgcatacaag  240
aatttgatca acctcaaacc tgcaattgaa gcaggctata caggacgcgg ggtagaggta  300
ggtatcgtcg acacaggcga atccgtcggc agcatatcct ttcccgaact gtatggcaga  360
aaagaacacg gctataacga aaattacaaa aactatacgg cgtatatgcg gaaggaagcg  420
cctgaagacg gaggcggtaa agacattgaa gcttctttcg acgatgaggc cgttatagag  480
actgaagcaa agccgacgga tatccgccac gtaaaagaaa tcggacacat cgatttggtc  540
tcccatatta ttggcgggcg ttccgtggac ggcagacctg caggcggtat tgcgcccgat  600
gcgacgctac acataatgaa tacgaatgat gaaaccaaga acgaaatgat ggttgcagcc  660
atccgcaatg catgggtcaa gctgggcgaa cgtggcgtgc gcatcgtcaa taacagtttt  720
ggaacaacat cgagggcagg cactgccgac cttttccaaa tagccaattc ggaggagcag  780
taccgccaag cgttgctcga ctattccggc ggtgataaaa cagacgaggg tatccgcctg  840
atgcaacaga gcgattacgg caacctgtcc taccacatcc gtaataaaaa catgcttttc  900
atcttttcga caggcaatga cgcacaagct cagcccaaca catatgccct attgccattt  960
tatgaaaaag acgctcaaaa aggcattatc acagtcgcag gcgtagaccg cagtggagaa 1020
aagttcaaac gggaaatgta tggagaaccg ggtacagaac cgcttgagta tggctccaac 1080
cattgcggaa ttactgccat gtggtgcctg tcggcaccct atgaagcaag cgtccgtttc 1140
acccgtacaa acccgattca aattgccgga acatcctttt ccgcacccat cgtaaccggc 1200
acggcggctc tgctgctgca gaaatacccg tggatgagca acgacaacct gcgtaccacg 1260
ttgctgacga cggctcagga catcggtgca gtcggcgtgg acagcaagtt cggctgggga 1320
ctgctggatg cgggtaaggc catgaacgga cccgcgtcct ttccgttcgg cgactttacc 1380
gccgatacga aaggtacatc cgatattgcc tactccttcc gtaacgacat ttcaggcacg 1440
ggcggcctga tcaaaaaagg cggcagccaa ctgcaactgc acggcaacaa cacctatacg 1500
ggcaaaacca ttatcgaagg cggttcgctg gtgttgtacg gcaacaacaa atcggatatg 1560
cgcgtcgaaa ccaaaggtgc gctgatttat aacggggcgg catccggcgg cagcctgaac 1620
agcgacggca ttgtctatct ggcagatacc gaccaatccg gcgcaaacga aaccgtacac 1680
atcaaaggca gtctgcagct ggacggcaaa ggtacgctgt acacacgttt gggcaaactg 1740
ctgaaagtgg acggtacggc gattatcggc ggcaagctgt acatgtcggc acgcggcaag 1800
gggggcaggct atctcaacag taccggacga cgtgttccct tcctgagtgc cgccaaaatc 1860
gggcaggatt attctttctt cacaaacatc gaaaccgacg gcggcctgct ggcttccctc 1920
gacagcgtcg aaaaaacagc gggcagtgaa ggcgacacgc tgtcctatta tgtccgtcgc 1980
ggcaatgcgg cacggactgc ttcggcagcg gcacattccg cgcccgccgg tctgaaacac 2040
gccgtagaac agggcggcag caatctggaa aacctgatgg tcgaactgga tgcctccgaa 2100
tcatccgcaa cacccgagac ggttgaaact gcggcagccg accgcacaga tatgccgggc 2160
atccgcccct acggcgcaac tttccgcgca gcggcagccg tacagcatgc gaatgccgcc 2220
gacggtgtac gcatcttcaa cagtctcgcc gctaccgtct atgccgacag taccgccgcc 2280
catgccgata tgcagggacg ccgcctgaaa gccgtatcgg acgggttgga ccacaacggc 2340
acgggtctgc gcgtcatcgc gcaaacccaa caggacggtg gaacgtggga acagggcggt 2400
gttgaaggca aaatgcgcgg cagtacccaa accgtcggca ttgccgcgaa aaccggcgaa 2460
aatacgacag cagccgccac actgggcatg ggacgcagca catggagcga aaacagtgca 2520
aatgcaaaaa ccgacagcat tagtctgttt gcaggcatac ggcacgatgc gggcgatatc 2580
ggctatctca aaggcctgtt ctcctacgga cgctacaaaa acagcatcag ccgcagcacc 2640
ggtgcggacg aacatgcgga aggcagcgtc aacggcacgc tgatgcagct gggcgcactg 2700
ggcggtgtca acgttccgtt tgccgcaacg ggagatttga cggtcgaagg cggtctgcgc 2760
tacgacctgc tcaaacagga tgcattcgcc gaaaaaggca gtgctttggg ctggagcggc 2820
aacagcctca ctgaaggcac gctggtcgga ctcgcgggtc tgaagctgtc gcaacccttg 2880
agcgataaag ccgtcctgtt tgcaacggcg ggcgtggaac gcgacctgaa cggacgcgac 2940
tacacggtaa cgggcggctt taccggcgcg actgcagcaa ccggcaagac gggggcacgc 3000
aatatgccgc acacccgtct ggttgccggc ctgggcgcgg atgtcgaatt cggcaacggc 3060
tggaacggct tggcacgtta cagctacgcc ggttccaaac agtacggcaa ccacagcgga 3120
cgagtcggcg taggctaccg gttcctcgac ggtggcggag gcactggatc ctcagatttg 3180
gcaaacgatt cttttatccg gcaggttctc gaccgtcagc atttcgaacc cgacgggaaa 3240
taccacctat tcggcagcag gggggaactt gccgagcgca gcggccatat cggattggga 3300
aaaatacaaa gccatcagtt gggcaacctg atgattcaac aggcggccat taaaggaaat 3360
atcggctaca ttgtccgctt ttccgatcac gggcacgaag tccattcccc cttcgacaac 3420
catgcctcac attccgattc tgatgaagcc ggtagtcccg ttgacggatt tagcctttac 3480
cgcatccatt gggacggata cgaacaccat cccgccgacg gctatgacgg gccacagggc 3540
```

```
ggcggctatc ccgctcccaa aggcgcgagg gatatataca gctacgacat aaaaggcgtt   3600
gcccaaaata tccgcctcaa cctgaccgac aaccgcagca ccggacaacg gcttgccgac   3660
cgtttccaca atgccggtag tatgctgacg caaggagtag gcgacggatt caaacgcgcc   3720
acccgataca gccccgagct ggacagatcg ggcaatgccg ccgaagcctt caacggcact   3780
gcagatatcg ttaaaaacat catcggcgcg gcaggagaaa ttgtcggcgc aggcgatgcc   3840
gtgcagggca taagcgaagg ctcaaacatt gctgtcatgc acggcttggg tctgctttcc   3900
accgaaaaca agatggcgcg catcaacgat ttggcagata tggcgcaact caaagactat   3960
gccgcagcag ccatccgcga ttgggcagtc caaaacccca atgccgcaca aggcatagaa   4020
gccgtcagca atatctttat ggcagccatc cccatcaaag ggattggagc tgttcgggga   4080
aaatacggct tgggcggcat cacggcacat cctatcaagc ggtcgcagat gggcgcgatc   4140
gcattgccga aagggaaatc cgccgtcagc gacaattttg ccgatgcggc atacgccaaa   4200
tacccgtccc cttaccattc ccgaaatatc cgttcaaact tggagcagcg ttacggcaaa   4260
gaaaacatca cctcctcaac cgtgccgccg tcaaacggca aaaatgtcaa actggcagac   4320
caacgccacc cgaagacagg cgtaccgttt gacggtaaag ggtttccgaa ttttgagaag   4380
cacgtgaaat atgatacgct cgagcaccac caccaccacc actga                   4425
```

<210> 18
<211> 1474
<212> PRT
<213> Artificial Sequence

<220>
<223> deltaG983-ORF46.1

<400> 18

```
Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly
            20                  25                  30

Ile Lys Asn Glu Met Cys Lys Asp Arg Ser Met Leu Cys Ala Gly Arg
        35                  40                  45

Asp Asp Val Ala Val Thr Asp Arg Asp Ala Lys Ile Asn Ala Pro Pro
    50                  55                  60

Pro Asn Leu His Thr Gly Asp Phe Pro Asn Pro Asn Asp Ala Tyr Lys
65                  70                  75                  80

Asn Leu Ile Asn Leu Lys Pro Ala Ile Glu Ala Gly Tyr Thr Gly Arg
                85                  90                  95

Gly Val Glu Val Gly Ile Val Asp Thr Gly Glu Ser Val Gly Ser Ile
            100                 105                 110

Ser Phe Pro Glu Leu Tyr Gly Arg Lys Glu His Gly Tyr Asn Glu Asn
        115                 120                 125

Tyr Lys Asn Tyr Thr Ala Tyr Met Arg Lys Glu Ala Pro Glu Asp Gly
    130                 135                 140

Gly Gly Lys Asp Ile Glu Ala Ser Phe Asp Asp Glu Ala Val Ile Glu
145                 150                 155                 160

Thr Glu Ala Lys Pro Thr Asp Ile Arg His Val Lys Glu Ile Gly His
                165                 170                 175

Ile Asp Leu Val Ser His Ile Ile Gly Gly Arg Ser Val Asp Gly Arg
            180                 185                 190
```

```
Pro Ala Gly Gly Ile Ala Pro Asp Ala Thr Leu His Ile Met Asn Thr
        195                 200                 205

Asn Asp Glu Thr Lys Asn Glu Met Met Val Ala Ala Ile Arg Asn Ala
    210                 215                 220

Trp Val Lys Leu Gly Glu Arg Gly Val Arg Ile Val Asn Asn Ser Phe
225                 230                 235                 240

Gly Thr Thr Ser Arg Ala Gly Thr Ala Asp Leu Phe Gln Ile Ala Asn
                245                 250                 255

Ser Glu Glu Gln Tyr Arg Gln Ala Leu Leu Asp Tyr Ser Gly Gly Asp
            260                 265                 270

Lys Thr Asp Glu Gly Ile Arg Leu Met Gln Gln Ser Asp Tyr Gly Asn
        275                 280                 285

Leu Ser Tyr His Ile Arg Asn Lys Asn Met Leu Phe Ile Phe Ser Thr
    290                 295                 300

Gly Asn Asp Ala Gln Ala Gln Pro Asn Thr Tyr Ala Leu Leu Pro Phe
305                 310                 315                 320

Tyr Glu Lys Asp Ala Gln Lys Gly Ile Ile Thr Val Ala Gly Val Asp
                325                 330                 335

Arg Ser Gly Glu Lys Phe Lys Arg Glu Met Tyr Gly Glu Pro Gly Thr
            340                 345                 350

Glu Pro Leu Glu Tyr Gly Ser Asn His Cys Gly Ile Thr Ala Met Trp
        355                 360                 365

Cys Leu Ser Ala Pro Tyr Glu Ala Ser Val Arg Phe Thr Arg Thr Asn
    370                 375                 380

Pro Ile Gln Ile Ala Gly Thr Ser Phe Ser Ala Pro Ile Val Thr Gly
385                 390                 395                 400

Thr Ala Ala Leu Leu Leu Gln Lys Tyr Pro Trp Met Ser Asn Asp Asn
                405                 410                 415

Leu Arg Thr Thr Leu Leu Thr Thr Ala Gln Asp Ile Gly Ala Val Gly
            420                 425                 430

Val Asp Ser Lys Phe Gly Trp Gly Leu Leu Asp Ala Gly Lys Ala Met
        435                 440                 445

Asn Gly Pro Ala Ser Phe Pro Phe Gly Asp Phe Thr Ala Asp Thr Lys
    450                 455                 460

Gly Thr Ser Asp Ile Ala Tyr Ser Phe Arg Asn Asp Ile Ser Gly Thr
465                 470                 475                 480

Gly Gly Leu Ile Lys Lys Gly Gly Ser Gln Leu Gln Leu His Gly Asn
                485                 490                 495

Asn Thr Tyr Thr Gly Lys Thr Ile Ile Glu Gly Gly Ser Leu Val Leu
            500                 505                 510

Tyr Gly Asn Asn Lys Ser Asp Met Arg Val Glu Thr Lys Gly Ala Leu
```

515 520 525

Ile Tyr Asn Gly Ala Ala Ser Gly Gly Ser Leu Asn Ser Asp Gly Ile
530 535 540

Val Tyr Leu Ala Asp Thr Asp Gln Ser Gly Ala Asn Glu Thr Val His
545 550 555 560

Ile Lys Gly Ser Leu Gln Leu Asp Gly Lys Gly Thr Leu Tyr Thr Arg
565 570 575

Leu Gly Lys Leu Leu Lys Val Asp Gly Thr Ala Ile Ile Gly Gly Lys
580 585 590

Leu Tyr Met Ser Ala Arg Gly Lys Gly Ala Gly Tyr Leu Asn Ser Thr
595 600 605

Gly Arg Arg Val Pro Phe Leu Ser Ala Ala Lys Ile Gly Gln Asp Tyr
610 615 620

Ser Phe Phe Thr Asn Ile Glu Thr Asp Gly Gly Leu Leu Ala Ser Leu
625 630 635 640

Asp Ser Val Glu Lys Thr Ala Gly Ser Glu Gly Asp Thr Leu Ser Tyr
645 650 655

Tyr Val Arg Arg Gly Asn Ala Ala Arg Thr Ala Ser Ala Ala Ala His
660 665 670

Ser Ala Pro Ala Gly Leu Lys His Ala Val Glu Gln Gly Gly Ser Asn
675 680 685

Leu Glu Asn Leu Met Val Glu Leu Asp Ala Ser Glu Ser Ser Ala Thr
690 695 700

Pro Glu Thr Val Glu Thr Ala Ala Ala Asp Arg Thr Asp Met Pro Gly
705 710 715 720

Ile Arg Pro Tyr Gly Ala Thr Phe Arg Ala Ala Ala Ala Val Gln His
725 730 735

Ala Asn Ala Ala Asp Gly Val Arg Ile Phe Asn Ser Leu Ala Ala Thr
740 745 750

Val Tyr Ala Asp Ser Thr Ala Ala His Ala Asp Met Gln Gly Arg Arg
755 760 765

Leu Lys Ala Val Ser Asp Gly Leu Asp His Asn Gly Thr Gly Leu Arg
770 775 780

Val Ile Ala Gln Thr Gln Gln Asp Gly Gly Thr Trp Glu Gln Gly Gly
785 790 795 800

Val Glu Gly Lys Met Arg Gly Ser Thr Gln Thr Val Gly Ile Ala Ala
805 810 815

Lys Thr Gly Glu Asn Thr Thr Ala Ala Ala Thr Leu Gly Met Gly Arg
820 825 830

Ser Thr Trp Ser Glu Asn Ser Ala Asn Ala Lys Thr Asp Ser Ile Ser
835 840 845

```
Leu Phe Ala Gly Ile Arg His Asp Ala Gly Asp Ile Gly Tyr Leu Lys
    850                 855                 860

Gly Leu Phe Ser Tyr Gly Arg Tyr Lys Asn Ser Ile Ser Arg Ser Thr
865                 870                 875                 880

Gly Ala Asp Glu His Ala Glu Gly Ser Val Asn Gly Thr Leu Met Gln
                885                 890                 895

Leu Gly Ala Leu Gly Gly Val Asn Val Pro Phe Ala Ala Thr Gly Asp
            900                 905                 910

Leu Thr Val Glu Gly Gly Leu Arg Tyr Asp Leu Leu Lys Gln Asp Ala
        915                 920                 925

Phe Ala Glu Lys Gly Ser Ala Leu Gly Trp Ser Gly Asn Ser Leu Thr
    930                 935                 940

Glu Gly Thr Leu Val Gly Leu Ala Gly Leu Lys Leu Ser Gln Pro Leu
945                 950                 955                 960

Ser Asp Lys Ala Val Leu Phe Ala Thr Ala Gly Val Glu Arg Asp Leu
                965                 970                 975

Asn Gly Arg Asp Tyr Thr Val Thr Gly Gly Phe Thr Gly Ala Thr Ala
            980                 985                 990

Ala Thr Gly Lys Thr Gly Ala Arg Asn Met Pro His Thr Arg Leu Val
        995                 1000                1005

Ala Gly Leu Gly Ala Asp Val Glu Phe Gly Asn Gly Trp Asn Gly Leu
    1010                1015                1020

Ala Arg Tyr Ser Tyr Ala Gly Ser Lys Gln Tyr Gly Asn His Ser Gly
1025                1030                1035                1040

Arg Val Gly Val Gly Tyr Arg Phe Leu Asp Gly Gly Gly Gly Thr Gly
                1045                1050                1055

Ser Ser Asp Leu Ala Asn Asp Ser Phe Ile Arg Gln Val Leu Asp Arg
            1060                1065                1070

Gln His Phe Glu Pro Asp Gly Lys Tyr His Leu Phe Gly Ser Arg Gly
        1075                1080                1085

Glu Leu Ala Glu Arg Ser Gly His Ile Gly Leu Gly Lys Ile Gln Ser
    1090                1095                1100

His Gln Leu Gly Asn Leu Met Ile Gln Gln Ala Ala Ile Lys Gly Asn
1105                1110                1115                1120

Ile Gly Tyr Ile Val Arg Phe Ser Asp His Gly His Glu Val His Ser
                1125                1130                1135

Pro Phe Asp Asn His Ala Ser His Ser Asp Ser Asp Glu Ala Gly Ser
            1140                1145                1150

Pro Val Asp Gly Phe Ser Leu Tyr Arg Ile His Trp Asp Gly Tyr Glu
        1155                1160                1165
```

His His Pro Ala Asp Gly Tyr Asp Gly Pro Gln Gly Gly Gly Tyr Pro
1170 1175 1180

Ala Pro Lys Gly Ala Arg Asp Ile Tyr Ser Tyr Asp Ile Lys Gly Val
1185 1190 1195 1200

Ala Gln Asn Ile Arg Leu Asn Leu Thr Asp Asn Arg Ser Thr Gly Gln
1205 1210 1215

Arg Leu Ala Asp Arg Phe His Asn Ala Gly Ser Met Leu Thr Gln Gly
1220 1225 1230

Val Gly Asp Gly Phe Lys Arg Ala Thr Arg Tyr Ser Pro Glu Leu Asp
1235 1240 1245

Arg Ser Gly Asn Ala Ala Glu Ala Phe Asn Gly Thr Ala Asp Ile Val
1250 1255 1260

Lys Asn Ile Ile Gly Ala Ala Gly Glu Ile Val Gly Ala Gly Asp Ala
1265 1270 1275 1280

Val Gln Gly Ile Ser Glu Gly Ser Asn Ile Ala Val Met His Gly Leu
1285 1290 1295

Gly Leu Leu Ser Thr Glu Asn Lys Met Ala Arg Ile Asn Asp Leu Ala
1300 1305 1310

Asp Met Ala Gln Leu Lys Asp Tyr Ala Ala Ala Ala Ile Arg Asp Trp
1315 1320 1325

Ala Val Gln Asn Pro Asn Ala Ala Gln Gly Ile Glu Ala Val Ser Asn
1330 1335 1340

Ile Phe Met Ala Ala Ile Pro Ile Lys Gly Ile Gly Ala Val Arg Gly
1345 1350 1355 1360

Lys Tyr Gly Leu Gly Gly Ile Thr Ala His Pro Ile Lys Arg Ser Gln
1365 1370 1375

Met Gly Ala Ile Ala Leu Pro Lys Gly Lys Ser Ala Val Ser Asp Asn
1380 1385 1390

Phe Ala Asp Ala Ala Tyr Ala Lys Tyr Pro Ser Pro Tyr His Ser Arg
1395 1400 1405

Asn Ile Arg Ser Asn Leu Glu Gln Arg Tyr Gly Lys Glu Asn Ile Thr
1410 1415 1420

Ser Ser Thr Val Pro Pro Ser Asn Gly Lys Asn Val Lys Leu Ala Asp
1425 1430 1435 1440

Gln Arg His Pro Lys Thr Gly Val Pro Phe Asp Gly Lys Gly Phe Pro
1445 1450 1455

Asn Phe Glu Lys His Val Lys Tyr Asp Thr Leu Glu His His His His
1460 1465 1470

His His

<210> 19
<211> 3939
<212> DNA
<213> Artificial Sequence

<220>
<223> deltaG983-741

<400> 19

```
atgacttctg cgcccgactt caatgcaggc ggtaccggta tcggcagcaa cagcagagca   60
acaacagcga aatcagcagc agtatcttac gccggtatca agaacgaaat gtgcaaagac  120
agaagcatgc tctgtgccgg tcgggatgac gttgcggtta cagacaggga tgccaaaatc  180
aatgcccccc ccccgaatct gcataccgga gactttccaa acccaaatga cgcatacaag  240
aatttgatca acctcaaacc tgcaattgaa gcaggctata caggacgcgg ggtagaggta  300
ggtatcgtcg acacaggcga atccgtcggc agcatatcct ttcccgaact gtatggcaga  360
aaagaacacg gctataacga aaattacaaa aactatacgg cgtatatgcg gaaggaagcg  420
cctgaagacg gaggcggtaa agacattgaa gcttctttcg acgatgaggc cgttatagag  480
actgaagcaa agccgacgga tatccgccac gtaaaagaaa tcggacacat cgatttggtc  540
tcccatatta ttggcgggcg ttccgtggac ggcagacctg caggcggtat tgcgcccgat  600
gcgacgctac acataatgaa tacgaatgat gaaaccaaga acgaaatgat ggttgcagcc  660
atccgcaatg catgggtcaa gctgggcgaa cgtggcgtgc gcatcgtcaa taacagtttt  720
ggaacaacat cgagggcagg cactgccgac cttttccaaa tagccaattc ggaggagcag  780
taccgccaag cgttgctcga ctattccggc ggtgataaaa cagacgaggg tatccgcctg  840
atgcaacaga gcgattacgg caacctgtcc taccacatcc gtaataaaaa catgcttttc  900
atcttttcga caggcaatga cgcacaagct cagcccaaca catatgccct attgccattt  960
tatgaaaaag acgctcaaaa aggcattatc acagtcgcag gcgtagaccg cagtggagaa 1020
aagttcaaac gggaaatgta tggagaaccg ggtacagaac cgcttgagta tggctccaac 1080
cattgcggaa ttactgccat gtggtgcctg tcggcaccct atgaagcaag cgtccgtttc 1140
acccgtacaa acccgattca aattgccgga acatcctttt ccgcacccat cgtaaccggc 1200
acggcggctc tgctgctgca gaaatacccg tggatgagca acgacaacct gcgtaccacg 1260
ttgctgacga cggctcagga catcggtgca gtcggcgtgg acagcaagtt cggctgggga 1320
ctgctggatg cgggtaaggc catgaacgga cccgcgtcct ttccgttcgg cgactttacc 1380
gccgatacga aaggtacatc cgatattgcc tactccttcc gtaacgacat ttcaggcacg 1440
ggcggcctga tcaaaaaagg cggcagccaa ctgcaactgc acggcaacaa cacctatacg 1500
ggcaaaacca ttatcgaagg cggttcgctg gtgttgtacg gcaacaacaa atcggatatg 1560
cgcgtcgaaa ccaaaggtgc gctgatttat aacggggcgg catccggcgg cagcctgaac 1620
agcgacggca ttgtctatct ggcagatacc gaccaatccg gcgcaaacga aaccgtacac 1680
atcaaaggca gtctgcagct ggacggcaaa ggtacgctgt acacacgttt gggcaaactg 1740
ctgaaagtgg acggtacggc gattatcggc ggcaagctgt acatgtcggc acgcggcaag 1800
ggggcaggct atctcaacag taccggacga cgtgttccct tcctgagtgc cgccaaaatc 1860
gggcaggatt attctttctt cacaaacatc gaaaccgacg gcggcctgct ggcttccctc 1920
gacagcgtcg aaaaaacagc gggcagtgaa ggcgacacgc tgtcctatta tgtccgtcgc 1980
ggcaatgcgg cacggactgc ttcggcagcg gcacattccg cgcccgccgg tctgaaacac 2040
gccgtagaac agggcggcag caatctggaa aacctgatgg tcgaactgga tgcctccgaa 2100
tcatccgcaa cacccgagac ggttgaaact gcggcagccg accgcacaga tatgccgggc 2160
atccgcccct acggcgcaac tttccgcgca gcggcagccg tacagcatgc gaatgccgcc 2220
gacggtgtac gcatcttcaa cagtctcgcc gctaccgtct atgccgacag taccgccgcc 2280
catgccgata tgcagggacg ccgcctgaaa gccgtatcgg acgggttgga ccacaacggc 2340
acgggtctgc gcgtcatcgc gcaaacccaa caggacggtg gaacgtggga acagggcggt 2400
gttgaaggca aaatgcgcgg cagtacccaa accgtcggca ttgccgcgaa aaccggcgaa 2460
aatacgacag cagccgccac actgggcatg ggacgcagca catggagcga aaacagtgca 2520
aatgcaaaaa ccgacagcat tagtctgttt gcaggcatac ggcacgatgc gggcgatatc 2580
ggctatctca aaggcctgtt ctcctacgga cgctacaaaa acagcatcag ccgcagcacc 2640
ggtgcggacg aacatgcgga aggcagcgtc aacggcacgc tgatgcagct gggcgcactg 2700
ggcggtgtca acgttccgtt tgccgcaacg ggagatttga cggtcgaagg cggtctgcgc 2760
tacgacctgc tcaaacagga tgcattcgcc gaaaaaggca gtgctttggg ctggagcggc 2820
aacagcctca ctgaaggcac gctggtcgga ctcgcgggtc tgaagctgtc gcaacccttg 2880
agcgataaag ccgtcctgtt tgcaacggcg ggcgtggaac gcgacctgaa cggacgcgac 2940
tacacggtaa cgggcggctt taccggcgcg actgcagcaa ccggcaagac gggggcacgc 3000
aatatgccgc acacccgtct ggttgccggc ctgggcgcgg atgtcgaatt cggcaacggc 3060
tggaacggct tggcacgtta cagctacgcc ggttccaaac agtacggcaa ccacagcgga 3120
cgagtcggcg taggctaccg gttcctcgag ggatccggag ggggtggtgt cgccgccgac 3180
```

```
atcggtgcgg ggcttgccga tgcactaacc gcaccgctcg accataaaga caaaggtttg   3240
cagtctttga cgctggatca gtccgtcagg aaaaacgaga aactgaagct ggcggcacaa   3300
ggtgcggaaa aaacttatgg aaacggtgac agcctcaata cgggcaaatt gaagaacgac   3360
aaggtcagcc gtttcgactt tatccgccaa atcgaagtgg acgggcagct cattaccttg   3420
gagagtggag agttccaagt atacaaacaa agccattccg ccttaaccgc ctttcagacc   3480
gagcaaatac aagattcgga gcattccggg aagatggttg cgaaacgcca gttcagaatc   3540
ggcgacatag cgggcgaaca tacatctttt gacaagcttc ccgaaggcgg cagggcgaca   3600
tatcgcggga cggcgttcgg ttcagacgat gccggcggaa aactgaccta caccatagat   3660
ttcgccgcca agcagggaaa cggcaaaatc gaacatttga aatcgccaga actcaatgtc   3720
gacctggccg ccgccgatat caagccggat ggaaaacgcc atgccgtcat cagcggttcc   3780
gtcctttaca accaagccga gaaaggcagt tactccctcg gtatctttgg cggaaaagcc   3840
caggaagttg ccggcagcgc ggaagtgaaa accgtaaacg gcatacgcca tatcggcctt   3900
gccgccaagc aactcgagca ccaccaccac caccactga                          3939
```

<210> 20
<211> 1312
<212> PRT
<213> Artificial Sequence

<220>
<223> deltaG983-741

<400> 20

```
Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly
            20                  25                  30

Ile Lys Asn Glu Met Cys Lys Asp Arg Ser Met Leu Cys Ala Gly Arg
        35                  40                  45

Asp Asp Val Ala Val Thr Asp Arg Asp Ala Lys Ile Asn Ala Pro Pro
    50                  55                  60

Pro Asn Leu His Thr Gly Asp Phe Pro Asn Pro Asn Asp Ala Tyr Lys
65                  70                  75                  80

Asn Leu Ile Asn Leu Lys Pro Ala Ile Glu Ala Gly Tyr Thr Gly Arg
                85                  90                  95

Gly Val Glu Val Gly Ile Val Asp Thr Gly Glu Ser Val Gly Ser Ile
            100                 105                 110

Ser Phe Pro Glu Leu Tyr Gly Arg Lys Glu His Gly Tyr Asn Glu Asn
        115                 120                 125

Tyr Lys Asn Tyr Thr Ala Tyr Met Arg Lys Glu Ala Pro Glu Asp Gly
    130                 135                 140

Gly Gly Lys Asp Ile Glu Ala Ser Phe Asp Asp Glu Ala Val Ile Glu
145                 150                 155                 160

Thr Glu Ala Lys Pro Thr Asp Ile Arg His Val Lys Glu Ile Gly His
                165                 170                 175

Ile Asp Leu Val Ser His Ile Ile Gly Gly Arg Ser Val Asp Gly Arg
            180                 185                 190

Pro Ala Gly Gly Ile Ala Pro Asp Ala Thr Leu His Ile Met Asn Thr
        195                 200                 205
```

```
Asn Asp Glu Thr Lys Asn Glu Met Met Val Ala Ala Ile Arg Asn Ala
    210                 215                 220

Trp Val Lys Leu Gly Glu Arg Gly Val Arg Ile Val Asn Asn Ser Phe
225                 230                 235                 240

Gly Thr Thr Ser Arg Ala Gly Thr Ala Asp Leu Phe Gln Ile Ala Asn
                245                 250                 255

Ser Glu Glu Gln Tyr Arg Gln Ala Leu Leu Asp Tyr Ser Gly Gly Asp
            260                 265                 270

Lys Thr Asp Glu Gly Ile Arg Leu Met Gln Gln Ser Asp Tyr Gly Asn
        275                 280                 285

Leu Ser Tyr His Ile Arg Asn Lys Asn Met Leu Phe Ile Phe Ser Thr
    290                 295                 300

Gly Asn Asp Ala Gln Ala Gln Pro Asn Thr Tyr Ala Leu Leu Pro Phe
305                 310                 315                 320

Tyr Glu Lys Asp Ala Gln Lys Gly Ile Ile Thr Val Ala Gly Val Asp
                325                 330                 335

Arg Ser Gly Glu Lys Phe Lys Arg Glu Met Tyr Gly Glu Pro Gly Thr
            340                 345                 350

Glu Pro Leu Glu Tyr Gly Ser Asn His Cys Gly Ile Thr Ala Met Trp
        355                 360                 365

Cys Leu Ser Ala Pro Tyr Glu Ala Ser Val Arg Phe Thr Arg Thr Asn
    370                 375                 380

Pro Ile Gln Ile Ala Gly Thr Ser Phe Ser Ala Pro Ile Val Thr Gly
385                 390                 395                 400

Thr Ala Ala Leu Leu Leu Gln Lys Tyr Pro Trp Met Ser Asn Asp Asn
                405                 410                 415

Leu Arg Thr Thr Leu Leu Thr Thr Ala Gln Asp Ile Gly Ala Val Gly
            420                 425                 430

Val Asp Ser Lys Phe Gly Trp Gly Leu Leu Asp Ala Gly Lys Ala Met
        435                 440                 445

Asn Gly Pro Ala Ser Phe Pro Phe Gly Asp Phe Thr Ala Asp Thr Lys
    450                 455                 460

Gly Thr Ser Asp Ile Ala Tyr Ser Phe Arg Asn Asp Ile Ser Gly Thr
465                 470                 475                 480

Gly Gly Leu Ile Lys Lys Gly Gly Ser Gln Leu Gln Leu His Gly Asn
                485                 490                 495

Asn Thr Tyr Thr Gly Lys Thr Ile Ile Glu Gly Gly Ser Leu Val Leu
            500                 505                 510

Tyr Gly Asn Asn Lys Ser Asp Met Arg Val Glu Thr Lys Gly Ala Leu
        515                 520                 525
```

Ile Tyr Asn Gly Ala Ala Ser Gly Gly Ser Leu Asn Ser Asp Gly Ile
530 535 540

Val Tyr Leu Ala Asp Thr Asp Gln Ser Gly Ala Asn Glu Thr Val His
545 550 555 560

Ile Lys Gly Ser Leu Gln Leu Asp Gly Lys Gly Thr Leu Tyr Thr Arg
565 570 575

Leu Gly Lys Leu Leu Lys Val Asp Gly Thr Ala Ile Ile Gly Gly Lys
580 585 590

Leu Tyr Met Ser Ala Arg Gly Lys Gly Ala Gly Tyr Leu Asn Ser Thr
595 600 605

Gly Arg Arg Val Pro Phe Leu Ser Ala Ala Lys Ile Gly Gln Asp Tyr
610 615 620

Ser Phe Phe Thr Asn Ile Glu Thr Asp Gly Gly Leu Leu Ala Ser Leu
625 630 635 640

Asp Ser Val Glu Lys Thr Ala Gly Ser Glu Gly Asp Thr Leu Ser Tyr
645 650 655

Tyr Val Arg Arg Gly Asn Ala Ala Arg Thr Ala Ser Ala Ala Ala His
660 665 670

Ser Ala Pro Ala Gly Leu Lys His Ala Val Glu Gln Gly Gly Ser Asn
675 680 685

Leu Glu Asn Leu Met Val Glu Leu Asp Ala Ser Glu Ser Ser Ala Thr
690 695 700

Pro Glu Thr Val Glu Thr Ala Ala Ala Asp Arg Thr Asp Met Pro Gly
705 710 715 720

Ile Arg Pro Tyr Gly Ala Thr Phe Arg Ala Ala Ala Ala Val Gln His
725 730 735

Ala Asn Ala Ala Asp Gly Val Arg Ile Phe Asn Ser Leu Ala Ala Thr
740 745 750

Val Tyr Ala Asp Ser Thr Ala Ala His Ala Asp Met Gln Gly Arg Arg
755 760 765

Leu Lys Ala Val Ser Asp Gly Leu Asp His Asn Gly Thr Gly Leu Arg
770 775 780

Val Ile Ala Gln Thr Gln Gln Asp Gly Gly Thr Trp Glu Gln Gly Gly
785 790 795 800

Val Glu Gly Lys Met Arg Gly Ser Thr Gln Thr Val Gly Ile Ala Ala
805 810 815

Lys Thr Gly Glu Asn Thr Thr Ala Ala Ala Thr Leu Gly Met Gly Arg
820 825 830

Ser Thr Trp Ser Glu Asn Ser Ala Asn Ala Lys Thr Asp Ser Ile Ser
835 840 845

Leu Phe Ala Gly Ile Arg His Asp Ala Gly Asp Ile Gly Tyr Leu Lys

850 855 860

Gly Leu Phe Ser Tyr Gly Arg Tyr Lys Asn Ser Ile Ser Arg Ser Thr
865 870 875 880

Gly Ala Asp Glu His Ala Glu Gly Ser Val Asn Gly Thr Leu Met Gln
885 890 895

Leu Gly Ala Leu Gly Gly Val Asn Val Pro Phe Ala Ala Thr Gly Asp
900 905 910

Leu Thr Val Glu Gly Gly Leu Arg Tyr Asp Leu Leu Lys Gln Asp Ala
915 920 925

Phe Ala Glu Lys Gly Ser Ala Leu Gly Trp Ser Gly Asn Ser Leu Thr
930 935 940

Glu Gly Thr Leu Val Gly Leu Ala Gly Leu Lys Leu Ser Gln Pro Leu
945 950 955 960

Ser Asp Lys Ala Val Leu Phe Ala Thr Ala Gly Val Glu Arg Asp Leu
965 970 975

Asn Gly Arg Asp Tyr Thr Val Thr Gly Gly Phe Thr Gly Ala Thr Ala
980 985 990

Ala Thr Gly Lys Thr Gly Ala Arg Asn Met Pro His Thr Arg Leu Val
995 1000 1005

Ala Gly Leu Gly Ala Asp Val Glu Phe Gly Asn Gly Trp Asn Gly Leu
1010 1015 1020

Ala Arg Tyr Ser Tyr Ala Gly Ser Lys Gln Tyr Gly Asn His Ser Gly
1025 1030 1035 1040

Arg Val Gly Val Gly Tyr Arg Phe Leu Glu Gly Ser Gly Gly Gly Gly
1045 1050 1055

Val Ala Ala Asp Ile Gly Ala Gly Leu Ala Asp Ala Leu Thr Ala Pro
1060 1065 1070

Leu Asp His Lys Asp Lys Gly Leu Gln Ser Leu Thr Leu Asp Gln Ser
1075 1080 1085

Val Arg Lys Asn Glu Lys Leu Lys Leu Ala Ala Gln Gly Ala Glu Lys
1090 1095 1100

Thr Tyr Gly Asn Gly Asp Ser Leu Asn Thr Gly Lys Leu Lys Asn Asp
1105 1110 1115 1120

Lys Val Ser Arg Phe Asp Phe Ile Arg Gln Ile Glu Val Asp Gly Gln
1125 1130 1135

Leu Ile Thr Leu Glu Ser Gly Glu Phe Gln Val Tyr Lys Gln Ser His
1140 1145 1150

Ser Ala Leu Thr Ala Phe Gln Thr Glu Gln Ile Gln Asp Ser Glu His
1155 1160 1165

Ser Gly Lys Met Val Ala Lys Arg Gln Phe Arg Ile Gly Asp Ile Ala
1170 1175 1180

```
Gly Glu His Thr Ser Phe Asp Lys Leu Pro Glu Gly Gly Arg Ala Thr
1185                1190                1195                1200

Tyr Arg Gly Thr Ala Phe Gly Ser Asp Asp Ala Gly Gly Lys Leu Thr
                1205                1210                1215

Tyr Thr Ile Asp Phe Ala Ala Lys Gln Gly Asn Gly Lys Ile Glu His
            1220                1225                1230

Leu Lys Ser Pro Glu Leu Asn Val Asp Leu Ala Ala Ala Asp Ile Lys
        1235                1240                1245

Pro Asp Gly Lys Arg His Ala Val Ile Ser Gly Ser Val Leu Tyr Asn
    1250                1255                1260

Gln Ala Glu Lys Gly Ser Tyr Ser Leu Gly Ile Phe Gly Gly Lys Ala
1265                1270                1275                1280

Gln Glu Val Ala Gly Ser Ala Glu Val Lys Thr Val Asn Gly Ile Arg
                1285                1290                1295

His Ile Gly Leu Ala Ala Lys Gln Leu Glu His His His His His His
            1300                1305                1310
```

<210> 21
<211> 4344
<212> DNA
<213> Artificial Sequence

<220>
<223> deltaG983-961

<400> 21

```
atgacttctg cgcccgactt caatgcaggc ggtaccggta tcggcagcaa cagcagagca    60
acaacagcga aatcagcagc agtatcttac gccggtatca agaacgaaat gtgcaaagac   120
agaagcatgc tctgtgccgg tcgggatgac gttgcggtta cagacaggga tgccaaaatc   180
aatgcccccc ccccgaatct gcataccgga gactttccaa acccaaatga cgcatacaag   240
aatttgatca acctcaaacc tgcaattgaa gcaggctata caggacgcgg ggtagaggta   300
ggtatcgtcg acacaggcga atccgtcggc agcatatcct ttcccgaact gtatggcaga   360
aaagaacacg gctataacga aaattacaaa aactatacgg cgtatatgcg gaaggaagcg   420
cctgaagacg gaggcggtaa agacattgaa gcttctttcg acgatgaggc cgttatagag   480
actgaagcaa agccgacgga tatccgccac gtaaaagaaa tcggacacat cgatttggtc   540
tcccatatta ttggcgggcg ttccgtggac ggcagacctg caggcggtat tgcgcccgat   600
gcgacgctac acataatgaa tacgaatgat gaaaccaaga acgaaatgat ggttgcagcc   660
atccgcaatg catgggtcaa gctgggcgaa cgtggcgtgc gcatcgtcaa taacagtttt   720
ggaacaacat cgagggcagg cactgccgac cttttccaaa tagccaattc ggaggagcag   780
taccgccaag cgttgctcga ctattccggc ggtgataaaa cagacgaggg tatccgcctg   840
atgcaacaga gcgattacgg caacctgtcc taccacatcc gtaataaaaa catgcttttc   900
atcttttcga caggcaatga cgcacaagct cagcccaaca catatgccct attgccattt   960
tatgaaaaag acgctcaaaa aggcattatc acagtcgcag gcgtagaccg cagtggagaa  1020
aagttcaaac gggaaatgta tggagaaccg ggtacagaac cgcttgagta tggctccaac  1080
cattgcggaa ttactgccat gtggtgcctg tcggcaccct atgaagcaag cgtccgtttc  1140
acccgtacaa acccgattca aattgccgga acatcctttt ccgcacccat cgtaaccggc  1200
acggcggctc tgctgctgca gaaataccgg tggatgagca acgacaacct gcgtaccacg  1260
ttgctgacga cggctcagga catcggtgca gtcggcgtgg acagcaagtt cggctgggga  1320
ctgctggatg cgggtaaggc catgaacgga cccgcgtcct ttccgttcgg cgactttacc  1380
gccgatacga aaggtacatc cgatattgcc tactccttcc gtaacgacat ttcaggcacg  1440
ggcggcctga tcaaaaaagg cggcagccaa ctgcaactgc acggcaacaa cacctatacg  1500
ggcaaaacca ttatcgaagg cggttcgctg gtgttgtacg gcaacaacaa atcggatatg  1560
```

```
cgcgtcgaaa ccaaaggtgc gctgatttat aacggggcgg catccggcgg cagcctgaac   1620
agcgacggca ttgtctatct ggcagatacc gaccaatccg gcgcaaacga aaccgtacac   1680
atcaaaggca gtctgcagct ggacggcaaa ggtacgctgt acacacgttt gggcaaactg   1740
ctgaaagtgg acggtacggc gattatcggc ggcaagctgt acatgtcggc acgcggcaag   1800
ggggcaggct atctcaacag taccggacga cgtgttccct tcctgagtgc cgccaaaatc   1860
gggcaggatt attctttctt cacaaacatc gaaaccgacg gcggcctgct ggcttccctc   1920
gacagcgtcg aaaaaacagc gggcagtgaa ggcgacacgc tgtcctatta tgtccgtcgc   1980
ggcaatgcgg cacggactgc ttcggcagcg gcacattccg cgcccgccgg tctgaaacac   2040
gccgtagaac agggcggcag caatctggaa aacctgatgg tcgaactgga tgcctccgaa   2100
tcatccgcaa cacccgagac ggttgaaact gcggcagccg accgcacaga tatgccgggc   2160
atccgcccct acggcgcaac tttccgcgca gcggcagccg tacagcatgc gaatgccgcc   2220
gacggtgtac gcatcttcaa cagtctcgcc gctaccgtct atgccgacag taccgccgcc   2280
catgccgata tgcagggacg ccgcctgaaa gccgtatcgg acgggttgga ccacaacggc   2340
acgggtctgc gcgtcatcgc gcaaacccaa caggacggtg gaacgtggga acagggcggt   2400
gttgaaggca aaatgcgcgg cagtacccaa accgtcggca ttgccgcgaa aaccggcgaa   2460
aatacgacag cagccgccac actgggcatg ggacgcagca catggagcga aaacagtgca   2520
aatgcaaaaa ccgacagcat tagtctgttt gcaggcatac ggcacgatgc gggcgatatc   2580
ggctatctca aaggcctgtt ctcctacgga cgctacaaaa acagcatcag ccgcagcacc   2640
ggtgcggacg aacatgcgga aggcagcgtc aacggcacgc tgatgcagct gggcgcactg   2700
ggcggtgtca acgttccgtt tgccgcaacg ggagatttga cggtcgaagg cggtctgcgc   2760
tacgacctgc tcaaacagga tgcattcgcc gaaaaaggca gtgctttggg ctggagcggc   2820
aacagcctca ctgaaggcac gctggtcgga ctcgcgggtc tgaagctgtc gcaacccttg   2880
agcgataaag ccgtcctgtt tgcaacggcg ggcgtggaac gcgacctgaa cggacgcgac   2940
tacacggtaa cgggcggctt taccggcgcg actgcagcaa ccggcaagac gggggcacgc   3000
aatatgccgc acacccgtct ggttgccggc ctgggcgcgg atgtcgaatt cggcaacggc   3060
tggaacggct tggcacgtta cagctacgcc ggttccaaac agtacggcaa ccacagcgga   3120
cgagtcggcg taggctaccg gttcctcgag ggtggcggag gcactggatc cgccacaaac   3180
gacgacgatg ttaaaaaagc tgccactgtg gccattgctg ctgcctacaa caatggccaa   3240
gaaatcaacg gtttcaaagc tggagagacc atctacgaca ttgatgaaga cggcacaatt   3300
accaaaaaag acgcaactgc agccgatgtt gaagccgacg actttaaagg tctgggtctg   3360
aaaaaagtcg tgactaacct gaccaaaacc gtcaatgaaa acaaacaaaa cgtcgatgcc   3420
aaagtaaaag ctgcagaatc tgaaatagaa aagttaacaa ccaagttagc agacactgat   3480
gccgctttag cagatactga tgccgctctg gatgcaacca ccaacgcctt gaataaattg   3540
ggagaaaata taacgacatt tgctgaagag actaagacaa atatcgtaaa aattgatgaa   3600
aaattagaag ccgtggctga taccgtcgac aagcatgccg aagcattcaa cgatatcgcc   3660
gattcattgg atgaaaccaa cactaaggca gacgaagccg tcaaaaccgc caatgaagcc   3720
aaacagacgg ccgaagaaac caaacaaaac gtcgatgcca aagtaaaagc tgcagaaact   3780
gcagcaggca aagccgaagc tgccgctggc acagctaata ctgcagccga caaggccgaa   3840
gctgtcgctg caaaagttac cgacatcaaa gctgatatcg ctacgaacaa agataatatt   3900
gctaaaaaag caaacagtgc cgacgtgtac accagagaag agtctgacag caaatttgtc   3960
agaattgatg gtctgaacgc tactaccgaa aaattggaca cacgcttggc ttctgctgaa   4020
aaatccattg ccgatcacga tactcgcctg aacggtttgg ataaaacagt gtcagacctg   4080
cgcaaagaaa cccgccaagg ccttgcagaa caagccgcgc tctccggtct gttccaacct   4140
tacaacgtgg gtcggttcaa tgtaacggct gcagtcggcg gctacaaatc cgaatcggca   4200
gtcgccatcg gtaccggctt ccgctttacc gaaaactttg ccgccaaagc aggcgtggca   4260
gtcggcactt cgtccggttc ttccgcagcc taccatgtcg gcgtcaatta cgagtggctc   4320
gagcaccacc accaccacca ctga                                          4344
```

<210> 22
<211> 1447
<212> PRT
<213> Artificial Sequence

<220>
<223> deltaG983-961

<400> 22

```
Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly
```

```
            20                  25             30

Ile Lys Asn Glu Met Cys Lys Asp Arg Ser Met Leu Cys Ala Gly Arg
        35                  40                  45

Asp Asp Val Ala Val Thr Asp Arg Asp Ala Lys Ile Asn Ala Pro Pro
    50                  55                  60

Pro Asn Leu His Thr Gly Asp Phe Pro Asn Pro Asn Asp Ala Tyr Lys
65                  70                  75                  80

Asn Leu Ile Asn Leu Lys Pro Ala Ile Glu Ala Gly Tyr Thr Gly Arg
                85                  90                  95

Gly Val Glu Val Gly Ile Val Asp Thr Gly Glu Ser Val Gly Ser Ile
            100                 105                 110

Ser Phe Pro Glu Leu Tyr Gly Arg Lys Glu His Gly Tyr Asn Glu Asn
        115                 120                 125

Tyr Lys Asn Tyr Thr Ala Tyr Met Arg Lys Glu Ala Pro Glu Asp Gly
    130                 135                 140

Gly Gly Lys Asp Ile Glu Ala Ser Phe Asp Asp Glu Ala Val Ile Glu
145                 150                 155                 160

Thr Glu Ala Lys Pro Thr Asp Ile Arg His Val Lys Glu Ile Gly His
                165                 170                 175

Ile Asp Leu Val Ser His Ile Ile Gly Gly Arg Ser Val Asp Gly Arg
            180                 185                 190

Pro Ala Gly Gly Ile Ala Pro Asp Ala Thr Leu His Ile Met Asn Thr
        195                 200                 205

Asn Asp Glu Thr Lys Asn Glu Met Met Val Ala Ala Ile Arg Asn Ala
    210                 215                 220

Trp Val Lys Leu Gly Glu Arg Gly Val Arg Ile Val Asn Asn Ser Phe
225                 230                 235                 240

Gly Thr Thr Ser Arg Ala Gly Thr Ala Asp Leu Phe Gln Ile Ala Asn
                245                 250                 255

Ser Glu Glu Gln Tyr Arg Gln Ala Leu Leu Asp Tyr Ser Gly Gly Asp
            260                 265                 270

Lys Thr Asp Glu Gly Ile Arg Leu Met Gln Gln Ser Asp Tyr Gly Asn
        275                 280                 285

Leu Ser Tyr His Ile Arg Asn Lys Asn Met Leu Phe Ile Phe Ser Thr
    290                 295                 300

Gly Asn Asp Ala Gln Ala Gln Pro Asn Thr Tyr Ala Leu Leu Pro Phe
305                 310                 315                 320

Tyr Glu Lys Asp Ala Gln Lys Gly Ile Ile Thr Val Ala Gly Val Asp
                325                 330                 335

Arg Ser Gly Glu Lys Phe Lys Arg Glu Met Tyr Gly Glu Pro Gly Thr
            340                 345                 350
```

Glu Pro Leu Glu Tyr Gly Ser Asn His Cys Gly Ile Thr Ala Met Trp
355 360 365

Cys Leu Ser Ala Pro Tyr Glu Ala Ser Val Arg Phe Thr Arg Thr Asn
370 375 380

Pro Ile Gln Ile Ala Gly Thr Ser Phe Ser Ala Pro Ile Val Thr Gly
385 390 395 400

Thr Ala Ala Leu Leu Leu Gln Lys Tyr Pro Trp Met Ser Asn Asp Asn
405 410 415

Leu Arg Thr Thr Leu Leu Thr Thr Ala Gln Asp Ile Gly Ala Val Gly
420 425 430

Val Asp Ser Lys Phe Gly Trp Gly Leu Leu Asp Ala Gly Lys Ala Met
435 440 445

Asn Gly Pro Ala Ser Phe Pro Phe Gly Asp Phe Thr Ala Asp Thr Lys
450 455 460

Gly Thr Ser Asp Ile Ala Tyr Ser Phe Arg Asn Asp Ile Ser Gly Thr
465 470 475 480

Gly Gly Leu Ile Lys Lys Gly Gly Ser Gln Leu Gln Leu His Gly Asn
485 490 495

Asn Thr Tyr Thr Gly Lys Thr Ile Ile Glu Gly Gly Ser Leu Val Leu
500 505 510

Tyr Gly Asn Asn Lys Ser Asp Met Arg Val Glu Thr Lys Gly Ala Leu
515 520 525

Ile Tyr Asn Gly Ala Ala Ser Gly Gly Ser Leu Asn Ser Asp Gly Ile
530 535 540

Val Tyr Leu Ala Asp Thr Asp Gln Ser Gly Ala Asn Glu Thr Val His
545 550 555 560

Ile Lys Gly Ser Leu Gln Leu Asp Gly Lys Gly Thr Leu Tyr Thr Arg
565 570 575

Leu Gly Lys Leu Leu Lys Val Asp Gly Thr Ala Ile Ile Gly Gly Lys
580 585 590

Leu Tyr Met Ser Ala Arg Gly Lys Gly Ala Gly Tyr Leu Asn Ser Thr
595 600 605

Gly Arg Arg Val Pro Phe Leu Ser Ala Ala Lys Ile Gly Gln Asp Tyr
610 615 620

Ser Phe Phe Thr Asn Ile Glu Thr Asp Gly Gly Leu Leu Ala Ser Leu
625 630 635 640

Asp Ser Val Glu Lys Thr Ala Gly Ser Glu Gly Asp Thr Leu Ser Tyr
645 650 655

Tyr Val Arg Arg Gly Asn Ala Ala Arg Thr Ala Ser Ala Ala Ala His
660 665 670

```
Ser Ala Pro Ala Gly Leu Lys His Ala Val Glu Gln Gly Gly Ser Asn
        675                 680                 685

Leu Glu Asn Leu Met Val Glu Leu Asp Ala Ser Glu Ser Ser Ala Thr
    690                 695                 700

Pro Glu Thr Val Glu Thr Ala Ala Ala Asp Arg Thr Asp Met Pro Gly
705                 710                 715                 720

Ile Arg Pro Tyr Gly Ala Thr Phe Arg Ala Ala Ala Ala Val Gln His
                725                 730                 735

Ala Asn Ala Ala Asp Gly Val Arg Ile Phe Asn Ser Leu Ala Ala Thr
            740                 745                 750

Val Tyr Ala Asp Ser Thr Ala Ala His Ala Asp Met Gln Gly Arg Arg
        755                 760                 765

Leu Lys Ala Val Ser Asp Gly Leu Asp His Asn Gly Thr Gly Leu Arg
    770                 775                 780

Val Ile Ala Gln Thr Gln Gln Asp Gly Gly Thr Trp Glu Gln Gly Gly
785                 790                 795                 800

Val Glu Gly Lys Met Arg Gly Ser Thr Gln Thr Val Gly Ile Ala Ala
                805                 810                 815

Lys Thr Gly Glu Asn Thr Thr Ala Ala Ala Thr Leu Gly Met Gly Arg
            820                 825                 830

Ser Thr Trp Ser Glu Asn Ser Ala Asn Ala Lys Thr Asp Ser Ile Ser
        835                 840                 845

Leu Phe Ala Gly Ile Arg His Asp Ala Gly Asp Ile Gly Tyr Leu Lys
    850                 855                 860

Gly Leu Phe Ser Tyr Gly Arg Tyr Lys Asn Ser Ile Ser Arg Ser Thr
865                 870                 875                 880

Gly Ala Asp Glu His Ala Glu Gly Ser Val Asn Gly Thr Leu Met Gln
                885                 890                 895

Leu Gly Ala Leu Gly Gly Val Asn Val Pro Phe Ala Ala Thr Gly Asp
            900                 905                 910

Leu Thr Val Glu Gly Gly Leu Arg Tyr Asp Leu Leu Lys Gln Asp Ala
        915                 920                 925

Phe Ala Glu Lys Gly Ser Ala Leu Gly Trp Ser Gly Asn Ser Leu Thr
    930                 935                 940

Glu Gly Thr Leu Val Gly Leu Ala Gly Leu Lys Leu Ser Gln Pro Leu
945                 950                 955                 960

Ser Asp Lys Ala Val Leu Phe Ala Thr Ala Gly Val Glu Arg Asp Leu
                965                 970                 975

Asn Gly Arg Asp Tyr Thr Val Thr Gly Gly Phe Thr Gly Ala Thr Ala
            980                 985                 990

Ala Thr Gly Lys Thr Gly Ala Arg Asn Met Pro His Thr Arg Leu Val
```

995 1000 1005

Ala Gly Leu Gly Ala Asp Val Glu Phe Gly Asn Gly Trp Asn Gly Leu
1010 1015 1020

Ala Arg Tyr Ser Tyr Ala Gly Ser Lys Gln Tyr Gly Asn His Ser Gly
1025 1030 1035 1040

Arg Val Gly Val Gly Tyr Arg Phe Leu Glu Gly Gly Gly Gly Thr Gly
1045 1050 1055

Ser Ala Thr Asn Asp Asp Asp Val Lys Lys Ala Ala Thr Val Ala Ile
1060 1065 1070

Ala Ala Ala Tyr Asn Asn Gly Gln Glu Ile Asn Gly Phe Lys Ala Gly
1075 1080 1085

Glu Thr Ile Tyr Asp Ile Asp Glu Asp Gly Thr Ile Thr Lys Lys Asp
1090 1095 1100

Ala Thr Ala Ala Asp Val Glu Ala Asp Asp Phe Lys Gly Leu Gly Leu
1105 1110 1115 1120

Lys Lys Val Val Thr Asn Leu Thr Lys Thr Val Asn Glu Asn Lys Gln
1125 1130 1135

Asn Val Asp Ala Lys Val Lys Ala Ala Glu Ser Glu Ile Glu Lys Leu
1140 1145 1150

Thr Thr Lys Leu Ala Asp Thr Asp Ala Ala Leu Ala Asp Thr Asp Ala
1155 1160 1165

Ala Leu Asp Ala Thr Thr Asn Ala Leu Asn Lys Leu Gly Glu Asn Ile
1170 1175 1180

Thr Thr Phe Ala Glu Glu Thr Lys Thr Asn Ile Val Lys Ile Asp Glu
1185 1190 1195 1200

Lys Leu Glu Ala Val Ala Asp Thr Val Asp Lys His Ala Glu Ala Phe
1205 1210 1215

Asn Asp Ile Ala Asp Ser Leu Asp Glu Thr Asn Thr Lys Ala Asp Glu
1220 1225 1230

Ala Val Lys Thr Ala Asn Glu Ala Lys Gln Thr Ala Glu Glu Thr Lys
1235 1240 1245

Gln Asn Val Asp Ala Lys Val Lys Ala Ala Glu Thr Ala Ala Gly Lys
1250 1255 1260

Ala Glu Ala Ala Ala Gly Thr Ala Asn Thr Ala Ala Asp Lys Ala Glu
1265 1270 1275 1280

Ala Val Ala Ala Lys Val Thr Asp Ile Lys Ala Asp Ile Ala Thr Asn
1285 1290 1295

Lys Asp Asn Ile Ala Lys Lys Ala Asn Ser Ala Asp Val Tyr Thr Arg
1300 1305 1310

Glu Glu Ser Asp Ser Lys Phe Val Arg Ile Asp Gly Leu Asn Ala Thr
1315 1320 1325

```
Thr Glu Lys Leu Asp Thr Arg Leu Ala Ser Ala Glu Lys Ser Ile Ala
    1330                1335                1340

Asp His Asp Thr Arg Leu Asn Gly Leu Asp Lys Thr Val Ser Asp Leu
1345                1350                1355                1360

Arg Lys Glu Thr Arg Gln Gly Leu Ala Glu Gln Ala Ala Leu Ser Gly
                1365                1370                1375

Leu Phe Gln Pro Tyr Asn Val Gly Arg Phe Asn Val Thr Ala Ala Val
            1380                1385                1390

Gly Gly Tyr Lys Ser Glu Ser Ala Val Ala Ile Gly Thr Gly Phe Arg
        1395                1400                1405

Phe Thr Glu Asn Phe Ala Ala Lys Ala Gly Val Ala Val Gly Thr Ser
    1410                1415                1420

Ser Gly Ser Ser Ala Ala Tyr His Val Gly Val Asn Tyr Glu Trp Leu
1425                1430                1435                1440

Glu His His His His His His
                1445
```

<210> 23
<211> 4179
<212> DNA
<213> Artificial Sequence

<220>
<223> deltaG983-961c

<400> 23

```
atgacttctg cgcccgactt caatgcaggc ggtaccggta tcggcagcaa cagcagagca    60
acaacagcga aatcagcagc agtatcttac gccggtatca agaacgaaat gtgcaaagac   120
agaagcatgc tctgtgccgg tcgggatgac gttgcggtta cagacaggga tgccaaaatc   180
aatgcccccc ccccgaatct gcataccgga gactttccaa acccaaatga cgcatacaag   240
aatttgatca acctcaaacc tgcaattgaa gcaggctata caggacgcgg ggtagaggta   300
ggtatcgtcg acacaggcga atccgtcggc agcatatcct ttcccgaact gtatggcaga   360
aaagaacacg gctataacga aaattacaaa aactatacgg cgtatatgcg gaaggaagcg   420
cctgaagacg gaggcggtaa agacattgaa gcttctttcg acgatgaggc cgttatagag   480
actgaagcaa agccgacgga tatccgccac gtaaaagaaa tcggacacat cgatttggtc   540
tcccatatta ttggcgggcg ttccgtggac ggcagacctg caggcggtat tgcgcccgat   600
gcgacgctac acataatgaa tacgaatgat gaaaccaaga acgaaatgat ggttgcagcc   660
atccgcaatg catgggtcaa gctgggcgaa cgtggcgtgc gcatcgtcaa taacagtttt   720
ggaacaacat cgagggcagg cactgccgac cttttccaaa tagccaattc ggaggagcag   780
taccgccaag cgttgctcga ctattccggc ggtgataaaa cagacgaggg tatccgcctg   840
atgcaacaga gcgattacgg caacctgtcc taccacatcc gtaataaaaa catgcttttc   900
atcttttcga caggcaatga cgcacaagct cagcccaaca catatgccct attgccattt   960
tatgaaaaag acgctcaaaa aggcattatc acagtcgcag gcgtagaccg cagtggagaa  1020
aagttcaaac gggaaatgta tggagaaccg ggtacagaac cgcttgagta tggctccaac  1080
cattgcggaa ttactgccat gtggtgcctg tcggcaccct atgaagcaag cgtccgtttc  1140
acccgtacaa acccgattca aattgccgga acatcctttt ccgcacccat cgtaaccggc  1200
acggcggctc tgctgctgca gaaatacccg tggatgagca acgacaacct gcgtaccacg  1260
ttgctgacga cggctcagga catcggtgca gtcggcgtgg acagcaagtt cggctgggga  1320
ctgctggatg cgggtaaggc catgaacgga cccgcgtcct ttccgttcgg cgactttacc  1380
gccgatacga aaggtacatc cgatattgcc tactccttcc gtaacgacat ttcaggcacg  1440
ggcggcctga tcaaaaaagg cggcagccaa ctgcaactgc acggcaacaa cacctatacg  1500
ggcaaaacca ttatcgaagg cggttcgctg gtgttgtacg gcaacaacaa atcggatatg  1560
cgcgtcgaaa ccaaaggtgc gctgatttat aacggggcgg catccggcgg cagcctgaac  1620
```

```
agcgacggca ttgtctatct ggcagatacc gaccaatccg gcgcaaacga aaccgtacac   1680
atcaaaggca gtctgcagct ggacggcaaa ggtacgctgt acacacgttt gggcaaactg   1740
ctgaaagtgg acggtacggc gattatcggc ggcaagctgt acatgtcggc acgcggcaag   1800
ggggcaggct atctcaacag taccggacga cgtgttccct tcctgagtgc cgccaaaatc   1860
gggcaggatt attctttctt cacaaacatc gaaaccgacg gcggcctgct ggcttccctc   1920
gacagcgtcg aaaaaacagc gggcagtgaa ggcgacacgc tgtcctatta tgtccgtcgc   1980
ggcaatgcgg cacggactgc ttcggcagcg gcacattccg cgcccgccgg tctgaaacac   2040
gccgtagaac agggcggcag caatctggaa aacctgatgg tcgaactgga tgcctccgaa   2100
tcatccgcaa cacccgagac ggttgaaact gcggcagccg accgcacaga tatgccgggc   2160
atccgcccct acggcgcaac tttccgcgca gcggcagccg tacagcatgc gaatgccgcc   2220
gacggtgtac gcatcttcaa cagtctcgcc gctaccgtct atgccgacag taccgccgcc   2280
catgccgata tgcagggacg ccgcctgaaa gccgtatcgg acgggttgga ccacaacggc   2340
acgggtctgc gcgtcatcgc gcaaacccaa caggacggtg gaacgtggga acagggcggt   2400
gttgaaggca aaatgcgcgg cagtacccaa accgtcggca ttgccgcgaa aaccggcgaa   2460
aatacgacag cagccgccac actgggcatg ggacgcagca catggagcga aaacagtgca   2520
aatgcaaaaa ccgacagcat tagtctgttt gcaggcatac ggcacgatgc gggcgatatc   2580
ggctatctca aggcctgtt ctcctacgga cgctacaaaa acagcatcag ccgcagcacc   2640
ggtgcggacg aacatgcgga aggcagcgtc aacggcacgc tgatgcagct gggcgcactg   2700
ggcggtgtca acgttccgtt tgccgcaacg ggagatttga cggtcgaagg cggtctgcgc   2760
tacgacctgc tcaaacagga tgcattcgcc gaaaaaggca gtgctttggg ctggagcggc   2820
aacagcctca ctgaaggcac gctggtcgga ctcgcgggtc tgaagctgtc gcaacccttg   2880
agcgataaag ccgtcctgtt tgcaacggcg ggcgtggaac gcgacctgaa cggacgcgac   2940
tacacggtaa cgggcggctt taccggcgcg actgcagcaa ccggcaagac gggggcacgc   3000
aatatgccgc acacccgtct ggttgccggc ctgggcgcgg atgtcgaatt cggcaacggc   3060
tggaacggct tggcacgtta cagctacgcc ggttccaaac agtacggcaa ccacagcgga   3120
cgagtcggcg taggctaccg gttcctcgag ggtggcggag gcactggatc cgccacaaac   3180
gacgacgatg ttaaaaaagc tgccactgtg gccattgctg ctgcctacaa caatggccaa   3240
gaaatcaacg gtttcaaagc tggagagacc atctacgaca ttgatgaaga cggcacaatt   3300
accaaaaaag acgcaactgc agccgatgtt gaagccgacg actttaaagg tctgggtctg   3360
aaaaaagtcg tgactaacct gaccaaaacc gtcaatgaaa acaaacaaaa cgtcgatgcc   3420
aaagtaaaag ctgcagaatc tgaaatagaa aagttaacaa ccaagttagc agacactgat   3480
gccgctttag cagatactga tgccgctctg gatgcaacca ccaacgcctt gaataaattg   3540
ggagaaaata taacgacatt tgctgaagag actaagacaa atatcgtaaa aattgatgaa   3600
aaattagaag ccgtggctga taccgtcgac aagcatgccg aagcattcaa cgatatcgcc   3660
gattcattgg atgaaaccaa cactaaggca gacgaagccg tcaaaaccgc caatgaagcc   3720
aaacagacgg ccgaagaaac caaacaaaac gtcgatgcca aagtaaaagc tgcagaaact   3780
gcagcaggca aagccgaagc tgccgctggc acagctaata ctgcagccga caaggccgaa   3840
gctgtcgctg caaaagttac cgacatcaaa gctgatatcg ctacgaacaa agataatatt   3900
gctaaaaaag caaacagtgc cgacgtgtac accagagaag agtctgacag caaatttgtc   3960
agaattgatg gtctgaacgc tactaccgaa aaattggaca cacgcttggc ttctgctgaa   4020
aaatccattg ccgatcacga tactcgcctg aacggtttgg ataaaacagt gtcagacctg   4080
cgcaaagaaa cccgccaagg ccttgcagaa caagccgcgc tctccggtct gttccaacct   4140
tacaacgtgg gtctcgagca ccaccaccac caccactga                          4179
```

<210> 24
<211> 1392
<212> PRT
<213> Artificial Sequence

<220>
<223> deltaG983-961c

<400> 24

```
Met Thr Ser Ala Pro Asp Phe Asn Ala Gly Gly Thr Gly Ile Gly Ser
1               5                   10                  15

Asn Ser Arg Ala Thr Thr Ala Lys Ser Ala Ala Val Ser Tyr Ala Gly
            20                  25                  30

Ile Lys Asn Glu Met Cys Lys Asp Arg Ser Met Leu Cys Ala Gly Arg
        35                  40                  45
```

```
Asp Asp Val Ala Val Thr Asp Arg Asp Ala Lys Ile Asn Ala Pro Pro
    50                  55                  60

Pro Asn Leu His Thr Gly Asp Phe Pro Asn Pro Asn Asp Ala Tyr Lys
65                  70                  75                  80

Asn Leu Ile Asn Leu Lys Pro Ala Ile Glu Ala Gly Tyr Thr Gly Arg
                85                  90                  95

Gly Val Glu Val Gly Ile Val Asp Thr Gly Glu Ser Val Gly Ser Ile
            100                 105                 110

Ser Phe Pro Glu Leu Tyr Gly Arg Lys Glu His Gly Tyr Asn Glu Asn
        115                 120                 125

Tyr Lys Asn Tyr Thr Ala Tyr Met Arg Lys Glu Ala Pro Glu Asp Gly
    130                 135                 140

Gly Gly Lys Asp Ile Glu Ala Ser Phe Asp Asp Glu Ala Val Ile Glu
145                 150                 155                 160

Thr Glu Ala Lys Pro Thr Asp Ile Arg His Val Lys Glu Ile Gly His
                165                 170                 175

Ile Asp Leu Val Ser His Ile Ile Gly Gly Arg Ser Val Asp Gly Arg
            180                 185                 190

Pro Ala Gly Gly Ile Ala Pro Asp Ala Thr Leu His Ile Met Asn Thr
        195                 200                 205

Asn Asp Glu Thr Lys Asn Glu Met Met Val Ala Ala Ile Arg Asn Ala
    210                 215                 220

Trp Val Lys Leu Gly Glu Arg Gly Val Arg Ile Val Asn Asn Ser Phe
225                 230                 235                 240

Gly Thr Thr Ser Arg Ala Gly Thr Ala Asp Leu Phe Gln Ile Ala Asn
                245                 250                 255

Ser Glu Glu Gln Tyr Arg Gln Ala Leu Leu Asp Tyr Ser Gly Gly Asp
            260                 265                 270

Lys Thr Asp Glu Gly Ile Arg Leu Met Gln Gln Ser Asp Tyr Gly Asn
        275                 280                 285

Leu Ser Tyr His Ile Arg Asn Lys Asn Met Leu Phe Ile Phe Ser Thr
    290                 295                 300

Gly Asn Asp Ala Gln Ala Gln Pro Asn Thr Tyr Ala Leu Leu Pro Phe
305                 310                 315                 320

Tyr Glu Lys Asp Ala Gln Lys Gly Ile Ile Thr Val Ala Gly Val Asp
                325                 330                 335

Arg Ser Gly Glu Lys Phe Lys Arg Glu Met Tyr Gly Glu Pro Gly Thr
            340                 345                 350

Glu Pro Leu Glu Tyr Gly Ser Asn His Cys Gly Ile Thr Ala Met Trp
        355                 360                 365
```

```
Cys Leu Ser Ala Pro Tyr Glu Ala Ser Val Arg Phe Thr Arg Thr Asn
    370                 375                 380

Pro Ile Gln Ile Ala Gly Thr Ser Phe Ser Ala Pro Ile Val Thr Gly
385                 390                 395                 400

Thr Ala Ala Leu Leu Leu Gln Lys Tyr Pro Trp Met Ser Asn Asp Asn
                405                 410                 415

Leu Arg Thr Thr Leu Leu Thr Thr Ala Gln Asp Ile Gly Ala Val Gly
            420                 425                 430

Val Asp Ser Lys Phe Gly Trp Gly Leu Leu Asp Ala Gly Lys Ala Met
        435                 440                 445

Asn Gly Pro Ala Ser Phe Pro Phe Gly Asp Phe Thr Ala Asp Thr Lys
    450                 455                 460

Gly Thr Ser Asp Ile Ala Tyr Ser Phe Arg Asn Asp Ile Ser Gly Thr
465                 470                 475                 480

Gly Gly Leu Ile Lys Lys Gly Gly Ser Gln Leu Gln Leu His Gly Asn
                485                 490                 495

Asn Thr Tyr Thr Gly Lys Thr Ile Ile Glu Gly Gly Ser Leu Val Leu
            500                 505                 510

Tyr Gly Asn Asn Lys Ser Asp Met Arg Val Glu Thr Lys Gly Ala Leu
        515                 520                 525

Ile Tyr Asn Gly Ala Ala Ser Gly Gly Ser Leu Asn Ser Asp Gly Ile
    530                 535                 540

Val Tyr Leu Ala Asp Thr Asp Gln Ser Gly Ala Asn Glu Thr Val His
545                 550                 555                 560

Ile Lys Gly Ser Leu Gln Leu Asp Gly Lys Gly Thr Leu Tyr Thr Arg
                565                 570                 575

Leu Gly Lys Leu Leu Lys Val Asp Gly Thr Ala Ile Ile Gly Gly Lys
            580                 585                 590

Leu Tyr Met Ser Ala Arg Gly Lys Gly Ala Gly Tyr Leu Asn Ser Thr
        595                 600                 605

Gly Arg Arg Val Pro Phe Leu Ser Ala Ala Lys Ile Gly Gln Asp Tyr
    610                 615                 620

Ser Phe Phe Thr Asn Ile Glu Thr Asp Gly Gly Leu Leu Ala Ser Leu
625                 630                 635                 640

Asp Ser Val Glu Lys Thr Ala Gly Ser Glu Gly Asp Thr Leu Ser Tyr
                645                 650                 655

Tyr Val Arg Arg Gly Asn Ala Ala Arg Thr Ala Ser Ala Ala Ala His
            660                 665                 670

Ser Ala Pro Ala Gly Leu Lys His Ala Val Glu Gln Gly Gly Ser Asn
        675                 680                 685

Leu Glu Asn Leu Met Val Glu Leu Asp Ala Ser Glu Ser Ser Ala Thr
```

690 695 700

Pro Glu Thr Val Glu Thr Ala Ala Ala Asp Arg Thr Asp Met Pro Gly
705 710 715 720

Ile Arg Pro Tyr Gly Ala Thr Phe Arg Ala Ala Ala Ala Val Gln His
725 730 735

Ala Asn Ala Ala Asp Gly Val Arg Ile Phe Asn Ser Leu Ala Ala Thr
740 745 750

Val Tyr Ala Asp Ser Thr Ala Ala His Ala Asp Met Gln Gly Arg Arg
755 760 765

Leu Lys Ala Val Ser Asp Gly Leu Asp His Asn Gly Thr Gly Leu Arg
770 775 780

Val Ile Ala Gln Thr Gln Gln Asp Gly Gly Thr Trp Glu Gln Gly Gly
785 790 795 800

Val Glu Gly Lys Met Arg Gly Ser Thr Gln Thr Val Gly Ile Ala Ala
805 810 815

Lys Thr Gly Glu Asn Thr Thr Ala Ala Ala Thr Leu Gly Met Gly Arg
820 825 830

Ser Thr Trp Ser Glu Asn Ser Ala Asn Ala Lys Thr Asp Ser Ile Ser
835 840 845

Leu Phe Ala Gly Ile Arg His Asp Ala Gly Asp Ile Gly Tyr Leu Lys
850 855 860

Gly Leu Phe Ser Tyr Gly Arg Tyr Lys Asn Ser Ile Ser Arg Ser Thr
865 870 875 880

Gly Ala Asp Glu His Ala Glu Gly Ser Val Asn Gly Thr Leu Met Gln
885 890 895

Leu Gly Ala Leu Gly Gly Val Asn Val Pro Phe Ala Ala Thr Gly Asp
900 905 910

Leu Thr Val Glu Gly Gly Leu Arg Tyr Asp Leu Leu Lys Gln Asp Ala
915 920 925

Phe Ala Glu Lys Gly Ser Ala Leu Gly Trp Ser Gly Asn Ser Leu Thr
930 935 940

Glu Gly Thr Leu Val Gly Leu Ala Gly Leu Lys Leu Ser Gln Pro Leu
945 950 955 960

Ser Asp Lys Ala Val Leu Phe Ala Thr Ala Gly Val Glu Arg Asp Leu
965 970 975

Asn Gly Arg Asp Tyr Thr Val Thr Gly Gly Phe Thr Gly Ala Thr Ala
980 985 990

Ala Thr Gly Lys Thr Gly Ala Arg Asn Met Pro His Thr Arg Leu Val
995 1000 1005

Ala Gly Leu Gly Ala Asp Val Glu Phe Gly Asn Gly Trp Asn Gly Leu
1010 1015 1020

Ala Arg Tyr Ser Tyr Ala Gly Ser Lys Gln Tyr Gly Asn His Ser Gly
1025 1030 1035 1040

Arg Val Gly Val Gly Tyr Arg Phe Leu Glu Gly Gly Gly Gly Thr Gly
1045 1050 1055

Ser Ala Thr Asn Asp Asp Asp Val Lys Lys Ala Ala Thr Val Ala Ile
1060 1065 1070

Ala Ala Ala Tyr Asn Asn Gly Gln Glu Ile Asn Gly Phe Lys Ala Gly
1075 1080 1085

Glu Thr Ile Tyr Asp Ile Asp Glu Asp Gly Thr Ile Thr Lys Lys Asp
1090 1095 1100

Ala Thr Ala Ala Asp Val Glu Ala Asp Asp Phe Lys Gly Leu Gly Leu
1105 1110 1115 1120

Lys Lys Val Val Thr Asn Leu Thr Lys Thr Val Asn Glu Asn Lys Gln
1125 1130 1135

Asn Val Asp Ala Lys Val Lys Ala Ala Glu Ser Glu Ile Glu Lys Leu
1140 1145 1150

Thr Thr Lys Leu Ala Asp Thr Asp Ala Ala Leu Ala Asp Thr Asp Ala
1155 1160 1165

Ala Leu Asp Ala Thr Thr Asn Ala Leu Asn Lys Leu Gly Glu Asn Ile
1170 1175 1180

Thr Thr Phe Ala Glu Glu Thr Lys Thr Asn Ile Val Lys Ile Asp Glu
1185 1190 1195 1200

Lys Leu Glu Ala Val Ala Asp Thr Val Asp Lys His Ala Glu Ala Phe
1205 1210 1215

Asn Asp Ile Ala Asp Ser Leu Asp Glu Thr Asn Thr Lys Ala Asp Glu
1220 1225 1230

Ala Val Lys Thr Ala Asn Glu Ala Lys Gln Thr Ala Glu Glu Thr Lys
1235 1240 1245

Gln Asn Val Asp Ala Lys Val Lys Ala Ala Glu Thr Ala Ala Gly Lys
1250 1255 1260

Ala Glu Ala Ala Ala Gly Thr Ala Asn Thr Ala Ala Asp Lys Ala Glu
1265 1270 1275 1280

Ala Val Ala Ala Lys Val Thr Asp Ile Lys Ala Asp Ile Ala Thr Asn
1285 1290 1295

Lys Asp Asn Ile Ala Lys Lys Ala Asn Ser Ala Asp Val Tyr Thr Arg
1300 1305 1310

Glu Glu Ser Asp Ser Lys Phe Val Arg Ile Asp Gly Leu Asn Ala Thr
1315 1320 1325

Thr Glu Lys Leu Asp Thr Arg Leu Ala Ser Ala Glu Lys Ser Ile Ala
1330 1335 1340

```
Asp His Asp Thr Arg Leu Asn Gly Leu Asp Lys Thr Val Ser Asp Leu
1345                1350                1355                1360

Arg Lys Glu Thr Arg Gln Gly Leu Ala Glu Gln Ala Ala Leu Ser Gly
                1365                1370                1375

Leu Phe Gln Pro Tyr Asn Val Gly Leu Glu His His His His His His
            1380                1385                1390
```

<210> 25
<211> 274
<212> PRT
<213> Artificial Sequence

<220>
<223> 741

<400> 25

Val Asn Arg Thr Ala Phe Cys Cys Leu Ser Leu Thr Thr Ala Leu Ile
1               5                   10                  15

Leu Thr Ala Cys Ser Ser Gly Gly Gly Gly Val Ala Ala Asp Ile Gly
            20                  25                  30

Ala Gly Leu Ala Asp Ala Leu Thr Ala Pro Leu Asp His Lys Asp Lys
        35                  40                  45

Gly Leu Gln Ser Leu Thr Leu Asp Gln Ser Val Arg Lys Asn Glu Lys
    50                  55                  60

Leu Lys Leu Ala Ala Gln Gly Ala Glu Lys Thr Tyr Gly Asn Gly Asp
65                  70                  75                  80

Ser Leu Asn Thr Gly Lys Leu Lys Asn Asp Lys Val Ser Arg Phe Asp
                85                  90                  95

Phe Ile Arg Gln Ile Glu Val Asp Gly Gln Leu Ile Thr Leu Glu Ser
            100                 105                 110

Gly Glu Phe Gln Val Tyr Lys Gln Ser His Ser Ala Leu Thr Ala Phe
        115                 120                 125

Gln Thr Glu Gln Ile Gln Asp Ser Glu His Ser Gly Lys Met Val Ala
    130                 135                 140

Lys Arg Gln Phe Arg Ile Gly Asp Ile Ala Gly Glu His Thr Ser Phe
145                 150                 155                 160

Asp Lys Leu Pro Glu Gly Gly Arg Ala Thr Tyr Arg Gly Thr Ala Phe
                165                 170                 175

Gly Ser Asp Asp Ala Gly Gly Lys Leu Thr Tyr Thr Ile Asp Phe Ala
            180                 185                 190

Ala Lys Gln Gly Asn Gly Lys Ile Glu His Leu Lys Ser Pro Glu Leu
        195                 200                 205

Asn Val Asp Leu Ala Ala Ala Asp Ile Lys Pro Asp Gly Lys Arg His
    210                 215                 220

Ala Val Ile Ser Gly Ser Val Leu Tyr Asn Gln Ala Glu Lys Gly Ser
225                 230                 235                 240

Tyr Ser Leu Gly Ile Phe Gly Gly Lys Ala Gln Glu Val Ala Gly Ser
                245                 250                 255

Ala Glu Val Lys Thr Val Asn Gly Ile Arg His Ile Gly Leu Ala Ala
            260                 265                 270

Lys Gln

<210> 26
<211> 248
<212> PRT

<213> Artificial Sequence

<220>
<223> deltaG741

<400> 26

```
Val Ala Ala Asp Ile Gly Ala Gly Leu Ala Asp Ala Leu Thr Ala Pro
1               5                   10                  15

Leu Asp His Lys Asp Lys Gly Leu Gln Ser Leu Thr Leu Asp Gln Ser
            20                  25                  30

Val Arg Lys Asn Glu Lys Leu Lys Leu Ala Ala Gln Gly Ala Glu Lys
        35                  40                  45

Thr Tyr Gly Asn Gly Asp Ser Leu Asn Thr Gly Lys Leu Lys Asn Asp
    50                  55                  60

Lys Val Ser Arg Phe Asp Phe Ile Arg Gln Ile Glu Val Asp Gly Gln
65                  70                  75                  80

Leu Ile Thr Leu Glu Ser Gly Glu Phe Gln Val Tyr Lys Gln Ser His
                85                  90                  95

Ser Ala Leu Thr Ala Phe Gln Thr Glu Gln Ile Gln Asp Ser Glu His
            100                 105                 110

Ser Gly Lys Met Val Ala Lys Arg Gln Phe Arg Ile Gly Asp Ile Ala
        115                 120                 125

Gly Glu His Thr Ser Phe Asp Lys Leu Pro Glu Gly Gly Arg Ala Thr
    130                 135                 140

Tyr Arg Gly Thr Ala Phe Gly Ser Asp Asp Ala Gly Gly Lys Leu Thr
145                 150                 155                 160

Tyr Thr Ile Asp Phe Ala Ala Lys Gln Gly Asn Gly Lys Ile Glu His
                165                 170                 175

Leu Lys Ser Pro Glu Leu Asn Val Asp Leu Ala Ala Ala Asp Ile Lys
            180                 185                 190

Pro Asp Gly Lys Arg His Ala Val Ile Ser Gly Ser Val Leu Tyr Asn
        195                 200                 205

Gln Ala Glu Lys Gly Ser Tyr Ser Leu Gly Ile Phe Gly Gly Lys Ala



    210                 215                 220

Gln Glu Val Ala Gly Ser Ala Glu Val Lys Thr Val Asn Gly Ile Arg
225                 230                 235                 240

His Ile Gly Leu Ala Ala Lys Gln
                245
```

<210> 27
<211> 1947
<212> DNA
<213> Artificial Sequence

<220>
<223> deltaG741-961

<400> 27

```
atggtcgccg ccgacatcgg tgcggggctt gccgatgcac taaccgcacc gctcgaccat   60
aaagacaaag gtttgcagtc tttgacgctg gatcagtccg tcaggaaaaa cgagaaactg   120
aagctggcgg cacaaggtgc ggaaaaaact tatggaaacg gtgacagcct caatacgggc   180
aaattgaaga acgacaaggt cagccgtttc gactttatcc gccaaatcga agtggacggg   240
cagctcatta ccttggagag tggagagttc caagtataca aacaaagcca ttccgcctta   300
accgcctttc agaccgagca aatacaagat tcggagcatt ccgggaagat ggttgcgaaa   360
cgccagttca gaatcggcga catagcgggc gaacatacat cttttgacaa gcttcccgaa   420
ggcggcaggg cgacatatcg cgggacggcg ttcggttcag acgatgccgg cggaaaactg   480
acctacacca tagatttcgc cgccaagcag ggaaacggca aaatcgaaca tttgaaatcg   540
ccagaactca atgtcgacct ggccgccgcc gatatcaagc cggatggaaa acgccatgcc   600
gtcatcagcg gttccgtcct ttacaaccaa gccgagaaag gcagttactc cctcggtatc   660
tttggcggaa aagcccagga agttgccggc agcgcggaag tgaaaaccgt aaacggcata   720
cgccatatcg gccttgccgc caagcaactc gagggtggcg gaggcactgg atccgccaca   780
aacgacgacg atgttaaaaa agctgccact gtggccattg ctgctgccta caacaatggc   840
caagaaatca acggtttcaa agctggagag accatctacg acattgatga agacggcaca   900
attaccaaaa aagacgcaac tgcagccgat gttgaagccg acgactttaa aggtctgggt   960
ctgaaaaaag tcgtgactaa cctgaccaaa accgtcaatg aaaacaaaca aaacgtcgat   1020
gccaaagtaa aagctgcaga atctgaaata gaaaagttaa caaccaagtt agcagacact   1080
gatgccgctt tagcagatac tgatgccgct ctggatgcaa ccaccaacgc cttgaataaa   1140
ttgggagaaa atataacgac atttgctgaa gagactaaga caaatatcgt aaaaattgat   1200
gaaaaattag aagccgtggc tgataccgtc gacaagcatg ccgaagcatt caacgatatc   1260
gccgattcat tggatgaaac caacactaag gcagacgaag ccgtcaaaac cgccaatgaa   1320
gccaaacaga cggccgaaga aaccaaacaa aacgtcgatg ccaaagtaaa agctgcagaa   1380
actgcagcag gcaaagccga agctgccgct ggcacagcta atactgcagc cgacaaggcc   1440
gaagctgtcg ctgcaaaagt taccgacatc aaagctgata tcgctacgaa caaagataat   1500
attgctaaaa aagcaaacag tgccgacgtg tacaccagag aagagtctga cagcaaattt   1560
gtcagaattg atggtctgaa cgctactacc gaaaaattgg acacacgctt ggcttctgct   1620
gaaaaatcca ttgccgatca cgatactcgc ctgaacggtt tggataaaac agtgtcagac   1680
ctgcgcaaag aaacccgcca aggccttgca gaacaagccg cgctctccgg tctgttccaa   1740
ccttacaacg tgggtcggtt caatgtaacg gctgcagtcg gcggctacaa atccgaatcg   1800
gcagtcgcca tcggtaccgg cttccgcttt accgaaaact ttgccgccaa agcaggcgtg   1860
gcagtcggca cttcgtccgg ttcttccgca gcctaccatg tcggcgtcaa ttacgagtgg   1920
ctcgagcacc accaccacca ccactga                                       1947
```

<210> 28
<211> 648
<212> PRT
<213> Artificial Sequence

<220>
<223> deltaG741-961

<400> 28

Met Val Ala Ala Asp Ile Gly Ala Gly Leu Ala Asp Ala Leu Thr Ala

```
1               5                   10                  15
Pro Leu Asp His Lys Asp Lys Gly Leu Gln Ser Leu Thr Leu Asp Gln
            20                  25                  30
Ser Val Arg Lys Asn Glu Lys Leu Lys Leu Ala Ala Gln Gly Ala Glu
        35                  40                  45
Lys Thr Tyr Gly Asn Gly Asp Ser Leu Asn Thr Gly Lys Leu Lys Asn
    50                  55                  60
Asp Lys Val Ser Arg Phe Asp Phe Ile Arg Gln Ile Glu Val Asp Gly
65                  70                  75                  80
Gln Leu Ile Thr Leu Glu Ser Gly Glu Phe Gln Val Tyr Lys Gln Ser
                85                  90                  95
His Ser Ala Leu Thr Ala Phe Gln Thr Glu Gln Ile Gln Asp Ser Glu
            100                 105                 110
His Ser Gly Lys Met Val Ala Lys Arg Gln Phe Arg Ile Gly Asp Ile
        115                 120                 125
Ala Gly Glu His Thr Ser Phe Asp Lys Leu Pro Glu Gly Gly Arg Ala
    130                 135                 140
Thr Tyr Arg Gly Thr Ala Phe Gly Ser Asp Asp Ala Gly Gly Lys Leu
145                 150                 155                 160
Thr Tyr Thr Ile Asp Phe Ala Ala Lys Gln Gly Asn Gly Lys Ile Glu
                165                 170                 175
His Leu Lys Ser Pro Glu Leu Asn Val Asp Leu Ala Ala Ala Asp Ile
            180                 185                 190
Lys Pro Asp Gly Lys Arg His Ala Val Ile Ser Gly Ser Val Leu Tyr
        195                 200                 205
Asn Gln Ala Glu Lys Gly Ser Tyr Ser Leu Gly Ile Phe Gly Gly Lys
    210                 215                 220
Ala Gln Glu Val Ala Gly Ser Ala Glu Val Lys Thr Val Asn Gly Ile
225                 230                 235                 240
Arg His Ile Gly Leu Ala Ala Lys Gln Leu Glu Gly Gly Gly Gly Thr
                245                 250                 255
Gly Ser Ala Thr Asn Asp Asp Asp Val Lys Lys Ala Ala Thr Val Ala
            260                 265                 270
Ile Ala Ala Ala Tyr Asn Asn Gly Gln Glu Ile Asn Gly Phe Lys Ala
        275                 280                 285
Gly Glu Thr Ile Tyr Asp Ile Asp Glu Asp Gly Thr Ile Thr Lys Lys
    290                 295                 300
Asp Ala Thr Ala Ala Asp Val Glu Ala Asp Asp Phe Lys Gly Leu Gly
305                 310                 315                 320
Leu Lys Lys Val Val Thr Asn Leu Thr Lys Thr Val Asn Glu Asn Lys
                325                 330                 335
```

```
Gln Asn Val Asp Ala Lys Val Lys Ala Ala Glu Ser Glu Ile Glu Lys
            340                 345                 350

Leu Thr Thr Lys Leu Ala Asp Thr Asp Ala Ala Leu Ala Asp Thr Asp
        355                 360                 365

Ala Ala Leu Asp Ala Thr Thr Asn Ala Leu Asn Lys Leu Gly Glu Asn
    370                 375                 380

Ile Thr Thr Phe Ala Glu Glu Thr Lys Thr Asn Ile Val Lys Ile Asp
385                 390                 395                 400

Glu Lys Leu Glu Ala Val Ala Asp Thr Val Asp Lys His Ala Glu Ala
                405                 410                 415

Phe Asn Asp Ile Ala Asp Ser Leu Asp Glu Thr Asn Thr Lys Ala Asp
            420                 425                 430

Glu Ala Val Lys Thr Ala Asn Glu Ala Lys Gln Thr Ala Glu Glu Thr
        435                 440                 445

Lys Gln Asn Val Asp Ala Lys Val Lys Ala Ala Glu Thr Ala Ala Gly
    450                 455                 460

Lys Ala Glu Ala Ala Ala Gly Thr Ala Asn Thr Ala Ala Asp Lys Ala
465                 470                 475                 480

Glu Ala Val Ala Ala Lys Val Thr Asp Ile Lys Ala Asp Ile Ala Thr
                485                 490                 495

Asn Lys Asp Asn Ile Ala Lys Lys Ala Asn Ser Ala Asp Val Tyr Thr
            500                 505                 510

Arg Glu Glu Ser Asp Ser Lys Phe Val Arg Ile Asp Gly Leu Asn Ala
        515                 520                 525

Thr Thr Glu Lys Leu Asp Thr Arg Leu Ala Ser Ala Glu Lys Ser Ile
    530                 535                 540

Ala Asp His Asp Thr Arg Leu Asn Gly Leu Asp Lys Thr Val Ser Asp
545                 550                 555                 560

Leu Arg Lys Glu Thr Arg Gln Gly Leu Ala Glu Gln Ala Ala Leu Ser
                565                 570                 575

Gly Leu Phe Gln Pro Tyr Asn Val Gly Arg Phe Asn Val Thr Ala Ala
            580                 585                 590

Val Gly Gly Tyr Lys Ser Glu Ser Ala Val Ala Ile Gly Thr Gly Phe
        595                 600                 605

Arg Phe Thr Glu Asn Phe Ala Ala Lys Ala Gly Val Ala Val Gly Thr
    610                 615                 620

Ser Ser Gly Ser Ser Ala Ala Tyr His Val Gly Val Asn Tyr Glu Trp
625                 630                 635                 640

Leu Glu His His His His His His
                645
```

<210> 29
<211> 1782
<212> DNA
<213> Artificial Sequence

<220>
<223> deltaG741-961c

<400> 29

```
atggtcgccg ccgacatcgg tgcggggctt gccgatgcac taaccgcacc gctcgaccat    60
aaagacaaag gtttgcagtc tttgacgctg gatcagtccg tcaggaaaaa cgagaaactg   120
aagctggcgg cacaaggtgc ggaaaaaact tatggaaacg gtgacagcct caatacgggc   180
aaattgaaga acgacaaggt cagccgtttc gactttatcc gccaaatcga agtggacggg   240
cagctcatta ccttggagag tggagagttc caagtataca aacaaagcca ttccgcctta   300
accgcctttc agaccgagca aatacaagat tcggagcatt ccgggaagat ggttgcgaaa   360
cgccagttca gaatcggcga catagcgggc gaacatacat cttttgacaa gcttcccgaa   420
ggcggcaggg cgacatatcg cgggacggcg ttcggttcag acgatgccgg cggaaaactg   480
acctacacca tagatttcgc cgccaagcag ggaaacggca aaatcgaaca tttgaaatcg   540
ccagaactca atgtcgacct ggccgccgcc gatatcaagc cggatggaaa acgccatgcc   600
gtcatcagcg gttccgtcct ttacaaccaa gccgagaaag gcagttactc cctcggtatc   660
tttggcggaa aagcccagga agttgccggc agcgcggaag tgaaaaccgt aaacggcata   720
cgccatatcg gccttgccgc caagcaactc gagggtggcg gaggcactgg atccgccaca   780
aacgacgacg atgttaaaaa agctgccact gtggccattg ctgctgccta caacaatggc   840
caagaaatca acggtttcaa agctggagag accatctacg acattgatga agacggcaca   900
attaccaaaa aagacgcaac tgcagccgat gttgaagccg acgactttaa aggtctgggt   960
ctgaaaaaag tcgtgactaa cctgaccaaa accgtcaatg aaaacaaaca aaacgtcgat  1020
gccaaagtaa aagctgcaga atctgaaata gaaaagttaa caaccaagtt agcagacact  1080
gatgccgctt tagcagatac tgatgccgct ctggatgcaa ccaccaacgc cttgaataaa  1140
ttgggagaaa atataacgac atttgctgaa gagactaaga caaatatcgt aaaaattgat  1200
gaaaaattag aagccgtggc tgataccgtc gacaagcatg ccgaagcatt caacgatatc  1260
gccgattcat tggatgaaac caacactaag gcagacgaag ccgtcaaaac cgccaatgaa  1320
gccaaacaga cggccgaaga aaccaaacaa aacgtcgatg ccaaagtaaa agctgcagaa  1380
actgcagcag gcaaagccga agctgccgct ggcacagcta atactgcagc cgacaaggcc  1440
gaagctgtcg ctgcaaaagt taccgacatc aaagctgata tcgctacgaa caaagataat  1500
attgctaaaa aagcaaacag tgccgacgtg tacaccagag aagagtctga cagcaaattt  1560
gtcagaattg atggtctgaa cgctactacc gaaaaattgg acacacgctt ggcttctgct  1620
gaaaaatcca ttgccgatca cgatactcgc ctgaacggtt tggataaaac agtgtcagac  1680
ctgcgcaaag aaacccgcca aggccttgca gaacaagccg cgctctccgg tctgttccaa  1740
ccttacaacg tgggtctcga gcaccaccac caccaccact ga                     1782
```

<210> 30
<211> 593
<212> PRT
<213> Artificial Sequence

<220>
<223> deltaG741-961c

<400> 30

Met Val Ala Ala Asp Ile Gly Ala Gly Leu Ala Asp Ala Leu Thr Ala
1               5                   10                  15

Pro Leu Asp His Lys Asp Lys Gly Leu Gln Ser Leu Thr Leu Asp Gln
            20                  25                  30

Ser Val Arg Lys Asn Glu Lys Leu Lys Leu Ala Ala Gln Gly Ala Glu
        35                  40                  45

Lys Thr Tyr Gly Asn Gly Asp Ser Leu Asn Thr Gly Lys Leu Lys Asn
    50                  55                  60

Asp Lys Val Ser Arg Phe Asp Phe Ile Arg Gln Ile Glu Val Asp Gly
65 70 75 80

Gln Leu Ile Thr Leu Glu Ser Gly Glu Phe Gln Val Tyr Lys Gln Ser
85 90 95

His Ser Ala Leu Thr Ala Phe Gln Thr Glu Gln Ile Gln Asp Ser Glu
100 105 110

His Ser Gly Lys Met Val Ala Lys Arg Gln Phe Arg Ile Gly Asp Ile
115 120 125

Ala Gly Glu His Thr Ser Phe Asp Lys Leu Pro Glu Gly Gly Arg Ala
130 135 140

Thr Tyr Arg Gly Thr Ala Phe Gly Ser Asp Asp Ala Gly Gly Lys Leu
145 150 155 160

Thr Tyr Thr Ile Asp Phe Ala Ala Lys Gln Gly Asn Gly Lys Ile Glu
165 170 175

His Leu Lys Ser Pro Glu Leu Asn Val Asp Leu Ala Ala Ala Asp Ile
180 185 190

Lys Pro Asp Gly Lys Arg His Ala Val Ile Ser Gly Ser Val Leu Tyr
195 200 205

Asn Gln Ala Glu Lys Gly Ser Tyr Ser Leu Gly Ile Phe Gly Gly Lys
210 215 220

Ala Gln Glu Val Ala Gly Ser Ala Glu Val Lys Thr Val Asn Gly Ile
225 230 235 240

Arg His Ile Gly Leu Ala Ala Lys Gln Leu Glu Gly Gly Gly Gly Thr
245 250 255

Gly Ser Ala Thr Asn Asp Asp Asp Val Lys Lys Ala Ala Thr Val Ala
260 265 270

Ile Ala Ala Ala Tyr Asn Asn Gly Gln Glu Ile Asn Gly Phe Lys Ala
275 280 285

Gly Glu Thr Ile Tyr Asp Ile Asp Glu Asp Gly Thr Ile Thr Lys Lys
290 295 300

Asp Ala Thr Ala Ala Asp Val Glu Ala Asp Asp Phe Lys Gly Leu Gly
305 310 315 320

Leu Lys Lys Val Val Thr Asn Leu Thr Lys Thr Val Asn Glu Asn Lys
325 330 335

Gln Asn Val Asp Ala Lys Val Lys Ala Ala Glu Ser Glu Ile Glu Lys
340 345 350

Leu Thr Thr Lys Leu Ala Asp Thr Asp Ala Ala Leu Ala Asp Thr Asp
355 360 365

Ala Ala Leu Asp Ala Thr Thr Asn Ala Leu Asn Lys Leu Gly Glu Asn
370 375 380

Ile Thr Thr Phe Ala Glu Glu Thr Lys Thr Asn Ile Val Lys Ile Asp

385 390 395 400

Glu Lys Leu Glu Ala Val Ala Asp Thr Val Asp Lys His Ala Glu Ala
405 410 415

Phe Asn Asp Ile Ala Asp Ser Leu Asp Glu Thr Asn Thr Lys Ala Asp
420 425 430

Glu Ala Val Lys Thr Ala Asn Glu Ala Lys Gln Thr Ala Glu Glu Thr
435 440 445

Lys Gln Asn Val Asp Ala Lys Val Lys Ala Ala Glu Thr Ala Ala Gly
450 455 460

Lys Ala Glu Ala Ala Ala Gly Thr Ala Asn Thr Ala Ala Asp Lys Ala
465 470 475 480

Glu Ala Val Ala Ala Lys Val Thr Asp Ile Lys Ala Asp Ile Ala Thr
485 490 495

Asn Lys Asp Asn Ile Ala Lys Lys Ala Asn Ser Ala Asp Val Tyr Thr
500 505 510

Arg Glu Glu Ser Asp Ser Lys Phe Val Arg Ile Asp Gly Leu Asn Ala
515 520 525

Thr Thr Glu Lys Leu Asp Thr Arg Leu Ala Ser Ala Glu Lys Ser Ile
530 535 540

Ala Asp His Asp Thr Arg Leu Asn Gly Leu Asp Lys Thr Val Ser Asp
545 550 555 560

Leu Arg Lys Glu Thr Arg Gln Gly Leu Ala Glu Gln Ala Ala Leu Ser
565 570 575

Gly Leu Phe Gln Pro Tyr Asn Val Gly Leu Glu His His His His His
580 585 590

His

<210> 31
<211> 3939
<212> DNA
<213> Artificial Sequence

<220>
<223> deltaG741-983

<400> 31

```
atggtcgccg ccgacatcgg tgcggggctt gccgatgcac taaccgcacc gctcgaccat   60
aaagacaaag gtttgcagtc tttgacgctg gatcagtccg tcaggaaaaa cgagaaactg   120
aagctggcgg cacaaggtgc ggaaaaaact tatggaaacg gtgacagcct caatacgggc   180
aaattgaaga acgacaaggt cagccgtttc gactttatcc gccaaatcga agtggacggg   240
cagctcatta ccttggagag tggagagttc caagtataca aacaaagcca ttccgcctta   300
accgcctttc agaccgagca aatacaagat tcggagcatt ccgggaagat ggttgcgaaa   360
cgccagttca gaatcggcga catagcgggc gaacatacat cttttgacaa gcttcccgaa   420
ggcggcaggg cgacatatcg cgggacggcg ttcggttcag acgatgccgg cggaaaactg   480
acctacacca tagatttcgc cgccaagcag ggaaacggca aaatcgaaca tttgaaatcg   540
ccagaactca atgtcgacct ggccgccgcc gatatcaagc cggatggaaa acgccatgcc   600
gtcatcagcg gttccgtcct ttacaaccaa gccgagaaag gcagttactc cctcggtatc   660
```

tttggcggaa aagcccagga agttgccggc agcgcggaag tgaaaaccgt aaacggcata 720
cgccatatcg gccttgccgc caagcaactc gagggatccg gcggaggcgg cacttctgcg 780
cccgacttca atgcaggcgg taccggtatc ggcagcaaca gcagagcaac aacagcgaaa 840
tcagcagcag tatcttacgc cggtatcaag aacgaaatgt gcaaagacag aagcatgctc 900
tgtgccggtc gggatgacgt tgcggttaca gacagggatg ccaaaatcaa tgcccccccc 960
ccgaatctgc ataccggaga ctttccaaac ccaaatgacg catacaagaa tttgatcaac 1020
ctcaaacctg caattgaagc aggctataca ggacgcgggg tagaggtagg tatcgtcgac 1080
acaggcgaat ccgtcggcag catatccttt cccgaactgt atggcagaaa agaacacggc 1140
tataacgaaa attacaaaaa ctatacggcg tatatgcgga aggaagcgcc tgaagacgga 1200
ggcggtaaag acattgaagc ttctttcgac gatgaggccg ttatagagac tgaagcaaag 1260
ccgacggata tccgccacgt aaaagaaatc ggacacatcg atttggtctc ccatattatt 1320
ggcgggcgtt ccgtggacgg cagacctgca ggcggtattg cgcccgatgc gacgctacac 1380
ataatgaata cgaatgatga aaccaagaac gaaatgatgg ttgcagccat ccgcaatgca 1440
tgggtcaagc tgggcgaacg tggcgtgcgc atcgtcaata acagtttttgg aacaacatcg 1500
agggcaggca ctgccgacct tttccaaata gccaattcgg aggagcagta ccgccaagcg 1560
ttgctcgact attccggcgg tgataaaaca gacgagggta tccgcctgat gcaacagagc 1620
gattacggca acctgtccta ccacatccgt aataaaaaca tgcttttcat cttttcgaca 1680
ggcaatgacg cacaagctca gcccaacaca tatgccctat tgccatttta tgaaaaagac 1740
gctcaaaaag gcattatcac agtcgcaggc gtagaccgca gtggagaaaa gttcaaacgg 1800
gaaatgtatg gagaaccggg tacagaaccg cttgagtatg gctccaacca ttgcggaatt 1860
actgccatgt ggtgcctgtc ggcaccctat gaagcaagcg tccgtttcac ccgtacaaac 1920
ccgattcaaa ttgccggaac atccttttcc gcacccatcg taaccggcac ggcggctctg 1980
ctgctgcaga aatacccgtg gatgagcaac gacaacctgc gtaccacgtt gctgacgacg 2040
gctcaggaca tcggtgcagt cggcgtggac agcaagttcg gctggggact gctggatgcg 2100
ggtaaggcca tgaacggacc cgcgtccttt ccgttcggcg actttaccgc cgatacgaaa 2160
ggtacatccg atattgccta ctccttccgt aacgacattt caggcacggg cggcctgatc 2220
aaaaaaggcg gcagccaact gcaactgcac ggcaacaaca cctatacggg caaaaccatt 2280
atcgaaggcg gttcgctggt gttgtacggc aacaacaaat cggatatgcg cgtcgaaacc 2340
aaaggtgcgc tgatttataa cggggcggca tccggcggca gcctgaacag cgacggcatt 2400
gtctatctgg cagataccga ccaatccggc gcaaacgaaa ccgtacacat caaaggcagt 2460
ctgcagctgg acggcaaagg tacgctgtac acacgtttgg gcaaactgct gaaagtggac 2520
ggtacggcga ttatcggcgg caagctgtac atgtcggcac gcggcaaggg ggcaggctat 2580
ctcaacagta ccggacgacg tgttcccttc ctgagtgccg ccaaaatcgg gcaggattat 2640
tctttcttca caaacatcga aaccgacggc ggcctgctgg cttccctcga cagcgtcgaa 2700
aaaacagcgg gcagtgaagg cgacacgctg tcctattatg tccgtcgcgg caatgcggca 2760
cggactgctt cggcagcggc acattccgcg cccgccggtc tgaaacacgc cgtagaacag 2820
ggcggcagca atctggaaaa cctgatggtc gaactggatg cctccgaatc atccgcaaca 2880
cccgagacgg ttgaaactgc ggcagccgac cgcacagata tgccgggcat ccgcccctac 2940
ggcgcaactt tccgcgcagc ggcagccgta cagcatgcga atgccgccga cggtgtacgc 3000
atcttcaaca gtctcgccgc taccgtctat gccgacagta ccgccgccca tgccgatatg 3060
cagggacgcc gcctgaaagc cgtatcggac gggttggacc acaacggcac gggtctgcgc 3120
gtcatcgcgc aaacccaaca ggacggtgga acgtgggaac agggcggtgt tgaaggcaaa 3180
atgcgcggca gtacccaaac cgtcggcatt gccgcgaaaa ccggcgaaaa tacgacagca 3240
gccgccacac tgggcatggg acgcagcaca tggagcgaaa acagtgcaaa tgcaaaaacc 3300
gacagcatta gtctgtttgc aggcatacgg cacgatgcgg gcgatatcgg ctatctcaaa 3360
ggcctgttct cctacggacg ctacaaaaac agcatcagcc gcagcaccgg tgcggacgaa 3420
catgcggaag gcagcgtcaa cggcacgctg atgcagctgg gcgcactggg cggtgtcaac 3480
gttccgtttg ccgcaacggg agatttgacg gtcgaaggcg gtctgcgcta cgacctgctc 3540
aaacaggatg cattcgccga aaaaggcagt gctttgggct ggagcggcaa cagcctcact 3600
gaaggcacgc tggtcggact cgcgggtctg aagctgtcgc aaccccttgag cgataaagcc 3660
gtcctgtttg caacggcggg cgtggaacgc gacctgaacg gacgcgacta cacggtaacg 3720
ggcggcttta ccggcgcgac tgcagcaacc ggcaagacgg gggcacgcaa tatgccgcac 3780
acccgtctgg ttgccggcct gggcgcggat gtcgaattcg gcaacggctg gaacggcttg 3840
gcacgttaca gctacgccgg ttccaaacag tacggcaacc acagcggacg agtcggcgta 3900
ggctaccggt tcctcgagca ccaccaccac caccactga 3939

<210> 32
<211> 1312

<212> PRT
<213> Artificial Sequence

<220>
<223> deltaG741-983

<400> 32

```
Met Val Ala Ala Asp Ile Gly Ala Gly Leu Ala Asp Ala Leu Thr Ala
1               5                   10                  15

Pro Leu Asp His Lys Asp Lys Gly Leu Gln Ser Leu Thr Leu Asp Gln
            20                  25                  30

Ser Val Arg Lys Asn Glu Lys Leu Lys Leu Ala Ala Gln Gly Ala Glu
        35                  40                  45

Lys Thr Tyr Gly Asn Gly Asp Ser Leu Asn Thr Gly Lys Leu Lys Asn
    50                  55                  60

Asp Lys Val Ser Arg Phe Asp Phe Ile Arg Gln Ile Glu Val Asp Gly
65                  70                  75                  80

Gln Leu Ile Thr Leu Glu Ser Gly Glu Phe Gln Val Tyr Lys Gln Ser
                85                  90                  95

His Ser Ala Leu Thr Ala Phe Gln Thr Glu Gln Ile Gln Asp Ser Glu
            100                 105                 110

His Ser Gly Lys Met Val Ala Lys Arg Gln Phe Arg Ile Gly Asp Ile
        115                 120                 125

Ala Gly Glu His Thr Ser Phe Asp Lys Leu Pro Glu Gly Gly Arg Ala
    130                 135                 140

Thr Tyr Arg Gly Thr Ala Phe Gly Ser Asp Asp Ala Gly Gly Lys Leu
145                 150                 155                 160

Thr Tyr Thr Ile Asp Phe Ala Ala Lys Gln Gly Asn Gly Lys Ile Glu
                165                 170                 175

His Leu Lys Ser Pro Glu Leu Asn Val Asp Leu Ala Ala Ala Asp Ile
            180                 185                 190

Lys Pro Asp Gly Lys Arg His Ala Val Ile Ser Gly Ser Val Leu Tyr
        195                 200                 205

Asn Gln Ala Glu Lys Gly Ser Tyr Ser Leu Gly Ile Phe Gly Gly Lys
    210                 215                 220

Ala Gln Glu Val Ala Gly Ser Ala Glu Val Lys Thr Val Asn Gly Ile
225                 230                 235                 240

Arg His Ile Gly Leu Ala Ala Lys Gln Leu Glu Gly Ser Gly Gly Gly
                245                 250                 255

Gly Thr Ser Ala Pro Asp Phe Asn Ala Gly Gly Thr Gly Ile Gly Ser
            260                 265                 270

Asn Ser Arg Ala Thr Thr Ala Lys Ser Ala Ala Val Ser Tyr Ala Gly
        275                 280                 285

Ile Lys Asn Glu Met Cys Lys Asp Arg Ser Met Leu Cys Ala Gly Arg
    290                 295                 300
```

Asp Asp Val Ala Val Thr Asp Arg Asp Ala Lys Ile Asn Ala Pro Pro
305 310 315 320

Pro Asn Leu His Thr Gly Asp Phe Pro Asn Pro Asn Asp Ala Tyr Lys
325 330 335

Asn Leu Ile Asn Leu Lys Pro Ala Ile Glu Ala Gly Tyr Thr Gly Arg
340 345 350

Gly Val Glu Val Gly Ile Val Asp Thr Gly Glu Ser Val Gly Ser Ile
355 360 365

Ser Phe Pro Glu Leu Tyr Gly Arg Lys Glu His Gly Tyr Asn Glu Asn
370 375 380

Tyr Lys Asn Tyr Thr Ala Tyr Met Arg Lys Glu Ala Pro Glu Asp Gly
385 390 395 400

Gly Gly Lys Asp Ile Glu Ala Ser Phe Asp Asp Glu Ala Val Ile Glu
405 410 415

Thr Glu Ala Lys Pro Thr Asp Ile Arg His Val Lys Glu Ile Gly His
420 425 430

Ile Asp Leu Val Ser His Ile Ile Gly Gly Arg Ser Val Asp Gly Arg
435 440 445

Pro Ala Gly Gly Ile Ala Pro Asp Ala Thr Leu His Ile Met Asn Thr
450 455 460

Asn Asp Glu Thr Lys Asn Glu Met Met Val Ala Ala Ile Arg Asn Ala
465 470 475 480

Trp Val Lys Leu Gly Glu Arg Gly Val Arg Ile Val Asn Asn Ser Phe
485 490 495

Gly Thr Thr Ser Arg Ala Gly Thr Ala Asp Leu Phe Gln Ile Ala Asn
500 505 510

Ser Glu Glu Gln Tyr Arg Gln Ala Leu Leu Asp Tyr Ser Gly Gly Asp
515 520 525

Lys Thr Asp Glu Gly Ile Arg Leu Met Gln Gln Ser Asp Tyr Gly Asn
530 535 540

Leu Ser Tyr His Ile Arg Asn Lys Asn Met Leu Phe Ile Phe Ser Thr
545 550 555 560

Gly Asn Asp Ala Gln Ala Gln Pro Asn Thr Tyr Ala Leu Leu Pro Phe
565 570 575

Tyr Glu Lys Asp Ala Gln Lys Gly Ile Ile Thr Val Ala Gly Val Asp
580 585 590

Arg Ser Gly Glu Lys Phe Lys Arg Glu Met Tyr Gly Glu Pro Gly Thr
595 600 605

Glu Pro Leu Glu Tyr Gly Ser Asn His Cys Gly Ile Thr Ala Met Trp
610 615 620

Cys Leu Ser Ala Pro Tyr Glu Ala Ser Val Arg Phe Thr Arg Thr Asn

```
625                 630                 635                 640

Pro Ile Gln Ile Ala Gly Thr Ser Phe Ser Ala Pro Ile Val Thr Gly
                645                 650                 655

Thr Ala Ala Leu Leu Leu Gln Lys Tyr Pro Trp Met Ser Asn Asp Asn
            660                 665                 670

Leu Arg Thr Thr Leu Leu Thr Thr Ala Gln Asp Ile Gly Ala Val Gly
        675                 680                 685

Val Asp Ser Lys Phe Gly Trp Gly Leu Leu Asp Ala Gly Lys Ala Met
    690                 695                 700

Asn Gly Pro Ala Ser Phe Pro Phe Gly Asp Phe Thr Ala Asp Thr Lys
705                 710                 715                 720

Gly Thr Ser Asp Ile Ala Tyr Ser Phe Arg Asn Asp Ile Ser Gly Thr
                725                 730                 735

Gly Gly Leu Ile Lys Lys Gly Gly Ser Gln Leu Gln Leu His Gly Asn
            740                 745                 750

Asn Thr Tyr Thr Gly Lys Thr Ile Ile Glu Gly Gly Ser Leu Val Leu
        755                 760                 765

Tyr Gly Asn Asn Lys Ser Asp Met Arg Val Glu Thr Lys Gly Ala Leu
    770                 775                 780

Ile Tyr Asn Gly Ala Ala Ser Gly Gly Ser Leu Asn Ser Asp Gly Ile
785                 790                 795                 800

Val Tyr Leu Ala Asp Thr Asp Gln Ser Gly Ala Asn Glu Thr Val His
                805                 810                 815

Ile Lys Gly Ser Leu Gln Leu Asp Gly Lys Gly Thr Leu Tyr Thr Arg
            820                 825                 830

Leu Gly Lys Leu Leu Lys Val Asp Gly Thr Ala Ile Ile Gly Gly Lys
        835                 840                 845

Leu Tyr Met Ser Ala Arg Gly Lys Gly Ala Gly Tyr Leu Asn Ser Thr
    850                 855                 860

Gly Arg Arg Val Pro Phe Leu Ser Ala Ala Lys Ile Gly Gln Asp Tyr
865                 870                 875                 880

Ser Phe Phe Thr Asn Ile Glu Thr Asp Gly Gly Leu Leu Ala Ser Leu
                885                 890                 895

Asp Ser Val Glu Lys Thr Ala Gly Ser Glu Gly Asp Thr Leu Ser Tyr
            900                 905                 910

Tyr Val Arg Arg Gly Asn Ala Ala Arg Thr Ala Ser Ala Ala Ala His
        915                 920                 925

Ser Ala Pro Ala Gly Leu Lys His Ala Val Glu Gln Gly Gly Ser Asn
    930                 935                 940

Leu Glu Asn Leu Met Val Glu Leu Asp Ala Ser Glu Ser Ser Ala Thr
945                 950                 955                 960
```

```
Pro Glu Thr Val Glu Thr Ala Ala Ala Asp Arg Thr Asp Met Pro Gly
                965                 970                 975

Ile Arg Pro Tyr Gly Ala Thr Phe Arg Ala Ala Ala Ala Val Gln His
            980                 985                 990

Ala Asn Ala Ala Asp Gly Val Arg Ile Phe Asn Ser Leu Ala Ala Thr
        995                 1000                1005

Val Tyr Ala Asp Ser Thr Ala Ala His Ala Asp Met Gln Gly Arg Arg
    1010                1015                1020

Leu Lys Ala Val Ser Asp Gly Leu Asp His Asn Gly Thr Gly Leu Arg
1025                1030                1035                1040

Val Ile Ala Gln Thr Gln Gln Asp Gly Gly Thr Trp Glu Gln Gly Gly
                1045                1050                1055

Val Glu Gly Lys Met Arg Gly Ser Thr Gln Thr Val Gly Ile Ala Ala
            1060                1065                1070

Lys Thr Gly Glu Asn Thr Thr Ala Ala Ala Thr Leu Gly Met Gly Arg
        1075                1080                1085

Ser Thr Trp Ser Glu Asn Ser Ala Asn Ala Lys Thr Asp Ser Ile Ser
    1090                1095                1100

Leu Phe Ala Gly Ile Arg His Asp Ala Gly Asp Ile Gly Tyr Leu Lys
1105                1110                1115                1120

Gly Leu Phe Ser Tyr Gly Arg Tyr Lys Asn Ser Ile Ser Arg Ser Thr
                1125                1130                1135

Gly Ala Asp Glu His Ala Glu Gly Ser Val Asn Gly Thr Leu Met Gln
            1140                1145                1150

Leu Gly Ala Leu Gly Gly Val Asn Val Pro Phe Ala Ala Thr Gly Asp
        1155                1160                1165

Leu Thr Val Glu Gly Gly Leu Arg Tyr Asp Leu Leu Lys Gln Asp Ala
    1170                1175                1180

Phe Ala Glu Lys Gly Ser Ala Leu Gly Trp Ser Gly Asn Ser Leu Thr
1185                1190                1195                1200

Glu Gly Thr Leu Val Gly Leu Ala Gly Leu Lys Leu Ser Gln Pro Leu
                1205                1210                1215

Ser Asp Lys Ala Val Leu Phe Ala Thr Ala Gly Val Glu Arg Asp Leu
            1220                1225                1230

Asn Gly Arg Asp Tyr Thr Val Thr Gly Gly Phe Thr Gly Ala Thr Ala
        1235                1240                1245

Ala Thr Gly Lys Thr Gly Ala Arg Asn Met Pro His Thr Arg Leu Val
    1250                1255                1260

Ala Gly Leu Gly Ala Asp Val Glu Phe Gly Asn Gly Trp Asn Gly Leu
1265                1270                1275                1280
```

```
Ala Arg Tyr Ser Tyr Ala Gly Ser Lys Gln Tyr Gly Asn His Ser Gly
                1285                1290                1295

Arg Val Gly Val Gly Tyr Arg Phe Leu Glu His His His His His His
            1300                1305                1310
```

<210> 33
<211> 2028
<212> DNA
<213> Artificial Sequence

<220>
<223> deltaG741-ORF46.1

<400> 33

```
atggtcgccg ccgacatcgg tgcggggctt gccgatgcac taaccgcacc gctcgaccat     60
aaagacaaag gtttgcagtc tttgacgctg gatcagtccg tcaggaaaaa cgagaaactg    120
aagctggcgg cacaaggtgc ggaaaaaact tatggaaacg gtgacagcct caatacgggc    180
aaattgaaga acgacaaggt cagccgtttc gactttatcc gccaaatcga agtggacggg    240
cagctcatta ccttggagag tggagagttc caagtataca aacaaagcca ttccgcctta    300
accgcctttc agaccgagca aatacaagat tcggagcatt ccgggaagat ggttgcgaaa    360
cgccagttca gaatcggcga catagcgggc gaacatacat cttttgacaa gcttcccgaa    420
ggcggcaggg cgacatatcg cgggacggcg ttcggttcag acgatgccgg cggaaaactg    480
acctacacca tagatttcgc cgccaagcag ggaaacggca aaatcgaaca tttgaaatcg    540
ccagaactca atgtcgacct ggccgccgcc gatatcaagc cggatggaaa acgccatgcc    600
gtcatcagcg gttccgtcct ttacaaccaa gccgagaaag gcagttactc cctcggtatc    660
tttggcggaa aagcccagga agttgccggc agcgcggaag tgaaaaccgt aaacggcata    720
cgccatatcg gccttgccgc caagcaactc gacggtggcg gaggcactgg atcctcagat    780
ttggcaaacg attcttttat ccggcaggtt ctcgaccgtc agcatttcga acccgacggg    840
aaataccacc tattcggcag caggggggaa cttgccgagc gcagcggcca tatcggattg    900
ggaaaaatac aaagccatca gttgggcaac ctgatgattc aacaggcggc cattaaagga    960
aatatcggct acattgtccg cttttccgat cacgggcacg aagtccattc cccccttcgac   1020
aaccatgcct cacattccga ttctgatgaa gccggtagtc ccgttgacgg atttagcctt   1080
taccgcatcc attgggacgg atacgaacac catcccgccg acggctatga cgggccacag   1140
ggcggcggct atcccgctcc caaaggcgcg agggatatat acagctacga cataaaaggc   1200
gttgcccaaa atatccgcct caacctgacc gacaaccgca gcaccggaca acggcttgcc   1260
gaccgtttcc acaatgccgg tagtatgctg acgcaaggag taggcgacgg attcaaacgc   1320
gccacccgat acagccccga gctggacaga tcgggcaatg ccgccgaagc cttcaacggc   1380
actgcagata tcgttaaaaa catcatcggc gcggcaggag aaattgtcgg cgcaggcgat   1440
gccgtgcagg gcataagcga aggctcaaac attgctgtca tgcacggctt gggtctgctt   1500
tccaccgaaa acaagatggc gcgcatcaac gatttggcag atatggcgca actcaaagac   1560
tatgccgcag cagccatccg cgattgggca gtccaaaacc ccaatgccgc acaaggcata   1620
gaagccgtca gcaatatctt tatggcagcc atccccatca aagggattgg agctgttcgg   1680
ggaaaatacg gcttgggcgg catcacggca catcctatca agcggtcgca gatgggcgcg   1740
atcgcattgc cgaaagggaa atccgccgtc agcgacaatt ttgccgatgc ggcatacgcc   1800
aaatacccgt ccccttacca ttcccgaaat atccgttcaa acttggagca gcgttacggc   1860
aaagaaaaca tcacctcctc aaccgtgccg ccgtcaaacg gcaaaaatgt caaactggca   1920
gaccaacgcc acccgaagac aggcgtaccg tttgacggta aagggtttcc gaattttgag   1980
aagcacgtga aatatgatac gctcgagcac caccaccacc accactga                2028
```

<210> 34
<211> 675
<212> PRT
<213> Artificial Sequence

<220>
<223> deltaG741-ORF46.1

<400> 34

Met Val Ala Ala Asp Ile Gly Ala Gly Leu Ala Asp Ala Leu Thr Ala

```
1               5                   10                  15
Pro Leu Asp His Lys Asp Lys Gly Leu Gln Ser Leu Thr Leu Asp Gln
            20                  25                  30
Ser Val Arg Lys Asn Glu Lys Leu Lys Leu Ala Ala Gln Gly Ala Glu
        35                  40                  45
Lys Thr Tyr Gly Asn Gly Asp Ser Leu Asn Thr Gly Lys Leu Lys Asn
    50                  55                  60
Asp Lys Val Ser Arg Phe Asp Phe Ile Arg Gln Ile Glu Val Asp Gly
65                  70                  75                  80
Gln Leu Ile Thr Leu Glu Ser Gly Glu Phe Gln Val Tyr Lys Gln Ser
                85                  90                  95
His Ser Ala Leu Thr Ala Phe Gln Thr Glu Gln Ile Gln Asp Ser Glu
            100                 105                 110
His Ser Gly Lys Met Val Ala Lys Arg Gln Phe Arg Ile Gly Asp Ile
        115                 120                 125
Ala Gly Glu His Thr Ser Phe Asp Lys Leu Pro Glu Gly Gly Arg Ala
    130                 135                 140
Thr Tyr Arg Gly Thr Ala Phe Gly Ser Asp Asp Ala Gly Gly Lys Leu
145                 150                 155                 160
Thr Tyr Thr Ile Asp Phe Ala Ala Lys Gln Gly Asn Gly Lys Ile Glu
                165                 170                 175
His Leu Lys Ser Pro Glu Leu Asn Val Asp Leu Ala Ala Ala Asp Ile
            180                 185                 190
Lys Pro Asp Gly Lys Arg His Ala Val Ile Ser Gly Ser Val Leu Tyr
        195                 200                 205
Asn Gln Ala Glu Lys Gly Ser Tyr Ser Leu Gly Ile Phe Gly Gly Lys
    210                 215                 220
Ala Gln Glu Val Ala Gly Ser Ala Glu Val Lys Thr Val Asn Gly Ile
225                 230                 235                 240
Arg His Ile Gly Leu Ala Ala Lys Gln Leu Asp Gly Gly Gly Gly Thr
                245                 250                 255
Gly Ser Ser Asp Leu Ala Asn Asp Ser Phe Ile Arg Gln Val Leu Asp
            260                 265                 270
Arg Gln His Phe Glu Pro Asp Gly Lys Tyr His Leu Phe Gly Ser Arg
        275                 280                 285
Gly Glu Leu Ala Glu Arg Ser Gly His Ile Gly Leu Gly Lys Ile Gln
    290                 295                 300
Ser His Gln Leu Gly Asn Leu Met Ile Gln Gln Ala Ala Ile Lys Gly
305                 310                 315                 320
Asn Ile Gly Tyr Ile Val Arg Phe Ser Asp His Gly His Glu Val His
                325                 330                 335
```

Ser Pro Phe Asp Asn His Ala Ser His Ser Asp Ser Asp Glu Ala Gly
340 345 350

Ser Pro Val Asp Gly Phe Ser Leu Tyr Arg Ile His Trp Asp Gly Tyr
355 360 365

Glu His His Pro Ala Asp Gly Tyr Asp Gly Pro Gln Gly Gly Gly Tyr
370 375 380

Pro Ala Pro Lys Gly Ala Arg Asp Ile Tyr Ser Tyr Asp Ile Lys Gly
385 390 395 400

Val Ala Gln Asn Ile Arg Leu Asn Leu Thr Asp Asn Arg Ser Thr Gly
405 410 415

Gln Arg Leu Ala Asp Arg Phe His Asn Ala Gly Ser Met Leu Thr Gln
420 425 430

Gly Val Gly Asp Gly Phe Lys Arg Ala Thr Arg Tyr Ser Pro Glu Leu
435 440 445

Asp Arg Ser Gly Asn Ala Ala Glu Ala Phe Asn Gly Thr Ala Asp Ile
450 455 460

Val Lys Asn Ile Ile Gly Ala Ala Gly Glu Ile Val Gly Ala Gly Asp
465 470 475 480

Ala Val Gln Gly Ile Ser Glu Gly Ser Asn Ile Ala Val Met His Gly
485 490 495

Leu Gly Leu Leu Ser Thr Glu Asn Lys Met Ala Arg Ile Asn Asp Leu
500 505 510

Ala Asp Met Ala Gln Leu Lys Asp Tyr Ala Ala Ala Ala Ile Arg Asp
515 520 525

Trp Ala Val Gln Asn Pro Asn Ala Ala Gln Gly Ile Glu Ala Val Ser
530 535 540

Asn Ile Phe Met Ala Ala Ile Pro Ile Lys Gly Ile Gly Ala Val Arg
545 550 555 560

Gly Lys Tyr Gly Leu Gly Gly Ile Thr Ala His Pro Ile Lys Arg Ser
565 570 575

Gln Met Gly Ala Ile Ala Leu Pro Lys Gly Lys Ser Ala Val Ser Asp
580 585 590

Asn Phe Ala Asp Ala Ala Tyr Ala Lys Tyr Pro Ser Pro Tyr His Ser
595 600 605

Arg Asn Ile Arg Ser Asn Leu Glu Gln Arg Tyr Gly Lys Glu Asn Ile
610 615 620

Thr Ser Ser Thr Val Pro Pro Ser Asn Gly Lys Asn Val Lys Leu Ala
625 630 635 640

Asp Gln Arg His Pro Lys Thr Gly Val Pro Phe Asp Gly Lys Gly Phe
645 650 655

```
Pro Asn Phe Glu Lys His Val Lys Tyr Asp Thr Leu Glu His His His
            660                 665                 670

His His His
        675
```

<210> 35
<211> 2019
<212> DNA
<213> Artificial Sequence

<220>
<223> ORF46.1-741

<400> 35

```
atgtcagatt tggcaaacga ttcttttatc cggcaggttc tcgaccgtca gcatttcgaa    60
cccgacggga aataccacct attcggcagc agggggggaac ttgccgagcg cagcggccat   120
atcggattgg gaaaaataca aagccatcag ttgggcaacc tgatgattca acaggcggcc   180
attaaaggaa atatcggcta cattgtccgc ttttccgatc acgggcacga agtccattcc   240
cccttcgaca accatgcctc acattccgat tctgatgaag ccggtagtcc cgttgacgga   300
tttagccttt accgcatcca ttgggacgga tacgaacacc atcccgccga cggctatgac   360
gggccacagg gcggcggcta tcccgctccc aaaggcgcga gggatatata cagctacgac   420
ataaaaggcg ttgcccaaaa tatccgcctc aacctgaccg acaaccgcag caccggacaa   480
cggcttgccg accgtttcca caatgccggt agtatgctga cgcaaggagt aggcgacgga   540
ttcaaacgcg ccacccgata cagccccgag ctggacagat cgggcaatgc cgccgaagcc   600
ttcaacggca ctgcagatat cgttaaaaac atcatcggcg cggcaggaga aattgtcggc   660
gcaggcgatg ccgtgcaggg cataagcgaa ggctcaaaca ttgctgtcat gcacggcttg   720
ggtctgcttt ccaccgaaaa caagatggcg cgcatcaacg atttggcaga tatggcgcaa   780
ctcaaagact atgccgcagc agccatccgc gattgggcag tccaaaaccc caatgccgca   840
caaggcatag aagccgtcag caatatcttt atggcagcca tccccatcaa agggattgga   900
gctgttcggg gaaaatacgg cttgggcggc atcacggcac atcctatcaa gcggtcgcag   960
atgggcgcga tcgcattgcc gaaagggaaa tccgccgtca gcgacaattt tgccgatgcg  1020
gcatacgcca aatacccgtc cccttaccat tcccgaaata tccgttcaaa cttggagcag  1080
cgttacggca aagaaaacat cacctcctca accgtgccgc cgtcaaacgg caaaaatgtc  1140
aaactggcag accaacgcca cccgaagaca ggcgtaccgt ttgacggtaa agggtttccg  1200
aattttgaga agcacgtgaa atatgatacg ggatccggag gggtggtgt cgccgccgac  1260
atcggtgcgg ggcttgccga tgcactaacc gcaccgctcg accataaaga caaaggtttg  1320
cagtctttga cgctggatca gtccgtcagg aaaaacgaga aactgaagct ggcggcacaa  1380
ggtgcggaaa aaacttatgg aaacggtgac agcctcaata cgggcaaatt gaagaacgac  1440
aaggtcagcc gtttcgactt tatccgccaa atcgaagtgg acgggcagct cattaccttg  1500
gagagtggag agttccaagt atacaaacaa agccattccg ccttaaccgc ctttcagacc  1560
gagcaaatac aagattcgga gcattccggg aagatggttg cgaaacgcca gttcagaatc  1620
ggcgacatag cgggcgaaca tacatctttt gacaagcttc ccgaaggcgg cagggcgaca  1680
tatcgcggga cggcgttcgg ttcagacgat gccggcggaa aactgaccta caccatagat  1740
ttcgccgcca agcagggaaa cggcaaaatc gaacatttga aatcgccaga actcaatgtc  1800
gacctggccg ccgccgatat caagccggat ggaaaacgcc atgccgtcat cagcggttcc  1860
gtcctttaca accaagccga gaaaggcagt tactccctcg gtatctttgg cggaaaagcc  1920
caggaagttg ccggcagcgc ggaagtgaaa accgtaaacg gcatacgcca tatcggcctt  1980
gccgccaagc aactcgagca ccaccaccac caccactga                           2019
```

<210> 36
<211> 672
<212> PRT
<213> Artificial Sequence <220>

<223> ORF46.1-741

<400> 36

```
Met Ser Asp Leu Ala Asn Asp Ser Phe Ile Arg Gln Val Leu Asp Arg
1               5                   10                  15
```

```
Gln His Phe Glu Pro Asp Gly Lys Tyr His Leu Phe Gly Ser Arg Gly
                20                  25                  30

Glu Leu Ala Glu Arg Ser Gly His Ile Gly Leu Gly Lys Ile Gln Ser
        35                  40                  45

His Gln Leu Gly Asn Leu Met Ile Gln Gln Ala Ala Ile Lys Gly Asn
    50                  55                  60

Ile Gly Tyr Ile Val Arg Phe Ser Asp His Gly His Glu Val His Ser
65                  70                  75                  80

Pro Phe Asp Asn His Ala Ser His Ser Asp Ser Asp Glu Ala Gly Ser
                85                  90                  95

Pro Val Asp Gly Phe Ser Leu Tyr Arg Ile His Trp Asp Gly Tyr Glu
            100                 105                 110

His His Pro Ala Asp Gly Tyr Asp Gly Pro Gln Gly Gly Gly Tyr Pro
        115                 120                 125

Ala Pro Lys Gly Ala Arg Asp Ile Tyr Ser Tyr Asp Ile Lys Gly Val
    130                 135                 140

Ala Gln Asn Ile Arg Leu Asn Leu Thr Asp Asn Arg Ser Thr Gly Gln
145                 150                 155                 160

Arg Leu Ala Asp Arg Phe His Asn Ala Gly Ser Met Leu Thr Gln Gly
                165                 170                 175

Val Gly Asp Gly Phe Lys Arg Ala Thr Arg Tyr Ser Pro Glu Leu Asp
            180                 185                 190

Arg Ser Gly Asn Ala Ala Glu Ala Phe Asn Gly Thr Ala Asp Ile Val
        195                 200                 205

Lys Asn Ile Ile Gly Ala Ala Gly Glu Ile Val Gly Ala Gly Asp Ala
    210                 215                 220

Val Gln Gly Ile Ser Glu Gly Ser Asn Ile Ala Val Met His Gly Leu
225                 230                 235                 240

Gly Leu Leu Ser Thr Glu Asn Lys Met Ala Arg Ile Asn Asp Leu Ala
                245                 250                 255

Asp Met Ala Gln Leu Lys Asp Tyr Ala Ala Ala Ala Ile Arg Asp Trp
            260                 265                 270

Ala Val Gln Asn Pro Asn Ala Ala Gln Gly Ile Glu Ala Val Ser Asn
        275                 280                 285

Ile Phe Met Ala Ala Ile Pro Ile Lys Gly Ile Gly Ala Val Arg Gly
    290                 295                 300

Lys Tyr Gly Leu Gly Gly Ile Thr Ala His Pro Ile Lys Arg Ser Gln
305                 310                 315                 320

Met Gly Ala Ile Ala Leu Pro Lys Gly Lys Ser Ala Val Ser Asp Asn
                325                 330                 335
```

```
Phe Ala Asp Ala Ala Tyr Ala Lys Tyr Pro Ser Pro Tyr His Ser Arg
            340                 345                 350

Asn Ile Arg Ser Asn Leu Glu Gln Arg Tyr Gly Lys Glu Asn Ile Thr
        355                 360                 365

Ser Ser Thr Val Pro Pro Ser Asn Gly Lys Asn Val Lys Leu Ala Asp
    370                 375                 380

Gln Arg His Pro Lys Thr Gly Val Pro Phe Asp Gly Lys Gly Phe Pro
385                 390                 395                 400

Asn Phe Glu Lys His Val Lys Tyr Asp Thr Gly Ser Gly Gly Gly Gly
                405                 410                 415

Val Ala Ala Asp Ile Gly Ala Gly Leu Ala Asp Ala Leu Thr Ala Pro
            420                 425                 430

Leu Asp His Lys Asp Lys Gly Leu Gln Ser Leu Thr Leu Asp Gln Ser
        435                 440                 445

Val Arg Lys Asn Glu Lys Leu Lys Leu Ala Ala Gln Gly Ala Glu Lys
    450                 455                 460

Thr Tyr Gly Asn Gly Asp Ser Leu Asn Thr Gly Lys Leu Lys Asn Asp
465                 470                 475                 480

Lys Val Ser Arg Phe Asp Phe Ile Arg Gln Ile Glu Val Asp Gly Gln
                485                 490                 495

Leu Ile Thr Leu Glu Ser Gly Glu Phe Gln Val Tyr Lys Gln Ser His
            500                 505                 510

Ser Ala Leu Thr Ala Phe Gln Thr Glu Gln Ile Gln Asp Ser Glu His
        515                 520                 525

Ser Gly Lys Met Val Ala Lys Arg Gln Phe Arg Ile Gly Asp Ile Ala
    530                 535                 540

Gly Glu His Thr Ser Phe Asp Lys Leu Pro Glu Gly Gly Arg Ala Thr
545                 550                 555                 560

Tyr Arg Gly Thr Ala Phe Gly Ser Asp Asp Ala Gly Gly Lys Leu Thr
                565                 570                 575

Tyr Thr Ile Asp Phe Ala Ala Lys Gln Gly Asn Gly Lys Ile Glu His
            580                 585                 590

Leu Lys Ser Pro Glu Leu Asn Val Asp Leu Ala Ala Ala Asp Ile Lys
        595                 600                 605

Pro Asp Gly Lys Arg His Ala Val Ile Ser Gly Ser Val Leu Tyr Asn
    610                 615                 620

Gln Ala Glu Lys Gly Ser Tyr Ser Leu Gly Ile Phe Gly Gly Lys Ala
625                 630                 635                 640

Gln Glu Val Ala Gly Ser Ala Glu Val Lys Thr Val Asn Gly Ile Arg
                645                 650                 655

His Ile Gly Leu Ala Ala Lys Gln Leu Glu His His His His His His
```

660 665 670

<210> 37
<211> 2421
<212> DNA
<213> Artificial Sequence

<220>
<223> ORF46.1-961

<400> 37

```
atgtcagatt tggcaaacga ttcttttatc cggcaggttc tcgaccgtca gcatttcgaa   60
cccgacggga aataccacct attcggcagc agggggggaac ttgccgagcg cagcggccat  120
atcggattgg gaaaaataca aagccatcag ttgggcaacc tgatgattca acaggcggcc  180
attaaaggaa atatcggcta cattgtccgc ttttccgatc acgggcacga agtccattcc  240
cccttcgaca accatgcctc acattccgat tctgatgaag ccggtagtcc cgttgacgga  300
tttagccttt accgcatcca ttgggacgga tacgaacacc atcccgccga cggctatgac  360
gggccacagg gcggcggcta tcccgctccc aaaggcgcga gggatatata cagctacgac  420
ataaaaggcg ttgcccaaaa tatccgcctc aacctgaccg acaaccgcag caccggacaa  480
cggcttgccg accgtttcca caatgccggt agtatgctga cgcaaggagt aggcgacgga  540
ttcaaacgcg ccacccgata cagccccgag ctggacagat cgggcaatgc cgccgaagcc  600
ttcaacggca ctgcagatat cgttaaaaac atcatcggcg cggcaggaga aattgtcggc  660
gcaggcgatg ccgtgcaggg cataagcgaa ggctcaaaca ttgctgtcat gcacggcttg  720
ggtctgcttt ccaccgaaaa caagatggcg cgcatcaacg atttggcaga tatggcgcaa  780
ctcaaagact atgccgcagc agccatccgc gattgggcag tccaaaaccc caatgccgca  840
caaggcatag aagccgtcag caatatcttt atggcagcca tccccatcaa agggattgga  900
gctgttcggg gaaaatacgg cttgggcggc atcacggcac atcctatcaa gcggtcgcag  960
atgggcgcga tcgcattgcc gaaagggaaa tccgccgtca gcgacaattt tgccgatgcg 1020
gcatacgcca aatacccgtc cccttaccat tcccgaaata tccgttcaaa cttggagcag 1080
cgttacggca aagaaaacat cacctcctca accgtgccgc cgtcaaacgg caaaaatgtc 1140
aaactggcag accaacgcca cccgaagaca ggcgtaccgt ttgacggtaa agggtttccg 1200
aattttgaga agcacgtgaa atatgatacg ggatccggag gaggaggagc cacaaacgac 1260
gacgatgtta aaaaagctgc cactgtggcc attgctgctg cctacaacaa tggccaagaa 1320
atcaacggtt tcaaagctgg agagaccatc tacgacattg atgaagacgg cacaattacc 1380
aaaaaagacg caactgcagc cgatgttgaa gccgacgact ttaaaggtct gggtctgaaa 1440
aaagtcgtga ctaacctgac caaaaccgtc aatgaaaaca aacaaaacgt cgatgccaaa 1500
gtaaaagctg cagaatctga aatagaaaag ttaacaacca agttagcaga cactgatgcc 1560
gctttagcag atactgatgc cgctctggat gcaaccacca acgccttgaa taaattggga 1620
gaaaatataa cgacatttgc tgaagagact aagacaaata tcgtaaaaat tgatgaaaaa 1680
ttagaagccg tggctgatac cgtcgacaag catgccgaag cattcaacga tatcgccgat 1740
tcattggatg aaaccaacac taaggcagac gaagccgtca aaaccgccaa tgaagccaaa 1800
cagacggccg aagaaaccaa acaaaacgtc gatgccaaag taaaagctgc agaaactgca 1860
gcaggcaaag ccgaagctgc cgctggcaca gctaatactg cagccgacaa ggccgaagct 1920
gtcgctgcaa aagttaccga catcaaagct gatatcgcta cgaacaaaga taatattgct 1980
aaaaaagcaa acagtgccga cgtgtacacc agagaagagt ctgacagcaa atttgtcaga 2040
attgatggtc tgaacgctac taccgaaaaa ttggacacac gcttggcttc tgctgaaaaa 2100
tccattgccg atcacgatac tcgcctgaac ggtttggata aaacagtgtc agacctgcgc 2160
aaagaaaccc gccaaggcct tgcagaacaa gccgcgctct ccggtctgtt ccaaccttac 2220
aacgtgggtc ggttcaatgt aacggctgca gtcggcggct acaaatccga atcggcagtc 2280
gccatcggta ccggcttccg ctttaccgaa aactttgccg ccaaagcagg cgtggcagtc 2340
ggcacttcgt ccggttcttc cgcagcctac catgtcggcg tcaattacga gtggctcgag 2400
caccaccacc accaccactg a                                           2421
```

<210> 38
<211> 806

<212> PRT
<213> Artificial Sequence

<220>
<223> ORF46.1-961

<400> 38

Met Ser Asp Leu Ala Asn Asp Ser Phe Ile Arg Gln Val Leu Asp Arg
1               5                   10                  15

Gln His Phe Glu Pro Asp Gly Lys Tyr His Leu Phe Gly Ser Arg Gly
            20                  25                  30

Glu Leu Ala Glu Arg Ser Gly His Ile Gly Leu Gly Lys Ile Gln Ser
        35                  40                  45

His Gln Leu Gly Asn Leu Met Ile Gln Gln Ala Ala Ile Lys Gly Asn
    50                  55                  60

Ile Gly Tyr Ile Val Arg Phe Ser Asp His Gly His Glu Val His Ser
65                  70                  75                  80

Pro Phe Asp Asn His Ala Ser His Ser Asp Ser Asp Glu Ala Gly Ser
                85                  90                  95

Pro Val Asp Gly Phe Ser Leu Tyr Arg Ile His Trp Asp Gly Tyr Glu
            100                 105                 110

His His Pro Ala Asp Gly Tyr Asp Gly Pro Gln Gly Gly Gly Tyr Pro
        115                 120                 125

Ala Pro Lys Gly Ala Arg Asp Ile Tyr Ser Tyr Asp Ile Lys Gly Val
    130                 135                 140

Ala Gln Asn Ile Arg Leu Asn Leu Thr Asp Asn Arg Ser Thr Gly Gln
145                 150                 155                 160

Arg Leu Ala Asp Arg Phe His Asn Ala Gly Ser Met Leu Thr Gln Gly
                165                 170                 175

Val Gly Asp Gly Phe Lys Arg Ala Thr Arg Tyr Ser Pro Glu Leu Asp
            180                 185                 190

Arg Ser Gly Asn Ala Ala Glu Ala Phe Asn Gly Thr Ala Asp Ile Val
        195                 200                 205

Lys Asn Ile Ile Gly Ala Ala Gly Glu Ile Val Gly Ala Gly Asp Ala
    210                 215                 220

Val Gln Gly Ile Ser Glu Gly Ser Asn Ile Ala Val Met His Gly Leu
225                 230                 235                 240

Gly Leu Leu Ser Thr Glu Asn Lys Met Ala Arg Ile Asn Asp Leu Ala
                245                 250                 255

Asp Met Ala Gln Leu Lys Asp Tyr Ala Ala Ala Ala Ile Arg Asp Trp
            260                 265                 270

Ala Val Gln Asn Pro Asn Ala Ala Gln Gly Ile Glu Ala Val Ser Asn
        275                 280                 285

Ile Phe Met Ala Ala Ile Pro Ile Lys Gly Ile Gly Ala Val Arg Gly
    290                 295                 300

Lys Tyr Gly Leu Gly Gly Ile Thr Ala His Pro Ile Lys Arg Ser Gln
305                 310                 315                 320

Met Gly Ala Ile Ala Leu Pro Lys Gly Lys Ser Ala Val Ser Asp Asn
325 330 335

Phe Ala Asp Ala Ala Tyr Ala Lys Tyr Pro Ser Pro Tyr His Ser Arg
340 345 350

Asn Ile Arg Ser Asn Leu Glu Gln Arg Tyr Gly Lys Glu Asn Ile Thr
355 360 365

Ser Ser Thr Val Pro Pro Ser Asn Gly Lys Asn Val Lys Leu Ala Asp
370 375 380

Gln Arg His Pro Lys Thr Gly Val Pro Phe Asp Gly Lys Gly Phe Pro
385 390 395 400

Asn Phe Glu Lys His Val Lys Tyr Asp Thr Gly Ser Gly Gly Gly Gly
405 410 415

Ala Thr Asn Asp Asp Asp Val Lys Lys Ala Ala Thr Val Ala Ile Ala
420 425 430

Ala Ala Tyr Asn Asn Gly Gln Glu Ile Asn Gly Phe Lys Ala Gly Glu
435 440 445

Thr Ile Tyr Asp Ile Asp Glu Asp Gly Thr Ile Thr Lys Lys Asp Ala
450 455 460

Thr Ala Ala Asp Val Glu Ala Asp Asp Phe Lys Gly Leu Gly Leu Lys
465 470 475 480

Lys Val Val Thr Asn Leu Thr Lys Thr Val Asn Glu Asn Lys Gln Asn
485 490 495

Val Asp Ala Lys Val Lys Ala Ala Glu Ser Glu Ile Glu Lys Leu Thr
500 505 510

Thr Lys Leu Ala Asp Thr Asp Ala Ala Leu Ala Asp Thr Asp Ala Ala
515 520 525

Leu Asp Ala Thr Thr Asn Ala Leu Asn Lys Leu Gly Glu Asn Ile Thr
530 535 540

Thr Phe Ala Glu Glu Thr Lys Thr Asn Ile Val Lys Ile Asp Glu Lys
545 550 555 560

Leu Glu Ala Val Ala Asp Thr Val Asp Lys His Ala Glu Ala Phe Asn
565 570 575

Asp Ile Ala Asp Ser Leu Asp Glu Thr Asn Thr Lys Ala Asp Glu Ala
580 585 590

Val Lys Thr Ala Asn Glu Ala Lys Gln Thr Ala Glu Glu Thr Lys Gln
595 600 605

Asn Val Asp Ala Lys Val Lys Ala Ala Glu Thr Ala Ala Gly Lys Ala
610 615 620

Glu Ala Ala Ala Gly Thr Ala Asn Thr Ala Ala Asp Lys Ala Glu Ala
625 630 635 640

```
Val Ala Ala Lys Val Thr Asp Ile Lys Ala Asp Ile Ala Thr Asn Lys
                645                 650                 655

Asp Asn Ile Ala Lys Lys Ala Asn Ser Ala Asp Val Tyr Thr Arg Glu
            660                 665                 670

Glu Ser Asp Ser Lys Phe Val Arg Ile Asp Gly Leu Asn Ala Thr Thr
        675                 680                 685

Glu Lys Leu Asp Thr Arg Leu Ala Ser Ala Glu Lys Ser Ile Ala Asp
    690                 695                 700

His Asp Thr Arg Leu Asn Gly Leu Asp Lys Thr Val Ser Asp Leu Arg
705                 710                 715                 720

Lys Glu Thr Arg Gln Gly Leu Ala Glu Gln Ala Ala Leu Ser Gly Leu
                725                 730                 735

Phe Gln Pro Tyr Asn Val Gly Arg Phe Asn Val Thr Ala Ala Val Gly
            740                 745                 750

Gly Tyr Lys Ser Glu Ser Ala Val Ala Ile Gly Thr Gly Phe Arg Phe
        755                 760                 765

Thr Glu Asn Phe Ala Ala Lys Ala Gly Val Ala Val Gly Thr Ser Ser
    770                 775                 780

Gly Ser Ser Ala Ala Tyr His Val Gly Val Asn Tyr Glu Trp Leu Glu
785                 790                 795                 800

His His His His His His
                805
```

<210> 39
<211> 2256
<212> DNA
<213> Artificial Sequence

<220>
<223> ORF46.1-961c

<400> 39

```
atgtcagatt tggcaaacga ttcttttatc cggcaggttc tcgaccgtca gcatttcgaa   60
cccgacggga aataccacct attcggcagc agggggggaac ttgccgagcg cagcggccat   120
atcggattgg gaaaaataca aagccatcag ttgggcaacc tgatgattca acaggcggcc   180
attaaaggaa atatcggcta cattgtccgc ttttccgatc acgggcacga agtccattcc   240
cccttcgaca accatgcctc acattccgat tctgatgaag ccggtagtcc cgttgacgga   300
tttagccttt accgcatcca ttgggacgga tacgaacacc atcccgccga cggctatgac   360
gggccacagg gcggcggcta tcccgctccc aaaggcgcga gggatatata cagctacgac   420
ataaaaggcg ttgcccaaaa tatccgcctc aacctgaccg acaaccgcag caccggacaa   480
cggcttgccg accgtttcca caatgccggt agtatgctga cgcaaggagt aggcgacgga   540
ttcaaacgcg ccacccgata cagccccgag ctggacagat cgggcaatgc cgccgaagcc   600
ttcaacggca ctgcagatat cgttaaaaac atcatcggcg cggcaggaga aattgtcggc   660
gcaggcgatg ccgtgcaggg cataagcgaa ggctcaaaca ttgctgtcat gcacggcttg   720
ggtctgcttt ccaccgaaaa caagatggcg cgcatcaacg atttggcaga tatggcgcaa   780
ctcaaagact atgccgcagc agccatccgc gattgggcag tccaaaaccc caatgccgca   840
caaggcatag aagccgtcag caatatcttt atggcagcca tccccatcaa agggattgga   900
gctgttcggg gaaaatacgg cttgggcggc atcacggcac atcctatcaa gcggtcgcag   960
atgggcgcga tcgcattgcc gaaagggaaa tccgccgtca gcgacaattt tgccgatgcg   1020
gcatacgcca aatacccgtc cccttaccat tcccgaaata tccgttcaaa cttggagcag   1080
cgttacggca aagaaaacat cacctcctca accgtgccgc cgtcaaacgg caaaaatgtc   1140

aaactggcag accaacgcca cccgaagaca ggcgtaccgt ttgacggtaa agggtttccg   1200
aattttgaga agcacgtgaa atatgatacg ggatccggag gaggaggagc cacaaacgac   1260
gacgatgtta aaaaagctgc cactgtggcc attgctgctg cctacaacaa tggccaagaa   1320
atcaacggtt tcaaagctgg agagaccatc tacgacattg atgaagacgg cacaattacc   1380
aaaaaagacg caactgcagc cgatgttgaa gccgacgact ttaaaggtct gggtctgaaa   1440
aaagtcgtga ctaacctgac caaaaccgtc aatgaaaaca aacaaaacgt cgatgccaaa   1500
gtaaaagctg cagaatctga aatagaaaag ttaacaacca agttagcaga cactgatgcc   1560
gctttagcag atactgatgc cgctctggat gcaaccacca acgccttgaa taaattggga   1620
gaaaatataa cgacatttgc tgaagagact aagacaaata tcgtaaaaat tgatgaaaaa   1680
ttagaagccg tggctgatac cgtcgacaag catgccgaag cattcaacga tatcgccgat   1740
tcattggatg aaaccaacac taaggcagac gaagccgtca aaaccgccaa tgaagccaaa   1800
cagacggccg aagaaaccaa acaaaacgtc gatgccaaag taaaagctgc agaaactgca   1860
gcaggcaaag ccgaagctgc cgctggcaca gctaatactg cagccgacaa ggccgaagct   1920
gtcgctgcaa aagttaccga catcaaagct gatatcgcta cgaacaaaga taatattgct   1980
aaaaaagcaa acagtgccga cgtgtacacc agagaagagt ctgacagcaa atttgtcaga   2040
attgatggtc tgaacgctac taccgaaaaa ttggacacac gcttggcttc tgctgaaaaa   2100
tccattgccg atcacgatac tcgcctgaac ggtttggata aaacagtgtc agacctgcgc   2160
aaagaaaccc gccaaggcct tgcagaacaa gccgcgctct ccggtctgtt ccaaccttac   2220
aacgtgggtc tcgagcacca ccaccaccac cactga                              2256
```

<210> 40
<211> 751
<212> PRT
<213> Artificial Sequence

<220>
<223> ORF46.1-961c

<400> 40

```
Met Ser Asp Leu Ala Asn Asp Ser Phe Ile Arg Gln Val Leu Asp Arg
1               5                   10                  15

Gln His Phe Glu Pro Asp Gly Lys Tyr His Leu Phe Gly Ser Arg Gly
            20                  25                  30

Glu Leu Ala Glu Arg Ser Gly His Ile Gly Leu Gly Lys Ile Gln Ser
        35                  40                  45

His Gln Leu Gly Asn Leu Met Ile Gln Gln Ala Ala Ile Lys Gly Asn
    50                  55                  60

Ile Gly Tyr Ile Val Arg Phe Ser Asp His Gly His Glu Val His Ser
65                  70                  75                  80

Pro Phe Asp Asn His Ala Ser His Ser Asp Ser Asp Glu Ala Gly Ser
                85                  90                  95

Pro Val Asp Gly Phe Ser Leu Tyr Arg Ile His Trp Asp Gly Tyr Glu
            100                 105                 110

His His Pro Ala Asp Gly Tyr Asp Gly Pro Gln Gly Gly Gly Tyr Pro
        115                 120                 125

Ala Pro Lys Gly Ala Arg Asp Ile Tyr Ser Tyr Asp Ile Lys Gly Val
    130                 135                 140

Ala Gln Asn Ile Arg Leu Asn Leu Thr Asp Asn Arg Ser Thr Gly Gln
145                 150                 155                 160

Arg Leu Ala Asp Arg Phe His Asn Ala Gly Ser Met Leu Thr Gln Gly
                165                 170                 175
```

```
Val Gly Asp Gly Phe Lys Arg Ala Thr Arg Tyr Ser Pro Glu Leu Asp
            180                185                190

Arg Ser Gly Asn Ala Ala Glu Ala Phe Asn Gly Thr Ala Asp Ile Val
        195                200                205

Lys Asn Ile Ile Gly Ala Ala Gly Glu Ile Val Gly Ala Gly Asp Ala
    210                215                220

Val Gln Gly Ile Ser Glu Gly Ser Asn Ile Ala Val Met His Gly Leu
225                230                235                240

Gly Leu Leu Ser Thr Glu Asn Lys Met Ala Arg Ile Asn Asp Leu Ala
                245                250                255

Asp Met Ala Gln Leu Lys Asp Tyr Ala Ala Ala Ala Ile Arg Asp Trp
            260                265                270

Ala Val Gln Asn Pro Asn Ala Ala Gln Gly Ile Glu Ala Val Ser Asn
        275                280                285

Ile Phe Met Ala Ala Ile Pro Ile Lys Gly Ile Gly Ala Val Arg Gly
    290                295                300

Lys Tyr Gly Leu Gly Gly Ile Thr Ala His Pro Ile Lys Arg Ser Gln
305                310                315                320

Met Gly Ala Ile Ala Leu Pro Lys Gly Lys Ser Ala Val Ser Asp Asn
                325                330                335

Phe Ala Asp Ala Ala Tyr Ala Lys Tyr Pro Ser Pro Tyr His Ser Arg
            340                345                350

Asn Ile Arg Ser Asn Leu Glu Gln Arg Tyr Gly Lys Glu Asn Ile Thr
        355                360                365

Ser Ser Thr Val Pro Pro Ser Asn Gly Lys Asn Val Lys Leu Ala Asp
    370                375                380

Gln Arg His Pro Lys Thr Gly Val Pro Phe Asp Gly Lys Gly Phe Pro
385                390                395                400

Asn Phe Glu Lys His Val Lys Tyr Asp Thr Gly Ser Gly Gly Gly Gly
                405                410                415

Ala Thr Asn Asp Asp Asp Val Lys Lys Ala Ala Thr Val Ala Ile Ala
            420                425                430

Ala Ala Tyr Asn Asn Gly Gln Glu Ile Asn Gly Phe Lys Ala Gly Glu
        435                440                445

Thr Ile Tyr Asp Ile Asp Glu Asp Gly Thr Ile Thr Lys Lys Asp Ala
    450                455                460

Thr Ala Ala Asp Val Glu Ala Asp Asp Phe Lys Gly Leu Gly Leu Lys
465                470                475                480

Lys Val Val Thr Asn Leu Thr Lys Thr Val Asn Glu Asn Lys Gln Asn
                485                490                495
```

```
Val Asp Ala Lys Val Lys Ala Ala Glu Ser Glu Ile Glu Lys Leu Thr
            500                 505                 510

Thr Lys Leu Ala Asp Thr Asp Ala Ala Leu Ala Asp Thr Asp Ala Ala
        515                 520                 525

Leu Asp Ala Thr Thr Asn Ala Leu Asn Lys Leu Gly Glu Asn Ile Thr
    530                 535                 540

Thr Phe Ala Glu Glu Thr Lys Thr Asn Ile Val Lys Ile Asp Glu Lys
545                 550                 555                 560

Leu Glu Ala Val Ala Asp Thr Val Asp Lys His Ala Glu Ala Phe Asn
                565                 570                 575

Asp Ile Ala Asp Ser Leu Asp Glu Thr Asn Thr Lys Ala Asp Glu Ala
            580                 585                 590

Val Lys Thr Ala Asn Glu Ala Lys Gln Thr Ala Glu Glu Thr Lys Gln
        595                 600                 605

Asn Val Asp Ala Lys Val Lys Ala Ala Glu Thr Ala Ala Gly Lys Ala
    610                 615                 620

Glu Ala Ala Ala Gly Thr Ala Asn Thr Ala Ala Asp Lys Ala Glu Ala
625                 630                 635                 640

Val Ala Ala Lys Val Thr Asp Ile Lys Ala Asp Ile Ala Thr Asn Lys
                645                 650                 655

Asp Asn Ile Ala Lys Lys Ala Asn Ser Ala Asp Val Tyr Thr Arg Glu
            660                 665                 670

Glu Ser Asp Ser Lys Phe Val Arg Ile Asp Gly Leu Asn Ala Thr Thr
        675                 680                 685

Glu Lys Leu Asp Thr Arg Leu Ala Ser Ala Glu Lys Ser Ile Ala Asp
    690                 695                 700

His Asp Thr Arg Leu Asn Gly Leu Asp Lys Thr Val Ser Asp Leu Arg
705                 710                 715                 720

Lys Glu Thr Arg Gln Gly Leu Ala Glu Gln Ala Ala Leu Ser Gly Leu
                725                 730                 735

Phe Gln Pro Tyr Asn Val Gly Leu Glu His His His His His His
            740                 745                 750
```

<210> 41
<211> 2421
<212> DNA
<213> Artificial Sequence

<220>
<223> 961-ORF46.1

<400> 41

```
atggccacaa acgacgacga tgttaaaaaa gctgccactg tggccattgc tgctgcctac    60
aacaatggcc aagaaatcaa cggtttcaaa gctggagaga ccatctacga cattgatgaa   120
gacggcacaa ttaccaaaaa agacgcaact gcagccgatg ttgaagccga cgactttaaa   180
ggtctgggtc tgaaaaaagt cgtgactaac ctgaccaaaa ccgtcaatga aaacaaacaa   240
```

```
aacgtcgatg ccaaagtaaa agctgcagaa tctgaaatag aaaagttaac aaccaagtta   300
gcagacactg atgccgcttt agcagatact gatgccgctc tggatgcaac caccaacgcc   360
ttgaataaat tgggagaaaa tataacgaca tttgctgaag agactaagac aaatatcgta   420
aaaattgatg aaaaattaga agccgtggct gataccgtcg acaagcatgc cgaagcattc   480
aacgatatcg ccgattcatt ggatgaaacc aacactaagg cagacgaagc cgtcaaaacc   540
gccaatgaag ccaaacagac ggccgaagaa accaaacaaa acgtcgatgc caaagtaaaa   600
gctgcagaaa ctgcagcagg caaagccgaa gctgccgctg gcacagctaa tactgcagcc   660
gacaaggccg aagctgtcgc tgcaaaagtt accgacatca aagctgatat cgctacgaac   720
aaagataata ttgctaaaaa agcaaacagt gccgacgtgt acaccagaga agagtctgac   780
agcaaatttg tcagaattga tggtctgaac gctactaccg aaaaattgga cacacgcttg   840
gcttctgctg aaaaatccat tgccgatcac gatactcgcc tgaacggttt ggataaaaca   900
gtgtcagacc tgcgcaaaga aacccgccaa ggccttgcag aacaagccgc gctctccggt   960
ctgttccaac cttacaacgt gggtcggttc aatgtaacgg ctgcagtcgg cggctacaaa  1020
tccgaatcgg cagtcgccat cggtaccggc ttccgcttta ccgaaaactt tgccgccaaa  1080
gcaggcgtgg cagtcggcac ttcgtccggt tcttccgcag cctaccatgt cggcgtcaat  1140
tacgagtggg gatccggagg aggaggatca gatttggcaa acgattcttt tatccggcag  1200
gttctcgacc gtcagcattt cgaacccgac gggaaatacc acctattcgg cagcaggggg  1260
gaacttgccg agcgcagcgg ccatatcgga ttgggaaaaa tacaaagcca tcagttgggc  1320
aacctgatga ttcaacaggc ggccattaaa ggaaatatcg gctacattgt ccgcttttcc  1380
gatcacgggc acgaagtcca ttcccccttc gacaaccatg cctcacattc cgattctgat  1440
gaagccggta gtcccgttga cggatttagc ctttaccgca tccattggga cggatacgaa  1500
caccatcccg ccgacggcta tgacgggcca cagggcggcg gctatcccgc tcccaaaggc  1560
gcgagggata tatacagcta cgacataaaa ggcgttgccc aaaatatccg cctcaacctg  1620
accgacaacc gcagcaccgg acaacggctt gccgaccgtt tccacaatgc cggtagtatg  1680
ctgacgcaag gagtaggcga cggattcaaa cgcgccaccc gatacagccc cgagctggac  1740
agatcgggca atgccgccga agccttcaac ggcactgcag atatcgttaa aaacatcatc  1800
ggcgcggcag gagaaattgt cggcgcaggc gatgccgtgc agggcataag cgaaggctca  1860
aacattgctg tcatgcacgg cttgggtctg ctttccaccg aaaacaagat ggcgcgcatc  1920
aacgatttgg cagatatggc gcaactcaaa gactatgccg cagcagccat ccgcgattgg  1980
gcagtccaaa accccaatgc cgcacaaggc atagaagccg tcagcaatat ctttatggca  2040
gccatcccca tcaaagggat tggagctgtt cggggaaaat acggcttggg cggcatcacg  2100
gcacatccta tcaagcggtc gcagatgggc gcgatcgcat tgccgaaagg gaaatccgcc  2160
gtcagcgaca attttgccga tgcggcatac gccaaatacc cgtcccctta ccattcccga  2220
aatatccgtt caaacttgga gcagcgttac ggcaaagaaa acatcacctc ctcaaccgtg  2280
ccgccgtcaa acggcaaaaa tgtcaaactg gcagaccaac gccacccgaa gacaggcgta  2340
ccgtttgacg gtaaagggtt tccgaatttt gagaagcacg tgaaatatga tacgctcgag  2400
caccaccacc accaccactg a                                            2421
```

<210> 42
<211> 806
<212> PRT
<213> Artificial Sequence

<220>
<223> 961-ORF46.1

<400> 42

```
Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80
```

Asn Val Asp Ala Lys Val Lys Ala Ala Glu Ser Glu Ile Glu Lys Leu
85 90 95

Thr Thr Lys Leu Ala Asp Thr Asp Ala Ala Leu Ala Asp Thr Asp Ala
100 105 110

Ala Leu Asp Ala Thr Thr Asn Ala Leu Asn Lys Leu Gly Glu Asn Ile
115 120 125

Thr Thr Phe Ala Glu Glu Thr Lys Thr Asn Ile Val Lys Ile Asp Glu
130 135 140

Lys Leu Glu Ala Val Ala Asp Thr Val Asp Lys His Ala Glu Ala Phe
145 150 155 160

Asn Asp Ile Ala Asp Ser Leu Asp Glu Thr Asn Thr Lys Ala Asp Glu
165 170 175

Ala Val Lys Thr Ala Asn Glu Ala Lys Gln Thr Ala Glu Glu Thr Lys
180 185 190

Gln Asn Val Asp Ala Lys Val Lys Ala Ala Glu Thr Ala Ala Gly Lys
195 200 205

Ala Glu Ala Ala Ala Gly Thr Ala Asn Thr Ala Ala Asp Lys Ala Glu
210 215 220

Ala Val Ala Ala Lys Val Thr Asp Ile Lys Ala Asp Ile Ala Thr Asn
225 230 235 240

Lys Asp Asn Ile Ala Lys Lys Ala Asn Ser Ala Asp Val Tyr Thr Arg
245 250 255

Glu Glu Ser Asp Ser Lys Phe Val Arg Ile Asp Gly Leu Asn Ala Thr
260 265 270

Thr Glu Lys Leu Asp Thr Arg Leu Ala Ser Ala Glu Lys Ser Ile Ala
275 280 285

Asp His Asp Thr Arg Leu Asn Gly Leu Asp Lys Thr Val Ser Asp Leu
290 295 300

Arg Lys Glu Thr Arg Gln Gly Leu Ala Glu Gln Ala Ala Leu Ser Gly
305 310 315 320

Leu Phe Gln Pro Tyr Asn Val Gly Arg Phe Asn Val Thr Ala Ala Val
325 330 335

Gly Gly Tyr Lys Ser Glu Ser Ala Val Ala Ile Gly Thr Gly Phe Arg
340 345 350

Phe Thr Glu Asn Phe Ala Ala Lys Ala Gly Val Ala Val Gly Thr Ser
355 360 365

Ser Gly Ser Ser Ala Ala Tyr His Val Gly Val Asn Tyr Glu Trp Gly
370 375 380

Ser Gly Gly Gly Gly Ser Asp Leu Ala Asn Asp Ser Phe Ile Arg Gln
385 390 395 400

Val Leu Asp Arg Gln His Phe Glu Pro Asp Gly Lys Tyr His Leu Phe
405 410 415

Gly Ser Arg Gly Glu Leu Ala Glu Arg Ser Gly His Ile Gly Leu Gly
420 425 430

Lys Ile Gln Ser His Gln Leu Gly Asn Leu Met Ile Gln Gln Ala Ala
435 440 445

Ile Lys Gly Asn Ile Gly Tyr Ile Val Arg Phe Ser Asp His Gly His
450 455 460

Glu Val His Ser Pro Phe Asp Asn His Ala Ser His Ser Asp Ser Asp
465 470 475 480

Glu Ala Gly Ser Pro Val Asp Gly Phe Ser Leu Tyr Arg Ile His Trp
485 490 495

Asp Gly Tyr Glu His His Pro Ala Asp Gly Tyr Asp Gly Pro Gln Gly
500 505 510

Gly Gly Tyr Pro Ala Pro Lys Gly Ala Arg Asp Ile Tyr Ser Tyr Asp
515 520 525

Ile Lys Gly Val Ala Gln Asn Ile Arg Leu Asn Leu Thr Asp Asn Arg
530 535 540

Ser Thr Gly Gln Arg Leu Ala Asp Arg Phe His Asn Ala Gly Ser Met
545 550 555 560

Leu Thr Gln Gly Val Gly Asp Gly Phe Lys Arg Ala Thr Arg Tyr Ser
565 570 575

Pro Glu Leu Asp Arg Ser Gly Asn Ala Ala Glu Ala Phe Asn Gly Thr
580 585 590

Ala Asp Ile Val Lys Asn Ile Ile Gly Ala Ala Gly Glu Ile Val Gly
595 600 605

Ala Gly Asp Ala Val Gln Gly Ile Ser Glu Gly Ser Asn Ile Ala Val
610 615 620

Met His Gly Leu Gly Leu Leu Ser Thr Glu Asn Lys Met Ala Arg Ile
625 630 635 640

Asn Asp Leu Ala Asp Met Ala Gln Leu Lys Asp Tyr Ala Ala Ala Ala
645 650 655

Ile Arg Asp Trp Ala Val Gln Asn Pro Asn Ala Ala Gln Gly Ile Glu
660 665 670

Ala Val Ser Asn Ile Phe Met Ala Ala Ile Pro Ile Lys Gly Ile Gly
675 680 685

Ala Val Arg Gly Lys Tyr Gly Leu Gly Gly Ile Thr Ala His Pro Ile
690 695 700

Lys Arg Ser Gln Met Gly Ala Ile Ala Leu Pro Lys Gly Lys Ser Ala
705 710 715 720

Val Ser Asp Asn Phe Ala Asp Ala Ala Tyr Ala Lys Tyr Pro Ser Pro

```
                725                 730                 735

Tyr His Ser Arg Asn Ile Arg Ser Asn Leu Glu Gln Arg Tyr Gly Lys
            740                 745                 750

Glu Asn Ile Thr Ser Ser Thr Val Pro Pro Ser Asn Gly Lys Asn Val
        755                 760                 765

Lys Leu Ala Asp Gln Arg His Pro Lys Thr Gly Val Pro Phe Asp Gly
    770                 775                 780

Lys Gly Phe Pro Asn Phe Glu Lys His Val Lys Tyr Asp Thr Leu Glu
785                 790                 795                 800

His His His His His His
                805
```

<210> 43
<211> 1938
<212> DNA
<213> Artificial Sequence

<220>
<223> 961-741

<400> 43

```
atggccacaa acgacgacga tgttaaaaaa gctgccactg tggccattgc tgctgcctac   60
aacaatggcc aagaaatcaa cggtttcaaa gctggagaga ccatctacga cattgatgaa  120
gacggcacaa ttaccaaaaa agacgcaact gcagccgatg ttgaagccga cgactttaaa  180
ggtctgggtc tgaaaaaagt cgtgactaac ctgaccaaaa ccgtcaatga aaacaaacaa  240
aacgtcgatg ccaaagtaaa agctgcagaa tctgaaatag aaaagttaac aaccaagtta  300
gcagacactg atgccgcttt agcagatact gatgccgctc tggatgcaac caccaacgcc  360
ttgaataaat tgggagaaaa tataacgaca tttgctgaag agactaagac aaatatcgta  420
aaaattgatg aaaaattaga agccgtggct gataccgtcg acaagcatgc cgaagcattc  480
aacgatatcg ccgattcatt ggatgaaacc aacactaagg cagacgaagc cgtcaaaacc  540
gccaatgaag ccaaacagac ggccgaagaa accaaacaaa acgtcgatgc caaagtaaaa  600
gctgcagaaa ctgcagcagg caaagccgaa gctgccgctg gcacagctaa tactgcagcc  660
gacaaggccg aagctgtcgc tgcaaaagtt accgacatca aagctgatat cgctacgaac  720
aaagataata ttgctaaaaa agcaaacagt gccgacgtgt acaccagaga agagtctgac  780
agcaaatttg tcagaattga tggtctgaac gctactaccg aaaaattgga cacacgcttg  840
gcttctgctg aaaaatccat tgccgatcac gatactcgcc tgaacggttt ggataaaaca  900
gtgtcagacc tgcgcaaaga aacccgccaa ggccttgcag aacaagccgc gctctccggt  960
ctgttccaac cttacaacgt gggtcggttc aatgtaacgg ctgcagtcgg cggctacaaa 1020
tccgaatcgg cagtcgccat cggtaccggc ttccgcttta ccgaaaactt tgccgccaaa 1080
gcaggcgtgg cagtcggcac ttcgtccggt tcttccgcag cctaccatgt cggcgtcaat 1140
tacgagtggg gatccggagg gggtggtgtc gccgccgaca tcggtgcggg gcttgccgat 1200
gcactaaccg caccgctcga ccataaagac aaaggtttgc agtctttgac gctggatcag 1260
tccgtcagga aaaacgagaa actgaagctg gcggcacaag gtgcggaaaa aacttatgga 1320
aacggtgaca gcctcaatac gggcaaattg aagaacgaca aggtcagccg tttcgacttt 1380
atccgccaaa tcgaagtgga cgggcagctc attaccttgg agagtggaga gttccaagta 1440
tacaaacaaa gccattccgc cttaaccgcc tttcagaccg agcaaataca agattcggag 1500
cattccggga agatggttgc gaaacgccag ttcagaatcg gcgacatagc gggcgaacat 1560
acatcttttg acaagcttcc cgaaggcggc agggcgacat atcgcgggac ggcgttcggt 1620
tcagacgatg ccggcggaaa actgacctac accatagatt tcgccgccaa gcagggaaac 1680
ggcaaaatcg aacatttgaa atcgccagaa ctcaatgtcg acctggccgc cgccgatatc 1740
aagccggatg gaaaacgcca tgccgtcatc agcggttccg tcctttacaa ccaagccgag 1800
aaaggcagtt actccctcgg tatctttggc ggaaaagccc aggaagttgc cggcagcgcg 1860
gaagtgaaaa ccgtaaacgg catacgccat atcggccttg ccgccaagca actcgagcac 1920
caccaccacc accactga                                               1938
```

<210> 44
<211> 645
<212> PRT
<213> Artificial Sequence

<220>
<223> 961-741

<400> 44

```
Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110

Ala Leu Asp Ala Thr Thr Asn Ala Leu Asn Lys Leu Gly Glu Asn Ile
        115                 120                 125

Thr Thr Phe Ala Glu Glu Thr Lys Thr Asn Ile Val Lys Ile Asp Glu
    130                 135                 140

Lys Leu Glu Ala Val Ala Asp Thr Val Asp Lys His Ala Glu Ala Phe
145                 150                 155                 160

Asn Asp Ile Ala Asp Ser Leu Asp Glu Thr Asn Thr Lys Ala Asp Glu
                165                 170                 175

Ala Val Lys Thr Ala Asn Glu Ala Lys Gln Thr Ala Glu Glu Thr Lys
            180                 185                 190

Gln Asn Val Asp Ala Lys Val Lys Ala Ala Glu Thr Ala Ala Gly Lys
        195                 200                 205

Ala Glu Ala Ala Ala Gly Thr Ala Asn Thr Ala Ala Asp Lys Ala Glu
    210                 215                 220

Ala Val Ala Ala Lys Val Thr Asp Ile Lys Ala Asp Ile Ala Thr Asn
225                 230                 235                 240

Lys Asp Asn Ile Ala Lys Lys Ala Asn Ser Ala Asp Val Tyr Thr Arg
                245                 250                 255

Glu Glu Ser Asp Ser Lys Phe Val Arg Ile Asp Gly Leu Asn Ala Thr
            260                 265                 270

Thr Glu Lys Leu Asp Thr Arg Leu Ala Ser Ala Glu Lys Ser Ile Ala
        275                 280                 285
```

```
Asp His Asp Thr Arg Leu Asn Gly Leu Asp Lys Thr Val Ser Asp Leu
    290                 295                 300

Arg Lys Glu Thr Arg Gln Gly Leu Ala Glu Gln Ala Ala Leu Ser Gly
305                 310                 315                 320

Leu Phe Gln Pro Tyr Asn Val Gly Arg Phe Asn Val Thr Ala Ala Val
                325                 330                 335

Gly Gly Tyr Lys Ser Glu Ser Ala Val Ala Ile Gly Thr Gly Phe Arg
            340                 345                 350

Phe Thr Glu Asn Phe Ala Ala Lys Ala Gly Val Ala Val Gly Thr Ser
        355                 360                 365

Ser Gly Ser Ser Ala Ala Tyr His Val Gly Val Asn Tyr Glu Trp Gly
    370                 375                 380

Ser Gly Gly Gly Gly Val Ala Ala Asp Ile Gly Ala Gly Leu Ala Asp
385                 390                 395                 400

Ala Leu Thr Ala Pro Leu Asp His Lys Asp Lys Gly Leu Gln Ser Leu
                405                 410                 415

Thr Leu Asp Gln Ser Val Arg Lys Asn Glu Lys Leu Lys Leu Ala Ala
            420                 425                 430

Gln Gly Ala Glu Lys Thr Tyr Gly Asn Gly Asp Ser Leu Asn Thr Gly
        435                 440                 445

Lys Leu Lys Asn Asp Lys Val Ser Arg Phe Asp Phe Ile Arg Gln Ile
    450                 455                 460

Glu Val Asp Gly Gln Leu Ile Thr Leu Glu Ser Gly Glu Phe Gln Val
465                 470                 475                 480

Tyr Lys Gln Ser His Ser Ala Leu Thr Ala Phe Gln Thr Glu Gln Ile
                485                 490                 495

Gln Asp Ser Glu His Ser Gly Lys Met Val Ala Lys Arg Gln Phe Arg
            500                 505                 510

Ile Gly Asp Ile Ala Gly Glu His Thr Ser Phe Asp Lys Leu Pro Glu
        515                 520                 525

Gly Gly Arg Ala Thr Tyr Arg Gly Thr Ala Phe Gly Ser Asp Asp Ala
    530                 535                 540

Gly Gly Lys Leu Thr Tyr Thr Ile Asp Phe Ala Ala Lys Gln Gly Asn
545                 550                 555                 560

Gly Lys Ile Glu His Leu Lys Ser Pro Glu Leu Asn Val Asp Leu Ala
                565                 570                 575

Ala Ala Asp Ile Lys Pro Asp Gly Lys Arg His Ala Val Ile Ser Gly
            580                 585                 590

Ser Val Leu Tyr Asn Gln Ala Glu Lys Gly Ser Tyr Ser Leu Gly Ile
        595                 600                 605
```

```
Phe Gly Gly Lys Ala Gln Glu Val Ala Gly Ser Ala Glu Val Lys Thr
    610                 615                     620

Val Asn Gly Ile Arg His Ile Gly Leu Ala Ala Lys Gln Leu Glu His
625                 630                     635                 640

His His His His His
                645
```

<210> 45
<211> 4335
<212> DNA
<213> Artificial Sequence

<220>
<223> 961-983

<400> 45

atggccacaa acgacgacga tgttaaaaaa gctgccactg tggccattgc tgctgcctac 60
aacaatggcc aagaaatcaa cggtttcaaa gctggagaga ccatctacga cattgatgaa 120
gacggcacaa ttaccaaaaa agacgcaact gcagccgatg ttgaagccga cgactttaaa 180
ggtctgggtc tgaaaaaagt cgtgactaac ctgaccaaaa ccgtcaatga aaacaaacaa 240
aacgtcgatg ccaaagtaaa agctgcagaa tctgaaatag aaaagttaac aaccaagtta 300
gcagacactg atgccgcttt agcagatact gatgccgctc tggatgcaac caccaacgcc 360
ttgaataaat tgggagaaaa tataacgaca tttgctgaag agactaagac aaatatcgta 420
aaaattgatg aaaaattaga agccgtggct gataccgtcg acaagcatgc cgaagcattc 480
aacgatatcg ccgattcatt ggatgaaacc aacactaagg cagacgaagc cgtcaaaacc 540
gccaatgaag ccaaacagac ggccgaagaa accaaacaaa acgtcgatgc caaagtaaaa 600
gctgcagaaa ctgcagcagg caaagccgaa gctgccgctg gcacagctaa tactgcagcc 660
gacaaggccg aagctgtcgc tgcaaaagtt accgacatca aagctgatat cgctacgaac 720
aaagataata ttgctaaaaa agcaaacagt gccgacgtgt acaccagaga agagtctgac 780
agcaaatttg tcagaattga tggtctgaac gctactaccg aaaaattgga cacacgcttg 840
gcttctgctg aaaaatccat tgccgatcac gatactcgcc tgaacggttt ggataaaaca 900
gtgtcagacc tgcgcaaaga aacccgccaa ggccttgcag aacaagccgc gctctccggt 960
ctgttccaac cttacaacgt gggtcggttc aatgtaacgg ctgcagtcgg cggctacaaa 1020
tccgaatcgg cagtcgccat cggtaccggc ttccgcttta ccgaaaactt tgccgccaaa 1080
gcaggcgtgg cagtcggcac ttcgtccggt tcttccgcag cctaccatgt cggcgtcaat 1140
tacgagtggg gatccggcgg aggcggcact tctgcgcccg acttcaatgc aggcggtacc 1200
ggtatcggca gcaacagcag agcaacaaca gcgaaatcag cagcagtatc ttacgccggt 1260
atcaagaacg aaatgtgcaa agacagaagc atgctctgtg ccggtcggga tgacgttgcg 1320
gttacagaca gggatgccaa aatcaatgcc cccccccga atctgcatac cggagacttt 1380
ccaaacccaa atgacgcata caagaatttg atcaacctca aacctgcaat tgaagcaggc 1440
tatacaggac gcggggtaga ggtaggtatc gtcgacacag gcgaatccgt cggcagcata 1500
tcctttcccg aactgtatgg cagaaaagaa cacggctata acgaaaatta caaaaactat 1560
acggcgtata tgcggaagga agcgcctgaa gacggaggcg gtaaagacat tgaagcttct 1620
ttcgacgatg aggccgttat agagactgaa gcaaagccga cggatatccg ccacgtaaaa 1680
gaaatcggac acatcgattt ggtctcccat attattggcg ggcgttccgt ggacggcaga 1740
cctgcaggcg gtattgcgcc cgatgcgacg ctacacataa tgaatacgaa tgatgaaacc 1800
aagaacgaaa tgatggttgc agccatccgc aatgcatggg tcaagctggg cgaacgtggc 1860
gtgcgcatcg tcaataacag ttttggaaca acatcgaggg caggcactgc cgaccttttc 1920
caaatagcca attcggagga gcagtaccgc caagcgttgc tcgactattc cggcggtgat 1980
aaaacagacg agggtatccg cctgatgcaa cagagcgatt acggcaacct gtcctaccac 2040
atccgtaata aaaacatgct tttcatcttt tcgacaggca atgacgcaca agctcagccc 2100
aacacatatg ccctattgcc attttatgaa aaagacgctc aaaaaggcat tatcacagtc 2160
gcaggcgtag accgcagtgg agaaaagttc aaacgggaaa tgtatggaga accgggtaca 2220
gaaccgcttg agtatggctc caaccattgc ggaattactg ccatgtggtg cctgtcggca 2280
ccctatgaag caagcgtccg tttcacccgt acaaacccga ttcaaattgc cggaacatcc 2340
ttttccgcac ccatcgtaac cggcacggcg gctctgctgc tgcagaaata cccgtggatg 2400
agcaacgaca acctgcgtac cacgttgctg acgacggctc aggacatcgg tgcagtcggc 2460
gtggacagca agttcggctg ggactgctg gatgcgggta aggccatgaa cggacccgcg 2520
tcctttccgt tcggcgactt taccgccgat acgaaaggta catccgatat tgcctactcc 2580

```
ttccgtaacg acatttcagg cacgggcggc ctgatcaaaa aaggcggcag ccaactgcaa   2640
ctgcacggca acaacaccta tacgggcaaa accattatcg aaggcggttc gctggtgttg   2700
tacggcaaca acaaatcgga tatgcgcgtc gaaaccaaag gtgcgctgat ttataacggg   2760
gcggcatccg gcggcagcct gaacagcgac ggcattgtct atctggcaga taccgaccaa   2820
tccggcgcaa acgaaaccgt acacatcaaa ggcagtctgc agctggacgg caaaggtacg   2880
ctgtacacac gtttgggcaa actgctgaaa gtggacggta cggcgattat cggcggcaag   2940
ctgtacatgt cggcacgcgg caagggggca ggctatctca acagtaccgg acgacgtgtt   3000
cccttcctga gtgccgccaa aatcgggcag gattattctt tcttcacaaa catcgaaacc   3060
gacggcggcc tgctggcttc cctcgacagc gtcgaaaaaa cagcgggcag tgaaggcgac   3120
acgctgtcct attatgtccg tcgcggcaat gcggcacgga ctgcttcggc agcggcacat   3180
tccgcgcccg ccggtctgaa acacgccgta gaacagggcg gcagcaatct ggaaaacctg   3240
atggtcgaac tggatgcctc cgaatcatcc gcaacacccg agacggttga aactgcggca   3300
gccgaccgca cagatatgcc gggcatccgc ccctacggcg caactttccg cgcagcggca   3360
gccgtacagc atgcgaatgc cgccgacggt gtacgcatct tcaacagtct cgccgctacc   3420
gtctatgccg acagtaccgc cgcccatgcc gatatgcagg gacgccgcct gaaagccgta   3480
tcggacgggt tggaccacaa cggcacgggt ctgcgcgtca tcgcgcaaac ccaacaggac   3540
ggtggaacgt gggaacaggg cggtgttgaa ggcaaaatgc gcggcagtac ccaaaccgtc   3600
ggcattgccg cgaaaaccgg cgaaaatacg acagcagccg ccacactggg catgggacgc   3660
agcacatgga gcgaaaacag tgcaaatgca aaaaccgaca gcattagtct gtttgcaggc   3720
atacggcacg atgcgggcga tatcggctat ctcaaaggcc tgttctccta cggacgctac   3780
aaaaacagca tcagccgcag caccggtgcg gacgaacatg cggaaggcag cgtcaacggc   3840
acgctgatgc agctgggcgc actgggcggt gtcaacgttc cgtttgccgc aacgggagat   3900
ttgacggtcg aaggcggtct gcgctacgac ctgctcaaac aggatgcatt cgccgaaaaa   3960
ggcagtgctt tgggctggag cggcaacagc ctcactgaag gcacgctggt cggactcgcg   4020
ggtctgaagc tgtcgcaacc cttgagcgat aaagccgtcc tgtttgcaac ggcgggcgtg   4080
gaacgcgacc tgaacggacg cgactacacg gtaacgggcg gctttaccgg cgcgactgca   4140
gcaaccggca agacgggggc acgcaatatg ccgcacaccc gtctggttgc cggcctgggc   4200
gcggatgtcg aattcggcaa cggctggaac ggcttggcac gttacagcta cgccggttcc   4260
aaacagtacg gcaaccacag cggacgagtc ggcgtaggct accggttcct cgagcaccac   4320
caccaccacc actga                                                    4335
```

<210> 46
<211> 1444
<212> PRT
<213> Artificial Sequence

<220>
<223> 961-983

<400> 46

```
Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110
```

Ala Leu Asp Ala Thr Thr Asn Ala Leu Asn Lys Leu Gly Glu Asn Ile
115 120 125

Thr Thr Phe Ala Glu Glu Thr Lys Thr Asn Ile Val Lys Ile Asp Glu
130 135 140

Lys Leu Glu Ala Val Ala Asp Thr Val Asp Lys His Ala Glu Ala Phe
145 150 155 160

Asn Asp Ile Ala Asp Ser Leu Asp Glu Thr Asn Thr Lys Ala Asp Glu
165 170 175

Ala Val Lys Thr Ala Asn Glu Ala Lys Gln Thr Ala Glu Glu Thr Lys
180 185 190

Gln Asn Val Asp Ala Lys Val Lys Ala Ala Glu Thr Ala Ala Gly Lys
195 200 205

Ala Glu Ala Ala Ala Gly Thr Ala Asn Thr Ala Ala Asp Lys Ala Glu
210 215 220

Ala Val Ala Ala Lys Val Thr Asp Ile Lys Ala Asp Ile Ala Thr Asn
225 230 235 240

Lys Asp Asn Ile Ala Lys Lys Ala Asn Ser Ala Asp Val Tyr Thr Arg
245 250 255

Glu Glu Ser Asp Ser Lys Phe Val Arg Ile Asp Gly Leu Asn Ala Thr
260 265 270

Thr Glu Lys Leu Asp Thr Arg Leu Ala Ser Ala Glu Lys Ser Ile Ala
275 280 285

Asp His Asp Thr Arg Leu Asn Gly Leu Asp Lys Thr Val Ser Asp Leu
290 295 300

Arg Lys Glu Thr Arg Gln Gly Leu Ala Glu Gln Ala Ala Leu Ser Gly
305 310 315 320

Leu Phe Gln Pro Tyr Asn Val Gly Arg Phe Asn Val Thr Ala Ala Val
325 330 335

Gly Gly Tyr Lys Ser Glu Ser Ala Val Ala Ile Gly Thr Gly Phe Arg
340 345 350

Phe Thr Glu Asn Phe Ala Ala Lys Ala Gly Val Ala Val Gly Thr Ser
355 360 365

Ser Gly Ser Ser Ala Ala Tyr His Val Gly Val Asn Tyr Glu Trp Gly
370 375 380

Ser Gly Gly Gly Gly Thr Ser Ala Pro Asp Phe Asn Ala Gly Gly Thr
385 390 395 400

Gly Ile Gly Ser Asn Ser Arg Ala Thr Thr Ala Lys Ser Ala Ala Val
405 410 415

Ser Tyr Ala Gly Ile Lys Asn Glu Met Cys Lys Asp Arg Ser Met Leu
420 425 430

Cys Ala Gly Arg Asp Asp Val Ala Val Thr Asp Arg Asp Ala Lys Ile

```
        435                 440                 445

Asn Ala Pro Pro Pro Asn Leu His Thr Gly Asp Phe Pro Asn Pro Asn
    450                 455                 460

Asp Ala Tyr Lys Asn Leu Ile Asn Leu Lys Pro Ala Ile Glu Ala Gly
465                 470                 475                 480

Tyr Thr Gly Arg Gly Val Glu Val Gly Ile Val Asp Thr Gly Glu Ser
                485                 490                 495

Val Gly Ser Ile Ser Phe Pro Glu Leu Tyr Gly Arg Lys Glu His Gly
            500                 505                 510

Tyr Asn Glu Asn Tyr Lys Asn Tyr Thr Ala Tyr Met Arg Lys Glu Ala
        515                 520                 525

Pro Glu Asp Gly Gly Gly Lys Asp Ile Glu Ala Ser Phe Asp Asp Glu
    530                 535                 540

Ala Val Ile Glu Thr Glu Ala Lys Pro Thr Asp Ile Arg His Val Lys
545                 550                 555                 560

Glu Ile Gly His Ile Asp Leu Val Ser His Ile Ile Gly Gly Arg Ser
                565                 570                 575

Val Asp Gly Arg Pro Ala Gly Gly Ile Ala Pro Asp Ala Thr Leu His
            580                 585                 590

Ile Met Asn Thr Asn Asp Glu Thr Lys Asn Glu Met Met Val Ala Ala
        595                 600                 605

Ile Arg Asn Ala Trp Val Lys Leu Gly Glu Arg Gly Val Arg Ile Val
    610                 615                 620

Asn Asn Ser Phe Gly Thr Thr Ser Arg Ala Gly Thr Ala Asp Leu Phe
625                 630                 635                 640

Gln Ile Ala Asn Ser Glu Glu Gln Tyr Arg Gln Ala Leu Leu Asp Tyr
                645                 650                 655

Ser Gly Gly Asp Lys Thr Asp Glu Gly Ile Arg Leu Met Gln Gln Ser
            660                 665                 670

Asp Tyr Gly Asn Leu Ser Tyr His Ile Arg Asn Lys Asn Met Leu Phe
        675                 680                 685

Ile Phe Ser Thr Gly Asn Asp Ala Gln Ala Gln Pro Asn Thr Tyr Ala
    690                 695                 700

Leu Leu Pro Phe Tyr Glu Lys Asp Ala Gln Lys Gly Ile Ile Thr Val
705                 710                 715                 720

Ala Gly Val Asp Arg Ser Gly Glu Lys Phe Lys Arg Glu Met Tyr Gly
                725                 730                 735

Glu Pro Gly Thr Glu Pro Leu Glu Tyr Gly Ser Asn His Cys Gly Ile
            740                 745                 750

Thr Ala Met Trp Cys Leu Ser Ala Pro Tyr Glu Ala Ser Val Arg Phe
        755                 760                 765
```

Thr Arg Thr Asn Pro Ile Gln Ile Ala Gly Thr Ser Phe Ser Ala Pro
    770                 775                 780

Ile Val Thr Gly Thr Ala Ala Leu Leu Leu Gln Lys Tyr Pro Trp Met
785                 790                 795                 800

Ser Asn Asp Asn Leu Arg Thr Thr Leu Leu Thr Thr Ala Gln Asp Ile
                805                 810                 815

Gly Ala Val Gly Val Asp Ser Lys Phe Gly Trp Gly Leu Leu Asp Ala
            820                 825                 830

Gly Lys Ala Met Asn Gly Pro Ala Ser Phe Pro Phe Gly Asp Phe Thr
        835                 840                 845

Ala Asp Thr Lys Gly Thr Ser Asp Ile Ala Tyr Ser Phe Arg Asn Asp
    850                 855                 860

Ile Ser Gly Thr Gly Gly Leu Ile Lys Lys Gly Gly Ser Gln Leu Gln
865                 870                 875                 880

Leu His Gly Asn Asn Thr Tyr Thr Gly Lys Thr Ile Ile Glu Gly Gly
                885                 890                 895

Ser Leu Val Leu Tyr Gly Asn Asn Lys Ser Asp Met Arg Val Glu Thr
            900                 905                 910

Lys Gly Ala Leu Ile Tyr Asn Gly Ala Ala Ser Gly Gly Ser Leu Asn
        915                 920                 925

Ser Asp Gly Ile Val Tyr Leu Ala Asp Thr Asp Gln Ser Gly Ala Asn
    930                 935                 940

Glu Thr Val His Ile Lys Gly Ser Leu Gln Leu Asp Gly Lys Gly Thr
945                 950                 955                 960

Leu Tyr Thr Arg Leu Gly Lys Leu Leu Lys Val Asp Gly Thr Ala Ile
                965                 970                 975

Ile Gly Gly Lys Leu Tyr Met Ser Ala Arg Gly Lys Gly Ala Gly Tyr
            980                 985                 990

Leu Asn Ser Thr Gly Arg Arg Val Pro Phe Leu Ser Ala Ala Lys Ile
        995                 1000                1005

Gly Gln Asp Tyr Ser Phe Phe Thr Asn Ile Glu Thr Asp Gly Gly Leu
    1010                1015                1020

Leu Ala Ser Leu Asp Ser Val Glu Lys Thr Ala Gly Ser Glu Gly Asp
1025                1030                1035                1040

Thr Leu Ser Tyr Tyr Val Arg Arg Gly Asn Ala Ala Arg Thr Ala Ser
                1045                1050                1055

Ala Ala Ala His Ser Ala Pro Ala Gly Leu Lys His Ala Val Glu Gln
            1060                1065                1070

Gly Gly Ser Asn Leu Glu Asn Leu Met Val Glu Leu Asp Ala Ser Glu
        1075                1080                1085

Ser Ser Ala Thr Pro Glu Thr Val Glu Thr Ala Ala Ala Asp Arg Thr
1090 1095 1100

Asp Met Pro Gly Ile Arg Pro Tyr Gly Ala Thr Phe Arg Ala Ala Ala
1105 1110 1115 1120

Ala Val Gln His Ala Asn Ala Ala Asp Gly Val Arg Ile Phe Asn Ser
1125 1130 1135

Leu Ala Ala Thr Val Tyr Ala Asp Ser Thr Ala Ala His Ala Asp Met
1140 1145 1150

Gln Gly Arg Arg Leu Lys Ala Val Ser Asp Gly Leu Asp His Asn Gly
1155 1160 1165

Thr Gly Leu Arg Val Ile Ala Gln Thr Gln Gln Asp Gly Gly Thr Trp
1170 1175 1180

Glu Gln Gly Gly Val Glu Gly Lys Met Arg Gly Ser Thr Gln Thr Val
1185 1190 1195 1200

Gly Ile Ala Ala Lys Thr Gly Glu Asn Thr Thr Ala Ala Ala Thr Leu
1205 1210 1215

Gly Met Gly Arg Ser Thr Trp Ser Glu Asn Ser Ala Asn Ala Lys Thr
1220 1225 1230

Asp Ser Ile Ser Leu Phe Ala Gly Ile Arg His Asp Ala Gly Asp Ile
1235 1240 1245

Gly Tyr Leu Lys Gly Leu Phe Ser Tyr Gly Arg Tyr Lys Asn Ser Ile
1250 1255 1260

Ser Arg Ser Thr Gly Ala Asp Glu His Ala Glu Gly Ser Val Asn Gly
1265 1270 1275 1280

Thr Leu Met Gln Leu Gly Ala Leu Gly Gly Val Asn Val Pro Phe Ala
1285 1290 1295

Ala Thr Gly Asp Leu Thr Val Glu Gly Gly Leu Arg Tyr Asp Leu Leu
1300 1305 1310

Lys Gln Asp Ala Phe Ala Glu Lys Gly Ser Ala Leu Gly Trp Ser Gly
1315 1320 1325

Asn Ser Leu Thr Glu Gly Thr Leu Val Gly Leu Ala Gly Leu Lys Leu
1330 1335 1340

Ser Gln Pro Leu Ser Asp Lys Ala Val Leu Phe Ala Thr Ala Gly Val
1345 1350 1355 1360

Glu Arg Asp Leu Asn Gly Arg Asp Tyr Thr Val Thr Gly Gly Phe Thr
1365 1370 1375

Gly Ala Thr Ala Ala Thr Gly Lys Thr Gly Ala Arg Asn Met Pro His
1380 1385 1390

Thr Arg Leu Val Ala Gly Leu Gly Ala Asp Val Glu Phe Gly Asn Gly
1395 1400 1405

Trp Asn Gly Leu Ala Arg Tyr Ser Tyr Ala Gly Ser Lys Gln Tyr Gly

```
    1410                1415                1420

Asn His Ser Gly Arg Val Gly Val Gly Tyr Arg Phe Leu Glu His His
1425                1430                1435                1440

His His His His
```

<210> 47
<211> 2256
<212> DNA
<213> Artificial Sequence

<220>
<223> 961c-ORF46.1

<400> 47

```
atggccacaa acgacgacga tgttaaaaaa gctgccactg tggccattgc tgctgcctac     60
aacaatggcc aagaaatcaa cggtttcaaa gctggagaga ccatctacga cattgatgaa    120
gacggcacaa ttaccaaaaa agacgcaact gcagccgatg ttgaagccga cgactttaaa    180
ggtctgggtc tgaaaaaagt cgtgactaac ctgaccaaaa ccgtcaatga aaacaaacaa    240
aacgtcgatg ccaaagtaaa agctgcagaa tctgaaatag aaaagttaac aaccaagtta    300
gcagacactg atgccgcttt agcagatact gatgccgctc tggatgcaac caccaacgcc    360
ttgaataaat tgggagaaaa tataacgaca tttgctgaag agactaagac aaatatcgta    420
aaaattgatg aaaaattaga agccgtggct gataccgtcg acaagcatgc cgaagcattc    480
aacgatatcg ccgattcatt ggatgaaacc aacactaagg cagacgaagc cgtcaaaacc    540
gccaatgaag ccaaacagac ggccgaagaa accaaacaaa acgtcgatgc caaagtaaaa    600
gctgcagaaa ctgcagcagg caaagccgaa gctgccgctg gcacagctaa tactgcagcc    660
gacaaggccg aagctgtcgc tgcaaaagtt accgacatca aagctgatat cgctacgaac    720
aaagataata ttgctaaaaa agcaaacagt gccgacgtgt acaccagaga agagtctgac    780
agcaaatttg tcagaattga tggtctgaac gctactaccg aaaaattgga cacacgcttg    840
gcttctgctg aaaaatccat tgccgatcac gatactcgcc tgaacggttt ggataaaaca    900
gtgtcagacc tgcgcaaaga aacccgccaa ggccttgcag aacaagccgc gctctccggt    960
ctgttccaac cttacaacgt gggtggatcc ggaggaggag gatcagattt ggcaaacgat   1020
tcttttatcc ggcaggttct cgaccgtcag catttcgaac ccgacgggaa ataccaccta   1080
ttcggcagca ggggggaact tgccgagcgc agcggccata tcggattggg aaaaatacaa   1140
agccatcagt tgggcaacct gatgattcaa caggcggcca ttaaaggaaa tatcggctac   1200
attgtccgct ttccgatca cgggcacgaa gtccattccc ccttcgacaa ccatgcctca   1260
cattccgatt ctgatgaagc cggtagtccc gttgacggat ttagccttta ccgcatccat   1320
tgggacggat acgaacacca tcccgccgac ggctatgacg ggccacaggg cggcggctat   1380
cccgctccca aggcgcgag ggatatatac agctacgaca taaaaggcgt tgcccaaaat   1440
atccgcctca acctgaccga caaccgcagc accggacaac ggcttgccga ccgtttccac   1500
aatgccggta gtatgctgac gcaaggagta ggcgacggat tcaaacgcgc cacccgatac   1560
agccccgagc tggacagatc gggcaatgcc gccgaagcct tcaacggcac tgcagatatc   1620
gttaaaaaca tcatcggcgc ggcaggagaa attgtcggcg caggcgatgc cgtgcagggc   1680
ataagcgaag gctcaaacat tgctgtcatg cacggcttgg gtctgctttc caccgaaaac   1740
aagatggcgc gcatcaacga tttggcagat atggcgcaac tcaaagacta tgccgcagca   1800
gccatccgcg attgggcagt ccaaaacccc aatgccgcac aaggcataga agccgtcagc   1860
aatatcttta tggcagccat ccccatcaaa gggattggag ctgttcgggg aaaatacggc   1920
ttgggcggca tcacggcaca tcctatcaag cggtcgcaga tgggcgcgat cgcattgccg   1980
aaagggaaat ccgccgtcag cgacaatttt gccgatgcgg catacgccaa atacccgtcc   2040
ccttaccatt cccgaaatat ccgttcaaac ttggagcagc gttacggcaa agaaaacatc   2100
acctcctcaa ccgtgccgcc gtcaaacggc aaaaatgtca aactggcaga ccaacgccac   2160
ccgaagacag gcgtaccgtt tgacggtaaa gggtttccga attttgagaa gcacgtgaaa   2220
tatgatacgc tcgagcacca ccaccaccac cactga                              2256
```

<210> 48
<211> 751
<212> PRT
<213> Artificial Sequence

<220>
<223> 961c-ORF46.1

<400> 48

```
Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110

Ala Leu Asp Ala Thr Thr Asn Ala Leu Asn Lys Leu Gly Glu Asn Ile
        115                 120                 125

Thr Thr Phe Ala Glu Glu Thr Lys Thr Asn Ile Val Lys Ile Asp Glu
    130                 135                 140

Lys Leu Glu Ala Val Ala Asp Thr Val Asp Lys His Ala Glu Ala Phe
145                 150                 155                 160

Asn Asp Ile Ala Asp Ser Leu Asp Glu Thr Asn Thr Lys Ala Asp Glu
                165                 170                 175

Ala Val Lys Thr Ala Asn Glu Ala Lys Gln Thr Ala Glu Glu Thr Lys
            180                 185                 190

Gln Asn Val Asp Ala Lys Val Lys Ala Ala Glu Thr Ala Ala Gly Lys
        195                 200                 205

Ala Glu Ala Ala Ala Gly Thr Ala Asn Thr Ala Ala Asp Lys Ala Glu
    210                 215                 220

Ala Val Ala Ala Lys Val Thr Asp Ile Lys Ala Asp Ile Ala Thr Asn
225                 230                 235                 240

Lys Asp Asn Ile Ala Lys Lys Ala Asn Ser Ala Asp Val Tyr Thr Arg
                245                 250                 255

Glu Glu Ser Asp Ser Lys Phe Val Arg Ile Asp Gly Leu Asn Ala Thr
            260                 265                 270

Thr Glu Lys Leu Asp Thr Arg Leu Ala Ser Ala Glu Lys Ser Ile Ala
        275                 280                 285

Asp His Asp Thr Arg Leu Asn Gly Leu Asp Lys Thr Val Ser Asp Leu
    290                 295                 300
```

Arg Lys Glu Thr Arg Gln Gly Leu Ala Glu Gln Ala Ala Leu Ser Gly
305 310 315 320

Leu Phe Gln Pro Tyr Asn Val Gly Gly Ser Gly Gly Gly Gly Ser Asp
325 330 335

Leu Ala Asn Asp Ser Phe Ile Arg Gln Val Leu Asp Arg Gln His Phe
340 345 350

Glu Pro Asp Gly Lys Tyr His Leu Phe Gly Ser Arg Gly Glu Leu Ala
355 360 365

Glu Arg Ser Gly His Ile Gly Leu Gly Lys Ile Gln Ser His Gln Leu
370 375 380

Gly Asn Leu Met Ile Gln Gln Ala Ala Ile Lys Gly Asn Ile Gly Tyr
385 390 395 400

Ile Val Arg Phe Ser Asp His Gly His Glu Val His Ser Pro Phe Asp
405 410 415

Asn His Ala Ser His Ser Asp Ser Asp Glu Ala Gly Ser Pro Val Asp
420 425 430

Gly Phe Ser Leu Tyr Arg Ile His Trp Asp Gly Tyr Glu His His Pro
435 440 445

Ala Asp Gly Tyr Asp Gly Pro Gln Gly Gly Gly Tyr Pro Ala Pro Lys
450 455 460

Gly Ala Arg Asp Ile Tyr Ser Tyr Asp Ile Lys Gly Val Ala Gln Asn
465 470 475 480

Ile Arg Leu Asn Leu Thr Asp Asn Arg Ser Thr Gly Gln Arg Leu Ala
485 490 495

Asp Arg Phe His Asn Ala Gly Ser Met Leu Thr Gln Gly Val Gly Asp
500 505 510

Gly Phe Lys Arg Ala Thr Arg Tyr Ser Pro Glu Leu Asp Arg Ser Gly
515 520 525

Asn Ala Ala Glu Ala Phe Asn Gly Thr Ala Asp Ile Val Lys Asn Ile
530 535 540

Ile Gly Ala Ala Gly Glu Ile Val Gly Ala Gly Asp Ala Val Gln Gly
545 550 555 560

Ile Ser Glu Gly Ser Asn Ile Ala Val Met His Gly Leu Gly Leu Leu
565 570 575

Ser Thr Glu Asn Lys Met Ala Arg Ile Asn Asp Leu Ala Asp Met Ala
580 585 590

Gln Leu Lys Asp Tyr Ala Ala Ala Ala Ile Arg Asp Trp Ala Val Gln
595 600 605

Asn Pro Asn Ala Ala Gln Gly Ile Glu Ala Val Ser Asn Ile Phe Met
610 615 620

Ala Ala Ile Pro Ile Lys Gly Ile Gly Ala Val Arg Gly Lys Tyr Gly

```
625                 630                 635                 640

Leu Gly Gly Ile Thr Ala His Pro Ile Lys Arg Ser Gln Met Gly Ala
                645                 650                 655

Ile Ala Leu Pro Lys Gly Lys Ser Ala Val Ser Asp Asn Phe Ala Asp
            660                 665                 670

Ala Ala Tyr Ala Lys Tyr Pro Ser Pro Tyr His Ser Arg Asn Ile Arg
        675                 680                 685

Ser Asn Leu Glu Gln Arg Tyr Gly Lys Glu Asn Ile Thr Ser Ser Thr
    690                 695                 700

Val Pro Pro Ser Asn Gly Lys Asn Val Lys Leu Ala Asp Gln Arg His
705                 710                 715                 720

Pro Lys Thr Gly Val Pro Phe Asp Gly Lys Gly Phe Pro Asn Phe Glu
                725                 730                 735

Lys His Val Lys Tyr Asp Thr Leu Glu His His His His His His
            740                 745                 750
```

<210> 49
<211> 1773
<212> DNA
<213> Artificial Sequence

<220>
<223> 961c-741

<400> 49

```
atggccacaa acgacgacga tgttaaaaaa gctgccactg tggccattgc tgctgcctac    60
aacaatggcc aagaaatcaa cggtttcaaa gctggagaga ccatctacga cattgatgaa   120
gacggcacaa ttaccaaaaa agacgcaact gcagccgatg ttgaagccga cgactttaaa   180
ggtctgggtc tgaaaaaagt cgtgactaac ctgaccaaaa ccgtcaatga aaacaaacaa   240
aacgtcgatg ccaaagtaaa agctgcagaa tctgaaatag aaaagttaac aaccaagtta   300
gcagacactg atgccgcttt agcagatact gatgccgctc tggatgcaac caccaacgcc   360
ttgaataaat tgggagaaaa tataacgaca tttgctgaag agactaagac aaatatcgta   420
aaaattgatg aaaaattaga agccgtggct gataccgtcg acaagcatgc cgaagcattc   480
aacgatatcg ccgattcatt ggatgaaacc aacactaagg cagacgaagc cgtcaaaacc   540
gccaatgaag ccaaacagac ggccgaagaa accaaacaaa acgtcgatgc caaagtaaaa   600
gctgcagaaa ctgcagcagg caaagccgaa gctgccgctg gcacagctaa tactgcagcc   660
gacaaggccg aagctgtcgc tgcaaaagtt accgacatca aagctgatat cgctacgaac   720
aaagataata ttgctaaaaa agcaaacagt gccgacgtgt acaccagaga agagtctgac   780
agcaaatttg tcagaattga tggtctgaac gctactaccg aaaaattgga cacacgcttg   840
gcttctgctg aaaaatccat tgccgatcac gatactcgcc tgaacggttt ggataaaaca   900
gtgtcagacc tgcgcaaaga aacccgccaa ggccttgcag aacaagccgc gctctccggt   960
ctgttccaac cttacaacgt gggtggatcc ggagggggtg gtgtcgccgc cgacatcggt  1020
gcggggcttg ccgatgcact aaccgcaccg ctcgaccata aagacaaagg tttgcagtct  1080
ttgacgctgg atcagtccgt caggaaaaac gagaaactga agctggcggc acaaggtgcg  1140
gaaaaaactt atggaaacgg tgacagcctc aatacgggca aattgaagaa cgacaaggtc  1200
agccgtttcg actttatccg ccaaatcgaa gtggacgggc agctcattac cttggagagt  1260
ggagagttcc aagtatacaa acaaagccat tccgccttaa ccgcctttca gaccgagcaa  1320
atacaagatt cggagcattc cgggaagatg gttgcgaaac gccagttcag aatcggcgac  1380
atagcgggcg aacatacatc ttttgacaag cttcccgaag gcggcagggc gacatatcgc  1440
gggacggcgt tcggttcaga cgatgccggc ggaaaactga cctacaccat agatttcgcc  1500
gccaagcagg gaaacggcaa aatcgaacat ttgaaatcgc cagaactcaa tgtcgacctg  1560
gccgccgccg atatcaagcc ggatggaaaa cgccatgccg tcatcagcgg ttccgtcctt  1620
tacaaccaag ccgagaaagg cagttactcc ctcggtatct ttggcggaaa agcccaggaa  1680
gttgccggca gcgcggaagt gaaaaccgta aacggcatac gccatatcgg ccttgccgcc  1740

aagcaactcg agcaccacca ccaccaccac tga                                1773
```

<210> 50
<211> 590
<212> PRT <213> Artificial Sequence

<220>
<223> 961c-741

<400> 50

```
Met Ala Thr Asn Asp Asp Asp Val Lys Lys Ala Ala Thr Val Ala Ile
1               5                   10                  15

Ala Ala Ala Tyr Asn Asn Gly Gln Glu Ile Asn Gly Phe Lys Ala Gly
            20                  25                  30

Glu Thr Ile Tyr Asp Ile Asp Glu Asp Gly Thr Ile Thr Lys Lys Asp
        35                  40                  45

Ala Thr Ala Ala Asp Val Glu Ala Asp Asp Phe Lys Gly Leu Gly Leu
    50                  55                  60

Lys Lys Val Val Thr Asn Leu Thr Lys Thr Val Asn Glu Asn Lys Gln
65                  70                  75                  80

Asn Val Asp Ala Lys Val Lys Ala Ala Glu Ser Glu Ile Glu Lys Leu
                85                  90                  95

Thr Thr Lys Leu Ala Asp Thr Asp Ala Ala Leu Ala Asp Thr Asp Ala
            100                 105                 110

Ala Leu Asp Ala Thr Thr Asn Ala Leu Asn Lys Leu Gly Glu Asn Ile
        115                 120                 125

Thr Thr Phe Ala Glu Glu Thr Lys Thr Asn Ile Val Lys Ile Asp Glu
    130                 135                 140

Lys Leu Glu Ala Val Ala Asp Thr Val Asp Lys His Ala Glu Ala Phe
145                 150                 155                 160

Asn Asp Ile Ala Asp Ser Leu Asp Glu Thr Asn Thr Lys Ala Asp Glu
                165                 170                 175

Ala Val Lys Thr Ala Asn Glu Ala Lys Gln Thr Ala Glu Glu Thr Lys
            180                 185                 190

Gln Asn Val Asp Ala Lys Val Lys Ala Ala Glu Thr Ala Ala Gly Lys
        195                 200                 205

Ala Glu Ala Ala Ala Gly Thr Ala Asn Thr Ala Ala Asp Lys Ala Glu
    210                 215                 220

Ala Val Ala Ala Lys Val Thr Asp Ile Lys Ala Asp Ile Ala Thr Asn
225                 230                 235                 240

Lys Asp Asn Ile Ala Lys Lys Ala Asn Ser Ala Asp Val Tyr Thr Arg
                245                 250                 255

Glu Glu Ser Asp Ser Lys Phe Val Arg Ile Asp Gly Leu Asn Ala Thr
            260                 265                 270
```

```
Thr Glu Lys Leu Asp Thr Arg Leu Ala Ser Ala Glu Lys Ser Ile Ala
        275                 280                 285

Asp His Asp Thr Arg Leu Asn Gly Leu Asp Lys Thr Val Ser Asp Leu
    290                 295                 300

Arg Lys Glu Thr Arg Gln Gly Leu Ala Glu Gln Ala Ala Leu Ser Gly
305                 310                 315                 320

Leu Phe Gln Pro Tyr Asn Val Gly Gly Ser Gly Gly Gly Gly Val Ala
                325                 330                 335

Ala Asp Ile Gly Ala Gly Leu Ala Asp Ala Leu Thr Ala Pro Leu Asp
            340                 345                 350

His Lys Asp Lys Gly Leu Gln Ser Leu Thr Leu Asp Gln Ser Val Arg
        355                 360                 365

Lys Asn Glu Lys Leu Lys Leu Ala Ala Gln Gly Ala Glu Lys Thr Tyr
    370                 375                 380

Gly Asn Gly Asp Ser Leu Asn Thr Gly Lys Leu Lys Asn Asp Lys Val
385                 390                 395                 400

Ser Arg Phe Asp Phe Ile Arg Gln Ile Glu Val Asp Gly Gln Leu Ile
                405                 410                 415

Thr Leu Glu Ser Gly Glu Phe Gln Val Tyr Lys Gln Ser His Ser Ala
            420                 425                 430

Leu Thr Ala Phe Gln Thr Glu Gln Ile Gln Asp Ser Glu His Ser Gly
        435                 440                 445

Lys Met Val Ala Lys Arg Gln Phe Arg Ile Gly Asp Ile Ala Gly Glu
    450                 455                 460

His Thr Ser Phe Asp Lys Leu Pro Glu Gly Gly Arg Ala Thr Tyr Arg
465                 470                 475                 480

Gly Thr Ala Phe Gly Ser Asp Asp Ala Gly Gly Lys Leu Thr Tyr Thr
                485                 490                 495

Ile Asp Phe Ala Ala Lys Gln Gly Asn Gly Lys Ile Glu His Leu Lys
            500                 505                 510

Ser Pro Glu Leu Asn Val Asp Leu Ala Ala Ala Asp Ile Lys Pro Asp
        515                 520                 525

Gly Lys Arg His Ala Val Ile Ser Gly Ser Val Leu Tyr Asn Gln Ala
    530                 535                 540

Glu Lys Gly Ser Tyr Ser Leu Gly Ile Phe Gly Gly Lys Ala Gln Glu
545                 550                 555                 560

Val Ala Gly Ser Ala Glu Val Lys Thr Val Asn Gly Ile Arg His Ile
                565                 570                 575

Gly Leu Ala Ala Lys Gln Leu Glu His His His His His His
            580                 585                 590
```

<210> 51
<211> 4170
<212> DNA
<213> Artificial Sequence <220>

<223> 961c-983

<400> 51

atggccacaa acgacgacga tgttaaaaaa gctgccactg tggccattgc tgctgcctac 60
aacaatggcc aagaaatcaa cggtttcaaa gctggagaga ccatctacga cattgatgaa 120
gacggcacaa ttaccaaaaa agacgcaact gcagccgatg ttgaagccga cgactttaaa 180
ggtctgggtc tgaaaaaagt cgtgactaac ctgaccaaaa ccgtcaatga aaacaaacaa 240
aacgtcgatg ccaaagtaaa agctgcagaa tctgaaatag aaaagttaac aaccaagtta 300
gcagacactg atgccgcttt agcagatact gatgccgctc tggatgcaac caccaacgcc 360
ttgaataaat tgggagaaaa tataacgaca tttgctgaag agactaagac aaatatcgta 420
aaaattgatg aaaaattaga agccgtggct gataccgtcg acaagcatgc cgaagcattc 480
aacgatatcg ccgattcatt ggatgaaacc aacactaagg cagacgaagc cgtcaaaacc 540
gccaatgaag ccaaacagac ggccgaagaa accaaacaaa acgtcgatgc caaagtaaaa 600
gctgcagaaa ctgcagcagg caaagccgaa gctgccgctg gcacagctaa tactgcagcc 660
gacaaggccg aagctgtcgc tgcaaaagtt accgacatca aagctgatat cgctacgaac 720
aaagataata ttgctaaaaa agcaaacagt gccgacgtgt acaccagaga agagtctgac 780
agcaaatttg tcagaattga tggtctgaac gctactaccg aaaaattgga cacacgcttg 840
gcttctgctg aaaaatccat tgccgatcac gatactcgcc tgaacggttt ggataaaaca 900
gtgtcagacc tgcgcaaaga aacccgccaa ggccttgcag aacaagccgc gctctccggt 960
ctgttccaac cttacaacgt gggtggatcc ggcggaggcg gcacttctgc gcccgacttc 1020
aatgcaggcg gtaccggtat cggcagcaac agcagagcaa caacagcgaa atcagcagca 1080
gtatcttacg ccggtatcaa gaacgaaatg tgcaaagaca gaagcatgct ctgtgccggt 1140
cgggatgacg ttgcggttac agacagggat gccaaaatca atgccccccc cccgaatctg 1200
cataccggag actttccaaa cccaaatgac gcatacaaga atttgatcaa cctcaaacct 1260
gcaattgaag caggctatac aggacgcggg gtagaggtag gtatcgtcga cacaggcgaa 1320
tccgtcggca gcatatcctt tcccgaactg tatggcagaa aagaacacgg ctataacgaa 1380
aattacaaaa actatacggc gtatatgcgg aaggaagcgc ctgaagacgg aggcggtaaa 1440
gacattgaag cttctttcga cgatgaggcc gttatagaga ctgaagcaaa gccgacggat 1500
atccgccacg taaaagaaat cggacacatc gatttggtct cccatattat tggcgggcgt 1560
tccgtggacg gcagacctgc aggcggtatt gcgcccgatg cgacgctaca cataatgaat 1620
acgaatgatg aaaccaagaa cgaaatgatg gttgcagcca tccgcaatgc atgggtcaag 1680
ctgggcgaac gtggcgtgcg catcgtcaat aacagttttg gaacaacatc gagggcaggc 1740
actgccgacc ttttccaaat agccaattcg gaggagcagt accgccaagc gttgctcgac 1800
tattccggcg gtgataaaac agacgagggt atccgcctga tgcaacagag cgattacggc 1860
aacctgtcct accacatccg taataaaaac atgcttttca tcttttcgac aggcaatgac 1920
gcacaagctc agcccaacac atatgcccta ttgccatttt atgaaaaaga cgctcaaaaa 1980
ggcattatca cagtcgcagg cgtagaccgc agtggagaaa agttcaaacg ggaaatgtat 2040
ggagaaccgg gtacagaacc gcttgagtat ggctccaacc attgcggaat tactgccatg 2100
tggtgcctgt cggcacccta tgaagcaagc gtccgtttca cccgtacaaa cccgattcaa 2160
attgccggaa catccttttc cgcacccatc gtaaccggca cggcggctct gctgctgcag 2220
aaatacccgt ggatgagcaa cgacaacctg cgtaccacgt tgctgacgac ggctcaggac 2280
atcggtgcag tcggcgtgga cagcaagttc ggctggggac tgctggatgc gggtaaggcc 2340
atgaacggac ccgcgtcctt tccgttcggc gactttaccg ccgatacgaa aggtacatcc 2400
gatattgcct actccttccg taacgacatt tcaggcacgg gcggcctgat caaaaaaggc 2460
ggcagccaac tgcaactgca cggcaacaac acctatacgg gcaaaaccat tatcgaaggc 2520
ggttcgctgg tgttgtacgg caacaacaaa tcggatatgc gcgtcgaaac caaaggtgcg 2580
ctgatttata acggggcggc atccggcggc agcctgaaca gcgacggcat tgtctatctg 2640
gcagataccg accaatccgg cgcaaacgaa accgtacaca tcaaaggcag tctgcagctg 2700
gacggcaaag gtacgctgta cacacgtttg ggcaaactgc tgaaagtgga cggtacggcg 2760
attatcggcg gcaagctgta catgtcggca cgcggcaagg ggcaggcta tctcaacagt 2820
accggacgac gtgttccctt cctgagtgcc gccaaaatcg ggcaggatta ttctttcttc 2880
acaaacatcg aaaccgacgg cggcctgctg gcttccctcg acagcgtcga aaaaacagcg 2940
ggcagtgaag gcgacacgct gtcctattat gtccgtcgcg gcaatgcggc acggactgct 3000
tcggcagcgg cacattccgc gcccgccggt ctgaaacacg ccgtagaaca gggcggcagc 3060
aatctggaaa acctgatggt cgaactggat gcctccgaat catccgcaac acccgagacg 3120

```
gttgaaactg cggcagccga ccgcacagat atgccgggca tccgccccta cggcgcaact   3180
ttccgcgcag cggcagccgt acagcatgcg aatgccgccg acggtgtacg catcttcaac   3240
agtctcgccg ctaccgtcta tgccgacagt accgccgccc atgccgatat gcagggacgc   3300
cgcctgaaag ccgtatcgga cgggttggac cacaacggca cgggtctgcg cgtcatcgcg   3360
caaacccaac aggacggtgg aacgtgggaa cagggcggtg ttgaaggcaa aatgcgcggc   3420
agtacccaaa ccgtcggcat tgccgcgaaa accggcgaaa atacgacagc agccgccaca   3480
ctgggcatgg gacgcagcac atggagcgaa aacagtgcaa atgcaaaaac cgacagcatt   3540
agtctgtttg caggcatacg gcacgatgcg ggcgatatcg gctatctcaa aggcctgttc   3600
tcctacggac gctacaaaaa cagcatcagc cgcagcaccg gtgcggacga acatgcggaa   3660
ggcagcgtca acggcacgct gatgcagctg ggcgcactgg gcggtgtcaa cgttccgttt   3720
gccgcaacgg gagatttgac ggtcgaaggc ggtctgcgct acgacctgct caaacaggat   3780
gcattcgccg aaaaaggcag tgctttgggc tggagcggca acagcctcac tgaaggcacg   3840
ctggtcggac tcgcgggtct gaagctgtcg caacccttga gcgataaagc cgtcctgttt   3900
gcaacggcgg gcgtggaacg cgacctgaac ggacgcgact acacggtaac gggcggcttt   3960
accggcgcga ctgcagcaac cggcaagacg gggcacgca atatgccgca cacccgtctg    4020
gttgccggcc tgggcgcgga tgtcgaattc ggcaacggct ggaacggctt ggcacgttac   4080
agctacgccg gttccaaaca gtacggcaac cacagcggac gagtcggcgt aggctaccgg   4140
ttcctcgagc accaccacca ccaccactga                                    4170
```

<210> 52
<211> 1389
<212> PRT
<213> Artificial Sequence

<220>
<223> 961c-983

<400> 52

Met Ala Thr Asn Asp Asp Asp Val Lys Lys Ala Ala Thr Val Ala Ile
1 5 10 15

Ala Ala Ala Tyr Asn Asn Gly Gln Glu Ile Asn Gly Phe Lys Ala Gly
20 25 30

Glu Thr Ile Tyr Asp Ile Asp Glu Asp Gly Thr Ile Thr Lys Lys Asp
35 40 45

Ala Thr Ala Ala Asp Val Glu Ala Asp Asp Phe Lys Gly Leu Gly Leu
50 55 60

Lys Lys Val Val Thr Asn Leu Thr Lys Thr Val Asn Glu Asn Lys Gln
65 70 75 80

Asn Val Asp Ala Lys Val Lys Ala Ala Glu Ser Glu Ile Glu Lys Leu
85 90 95

Thr Thr Lys Leu Ala Asp Thr Asp Ala Ala Leu Ala Asp Thr Asp Ala
100 105 110

Ala Leu Asp Ala Thr Thr Asn Ala Leu Asn Lys Leu Gly Glu Asn Ile
115 120 125

Thr Thr Phe Ala Glu Glu Thr Lys Thr Asn Ile Val Lys Ile Asp Glu
130 135 140

Lys Leu Glu Ala Val Ala Asp Thr Val Asp Lys His Ala Glu Ala Phe
145 150 155 160

Asn Asp Ile Ala Asp Ser Leu Asp Glu Thr Asn Thr Lys Ala Asp Glu
165 170 175

```
Ala Val Lys Thr Ala Asn Glu Ala Lys Gln Thr Ala Glu Glu Thr Lys
            180                 185                 190

Gln Asn Val Asp Ala Lys Val Lys Ala Ala Glu Thr Ala Ala Gly Lys
        195                 200                 205

Ala Glu Ala Ala Ala Gly Thr Ala Asn Thr Ala Ala Asp Lys Ala Glu
    210                 215                 220

Ala Val Ala Ala Lys Val Thr Asp Ile Lys Ala Asp Ile Ala Thr Asn
225                 230                 235                 240

Lys Asp Asn Ile Ala Lys Lys Ala Asn Ser Ala Asp Val Tyr Thr Arg
                245                 250                 255

Glu Glu Ser Asp Ser Lys Phe Val Arg Ile Asp Gly Leu Asn Ala Thr
            260                 265                 270

Thr Glu Lys Leu Asp Thr Arg Leu Ala Ser Ala Glu Lys Ser Ile Ala
        275                 280                 285

Asp His Asp Thr Arg Leu Asn Gly Leu Asp Lys Thr Val Ser Asp Leu
    290                 295                 300

Arg Lys Glu Thr Arg Gln Gly Leu Ala Glu Gln Ala Ala Leu Ser Gly
305                 310                 315                 320

Leu Phe Gln Pro Tyr Asn Val Gly Gly Ser Gly Gly Gly Gly Thr Ser
                325                 330                 335

Ala Pro Asp Phe Asn Ala Gly Gly Thr Gly Ile Gly Ser Asn Ser Arg
            340                 345                 350

Ala Thr Thr Ala Lys Ser Ala Ala Val Ser Tyr Ala Gly Ile Lys Asn
        355                 360                 365

Glu Met Cys Lys Asp Arg Ser Met Leu Cys Ala Gly Arg Asp Asp Val
    370                 375                 380

Ala Val Thr Asp Arg Asp Ala Lys Ile Asn Ala Pro Pro Pro Asn Leu
385                 390                 395                 400

His Thr Gly Asp Phe Pro Asn Pro Asn Asp Ala Tyr Lys Asn Leu Ile
                405                 410                 415

Asn Leu Lys Pro Ala Ile Glu Ala Gly Tyr Thr Gly Arg Gly Val Glu
            420                 425                 430

Val Gly Ile Val Asp Thr Gly Glu Ser Val Gly Ser Ile Ser Phe Pro
        435                 440                 445

Glu Leu Tyr Gly Arg Lys Glu His Gly Tyr Asn Glu Asn Tyr Lys Asn
    450                 455                 460

Tyr Thr Ala Tyr Met Arg Lys Glu Ala Pro Glu Asp Gly Gly Gly Lys
465                 470                 475                 480

Asp Ile Glu Ala Ser Phe Asp Asp Glu Ala Val Ile Glu Thr Glu Ala
                485                 490                 495

Lys Pro Thr Asp Ile Arg His Val Lys Glu Ile Gly His Ile Asp Leu
```

500 505 510

Val Ser His Ile Ile Gly Gly Arg Ser Val Asp Gly Arg Pro Ala Gly
515 520 525

Gly Ile Ala Pro Asp Ala Thr Leu His Ile Met Asn Thr Asn Asp Glu
530 535 540

Thr Lys Asn Glu Met Met Val Ala Ala Ile Arg Asn Ala Trp Val Lys
545 550 555 560

Leu Gly Glu Arg Gly Val Arg Ile Val Asn Asn Ser Phe Gly Thr Thr
565 570 575

Ser Arg Ala Gly Thr Ala Asp Leu Phe Gln Ile Ala Asn Ser Glu Glu
580 585 590

Gln Tyr Arg Gln Ala Leu Leu Asp Tyr Ser Gly Gly Asp Lys Thr Asp
595 600 605

Glu Gly Ile Arg Leu Met Gln Gln Ser Asp Tyr Gly Asn Leu Ser Tyr
610 615 620

His Ile Arg Asn Lys Asn Met Leu Phe Ile Phe Ser Thr Gly Asn Asp
625 630 635 640

Ala Gln Ala Gln Pro Asn Thr Tyr Ala Leu Leu Pro Phe Tyr Glu Lys
645 650 655

Asp Ala Gln Lys Gly Ile Ile Thr Val Ala Gly Val Asp Arg Ser Gly
660 665 670

Glu Lys Phe Lys Arg Glu Met Tyr Gly Glu Pro Gly Thr Glu Pro Leu
675 680 685

Glu Tyr Gly Ser Asn His Cys Gly Ile Thr Ala Met Trp Cys Leu Ser
690 695 700

Ala Pro Tyr Glu Ala Ser Val Arg Phe Thr Arg Thr Asn Pro Ile Gln
705 710 715 720

Ile Ala Gly Thr Ser Phe Ser Ala Pro Ile Val Thr Gly Thr Ala Ala
725 730 735

Leu Leu Leu Gln Lys Tyr Pro Trp Met Ser Asn Asp Asn Leu Arg Thr
740 745 750

Thr Leu Leu Thr Thr Ala Gln Asp Ile Gly Ala Val Gly Val Asp Ser
755 760 765

Lys Phe Gly Trp Gly Leu Leu Asp Ala Gly Lys Ala Met Asn Gly Pro
770 775 780

Ala Ser Phe Pro Phe Gly Asp Phe Thr Ala Asp Thr Lys Gly Thr Ser
785 790 795 800

Asp Ile Ala Tyr Ser Phe Arg Asn Asp Ile Ser Gly Thr Gly Gly Leu
805 810 815

Ile Lys Lys Gly Gly Ser Gln Leu Gln Leu His Gly Asn Asn Thr Tyr
820 825 830

Thr Gly Lys Thr Ile Ile Glu Gly Gly Ser Leu Val Leu Tyr Gly Asn
835 840 845

Asn Lys Ser Asp Met Arg Val Glu Thr Lys Gly Ala Leu Ile Tyr Asn
850 855 860

Gly Ala Ala Ser Gly Gly Ser Leu Asn Ser Asp Gly Ile Val Tyr Leu
865 870 875 880

Ala Asp Thr Asp Gln Ser Gly Ala Asn Glu Thr Val His Ile Lys Gly
885 890 895

Ser Leu Gln Leu Asp Gly Lys Gly Thr Leu Tyr Thr Arg Leu Gly Lys
900 905 910

Leu Leu Lys Val Asp Gly Thr Ala Ile Ile Gly Gly Lys Leu Tyr Met
915 920 925

Ser Ala Arg Gly Lys Gly Ala Gly Tyr Leu Asn Ser Thr Gly Arg Arg
930 935 940

Val Pro Phe Leu Ser Ala Ala Lys Ile Gly Gln Asp Tyr Ser Phe Phe
945 950 955 960

Thr Asn Ile Glu Thr Asp Gly Gly Leu Leu Ala Ser Leu Asp Ser Val
965 970 975

Glu Lys Thr Ala Gly Ser Glu Gly Asp Thr Leu Ser Tyr Tyr Val Arg
980 985 990

Arg Gly Asn Ala Ala Arg Thr Ala Ser Ala Ala Ala His Ser Ala Pro
995 1000 1005

Ala Gly Leu Lys His Ala Val Glu Gln Gly Gly Ser Asn Leu Glu Asn
1010 1015 1020

Leu Met Val Glu Leu Asp Ala Ser Glu Ser Ser Ala Thr Pro Glu Thr
1025 1030 1035 1040

Val Glu Thr Ala Ala Ala Asp Arg Thr Asp Met Pro Gly Ile Arg Pro
1045 1050 1055

Tyr Gly Ala Thr Phe Arg Ala Ala Ala Ala Val Gln His Ala Asn Ala
1060 1065 1070

Ala Asp Gly Val Arg Ile Phe Asn Ser Leu Ala Ala Thr Val Tyr Ala
1075 1080 1085

Asp Ser Thr Ala Ala His Ala Asp Met Gln Gly Arg Arg Leu Lys Ala
1090 1095 1100

Val Ser Asp Gly Leu Asp His Asn Gly Thr Gly Leu Arg Val Ile Ala
1105 1110 1115 1120

Gln Thr Gln Gln Asp Gly Gly Thr Trp Glu Gln Gly Gly Val Glu Gly
1125 1130 1135

Lys Met Arg Gly Ser Thr Gln Thr Val Gly Ile Ala Ala Lys Thr Gly
1140 1145 1150

```
Glu Asn Thr Thr Ala Ala Ala Thr Leu Gly Met Gly Arg Ser Thr Trp
          1155                1160                1165

Ser Glu Asn Ser Ala Asn Ala Lys Thr Asp Ser Ile Ser Leu Phe Ala
    1170                1175                1180

Gly Ile Arg His Asp Ala Gly Asp Ile Gly Tyr Leu Lys Gly Leu Phe
1185                1190                1195                1200

Ser Tyr Gly Arg Tyr Lys Asn Ser Ile Ser Arg Ser Thr Gly Ala Asp
                1205                1210                1215

Glu His Ala Glu Gly Ser Val Asn Gly Thr Leu Met Gln Leu Gly Ala
            1220                1225                1230

Leu Gly Gly Val Asn Val Pro Phe Ala Ala Thr Gly Asp Leu Thr Val
        1235                1240                1245

Glu Gly Gly Leu Arg Tyr Asp Leu Leu Lys Gln Asp Ala Phe Ala Glu
    1250                1255                1260

Lys Gly Ser Ala Leu Gly Trp Ser Gly Asn Ser Leu Thr Glu Gly Thr
1265                1270                1275                1280

Leu Val Gly Leu Ala Gly Leu Lys Leu Ser Gln Pro Leu Ser Asp Lys
                1285                1290                1295

Ala Val Leu Phe Ala Thr Ala Gly Val Glu Arg Asp Leu Asn Gly Arg
            1300                1305                1310

Asp Tyr Thr Val Thr Gly Gly Phe Thr Gly Ala Thr Ala Ala Thr Gly
        1315                1320                1325

Lys Thr Gly Ala Arg Asn Met Pro His Thr Arg Leu Val Ala Gly Leu
    1330                1335                1340

Gly Ala Asp Val Glu Phe Gly Asn Gly Trp Asn Gly Leu Ala Arg Tyr
1345                1350                1355                1360

Ser Tyr Ala Gly Ser Lys Gln Tyr Gly Asn His Ser Gly Arg Val Gly
                1365                1370                1375

Val Gly Tyr Arg Phe Leu Glu His His His His His His
            1380                1385
```

<210> 53
<211> 2304
<212> DNA
<213> Artificial Sequence

<220>
<223> 961cL-ORF46.1

<400> 53

```
atgaaacact ttccatccaa agtactgacc acagccatcc ttgccacttt ctgtagcggc   60
gcactggcag ccacaaacga cgacgatgtt aaaaaagctg ccactgtggc cattgctgct  120
gcctacaaca atggccaaga aatcaacggt ttcaaagctg gagagaccat ctacgacatt  180
gatgaagacg gcacaattac caaaaaagac gcaactgcag ccgatgttga agccgacgac  240
tttaaaggtc tgggtctgaa aaaagtcgtg actaacctga ccaaaaccgt caatgaaaac  300
aaacaaaacg tcgatgccaa agtaaaagct gcagaatctg aaatagaaaa gttaacaacc  360
aagttagcag acactgatgc cgctttagca gatactgatg ccgctctgga tgcaaccacc  420
```

```
aacgccttga ataaattggg agaaaatata acgacatttg ctgaagagac taagacaaat   480
atcgtaaaaa ttgatgaaaa attagaagcc gtggctgata ccgtcgacaa gcatgccgaa   540
gcattcaacg atatcgccga ttcattggat gaaaccaaca ctaaggcaga cgaagccgtc   600
aaaaccgcca atgaagccaa acagacggcc gaagaaacca aacaaaacgt cgatgccaaa   660
gtaaaagctg cagaaactgc agcaggcaaa gccgaagctg ccgctggcac agctaatact   720
gcagccgaca aggccgaagc tgtcgctgca aaagttaccg acatcaaagc tgatatcgct   780
acgaacaaag ataatattgc taaaaaagca aacagtgccg acgtgtacac cagagaagag   840
tctgacagca aatttgtcag aattgatggt ctgaacgcta ctaccgaaaa attggacaca   900
cgcttggctt ctgctgaaaa atccattgcc gatcacgata ctcgcctgaa cggtttggat   960
aaaacagtgt cagacctgcg caaagaaacc cgccaaggcc ttgcagaaca agccgcgctc  1020
tccggtctgt tccaacctta caacgtgggt ggatccggag gaggaggatc agatttggca  1080
aacgattctt ttatccggca ggttctcgac cgtcagcatt tcgaacccga cgggaaatac  1140
cacctattcg gcagcagggg ggaacttgcc gagcgcagcg gccatatcgg attgggaaaa  1200
atacaaagcc atcagttggg caacctgatg attcaacagg cggccattaa aggaaatatc  1260
ggctacattg tccgcttttc cgatcacggg cacgaagtcc attcccctt cgacaaccat   1320
gcctcacatt ccgattctga tgaagccggt agtcccgttg acggatttag cctttaccgc  1380
atccattggg acggatacga acaccatccc gccgacggct atgacgggcc acagggcggc  1440
ggctatcccg ctcccaaagg cgcgagggat atatacagct acgacataaa aggcgttgcc  1500
caaaatatcc gcctcaacct gaccgacaac cgcagcaccg gacaacggct tgccgaccgt  1560
ttccacaatg ccggtagtat gctgacgcaa ggagtaggcg acggattcaa acgcgccacc  1620
cgatacagcc ccgagctgga cagatcgggc aatgccgccg aagccttcaa cggcactgca  1680
gatatcgtta aaaacatcat cggcgcggca ggagaaattg tcggcgcagg cgatgccgtg  1740
cagggcataa gcgaaggctc aaacattgct gtcatgcacg gcttgggtct gctttccacc  1800
gaaaacaaga tggcgcgcat caacgatttg gcagatatgg cgcaactcaa agactatgcc  1860
gcagcagcca tccgcgattg ggcagtccaa aaccccaatg ccgcacaagg catagaagcc  1920
gtcagcaata tctttatggc agccatcccc atcaaaggga ttggagctgt tcggggaaaa  1980
tacggcttgg gcggcatcac ggcacatcct atcaagcggt cgcagatggg cgcgatcgca  2040
ttgccgaaag ggaaatccgc cgtcagcgac aattttgccg atgcggcata cgccaaatac  2100
ccgtcccctt accattcccg aaatatccgt tcaaacttgg agcagcgtta cggcaaagaa  2160
aacatcacct cctcaaccgt gccgccgtca aacggcaaaa atgtcaaact ggcagaccaa  2220
cgccacccga agacaggcgt accgtttgac ggtaaagggt ttccgaattt tgagaagcac  2280
gtgaaatatg atacgtaact cgag                                         2304
```

<210> 54
<211> 765
<212> PRT
<213> Artificial Sequence

<220>
<223> 961cL-ORF46.1

<400> 54

```
Met Lys His Phe Pro Ser Lys Val Leu Thr Thr Ala Ile Leu Ala Thr
1               5                   10                  15

Phe Cys Ser Gly Ala Leu Ala Ala Thr Asn Asp Asp Asp Val Lys Lys
            20                  25                  30

Ala Ala Thr Val Ala Ile Ala Ala Ala Tyr Asn Asn Gly Gln Glu Ile
        35                  40                  45

Asn Gly Phe Lys Ala Gly Glu Thr Ile Tyr Asp Ile Asp Glu Asp Gly
    50                  55                  60

Thr Ile Thr Lys Lys Asp Ala Thr Ala Ala Asp Val Glu Ala Asp Asp
65                  70                  75                  80

Phe Lys Gly Leu Gly Leu Lys Lys Val Val Thr Asn Leu Thr Lys Thr
                85                  90                  95

Val Asn Glu Asn Lys Gln Asn Val Asp Ala Lys Val Lys Ala Ala Glu
```

100 105 110

Ser Glu Ile Glu Lys Leu Thr Thr Lys Leu Ala Asp Thr Asp Ala Ala
115 120 125

Leu Ala Asp Thr Asp Ala Ala Leu Asp Ala Thr Thr Asn Ala Leu Asn
130 135 140

Lys Leu Gly Glu Asn Ile Thr Thr Phe Ala Glu Glu Thr Lys Thr Asn
145 150 155 160

Ile Val Lys Ile Asp Glu Lys Leu Glu Ala Val Ala Asp Thr Val Asp
165 170 175

Lys His Ala Glu Ala Phe Asn Asp Ile Ala Asp Ser Leu Asp Glu Thr
180 185 190

Asn Thr Lys Ala Asp Glu Ala Val Lys Thr Ala Asn Glu Ala Lys Gln
195 200 205

Thr Ala Glu Glu Thr Lys Gln Asn Val Asp Ala Lys Val Lys Ala Ala
210 215 220

Glu Thr Ala Ala Gly Lys Ala Glu Ala Ala Ala Gly Thr Ala Asn Thr
225 230 235 240

Ala Ala Asp Lys Ala Glu Ala Val Ala Ala Lys Val Thr Asp Ile Lys
245 250 255

Ala Asp Ile Ala Thr Asn Lys Asp Asn Ile Ala Lys Lys Ala Asn Ser
260 265 270

Ala Asp Val Tyr Thr Arg Glu Glu Ser Asp Ser Lys Phe Val Arg Ile
275 280 285

Asp Gly Leu Asn Ala Thr Thr Glu Lys Leu Asp Thr Arg Leu Ala Ser
290 295 300

Ala Glu Lys Ser Ile Ala Asp His Asp Thr Arg Leu Asn Gly Leu Asp
305 310 315 320

Lys Thr Val Ser Asp Leu Arg Lys Glu Thr Arg Gln Gly Leu Ala Glu
325 330 335

Gln Ala Ala Leu Ser Gly Leu Phe Gln Pro Tyr Asn Val Gly Gly Ser
340 345 350

Gly Gly Gly Gly Ser Asp Leu Ala Asn Asp Ser Phe Ile Arg Gln Val
355 360 365

Leu Asp Arg Gln His Phe Glu Pro Asp Gly Lys Tyr His Leu Phe Gly
370 375 380

Ser Arg Gly Glu Leu Ala Glu Arg Ser Gly His Ile Gly Leu Gly Lys
385 390 395 400

Ile Gln Ser His Gln Leu Gly Asn Leu Met Ile Gln Gln Ala Ala Ile
405 410 415

Lys Gly Asn Ile Gly Tyr Ile Val Arg Phe Ser Asp His Gly His Glu
420 425 430

```
Val His Ser Pro Phe Asp Asn His Ala Ser His Ser Asp Ser Asp Glu
        435                 440                 445

Ala Gly Ser Pro Val Asp Gly Phe Ser Leu Tyr Arg Ile His Trp Asp
    450                 455                 460

Gly Tyr Glu His His Pro Ala Asp Gly Tyr Asp Gly Pro Gln Gly Gly
465                 470                 475                 480

Gly Tyr Pro Ala Pro Lys Gly Ala Arg Asp Ile Tyr Ser Tyr Asp Ile
                485                 490                 495

Lys Gly Val Ala Gln Asn Ile Arg Leu Asn Leu Thr Asp Asn Arg Ser
            500                 505                 510

Thr Gly Gln Arg Leu Ala Asp Arg Phe His Asn Ala Gly Ser Met Leu
        515                 520                 525

Thr Gln Gly Val Gly Asp Gly Phe Lys Arg Ala Thr Arg Tyr Ser Pro
    530                 535                 540

Glu Leu Asp Arg Ser Gly Asn Ala Ala Glu Ala Phe Asn Gly Thr Ala
545                 550                 555                 560

Asp Ile Val Lys Asn Ile Ile Gly Ala Ala Gly Glu Ile Val Gly Ala
                565                 570                 575

Gly Asp Ala Val Gln Gly Ile Ser Glu Gly Ser Asn Ile Ala Val Met
            580                 585                 590

His Gly Leu Gly Leu Leu Ser Thr Glu Asn Lys Met Ala Arg Ile Asn
        595                 600                 605

Asp Leu Ala Asp Met Ala Gln Leu Lys Asp Tyr Ala Ala Ala Ala Ile
    610                 615                 620

Arg Asp Trp Ala Val Gln Asn Pro Asn Ala Ala Gln Gly Ile Glu Ala
625                 630                 635                 640

Val Ser Asn Ile Phe Met Ala Ala Ile Pro Ile Lys Gly Ile Gly Ala
                645                 650                 655

Val Arg Gly Lys Tyr Gly Leu Gly Gly Ile Thr Ala His Pro Ile Lys
            660                 665                 670

Arg Ser Gln Met Gly Ala Ile Ala Leu Pro Lys Gly Lys Ser Ala Val
        675                 680                 685

Ser Asp Asn Phe Ala Asp Ala Ala Tyr Ala Lys Tyr Pro Ser Pro Tyr
    690                 695                 700

His Ser Arg Asn Ile Arg Ser Asn Leu Glu Gln Arg Tyr Gly Lys Glu
705                 710                 715                 720

Asn Ile Thr Ser Ser Thr Val Pro Pro Ser Asn Gly Lys Asn Val Lys
                725                 730                 735

Leu Ala Asp Gln Arg His Pro Lys Thr Gly Val Pro Phe Asp Gly Lys
            740                 745                 750
```

Gly Phe Pro Asn Phe Glu Lys His Val Lys Tyr Asp Thr
755 760 765

<210> 55
<211> 1839
<212> DNA
<213> Artificial Sequence

<220>
<223> 961cL-741

<400> 55

```
atgaaacact ttccatccaa agtactgacc acagccatcc ttgccacttt ctgtagcggc   60
gcactggcag ccacaaacga cgacgatgtt aaaaaagctg ccactgtggc cattgctgct  120
gcctacaaca atggccaaga aatcaacggt ttcaaagctg gagagaccat ctacgacatt  180
gatgaagacg gcacaattac caaaaaagac gcaactgcag ccgatgttga agccgacgac  240
tttaaaggtc tgggtctgaa aaaagtcgtg actaacctga ccaaaaccgt caatgaaaac  300
aaacaaaacg tcgatgccaa agtaaaagct gcagaatctg aaatagaaaa gttaacaacc  360
aagttagcag acactgatgc cgctttagca gatactgatg ccgctctgga tgcaaccacc  420
aacgccttga ataaattggg agaaaatata acgacatttg ctgaagagac taagacaaat  480
atcgtaaaaa ttgatgaaaa attagaagcc gtggctgata ccgtcgacaa gcatgccgaa  540
gcattcaacg atatcgccga ttcattggat gaaaccaaca ctaaggcaga cgaagccgtc  600
aaaaccgcca atgaagccaa acagacggcc gaagaaacca aacaaaacgt cgatgccaaa  660
gtaaaagctg cagaaactgc agcaggcaaa gccgaagctg ccgctggcac agctaatact  720
gcagccgaca aggccgaagc tgtcgctgca aaagttaccg acatcaaagc tgatatcgct  780
acgaacaaag ataatattgc taaaaaagca aacagtgccg acgtgtacac cagagaagag  840
tctgacagca aatttgtcag aattgatggt ctgaacgcta ctaccgaaaa attggacaca  900
cgcttggctt ctgctgaaaa atccattgcc gatcacgata ctcgcctgaa cggtttggat  960
aaaacagtgt cagacctgcg caaagaaacc cgccaaggcc ttgcagaaca agccgcgctc 1020
tccggtctgt tccaacctta caacgtgggt ggatccggag ggggtggtgt cgccgccgac 1080
atcggtgcgg ggcttgccga tgcactaacc gcaccgctcg accataaaga caaaggtttg 1140
cagtctttga cgctggatca gtccgtcagg aaaaacgaga aactgaagct ggcggcacaa 1200
ggtgcggaaa aaacttatgg aaacggtgac agcctcaata cgggcaaatt gaagaacgac 1260
aaggtcagcc gtttcgactt tatccgccaa atcgaagtgg acgggcagct cattaccttg 1320
gagagtggag agttccaagt atacaaacaa agccattccg ccttaaccgc ctttcagacc 1380
gagcaaatac aagattcgga gcattccggg aagatggttg cgaaacgcca gttcagaatc 1440
ggcgacatag cgggcgaaca tacatctttt gacaagcttc ccgaaggcgg cagggcgaca 1500
tatcgcggga cggcgttcgg ttcagacgat gccggcggaa aactgaccta caccatagat 1560
ttcgccgcca agcagggaaa cggcaaaatc gaacatttga aatcgccaga actcaatgtc 1620
gacctggccg ccgccgatat caagccggat ggaaaacgcc atgccgtcat cagcggttcc 1680
gtcctttaca accaagccga gaaaggcagt tactccctcg gtatctttgg cggaaaagcc 1740
caggaagttg ccggcagcgc ggaagtgaaa accgtaaacg gcatacgcca tatcggcctt 1800
gccgccaagc aactcgagca ccaccaccac caccactga                        1839
```

<210> 56
<211> 612
<212> PRT
<213> Artificial Sequence

<220>
<223> 961cL-741

<400> 56

```
Met Lys His Phe Pro Ser Lys Val Leu Thr Thr Ala Ile Leu Ala Thr
1               5                   10                  15

Phe Cys Ser Gly Ala Leu Ala Ala Thr Asn Asp Asp Asp Val Lys Lys
            20                  25                  30

Ala Ala Thr Val Ala Ile Ala Ala Ala Tyr Asn Asn Gly Gln Glu Ile
        35                  40                  45
```

Asn Gly Phe Lys Ala Gly Glu Thr Ile Tyr Asp Ile Asp Glu Asp Gly
50 55 60

Thr Ile Thr Lys Lys Asp Ala Thr Ala Ala Asp Val Glu Ala Asp Asp
65 70 75 80

Phe Lys Gly Leu Gly Leu Lys Lys Val Val Thr Asn Leu Thr Lys Thr
85 90 95

Val Asn Glu Asn Lys Gln Asn Val Asp Ala Lys Val Lys Ala Ala Glu
100 105 110

Ser Glu Ile Glu Lys Leu Thr Thr Lys Leu Ala Asp Thr Asp Ala Ala
115 120 125

Leu Ala Asp Thr Asp Ala Ala Leu Asp Ala Thr Thr Asn Ala Leu Asn
130 135 140

Lys Leu Gly Glu Asn Ile Thr Thr Phe Ala Glu Glu Thr Lys Thr Asn
145 150 155 160

Ile Val Lys Ile Asp Glu Lys Leu Glu Ala Val Ala Asp Thr Val Asp
165 170 175

Lys His Ala Glu Ala Phe Asn Asp Ile Ala Asp Ser Leu Asp Glu Thr
180 185 190

Asn Thr Lys Ala Asp Glu Ala Val Lys Thr Ala Asn Glu Ala Lys Gln
195 200 205

Thr Ala Glu Glu Thr Lys Gln Asn Val Asp Ala Lys Val Lys Ala Ala
210 215 220

Glu Thr Ala Ala Gly Lys Ala Glu Ala Ala Ala Gly Thr Ala Asn Thr
225 230 235 240

Ala Ala Asp Lys Ala Glu Ala Val Ala Ala Lys Val Thr Asp Ile Lys
245 250 255

Ala Asp Ile Ala Thr Asn Lys Asp Asn Ile Ala Lys Lys Ala Asn Ser
260 265 270

Ala Asp Val Tyr Thr Arg Glu Glu Ser Asp Ser Lys Phe Val Arg Ile
275 280 285

Asp Gly Leu Asn Ala Thr Thr Glu Lys Leu Asp Thr Arg Leu Ala Ser
290 295 300

Ala Glu Lys Ser Ile Ala Asp His Asp Thr Arg Leu Asn Gly Leu Asp
305 310 315 320

Lys Thr Val Ser Asp Leu Arg Lys Glu Thr Arg Gln Gly Leu Ala Glu
325 330 335

Gln Ala Ala Leu Ser Gly Leu Phe Gln Pro Tyr Asn Val Gly Gly Ser
340 345 350

Gly Gly Gly Gly Val Ala Ala Asp Ile Gly Ala Gly Leu Ala Asp Ala
355 360 365

Leu Thr Ala Pro Leu Asp His Lys Asp Lys Gly Leu Gln Ser Leu Thr
    370                 375                 380

Leu Asp Gln Ser Val Arg Lys Asn Glu Lys Leu Lys Leu Ala Ala Gln
385                 390                 395                 400

Gly Ala Glu Lys Thr Tyr Gly Asn Gly Asp Ser Leu Asn Thr Gly Lys
                405                 410                 415

Leu Lys Asn Asp Lys Val Ser Arg Phe Asp Phe Ile Arg Gln Ile Glu
            420                 425                 430

Val Asp Gly Gln Leu Ile Thr Leu Glu Ser Gly Glu Phe Gln Val Tyr
        435                 440                 445

Lys Gln Ser His Ser Ala Leu Thr Ala Phe Gln Thr Glu Gln Ile Gln
    450                 455                 460

Asp Ser Glu His Ser Gly Lys Met Val Ala Lys Arg Gln Phe Arg Ile
465                 470                 475                 480

Gly Asp Ile Ala Gly Glu His Thr Ser Phe Asp Lys Leu Pro Glu Gly
                485                 490                 495

Gly Arg Ala Thr Tyr Arg Gly Thr Ala Phe Gly Ser Asp Asp Ala Gly
            500                 505                 510

Gly Lys Leu Thr Tyr Thr Ile Asp Phe Ala Ala Lys Gln Gly Asn Gly
        515                 520                 525

Lys Ile Glu His Leu Lys Ser Pro Glu Leu Asn Val Asp Leu Ala Ala
    530                 535                 540

Ala Asp Ile Lys Pro Asp Gly Lys Arg His Ala Val Ile Ser Gly Ser
545                 550                 555                 560

Val Leu Tyr Asn Gln Ala Glu Lys Gly Ser Tyr Ser Leu Gly Ile Phe
                565                 570                 575

Gly Gly Lys Ala Gln Glu Val Ala Gly Ser Ala Glu Val Lys Thr Val
            580                 585                 590

Asn Gly Ile Arg His Ile Gly Leu Ala Ala Lys Gln Leu Glu His His
        595                 600                 605

His His His His
    610

<210> 57
<211> 4218
<212> DNA
<213> Artificial Sequence

<220>
<223> 961cL-983

<400> 57

```
atgaaacact ttccatccaa agtactgacc acagccatcc ttgccacttt ctgtagcggc   60
gcactggcag ccacaaacga cgacgatgtt aaaaaagctg ccactgtggc cattgctgct  120
gcctacaaca atggccaaga aatcaacggt ttcaaagctg gagagaccat ctacgacatt  180
gatgaagacg gcacaattac caaaaaagac gcaactgcag ccgatgttga agccgacgac  240
```

```
tttaaaggtc tgggtctgaa aaaagtcgtg actaacctga ccaaaaccgt caatgaaaac   300
aaacaaaacg tcgatgccaa agtaaaagct gcagaatctg aaatagaaaa gttaacaacc   360
aagttagcag acactgatgc cgctttagca gatactgatg ccgctctgga tgcaaccacc   420
aacgccttga ataaattggg agaaaatata acgacatttg ctgaagagac taagacaaat   480
atcgtaaaaa ttgatgaaaa attagaagcc gtggctgata ccgtcgacaa gcatgccgaa   540
gcattcaacg atatcgccga ttcattggat gaaaccaaca ctaaggcaga cgaagccgtc   600
aaaaccgcca atgaagccaa acagacggcc gaagaaacca aacaaaacgt cgatgccaaa   660
gtaaaagctg cagaaactgc agcaggcaaa gccgaagctg ccgctggcac agctaatact   720
gcagccgaca aggccgaagc tgtcgctgca aaagttaccg acatcaaagc tgatatcgct   780
acgaacaaag ataatattgc taaaaaagca aacagtgccg acgtgtacac cagagaagag   840
tctgacagca aatttgtcag aattgatggt ctgaacgcta ctaccgaaaa attggacaca   900
cgcttggctt ctgctgaaaa atccattgcc gatcacgata ctcgcctgaa cggtttggat   960
aaaacagtgt cagacctgcg caaagaaacc cgccaaggcc ttgcagaaca agccgcgctc   1020
tccggtctgt tccaacctta caacgtgggt ggatccggcg gaggcggcac ttctgcgccc   1080
gacttcaatg caggcggtac cggtatcggc agcaacagca gagcaacaac agcgaaatca   1140
gcagcagtat cttacgccgg tatcaagaac gaaatgtgca aagacagaag catgctctgt   1200
gccggtcggg atgacgttgc ggttacagac agggatgcca aaatcaatgc cccccccccg   1260
aatctgcata ccggagactt tccaaaccca aatgacgcat acaagaattt gatcaacctc   1320
aaacctgcaa ttgaagcagg ctatacagga cgcggggtag aggtaggtat cgtcgacaca   1380
ggcgaatccg tcggcagcat atcctttccc gaactgtatg gcagaaaaga acacggctat   1440
aacgaaaatt acaaaaacta tacggcgtat atgcggaagg aagcgcctga agacggaggc   1500
ggtaaagaca ttgaagcttc tttcgacgat gaggccgtta tagagactga agcaaagccg   1560
acggatatcc gccacgtaaa agaaatcgga cacatcgatt tggtctccca tattattggc   1620
gggcgttccg tggacggcag acctgcaggc ggtattgcgc ccgatgcgac gctacacata   1680
atgaatacga atgatgaaac caagaacgaa atgatggttg cagccatccg caatgcatgg   1740
gtcaagctgg gcgaacgtgg cgtgcgcatc gtcaataaca gttttggaac aacatcgagg   1800
gcaggcactg ccgacctttt ccaaatagcc aattcggagg agcagtaccg ccaagcgttg   1860
ctcgactatt ccggcggtga taaaacagac gagggtatcc gcctgatgca acagagcgat   1920
tacggcaacc tgtcctacca catccgtaat aaaaacatgc ttttcatctt ttcgacaggc   1980
aatgacgcac aagctcagcc caacacatat gccctattgc cattttatga aaagacgct    2040
caaaaaggca ttatcacagt cgcaggcgta gaccgcagtg gagaaaagtt caaacgggaa   2100
atgtatggag aaccgggtac agaaccgctt gagtatggct ccaaccattg cggaattact   2160
gccatgtggt gcctgtcggc accctatgaa gcaagcgtcc gtttcacccg tacaaacccg   2220
attcaaattg ccggaacatc cttttccgca cccatcgtaa ccggcacggc ggctctgctg   2280
ctgcagaaat acccgtggat gagcaacgac aacctgcgta ccacgttgct gacgacggct   2340
caggacatcg gtgcagtcgg cgtggacagc aagttcggct ggggactgct ggatgcgggt   2400
aaggccatga acggacccgc gtcctttccg ttcggcgact ttaccgccga tacgaaaggt   2460
acatccgata ttgcctactc cttccgtaac gacatttcag gcacgggcgg cctgatcaaa   2520
aaaggcggca gccaactgca actgcacggc aacaacacct atacgggcaa aaccattatc   2580
gaaggcggtt cgctggtgtt gtacggcaac aacaaatcgg atatgcgcgt cgaaaccaaa   2640
ggtgcgctga tttataacgg ggcggcatcc ggcggcagcc tgaacagcga cggcattgtc   2700
tatctggcag ataccgacca atccggcgca aacgaaaccg tacacatcaa aggcagtctg   2760
cagctggacg gcaaaggtac gctgtacaca cgtttgggca aactgctgaa agtggacggt   2820
acggcgatta tcggcggcaa gctgtacatg tcggcacgcg gcaagggggc aggctatctc   2880
aacagtaccg gacgacgtgt tcccttcctg agtgccgcca aaatcgggca ggattattct   2940
ttcttcacaa acatcgaaac cgacggcggc ctgctggctt ccctcgacag cgtcgaaaaa   3000
acagcgggca gtgaaggcga cacgctgtcc tattatgtcc gtcgcggcaa tgcggcacgg   3060
actgcttcgg cagcggcaca ttccgcgccc gccggtctga aacacgccgt agaacagggc   3120
ggcagcaatc tggaaaacct gatggtcgaa ctggatgcct ccgaatcatc cgcaacaccc   3180
gagacggttg aaactgcggc agccgaccgc acagatatgc cgggcatccg cccctacggc   3240
gcaactttcc gcgcagcggc agccgtacag catgcgaatg ccgccgacgg tgtacgcatc   3300
ttcaacagtc tcgccgctac cgtctatgcc gacagtaccg ccgcccatgc cgatatgcag   3360
ggacgccgcc tgaaagccgt atcggacggg ttggaccaca acggcacggg tctgcgcgtc   3420
atcgcgcaaa cccaacagga cggtggaacg tgggaacagg gcggtgttga aggcaaaatg   3480
cgcggcagta cccaaaccgt cggcattgcc gcgaaaaccg gcgaaaatac gacagcagcc   3540
gccacactgg gcatgggacg cagcacatgg agcgaaaaca gtgcaaatgc aaaaaccgac   3600
agcattagtc tgtttgcagg catacggcac gatgcgggcg atatcggcta tctcaaaggc   3660
ctgttctcct acggacgcta caaaaacagc atcagccgca gcaccggtgc ggacgaacat   3720
gcggaaggca gcgtcaacgg cacgctgatg cagctgggcg cactgggcgg tgtcaacgtt   3780
ccgtttgccg caacgggaga tttgacggtc gaaggcggtc tgcgctacga cctgctcaaa   3840
caggatgcat tcgccgaaaa aggcagtgct ttgggctgga gcggcaacag cctcactgaa   3900
```

```
ggcacgctgg tcggactcgc gggtctgaag ctgtcgcaac ccttgagcga taaagccgtc   3960
ctgtttgcaa cggcgggcgt ggaacgcgac ctgaacggac gcgactacac ggtaacgggc   4020
ggctttaccg gcgcgactgc agcaaccggc aagacggggg cacgcaatat gccgcacacc   4080
cgtctggttg ccggcctggg cgcggatgtc gaattcggca acggctggaa cggcttggca   4140
cgttacagct acgccggttc caaacagtac ggcaaccaca gcggacgagt cggcgtaggc   4200
taccggttct gactcgag                                                 4218
```

<210> 58
<211> 1403
<212> PRT
<213> Artificial Sequence

<220>
<223> 961cL-983

<400> 58

```
Met Lys His Phe Pro Ser Lys Val Leu Thr Thr Ala Ile Leu Ala Thr
1               5                   10                  15

Phe Cys Ser Gly Ala Leu Ala Ala Thr Asn Asp Asp Asp Val Lys Lys
            20                  25                  30

Ala Ala Thr Val Ala Ile Ala Ala Ala Tyr Asn Asn Gly Gln Glu Ile
        35                  40                  45

Asn Gly Phe Lys Ala Gly Glu Thr Ile Tyr Asp Ile Asp Glu Asp Gly
    50                  55                  60

Thr Ile Thr Lys Lys Asp Ala Thr Ala Ala Asp Val Glu Ala Asp Asp
65                  70                  75                  80

Phe Lys Gly Leu Gly Leu Lys Lys Val Val Thr Asn Leu Thr Lys Thr
                85                  90                  95

Val Asn Glu Asn Lys Gln Asn Val Asp Ala Lys Val Lys Ala Ala Glu
            100                 105                 110

Ser Glu Ile Glu Lys Leu Thr Thr Lys Leu Ala Asp Thr Asp Ala Ala
        115                 120                 125

Leu Ala Asp Thr Asp Ala Ala Leu Asp Ala Thr Thr Asn Ala Leu Asn
    130                 135                 140

Lys Leu Gly Glu Asn Ile Thr Thr Phe Ala Glu Glu Thr Lys Thr Asn
145                 150                 155                 160

Ile Val Lys Ile Asp Glu Lys Leu Glu Ala Val Ala Asp Thr Val Asp
                165                 170                 175

Lys His Ala Glu Ala Phe Asn Asp Ile Ala Asp Ser Leu Asp Glu Thr
            180                 185                 190

Asn Thr Lys Ala Asp Glu Ala Val Lys Thr Ala Asn Glu Ala Lys Gln
        195                 200                 205

Thr Ala Glu Glu Thr Lys Gln Asn Val Asp Ala Lys Val Lys Ala Ala
    210                 215                 220

Glu Thr Ala Ala Gly Lys Ala Glu Ala Ala Ala Gly Thr Ala Asn Thr
225                 230                 235                 240
```

```
Ala Ala Asp Lys Ala Glu Ala Val Ala Ala Lys Val Thr Asp Ile Lys
                245                 250                 255

Ala Asp Ile Ala Thr Asn Lys Asp Asn Ile Ala Lys Lys Ala Asn Ser
            260                 265                 270

Ala Asp Val Tyr Thr Arg Glu Glu Ser Asp Ser Lys Phe Val Arg Ile
        275                 280                 285

Asp Gly Leu Asn Ala Thr Thr Glu Lys Leu Asp Thr Arg Leu Ala Ser
    290                 295                 300

Ala Glu Lys Ser Ile Ala Asp His Asp Thr Arg Leu Asn Gly Leu Asp
305                 310                 315                 320

Lys Thr Val Ser Asp Leu Arg Lys Glu Thr Arg Gln Gly Leu Ala Glu
                325                 330                 335

Gln Ala Ala Leu Ser Gly Leu Phe Gln Pro Tyr Asn Val Gly Gly Ser
            340                 345                 350

Gly Gly Gly Gly Thr Ser Ala Pro Asp Phe Asn Ala Gly Gly Thr Gly
        355                 360                 365

Ile Gly Ser Asn Ser Arg Ala Thr Thr Ala Lys Ser Ala Ala Val Ser
    370                 375                 380

Tyr Ala Gly Ile Lys Asn Glu Met Cys Lys Asp Arg Ser Met Leu Cys
385                 390                 395                 400

Ala Gly Arg Asp Asp Val Ala Val Thr Asp Arg Asp Ala Lys Ile Asn
                405                 410                 415

Ala Pro Pro Pro Asn Leu His Thr Gly Asp Phe Pro Asn Pro Asn Asp
            420                 425                 430

Ala Tyr Lys Asn Leu Ile Asn Leu Lys Pro Ala Ile Glu Ala Gly Tyr
        435                 440                 445

Thr Gly Arg Gly Val Glu Val Gly Ile Val Asp Thr Gly Glu Ser Val
    450                 455                 460

Gly Ser Ile Ser Phe Pro Glu Leu Tyr Gly Arg Lys Glu His Gly Tyr
465                 470                 475                 480

Asn Glu Asn Tyr Lys Asn Tyr Thr Ala Tyr Met Arg Lys Glu Ala Pro
                485                 490                 495

Glu Asp Gly Gly Gly Lys Asp Ile Glu Ala Ser Phe Asp Asp Glu Ala
            500                 505                 510

Val Ile Glu Thr Glu Ala Lys Pro Thr Asp Ile Arg His Val Lys Glu
        515                 520                 525

Ile Gly His Ile Asp Leu Val Ser His Ile Ile Gly Gly Arg Ser Val
    530                 535                 540

Asp Gly Arg Pro Ala Gly Gly Ile Ala Pro Asp Ala Thr Leu His Ile
545                 550                 555                 560

Met Asn Thr Asn Asp Glu Thr Lys Asn Glu Met Met Val Ala Ala Ile
```

565 570 575

Arg Asn Ala Trp Val Lys Leu Gly Glu Arg Gly Val Arg Ile Val Asn
580 585 590

Asn Ser Phe Gly Thr Thr Ser Arg Ala Gly Thr Ala Asp Leu Phe Gln
595 600 605

Ile Ala Asn Ser Glu Glu Gln Tyr Arg Gln Ala Leu Leu Asp Tyr Ser
610 615 620

Gly Gly Asp Lys Thr Asp Glu Gly Ile Arg Leu Met Gln Gln Ser Asp
625 630 635 640

Tyr Gly Asn Leu Ser Tyr His Ile Arg Asn Lys Asn Met Leu Phe Ile
645 650 655

Phe Ser Thr Gly Asn Asp Ala Gln Ala Gln Pro Asn Thr Tyr Ala Leu
660 665 670

Leu Pro Phe Tyr Glu Lys Asp Ala Gln Lys Gly Ile Ile Thr Val Ala
675 680 685

Gly Val Asp Arg Ser Gly Glu Lys Phe Lys Arg Glu Met Tyr Gly Glu
690 695 700

Pro Gly Thr Glu Pro Leu Glu Tyr Gly Ser Asn His Cys Gly Ile Thr
705 710 715 720

Ala Met Trp Cys Leu Ser Ala Pro Tyr Glu Ala Ser Val Arg Phe Thr
725 730 735

Arg Thr Asn Pro Ile Gln Ile Ala Gly Thr Ser Phe Ser Ala Pro Ile
740 745 750

Val Thr Gly Thr Ala Ala Leu Leu Leu Gln Lys Tyr Pro Trp Met Ser
755 760 765

Asn Asp Asn Leu Arg Thr Thr Leu Leu Thr Thr Ala Gln Asp Ile Gly
770 775 780

Ala Val Gly Val Asp Ser Lys Phe Gly Trp Gly Leu Leu Asp Ala Gly
785 790 795 800

Lys Ala Met Asn Gly Pro Ala Ser Phe Pro Phe Gly Asp Phe Thr Ala
805 810 815

Asp Thr Lys Gly Thr Ser Asp Ile Ala Tyr Ser Phe Arg Asn Asp Ile
820 825 830

Ser Gly Thr Gly Gly Leu Ile Lys Lys Gly Gly Ser Gln Leu Gln Leu
835 840 845

His Gly Asn Asn Thr Tyr Thr Gly Lys Thr Ile Ile Glu Gly Gly Ser
850 855 860

Leu Val Leu Tyr Gly Asn Asn Lys Ser Asp Met Arg Val Glu Thr Lys
865 870 875 880

Gly Ala Leu Ile Tyr Asn Gly Ala Ala Ser Gly Gly Ser Leu Asn Ser
885 890 895

Asp Gly Ile Val Tyr Leu Ala Asp Thr Asp Gln Ser Gly Ala Asn Glu
900 905 910

Thr Val His Ile Lys Gly Ser Leu Gln Leu Asp Gly Lys Gly Thr Leu
915 920 925

Tyr Thr Arg Leu Gly Lys Leu Leu Lys Val Asp Gly Thr Ala Ile Ile
930 935 940

Gly Gly Lys Leu Tyr Met Ser Ala Arg Gly Lys Gly Ala Gly Tyr Leu
945 950 955 960

Asn Ser Thr Gly Arg Arg Val Pro Phe Leu Ser Ala Ala Lys Ile Gly
965 970 975

Gln Asp Tyr Ser Phe Phe Thr Asn Ile Glu Thr Asp Gly Gly Leu Leu
980 985 990

Ala Ser Leu Asp Ser Val Glu Lys Thr Ala Gly Ser Glu Gly Asp Thr
995 1000 1005

Leu Ser Tyr Tyr Val Arg Arg Gly Asn Ala Ala Arg Thr Ala Ser Ala
1010 1015 1020

Ala Ala His Ser Ala Pro Ala Gly Leu Lys His Ala Val Glu Gln Gly
1025 1030 1035 1040

Gly Ser Asn Leu Glu Asn Leu Met Val Glu Leu Asp Ala Ser Glu Ser
1045 1050 1055

Ser Ala Thr Pro Glu Thr Val Glu Thr Ala Ala Ala Asp Arg Thr Asp
1060 1065 1070

Met Pro Gly Ile Arg Pro Tyr Gly Ala Thr Phe Arg Ala Ala Ala Ala
1075 1080 1085

Val Gln His Ala Asn Ala Ala Asp Gly Val Arg Ile Phe Asn Ser Leu
1090 1095 1100

Ala Ala Thr Val Tyr Ala Asp Ser Thr Ala Ala His Ala Asp Met Gln
1105 1110 1115 1120

Gly Arg Arg Leu Lys Ala Val Ser Asp Gly Leu Asp His Asn Gly Thr
1125 1130 1135

Gly Leu Arg Val Ile Ala Gln Thr Gln Gln Asp Gly Gly Thr Trp Glu
1140 1145 1150

Gln Gly Gly Val Glu Gly Lys Met Arg Gly Ser Thr Gln Thr Val Gly
1155 1160 1165

Ile Ala Ala Lys Thr Gly Glu Asn Thr Thr Ala Ala Ala Thr Leu Gly
1170 1175 1180

Met Gly Arg Ser Thr Trp Ser Glu Asn Ser Ala Asn Ala Lys Thr Asp
1185 1190 1195 1200

Ser Ile Ser Leu Phe Ala Gly Ile Arg His Asp Ala Gly Asp Ile Gly
1205 1210 1215

```
Tyr Leu Lys Gly Leu Phe Ser Tyr Gly Arg Tyr Lys Asn Ser Ile Ser
            1220                1225                1230

Arg Ser Thr Gly Ala Asp Glu His Ala Glu Gly Ser Val Asn Gly Thr
        1235                1240                1245

Leu Met Gln Leu Gly Ala Leu Gly Gly Val Asn Val Pro Phe Ala Ala
    1250                1255                1260

Thr Gly Asp Leu Thr Val Glu Gly Gly Leu Arg Tyr Asp Leu Leu Lys
1265                1270                1275                1280

Gln Asp Ala Phe Ala Glu Lys Gly Ser Ala Leu Gly Trp Ser Gly Asn
                1285                1290                1295

Ser Leu Thr Glu Gly Thr Leu Val Gly Leu Ala Gly Leu Lys Leu Ser
            1300                1305                1310

Gln Pro Leu Ser Asp Lys Ala Val Leu Phe Ala Thr Ala Gly Val Glu
        1315                1320                1325

Arg Asp Leu Asn Gly Arg Asp Tyr Thr Val Thr Gly Gly Phe Thr Gly
    1330                1335                1340

Ala Thr Ala Ala Thr Gly Lys Thr Gly Ala Arg Asn Met Pro His Thr
1345                1350                1355                1360

Arg Leu Val Ala Gly Leu Gly Ala Asp Val Glu Phe Gly Asn Gly Trp
                1365                1370                1375

Asn Gly Leu Ala Arg Tyr Ser Tyr Ala Gly Ser Lys Gln Tyr Gly Asn
            1380                1385                1390

His Ser Gly Arg Val Gly Val Gly Tyr Arg Phe
        1395                1400
```

<210> 59
<211> 25
<212> DNA
<213> Artificial Sequence

<220>
<223> Oligonucleotide

<400> 59
cgcggatccg gagggggtgg tgtcg 25

<210> 60
<211> 27
<212> DNA
<213> Artificial Sequence

<220>
<223> Oligonucleotide

<400> 60
cccgctcgag ttgcttggcg gcaaggc 27

<210> 61
<211> 25
<212> DNA
<213> Artificial Sequence

<220>
<223> Oligonucleotide

<400> 61
cgcggatccg gcggaggcgg cactt 25

<210> 62
<211> 26
<212> DNA
<213> Artificial Sequence

<220>
<223> Oligonucleotide

<400> 62
cccgctcgag gaaccggtag cctacg 26

<210> 63
<211> 41
<212> DNA
<213> Artificial Sequence

<220>
<223> Oligonucleotide

<400> 63
cgcggatccg gtggtggtgg ttcagatttg gcaaacgatt c 41

<210> 64
<211> 29
<212> DNA
<213> Artificial Sequence

<220>
<223> Oligonucleotide

<400> 64
cccgctcgag cgtatcatat ttcacgtgc 29

<210> 65
<211> 25
<212> DNA
<213> Artificial Sequence

<220>
<223> Oligonucleotide

<400> 65
cgcggatccg gagggggtgg tgtcg 25

<210> 66
<211> 28
<212> DNA

<213> Artificial Sequence

<220>
<223> Oligonucleotide

<400> 66
cccgctcgag ttattgcttg gcggcaag 28

<210> 67
<211> 25
<212> DNA
<213> Artificial Sequence

<220>
<223> Oligonucleotide

<400> 67
cgcggatccg gcggaggcgg cactt 25

<210> 68
<211> 28
<212> DNA
<213> Artificial Sequence

<220>
<223> Oligonucleotide

<400> 68
cccgctcgag tcagaaccgg tagcctac 28

<210> 69
<211> 41
<212> DNA
<213> Artificial Sequence

<220>
<223> Oligonucleotide

<400> 69
cgcggatccg gtggtggtgg ttcagatttg gcaaacgatt c 41

<210> 70
<211> 32
<212> DNA
<213> Artificial Sequence

<220>
<223> Oligonucleotide

<400> 70
cccgctcgag ttacgtatca tatttcacgt gc 32

<210> 71
<211> 42
<212> DNA
<213> Artificial Sequence

<220>

<223> Oligonucleotide

<400> 71
cgcggatccg gtggtggtgg tcaaagcaag agcatccaaa cc 42

<210> 72
<211> 30
<212> DNA
<213> Artificial Sequence

<220>
<223> Oligonucleotide

<400> 72
cccaagcttt tcgggcggta ttcgggcttc 30

<210> 73
<211> 39
<212> DNA
<213> Artificial Sequence

<220>
<223> Oligonucleotide

<400> 73
cgcggatccg gtggtggtgg tgccacctac aaagtggac 39

<210> 74
<211> 28
<212> DNA
<213> Artificial Sequence

<220>
<223> Oligonucleotide

<400> 74

gcccaagctt ttgtttggct gcctcgat 28

<210> 75

<211> 34
<212> DNA
<213> Artificial Sequence

<220>
<223> Oligonucleotide

<400> 75
cgcggatccg gtggtggtgg tacaagcgac gacg 34

<210> 76
<211> 28
<212> DNA
<213> Artificial Sequence

<220>
<223> Oligonucleotide

<400> 76
gcccaagctt ccactcgtaa ttgacgcc 28

<210> 77
<211> 41
<212> DNA
<213> Artificial Sequence

<220>
<223> Oligonucleotide

<400> 77
cgcggatccg gtggtggtgg ttcagatttg gcaaacgatt c 41

<210> 78
<211> 28
<212> DNA
<213> Artificial Sequence

<220>
<223> Oligonucleotide

<400> 78
cccaagcttc gtatcatatt tcacgtgc 28

<210> 79
<211> 44
<212> DNA
<213> Artificial Sequence

<220>
<223> Oligonucleotide

<400> 79
cccaagcttg gtggtggtgg tggttcagat ttggcaaacg attc 44

<210> 80
<211> 29
<212> DNA
<213> Artificial Sequence

<220>
<223> Oligonucleotide

<400> 80
cccgctcgag cgtatcatat ttcacgtgc 29

<210> 81
<211> 45
<212> DNA
<213> Artificial Sequence

<220>

<223> Oligonucleotide

<400> 81
cccaagcttg gtggtggtgg tggtcaaagc aagagcatcc aaacc 45

<210> 82
<211> 28
<212> DNA
<213> Artificial Sequence

<220>
<223> Oligonucleotide

<400> 82
cccgctcgag cgggcggtat tcgggctt 28

<210> 83
<211> 32
<212> DNA
<213> Artificial Sequence

<220>
<223> Oligonucleotide

<400> 83
cgcggatccg ctagccccga tgttaaatcg gc 32

<210> 84
<211> 29
<212> DNA
<213> Artificial Sequence

<220>
<223> Oligonucleotide

<400> 84
cggggatcca tcctgctctt ttttgccgg 29

<210> 85
<211> 36
<212> DNA
<213> Artificial Sequence

<220>
<223> Oligonucleotide

<400> 85
cgcggatccg ctagcggaca cacttatttc ggcatc 36

<210> 86
<211> 30
<212> DNA
<213> Artificial Sequence

<220>
<223> Oligonucleotide

<400> 86
cgcggatccc cagcggtagc ctaatttgat 30

<210> 87
<211> 41
<212> DNA

<213> Artificial Sequence

<220>
<223> Oligonucleotide

<400> 87
cgcggatccg gtggtggtgg ttcagatttg gcaaacgatt c 41

<210> 88
<211> 28
<212> DNA
<213> Artificial Sequence

<220>
<223> Oligonucleotide

<400> 88
cccaagcttc gtatcatatt tcacgtgc 28

<210> 89
<211> 36
<212> DNA
<213> Artificial Sequence

<220>
<223> Oligonucleotide

<400> 89
gcggcgtcga cggtggcgga ggcactggat cctcag 36

<210> 90
<211> 35
<212> DNA
<213> Artificial Sequence

<220>
<223> Oligonucleotide

<400> 90
ggaggcactg gatcctcaga tttggcaaac gattc 35

<210> 91
<211> 29
<212> DNA
<213> Artificial Sequence

<220>
<223> Oligonucleotide

<400> 91
cccgctcgag cgtatcatat ttcacgtgc 29

<210> 92
<211> 25
<212> DNA
<213> Artificial Sequence

<220>

<223> Oligonucleotide

<400> 92
cggggatccg ggggcggcgg tggcg 25

<210> 93
<211> 30
<212> DNA
<213> Artificial Sequence

<220>
<223> Oligonucleotide

<400> 93
cccaagctta tcctgctctt ttttgccggc 30

<210> 94
<211> 42
<212> DNA
<213> Artificial Sequence

<220>
<223> Oligonucleotide

<400> 94
cgcggatccg gtggtggtgg tcaaagcaag agcatccaaa cc 42

<210> 95
<211> 28
<212> DNA
<213> Artificial Sequence

<220>
<223> Oligonucleotide

<400> 95
cccaagcttc gggcggtatt cgggcttc 28

<210> 96
<211> 26
<212> DNA
<213> Artificial Sequence

<220>
<223> Oligonucleotide

<400> 96
ccccaagctt gggggcggcg gtggcg 26

<210> 97
<211> 31
<212> DNA
<213> Artificial Sequence

<220>
<223> Oligonucleotide

<400> 97

cccgctcgag atcctgctct tttttgccgg c 31

<210> 98
<211> 45
<212> DNA
<213> Artificial Sequence

<220>
<223> Oligonucleotide

<400> 98
cccaagcttg gtggtggtgg tggtcaaagc aagagcatcc aaacc 45

<210> 99
<211> 28
<212> DNA
<213> Artificial Sequence

<220>
<223> Oligonucleotide

<400> 99
cccgctcgag cgggcggtat tcgggctt 28

<210> 100
<211> 35
<212> DNA
<213> Artificial Sequence

<220>
<223> Oligonucleotide

<400> 100
ggaggcactg gatccgcagc cacaaacgac gacga 35

<210> 101
<211> 36
<212> DNA
<213> Artificial Sequence

<220>
<223> Oligonucleotide

<400> 101

gcggcctcga gggtggcgga ggcactggat ccgcag 36

<210> 102
<211> 28
<212> DNA
<213> Artificial Sequence

<220>
<223> Oligonucleotide

<400> 102
cccgctcgag acccagcttg taaggttg 28

<210> 103
<211> 35
<212> DNA
<213> Artificial Sequence

<220>
<223> Oligonucleotide

<400> 103
ggaggcactg gatccgcagc cacaaacgac gacga 35

<210> 104
<211> 36
<212> DNA
<213> Artificial Sequence

<220>
<223> Oligonucleotide

<400> 104
gcggcctcga gggtggcgga ggcactggat ccgcag 36

<210> 105
<211> 28
<212> DNA
<213> Artificial Sequence

<220>
<223> Oligonucleotide

<400> 105
cccgctcgag ccactcgtaa ttgacgcc 28

<210> 106
<211> 38
<212> DNA
<213> Artificial Sequence

<220>
<223> Oligonucleotide

<400> 106
gcggcctcga gggatccggc ggaggcggca cttctgcg 38

<210> 107
<211> 26
<212> DNA
<213> Artificial Sequence

<220>
<223> Oligonucleotide

<400> 107
cccgctcgag gaaccggtag cctacg 26

<210> 108
<211> 35
<212> DNA

<213> Artificial Sequence

<220>
<223> Oligonucleotide

<400> 108
ggaggcactg gatcctcaga tttggcaaac gattc 35

<210> 109
<211> 37
<212> DNA
<213> Artificial Sequence

<220>
<223> Oligonucleotide

<400> 109
gcggcgtcga cggtggcgga ggcactggat cctcaga 37

<210> 110
<211> 29
<212> DNA
<213> Artificial Sequence

<220>
<223> Oligonucleotide

<400> 110
cccgctcgag cgtatcatat ttcacgtgc 29

<210> 111
<211> 35
<212> DNA
<213> Artificial Sequence

<220>
<223> Oligonucleotide

<400> 111
gcggcctcga gggatccgga gggggtggtg tcgcc 35

<210> 112
<211> 25
<212> DNA
<213> Artificial Sequence

<220>
<223> Oligonucleotide

<400> 112
cccgctcgag ttgcttggcg gcaag 25

<210> 113
<211> 35
<212> DNA
<213> Artificial Sequence

<220>

<223> Oligonucleotide

<400> 113
ggaggcactg gatccgcagc cacaaacgac gacga 35

<210> 114
<211> 36
<212> DNA
<213> Artificial Sequence

<220>
<223> Oligonucleotide

<400> 114
gcggcctcga gggtggcgga ggcactggat ccgcag 36

<210> 115
<211> 28
<212> DNA
<213> Artificial Sequence

<220>
<223> Oligonucleotide

<400> 115
cccgctcgag acccagcttg taaggttg 28

<210> 116
<211> 35
<212> DNA
<213> Artificial Sequence

<220>
<223> Oligonucleotide

<400> 116
ggaggcactg gatccgcagc cacaaacgac gacga 35

<210> 117
<211> 36
<212> DNA
<213> Artificial Sequence

<220>
<223> Oligonucleotide

<400> 117
gcggcctcga gggtggcgga ggcactggat ccgcag 36

<210> 118
<211> 28
<212> DNA
<213> Artificial-Sequence

<220>
<223> Oligonucleotide

<400> 118

cccgctcgag ccactcgtaa ttgacgcc 28

<210> 119
<211> 35
<212> DNA
<213> Artificial Sequence

<220>
<223> Oligonucleotide

<400> 119
ggaggcactg gatcctcaga tttggcaaac gattc 35

<210> 120
<211> 37
<212> DNA
<213> Artificial Sequence

<220>
<223> Oligonucleotide

<400> 120
gcggcgtcga cggtggcgga ggcactggat cctcaga 37

<210> 121
<211> 29
<212> DNA
<213> Artificial Sequence

<220>
<223> Oligonucleotide

<400> 121
cccgctcgag cgtatcatat ttcacgtgc 29